# EUROPEAN PATENT SPECIFICATION

(11) **EP 4 146 658 B1**
(45) Date of publication and mention of the grant of the patent: **29.10.2025**
(21) Application number: 21722503.6
(22) Date of filing: 05.05.2021
(51) Int. Cl.: C07D 498/16, C07D 498/18, A61P 25/28, A61K 31/4162

(54) **NEW MACROCYCLIC LRRK2 KINASE INHIBITORS**
NEUE MAKROCYCLISCHE LRRK2-KINASEINHIBITOREN
NOUVEAUX INHIBITEURS DE KINASE LRRK2 MACROCYCLIQUE

(30) Priority: 06.05.2020 EP 20315236; 28.01.2021 EP 21305112
(43) Date of publication of application: 15.03.2023
(73) Proprietor: Oncodesign Precision Medicine (OPM), 21000 Dijon (FR)
(72) Inventor: BLOM, Petra Marcella, 9070 Destelbergen (BE); HOUSSEMAN, Christopher Gaétan, 91300 Massy (FR); DAUGAN, Alain, 91640 Vaugrigneuse (FR); DUMOULIN, Audrey, 92160 Antony (FR); LAUGEOIS, Maxime, 75017 Paris (FR); DENIS, Alexis, 75011 Paris (FR); FAUCHER, Nicolas, 92150 Suresnes (FR); BOTEZ, Iuliana, 92500 Rueil Malmaison (FR); LE TIRAN, Arnaud, 78290 Croissy sur Seine (FR); CHRISTENSEN, Kenneth, 2100 Copenhagen (DK); LAMOTTE, Yann, 92160 Antony (FR)
(74) Representative: Arnold & Siedsma
(86) International application number: PCT/EP2021/061827
(87) International publication number: WO 2021/224320

(56) References cited:
- WO-A1-2015/026683
- WO-A1-2019/241540
- US-A1- 2015 290 198

## Description

### Field of the invention

The present invention relates to novel macrocyclic compounds and compositions containing said compounds acting as kinase inhibitors, in particular as inhibitors of LRRK2 (Leucine-Rich Repeat Kinase 2). Moreover, the present invention provides processes for the preparation of the disclosed compounds, pharmaceutical compositions containing them, as well as methods of using them, for instance as a medicine or diagnostic agent, in particular for the treatment and/or diagnosis of diseases impacted or modulated by LRRK2 kinase activity such as neurological disorders including Parkinson's disease and Alzheimer's disease, but also cardiac diseases or inflammatory disorders such as Crohn's disease.

### Background of the invention

Parkinson's disease is the most common movement disorder and the second most common neurodegenerative disease after Alzheimer's disease. Parkinson's disease affects approximately 1% of the population above 65 years and is characterized by the four classical core motor complications: resting tremor, bradykinesia, postural instability and muscular rigidity. Patients with Parkinson's disease are also impacted by a host of non-motor symptoms such as constipation, hyposmia, orthostatic hypotension, sleep disturbances including REM sleep behavior disorder, dementia, visual disturbances, depression, anxiety, hallucinations and mood swings.

Standard of care in Parkinson's disease is symptomatic relief of motor complications using dopamine replacement therapy such as the dopamine precursor L-dopa, dopamine agonists or compounds that impact the half-life of dopamine such as MAO-B inhibitors. As of today, there is no approved therapy to prevent, cure or delay the progression of Parkinson's disease.

The pathological hallmarks of Parkinson's disease are the loss of dopaminergic neurons in the substantia nigra pars compacta as well as postmortem evidence of protein inclusions, also known as Lewy bodies and Lewy neurites. In postmortem tissue from Parkinson's disease patients Lewy bodies and neurites are seen throughout the central nervous system and in peripheral tissues as well. A major component of the inclusions is the aggregated and misfolded α-synuclein protein phosphorylated at a serine at amino acid position 129 *(*Nature 388, 839-840, 1997*;* Nat Cell Biol 4, 160 64, 2002*).* Lewy bodies and neurites also contain proteins implicated in other neurodegenerative diseases such as the hyperphosphorylated tau protein which is a pathological hallmark of tauopathies such as Alzheimer's disease (AD), frontotemporal dementia (FTD), progressive supranuclear palsy (PSP) and corticobasal degeneration (CBD) *(*Biochem Soc Trans 26(3), 463-71, 1998*;* Am J Hum Genet 64(2), 414-21, 1999*;* J Neuropathol Exp Neurol 62(4), 389-97, 2003*).* The pathological process in Parkinson's disease is not restricted to the loss of dopaminergic neurons in the basal ganglia system. Distinct neuronal populations in other brain regions such as the neocortex, sleep nuclei or the raphe nucleus as well as peripheral organs and tissues such as the heart and the gastro-intestinal system are also impacted by degenerative processes in Parkinson's disease patients.

Leucine-rich repeat kinase 2 (LRRK2) is a 2527 amino acid protein with a molecular weight of 286 kDa that is encoded by the LRRK2 gene. It consists of the following functional and structural proteins domains: armadillo (ARM), ankyrin (ANK), leucine rich repeat (LRR), Ras of complex domain (Roc), c-terminal of Roc (COR), map kinase (MAPK) and tryptophan-aspartate repeat domain (WD40). LRRK2 exists primarily as a dimeric protein either associated with membrane structures or cytoplasmic localized. The armadillo, ankyrin, LRR and WD40 protein-protein interaction domains enables LRRK2 to interact with a host of different protein partners to impact its own as well as its partner proteins subcellular localization. The central enzymatic core of the LRRK2 protein containing the Roc-COR and the MAPK domain have distinct GTPase and ATPase enzymatic activities enabling LRRK2 to phosphorylate and control the function of intracellular substrates. LRRK2 impacts, via its enzymatic activity and substrate interactions, various subcellular processes and biological mechanisms important for trafficking of intracellular vesicular structures and organelles such as lysosomes, endosomes, autophagosomes, the Golgi and mitochondria. Structural work as well as modelling highlights how naturally occurring missense variation in functional and structural domains of LRRK2 impacts enzymatic activity *(bioRxiv 2020.01.06.895367).* In the inactive (open) LRRK2 conformation there are major interactions between the enzymatic GTPase (Roc-COR) and ATPase (MAPK) domains. In addition, the ultimate C-terminal proceeding the WD40 domain binds along the entire kinase (MAPK) domain. In the active (closed) LRRK2 conformation the LRR domain positions the autophosphorylation site Ser1292 in proximity to the kinase active site. Phosphorylation of LRRK2 at a cluster of serines immediately preceding the LRR domain enables the LRR domain of LRRK2 to bind to 14-3-3 proteins. Among those phosphorylation sites are serines (Ser) at the following amino acid positions: Ser910, Ser935, Ser955 and Ser973. Pathogenic LRRK2 mutations originating in the GTPase domain has diminished phosphorylation at these sites and therefore reduced 14-3-3 binding leading to increased microtubule network recruitment. All ATP-competitive LRRK2 inhibitors induce dephosphorylation at the Ser910, Ser935, Ser955 and Ser973 sites making these sites useful as surrogate target engagement markers *(*Biochem J 430(3), 405-13, 2010*;* J Neurochem 120(1), 37-45, 2012*).* The bona fide LRRK2 substrates consists of a subset of small Rab GTPases including Rab10 and Rab29. The Golgi-resident protein Rab29 also known as Rab7L1 is a Parkinson's disease susceptibility gene located at the PARK16 locus *(*Nat Genet 41(12), 1308-12, 2009*).*

Rare protein-encoding variants in the LRRK2 gene cause Parkinson's disease. The most common pathogenic variant causing autosomal dominant familial Parkinson's disease is the p.G2019S substitution which changes a glycine to a serine in the activation loop of the LRRK2 kinase domain rendering the p.G2019S variant more active than the wild type LRRK2 protein *(*Lancet 365(9457), 412-5, 2005*).* This results in increased autophosphorylation at the serine at amino acid position 1292 *(*Sci Transl Med, 4(164), 164ra161, 2012*).* The estimated worldwide prevalence of the p.G2019S mutation in patients with PD is 1-2%; whereas , in Ashkenazi Jewish and North African Arab-Berber populations the p.G2019S prevalence in PD patients is up to 30% and 40%, respectively (Lancet Neurol 7, 583-90, 2008*;* N Engl J Med 354(4), 424-5, 2006*;* Lancet Neurol 7, 591-4, 2008*).* The clinical manifestation of Parkinson's disease in patients carrying the p.G2019S mutation is indistinguishable from patients with the sporadic form of Parkinson's disease *(*Ann Neurol 57(5), 762-5, 2005*).* Besides p.G2019S seven additional rare LRRK2 exonic variants having non-synonymous amino acid substitutions in the central enzymatic core (p.N1437H; p.R1441C/G/H; p.Y1699C; p.S1761R; p.I2020T) also cause autosomal dominant Parkinson's disease *(*Parkinsonism Relat Disord 15(6), 466-7, 2009*;* Mov Disord 25(14), 2340-5, 2010*;* Neuron 44(4), 601-7, 2004*;* Parkinsonism Relat Disord 18(4), 332-8, 2012*;* Ann Neurol 57(6), 918-21, 2005*;* Mov Disord 27(1), 146-51, 2012*).* As with p.G2019S the clinical representations are indistinguishable from idiopathic PD *(*Neurology 70, 1456-60, 2008*).* LRRK2 missense variants exhibit increased Ser1292 phosphorylation, increased trans-Golgi recruitment by Rab29 and increased phosphorylation of Rab10 at amino acid position 73 (Rab10-Thr73) that can be reversed by LRRK2 inhibition *(*Sci Transl Med 4(164), 164ra161, 2012*;* EMBO J 37(1), 1-18, 2018*;* Proc Natl Acad Sci USA 111, 2626-31, 2014*).* Common protein-coding variants in the LRRK2 gene are also associated with risk of Parkinson's disease. Variants such as p.A419V, p.M1646T, p.R1628P and p.G2385R increase the risk of Parkinson's disease and have increased kinase activity *(bioRxiv 447946, 2018) (*Proc Natl Acad Sci USA 116(5), 1579-1584, 2019*)* whereas the p.N551K variant is associated with reduced risk of Parkinson's disease *(*Lancet Neurol 10(10), 898-908, 2011*)* and have reduced kinase activity *(bioRxiv 447946, 2018).* Evidence that LRRK2 also plays a role in sporadic Parkinson's disease comes from both genetic studies as well as postmortem analyses of PD brains. A single nucleotide polymorphism (SNP) at the LRRK2 genetic locus is genome-wide associated with risk of Parkinson's disease *(*Nat Genet 46(9), 989-93, 2014*).* This particular SNP variant is associated with increased LRRK2 expression *(*Sci Transl Med 9 (421), 2017*)* which is in agreement with the increased LRRK2 kinase activity observed in surviving dopamine neurons from postmortem brains of sporadic PD patients *(*Sci Transl Med 10 (451), 2018*).*

Thus, inhibitors of LRRK2 kinase activity can be used as therapies for both sporadic PD patients as well as for PD patients with LRRK2 mutations or Rab29/Rab7L1 polymorphisms. Parkinson's disease risk loci containing several genes encoding proteins involved in endosomal-lysosomal processes such as GBA, SCARB2, GALC, VPS35, LAMP1, VPS13C, VPS35, TMEM175, ATP6V0A1 and CTSB have been identified by Genome Wide Association Study (GWAS) and linkage studies. LRRK2 also plays a key role in the endosomal-lysosomal system and in the processes linked to endosomal function such as autophagy and mitophagy. LRRK2 interacts with the vacuolar H+-ATPase α subunit to regulate lysosomal pH and endosomal-lysosomal dysfunction induced by rotenone, a toxin known to be associated with increased risk of Parkinson's disease, can be alleviated by LRRK2 inhibition *(*Neurobiol Dis 134, 104626, 2020*).* Disease-causing LRRK2 mutations induce lysosomal stress by enlarging lysosomes *(*Hum Mol Genet 24(21), 6013-28, 2015*).* Likewise, an aspartate to asparagine missense mutation in the retromer complex protein VPS35 at amino acid position 620 (VPS35-D620N) causes late onset autosomal dominant familial Parkinson's disease. In the disease state the VPS35-D620N missense mutation disrupts trafficking of cathepsin D, the protease responsible for degradation of α-synuclein (Traffic 15(2), 230-44, 2014*)* and activates LRRK2 which leads to increased autophosphorylation at the LRRK2-Ser1292 site and increased Rab10-Thr73 phosphorylation *(*Biochem J 475(11), 1861-1883, 2018*).* In the lysosomes LRRK2 interacts with GBA that is causally linked with the lysosomal storage disorder Gaucher's disease and a risk gene for Parkinson's disease. LRRK2 missense mutations reduce GBA activity that can be counteracted by LRRK2 inhibition *(*Nat Commun 10(1), 5570, 2019*).* Reversely, GBA disease-relevant deficits in lysosomal biology processes in astrocytes can also be alleviated by LRRK2 inhibition *(*Mov Disord Feb 8, 2020, doi: 10.1002/mds.27994*).* Missense mutations in the mitochondrial kinase PINK1 and the E3 ligase PARKIN both cause autosomal recessive early onset Parkinson's disease that is associated with mitochondrial dysfunction *(*Science 304(5674), 1158-60, 2004*;* Nature 392(6676), 605-8, 1998*).* LRRK2-dependent phoshorylation of Rab8a on threonine at amino acid position 72 is modulated by PINK1 phosphorylation of serine on amino acid position 111 on Rab8a *(*Biochem J. Mar 30, 2020, doi: 10.1042/BCJ20190664). Besides this LRRK2 activity impairs mitophagy that under normal conditions is regulated by the PINK1/PARKIN pathway. This can be reversed by LRRK2 inhibition *(*Hum Mol Genet 28(10), 1645-1660, 2019*).* LRRK2 missense mutations cause mitochondrial DNA damage that can be reversed by gene corrections *(*Neurobiol Dis 62, 381-6, 2014*)* as well as with inhibitors of LRRK2 *(*Hum Mol Genet. 26(22), 4340-4351, 2017*).* This suggests that LRRK2 inhibitors are useful for treating lysosomal storage disorders such as Gaucher's disease, Krabbe's disease, Niemann-Pick's disease and Fabry's disease, disorders with mitochondrial deficits including early onset Parkinson's disease associated with PINK1 and PARKIN missense mutations as well as Parkinson's disease in patients with polymorphisms in genes encoding proteins involved in the endosomal-lysosomal system such as GBA, GALC, VPS35, VPS13C, ATP6V0A1, LAMP1, SCARB2, TMEM175 and CTSB. Postmortem analysis of brains from Parkinson's disease patient carrying LRRK2 mutations show presence of α-synuclein pathology *(*JAMA Neurol. 72(1), 100-5, 2015*).* In preclinical Parkinson's disease (PD) models, p.G2019S aggravates PD-related pathology that can be reversed by LRRK2 inhibition. LRRK2 has been identified in Lewy bodies in nigral and brain stem regions *(*Neuropathol Appl Neurobiol 34(3), 272-83, 2008*)* and has also been shown to phosphorylate α-synuclein on Ser129 *(*Biochem Biophys Res Commun 387(1), 149-52, 2009*).* LRRK2 exonic variation is associated with risk of multiple system atrophy *(*Neurology 83(24), 2256-61, 2014*)* and LRRK2 missense mutations have also been reported in patients with multiple system atrophy *(*J Parkinsons Dis;8(1), 93-100, 2018*).* Single nucleotide polymorphisms in the MAPT (tau) locus is associated with increased risk of Parkinson's disease and multiple system atrophy *(*Hum Genet 124(6), 593-605, 2009*;* Parkinsonism Relat Disord 30, 40-5, 2016*).* Tau pathology is also a prominent feature seen in Parkinson's disease patients with LRRK2 missense mutations *(*Acta Neuropathol Commun 7(1), 183, 2019*).* Overexpression of pathogenic LRRK2 in animal models increase tau pathology *(*Neurobiol Dis 40(3), 503-17, 2010*).* LRRK2 missense mutations have been reported in patients suffering from tauopathies such as progressive supranuclear palsy and corticobasal degeneration *(*Mov Disord. 32(1), 115-123, 2017*).* Common variation at the LRRK2 locus is associated with survival in the primary tauopathy progressive supranuclear palsy *(bioRxiv 2020.02.04.932335)* and GWAS studies have identified risk for frontotemporal dementia at the LRRK2 locus *(*PLoS Med 15(1), e1002487, 2018*).*

This suggests that LRRK2 inhibitors are useful for treating synucleinopathies and tauopathies including frontotemporal dementia, progressive supranuclear palsy, corticobasal degeneration and Alzheimer's disease.

LRRK2 mRNA and protein are broadly expressed but particular enriched in brain tissue as well as in peripheral organs more specifically kidney, lung, intestine and spleen. Besides this LRRK2 expression is highly enriched in immune cells in the brain and in neutrophils, B-cells, macrophages and monocytes in the periphery. LRRK2 mRNA and protein expression is induced after pro-inflammatory stimuli or pathogens thereby increasing LRRK2 kinase activity. In human peripheral blood mononuclear cells, the LRRK2 substrates Rab10 and Rab12 are phosphorylated after stimulation with reagents mimicking viral infections *(*Sci Rep 7(1), 10300, 2017*).* Consistent with LRRK2 biology playing a role in response to inflammatory stimuli LRRK2 missense mutations are associated with risk of the inflammatory bowel disorder Crohn's disease and GWAS studies has identified single nucleotide polymorphisms in the LRRK2 locus associated with genome wide significant risk of Crohn's disease *(*Inflamm Bowel Dis 17(12), 2407-15, 2011*).* In Ashkenazi Jewish populations there is a two- to four-fold increased prevalence of Crohn's disease and in the same population LRRK2 variants are associated with increased risk of Crohn's disease *(*PLoS Genet 14(5), e1007329, 2018*).* LRRK2 exonic variants such as p.N2081D and p.M2397T increase the risk of Crohn's disease and as observed for Parkinson's disease the protective haplotype variant p.N551K/p.R1348H lowers the risk of Crohn's disease. In cell-based studies the p.N2081D variant has increased kinase activity which leads to augmented Rab10 phosphorylation *(bioRxiv 447946, 2018;* Sci Transl Med 10(423), 2018). The biological link between Parkinson's disease and autoimmune disorders are further supported by studies finding that common genetic pathways which also includes LRRK2 are shared between Parkinson's disease and autoimmune disorders such as rheumatoid arthritis, ulcerative colitis and Crohn's disease *(*JAMA Neurol 74(7), 780-92, 2017*).* Consistent with this LRRK2 is also associated with risk of lupus *(*Oncotarget8, 13754-61, 2017*;* J Transl Med 17(1), 37, 2019*)* and leprosy (N Engl J Med 361(27), 2609-18, 2009*;* PLoS One 8(8), e73103, 2013*;* PLoS Negl Trop Dis 10(2), e0004412, 2016*).*

Thus, LRRK2 inhibitors can be used for treatment of Crohn's disease and other autoimmune disorder such as but not restricted to rheumatoid arthritis, ulcerative colitis, lupus and leprosy. LRRK2 plays a role in tumor growth in renal and thyroid cancers by impacting MET signaling, and lowering of LRRK2 expression induces growth arrest *(*Proc Natl Acad Sci USA 108(4), 1439-44, 2011*).* LRRK2-PD patients have increased risks of leukemia as well as skin and colon cancers *(*Mov Disord 34(9), 1392-8, 2019*).* Carriers of p.G2019S also have an overall increased risk of non-skin cancer;in particular breast cancer and hormone-related cancers in females *(*JAMA Neurol 72(1), 58-65, 2015*).* Studies have shown that LRRK2 silencing promotes T-cell growth inhibition and facilitates apoptosis and cell cycle arrest (Int J Oncol 55(1), 21-34, 2019*).*

LRRK2 is also differentially expressed in lung adeno- and lung squamous cell carcinomas as well as non-small-cell lung cancer *(*J Cell Physiol 234(7), 10918-25, 2019*;* J Cell Physiol 234(12), 22742-52, 2019*).*

Thus, LRRK2 inhibitors have anti-carcinogenic effects and can be used for treatment of skin cancer and non-skin cancers such as renal cancer, colon cancer, adeno- and squamous lung cancers, non-small-cell lung cancer, hormone-related cancer, thyroid cancer, leukemia and breast cancer.

Extended prior art is known in the field of LRRK2 inhibitors. The most recent patent applications filed in the field cover oligomeric derivatives such as compounds disclosed in WO2020/006267, non-macrocyclic or polycyclic structures such as compounds disclosed in WO2019/222173, WO2019/112269, WO2019/074809, WO2018/217946, WO2018/163066, WO2018/155916, WO2018/137618, WO2018/06931, WO2015/026683, and also macrocyclic derivatives such as compounds disclosed in WO2019/012093, WO2016/042089, US2015/290198. Notwithstanding the huge amounts of structures elaborated over the last years, there is a continuing need to design new scaffolds having a better potency and selectivity to meet the unmet medical needs.

### Detailed description of the invention

The present invention will be described below. In the following passages, different aspects of the invention are defined in more details. Each aspect so defined may be combined with any other aspect or aspects unless clearly indicated to the contrary. In particular, any feature indicated as being preferred or advantageous may be combined with any other feature or features indicated as being preferred or advantageous.

In a first aspect the present invention provides a compound of Formula (I) 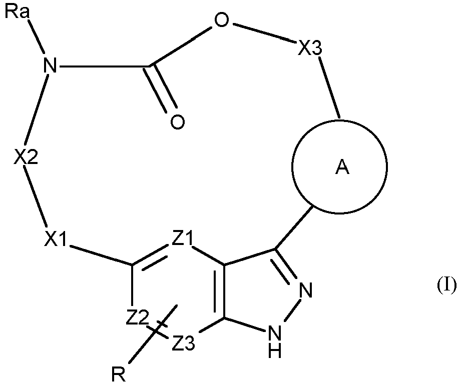 wherein:
◆ R represents a hydrogen atom, a halogen atom or an alkyl group,
◆ Z1, Z2, Z3, independently each represents a carbon or a nitrogen atom, it being understood that the 6-membered cycle containing Z1, Z2 and Z3 can have 0, 1 or 2 nitrogen atoms,
◆ -X1- is absent or represents -O-, -S-, or -N(R'a)-, wherein R'a represents a hydrogen atom or an alkyl group,
◆ -X2- represents an alkanediyl group optionally substituted with one or more substituents, identical or different, selected from halogen atoms, polyhalogenoalkyl group, alkoxy group, hydroxy group, amino group, alkylamino group, dialkylamino group and cyano group,
   it being understood that the carbon atom in the alpha position of -N(Ra), and the carbon atom in alpha position of -X1- when -X1- represents -O-, -S-, or -N(R'a)-, cannot be substituted with an oxygen or a nitrogen heteroatom,
◆ -X3- represents an alkanediyl group optionally substituted with one or more substituents, identical or different, selected from halogen atoms, polyhalogenoalkyl group, alkoxy group, hydroxy group, amino group, alkylamino group, dialkylamino group, cyano group, cycloalkyl group and heterocycloalkyl group,
   it being understood that the carbon atom in alpha position of -O-, and the carbon atom in alpha position of A1 when A1 represents a nitrogen atom, cannot be substituted with an oxygen or a nitrogen heteroatom,
◆ Ra represents a hydrogen atom or an alkyl group,
   it being understood that when Ra represents an alkyl group, one carbon atom of Ra can be linked to a carbon atom of -X2-, or to a carbon atom of -X3- to form a cyclic moiety containing 5 or 6 ring-members,
◆ A represents
   - an aromatic or partially hydrogenated cyclic group of the formula (a): 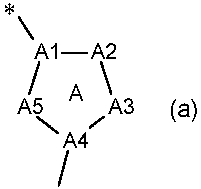 wherein
      ✔ A1, A4 each independently represents a carbon atom or a nitrogen atom,
      ✔ A2, A3, A5 each independently represents a carbon atom, an oxygen atom, a sulfur atom or a nitrogen atom,
      it being understood that A1, A2, A3, A4 and A5 cannot simultaneously represent a heteroatom,
   - or an aromatic or partially hydrogenated cyclic group of the formula (b): 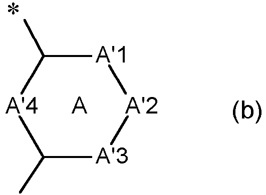 wherein A'1, A'2, A'3, A'4 each independently represents a carbon atom or a nitrogen atom,
   it being understood that * means that the bond is linked to X3,
   the aromatic or partially hydrogenated cyclic group A such defined being optionally substituted with one or more substituents, identical or different, selected from halogen atoms, alkyl group, alkoxy group, hydroxy group, oxo group, alkoxyalkyl group, alkoxyalkoxy group, polyhalogenoalkyl group, polyhalogenoalkoxy group, heterocycloalkyl group, heterocycloalkylalkyl group, (alkoxyalkyl)(alkyl)amino group, amino group, alkylamino group, dialkylamino group, cycloalkyl group, (heterocycloalkyl)(alkyl)amino group, dialkylaminoalkyl group, heterocycloalkylalkoxy group, cyano group and cyanoalkyl group,
   wherein the heterocycloalkyl and cycloalkyl group such defined can be optionally substituted by one or more substituents chosen from alkyl group, halogen atoms, polyhalogenoalkyl group, polyhalogenoalkoxy group, alkoxy group, alkoxyalkyl group, hydroxy group, cyano group and oxo group,
   their enantiomers, diastereoisomers, tautomers, racemic, hydrates, solvates, N-oxide, isotopes, deuterated derivatives and addition salts thereof with a pharmaceutically acceptable acid or base.

When describing the compounds of the invention, the terms used are to be construed in accordance with the following definitions, unless a context dictates otherwise:
The term "alkyl" by itself or as part of another substituent refers to fully saturated monovalent hydrocarbon radical, including corresponding deuterated derivatives. Alkyl groups of this invention comprise from 1 to 6 carbon atoms. Alkyl groups may be linear or branched, may include spiranic structure, and may be optionally substituted as indicated herein. Examples of alkyl groups are methyl, ethyl, n-propyl, i-propyl, butyl and its isomers (e.g. n-butyl, i-butyl and t-butyl), pentyl and its isomers, hexyl and its isomers. The term "alkanediyl" means a fully saturated divalent hydrocarbon radical having two single bonds for attachment to two other groups, and can be represented as "-(alkyl)- " group wherein alkyl is as defined above. Alkanediyl groups of this invention comprise from 1 to 6 carbon atoms, may be linear or branched, may include spiranic structure, and may be substituted as indicated herein. Non-limiting examples of alkanediyl groups includes: -CH₂-, -CH₂-CH₂-, -CD₂-, -CD₂-CD₂-, -CH(CH₃)-, -CH(CH₂-CH₃)-, -CH(i-Pr)-, -C(CH₃)(CH₃)-, -CH₂-C(CH₃)(CH₃)-, -CH₂-CH₂-C(CH₃)(CH₃)-, 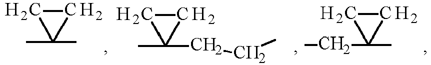 -CH₂-CH(i-Pr)-, -CH(i-Pr)-CH₂-, -CH₂-CH(i-Bu)-, -CH(i-Bu)-CH₂-, -CH(CH₃)-CH₂-, -CH₂-CH(CH₃)-, -CH₂-CH₂-CH₂-, -CD₂-CD₂-CD₂-, -CH(CH₃)-CH₂-CH₂-, -CH₂-CH₂-CH(CH₃)-, -CH₂-CH(CH₃)-CH₂-, -CH(CH₃)-CH₂-CH(CH₃)-, -CH₂-CH₂-CH(CH₂-CH₃)-, -CH(CH₂-CH₃)-CH₂-CH₂-, -CH(CH₂-CH₃)-CH₂-CH(CH₃)-, -CH(CH₃)-CH₂-CH(CH₂-CH₃)-, it being possible for those groups, when indicated, to be further substituted. For example, an alkanediyl group substituted by an alkoxy group will include, but will not be limited to, -CH(OCH₃)-, -CH(OCH₃)-CH(CH₃)-, -CH₂-CH₂-CH(OCH₃)-, -CH(OCH₃)-CH₂-CH₂-, -CH₂-CH₂-CH(CH₂-OCH₃)-, -CH(CH₂-OCH₃)-CH₂-CH₂-, -CH(O-CH₂-CH₃)-CH₂-, -CH₂-CH(O-CH₂-CH₃)-. As nonlimited other example, an alkanediyl group substituted by a cycloalkyl group will include -CH₂-CH(Cy-Pr)-, -CH(Cy-Pr)-CH₂-, wherein Cy-Pr means cyclopropyl. An alkanediyl group substituted by one or more halogen atoms includes for example, but will not be limited to -CHF-, -CHF-CH₂-, -CF₂-, -CF₂-CH₂-, -CH₂-CF₂-. An alkanediyl group substituted by a heterocycloalkyl group will include for example but will not be limited to -CH₂-CH(tetrahydropyranyl)-, -CH(tetrahydropyranyl)-CH₂-, -CH₂-CH(oxolanyl)-, -CH(oxolanyl)-CH₂-.

The term "cycloalkyl" by itself or as part of another substituent is a monovalent, saturated, or unsaturated hydrocarbon group having one or two cyclic structures. Cycloalkyl includes all saturated, partially saturated or aromatic hydrocarbon groups having one or two cyclic structures. Cycloalkyl groups comprise 3 or more carbon atoms and generally, according to this invention comprise from 3 to 10 carbon atoms.

Examples of cycloalkyl groups having one cyclic structure include but are not limited to phenyl, cyclopropyl, cyclobutyl, cyclopentyl, cyclohexyl.

When a bi-cyclic ring structure is envisaged, the two rings can be:
- fused, meaning they share a common bond; exemplary cycloalkyl bi-cyclic fused systems include but is not limited to naphthalenyl, bicyclo[1.1.0]butanyl, octahydropentalenyl, decahydronaphthalenyl, octahydro-1H-indenyl;
- linked via a bond between the two cyclic structures; exemplary cycloalkyl bi-cyclic linked systems include but is not limited to bi-phenyl, bi-cyclopropanyl, bi-cyclopentenyl, bi-cyclohexanyl, cyclopropylcyclohexanyl, cyclopropylcyclopentanyl;
- bridged meaning that the two rings share three or more atoms, separating the two bridgehead atoms by a bridge containing at least one atom; exemplary cycloalkyl bi-cyclic bridged systems include but is not limited to bicyclo[2.2.1]heptanyl, bicyclo[2.2.2]octanyl;
- or represent a spiro bi-cyclic ring system wherein the two rings are connected through a single atom; exemplary cycloalkyl spiro bi-cyclic systems include but is not limited to spiro[2.2]pentanyl, spiro[2.4]heptanyl, spiro[4.4]nonanyl, spiro[5.5]undecanyl.

The "cycloalkyl group" such defined can be optionally substituted by 1 to 3 substituents chosen from alkyl group, halogen atoms, polyhalogenoalkyl group, polyhalogenoalkoxy group, alkoxy group, alkoxyalkyl group, hydroxy group, cyano group and oxo group. When the cycloalkyl group is substituted by 2 or 3 substituents, substituents can be beared by the same atom or different atoms, provided the valency of each atom is respected.

The term "alkoxy" by itself or as part of another substituent refers to an "(alkyl)-O-" group wherein "alkyl" is as defined above. Non-limiting examples of alkoxy groups includes methoxy, ethyloxy, n-propyloxy, i-propyloxy, butyloxy (and its isomers), pentyloxy (and its isomers), hexyloxy (and its isomers).

The term "alkoxyalkyl" refers to an "(alkyl)-O-(alkyl)-" group wherein "alkyl" is as defined above. Non-limiting examples include CH₃-O-CH₂-, CH₃-O-CH₂-CH₂-.

The term "alkoxyalkoxy" refers to an "(alkyl)-O-(alkyl)-O-" group wherein "alkyl" is as defined above. Non-limiting examples include CH₃-O-CH₂-O-, CH₃-O-CH₂-CH₂-O-.

The term "alkylamino" refers to an "-NH-(alkyl)" group wherein "alkyl" is as defined above. Non-limiting examples include -NH-CH₃, -NH-CH₂-CH₃, -NH-CH(CH₃)(CH₃).

The term "dialkylamino" refers to an "-N(alkyl)(alkyl)" group wherein "alkyl" is as defined above. Non-limiting examples include -N(CH₃)₂, -N(CH₃)(CH₂-CH₃).

The term "polyhalogenoalkyl" refers to an alkyl group as defined above wherein one or more hydrogen atom, carried by the same or different carbon atoms, is replaced by one or more halogen atoms. Non-limiting examples includes fluoromethyl, difluoromethyl, trifluoromethyl, 2-chloroethyl.

The term "polyhalogenoalkoxy" refers to a "(polyhalogenoalkyl)-O-" group wherein "polyhalogenoalkyl" is as defined above. Non-limiting examples includes fluoromethoxy, difluoromethoxy, trifluoromethoxy, 2-chloroethoxy.

The term "heterocycloalkyl" means a monovalent mono- or bi-cyclic aromatic or non-aromatic carbocyclic group containing from 3 to 10 ring members and containing from 1 to 3 heteroatoms selected from oxygen atom, sulfur atom and nitrogen atom. The heterocycloalkyl group can be linked by a carbon or a nitrogen atom when possible. The heterocycloalkyl group such defined can be a monocyclic ring system or a bi-cyclic ring system. Heterocycloalkyl monocyclic ring system include but is not limited to pyridinyl, piperazinyl, piperidinyl, tetrahydropyridinyl, tetrahydropyranyl, pyrrolidinyl, dihydropyrrolyl, oxolanyl, dihydrofuranyl, morpholinyl, pyrazolyl, azetidinyl, oxetanyl. When a bi-cyclic ring system is envisaged, the two rings can be:
- fused, meaning they share a common bond; exemplary heterocycloalkyl bi-cyclic fused systems include but is not limited to indolyl, indolinyl, benzopyranyl, benzofuranyl, naphthyridinyl, quinolinyl, pyridopyrazinyl, pyridopyridazinyl, pyridopyrimidinyl, dihydroquinolinyl, tetrahydroquinolinyl, dihydrobenzofuranyl, benzopyranyl, dihydrobenzopyranyl;
- linked via a bond between the two cyclic structures; exemplary heterocycloalkyl bi-cyclic linked systems include but is not limited to phenylpyridinyl, bipyridinyl, oxetanylpyridinyl, oxetanylpiperidinyl oxetanyltetrahydropyridinyl, pyrrolidinylpiperidinyl, morpholinopiperidinyl, pyrrolidinyltetrahydropyridinyl, pyrrolidinylpyridinyl, oxetanylpiperazinyl, pyrrolidinylpiperazinyl;
- bridged meaning that the two rings share three or more atoms, separating the two bridgehead atoms by a bridge containing at least one atom; exemplary heterocycloalkyl bi-cyclic bridged systems include but is not limited to azabicyclo[2.2.1]heptanyl, oxaazabicyclo[2.2.1]heptanyl;
- or represent a spiro bi-cyclic ring system wherein the two rings are connected through a single atom; exemplary heterocycloalkyl spiro bi-cyclic systems include but is not limited to oxaspirooctane, azaspirooctane, diazaspirooctane, oxaazaspirooctane, oxaspirononane, azaspirononane, diazaspirononane, oxaazaspirononane.

The "heterocycloalkyl group" such defined can be optionally substituted by 1 to 3 substituents chosen from alkyl group, halogen atoms, polyhalogenoalkyl group, polyhalogenoalkoxy group, alkoxy group, alkoxyalkyl group, hydroxy group, cyano group and oxo group. When the heterocycloalkyl group is substituted by 2 or 3 substituents, substituents can be beared by the same atom or different atoms, provided the valency of each atom is respected.

The term "heterocycloalkylalkyl" refers to a "(heterocycloalkyl)-(alkyl)-" group wherein the heterocycloalkyl and the alkyl moieties are as defined above. Non-limiting examples include **morpholinylmethyl, pyrrolidinylmethyl, piperazinylmethyl,** piperidinylmethyl.

The term "halogen atoms" means a fluorine, chlorine, bromine or iodine atom.

Among the pharmaceutically acceptable acids there may be mentioned, without implying any limitation, hydrochloric acid, hydrobromic acid, sulphuric acid, phosphonic acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, camphoric acid etc.

Among the pharmaceutically acceptable bases there may be mentioned, without implying any limitation, sodium hydroxide, potassium hydroxide, triethylamine, tert-butylamine etc.

Specific embodiments of compounds of formula (I) of the invention are described below. Characteristics of those specific embodiments can be taken alone or combined to generate new specific embodiments.

In a specific embodiment, the invention more preferably refers to compounds of formula (I) wherein R represents a hydrogen atom.

In another embodiment, R represents advantageously a halogen atom, and most preferably a fluorine or a chlorine atom.

When R is an alkyl group, it is preferably a methyl group.

R is preferably linked to Z2 when Z2 represents a carbon atom.

In another specific preferred embodiment of the invention, Z1, Z2 and Z3 represent simultaneously a carbon atom.

In an advantageous alternative embodiment, one of Z1, Z2 and Z3 is a nitrogen atom while the two others represent a carbon atom. More particularly when one of Z1, Z2 and Z3 represents a nitrogen atom, it is preferentially Z1 or Z2.

Another specific embodiment of the invention relates to compounds of formula (I) wherein -X1- represents -O- or -NH-. More preferably, -X1- represents -O-.

In another specific embodiment of the invention, -X2- represents an alkanediyl group linear or branched having 2, 3, 4 or 5 carbon atoms, and more preferably 3, 4 or 5 carbon atoms. -X2- is preferably not substituted. When -X2- is substituted, fluor or methoxy group is preferred. Advantageously -X2- represents -(CH₂)₂-, -(CH₂)₃-, -CH(CH₃)-(CH₂)₂-, -(CH₂)₂-CH(CH₃)-, -CH₂-CH(CH₃)-CH₂-, 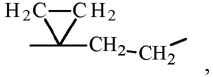 -CH₂-CHF-CH₂-, -CH₂-CF₂-CH₂-, -(CH₂)₂-CH(CH₂-CH₃)- or -CH(CH₂-CH₃)-(CH₂)₂-. Even more preferably, -X2- represents -(CH₂)₃-, -CH(CH₃)-(CH₂)₂-, -(CH₂)₂-CH(CH₃)-, -CH₂-CF₂-CH₂- or -CH₂-CHF-CH₂-.

The preferred value for Ra in compounds of formula (I) is hydrogen atom.

In another specific embodiment of the invention, -X3- represents an alkanediyl group linear or branched having 1, 2, 3, 4 or 5 carbon atoms, and more preferably 1 or 2 carbon atoms. -X3- is preferably not substituted. Advantageously -X3- represents -CH₂-, -CH(CH₃)-, -(CH₂)₂-, -(CH₂)₃-, -CH(CH₂-CH₃)-, -CH(CH₃)-CH₂-, -CH₂-CH(CH₃)-, -CH₂-CH(i-Pr)-, -CH(i-Pr)-CH₂-, -CH₂-CH(Cy-Pr)-, -CH(Cy-Pr)-CH₂-. Even more preferably, -X3- represents -(CH₂)₂-, -CH₂- or -CH(CH₃)-.

Another specific embodiment of the present invention is represented by compounds of formula (I) for which A represents a group of formula (b):

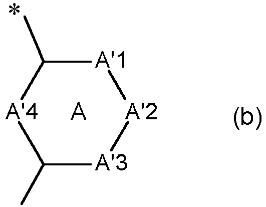

Preferred values for (A'1, A'2, A'3, A'4) are:
- four carbon atoms, or
- three carbon atoms and one nitrogen atom, more preferably the nitrogen atom being in A'4,
- or two carbon atoms and two nitrogen atoms.

A'3 is advantageously a carbon atom.

As a particular embodiment of the invention, A represents the following preferred scaffolds, being represented herein without any substitution:

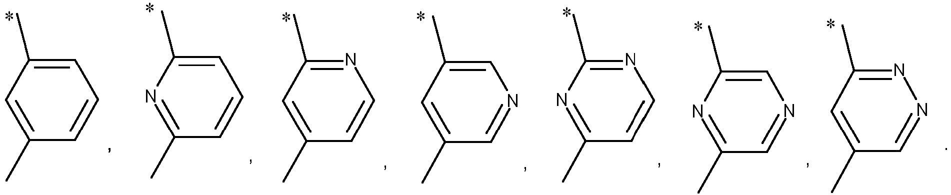

Most preferred embodiment for A of formula (b) is phenyl or pyridinyl group. An advantageous alternative for A is pyrazinyl group.

An advantageous alternative for A is represented by a group of formula (a):

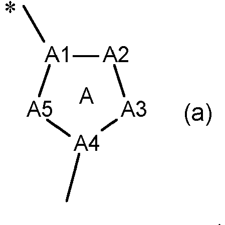

Most preferred scaffold of formula (a) contains one, two, or three heteroatoms, one of them being a nitrogen atom. Representative preferred scaffolds of formula (a) are as follows, being represented herein without any substitution:

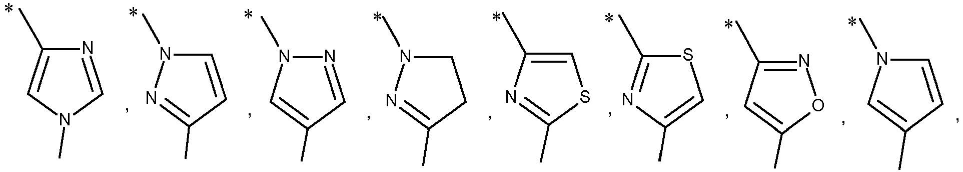

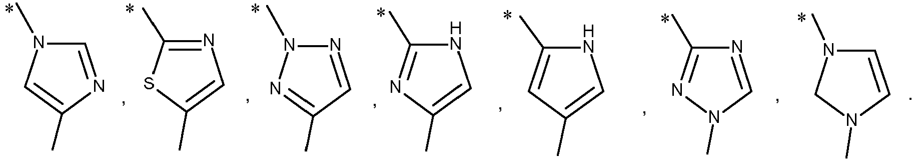

Most preferred embodiment for A of formula (a) is triazolyl or pyrazolyl group.

Preferentially the group A of the compounds of formula (I) is not substituted.

When the group A of the compounds of formula (I) is substituted, the substitution can occur on any carbon or nitrogen atom of the A scaffolds having at least one free valence. Most preferred substitutions include halogen atoms, cyano group, cyanoalkyl group, oxo group, alkoxy group, alkyl group, cycloalkyl group and heterocycloalkyl group. Particularly, preferred substitutions include fluor, bromine, or chlorine atoms, methyl, ethyl, cyclopropyl, methoxy, isopropyloxy, cyano, cyanomethyl and oxo groups.

Most preferred heterocycloalkyl group include pyrrolidinyl group, piperazinyl group, morpholinyl group, azetidinyl group, piperidinyl, tetrahydropyridinyl, tetrahydrofuranyl, dihydrofuranyl, oxetanyl, pyrazolidinyl.

Most preferred substitutions of the group A are fluorine or bromine atom, methoxy group, methyl group, ethyl group, pyrrolidinyl group unsubstituted or substituted, piperazinyl group unsubstituted or substituted.

Another specific embodiment of the invention is represented by compounds of formula (I-a): 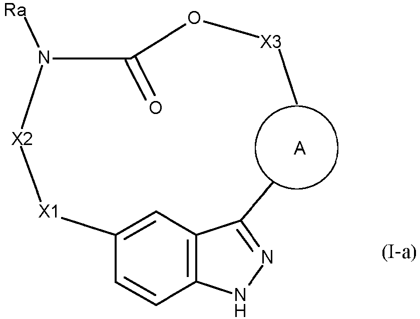 wherein X1, X2, X3, Ra and A are as defined for formula (I).

In another preferred embodiment the invention concerns compounds of formula (I-b): 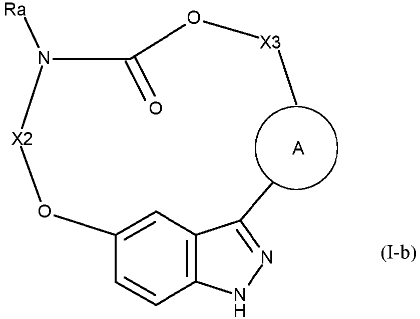 wherein X2, X3, Ra and A are as defined for formula (I). Most preferred compounds of formula (I-b) are those for which -X2- represents -(CH₂)₃-, -CH(CH₃)-(CH₂)₂-, -CH₂-CHF-CH₂-, -CH₂-CF₂-CH₂- or -(CH₂)₂-CH(CH₃)-. Another most preferred compounds of formula (I-b) are those for which -X₃- represents -CH₂- or -(CH)(CH₃)-.

Another specific embodiment of the invention concerns compounds of formula (I) for which the -X1-X2-N(Ra)-C(O)O-X3- chain represents preferentially -O-(CH₂)₃-NH-C(O)O-CH₂-, -O-CH(CH₃)-(CH₂)₂-NH-C(O)O-CH₂-, -O-CH₂-CHF-CH₂-NHC(O)O-CH₂-, -O-CH₂-CF₂-CH₂-NHC(O)O-CH₂-, -O-CH(CH₃)-(CH₂)₂-NHC(O)O-(CH₂)₂- or -O-CH(CH₃)-(CH₂)₂-NH-C(O)O-CH(CH₃)-.

Preferentially, compounds of the invention are compounds of formula (I-c) or (I-c'): 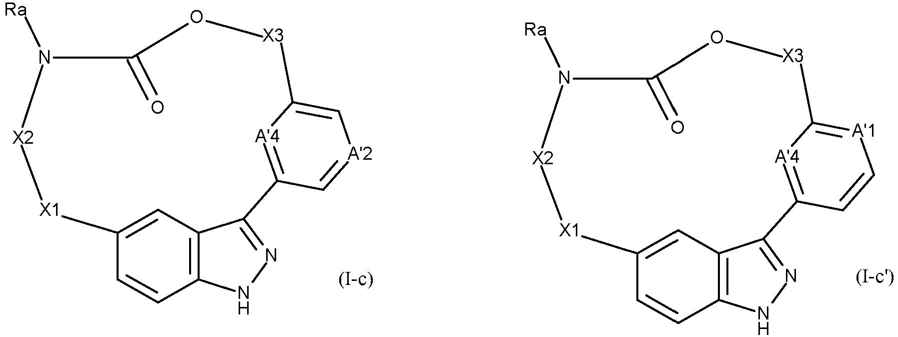 wherein X1, X2, X3, Ra, A'1, A'2 and A'4 are as defined for formula (I).

Another specific embodiment is related to compounds of formula (I-d) or (I-d'): 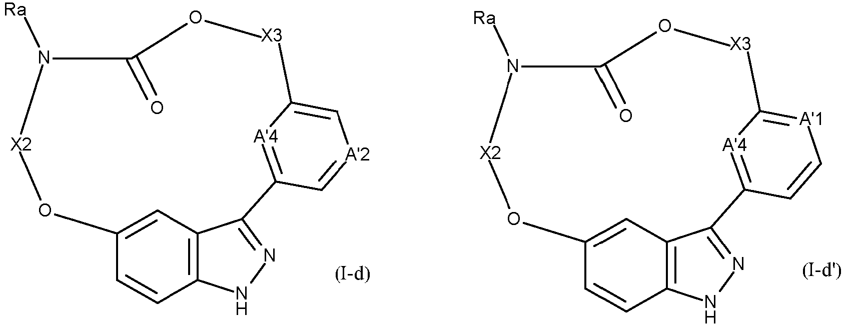 wherein X2, X3, Ra, A'1, A'2 and A'4 are as defined for formula (I). Most preferred compounds of formula (I-d) or (I-d') are those for which -X2- represents -(CH₂)₃-, -CH(CH₃)-(CH₂)₂-, -CH₂-CHF-CH₂-, -CH₂-CF₂-CH₂- or -(CH₂)₂-CH(CH₃)-. Another most preferred compounds of formula (I-d) or (I-d') are those for which -X3- represents -CH₂- or -(CH₂)₂-.

Another preferred compounds of the invention are compounds of formula (I-e): 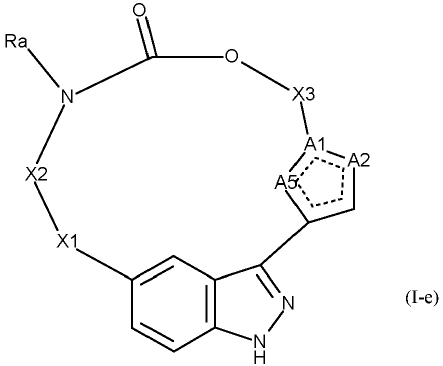 wherein X1, X2, X3, Ra, A1, A2 and A5 are as defined for formula (I).

Another preferred compounds of the invention are compounds of formula (I-f): 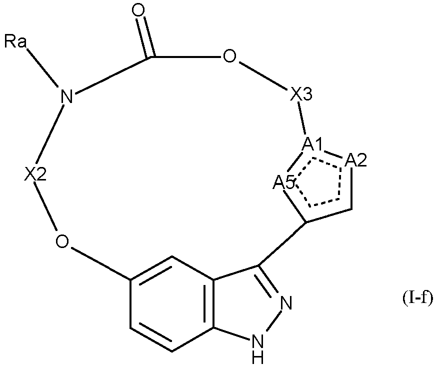 wherein X2, X3, Ra, A1, A2 and A5 are as defined for formula (I). Most preferred compounds of formula (I-f) are those for which -X2- represents -(CH₂)₃-, -CH(CH₃)-(CH₂)₂-, -CH₂-CHF-CH₂-, -CH₂-CF₂-CH₂- or -(CH₂)₂-CH(CH₃)-. Another most preferred compounds of formula (I-f) are those for which -X3- represents -CH₂- or -(CH₂)₂-.

In another specific embodiment, preferred compounds of the invention are:
- 8,14-dioxa-4,10,19,20-tetraazatetracyclo[13.5.2.1^{2,6}.0^{18,21}]tricosa-1(20),2,4,6(23),15,17, 21-heptaen-9-one;
- 10-methyl-8,14-dioxa-4,10,19,20-tetraazatetracyclo[13.5.2.1^{2,6}.0^{18,21}]tricosa-1(20),2,4, 6(23),15,17,21-heptaen-9-one;
- 4-fluoro-8,14-dioxa-10,19,20-triazatetracyclo[13.5.2.1^{2,6}.0^{18,21}]tricosa-1(20),2,4,6(23), 15,17,21-heptaen-9-one;
- 8,14-dioxa-10,19,20,23-tetraazatetracyclo[13.5.2.1^{2,6}.0^{18,21}]tricosa-1(20),2,4,6(23), 15,17,21-heptaen-9-one;
- 8,14-dioxa-10,19,20-triazatetracyclo[13.5.2.1^{2,6}.0^{18,21}]tricosa-1(20),2,4,6(23),15,17,21-heptaen-9-one;
- 10-(propan-2-yl)-8,14-dioxa-4,10,19,20-tetraazatetracyclo[13.5.2.1^{2,6}.0^{18,21}]tricosa-1(20),2,4,6(23),15,17,21-heptaen-9-one;
- 8,14-dioxa-5,10,19,20-tetraazatetracyclo[13.5.2.1^{2,6}.0^{18,21}]tricosa-1(20),2,4,6(23), 15,17,21-heptaen-9-one;
- 4-methoxy-8,14-dioxa-10,19,20-triazatetracyclo[13.5.2.1^{2,6}.0^{18,21}]tricosa-1(20),2,4, 6(23),15,17,21-heptaen-9-one;
- 4-bromo-8,14-dioxa-10,19,20-triazatetracyclo[13.5.2.1^{2,6}.0^{18,21}]tricosa-1(20),2,4,6(23), 15,17,21-heptaen-9-one;
- 5-fluoro-8,14-dioxa-10,19,20-triazatetracyclo[13.5.2.1^{2,6}.0^{18,21}]tricosa-1(20),2,4,6(23), 15,17,21-heptaen-9-one;
- 5-methyl-8,14-dioxa-10,19,20-triazatetracyclo[13.5.2.1^{2,6}.0^{18,21}]tricosa-1(20),2,4,6(23), 15,17,21-heptaen-9-one;
- 4-(pyrrolidin-1-yl)-8,14-dioxa-10,19,20-triazatetracyclo[13.5.2.1^{2,6}.0^{18,21}]tricosa-1(20), 2,4,6(23),15,17,21-heptaen-9-one;
- 4-[4-(propan-2-yl)piperazin-1-yl]-8,14-dioxa-10,19,20-triazatetracyclo[13.5.2.1^{2,6}.0^{18,21}] tricosa-1(20),2,4,6(23),15,17,21-heptaen-9-one;
- 4-{2-oxa-6-azaspiro[3.4]octan-6-yl }-8,14-dioxa-10,19,20-triazatetracyclo [13.5.2.1^{2,6}.0^{18,21}]tricosa-1(20),2,4,6(23),15,17,21-heptaen-9-one;
- 4-[4-(oxetan-3-yl)piperazin-1-yl]-8,14-dioxa-10,19,20-triazatetracyclo[13.5.2.1^{2,6}.0^{18,21}] tricosa-1(20),2,4,6(23),15,17,21-heptaen-9-one;
- 4-(morpholin-4-yl)-8,14-dioxa-10,19,20-triazatetracyclo[13.5.2.1^{2,6}.0^{18,21}]tricosa-1(20), 2,4,6(23),15,17,21-heptaen-9-one;
- 4-[(2R,6S)-2,6-dimethylmorpholin-4-yl]-8,14-dioxa-10,19,20-triazatetracyclo [13.5.2.1^{2,6}.0^{18,21}]tricosa-1(20),2,4,6(23),15,17,21-heptaen-9-one;
- 4-methyl-8,14-dioxa-10,19,20-triazatetracyclo[13.5.2.1^{2,6}.0^{18,21}]tricosa-1(20),2,4,6(23), 15,17,21-heptaen-9-one;
- 5-methoxy-8,14-dioxa-10,19,20-triazatetracyclo[13.5.2.1^{2,6}.0^{18,21}]tricosa-1(20),2,4, 6(23),15, 17,21-heptaen-9-one;
- 4-(4,4-difluoropiperidin-1-yl)-8,14-dioxa-10,19,20-triazatetracyclo[13.5.2.1^{2,6}.0^{18,21}] tricosa-1(20),2,4,6(23),15,17,21-heptaen-9-one;
- 4-(3,3-difluoropyrrolidin-1-yl)-8,14-dioxa-10,19,20-triazatetracyclo[13.5.2.1^{2,6}.0^{18,21}] tricosa-1(20),2,4,6(23),15,17,21-heptaen-9-one;
- 7-methyl-8,14-dioxa-10,19,20-triazatetracyclo[13.5.2.1^{2,6}.0^{18,21}]tricosa-1(20),2,4,6(23), 15,17,21-heptaen-9-one;
- 4-[4-(2-methoxyethyl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 9,14-dioxa-11,19,20-triazatetracyclo[13.5.2.1^{2,6}.0^{18,21}]tricosa-1(20),2,4,6(23),15,17,21-heptaen-10-one;
- 4-[(3R)-3-hydroxypyrrolidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-[(2-methoxyethyl)(methyl)amino]-8,14-dioxa-10,19,20-triazatetracyclo [13.5.2.1^{2,6}.0^{18,21}]tricosa-1(20),2,4,6(23),15,17,21-heptaen-9-one;
- 4-chloro-8,14-dioxa-10,19,20-triazatetracyclo[13.5.2.1^{2,6}.0^{18,21}]tricosa-1(20),2,4,6(23), 15,17,21-heptaen-9-one;
- 4-fluoro-5-methyl-8,14-dioxa-10,19,20-triazatetracyclo[13.5.2.1^{2,6}.0^{18,21}]tricosa-1(20),2, 4,6(23),15,17,21-heptaen-9-one;
- 4,5-difluoro-8,14-dioxa-10,19,20-triazatetracyclo[13.5.2.1^{2,6}.0^{18,21}]tricosa-1(20),2,4, 6(23),15,17,21-heptaen-9-one;
- 5-bromo-8,14-dioxa-10,19,20-triazatetracyclo[13.5.2.1^{2,6}.0^{18,21}]tricosa-1(20),2,4,6(23), 15,17,21-heptaen-9-one;
- 4-(4-methylpiperazin-1-yl)-8,14-dioxa-10,19,20-triazatetracyclo[13.5.2.1^{2,6}.0^{18,21}] tricosa-1(20),2,4,6(23),15,17,21-heptaen-9-one;
- 4-(3-methoxyazetidin-1-yl)-8,14-dioxa-10,19,20-triazatetracyclo[13.5.2.1^{2,6}.0^{18,21}] tricosa-1(20),2, 4,6(23),15,17,21-heptaen-9-one;
- 1-{9-oxo-8,14-dioxa-10,19,20-triazatetracyclo[13.5.2.1^{2,6}.0^{18,21}]tricosa-1(20),2,4,6(23), 15,17,21-heptaen-4-yl}piperidine-4-carbonitrile;
- 4-[4-(pyrrolidin-1-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(azetidin-1-yl)-8,14-dioxa-10,19,20-triazatetracyclo[13.5.2.1^{2,6}.0^{18,21}]tricosa-1(20),2, 4,6(23),15,17,21-heptaen-9-one;
- 4-(piperidin-1-yl)-8,14-dioxa-10,19,20-triazatetracyclo[13.5.2.1^{2,6}.0^{18,21}]tricosa-1(20),2, 4,6(23),15,17,21-heptaen-9-one;
- 4-(2,5-dihydrofuran-3-yl)-8,14-dioxa-10,19,20-triazatetracyclo[13.5.2. 1^{2,6}.0^{18,21}]tricosa-1(20),2,4,6(23),15,17,21-heptaen-9-one;
- 4-[4-(morpholin-4-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(1-methyl-1,2,3,6-tetrahydropyridin-4-yl)-8,14-dioxa-10,19,20-triazatetracyclo [13.5.2.1^{2,6}.0^{18,21}]tricosa-1(20),2,4,6(23),15,17,21-heptaen-9-one;
- 4-[(2S,5S)-2,5-dimethylmorpholin-4-yl]-8,14-dioxa-10,19,20-triazatetracyclo [13.5.2.1^{2,6}.0^{18,21}]tricosa-1(20),2,4,6(23),15,17,21-heptaen-9-one;
- 4-[(morpholin-4-yl)methyl]-8,14-dioxa-10,19,20-triazatetracyclo[13.5.2. 1^{2,6}.0^{18,21}]tricosa-1(20),2,4,6(23),15,17,21-heptaen-9-one;
- 4-[(pyrrolidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one;
- 4-[(pyrrolidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one;
- 4-[(4-methylpiperazin-1-yl)methyl]-8,14-dioxa-10,19,20-triazatetracyclo [13.5.2.1^{2,6}.0^{18,21}]tricosa-1(20),2,4,6(23),15,17,21-heptaen-9-one;
- 5-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one;
- 4-[4-(2-methoxyethyl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(diethylamino)-8,14-dioxa-10,19,20-triazatetracyclo[13.5.2.1^{2,6}.0^{18,21}]tricosa-1(20),2, 4,6(23),15,17,21-heptaen-9-one;
- 4-[(pyrrolidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one;
- 4-[(piperidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one;
- 4-[(4-methylpiperazin-1-yl)methyl]-8,14-dioxa-10,19,20-triazatetracyclo [13.5.2.1^{2,6}.0^{18,21}]tricosa-1(20),2,4,6(23),15,17,21-heptaen-9-one;
- 5-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one;
- 4-[4-(2-methoxyethyl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(diethylamino)-8,14-dioxa-10,19,20-triazatetracyclo[13.5.2.1^{2,6}.0^{18,21}]tricosa-1(20),2, 4,6(23),15,17,21-heptaen-9-one;
- 4-cyclopropyl-8,14-dioxa-10,19,20-triazatetracyclo[13.5.2. 1^{2,6}.0^{18,21}]tricosa-1(20),2, 4,6(23),15,17,21-heptaen-9-one;
- 5-(4-methylpiperazin-1-yl)-8,14-dioxa-10,19,20-triazatetracyclo[13.5.2.1^{2,6}.0^{18,21}]tricosa-1(20),2,4,6(23),15,17,21-heptaen-9-one;
- 13-methyl-8,14-dioxa-10,19,20-triazatetracyclo[13.5.2.1^{2,6}.0^{18,21}]tricosa-1(20),2,4,6(23), 15,17,21-heptaen-9-one;
- 8,14-dioxa-4,5,10,19,20-pentaazatetracyclo[13.5.2.1^{2,5}.0^{18,21}]tricosa-1(20),2(23),3, 15(22),16,18(21)-hexaen-9-one;
- 4-[methyl(oxetan-3-yl)amino]-8,14-dioxa-10,19,20-triazatetracyclo[13.5.2.1^{2,6}.0^{18,21}] tricosa-1(20),2,4,6(23),15,17,21-heptaen-9-one;
- 4-[(dimethylamino)methyl]-8,14-dioxa-10,19,20-triazatetracyclo[13.5.2.1^{2,6}.0^{18,21}]tricosa-1(20),2,4,6(23),15,17,21-heptaen-9-one;
- 4,10-dimethyl-8,14-dioxa-10,19,20-triazatetracyclo[13.5.2.1^{2,6}.0^{18,21}]tricosa-1(20),2,4, 6(23),15,17,21-heptaen-9-one;
- 4-(propan-2-yloxy)-8,14-dioxa-10,19,20-triazatetracyclo[13.5.2.1^{2,6}.0^{18,21}]tricosa-1(20),2,4,6(23),15,17,21-heptaen-9-one;
- 4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one;
- 4-[1-(oxetan-3-yl)-1,2,3,6-tetrahydropyridin-4-yl]-8,14-dioxa-10,19,20-triazatetracyclo [13.5.2.1^{2,6}.0^{18,21}]tricosa-1(20),2,4,6(23),15,17,21-heptaen-9-one;
- 4-(1-methyl-1H-pyrazol-3-yl)-8,14-dioxa-10,19,20-triazatetracyclo[13.5.2.1^{2,6}.0^{18,21}] tricosa-1(20),2(23),3,5,15(22),16,18(21)-heptaen-9-one;
- (7S)-7-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-[2-(morpholin-4-yl)ethoxy]-8,14-dioxa-10,19,20-triazatetracyclo[13.5.2.1^{2,6}.0^{18,21}] tricosa-1(20),2(23),3,5,15(22),16,18(21)-heptaen-9-one;
- 4-(2-methoxyethyl)-8,14-dioxa-10,19,20-triazatetracyclo[13.5.2.1^{2,6}.0^{18,21}]tricosa-1(20), 2(23), 3,5,15(22),16,18(21)-heptaen-9-one;
- (7R)-7-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 5-cyclopropyl-8,14-dioxa-10,19,20-triazatetracyclo[13.5.2.1^{2,6}.0^{18,21}]tricosa-1(20),2,4,6(23),15,17,21-heptaen-9-one;
- 4-(2-methoxyethoxy)-8,14-dioxa-10,19,20-triazatetracyclo[13.5.2.1^{2,6}.0^{18,21}]tricosa-1(20),2,4,6(23),15,17,21-heptaen-9-one;
- 4-fluoro-13-methyl-8,14-dioxa-10,19,20-triazatetracyclo[13.5.2.1^{2,6}.0^{18,21}]tricosa-1(20), 2,4,6(23),15,17,21- heptaen-9-one;
- 11-methyl-8,14-dioxa-10,19,20-triazatetracyclo[13.5.2.1^{2,6}.0^{18,21}]tricosa-1(20),2,4,6(23), 15,17,21-heptaen-9-one;
- 4-(3-oxomorpholin-4-yl)-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-(2-oxopyrrolidin-1-yl)-8,14-dioxa-10,19,20-triazatetracyclo[13.5.2.1^{2,6}.0^{18,21}]tricosa-1(20),2(23), 3,5,15(22),16,18(21)-heptaen-9-one;
- 5-(2-oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one;
- 4-(2-methylpyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one;
- 2-{9-oxo-8,14-dioxa-10,19,20-triazatetracyclo[13.5.2.1^{2,6}.0^{18,21}]tricosa-1(20),2(23), 3,5,15(22),16,18(21)-heptaen-4-yl}acetonitrile;
- (11R)-11-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- (11S)-11-methyl-8,14-dioxa-10,19,20-triazatetracyclo[13.5.2. 1^{2,6}.0^{18,21}]tricosa-1(20), 2(23),3,5,15(22),16,18(21)-heptaen-9-one;
- 4-ethynyl-8,14-dioxa-10,19,20-triazatetracyclo[13.5.2.1^{2,6}.0^{18,21}]tricosa-1(20),2(23),3,5, 15(22),16,18(21)-heptaen-9-one;
- 4-(piperazin-1-yl)-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-(1,2,3,6-tetrahydropyridin-4-yl)-8,14-dioxa-10,19,20-triazatetracyclo [13.5.2.1^{2,6}.0^{18,21}]tricosa-1(20),2(23),3,5,15(22),16,18(21)-heptaen-9-one;
- 11-(methoxymethyl)-8,14-dioxa-10,19,20-triazatetracyclo[13.5.2.1^{2,6}.0^{18,21}]tricosa-1(20),2(23),3,5,15(22),16,18(21)-heptaen-9-one;
- 8,14-dioxa-5,10,19,20,23-pentaazatetracyclo[13.5.2.1^{2,5}.0^{18,21}]tricosa-1(20),2(23),3, 15(22),16,18(21)-hexaen-9-one;
- 11-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 12-methyl-8,14-dioxa-10,19,20-triazatetracyclo[13.5.2.1^{2,6}.0^{18,21}]tricosa-1(20),2(23),3,5, 15(22),16,18(21)-heptaen-9-one;
- 11-ethyl-8,14-dioxa-10,19,20-triazatetracyclo[13.5.2.1^{2,6}.0^{18,21}]tricosa-1(20),2(23),3,5, 15(22),16,18(21)-heptaen-9-one;
- 4-fluoro-5,7-dimethyl-8,14-dioxa-10,19,20-triazatetracyclo[13.5.2.1^{2,6}.0^{18,21}]tricosa-1(20),2(23), 3,5,15(22),16,18(21)-heptaen-9-one;
- 4-fluoro-5-methoxy-7-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- 5-fluoro-4,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-one;
- 8,14-dioxa-10,19,20-triazapentacyclo[13.5.2.1^{2,6}.1^{7,10}.0^{18,21}]tetracosa-1(20),2(24),3,5, 15(22),16,18(21)-heptaen-9-one;
- 13-methyl-8,14-dioxa-10,19,20,23-tetraazatetracyclo[13.5.2.1^{2,6}.0^{18,21}]tricosa-1(20), 2(23),3,5,15(22),16,18(21)-heptaen-9-one;
- 12-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 7-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 5-fluoro-4-methoxy-7-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- 5-fluoro-4,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-one;
- 8,14-dioxa-10,19,20-triazapentacyclo[13.5.2.1^{2,6}.1^{7,10}.0^{18,21}]tetracosa-1(20),2(24),3,5, 15(22),16,18(21)-heptaen-9-one;
- 13-methyl-8,14-dioxa-10,19,20,23-tetraazatetracyclo[13.5.2.1^{2,6}.0^{18,21}]tricosa-1(20), 2(23),3,5,15(22),16,18(21)-heptaen-9-one;
- 12-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 7-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 5-fluoro-4-methoxy-7-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- (7R,13R)-7,13-dimethyl-8,14-dioxa-10,19,20-triazatetracyclo[13.5.2.1^{2,6}.0^{18,21}]tricosa-1(20),2(23),3,5,15(22),16,18(21)-heptaen-9-one;
- (13R)-13-methyl-8,14-dioxa-4,5,10,19,20-pentaazatetracyclo[13.5.2.1^{2,5}.0^{18,21}]tricosa-1(20),2(23),3,15,17,21-hexaen-9-one;
- 8,15-dioxa-4,10,20,21-tetraazapentacyclo[14.5.2.1^{2,6}.1^{10,13}.0^{19,22}]pentacosa-1(21),2(25), 3,5,16(23),17,19(22)-heptaen-9-one;
- 8,14-dioxa-5,10,19,20-tetraazatetracyclo[13.5.2.1^{2,5}.0^{18,21}]tricosa-1(20),2(23),3,15(22), 16,18(21)-hexaen-9-one;
- (13S)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one;
- (13R)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one;
- (13R)-13-methyl-8,14-dioxa-4,10,19,20-tetraazatetracyclo[13.5.2.1^{2,6}.0^{18,21}]tricosa-1(20),2(23), 3,5,15(22),16,18(21)-heptaen-9-one;
- 6-cyclopropyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 7-ethyl-8,14-dioxa-10,19,20-triazatetracyclo[13.5.2.1^{2,6}.0^{18,21}]tricosa-1(20),2,4,6(23), 15,17,21-heptaen-9-one;
- (13R)-13-methyl-8,14-dioxa-5,10,19,20,23-pentaazatetracyclo[13.5.2.1^{2,6}.0^{18,21}]tricosa-1(20),2,4,6(23), 15,17,21-heptaen-9-one;
- 6-(propan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one;
- (13R)-7,13-dimethyl-8,14-dioxa-4,5,10,19,20-pentaazatetracyclo[13.5.2.1^{2,5}.0^{18,21}]tricosa-1(20),2(23),3,15(22),16,18(21)-hexaen-9-one;
- (13R)-13-methyl-8,14-dioxa-10,19,20,23-tetraazatetracyclo[13.5.2.1^{2,6}.0^{18,21}]tricosa-1(20),2,4,6(23),15,17,21-heptaen-9-one;
- (7R)-7-ethyl-8,14-dioxa-10,19,20-triazatetracyclo[13.5.2.1^{2,6}.0^{18,21}]tricosa-1(20),2,4, 6(23),15, 17,21-heptaen-9-one;
- (7S)-7-ethyl-8,14-dioxa-10,19,20-triazatetracyclo[13.5.2.1^{2,6}.0^{18,21}]tricosa-1(20),2,4, 6(23),15,17,21-heptaen-9-one;
- (13R)-13-methyl-8,14-dioxa-5,10,19,20-tetraazatetracyclo[13.5.2.1^{2,6}.0^{18,21}]tricosa-1(20),2,4,6(23),15,17,21-heptaen-9-one;
- 6-(oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-one;
- 4-ethyl-8,14-dioxa-5,10,19,20,23-pentaazatetracyclo[13.5.2.1^{2,5}.0^{18,21}]tricosa-1(20), 2(23),15,17,21-pentaen-9-one;
- (13R)-23-fluoro-13-methyl-8,14-dioxa-10,19,20-triazatetracyclo[13.5.2.1^{2,6}.0^{18,21}]tricosa-1(20),2,4,6(23),15,17,21-heptaen-9-one;
- 9,14-dioxa-4,5,11,19,20-pentaazatetracyclo[13.5.2.1^{2,5}.0^{18,21}]tricosa-1(20),2(23), 3,15,17,21-hexaen-10-one;
- 4-ethyl-8,14-dioxa-5,10,19,20,23-pentaazatetracyclo[13.5.2.1^{2,5}.0^{18,21}]tricosa-1(20), 2(23),3,15,17,21-hexaen-9-one;
- 3,9,15-trioxa-4,11,20,21-tetraazatetracyclo[14.5.2.1^{2,5}.0^{19,22}]tetracosa-1(21),2(24), 4,16,18,22-hexaen-10-one;
- (13R)-16-fluoro-13-methyl-8,14-dioxa-4,10,19,20-tetraazatetracyclo[13.5.2.1^{2,6}.0^{18,21}] tricosa-1(20),2,4,6(23),15,17,21-heptaen-9-one;
- (13R)-4-chloro-13-methyl-8,14-dioxa-10,19,20,23-tetraazatetracyclo[13.5.2.1^{2,6}.0^{18,21}] tricosa-1(20),2(23),3,5,15(22),16,18(21)-heptaen-9-one;
- 8,14-dioxa-2,4,10,19,20-pentaazatetracyclo[13.5.2.1^{2,5}.0^{18,21}]tricosa-1(20),3,5(23), 15(22),16,18(21)-hexaen-9-one;
- (13R)-4-methoxy-13-methyl-8,14-dioxa-10,19,20,23-tetraazatetracyclo [13.5.2.1^{2,6}.0^{18,21}]tricosa-1(20),2,4,6(23),15,17,21-heptaen-9-one;
- (13R)-13-methyl-9-oxo-8,14-dioxa-10,19,20-triazatetracyclo[13.5.2.1^{2,6}.0^{18,21}]tricosa-1(20),2,4,6(23),15,17,21-heptaene-5-carbonitrile;
- (13R)-13-methyl-4-(pyrrolidin-1-yl)-8,14-dioxa-5,10,19,20,23-pentaazatetracyclo [13.5.2.1^{2,6}.0^{18,21}]tricosa-1(20),2,4,6(23),15,17,21-heptaen-9-one;
- (7S,13R)-7,13-dimethyl-8,14-dioxa-5,10,19,20,23-pentaazatetracyclo[13.5.2.1^{2,6}.0^{18,21}] tricosa-1(20),2,4,6(23),15,17,21-heptaen-9-one;
- (7R,13R)-7,13-dimethyl-8,14-dioxa-5,10,19,20,23-pentaazatetracyclo[13.5.2.1^{2,6}.0^{18,21}] tricosa-1(20),2,4,6(23),15,17,21-heptaen-9-one;
- (13R)-16-fluoro-13-methyl-8,14-dioxa-10,19,20-triazatetracyclo[13.5.2.1^{2,6}.0^{18,21}]tricosa-1(20),2,4,6(23),15,17,21-heptaen-9-one;
- (13R)-13-methyl-8,14-dioxa-4,10,19,20,23-pentaazatetracyclo[13.5.2.1^{2,6}.0^{18,21}]tricosa-1(20),2,4,6(23),15,17,21-heptaen-9-one;
- 8,14-dioxa-4-thia-10,19,20,23-tetraazatetracyclo[13.5.2.1^{2,5}.0^{18,21}]tricosa-1(20),2,5(23), 15,17,21-hexaen-9-one;
- 8,14-dioxa-3-thia-10,19,20,23-tetraazatetracyclo[13.5.2.1^{2,5}.0^{18,21}]tricosa-1(20),2(23), 4,15,17,21-hexaen-9-one;
- (7R,13R)-7,13-dimethyl-8,14-dioxa-10,19,20,23-tetraazatetracyclo[13.5.2.1^{2,6}.0^{18,21}] tricosa-1(20),2,4,6(23),15,17,21-heptaen-9-one;
- (13R)-4-[(3R)-3-methoxypyrrolidin-1-yl]-13-methyl-8,14-dioxa-5,10,19,20,23-pentaazatetracyclo[13.5.2.1^{2,6}.0^{18,21}]tricosa-1(20),2,4,6(23),15,17,21-heptaen-9-one;
- (13R)-16-chloro-13-methyl-8,14-dioxa-10,19,20-triazatetracyclo[13.5.2.1^{2,6}.0^{18,21}]tricosa-1(20),2, 4,6(23),15,17,21-heptaen-9-one;
- (13R)-13,16-dimethyl-8,14-dioxa-10,19,20-triazatetracyclo[13.5.2.1^{2,6}.0^{18,21}]tricosa-1(20),2,4, 6(23),15,17,21-heptaen-9-one;
- (13R)-13-methyl-8,14-dioxa-3,10,19,20,23-pentaazatetracyclo[13.5.2.1^{2,6}.0^{18,21}]tricosa-1(20),2,4, 6(23),15,17,21-heptaen-9-one;
- 8-oxa-10,14,19,20-tetraazatetracyclo[13.5.2.1^{2,6}.0^{18,21}]tricosa-1(20),2(23),3,5,15(22), 16,18(21)-heptaen-9-one hydrochloride;
- 8-oxa-10,19,20-triazatetracyclo[13.5.2.1^{2,6}.0^{18,21}]tricosa-1(20),2(23),3,5,15(22),16,18(21)-heptaen-9-one;
- (13R)-5-methoxy-13-methyl-8,14-dioxa-4,10,19,20-tetraazatetracyclo[13.5.2.1^{2,6}.0^{18,21}] tricosa-1(20),2,4,6(23),15,17,21-heptaen-9-one;
- (13R)-13-methyl-8,14-dioxa-4,10,19,20-tetraazatetracyclo[13.5.2.1^{2,6}.0^{18,21}]tricosa-1(20),2, 6(23),15,17,21-hexaene-5,9-dione;
- 4-methyl-8,14-dioxa-3,4,10,19,20-pentaazatetracyclo[13.5.2.1^{2,5}.0^{18,21}]tricosa-1(20),2, 5(23),15(22),16,18(21)-hexaen-9-one;
- (13R)-16-fluoro-13-methyl-8,14-dioxa-10,19,20,23-tetraazatetracyclo[13.5.2.1^{2,6}.0^{18,21}] tricosa-1(20),2,4,6(23),15,17,21-heptaen-9-one;
- 7,13-dioxa-4-thia-9,18,19,22-tetraazatetracyclo[12.5.2.1^{2,5}.0^{17,20}]docosa-1(19),2,5(22), 14(21),15,17(20)-hexaen-8-one;
- (13R)-4,13-dimethyl-8,14-dioxa-5,10,19,20,23-pentaazatetracyclo[13.5.2.1^{2,6}.0^{18,21}]tricosa-1(20),2(23),3,5,15(22),16,18(21)-heptaen-9-one;
- 8,14-dioxa-23-thia-4,10,19,20-tetraazatetracyclo[13.5.2.1^{2,5}.0^{18,21}]tricosa-1(20),2,4,15(22), 16,18(21)-hexaen-9-one;
- (7S,13R)-7,13-dimethyl-8,14-dioxa-10,19,20,23-tetraazatetracyclo[13.5.2.1^{2,6}.0^{18,21}] tricosa-1(20),2(23),3,5,15(22),16,18(21)-heptaen-9-one;
- (13R)-13-methyl-9-oxo-8,14-dioxa-5,10,19,20-tetraazatetracyclo[13.5.2.1^{2,5}.0^{18,21}]tricosa-1(20), 2(23),3,15(22),16,18(21)-hexaene-4-carbonitrile;
- 12,12-difluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- (13R)-17-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one;
- (7S,13R)-7,13-dimethyl-8,14-dioxa-4,10,19,20,23-pentaazatetracyclo[13.5.2.1^{2,6}.0^{18,21}] tricosa-1(20),2(23),3,5,15(22),16,18(21)-heptaen-9-one;
- (7R,13R)-7,13-dimethyl-8,14-dioxa-4,10,19,20,23-pentaazatetracyclo[13.5.2.1^{2,6}.0^{18,21}] tricosa-1(20),2(23),3,5,15(22),16,18(21)-heptaen-9-one;
- (13S)-13-methyl-8,14-dioxa-4,10,19,20,23-pentaazatetracyclo[13.5.2.1^{2,6}.0^{18,21}]tricosa-1(20),2(23),3,5,15(22),16,18(21)-heptaen-9-one;
- (13R)-13-methyl-8,14-dioxa-10,19,20,22-tetraazatetracyclo[13.5.2.1^{2,6}.0^{18,21}]tricosa-1(20), 2(23),3,5,15,17,21-heptaen-9-one;
- (12R)-4,12-dimethyl-7,13-dioxa-4,9,18,19,22-pentaazatetracyclo[12.5.2.1^{2,5}.0^{17,20}]docosa-1(19),2,5(22),14(21),15,17(20)-hexaen-8-one;
- (13R)-13-methyl-8,14-dioxa-4,5,10,19,20,23-hexaazatetracyclo[13.5.2.1^{2,5}.0^{18,21}]tricosa-1(20),2(23),3,15,17,21-hexaen-9-one;
- (13R)-13-methyl-8,14-dioxa-23-thia-4,10,19,20-tetraazatetracyclo[13.5.2.1^{2,5}.0^{18,21}]tricosa-1(20),2,4,15,17,21-hexaen-9-one;
- (13R)-4,13-dimethyl-8,14-dioxa-4,10,19,20,23-pentaazatetracyclo[13.5.2.1^{2,5}.0¹⁸,²¹]tricosa-1(20),2,5(23),15(22),16,18(21)-hexaen-9-one;
- (13R)-13-methyl-8,14-dioxa-10,16,19,20-tetraazatetracyclo[13.5.2.1^{2,6}.0^{18,21}]tricosa-1(20), 2(23),3,5,15(22),16,18(21)-heptaen-9-one;
- 14-methyl-8-oxa-10,14,19,20-tetraazatetracyclo[13.5.2.1^{2,6}.0^{18,21}]tricosa-1(20),2(23),3,5, 15,17,21-heptaen-9-one;
- (13R)-13-methyl-8,14-dioxa-4,10,19,20,22-pentaazatetracyclo[13.5.2.1^{2,6}.0^{18,21}]tricosa-1(20),2(23),3,5,15,17,21-heptaen-9-one;
- (13R)-13-methyl-8,14-dioxa-10,17,19,20-tetraazatetracyclo[13.5.2.1^{2,6}.0^{18,21}]tricosa-1(20), 2(23),3,5,15(22),16,18(21)-heptaen-9-one;
- 8,14-dioxa-4,5,10,19,20,23-hexaazatetracyclo[13.5.2.1^{2,5}.0^{18,21}]tricosa-1(20),2(23),3, 15(22),16,18(21)-hexaen-9-one;
- 12,12-difluoro-8,14-dioxa-4,5,10,19,20,23-hexaazatetracyclo[13.5.2.1^{2,5}.0^{18,21}]tricosa-1(20),2(23),3,15(22),16,18(21)-hexaen-9-one);
- (12R)-12-fluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- (12S)-12-fluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- 12,12-difluoro-8,14-dioxa-4,10,19,20,23-pentaazatetracyclo[13.5.2.1^{2,6}.0^{18,21}]tricosa-1(20),2(23),3,5,15(22),16,18(21)-heptaen-9-one;
- (12S)-12-fluoro-8,14-dioxa-4,10,19,20,23-pentaazatetracyclo[13.5.2.1^{2,6}.0^{18,21}]tricosa-1(20),2(23),3,5,15,17,21-heptaen-9-one;
- (12R)-12-fluoro-8,14-dioxa-4,10,19,20,23-pentaazatetracyclo[13.5.2.1^{2,6}.0^{18,21}]tricosa-1(20),2(23),3,5,15,17,21-heptaen-9-one;
- (12S)-12-fluoro-8,14-dioxa-4,5,10,19,20,23-hexaazatetracyclo[13.5.2.1^{2,5}.0^{18,21}]tricosa-1(20),2(23),3,15,17,21-hexaen-9-one;
- (12R)-12-fluoro-8,14-dioxa-4,5,10,19,20,23-hexaazatetracyclo[13.5.2.1^{2,5}.0^{18,21}]tricosa-1(20),2(23),3,15,17,21-hexaen-9-one;
- 8',14'-dioxa-10',19',20'-triazaspiro[cyclopropane-1,13'-tetracyclo[13.5.2.1^{2,6}.0^{18,21}] tricosane]-1'(20'),2'(23'),3',5',15'(22'),16',18'(21')-heptaen-9'-one.

The invention relates also to a global process for the preparation of compounds of formula (I), which process is characterized that there is used as starting material the compound of formula (I-1): 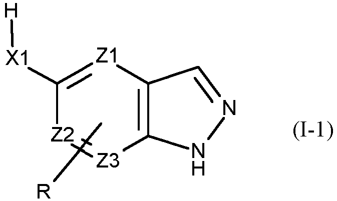 wherein R, X1, Z1, Z2 and Z3 are as defined for formula (I),
on which is condensed first a compound PG1-LG1, then a compound PG2-LG2, or first a compound PG2-LG2 then a compound PG1-LG1 wherein PG1 is a protecting group or, when -X1- is a bond PG1 represents a halogen, and PG2 is a protecting group and LG1 and LG2 are leaving groups, to yield the compound of formula (I-2): 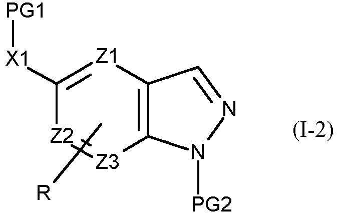 wherein R, X1, Z1, Z2, Z3, PG1 and PG2 are as defined hereinbefore, compound of formula (I-2) on which:
- is condensed a leaving group LG3 to yield the compound of formula (I-3): 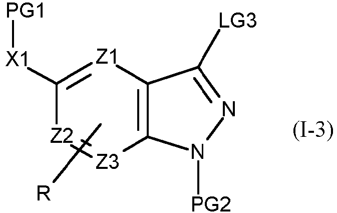 wherein R, X1, Z1, Z2, Z3, PG1, PG2 and LG3 are as defined hereinbefore, compound of formula (I-3):
   - on which is condensed, after deprotection of X1, a compound LG4-X2-NPG3 wherein LG4 is a leaving group, PG3 is a protecting group and X2 is as defined for formula (I) to yield the compound of formula (I-4): 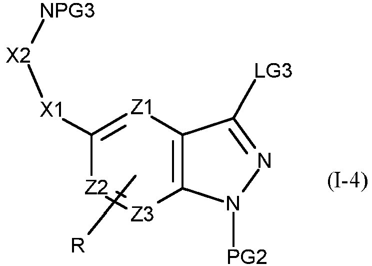 wherein R, X1, X2, Z1, Z2, Z3, PG2, PG3 and LG3 are as defined hereinbefore, compound of formula (I-4) on which is condensed a compound of formula (I-5): 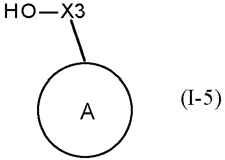 wherein A and X3 are as defined in formula (I), or an organometallic derivative of compound of formula (I-5) such as a boronate, to yield the compound of formula (I-6): 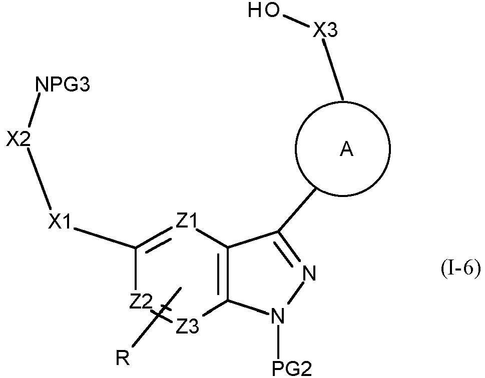 wherein R, X1, X2, X3, A, Z1, Z2, Z3, PG2 and PG3 are as defined hereinbefore,
      compound of formula (I-6) which is subjected to a deprotection of -X2-NPG3, then to a cyclization to give the compound of formula (I-7): 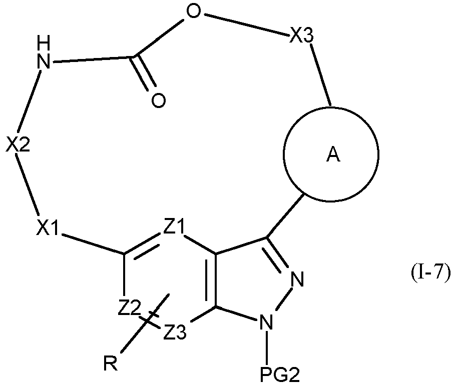 wherein R, X1, X2, X3, A, Z1, Z2, Z3 and PG2 are as defined hereinbefore, compound of formula (I-7) which is optionally alkylated on the carbamate function, and/or optionally substituted on the A ring, then submitted to the deprotection of -N(PG2)- to give the compound of formula (I),
   - or compound of formula (I-3) on which is condensed a compound of formula (I-8): 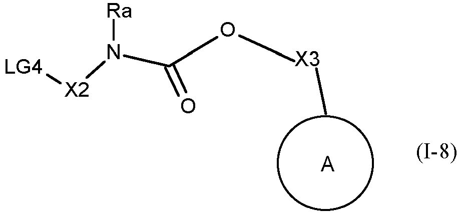 wherein Ra, X2, X3, and A are as defined hereinbefore and LG4 is a leaving group, or an organometallic derivative of compound of formula (I-8) such as a boronate, to yield the compound of formula (I-9): 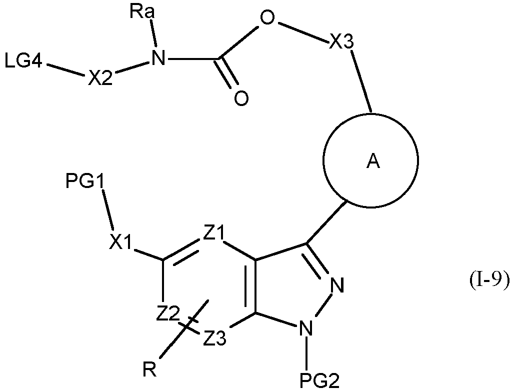 wherein R, Ra, X1, X2, X3, A, Z1, Z2, Z3, PG1, PG2 and LG4 are as defined hereinbefore, compound of formula (I-9) which is subjected, after deprotection of X1, to a cyclization to yield the compound of formula (I-7) as defined above, which, after deprotection of -N(PG2)-, and/or optional substitution on the A ring, gives the compound of formula (I),
   - or compound of formula (I-3) on which is condensed, after deprotection of X1, a compound LG5-X2-NRaCOOBn wherein X2 and Ra are as defined in formula (I) and LG5 is a leaving group, to yield the compound of formula (I-10): 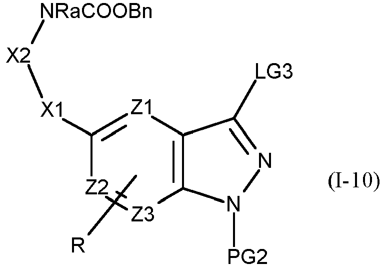 wherein R, Ra, X1, X2, Z1, Z2, Z3, PG2 and LG3 are as defined hereinbefore, compound of formula (I-10) on which is condensed a compound of formula (I-5): 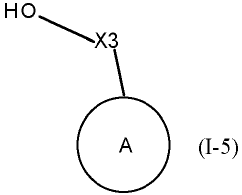 wherein X3 and A are as defined hereinbefore, or an organometallic derivative of compound of formula (I-5) such as a boronate, to yield the compound of formula (I-11): 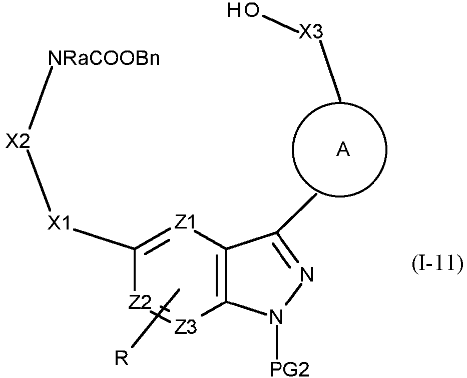 wherein R, Ra, X1, X2, X3, Z1, Z2, Z3, A and PG2 are as defined hereinbefore,
      compound of formula (I-11) which is subjected to a cyclization to yield the compound of formula (I-7) as defined above, which, after deprotection of -N(PG2)-, and/or optional substitution on the A ring, gives the compound of formula (I),
- or compound of formula (I-2) on which is condensed, after deprotection of X1, a compound of formula (I-12): 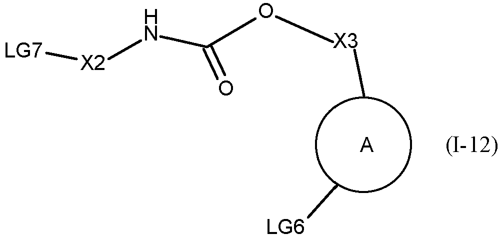 wherein A, X3 and X2 are as defined hereinbefore and LG6 and LG7 are leaving groups, to yield a compound of formula (I-13) : 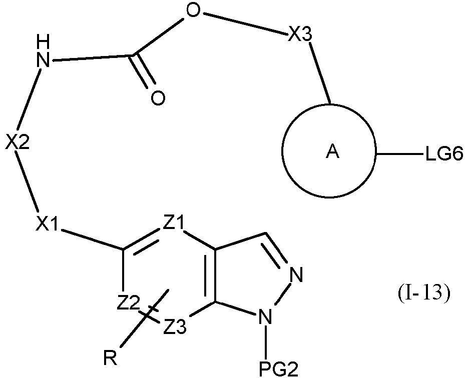 wherein R, X1, X2, X3, A, Z1, Z2, Z3, PG2 and LG6 are as defined hereinbefore, compound of formula (I-13) that is cyclized to yield the compound of formula (I-7) which is optionally alkylated on the carbamate function, then submitted to the deprotection of -N(PG2), and/or optionally substituted on the A ring, to give the compound of formula (I),
- or compound of formula (I-2) which is transformed in a boronic derivative of formula (I-14): 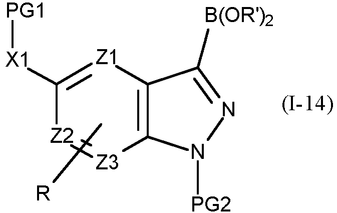 wherein R, X1, Z1, Z2, Z3, PG1 and PG2 are as defined hereinbefore, and R' represents a hydrogen atom or an alkyl group, it being understood that the two R' alkyl group can be linked to form a cyclic structure,
   - compound of formula (I-14) on which is condensed a compound of formula (I-15): 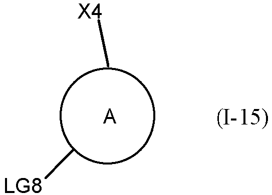 wherein A is as defined herein before, X4 is a carboxylic acid or an ester or a carbonyl derivative of X3, and LG8 is a leaving group, to yield the compound of formula (I-16): 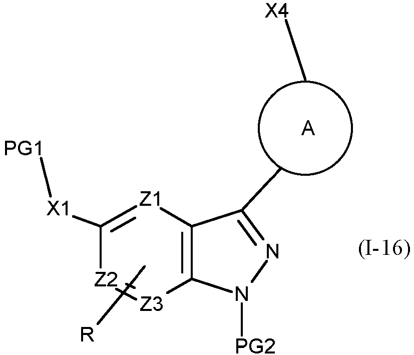 wherein R, X1, Z1, Z2, Z3, X4, PG1 and PG2 are as defined hereinbefore,
      compound of formula (I-16) on which is condensed, after deprotection of X1 a compound LG5-X2-NRaCOOBn as defined hereinbefore to yield the compound of formula (I-17): 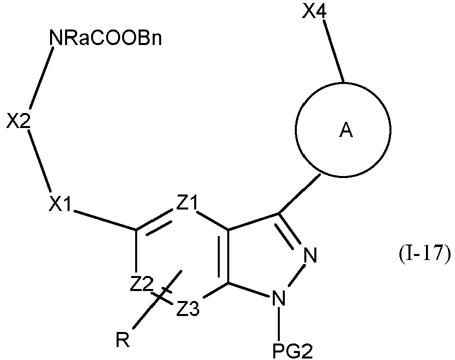 wherein R, Ra, X1, X2, Z1, Z2, Z3, X4 and PG2 are as defined hereinbefore,
      which is submitted to a reduction to yield the compound of formula (I-11) that is converted to compound of formula (I) as described hereinabove,
   - or compound of formula (I-14) on which is condensed a compound of formula (I-18): 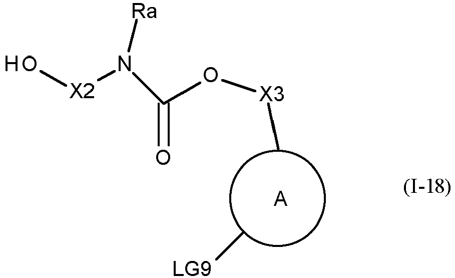 wherein A, X2, X3 and Ra are as defined herein before, and LG9 is a leaving group, to yield the compound of formula (I-19): 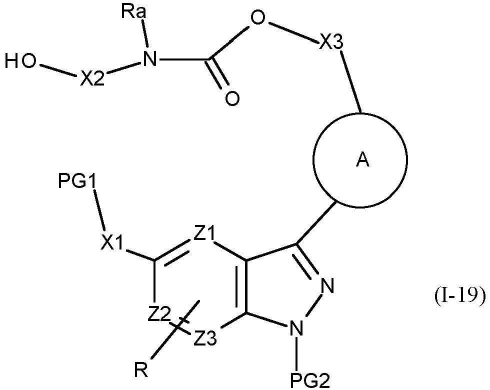 wherein R, Ra, A, X1, X2, X3, Z1, Z2, Z3, PG1 and PG2 are as defined hereinbefore, compound of formula (I-19) on which is introduced a leaving group to yield the compound of formula (I-9) as defined above, that is converted to compound of formula (I) as described above,
- or compound of formula (I-2) on which is condensed, after deprotection of X1, a compound LG5-X2-NRaCOOBn as defined hereinbefore to yield a compound of formula (I-20): 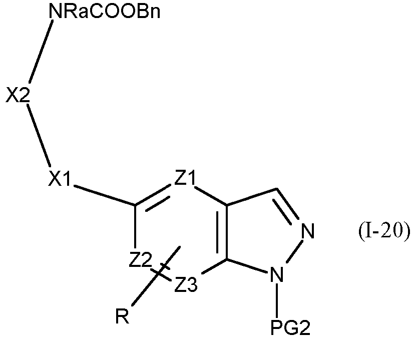 wherein R, Ra, X1, X2, Z1, Z2, Z3 and PG2 are as defined hereinbefore,
   compound of formula (I-20) which is transformed in a boronic derivative of formula (I-21): 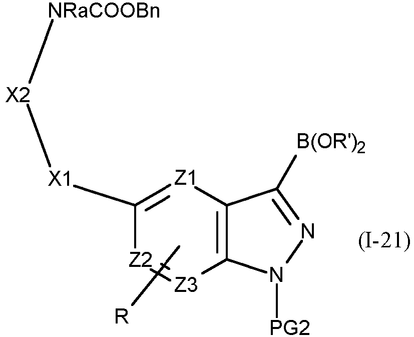 wherein R, Ra, X1, X2, Z1, Z2, Z3, PG2 and R' are as defined hereinbefore,
   - compound of formula (I-21) on which is condensed a compound of formula (I-22): 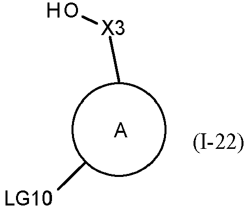 wherein X3 and A are as defined hereinbefore, and LG10 is a leaving group, to yield the compound of formula (I-11) that is converted to compound of formula (I) as described hereinabove,
   - or compound of formula (I-21) on which is condensed a compound of formula (I-15) as defined hereinbefore to yield the compound of formula (I-17) that is converted to compound of formula (I) as described hereinabove,
   the compound of formula (I), may then be purified according to a conventional separation technique, and is converted, if desired, into its addition salts with a pharmaceutically acceptable acid or base and which is optionally separated into its isomers according to a conventional separation technique,
   it being understood that at any moment considered appropriate during the course of the process described above, some groups of the starting reagents or of the synthesis intermediates can be protected, subsequently deprotected and functionalized, as required by the synthesis.

The compounds of formulae (I-5), (I-8), (I-12), (I-15), (I-18) and (I-22) are either commercially available or can be obtained by the person skilled in the art using conventional chemical reactions described in the literature.

Pharmacological studies of the compounds of the invention of formula (I) exhibit inhibitory activity against LRRK2 kinase, including LRRK2 mutant kinase, such as mutant p.G2019S. Kinase activity can be determined using a kinase assay, which typically employs a kinase substrate and a phosphate group donor such as ATP (or a derivative thereof). An exemplary kinase assay is described in the Pharmacological Study.

Compounds of formula (I) of the invention or pharmaceutically acceptable salts thereof are inhibitors of LRRK2 kinase activity and are thus believed to be of potential use in the treatment of or prevention of diseases associated with or characterized by LRRK2 kinase activity such as neurological diseases, endosomal-lysosomal disorders, inflammatory diseases, bacterial, viral and parasitic infections, cardiovascular diseases, autoimmune diseases and cancers. Particularly compounds of the invention are useful in the treatment of neurological diseases including but not limited to Parkinson's disease (including sporadic Parkinson's disease patients as well as patients with LRRK2 mutations such as p.G2019S or Rab29/Rab7L1 polymorphisms), Alzheimer's disease, amyotrophic lateral sclerosis (ALS), dementia (including Lewy body dementia and vascular dementia, HIV-induced dementia), diabetic neuropathy, age related memory disfunction, mild cognitive impairment, argyrophilic grain disease, Pick's disease, epilepsy, tauopathies such as progressive supranuclear palsy and corticobasal degeneration, other synucleinopathies such as multiple system atrophy, frontotemporal dementia, inherited frontotemporal dementia and parkinsonism linked to chromosome 17 (FTDP-17), withdrawal symptoms/relapse associated with drug addiction, L-Dopa induced dyskinesia, ischemic stroke, traumatic brain injury, spinal cord injury and multiple sclerosis.

Other diseases potentially treatable by inhibition of LRRK2 activity are endosomal-lysosomal diseases including but not limited to Niemann-Pick Type A, B or C disease, Gaucher's disease, Krabbe's disease, Fabry's disease and disorders with mitochondrial deficits; inflammatory diseases including but not limited to vasculitis, pulmonary diseases such as chronic obstructive pulmonary disease, idiopathic pulmonary fibrosis, inflammatory myopathies, ankylosing spondylitis; autoimmune diseases including but not limited to Crohn's disease, inflammatory bowel disease, rheumatoid arthritis, ulcerative colitis, lupus, autoimmune hemolytic anemia, pure red cell aplasia, idiopathic thrombocytopenic purpura, type I diabetes mellitus, obesity, Evans syndrome, bullous skin disorders, Sjogren's syndrome, Devic's disease and leprosy. Compounds of the invention have also anti carcinogenic effects and are potentially useful in the treatment of cancers including but not limited to thyroid cancer, renal cancer (including papillary renal), breast cancer, hormone-related cancer, adeno-and squamous lung cancer, non-small-cell lung cancer, colon cancer, prostate cancers, skin cancers, leukemias (including acute myelogenous leukemia) and lymphomas.

Compounds of the invention are also potentially useful in the treatment of cardiovascular diseases including but not limited to stroke.

Other diseases potentially treatable by compounds of the invention are bacterial infections such as but not limited to leprosy and tuberculosis; viral infections such as but not limited to coronavirus such as SARS-CoV, MERS-CoV and SARS-CoV-2, HIV, West Nile virus and chikungunya virus.

Another aspect of the invention is related to pharmaceutical compositions comprising at least one compound of formula (I) in combination with one or more pharmaceutically acceptable excipients. In particular, these pharmaceutical compositions are interesting for use in the treatment or prevention of diseases associated with or characterized by LRRK2 kinase activity such as but not limited to neurological diseases, endosomal-lysosomal disorders, inflammatory diseases, bacterial, viral and parasitic infections, cardiovascular diseases, autoimmune diseases and cancers. In a specific embodiment, parmaceutical compositions of the invention are useful for the treatment or prevention of Parkinson's disease (including sporadic Parkinson's disease patients as well as patients with LRRK2 mutations or Rab29/Rab7L1 polymorphisms), Alzheimer's disease, amyotrophic lateral sclerosis (ALS), dementia (including Lewy body dementia and vascular dementia, HIV-induced dementia), diabetic neuropathy, age related memory disfunction, mild cognitive impairment, argyrophilic grain disease, Pick's disease, epilepsy, tauopathies such as progressive supranuclear palsy and corticobasal degeneration, other synucleinopathies such as multiple system atrophy, frontotemporal dementia, inherited frontotemporal dementia and parkinsonism linked to chromosome 17 (FTDP-17), withdrawal symptoms/ relapse associated with drug addiction, L-Dopa induced dyskinesia, ischemic stroke, traumatic brain injury, spinal cord injury, multiple sclerosis, Niemann-Pick Type A, B or C disease, Gaucher's disease, Krabbe's disease, Fabry's disease, disorders with mitochondrial deficits, Crohn's disease, inflammatory bowel disease, rheumatoid arthritis, ulcerative colitis, lupus, autoimmune hemolytic anemia, pure red cell aplasia, idiopathic thrombocytopenic purpura, type I diabetes mellitus, obesity, Evans syndrome, bullous skin disorders, Sjogren's syndrome, Devic's disease, leprosy, thyroid cancer, renal cancer (including papillary renal), breast cancer, hormone-related cancer, adeno-and squamous lung cancer, non-small-cell lung cancer, colon cancer, prostate cancers, skin cancers, leukemias (including acute myelogenous leukemia), lymphomas, stroke, leprosy, tuberculosis, and SARS-CoV, MERS-CoV, SARS-CoV-2, HIV, West Nile virus and chikungunya virus infections.

Among the pharmaceutical compositions according to the invention there may be mentioned more especially those that are suitable for oral, parenteral, nasal, per- or trans-cutaneous, rectal, perlingual, ocular or respiratory administration, especially tablets or dragées, sublingual tablets, sachets, paquets, capsules, glossettes, lozenges, suppositories, creams, ointments, dermal gels, and drinkable or injectable ampoules.

The pharmaceutical compositions according to the invention comprise one or more excipients or carriers selected from diluents, lubricants, binders, disintegration agents, stabilisers, preservatives, absorbents, colorants, sweeteners, flavourings etc.

By way of non-limiting example there may be mentioned:
◆ *as diluents:* lactose, dextrose, sucrose, mannitol, sorbitol, cellulose, glycerol,
◆ *as lubricants:* silica, talc, stearic acid and its magnesium and calcium salts, polyethylene glycol,
◆ *as binders:* magnesium aluminium silicate, starch, gelatin, tragacanth, methylcellulose, sodium carboxymethylcellulose and polyvinylpyrrolidone,
◆ *as disintegrants:* agar, alginic acid and its sodium salt, effervescent mixtures.

The dosage varies according to the sex, age and weight of the patient, the administration route, the nature of the therapeutic indication, or of any associated treatments, and ranges from 0.01 mg to 1 g per 24 hours in one or more administrations.

The following Preparations and Examples illustrate the invention but do not limit it in any way. The compounds of this invention can be prepared by any of several standard synthetic processes commonly used by those skilled in the art of organic chemistry. The compounds are generally prepared from starting materials which are either commercially available or prepared by standard means obvious to those skilled in the art.

### GENERAL SCHEMES

As indicated herein before, the present invention provides compounds according to formula (I): 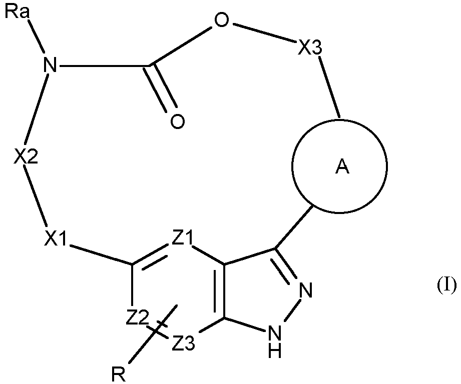 wherein R, Z1, Z2, Z3, X1, X2, X3, Ra and A are as defined for formula (I).

With reference to the general reaction schemes suitable for preparing said compounds, these compounds can be represented by formula (I), for which the general reaction schemes can be found herein below. In the general schemes below, R, Z1, Z2, Z3, X1, X2, X3, Ra and A will have the same meaning as defined for formula (I).

The fused pyrazolo bicyclic structure containing Z1, Z2, Z3 and R will be referred to as fused pyrazolo structure in the followings.

In the general schemes below, Lg₁ and Lg₂ each independently represent suitable leaving groups. Pg₁ and Pg₃ each independently represent a suitable protecting group that can be used to protect X1 and/or X2. Pg₂ represents a protective group suitable to protect the NH of the fused pyrazolo structure.

Rb in the schemes below can be either H, alkyl or a cyclic alkyl.

For those compounds for which a transcarbamylation reaction is used, the CbzX2Lg2 moiety can be made either by reaction from the corresponding bromo alkyl amine through reaction with Cbz chloride or by reaction between the hydroxyalkylamine through reaction with Cbz chloride followed by mesylation or tosylation.

In all of the general schemes below, before deprotection of the NH of the fused pyrazolo structure, the carbamate can be optionally substituted by an alkylation reaction to give a compound of formula (XIIIa) after which the NH of the fused pyrazolo structure can be deprotected to result in the final compound of formula (I).

Alternatively, in all of the general schemes below, before deprotection of the NH of the fused pyrazolo structure, an optional cross-coupling reaction such as a Buchwald, Suzuki, Sonogashira reaction or alternatively an O-alkylation or nucleophilic aromatic substitution can be carried out on the (hetero-) aromatic ring which contains a leaving group such as a halide, to form a compound of formula (XIIIa). After the cross-coupling reaction such as a Buchwald, Suzuki, Sonogashira reaction or alternatively an O-alkylation or nucleophilic aromatic substitution, the NH of the fused pyrazolo structure can be deprotected to result in the final compound of formula (I).

The compounds of formula (I) can be prepared as shown in general **Scheme A** below wherein the compound of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) which is then alkylated with an intermediate of formula (VIII) containing a leaving group resulting in a compound of formula (IX). The compound of formula (VIII) can be prepared from a compound of formula (VII) through a nucleophilic substitution. The compound of formula (IX) can be coupled via organometallic cross-coupling such as Suzuki or Ullmann coupling with a (hetero-)aryl of formula (X) or (Xa) to form a compound of formula (XI). The compound of formula (XI) can then be selectively deprotected to a compound of formula (XII) before being cyclized to form a compound of formula (XIII). Final deprotection of the nitrogen of the fused pyrazolo structure, either or not after alkylation of the carbamate moiety and/or substitution of the A ring, results in the compound of formula (I).

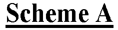

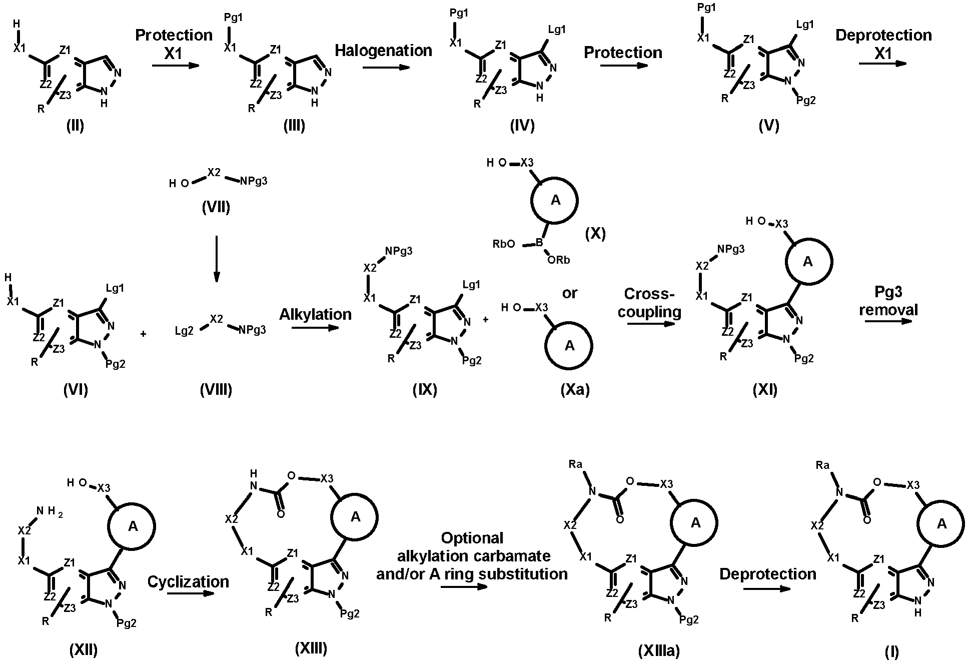

In the above reaction **Scheme** A, the reaction between a compound of formula (VI) and a compound of formula (VIII) can be accomplished in a solvent such as N,N-dimethylformamide or acetonitrile and a base such as cesium carbonate or potassium carbonate.

In the above reaction between compound of formula (IX) and compound of formula (X), the leaving groups Lg₁ is advantageously a halogen atom such as chlorine, bromine or iodine. Such a halogen displacement reaction can be effected under cross-coupling conditions such as Suzuki conditions using palladium catalysts such as for example tetrakis(triphenylphosphine) palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

Alternatively, the halogen displacement reaction can be effected under Ullmann conditions using copper iodide in the presence of potassium carbonate and 8-hydroxyquinoline in a solvent such as for example dimethyl sulfoxide at an elevated temperature such as for example 70°C. Suitable compounds of formula (X) or formula (Xa) may be either commercially acquired or obtained through various selective protection and deprotection steps known to the person skilled in the art. For the synthesis of compounds of formula (Xa) a borylation step might be required.

The deprotection of Pg₃ results in a compound of formula (XII).

The cyclisation of the compound of formula (XII) to give compound of formula (XIII) can be performed by a method known by the person skilled in the art as a carbamylation reaction, for example by treatment with 1,1'-carbonyldiimidazole and *N,N-*diisopropylethylamine or sodium hydride in a solvent such as *N,N-*dimethylacetamide at for example 90°C. Final deprotection of the NH of the fused pyrazolo structure under acidic conditions, either or not after alkylation of the carbamate and/or substitution of the A ring yields the final compound of formula (I).

Alternatively, the compounds of formula (I) can be prepared as shown in general **Scheme B** below wherein the fused pyrazolo structure of formula (II) is converted to a protected compound of formula (III). The NH of the fused pyrazolo structure can be protected to a compound of formula (XIV). This compound of formula (XIV) can be converted to a boronic acid (or boronate ester) of formula (XV). The compound of formula (XV) can be coupled via organometallic cross-coupling reaction such as Suzuki coupling with a (hetero-)aryl of formula (XVI) to form a compound of formula (XVII). The compound of formula (XVII) can be alkylated with an intermediate of formula (XIX) containing a carbamate such as a benzyl carbamate resulting in a compound of formula (XX). The compound of formula (XIX) can commercially be acquired or being prepared from a compound of formula (XVIII) through a reaction with CbzCl or through the introduction of a leaving group Lg2 on the compound of formula (XVIIIa). The X4 moiety of compound (XX) can be transformed into X3-OH usually by a reduction of a carboxylic acid or a carboxylic ester or a (cyclo)alkyl-carbonyl or a heterocycloalkyl-carbonyl. The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

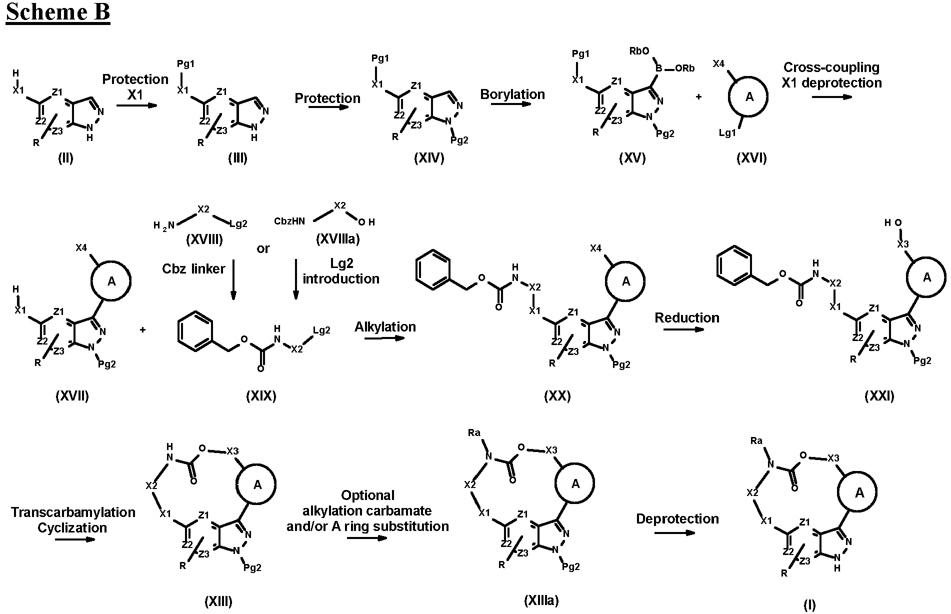

In the above reaction Scheme **B,** fused pyrazolo structure borylation of a compound of formula (XV) to the compound of formula (XVI) can be accomplished using an iridium catalyst and bis(pinacolato)diboron in a solvent such as TBME.

In the above reaction between compound of formula (XV) and compound of formula (XVI), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine) palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 110°C either under microwave conditions or not.

In the above reaction scheme, the alkylation between a compound of formula (XVII) with a compound of formula (XIX) can be accomplished in a solvent such as*N,N-*dimethylformamide or acetonitrile and a base such as cesium carbonate or potassium carbonate at an elevated temperature such as 120°C. Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

X4 in the compound of formula (XX) can be a (cyclo)alkyl-carbonyl, heterocycloalkyl-carbonyl or carboxylic derivative (carboxylic acid or ester) which can be reduced into the corresponding alcohol making use of sodium borohydride or lithium aluminium hydride in a solvent such as THF at an elevated temperature such as 120°C.

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such as toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme C** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) which is then alkylated with an intermediate of formula (XIX) containing a leaving group resulting in a compound of formula (XXII). The compound of formula (XIX) can commercially be acquired or being prepared from a compound of formula (XVIII) through a reaction with CbzCl or through the introduction of a leaving group Lg2 on the compound of formula (XVIIIa). The compound of formula (XXII) can be coupled via organometallic cross coupling reaction such as Suzuki coupling with a (hetero-)aryl of formula (X) to form a compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

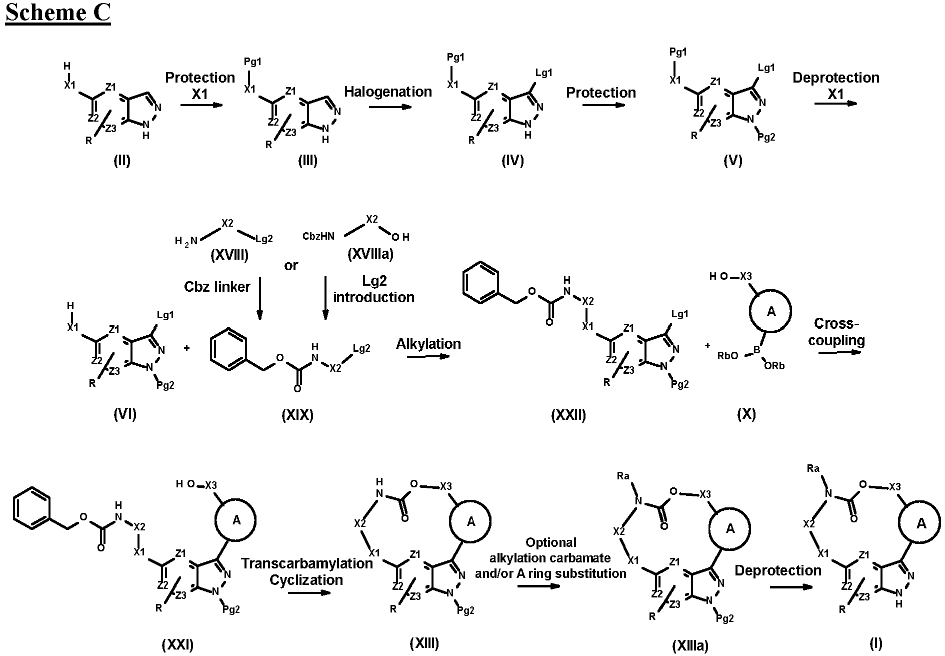

In the above reaction **Scheme C,** the alkylation between a compound of formula (VI) with a compound of formula (XIX) can be accomplished in a solvent such as*N,N-*dimethylformamide or acetonitrile and a base such as cesium carbonate or potassium carbonate at an elevated temperature such as 120°C.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

In the above reaction between compound of formula (XXII) and compound of formula (X), the leaving groups Lg₁ is advantageously a halogen atom such as chlorine, bromine or iodine. Such a halogen displacement reaction can be effected under organometallic coupling conditions such as Suzuki conditions using palladium catalysts such as for example tetrakis (triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 110°C either under microwave conditions or not.

Suitable compounds of formula (X) may be either commercially acquired or obtained through various selective protection and deprotection steps known to the person skilled in the art. A borylation step might be required to obtain compounds of formula (X).

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such as toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme D** below wherein the fused pyrazolo structure of formula (II) is converted to a protected compound of formula (III) and then into a nitrogen protected compound of formula (XIV). The compound of formula (XIV) can be converted into a selectively protected fused pyrazolo structure of formula (XXIII) which is then alkylated with a compound of intermediate (XIX) containing a Cbz group to result in a compound of formula (XXIV). Compound of formula (XIX) can be prepared from a compound of formula (XVIII) through a reaction with CbzCl or through the introduction of a leaving group Lg2 on the compound of formula (XVIIIa). The compound of formula (XXIV) can be boronated to a compound of formula (XXV). The boronated compounds of formula (XXV) can be reacted in a cross-coupling reaction such as a Suzuki coupling with a (hetero-)aryl of formula (XXVI) to form a compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

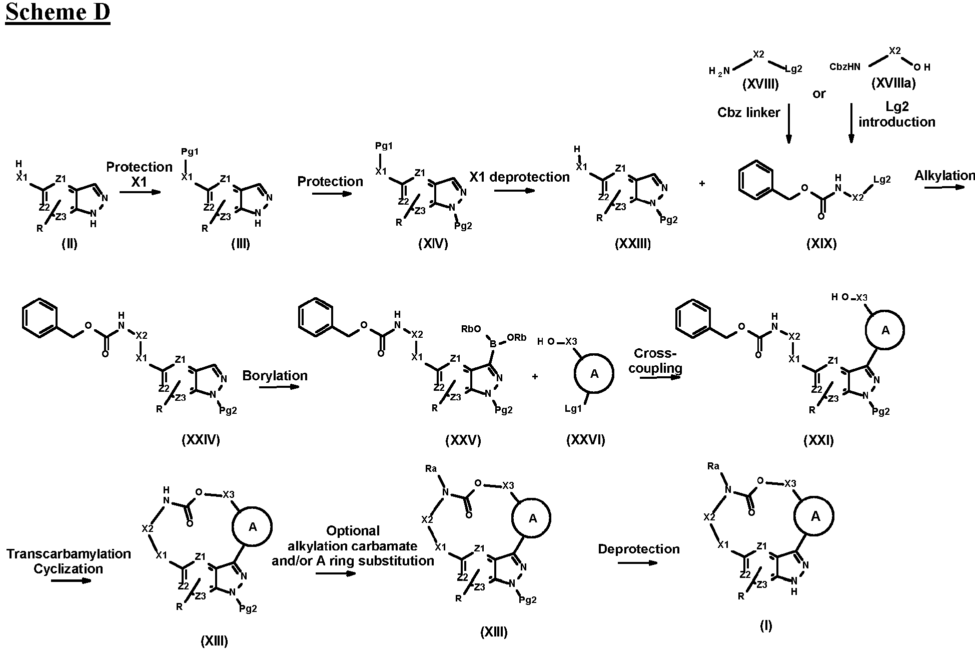

In the above reaction **Scheme D,** the reaction between a compound of formula (XXIII) and a compound of formula (XIX) can be accomplished in a solvent such as*N*,*N*-dimethylformamide or acetonitrile and a base such as cesium carbonate or potassium carbonate.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

In the above reaction scheme, fused pyrazolo structure borylation of a compound of formula (XXIV) to the compound of formula (XXV) can be accomplished using an iridium catalyst and bis(pinacolato)diboron in a solvent such as TBME.

In the above reaction between compound of formula (XXV) and compound of formula (XXVI), the leaving groups Lg₁ is advantageously a halogen atom such as chlorine, bromine or iodine. Such a halogen displacement reaction can be effected under organometallic cross coupling conditions such as Suzuki conditions using palladium catalysts such as for example tetrakis (triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

Suitable compounds of formula (XXVI) may be either commercially acquired or obtained through various reactions including selective protection and deprotection steps known to the person skilled in the art.

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme E** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) which is then coupled in a cross-coupling reaction such as a Suzuki coupling with a (hetero-)aryl of formula (XXVII) to form a compound of formula (XXVIII). The X4 moiety in the compound of formula (XXVII) contains a carbonyl precursor such as (cyclo)alkyl-carbonyl, heterocycloalkyl-carbonyl, carboxylic acid or ester which can be reduced into a compound of formula (XXIX). The compound of formula (XXIX) is then alkylated with an intermediate of formula (XIX) containing a leaving group resulting in a compound of formula (XXI). The compound of formula (XIX) can commercially be acquired or being prepared from a compound of formula (XVIII) through a reaction with CbzCl or through the introduction of a leaving group Lg2 on the compound of formula (XVIIIa). The compound of formula (XXI) can then be cyclized by a transcarbamylation reaction to form a compound of formula (XIII). Final deprotection of the nitrogen fused pyrazolo structure, either or not after alkylation of the carbamate and/or substitution of the A ring results in the compound of formula (I).

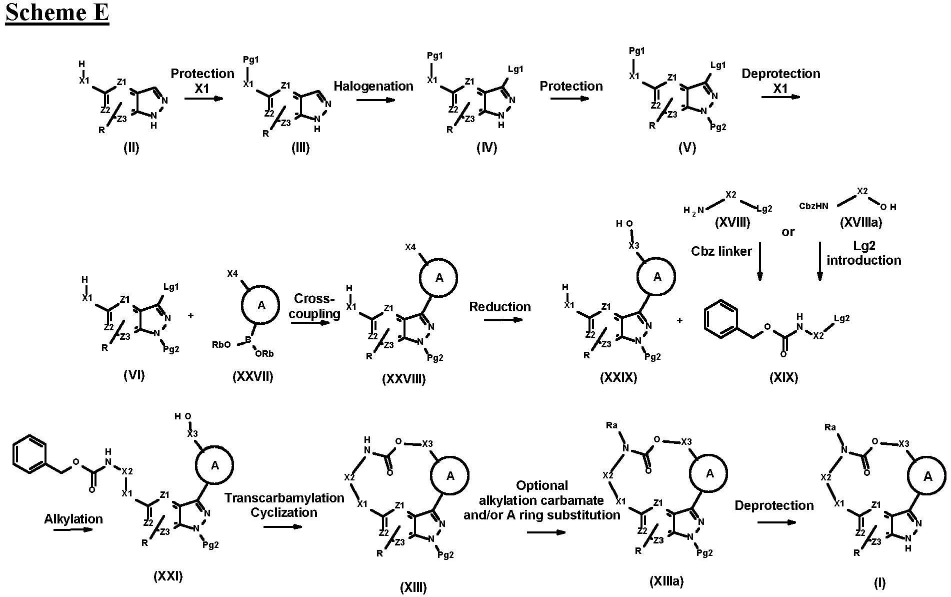

In the above **Scheme E,** reaction between compounds of formula (VI), the leaving groups Lg₁ is advantageously a halogen atom such as chlorine, bromine or iodine. Such a halogen displacement reaction can be affected under organometallic cross coupling conditions such as Suzuki conditions using palladium catalysts such as for example tetrakis(triphenylphosphine) palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 110°C either under microwave conditions or not.

Suitable compounds of formula (XXVII) contain a precursor moiety of the alcohol such as an ester or a carboxylic acid. Compounds of formula (XXVII) may be either commercially acquired or obtained through various reactions including selective protection and deprotection steps known to the person skilled in the art. For the compounds of formula (XXVII) a borylation step might be required.

Reduction of the X4 carbonyl in the compound of formula (XXVIII) results in a compound of formula (XXIX).

In the above reaction scheme, the alkylation between a compound of formula (XXIX) with a compound of formula (XIX) can be accomplished in a solvent such as *N*,*N-*dimethylformamide or acetonitrile and a base such as cesium carbonate or potassium carbonate at an elevated temperature such as 120°C.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such as toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme F** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) which is then alkylated with an intermediate of formula (XIX) containing a leaving group resulting in a compound of formula (XXII). The compound of formula (XIX) can commercially be acquired or being prepared from a compound of formula (XVIII) through a reaction with CbzCl or through the introduction of a leaving group Lg2 on the compound of formula (XVIIIa). The compound of formula (XXII) can be coupled via organometallic cross coupling reaction such as Suzuki coupling with a (hetero-)aryl of formula (XXVII) to form a compound of formula (XX). The X4 moiety in the compound of formula (XX) contains a carbonyl precursor such as (cyclo)alkyl-carbonyl, heterocycloalkyl-carbonyl, carboxylic acid or ester which can be reduced into a compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

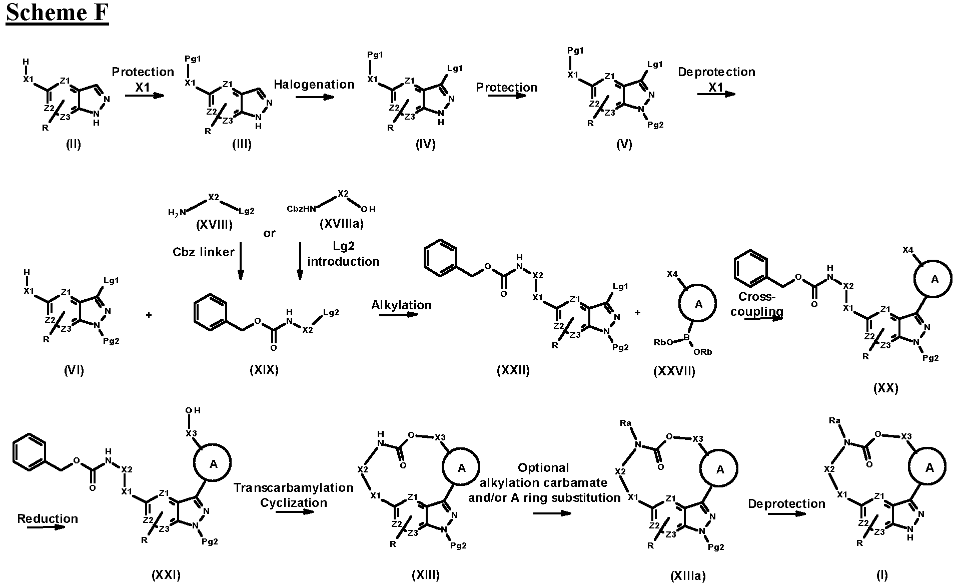

In the above reaction **Scheme F,** the alkylation between a compound of formula (VI) with a compound of formula (XIX) can be accomplished in a solvent such as*N*,*N*-dimethylformamide or acetonitrile and a base such as cesium carbonate or potassium carbonate at an elevated temperature such as 120°C.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

In the above reaction between compound of formula (XXII) and compound of formula (XXVII), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 110°C either under microwave conditions or not.

Suitable compounds of formula (XXVII) may be either commercially acquired or obtained through various reactions including selective protection and deprotection steps known to the person skilled in the art. For the compounds of formula (XXVII) a borylation step might be required.

Reduction of the X4 carbonyl in the compound of formula (XX) results in a compound of formula (XXI).

Transcarbamylation of the compound of formula (XXI) to the macrocycle of formula (XIII) can be achieved using sodium hydride in dry toluene at an elevated temperature such as ranging from 130°C or 150°C. Alternatively, the transcarbamylation can be done using potassium carbonate or KOH in a solvent such as acetonitrile at an elevated temperature such as 140°C. Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme G** below wherein the fused pyrazolo structure of formula (II) is converted to a protected compound of formula (III) and then into a nitrogen protected compound of formula (XIV). The compound of formula (XIV) can be converted into a selectively protected fused pyrazolo structure of formula (XXIII) which is then alkylated with a compound of intermediate (XIX) containing a Cbz group to result in a compound of formula (XXIV). Compound of formula (XIX) can be prepared from a compound of formula (XVIII) through a reaction with CbzCl or through the introduction of a leaving group Lg2 on the compound of formula (XVIIIa). The compound of formula (XXIV) can be boronated to a compound of formula (XXV). The boronated compounds of formula (XXV) can be reacted in a cross-coupling such as a Suzuki coupling with a (hetero-)aryl of formula (XVI) to form a compound of formula (XX). The X4 moiety in the compound of formula (XX) contains a carbonyl precursor such as (cyclo)alkyl-carbonyl, heterocycloalkyl-carbonyl, carboxylic acid or ester which can be reduced into a compound of formula (XXI).. The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

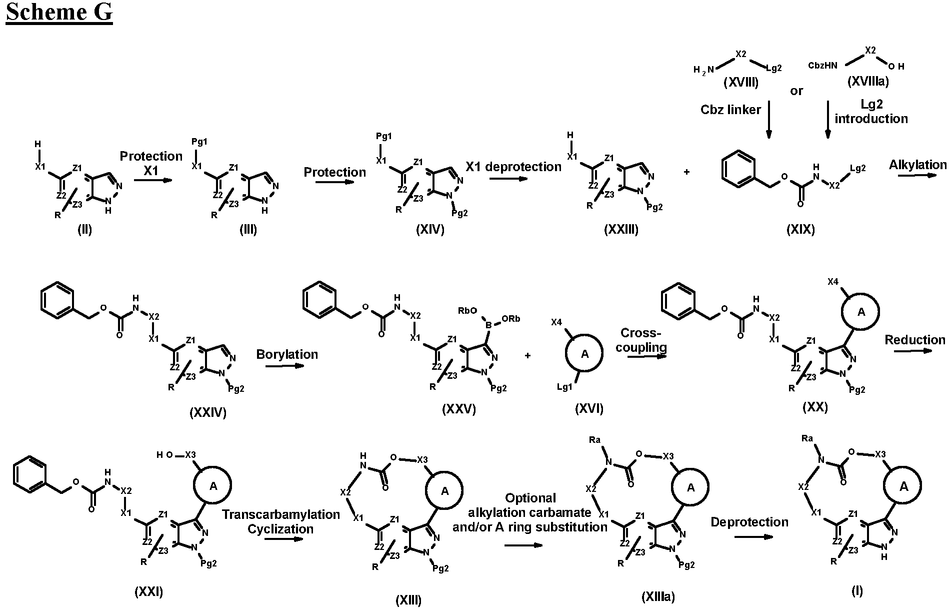

In the above reaction **Scheme G,** the reaction between a compound of formula (XXIII) and a compound of formula (XIX) can be accomplished in a solvent such as*N*,*N*-dimethylformamide or acetonitrile and a base such as cesium carbonate or potassium carbonate.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

In the above reaction scheme, fused pyrazolo structure borylation of a compound of formula (XXIV) to a compound of formula (XXV) can be accomplished using an iridium catalyst and bis(pinacolato)diboron in a solvent such as TBME.

In the above reaction between compound of formula (XXV) and compound of formula (XVI), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine) palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

Suitable compounds of formula (XVI) may be either commercially acquired or obtained through various reactions including selective protection and deprotection steps known to the person skilled in the art.

The carbonyl moiety of X4 in the compound of formula (XX) can be reduced into the corresponding alcohol making use of, for instance, sodium borohydride or lithium aluminium hydride in a solvent such as THF at an elevated temperature such as 120°C.

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme H** below wherein the fused pyrazolo structure of formula (II) is converted to a protected compound of formula (III) and then into a nitrogen protected compound of formula (XIV). The compound of formula (XIV) can be converted into a selectively protected fused pyrazolo structure of formula (XXIII) which is then alkylated with a compound of intermediate (XXX) containing a (hetero-)aromatic group to result in a compound of formula (XXXI). Compound of formula (XXX) can be prepared using different reaction steps known to the person skilled in the art and is in detail described for the exemplified compounds. The compound of formula (XXXI) can be macrocyclized through a CH-activation reaction. Final deprotection of the nitrogen of the fused pyrazolo structure, either or not after alkylation of the carbamate and/or substitution of the A ring results in the compound of formula (I).

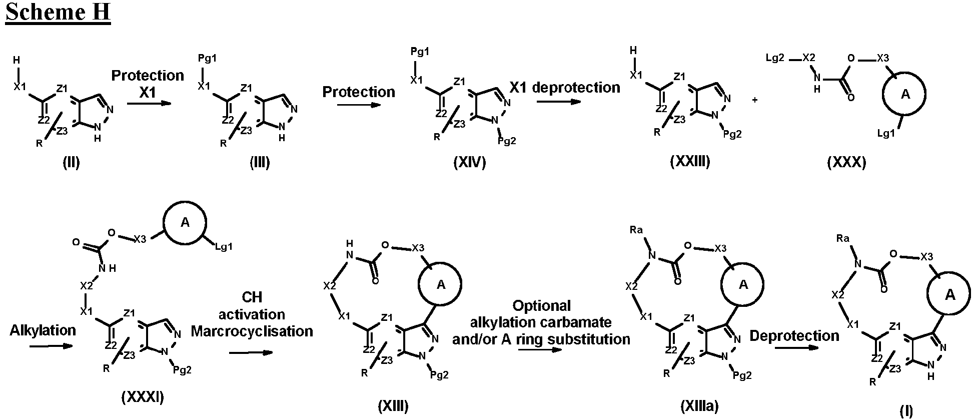

In the above reaction **Scheme H,** the alkylation between a compound of formula (XXIII) with a compound of formula (XXX) can be accomplished in a solvent such as *N,N-*dimethylformamide or acetonitrile and a base such as cesium carbonate or potassium carbonate at an elevated temperature such as 80°C.

Suitable compounds of formula (XXX) may be either commercially acquired or obtained through synthesis routes available in the literature. In the above reaction between compound of formula (XXX) and compound of formula (XXIII), the leaving groups Lg₂ is advantageously a mesylate group.

CH activation of the compound of formula (XXXI) to the macrocycle of formula (XIII) can be achieved using CataCXium, palladium acetate and potassium acetate in dry toluene under microwave conditions at an elevated temperature such as 140°C. The leaving group Lg₁ is advantageously a halogen atom such as chlorine, bromine or iodine. Final deprotection of the nitrogen of the fused pyrazolo structure under acidic conditions, either or not after alkylation of the carbamate and/or substitution of the A ring yields the final compound of formula (I).

Alternatively, the compounds of formula (I), a particular case of compounds of formula (I) wherein X1 is NR'a can be prepared as shown in general **Scheme I** below wherein the fused pyrazolo structure of formula (XXXII) in which X5 is for instance a nitro group is converted to a protected compound of formula (XXXIII) and then into a nitrogen protected compound of formula (XXXIV). The compound of formula (XXXIV) can be converted into a selectively protected fused pyrazolo structure of formula (VI) which is then alkylated with a compound of formula (VIII) containing a protecting group Pg3. After alkylation, deprotection of X2 results in a compound of formula (XXXVI) which is then coupled in a cross-coupling reaction such as a Suzuki reaction with a compound of formula (X). The resulting compound of formula (XII) can be macrocyclized affording a compound of formula (XIII). Final deprotection of the nitrogen of the fused pyrazolo structure, either or not after alkylation of the carbamate and/or substitution of the A ring results in the compound of formula (I).

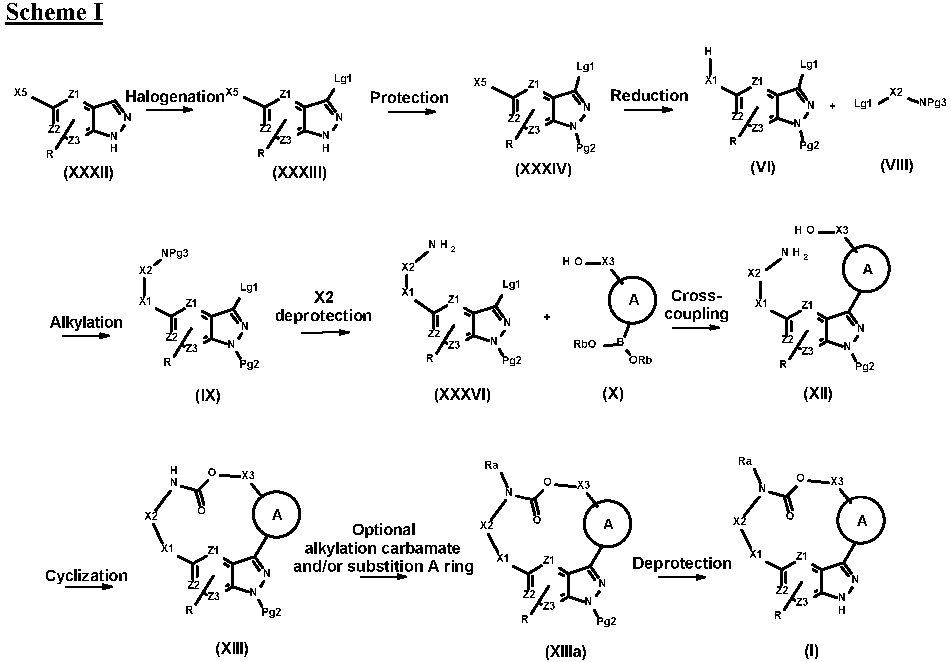

In the above reaction **Scheme I,** X5 is a nitro group and X1 is in this scheme particularly NR'a. Halogenation of the fused pyrazolo structure can be achieved using for example iodine and potassium hydroxide in a solvent such as N,N-dimethylformamide at an elevated temperature such as 60°C.

Reduction of the nitro group can be obtained using iron in the presence of ammonia chloride in a mixture of solvents such as EtOH, THF and water at an elevated temperature such as 80°C to yield a compound of formula (VI).

The alkylation between a compound of formula (VI) with a compound of formula (VIII) can be accomplished in a solvent such as *N*,*N*-dimethylformamide or acetonitrile and a base such as cesium carbonate or potassium carbonate at an elevated temperature such as 80 or 90°C. The compound of formula (VIII) contains a protecting group Pg3, which can be a phthalimide group. Deprotection of X2-NPg3 in a compound of formula (IX) can be achieved using a reagent such as hydrazine in a solvent such as EtOH at an elevated temperature such as 60°C.

Organometallic cross coupling such as Suzuki coupling of the compound of formula (XXXVI) with a compound of formula (X) can be done using palladium catalysts such as for example tetrakis(triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 120°C either under microwave conditions or not.

The cyclisation of the compound of formula (XII) to give compound of formula (XIII) can be performed by a method known by the person skilled in the art as a carbamylation reaction, for example by treatment with 1,1'-carbonyldiimidazole and *N*,*N*-diisopropylethylamine in a solvent such as *N*,*N*-dimethylacetamide at for example 90°C. Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme J** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) in which then X1 is protected to form a compound of formula (XXXVII). The compound of formula (XXXVII) can be coupled via organometallic cross coupling such as Suzuki coupling with a (hetero-)aryl of formula (XXXVIII) to form a compound of formula (XXXIX). Alkylation of the (hetero-)aromatic ring gives rise to a compound of formula (XL). Deprotection of X1 followed by alkylation with a compound of formula (XIX) results in a compound of formula (XLII). The compound of formula (XIX) can commercially be acquired or being prepared from a compound of formula (XVIII) through a reaction with CbzCl or through the introduction of a leaving group Lg2 on the compound of formula (XVIIIa). Deprotection of X3 leads to a compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

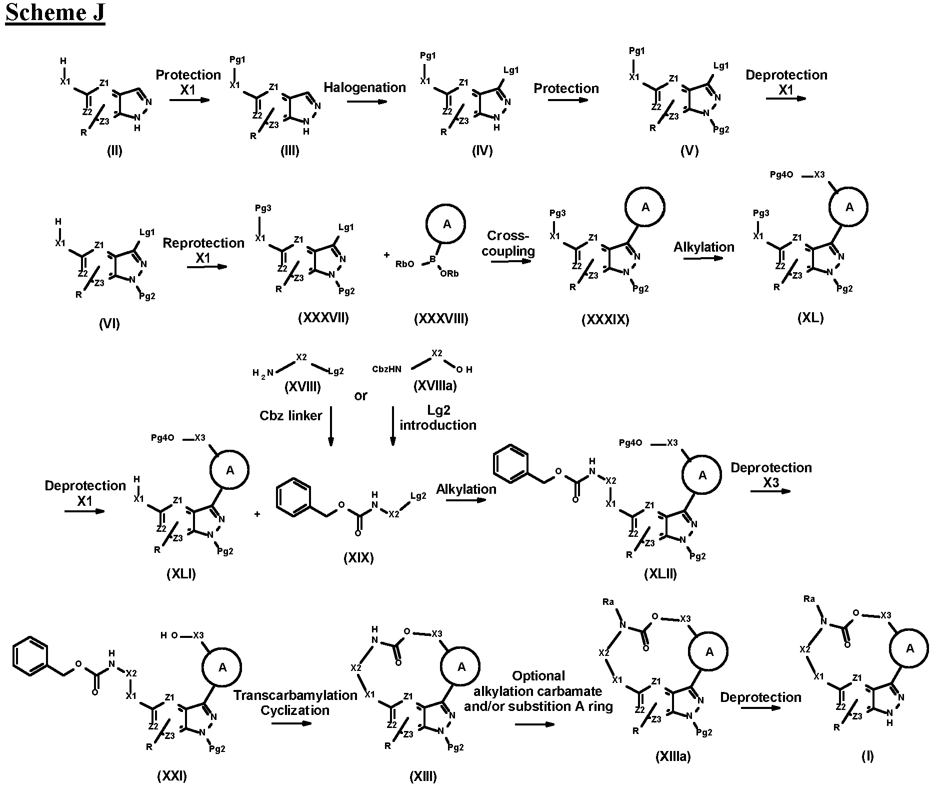

In the above reaction **Scheme J,** the protection of X1 of compound of formula (VI) can be accomplished with benzyl chloride in a solvent such as *N*,*N-*dimethylformamide or acetonitrile and a base such as cesium carbonate or potassium carbonate at room temperature or at an elevated temperature.

In the above reaction between compound of formula (XXXVII) and compound of formula (XXXVIII), the leaving groups Lg₁ is advantageously a halogen atom such as chlorine, bromine or iodine. Such a halogen displacement reaction can be effected via organometallic cross coupling conditions such as Suzuki conditions using palladium catalysts such as for example tetrakis(triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 110°C either under microwave conditions or not.

Suitable compounds of formula (XXXVIII) may be either commercially acquired or obtained through various reactions including selective protection and deprotection steps known to the person skilled in the art. For the compounds of formula (XXXVIII) a borylation step might be required.

In the above reaction scheme, the alkylation of compound of formula (XXXIX) can be accomplished using (2-bromoethoxy)(tert-butyl)dimethylsilane in a solvent such as *N,N-*dimethylformamide and a base such as sodium hydride at 0°C or at room temperature. Deprotection of X1 in the compound of formula (XL) can be accomplished using hydrogen gas in the presence of Pd/C in a solvent such as EtOH at room temperature.

The alkylation between a compound of formula (XLI) with a compound of formula (XIX) can be accomplished in a solvent such as *N*,*N*-dimethylformamide or acetonitrile and a base such as cesium carbonate or potassium carbonate at an elevated temperature such as 120°C. Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

Deprotection of X3-OPg4 in the compound of formula (XLII) can be done using TBAF in a solvent such as THF at room temperature.

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme K** below wherein the fused pyrazolo structure of formula (II) is converted to a protected compound of formula (III). The NH of the fused pyrazolo structure can be protected to a compound of formula (XIV). This compound of formula (XIV) can be converted to a boronic acid (or boronate ester) of formula (XV). The compound of formula (XV) can be coupled via organometallic cross coupling such as Suzuki coupling with a (hetero-)aryl of formula (XLIII) or of formula (XXVI) to form a compound of formula (XLIV) or a compound of formula (XLIVa). Deprotection of X1 results in a compound of formula (XLV) or a compound of formula (XLVa). The compound of formula (XLV) or the compound of formula (XLVa) can be alkylated with an intermediate of formula (XIX) containing a carbamate resulting in a compound of formula (XLVI) or in a compound of formula (XXI). Deprotection of X3-OPg4 in the compound of formula (XLVI) results in the compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

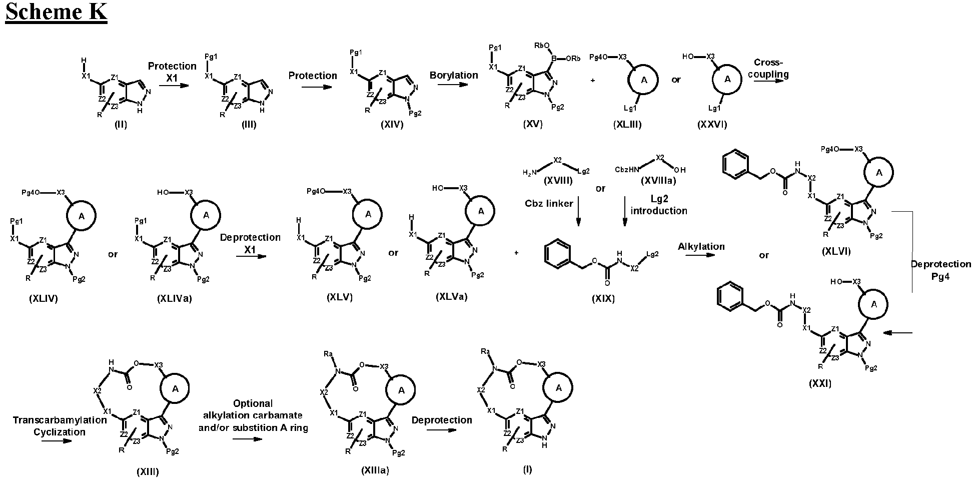

In the above reaction **Scheme K,** fused pyrazolo structure borylation of a compound of formula (XIV) to a compound of formula (XV) can be accomplished using an iridium catalyst and bis(pinacolato)diboron in a solvent such as TBME.

In the above reaction between compound of formula (XV) and compound of formula (XLIII) or compound of formula (XXVI), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine)palladium(0) combined with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

Deprotection of X1 in the compound of formula (XLIV) or in the compound of formula (XLIVa) can be achieved using a reagent such as TBAF in a solvent such as THF at room temperature.

In the above reaction scheme, the alkylation between a compound of formula (XLV) or a compound of formula (XLVa) with a compound of formula (XIX) can be accomplished in a solvent such as *N*,*N-*dimethylformamide or acetonitrile and a base such as cesium carbonate or potassium carbonate at an elevated temperature such as 50°C. Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

Deprotection of X3-OPg4 in the compound of formula (XLVI) can be achieved using conditions such as potassium carbonate in a solvent such as MeOH at room temperature.

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme L** below wherein the fused pyrazolo structure of formula (II) is converted to a protected compound of formula (III). The NH of the fused pyrazolo structure can be protected to a compound of formula (XIV). This compound of formula (XIV) can be converted to a boronic acid (or boronate ester) of formula (XV). The compound of formula (XV) can be coupled in a cross coupling reaction such as a Suzuki coupling with a (hetero-)aryl of formula (XLVIII) to form a compound of formula (XLIX). Introduction of a leaving group on X2 results in a compound of formula (L). Deprotection of X1 results in a compound of formula (LI). The compound of formula (LI) can then be cyclized by a nucleophilic substitution to form a compound of formula (XIII). Final deprotection of the nitrogen of the fused pyrazolo structure, either or not after alkylation of the carbamate and/or substitution of the A ring results in the compound of formula (I).

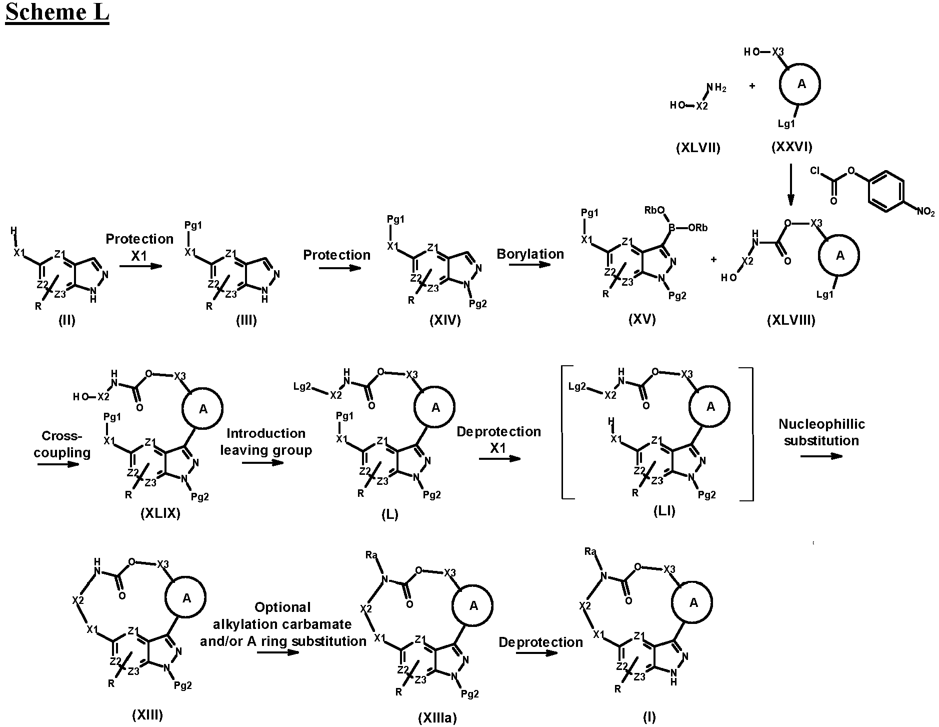

In the above reaction **Scheme L,** fused pyrazolo structure borylation a compound of formula (XIV) to a compound of formula (XV) can be accomplished using an iridium catalyst and bis(pinacolato)diboron in a solvent such as TBME.

In the above reaction between compound of formula (XV) and compound of formula (XLVIII), the leaving groups Lg₁ is advantageously a halogen atom such as chlorine, bromine or iodine. Such a halogen displacement reaction can be effected via organometallic cross coupling conditions such as Suzuki conditions using palladium catalysts such as for example tetrakis(triphenylphosphine)palladium(0) combined or not with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

The compound of formula (XLVIII) can be made from a reaction of an alcohol of formula (XXVI), a chloroformate such as nitro-phenyl chloroformate and an amine of formula (XLVII). Introduction of a leaving group on X2 such as a mesylate on the compound of formula (XLIX) can be achieved using mesyl chloride in the presence of a base such as trimethylamine in a solvent such as DCM at room temperature and results in a compound of formula (L).

Deprotection of X1 to the compound of formula (LI) can be achieved using a reagent such as TBAF in a solvent such as THF at room temperature.

The macrocyclization of the compound of formula (LI) by nucleophilic substitution can be done on using cesium carbonate in a solvent such as *N*,*N*-dimethylformamide at an elevated temperature such as 80°C and results in a compound of formula (XIII).

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme M** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI) which is then alkylated with an intermediate of formula (XIX) containing a leaving group resulting in a compound of formula (XXII). The compound of formula (XIX) can commercially be acquired or being prepared from a compound of formula (XVIII) through a reaction with CbzCl or through the introduction of a leaving group Lg2 on the compound of formula (XVIIIa). The compound of formula (XXII) can be coupled in a copper mediated coupling with a protected alkyne (LII) to form a compound of formula (LIII).

Deprotection of the alkyne leads to a compound of formula (LIV). From the alkyne the (hetero-)aromatic ring can be formed resulting in a compound of formula (XLII). Deprotection of X3-OPg4 results in a compound of formula (XXI). The compound of formula (XXI) can then be cyclized by a transcarbamylation reaction to form a compound of formula (XIII). Final deprotection of the nitrogen of the fused pyrazolo structure, either or not after alkylation of the carbamate and/or substitution of the A ring results in the compound of formula (I).

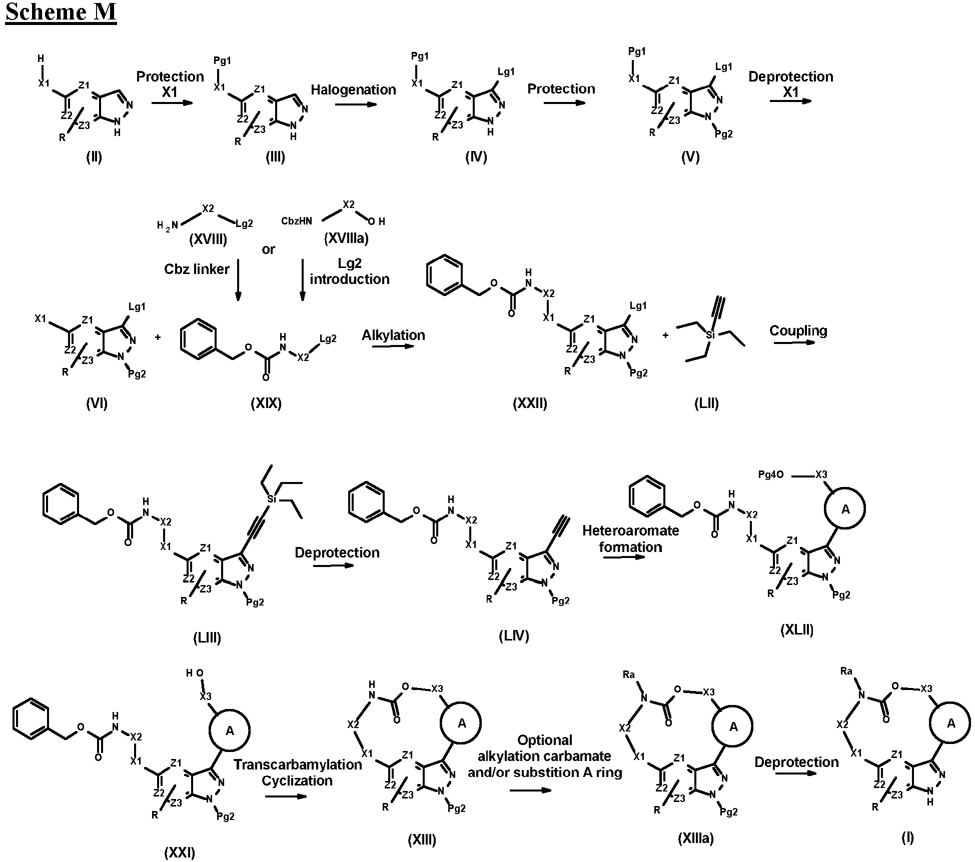

In the above **Scheme M,** A is a 5-membered aromatic cyclic group as define in formula (a) with A4 is a carbon atom and A5 represent a carbon atom optionally substituted.

In the above reaction scheme, the alkylation between a compound of formula (VI) with a compound of formula (XIX) can be accomplished in a solvent such as*N*,*N*-dimethylformamide or acetonitrile and a base such as cesium carbonate or potassium carbonate at an elevated temperature such as 120°C.

Suitable compounds of formula (XIX) may be either commercially acquired or obtained through reaction with CbzCl and sodium hydroxide of a compound of formula (XVIII) in water as a solvent. Alternatively, the compound of formula (XIX) can be made by introduction of Lg2 on the compound of the formula (XVIIIa).

In the above reaction between compound of formula (XXII) and compound of formula (LII), the leaving groups Lg₁ is advantageously a halogen atom such as chlorine, bromine or iodine. Such a halogen displacement reaction can be effected under conditions using palladium catalysts such as for example tetrakis(triphenylphosphine)palladium(0) combined or not with CuI in the presence of triethylamine in a solvent such as for example THF at an elevated temperature such as for example 80°C.

Alkyne deprotection can be achieved using TBAF in a solvent such as THF at room temperature giving a compound of formula (LIV).

Heteroaromatic ring formation to a compound of formula (XLII) can be effected through reaction with a reagent such as tert-butyl-(3-nitropropoxy)-diphenyl-silane in the presence of PhNCO and trimethylamine in a solvent such as THF at an elevated temperature such as 80°C. Deprotection of X3-OPg4 in compound (XLII) can be done using TBAF in a solvent such as THF at room temperature giving a compound of formula (XXI).

The transcarbamylation of the compound of formula (XXI) to the macrocycle of formula (XIII) can be done using potassium carbonate or cesium carbonate or potassium hydroxyde in a solvent such as acetonitrile at a temperature ranging from RT to refluxing solvent, or using sodium hydride in a dry solvent such toluene at a temperature ranging from 0°C to refluxing solvent, either under microwave conditions or not.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme N** below wherein the fused pyrazolo structure of formula (II) is converted to a protected compound of formula (III). This compound of formula (III) can be converted to a compound of formula (IV) containing a leaving group on the fused pyrazolo structure and then into a nitrogen protected compound of formula (V). The compound of formula (V) can be converted into a selectively protected fused pyrazolo structure of formula (VI). The compound of formula (VI) is alkylated with a compound of formula (VIIIa) to form a compound of formula (LV). Deprotection of X2-N(Ra)Pg3 results in a compound of formula (LVI). The compound of formula (LVI) can be coupled to the (hetero-)aromatic compound of formula (LVII) through a reaction with CDI. The compound of formula (LVIII) can then be cyclized by a CH activation reaction to form a compound of formula (XIII). Final deprotection of the nitrogen of the fused pyrazolo structure, either or not after alkylation of the carbamate and/or substitution of the A ring results in the compound of formula (I).

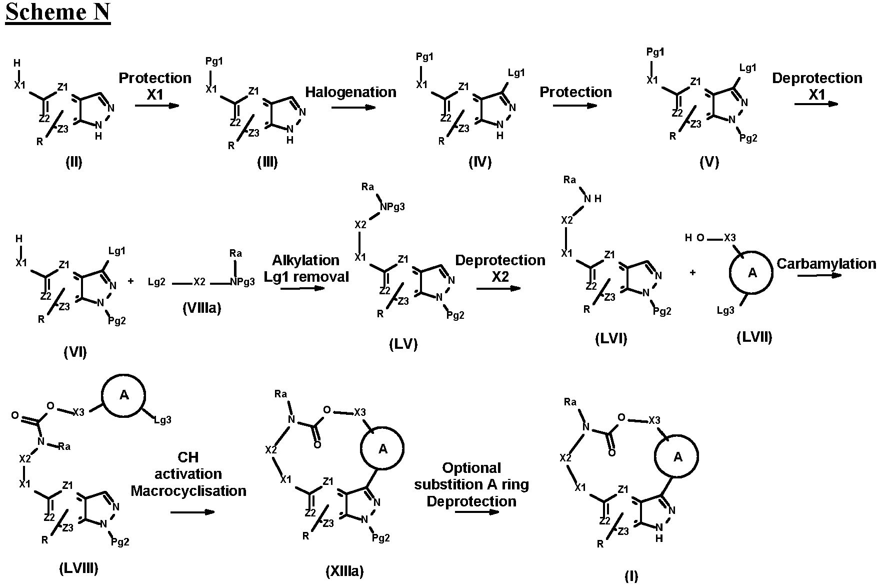

In the above reaction **Scheme N,** the alkylation between a compound of formula (VI) with a compound of formula (VIIIa) can be accomplished in a solvent such as*N*,*N*-dimethylformamide or acetonitrile and a base such as cesium carbonate or potassium carbonate at room temperature or at an elevated temperature. Suitable compounds of formula (VIIIa) may be either commercially acquired or obtained through various selective protection and deprotection steps known to the person skilled in the art.

Deprotection of the compound of formula (LV) can be affected using palladium over carbon on charcoal and hydrogen gas at room temperature in a solvent such as MeOH.

Coupling of the (hetero-)aromatic part on formula (LVI) can be achieved at room temperature using 1,1'-carbonyldiimidazole and a base such as cesium carbonate in a solvent such as *N,N-*dimethylacetamide
Ring closure through CH activation of the compound of formula (LVIII) to the macrocycle of formula (XIII) can be achieved using cataCXium, palladium acetate and potassium acetate in dry toluene under microwave conditions at an elevated temperature such as 150°C.

Final deprotection of the nitrogen of the fused pyrazolo structure under acidic conditions, either or not after alkylation of the carbamate and/or substitution of the A ring yields the final compound of formula **(I).**

Alternatively, the compounds of formula (I) can be prepared as shown in general **Scheme O** below wherein the fused pyrazolo structure of formula (II) is converted to a protected compound of formula (III). The NH of the fused pyrazolo structure can be protected to a compound of formula (XIV). This compound of formula (XIV) can be converted to a boronic acid (or boronate ester) of formula (XV). The compound of formula (XV) can be coupled via organometallic cross coupling such as Suzuki coupling with a (hetero-)aryl of formula (XLIII) or of formula (XXVI) to form a compound of formula (XLIV) or a compound of formula (XLIVa), which can then be alkylated with a compound of formula (XIX) and cyclized by a transcarbamylation reaction in a one-pot reaction to form a compound of formula (XIII). Alternatively the compound of formula (XLIVa) can be first deprotected to a compound of formula (XLIVb) before the one-pot alkylation and cyclisation. Final deprotection of the nitrogen of the fused pyrazolo structure, either or not after alkylation of the carbamate and/or substitution of the A ring results in the compound of formula (I).

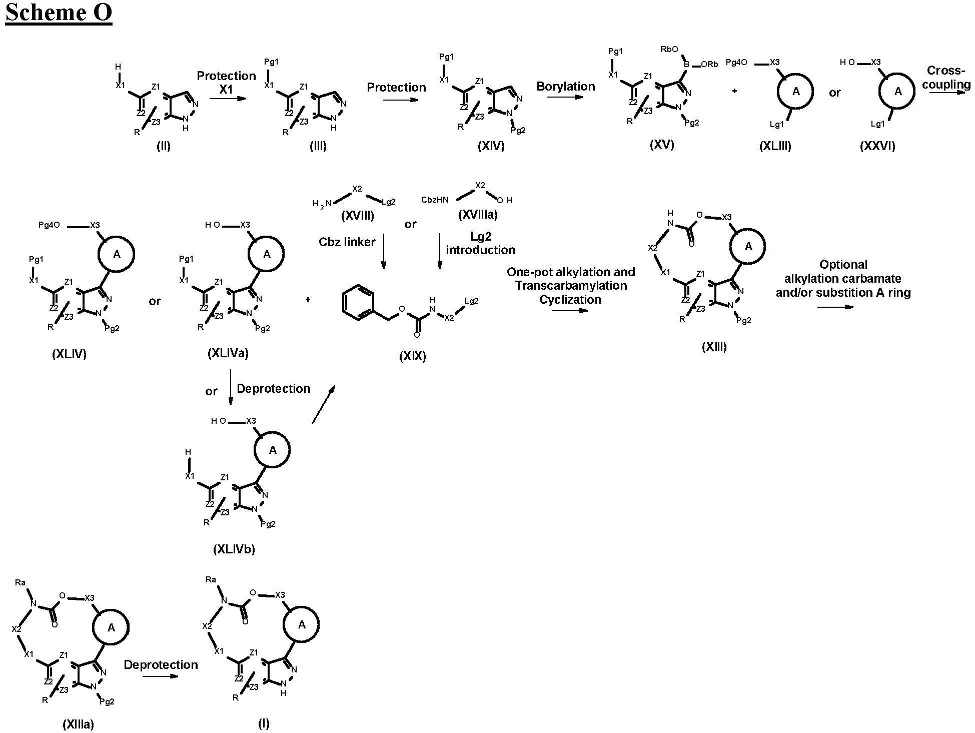

In the above reaction **Scheme O,** fused pyrazolo structure borylation of a compound of formula (XIV) to a compound of formula (XV) can be accomplished using an iridium catalyst and bis(pinacolato)diboron in a solvent such as TBME.

In the above reaction between compound of formula (XV) and compound of formula (XLIII) or compound of formula (XXVI), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine)palladium(0) combined with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

The possibly deprotection of X1 can be done using TBAF in a solvent such as THF at a temperature such as room temperature.

The possibly one-pot alkylation with a compound of formula (XIX) and transcarbamylation to the macrocycle of formula (XIII) can be done using cesium carbonate in a solvent such as acetonitrile at a temperature ranging from RT to 80°C.

Final deprotection of the nitrogen of the fused pyrazolo structure under acidic conditions, either or not after alkylation of the carbamate and/or substitution of the A ring yields the final compound of formula (I).

Alternatively, the compounds of formula (I) can be prepared as shown in general **Scheme P** below wherein the fused pyrazolo structure of formula (II) is converted to a protected compound of formula (III). The NH of the fused pyrazolo structure can be protected to a compound of formula (XIV). This compound of formula (XIV) can be converted to a boronic acid (or boronate ester) of formula (XV). The compound of formula (XV) can be coupled via organometallic cross coupling such as Suzuki coupling with a (hetero-)aryl of formula (XXVI) to form a compound of formula (XLIVa), which can then be alkylated with a compound of formula (XLVI) and cyclized by a carbamylation reaction to form a compound of formula (XLVIII). Final deprotection of the nitrogen of the fused pyrazolo structure, either or not after alkylation of the carbamate and/or substitution of the A ring results in the compound of formula (I).

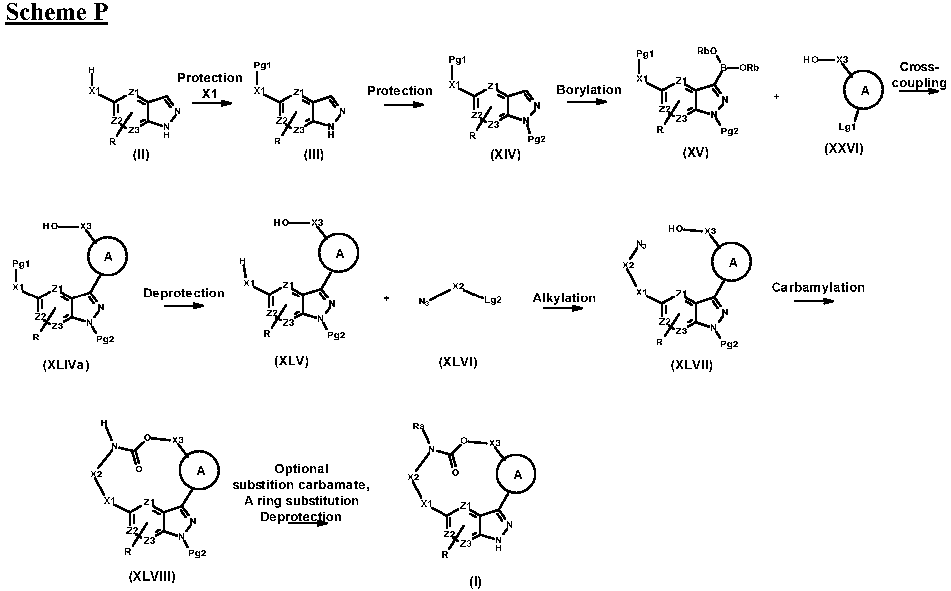

In the above reaction **Scheme P,** fused pyrazolo structure borylation of a compound of formula (XIV) to a compound of formula (XV) can be accomplished using an iridium catalyst and bis(pinacolato)diboron in a solvent such as TBME.

In the above reaction between compound of formula (XV) and compound of formula (XXVI), the leaving groups Lg₁ is advantageously a halogen atom such as chlorine, bromine or iodine. Such a halogen displacement reaction can be affected under cross-coupling conditions such as Suzuki conditions using palladium catalysts such as for example tetrakis(triphenylphosphine)palladium(0) combined with 2-dicyclohexylphosphino-2',4',6'-triisopropylbiphenyl (Xphos) in the presence of potassium phosphate tribasic in a solvent mixture such as for example 1,4-dioxane/water at an elevated temperature such as for example 90°C either under microwave conditions or not.

The alkylation of the compound of formula (XLV) with a compound of formula (XLVI) can be done using cesium carbonate in a solvent such as acetonitrile at a temperature ranging from RT to 80°C.

Carbamylation of the compound of formula (XLVIII) can be achieved using a reagent such as CDI, COCl₂, CO₂ or CO.

Final deprotection of the nitrogen of the fused pyrazolo structure under acidic conditions, either or not after alkylation of the carbamate and/or substitution of the A ring yields the final compound of formula (I).

### EXAMPLES

IUPAC names of compounds of the invention were generated using the following software:
Product version: MarvinSketch 18.3.0
Build Date: 2018-01-26
Internal Build id: 18.3.0-7913
Operating System: amd64 Windows 10.10.0
Character encoding: windows-1252
Java: Jeroen Frijters Java 1.8.0
Memory: 43,8M total, 10,0M free
Environment: Application
.NET Version: v2.0.50727
IKVM Version : 8.10.1.2
JChem .NET API Assembly Version : 18.3.07913
JChem .NET API File Version : 18.3.0.7913
Marvin .NET Version : 18.3.0.137
Process type : x64
http://www.chemaxon.com

In case of a discrepancy between the drawn chemical structures and the corresponding chemical names, the drawn chemical structures will be considered as true structures.

To prepare the compounds described in the examples, the following experimental protocols were followed unless otherwise indicated.

Unless otherwise stated, reaction mixtures were stirred magnetically at room temperature. When organic solutions were "dried", they were generally dried over a drying agent such as sodium sulfate or magnesium sulfate. When mixtures, solutions and extracts were "concentrated", they were typically concentrated on a rotary evaporator under reduced pressure.

All intermediates and final exemplified compounds were analyzed by high-performance liquid chromatography (HPLC) following one of the described methods below.

### LCMS method A

Analyses were carried out on a Thermo Scientific Accucore C18 (50 mm long x 2.1 mm I.D., 2.6 µm) at 35 °C, with a flow rate of 1.50 mL/min. A gradient elution was performed from 95% (Water + 0.1% Formic acid) / 5% Acetonitrile to 5% (Water + 0.1% Formic acid) / 95% Acetonitrile in 1.30 minutes; the resulting composition was held for 0.5 min; then the final mobile phase composition; from 5% (Water + 0.1% Formic acid) / 95% Acetonitrile to 90% (Water + 0.1% Formic acid) / 10% Acetonitrile in 0.10 minutes. The injection volume was 1 µL. MS acquisition range and UV detector were set to 100-1000 m/z and 190-400 nm respectively.

### LCMS method B

Analyses were carried out on a Phenomenex Kinetex 00B-4475-AN C18 column (50 mm long x 2.1 mm I.D.; 1.7 µm particles) at 60 °C, with a flow rate of 1.5 mL/min. A gradient elution was performed from 90% (Water + 0.1% Formic acid) / 10% Acetonitrile to 10% (Water + 0.1% Formic acid) / 90% Acetonitrile in 1.50 minutes; the resulting composition was held for 0.40 min; then the final mobile phase composition; from 10% (Water + 0.1% Formic acid) / 90% Acetonitrile to 90% (Water + 0.1% Formic acid) / 10% Acetonitrile in 0.10 minutes. The injection volume was 2 µL with Agilent autosampler injector or 5 µL with Gerstel MPS injector. MS acquisition range and DAD detector were set to 100-800 m/z and 190-400 nm respectively.

### LCMS method C

Analyses were carried out on an YMC pack ODS-AQ C18 column (50 mm long x 4.6 mm I.D..; 3 µm particle size) at 35 °C, with a flow rate of 2.6 mL/min. A gradient elution was performed from 95% (Water + 0.1% Formic acid)/5% Acetonitrile to 5% (Water + 0.1% Formic acid)/95% Acetonitrile in 4.8 min; the resulting composition was held for 1.0 min; from 5% (Water + 0.1% formic acid)/95% Acetonitrile to 95% (Water + 0.1% formic acid)/5% Acetonitrile in 0.2 min. The standard injection volume was 2 µL. Acquisition ranges were set to 190-400 nm for the UV-PDA detector and 100-1400 m/z for the TOF-LCMS detector. Total run time: 6.2 minutes.

### LCMS method D

Analyses were carried out on a Phenomenex Kinetex C18 column (50 mm long x 2.1 mm I.D..; 2.6 µm particle size) at 35 °C, with a flow rate of 0.7 mL/min. A gradient elution was performed from 95% (Water + 50mM Ammonium Acetate)/5% Acetonitrile to 5% (Water + 50mM Ammonium Acetate)/95% Acetonitrile in 4.8 min; the resulting composition was held for 1.0 min; from 5% (Water + 50mM Ammonium Acetate)/95% Acetonitrile to 95% (Water + 50mM Ammonium Acetate)/5% Acetonitrile in 0.2 min. The standard injection volume was 2 µL. Acquisition ranges were set to 190-400 nm for the UV-PDA detector and 100-1400 m/z for the MS detector. Total run time: 6.2 minutes.

### LCMS method E

Analyses were carried out on an YMC pack ODS-AQ C18 column (50 mm long x 4.6 mm I.D..; 3 µm particle size) at 35 °C, with a flow rate of 2.6 mL/min. A gradient elution was performed from 95% (Water + 0.1% Formic acid)/5% Acetonitrile to 5% (Water + 0.1% Formic acid)/95% Acetonitrile in 4.8 min; the resulting composition was held for 1.0 min; from 5% (Water + 0.1% formic acid)/95% Acetonitrile to 95% (Water + 0.1% formic acid)/5% Acetonitrile in 0.2 min. The standard injection volume was 2 µL. Acquisition ranges were set to 190-400 nm for the UV-PDA detector and 100-1400 m/z for the MS detector.

### LCMS method F

Analytical HPLC was conducted on a X-Select CSH C18 XP column (2.5 µm 30 x 4.6 mm id) eluting with 0.1% formic acid in water (solvent A) and 0.1% formic acid in acetonitrile (solvent B), using the following elution gradient 0-3 minutes: 5% to 100% B, 3-4 minutes 100% B, at a flow rate of 1.8 mL/minute at 40°C. The mass spectra (MS) were recorded on a Waters ZQ mass spectrometer (scan 200-900 uma) using electrospray positive ionisation [ES+ to give [M+H]⁺ molecular ions] or electrospray negative ionisation [ES- to give [M-H]⁻ molecular ions] modes with a 20 V cone voltage.

### LCMS method G

Analytical HPLC was conducted on a X-Select CSH C18 XP column (2.5 µm 30 x 4.6 mm id) eluting with (NH₄)₂CO₃ aq. 2g/L in water (solvent A) and acetonitrile (solvent B), using the following elution gradient 0-3 minutes: 5% to 100% B, 3-4 minutes 100% B, at a flow rate of 1.8 mL/minute at 40°C. The mass spectra (MS) were recorded on a Waters ZQ mass spectrometer (scan 200-900 uma) using electrospray positive ionisation [ES+ to give [M+H]⁺ molecular ions] or electrospray negative ionisation [ES- to give [M-H]⁻ molecular ions] modes with a 20 V cone voltage.

### LCMS method H

Analytical HPLC was conducted on a X-Select CSH C18 XP column (2.5 µm 30 x 4.6 mm id) eluting with 0.1% formic acid in water (solvent A) and 0.1% formic acid in acetonitrile (solvent B), using the following elution gradient 0-4 minutes: 0% to 50% B at a flow rate of 1.8 mL/minute at 40°C. The mass spectra (MS) were recorded on a Waters ZQ mass spectrometer (scan 200-900 uma) using electrospray positive ionisation [ES+ to give [M+H]⁺ molecular ions] or electrospray negative ionisation [ES- to give [M-H]⁻ molecular ions] modes with a 20 V cone voltage.

### LCMS method I

Analytical HPLC was conducted on a X-Select CSH C18 XP column (2.5 µm 30 x 4.6 mm id) eluting with 0.1% formic acid in water (solvent A) and 0.1% formic acid in acetonitrile (solvent B), using the following elution gradient 0-4 minutes: 40% to 100% B, 4-5min: 100%B at a flow rate of 1.8 mL/minute at 40°C. The mass spectra (MS) were recorded on a Waters ZQ mass spectrometer (scan 200-900 uma) using electrospray positive ionisation [ES+ to give [M+H]⁺ molecular ions] or electrospray negative ionisation [ES- to give [M-H]⁻ molecular ions] modes with a 20 V cone voltage.

### LCMS method J

Analytical HPLC was conducted on a X-Select CSH C18 XP column (2.5 µm 30 x 4.6 mm id) eluting with 0.1% formic acid in water (solvent A) and 0.1% formic acid in acetonitrile (solvent B), using the following elution gradient 0-6 minutes: 5% to 100% B, 6-7min: 100%B at a flow rate of 1.8 mL/minute at 40°C. The mass spectra (MS) were recorded on a Waters ZQ mass spectrometer (scan 200-900 uma) using electrospray positive ionisation [ES+ to give [M+H]⁺ molecular ions] or electrospray negative ionisation [ES- to give [M-H]⁻ molecular ions] modes with a 20 V cone voltage.

Chiral analytical SFC was conducted on a Whelk O1 (R,R) column (1.8µm 100 x 4.6 mmid) eluting with CO2/methanol (70/30)at a flow rate of 2.5mL/minute at 35°C.

All final exemplified compounds were analysed by proton NMR.

¹H NMR spectra were recorded in either CDCl₃, d6-DMSO or CD₃OD on a Bruker Avance 400MHz or were recorded on a Bruker Ultrashield AV300 MHz spectrometer, with a Bruker 5mm BBI 1H/D-BB Z-GRD probe, using a BACS-60 sample changer, and registered with Bruker Topspin 2.1 software. Chemical shifts are reported in parts per million (ppm) relative to the residual protiated solvent (7.26 ppm for CDCl₃, 2.50 ppm for d6-DMSO and 3.31 ppm for CD₃OD). For ¹H NMR spectra, multiplicities, coupling constants in hertz and numbers of protons are indicated parenthetically. Abbreviations for NMR data are as follows: s = singlet, d = doublet, t = triplet, q = quadruplet, m = multiplet, br s = broad singlet.

Alternatively, the ¹H-NMR measurements were performed on Bruker Avance III 500 MHz spectrometer, using DMSO-d6 (hexadeutero-dimethylsulfoxide) or CDCl₃ (deuterochloroform) as solvent. ¹H-NMR data is in the form of delta values, given in part per million (ppm), using the residual peak of the solvent (2.50 ppm for DMSO-d6 and 7.26 ppm for CDCl₃) as internal standard. Splitting patterns are designated as: s (singlet), 2s (2xsinglet), d (doublet), 2d (2xdoublet), t (triplet), 2t (2xtriplet), q (quartet), 2q (2xquartet), quint (quintet), sept (septet), m (multiplet), 2m (2xmultiplet), brs (broad singlet), brd (broad doublet), brt (broad triplet), brq (broad quartet), brm (broad multiplet), vbrs (very broad singlet), dd (doublet of doublets), td (triplet of doublets), dt (doublet of triplets), dq (doublet of quartet), ddd (doublet of doublet of doublets), dm (doublet of multiplets), tm (triplet of multiplets), qm (quartet of multiplets).

### Abbreviations:

The following abbreviations are employed herein:
Ph = phenyl
Ac = acetate
Bn = benzyl
t-Bu = tert-butyl
n-Bu = linear butyl
Me = methyl
Et = ethyl
Pr = propyl
iPr = isopropyl
Bu = butyl
TMS = trimethylsilyl
TBS = tert-butyldimethylsilyl
TFA = trifluoroacetic acid
i-Pr₂NEt or DIPEA = *N*,*N*-diisopropylethylamine
TEA = triethylamine
DMAP = 4-dimethylaminopyridine
Pd/C = palladium on carbon
KOH = potassium hydroxide
NaOH = sodium hydroxide
LiOH = lithium hydroxide
Ar = argon
N₂ = nitrogen
H₂ = hydrogen
LAH = lithium Aluminium Hydride
Boc = tert-butoxycarbonyl
Cbz = carboxybenzyl
LDA = lithium diisopropylamide
NBS = *N*-bromosuccinimide
NIS = *N*-iodosuccinimide
ACN = acetonitrile
PTSA = p-toluenesulfonic acid
THF = tetrahydrofuran
DCM = dichloromethane
DMF = *N,N*-dimethylformamide
AA = acetic acid
TBME = methyl tert-butyl ether
Hept = heptane
EtOAc = ethyl acetate
DHP = 3,4-Dihydro-2H-pyran
THP = Tetrahydropyran
TBAF = tetrabutylammonium fluoride
cataCXium = di(1-adamantyl)-n-butylphosphine
XPhos = 2-Dicyclohexylphosphino-2',4',6'-triisopropylbiphenyl
dppf = 1,1'-Bis(diphenylphosphino)ferrocene
wt% = weight %
e.e. = enantiomeric excess
min = minute(s)
h or hr = hour(s)
L = liter(s)
mL = milliliter(s)
µL = microliter(s)
g = gram(s)
mg = milligram(s)
mol = moles
mmol = millimole(s)
RT = room temperature
t_{R} = retention time
sat = saturated
aq. = aqueous
TLC = thin layer chromatography
HPLC = high performance liquid chromatography
LC/MS = high performance liquid chromatography/mass spectrometry
MS or Mass Spec = mass spectrometry
NMR = nuclear magnetic resonance
ppm = parts per million

### Example 1: 8,14-dioxa-4,10,19,20-tetraazatetracyclo[13.5.2.1^{2,6}.0^{18,21}]tricosa-1(20),2,4, 6(23),15,17,21-heptaen-9-one

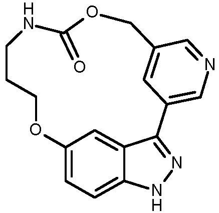

Example 1 is prepared according to the synthesis route described in general Scheme A.

### Preparation of intermediate 1 : 5-((tert-butyldimethylsilyl)oxy)-1H-indazole

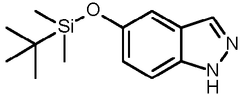

1H-indazol-5-ol (19 g, 141.643 mmol) was dissolved in 425 mL of DCM, then imidazole (11.572 g, 169.972 mmol) and tert-butylchlorodimethylsilane (23.485 g, 155.807 mmol) were added and the mixture was stirred at RT for 16 hours. A saturated NaHCO₃ solution was added and the reaction mixture was extracted with DCM (2x). The combined organic layers were dried over MgSO₄, filtered and the solvent was removed under reduced pressure. The crude product was purified by flash chromatography on silica gel using Hept/EtOAc (100:0 to 70:30). The desired fractions were combined and concentrated under reduced pressure yielding 5-((tert-butyldimethylsilyl)oxy)-1H-indazole 1 as a salmon solid.
LCMS method A: [M+H]⁺ = 249.0, t_{R} = 0.997 min

### Preparation of intermediate 2 : 5-((tert-butyldimethylsilyl)oxy)-3-iodo-1H-indazole

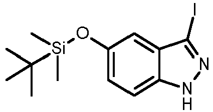

5-((tert-butyldimethylsilyl)oxy)-3-iodo-1H-indazole 1 (20 g, 80.515 mmol) was dissolved in 240 mL of DCM, N-iodosuccinimide (19.021 g, 84.541 mmol) was added and the mixture was stirred at RT for 16 hours. The reaction mixture was diluted with DCM and a saturated NaHCO₃ solution was added. The two layers were separated and the water layer was extracted with DCM (2x). The combined organic layers were dried over MgSO₄, filtered and the solvent was removed under reduced pressure affording the crude product which was purified by flash chromatography on silica gel using Hept/EtOAc (100:0 to 80:20) as eluents. The desired fractions were combined and the solvent was removed under reduced pressure yielding 5-((tert-butyldimethylsilyl)oxy)-3-iodo-1H-indazole 2 as a light brown solid.
LCMS method A: [M+H]⁺ = 374.9, t_{R} = 1.156 min

### Preparation of intermediate 3 : 5-((tert-butyldimethylsilyl)oxy)-3-iodo-1-(tetrahydro-2H-pyran-2-yl)-1H-indazole

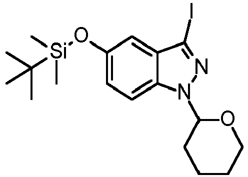

To a solution of 5-((tert-butyldimethylsilyl)oxy)-3-iodo-1H-indazole **2** (27.960 g, 74.699 mmol) in 224 mL of DCM, 4-methylbenzenesulfonic acid monohydrate (1.421 g, 7.470 mmol) and 3,4-dihydro-2H-pyran (20.490 mL, 224.097 mmol) were added. The reaction mixture was stirred at RT for 16 hours. The mixture was diluted with DCM and a saturated NaHCO₃ solution was added. The two layers were separated and the water layer was extracted with DCM (2x). The combined organic layers were dried over MgSO₄, filtered and the solvent was removed under reduced pressure. The concentrated was purified by flash chromatography (silica; Heptane/EtOAc 100:0 to 95:5). The desired fractions were combined and the solvent was removed under reduced pressure affording 5-((tert-butyldimethylsilyl)oxy)-3-iodo-1-(tetrahydro-2H-pyran-2-yl)-1H-indazole 3 as a light orange oil.
LCMS method A: [M+H]⁺ = 458.9, t_{R} = 1.377 min

### Preparation of intermediate 4 : 3-iodo-1-(tetrahydro-pyran-2-yl)-1H-indazol-5-ol

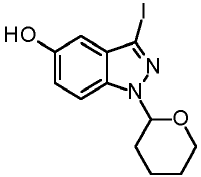

5-((tert-butyldimethylsilyl)oxy)-3-iodo-1-(tetrahydro-2H-pyran-2-yl)-1H-indazole **3** (10.000 g, 21.814 mmol) was dissolved in 62 mL of THF. TBAF [1M] in THF (32.8 mL, 32.800 mmol) was added at 0 °C. The reaction was stirred at RT for 16 h. A saturated NaHCO₃ solution was added and the two layers were separated. The water layer was extracted with DCM (2x). The combined organic layers were dried over MgSO₄, filtered and the solvent was removed under reduced pressure. The crude was purified by flash chromatography (silica; Heptane/EtOAc 100:0 to 60:40). The fractions containing the desired product were combined and the solvent was evaporated under reduced pressure to yield 3-Iodo-1-(tetrahydro-pyran-2-yl)-1H-indazol-5-ol **4** as a creamy solid.
LCMS method B: [M+H]⁺ = 345.0, t_{R} = 0.767 min

### Preparation of intermediate 5: 3-(dibenzylamino)propan-1-ol

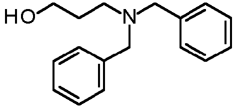

To a solution of 3-aminopropan-1-ol (5 g, 66.569 mmol) in 200 mL of EtOH, potassium carbonate (18.861 g, 136.466 mmol) and benzyl bromide (17.395 mL, 146.452 mmol) were carefully added and the resulting mixture was stirred at 70°C under reflux for 4 hours. The mixture was filtered and the filtrate was washed with water. The aqueous layer was extracted with EtOAc (2x) and the combined organic layers were dried over MgSO4, filtered and concentrated under reduced pressure giving the crude product which was purified by flash chromatography on silicagel using Hept/EtOAc (100:0 to 80:20) as eluents. The desired fractions were combined and the solvent was removed under reduced pressure yielding 3-(dibenzylamino)propan-1-ol **5** as a yellowish oil.
LCMS method B: no m/z detected, t_{R} = 0.248 min

### Preparation of intermediate 6: 3-(dibenzylamino)propyl methane sulfonate

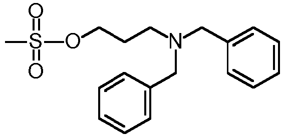

3-(dibenzylamino)propan-1-ol 5 (5.000 g,19.580 mmol) was dissolved in 60 mL of DCM and triethylamine (8.187 mL, 58.740 mmol) was added. The mixture was cooled to 0°C and methane sulfonyl chloride (1.970 mL, 25.454 mmol) was added. The mixture was stirred at RT for 16 hours. DCM and a saturated solution of NaHCO₃ were added. The two layers were separated and the mixture was extracted with DCM (x2). The combined organic layers were dried over MgSO₄, filtered and the solvent under reduced pressure yielding 3-(dibenzylamino)propyl methane sulfonate **6** as a yellow oil which was used in the next step without purification.
LCMS method B: no m/z detected, t_{R} = 0.380 min

### Preparation of intermediate 7: N,N-dibenzyl-3-((3-iodo-1-(tetrahydro-2H-pyran-2-yl)-1H-indazol-5-yl)oxy)propan-1-amine

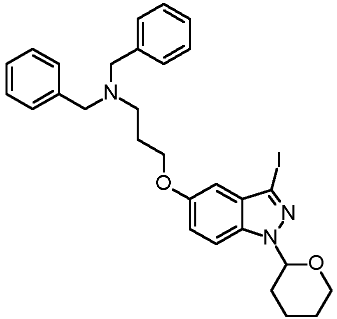

3-(dibenzylamino)propyl methane sulfonate **6** (crude, 6.298 g, 18.888 mmol) dissolved in 10 mL of *N*,*N*-dimethylformamide and was added to a stirred mixture of 3-iodo-1-(tetrahydropyran-2-yl)-1H-indazol-5-ol 4 (5.000 g,14.529 mmol) and cesium carbonate (7.101 g, 21.794 mmol) in 40 mL of *N*,*N-*dimethylformamide. The reaction was stirred at RT for 30 minutes and then heated at 85°C for 2 hours. The mixture was diluted with EtOAc and water was added. The two layers were separated and the water layer was extracted with DCM (x2). The combined organic layers were dried over MgSO₄, filtered and concentrated under reduced pressure. The crude was purified by flash chromatography on silica gel, using Hept/EtOAc, (100:0 to 80:20). The fractions containing the desired compound were combined and the solvent is removed under reduced pressure to yield N,N-dibenzyl-3-((3-iodo-1-(tetrahydro-2H-pyran-2-yl)-1H-indazol-5-yl)oxy)propan-1-amine 7 as a yellowish oil.
LCMS method B: [M+H]⁺ = 582.2, t_{R} = 0.890 min

### Preparation of intermediate 8: (5-(hydroxymethyl)pyridin-3-yl)boronic acid

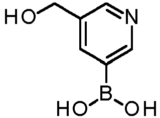

(5-bromopyridin-3-yl)methanol (3.000 g,15.956 mmol), bis(pinacolato)diboron (4.862 g, 19.147 mmol) and potassium acetate (4.698g, 47.868 mmol) were dissolved in 50 mL of 1,4-dioxane. After degassing with N2 for 5 minutes, Pd(dppf)Cl₂ · DCM (1.303 g, 1.596 mmol) was added and the reaction mixture was stirred at 110°C for 4 hours. The mixture was diluted with EtOAc and filtered over a pad of celite. The solvent was evaporated under reduced pressure, yielding (5-(hydroxymethyl)pyridin-3-yl)boronic acid **8** as a dark brown solid. The crude was used in the next step without purification.
LCMS method B: [M+H]⁺ = 154.1, t_{R} = 0.107 min

### Preparation of intermediate 9: {5-[5-(3-dibenzylamino-propoxy)-1-(tetrahydro-pyran-2-yl)-1H-indazol-3-yl]-pyridin-3-yl}-methanol

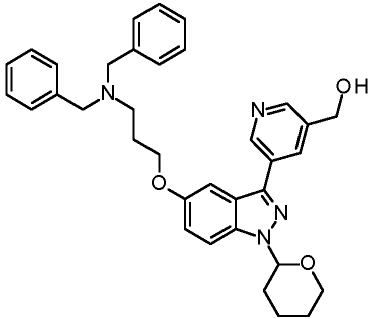

Tetrakis(triphenylphosphine)palladium(0) (1.411 g, 1.221 mmol) and XPhos (0.291 g, 0.611 mmol) were added to a mixture of N,N-dibenzyl-3-((3-iodo-1-(tetrahydro-2H-pyran-2-yl)-1H-indazol-5-yl)oxy)propan-1-amine 7 (7.100 g, 12.210 mmol), (5-(hydroxymethyl)pyridin-3-yl)boronic acid **8** (crude, 8.84 g, 15.873 mmol) and potassium phosphate tribasic (7.77 g, 36.63 mmol) in 122.00 mL of 1,4-dioxane/H₂O (3:1). The mixture was degassed with N₂ for 5 min and stirred at 90°C for 16 hours. The mixture was diluted with EtOAc and water was added. The two layers were separated and the water layer was extracted with DCM (x2). The combined organic layers were dried over MgSO₄, filtered and concentrated under reduced pressure. The crude was purified by flash chromatography on silica gel, using DCM:MeOH (100:0 to 98:2). The desired fractions were combined and the solvent was removed under reduced pressure to obtain {5-[5-(3-dibenzylamino-propoxy)-1-(tetrahydro-pyran-2-yl)-1H-indazol-3-yl]-pyridin-3-yl}-methanol **9** as a yellow oil.
LCMS method B: [M+H]⁺ = 563.3, t_{R} = 0.749 min

### Preparation of intermediate 10: {5-[5-(3-amino-propoxy)-1-(tetrahydro-pyran-2-yl)-1H-indazol-3-yl]-pyridin-3-yl}-methanol

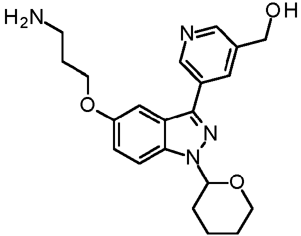

{5-[5-(3-Dibenzylamino-propoxy)-1-(tetrahydro-pyran-2-yl)-1H-indazol-3-yl]-pyridin-3-yl}-methanol 9 (6.000 g, 10.662 mmol) was dissolved in 106 mL of EtOAc, degassed with N2. Pd/C 10% w/w (6.000 g) was added and the reaction mixture was stirred under H2 atmosphere with a balloon at RT for 66 hours. The reaction mixture was filtered over a pad of celite and washed with a mixture of DCM:MeOH:DMA (9:1:1). The filtrate was concentrated under reduced pressure to afford the crude product which was purified by flash chromatography (silica gel, DCM/MeOH/MeOH (NH3) (100:0:0 to 90:9: 1). The desired fractions were combined and the solvent was removed under reduced pressure to yield {5-[5-(3-amino-propoxy)-1-(tetrahydro-pyran-2-yl)-1H-indazol-3-yl]-pyridin-3-yl}-methanol 10 as a cream solid.
LCMS method B: [M+H]⁺ = 383.3, t_{R} = 0.316 min

### Preparation of intermediate 11: 19-(oxan-2-yl)-8,14-dioxa-4,10,19,20-tetraazatetra-cyclo[13.5.2.1^{2,6}.0^{18,21}]tricosa-1(20),2,4,6(23),15,17,21-heptaen-9-one

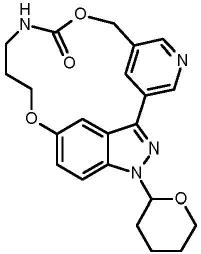

CDI (0.103 g, 0.633 mmol) was added to a solution of (5-(5-(3-aminopropoxy)-1-(tetrahydro-2H-pyran-2-yl)-1H-indazol-3-yl)pyridin-3-yl)methanol **10** (0.220 g, 0.575 mmol) in 133 mL of DMA. The mixture was stirred at RT for 2 hours and at 90°C for 72 hours. The reaction was diluted with EtOAc, cooled to 0°C and a saturated solution of NaHCO3 was added. The two layers were separated and the water layer was extracted with EtOAc (x2). The combined organic layers were dried over MgSO₄, filtered and concentrated under reduced pressure. The product was purified by flash chromatography (silica gel, DCM:MeOH 100:0 to 97.5:2.5). The desired fractions were combined and the solvent was removed under reduced pressure to afford 19-(oxan-2-yl)-8,14-dioxa-4,10,19,20-tetraazatetracyclo[13.5.2.1^{2,6}.0^{18,21}]tricosa-1(20),2,4, 6(23),15,17,21-heptaen-9-one **11** as a colorless foam.
LCMS method B: [M+H]⁺ = 409.1, t_{R} = 0.753 min

### Preparation of Example 1: 8,14-dioxa-4,10,19,20-tetraazatetracyclo[13.5.2.1^{2,6}.0^{18,21}] tricosa-1(20),2,4, 6(23),15,17,21-heptaen-9-one

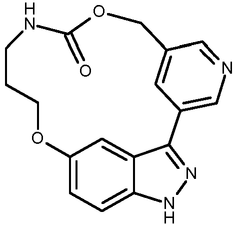

A mixture of 19-(oxan-2-yl)-8,14-dioxa-4,10,19,20-tetraazatetracyclo[13.5.2.1^{2,6}.0^{18,21}]tricosa-1(20),2,4,6(23),15,17,21-heptaen-9-one **11** (0.135 g, 0.331 mmol) in HCl in 1,4-dioxane [4N] (33 mL) was stirred at RT for 2 hours. The mixture was cooled to 0°C, diluted with DCM and quenched carefully with a saturated solution of NaHCO₃. The two layers were separated and the water layer was extracted with DCM (x2). The combined organic layers were dried over MgSO₄, filtered and concentrated under reduced pressure. The product was purified by flash chromatography on silica gel (DCM:MeOH,100:0 to 94:6). The desired fractions were combined and the solvent was removed under reduced pressure yielding 8,14-dioxa-4,10,19,20-tetraazatetracyclo[13.5.2.1^{2,6}.0^{18,21}]tricosa-1(20),2,4,6(23),15,17,21-heptaen-9-one **example 1** as a white solid.
LCMS method C: [M+H]⁺ = 325.05, t_{R} = 2.020 min
LCMS method D: [M+H]⁺ = 325.1, t_{R} = 3.945 min
¹H NMR (300 MHz, DMSO) δ 13.32 (s, 1H), 9.03 (s, 1H), 8.53 (s, 1H), 8.15 (s, 1H), 7.99 (t, J = 5.9 Hz, 1H), 7.54 (d, J = 9.0 Hz, 1H), 7.21 (s, 1H), 7.01 (d, J = 8.9 Hz, 1H), 5.28 (brs, 2H), 4.29 (t, J = 8.3 Hz, 2H), 3.17 (d, J = 4.6 Hz, 2H), 1.97 (brs, 2H) ppm.

### Example 2: 10-methyl-8,14-dioxa-4,10,19,20-tetraazatetracyclo[13.5.2.1^{2,6}.0^{18,21}]tricosa-1(20),2,4,6(23),15,17,21-heptaen-9-one

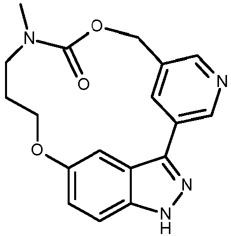

Example 2 is prepared according to the synthesis route described in general Scheme A.

### Preparation of intermediate 12 : 10-methyl-19-(oxan-2-yl)-8,14-dioxa-4,10,19,20-tetraaza-tetracyclo[13.5.2.1^{2,6}.0^{18,2}]tricosa-1(20),2,4,6(23),15,17,21-heptaen-9-one

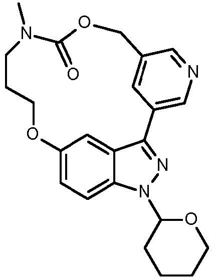

To a solution of intermediate **11** (0.05 g, 0.12 mmol) in 5 mL of dry *N*,*N-*dimethylformamide, under nitrogen atmosphere, at 0°C, sodium hydride 60% in mineral oil (0.007 g, 0.15 mmol) was added. The mixture was stirred at 0°C for 15 minutes, then iodomethane (0.02 mL, 0.33 mmol) was added and the mixture was stirred at RT for 15 minutes. The mixture was cooled to 0°C, diluted with EtOAc and quenched carefully with water. The two layers were separated and the water layer was extracted with EtOAc (x2). The combined organic layers were washed with brine, dried over MgSO₄, filtered and the solvent was removed under reduced pressure. The product was purified by flash chromatography on silica gel (DCM:MeOH 100:0 to 97.5:2.5). The desired fractions were combined and the solvent was removed under reduced pressure affording 10-methyl-19-(oxan-2-yl)-8,14-dioxa-4,10,19,20-tetraazatetracyclo[13.5.2.1^{2,6}. 0^{18,21}]tricosa-1(20),2,4,6(23),15,17,21-heptaen-9-one **12** as a yellow oil.
LCMS method B: [M+H]⁺ = 423.1, t_{R} = 0.897 min

### Preparation of Example 2: 10-methyl-8,14-dioxa-4,10,19,20-tetraazatetracyclo[13.5.2. 1^{2,6}.0^{18,21}]tricosa-1(20),2,4,6(23),15,17,21-heptaen-9-one

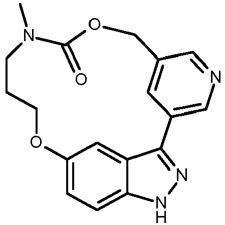

A mixture of 10-methyl-19-(oxan-2-yl)-8,14-dioxa-4,10,19,20-tetraazatetracyclo[13.5.2. 1^{2,6}.0^{18,21}]tricosa-1(20),2,4,6(23),15,17,21-heptaen-9-one **12** (0.042 g, 0.099 mmol) in HCl in 1,4-dioxane [4N] (5.0 mL) was stirred at RT for 2h. The mixture was cooled at 0°C, diluted with DCM and quenched carefully with a saturated solution of NaHCO₃. The two layers were separated and the water layer was extracted with DCM (x2). The combined organic layers were dried over MgSO₄, filtered and the solvent was removed under reduced pressure. The product was purified by flash chromatography on silica gel (DCM:MeOH,100:0 to 94:6). The desired fractions were combined and the solvent was removed under reduced pressure to afford 10-methyl-8,14-dioxa-4,10,19,20-tetraazatetracyclo[13.5.2.1^{2,6}.0^{18,21}]tricosa-1(20),2,4,6(23), 15,17,21-heptaen-9-one **example 2** as a white solid.
LCMS method E: [M+H]⁺ = 339.1, t_{R} = 2.298 min
LCMS method D: [M+H]⁺ = 339.1, t_{R} = 3.425 min
¹H NMR (300 MHz, 100°C, *d*6-DMSO) δ 13.00 (s, 1H), 9.03 (s, 1H), 8.55 (s, 1H), 8.28 (s, 1H), 7.52 (d, J = 9.0 Hz, 1H), 7.20 (s, 1H), 7.02 (dd, J = 9.0, 2.3 Hz, 1H), 5.40 (brs, J = 17.6 Hz, 2H), 4.32 (t, J = 8.4 Hz, 2H), 3.68 - 3.21 (m, 2H), 3.03 (s, 3H), 2.33 - 2.04 (m, 2H) ppm.

### Example 3: 4-fluoro-8,14-dioxa-10,19,20-triazatetracyclo[13.5.2.1^{2,6}.0^{18,21}]tricosa-1(20), 2,4,6(23),15,17,21-heptaen-9-one

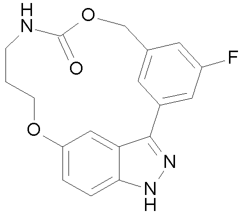

Example 3 is prepared according to the synthesis route described in general Scheme A.

### Preparation of intermediate 13: 2-[3-(3-iodo-1-tetrahydropyran-2-yl-indazol-5-yl)oxy propyl]isoindoline-1,3-dione

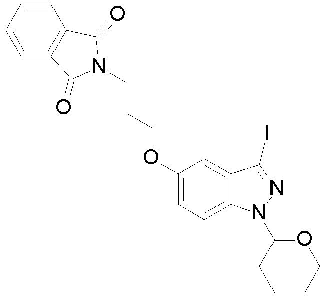

A suspension of 3-iodo-1-tetrahydropyran-2-yl-indazol-5-ol **4** (4 g, 11.63 mmol), cesium carbonate (7.560 g, 23.26 mmol) and *N*-(3-bromopropyl)phthalimide (4.679 g, 17.45 mmol) in *N*,*N*-dimethylformamide (48 mL) was heated at 60°C for 16h. The reaction mixture was concentrated under reduced pressure. The resulting white solid was triturated with ethyl acetate and recovered. The recovered filtrate was washed with water. The aqueous layer was extracted with ethyl acetate (3 x). The combined organic layer was washed with water then brine, dried over sodium sulfate, filtered and evaporated under vacuum to give a cream solid. Both white and cream solids were gathered to give 2-[3-(3-iodo-1-tetrahydropyran-2-yl-indazol-5-yl)oxypropyl]isoindoline-1,3-dione **13** as a cream solid.
LCMS method F: [M+H]⁺ = 532, t_{R} = 3.12 min

### Preparation of intermediate 14: 3-(3-iodo-1-tetrahydropyran-2-yl-indazol-5-yl)oxypropan-1-amine

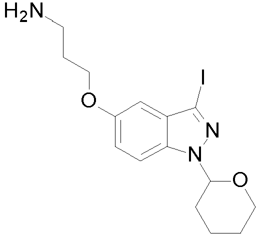

A mixture of 2-[3-(3-iodo-1-tetrahydropyran-2-yl-indazol-5-yl)oxypropyl]isoindoline-1,3-dione **13** (6.176 g, 11.63 mmol) and hydrazine monohydrate (2.04 mL, 58.15 mmol) in EtOH (40 mL) was heated to 50°C for 16 h. The reaction mixture was evaporated under reduced pressure and EtOH was added to the white solid. The solid was filtrated, washed with EtOH (3 x) and the filtrate was evaporated under reduced pressure to give 3-(3-iodo-1-tetrahydropyran-2-yl-indazol-5-yl)oxypropan-1-amine **14** as a pale brown oil.
LCMS method F: [M+H]⁺ = 402, t_{R} = 1.65 min

### Preparation of intermediate 15: [3-[5-(3-aminopropoxy)-1-tetrahydropyran-2-yl-indazol-3-yl]-5-fluoro-phenyl]methanol

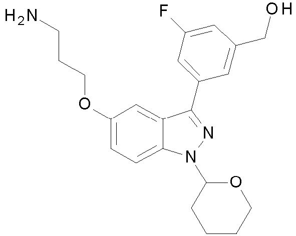

To a degassed solution of 3-(3-iodo-1-tetrahydropyran-2-yl-indazol-5-yl)oxypropan-1-amine **14** (200 mg, 0.500 mmol), 3-fluoro-5-(hydroxymethyl)phenylboronic acid (127 mg, 0.750 mmol), tripotassium phosphate (318 mg, 1.500 mmol) and xPhos (24 mg, 0.050 mmol) in 1,4-dioxane (3.2 mL) and water (1.4 mL) was added tetrakis(triphenylphosphine)palladium(0) (29 mg, 0.025 mmol). The reaction mixture was irradiated under µ-waves (Biotage initiator+), absorption level: high at 120°C for 1h. The reaction mixture was filtered through a celite bed then the celite was washed with ethyl acetate. The filtrate was diluted with water and extracted with ethyl acetate (3x). The organic layer was washed with water then brine, dried over sodium sulfate and concentrated under reduced pressure to give [3-[5-(3-aminopropoxy)-1-tetrahydropyran-2-yl-indazol-3-yl]-5-fluoro-phenyl]methanol **15** as a pale yellow oil.
LCMS method F: [M+H]⁺ = 400, t_{R} = 1.76 min

### Preparation of intermediate 16: 4-fluoro-19-(oxan-2-yl)-8,14-dioxa-10,19,20-triazatetra cyclo[13.5.2.1^{2,6}.0^{18,21}]tricosa-1(20),2(23),3,5,15(22),16,18(21)-heptaen-9-one

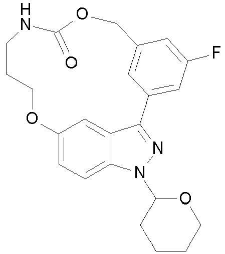

To a solution of [3-[5-(3-aminopropoxy)-1-tetrahydropyran-2-yl-indazol-3-yl]-5-fluorophenyl]methanol 15 (199 mg, 0.499 mmol) in DMA (150 mL) was added 1,1'-carbonyldiimidazole (89 mg, 0.549 mmol). The reaction mixture was stirred at RT for 2 hours then heated to 90°C for 48 hours. The reaction was concentrated under vacuum then ethyl acetate and a saturated aqueous solution of NaHCO₃ were added. The mixture was extracted with ethyl acetate (2x). The combined organic layers were washed with water then brine, dried over sodium sulfate, filtered and the solvent was removed under reduced pressure. The crude product was purified by column chromatography eluting with cyclohexane/EtOAc/EtOH (3-1): 100/0 to 70/30 to give 4-fluoro-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one 16 as a white solid.
LCMS method F: [M+H]⁺ = 426, t_{R} = 2.84 min

### Preparation of Example 3: 4-fluoro-8,14-dioxa-10,19,20-triazatetracyclo[13.5.2.1^{2,6}.0^{18,21}] tricosa-1(20), 2,4,6(23),15,17,21-heptaen-9-one

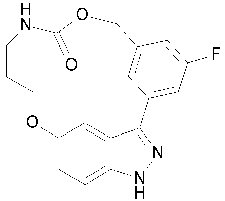

To a solution of 4-fluoro-19-(oxan-2-yl)-8,14-dioxa-10,19,20-triazatetracyclo[13.5.2. 1^{2,6}.0^{18,21}]tricosa-1(20),2(23),3,5,15(22),16,18(21)-heptaen-9-one **16** (92 mg, 0.217 mmol) in 1,4-dioxane (2.6 mL) was added 4M HCl in 1,4-dioxane (0.54 mL, 2.17 mmol) and the reaction was stirred at RT for 1h30. The reaction mixture was heated to 50°C for 60 hours. The solvent was removed under reduced pressure and the cream solid was recrystallized with acetonitrile to give 4-fluoro-8,14-dioxa-10,19,20-triazatetracyclo[13.5.2.1^{2,6}.0^{18,21}]tricosa-1(20),2,4,6(23),15, 17,21-heptaen-9-one **example 3** as a white solid.
LCMS method F: [M+H]⁺ = 342, t_{R} = 2.16 min
LCMS method G: [M+H]⁺ = 342, t_{R} = 2.24 min
¹H NMR (400 MHz, *d*6-DMSO) δ 13.06 (1H, s), 7.74 (2H, m), 7.62 - 7.58 (1H, m), 7.52 - 7.49 (1H, m), 7.35 (1H, m), 7.14 - 7.11 (1H, m), 7.00 (1H, m), 5.29 (2H, s), 4.33 (2H, t), 3.22 - 3.18 (2H, m), 2.06 - 2.05 (2H, m) ppm.

### Example 4: 8,14-dioxa-10,19,20,23-tetraazatetracyclo[13.5.2.1^{2,6}.0^{18,21}]tricosa-1(20),2,4, 6(23),15,17,21-heptaen-9-one

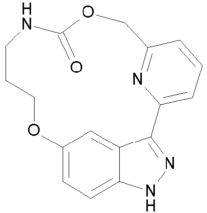

Example 4 is prepared according to the synthesis route described in general Scheme B.

### Preparation of intermediate 17: tert-butyl-dimethyl-(1-tetrahydropyran-2-ylindazol-5-yl)oxy-silane

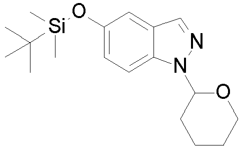

To a solution of tert-butyl-(1H-indazol-5-yloxy)-dimethyl-silane **1** (15.95 g, 64.28 mmol) in DCM (200 mL) and THF (100 mL) was added at RT methane sulfonic acid (0.834 mL, 12.86 mmol) and DHP (17.59 mL, 192.84 mmol). The resulting reaction mixture was stirred at RT overnight. The residue was diluted with saturated sodium bicarbonate solution and extracted with EtOAc twice. The combined organic layers were dried over anhydrous sodium sulfate and concentrated under reduced pressure. The residue was purified by flash-column chromatography (120 g silica Biotage) chromatography (cyclohexane - ethyl acetate, 1:0 to 90/10) . The desired fractions were combined and the solvent was removed under reduced pressure to give tert-butyl-dimethyl-(1-tetrahydropyran-2-ylindazol-5-yl)oxy-silane **17** as white crystals.
LCMS method F: [M+H]⁺ = 333.2, t_{R} = 3.53 min

### Preparation of intermediate 18: [5-[tert-butyl(dimethyl)silylfoxy-1-tetrahydropyran-2-yl-indazol-3-yl]boronic acid and 3-[(tert-butyldimethylsilyl)oxy]-1-(oxan-2-yl)-3-(tetramethyl-1,3,2-dioxaborolan-2-yl)-1H-indazole

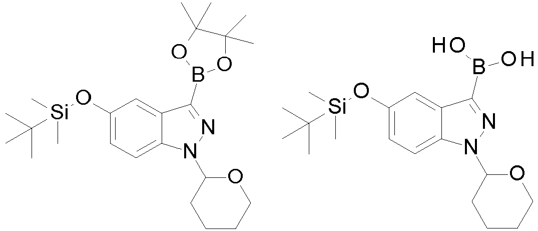

In a sealed tube was added tert-butyl-dimethyl-(1-tetrahydropyran-2-ylindazol-5-yl)oxy-silane **17** (3 g; 9.03 mmol), TBME(15 mL), 4,4,5,5-tetramethyl-2-(4,4,5,5-tetramethyl-1,3,2-dioxaborolan-2-yl)-1,3,2-dioxaborolane(2.3 g; 9.03 mmol), 4,4'-di-tert-butyl-2,2'-bipyridine(145 mg; 0.54mmol) and (1,5-cyclooctadiene)(methoxy)iridium(I) dimer (119 mg; 0.18 mmol). The reaction was degassed with Argon during 10 min then it was put to react overnight at 80°C. The solvent was removed under reduced pressure, then the oil was dissolved with ethyl acetate and water. The layers were separated and the aqueous layer was extracted twice with ethyl acetate. The organic layers were combined and the solvent was removed under reduced pressure to give a mixture of [5-[tert-butyl(dimethyl)silyl]oxy-1-tetrahydropyran-2-yl-indazol-3-yl]boronic acid and 5-[(tert-butyldimethylsilyl)oxy]-1-(oxan-2-yl)-3-(tetramethyl-1,3,2-dioxaborolan-2-yl)-1H-indazole **18** as a brown oil. The product was used in next step without further purification.
LCMS method F: [M+H]⁺ = 459, t_{R} = 3.80 min
LCMS method G: [M+H]⁺ = 377.2, t_{R} = 3.15 min

### Preparation of intermediate 19: 2-(5-hydroxy-1-tetrahydropyran-2-yl-indazol-3-yl)pyridine-4-carboxylate

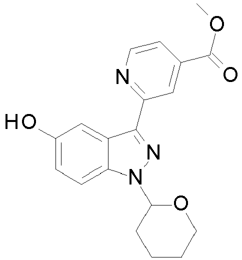

To a solution of [5-[tert-butyl(dimethyl)silyl]oxy-1-tetrahydropyran-2-yl-indazol-3-yl]boronic acid and 5-[(tert-butyldimethylsilyl)oxy]-1-(oxan-2-yl)-3-(tetramethyl-1,3,2-dioxaborolan-2-yl)-1H-indazole **18** (1.5 g, 3.99 mmol) in N,N-dimethylformamide (5 mL) were added at RT methyl 6-bromopyridine-2-carboxylate (1.030 g, 4.78 mmol), cesium carbonate (3.8 g, 11.96 mmol) and PdCl₂dppf.DCM (163 mg, 0.2 mmol). The resulting reaction mixture was stirred at 110°C overnight. The solvent was removed under reduced pressure and the oil was dissolved in EtOAc and water. The two layers were separated and the aqueous phase was extracted with ethyl acetate twice. The combined organic layers were dried over anhydrous sodium sulfate and concentrated under reduced pressure. The residue was purified by flash-column chromatography (30 g silica BIOTAGE) chromatography (cyclohexane - ethyl acetate, 100/0 to 50/50) affording methyl 2-(5-hydroxy-1-tetrahydropyran-2-yl-indazol-3-yl)pyridine-4-carboxylate **19** as a yellow powder.
LCMS method F: [M+H]⁺ = 354.1, t_{R} = 2.59 min

### Preparation of intermediate 20: benzyl N-(3-bromopropyl)carbamate

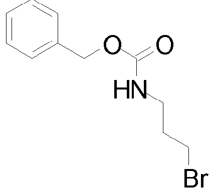

To a solution of 3-bromopropylamine hydrochloride (6 g, 27 mmol) in aqueous NaOH 10 % (40 mL) at 0°C were added slowly CbzCl (4.3 mL, 30 mmol) and NaOH 10 % (40 mL). After 12 h, the reaction mixture was diluted with DCM. The aqueous layer was extracted two times with DCM (100 mL). The combined organic layers were washed with brine, dried over magnesium sulfate, filtered and concentrated under reduced pressure. This residue was purified by flash chromatography on silica gel (Macherey Nagel, 80 g) with gradient elution: cyclohexane/EtOAc 0-20 % to give benzyl N-(3-bromopropyl)carbamate **20** as a transparent oil.
LCMS method F: [M+H]⁺ = 274, t_{R} = 2.41 min

### Preparation of intermediate 21: methyl 6-[5-(3-{[(benzyloxy)carbonyl]amino}propoxy)-1-(oxan-2-yl)-1H-indazol-3-yl]pyridine-2-carboxylate

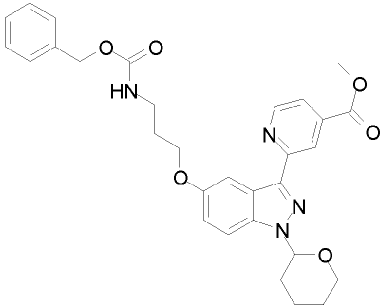

To a solution of methyl 6-[5-hydroxy-1-(oxan-2-yl)-1H-indazol-3-yl]pyridine-2-carboxylate **19** (1 g, 2.82 mmol) in N,N-dimethylformamide (100 mL), cesium carbonate (1.83 g, 5.6 mmol) and benzyl N-(3-bromopropyl)carbamate 20 (0 .765 g, 2.82 mmol) were added. The reaction was stirred at 120°C for 16 hours. The mixture was concentrated under reduced pressure. Water (200 mL) was added and the resulting mixture was extracted with EtOAc (4 x 100 mL). The combined organic layers were washed with brine (2 x 50 mL). The organic layer was dried over sodium sulfate, filtered off and evaporated under reduced pressure to afford brown/orange oil.

This residue was purified by flash chromatography on silica gel (Macherey Nagel, 120 g) with gradient elution : cyclohexane/EtOAc 0-70 % to give methyl 6-[5-(3-{[(benzyloxy)carbonyl] amino}propoxy)-1-(oxan-2-yl)-1H-indazol-3-yl]pyridine-2-carboxylate **21** as a white solid.
LCMS method F: [M+H]⁺ = 545.2, t_{R} = 3.21 min

### Preparation of intermediate 22: benzyl N-[3-({3-[6-(hydroxymethyl)pyridin-2-yl]-1-(oxan-2-yl)-1H-indazol-5-yl}oxy)propyl]carbamate

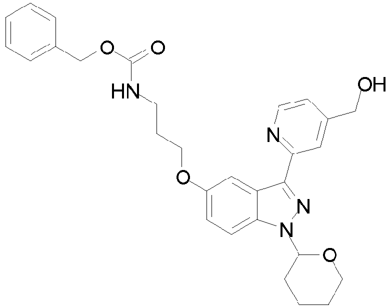

To methyl 6-[5-(3-{[(benzyloxy)carbonyl]amino}propoxy)-1-(oxan-2-yl)-1H-indazol-3-yl]pyridine-2-carboxylate **21** (1.2 g, 2.2 mmol) in THF (50 mL) was added a 1 M solution of lithium aluminium tetrahydride (4.4 mL, 4.2 mmol) at 0°C. The mixture was stirred at 0 °C for 1 hour. To the reaction mixture, EtOAc (10 mL) was added at 0°C and poured in a 10% solution of Rochelle's salt (100 mL) and EtOAc (100 mL). The mixture was stirred at RT for 2 hours. After separation, the aqueous layer was extracted with EtOAc (2x50 mL). The combined organic layers were washed with brine, dried over sodium sulfate and concentrated under reduced pressure to brown/orange oil. This residue was purified by flash chromatography on silica gel (Macherey Nagel, 120 g) with gradient elution : cyclohexane/EtOAc 0-100 % to give benzyl N-[3-({3-[6-(hydroxymethyl)pyridin-2-yl]-1-(oxan-2-yl)-1H-indazol-5-yl}oxy)propyl] carbamate **22** as a yellow oil.
LCMS method F: [M+H]⁺ = 517.3, t_{R} = 2.76 min

### Preparation of intermediate 23: 19-(oxan-2-yl)-8,14-dioxa-10,19,20,23-tetraazatetracyclo [13.5.2.1^{2,6}.0^{18,21}]tricosa-1(20),2,4,6(23),15,17,21-heptaen-9-one

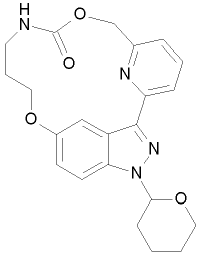

A solution of benzyl N-[3-({3-[6-(hydroxymethyl)pyridin-2-yl]-1-(oxan-2-yl)-1H-indazol-5-yl}oxy)propyl]carbamate **22** (0.4 g; 0.775 mmol) in 100 mL of toluene was added over 30 min to a solution a solution of sodium hydride (60% suspension in paraffin oil) (310 mg, 7.75 mmol) in 100 mL of toluene at room temperature. The reaction mixture was stirred at RT for 5 min and then one hour at 130°C. The reaction is allowed to cool down and then 10 mL of EtOH is added carefully. 100 mL of water in added. After separation, the aqueous layer was extracted with ethyl acetate (2 x 100 mL). The combined organic layers were washed with brine, dried over sodium sulfate and concentrated under reduced pressure to give an orange oil. A purification by column chromatography (DCM/MeOH 0-10 %) afforded pure 19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one 23 as a whitish solid.
LCMS method F: [M+H]⁺ = 409.2, t_{R} = 2.53 min

### Preparation of Example 4: 8,14-dioxa-10,19,20,23-tetraazatetracyclo[13.5.2.1^{2,6}.0^{18,21}] tricosa-1(20),2,4, 6(23),15,17,21-heptaen-9-one

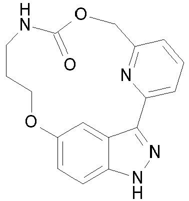

To a solution of 19-(oxan-2-yl)-8,14-dioxa-10,19,20,23-tetraazatetracyclo [13.5.2.1^{2,6}.0^{18,21}]tricosa-1(20),2,4,6(23),15,17,21-heptaen-9-one **23** (0.2 g; 0.489 mmol) in DCM (20 mL) was added Trifluoroacetic acid (0.38 mL, 4.89 mmol) at room temperature. The mixture was stirred at 50 °C for 24 hours. The reaction is allowed to cool down. 50 mL of toluene were added to the solution and the reaction mixture was concentrated under reduced pressure to give an orange oil. 25 mL of water and 25 mL of DCM and a 25 wt% aqueous solution of ammonia (1.5 mL) were added. After separation, the aqueous layer was extracted with DCM (2x20 mL). The combined organic layers were washed with brine, dried over sodium sulfate and concentrated under reduced pressure to give an orange oil. A purification by column chromatography (DCM/MeOH 0-5 %) afforded pure 8,14-dioxa-10,19,20,23-tetraazatetracyclo [13.5.2.1^{2,6}.0^{18,21}]tricosa-1(20),2,4,6(23),15,17,21-heptaen-9-one **example 4** as a whitish solid.
LCMS method F: [M+H]⁺ = 325.2, t_{R} = 1.93 min
LCMS method G: [M+H]⁺ = 325.2, t_{R} = 1.94 min
¹H NMR (400 MHz, d6-DMSO) δ 13.2 (1H, m), 8.08 (1H, d, J = 9.7 Hz), 7.90 (1H, d, J = 3.5 Hz), 7.83 (1H, t, J = 8.3 Hz), 7.75 (1H, t, J = 5.9 Hz), 7.47 (1H, d, J = 8.3 Hz), 7.26 (1H, d, J = 8.3 Hz), 6.97 (1H, dd, J = 2.5, 9.1 Hz), 5.31 (2H, m), 4.31 (2H, dd, J = 7.7, 8.6 Hz), 3.11 - 3.09 (2H, m), 1.97- 2.03 (2H, m) ppm.

### Example 5: 8,14-dioxa-10,19,20-triazatetracyclo[13.5.2.1^{2,6}.0^{18,21}]tricosa-1(20),2,4,6(23), 15,17,21-heptaen-9-one

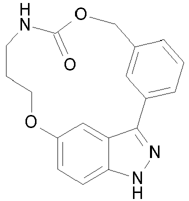

Example 5 is prepared according to the synthesis route described in general Scheme A.

### Preparation of intermediate 24: [3-[5-(3-aminopropoxy)-1-tetrahydropyran-2-yl-indazol-3-yl]phenyl]methanol

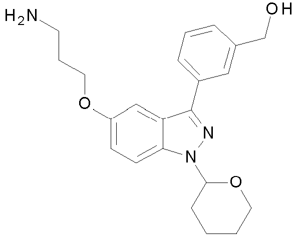

To a degassed solution of 3-(3-iodo-1-tetrahydropyran-2-yl-indazol-5-yl)oxypropan-1-amine **14** (400 mg, 0.998 mmol), 3-(Hydroxymethyl)phenylboronic acid (227 mg, 1.497 mmol), tripotassium phosphate (636 mg, 2.994 mmol) and xPhos (48 mg, 0.100 mmol) in dioxane (6.4 mL) and water (2.8 mL) was added tetrakis(triphenylphosphine)palladium(0) (58 mg, 0.050 mmol). The reaction mixture was heated under microwave conditions (Biotage initiator+) at 120°C for 1h. The reaction mixture was filtered through celite bed then the celite was washed with ethyl acetate. The filtrate was then diluted with water and extracted with ethyl acetate (3x). The organic layer was washed with water then brine, dried over sodium sulfate and concentrated under reduced pressure to give [3-[5-(3-aminopropoxy)-1-tetrahydropyran-2-yl-indazol-3-yl]phenyl]methanol 24 as a pale yellow oil.
LCMS method F: [M+H]⁺ = 382, t_{R} = 1.64 min

### Preparation of intermediate 25: 19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one

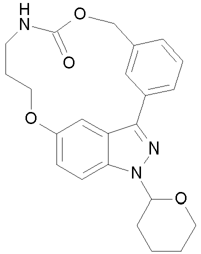

To a solution of [3-[5-(3-aminopropoxy)-1-tetrahydropyran-2-yl-indazol-3-yl]phenyl] methanol **24** (380 mg, 0.998 mmol) in DMA (300 mL) was added 1,1'-Carbonyldiimidazole (178 mg, 1.100 mmol). The reaction mixture was stirred at RT for 2h then 90°C for 64h. The reaction was concentrated under vacuum then ethyl acetate and a saturated aqueous solution of NaHCO3 were added. The mixture was extracted with ethyl acetate (2 x). The combined organic layers were washed with water then brine, dried over sodium sulfate, filtered and concentrated under reduced pressure. The crude product was purified by column chromatography eluting with cyclohexane/ethyl acetate-EtOH (3-1) : 100/0 to 70/30 to give a white solid. The solid was recrystallized with acetonitrile to give 19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **25** as a white solid.
LCMS method F: [M+H]⁺ = 408, t_{R} = 2.76 min

### Preparation of Example 5: 8,14-dioxa-10,19,20-triazatetracyclo[13.5.2.1^{2,6}.0^{18,21}]tricosa-1(20),2,4,6(23), 15,17,21-heptaen-9-one

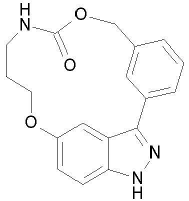

To a solution of 19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **25** (81 mg, 0.199 mmol) in dioxane (2.4 mL) was added 4M HCl in dioxane (0.75 mL, 2.985 mmol) and the reaction was heated to 50°C for 24h. The reaction mixture was cooled down to RT and the solid was filtered then rinsed with diisopropyl ether (3 x) to give 8,14-dioxa-10,19,20-triazatetracyclo[13.5.2.1^{2,6}.0^{18,21}]tricosa-1(20),2,4,6(23),15,17,21-heptaen-9-one **example 5** as a white solid.
LCMS method F: [M+H]⁺ = 324, t_{R} = 2.02 min
LCMS method G: [M+H]⁺ = 324, t_{R} = 2.10 min
¹H NMR (400 MHz, *d*6-DMSO) δ 7.93 - 7.87 (2H, m), 7.69 - 7.66 (1H, m), 7.50 - 7.44 (2H, m), 7.36 (1H, d, J = 2.3 Hz), 7.28 - 7.25 (1H, m), 6.98 (1H, dd, J = 2.3, 8.9 Hz), 5.33 - 5.29 (3H, m), 4.32 (2H, m), 3.18 (2H, m), 2.04 (2H, m) ppm.

### Example 6: 10-(propan-2-yl)-8,14-dioxa-4,10,19,20-tetraazatetracyclo[13.5.2.1^{2,6}.0^{18,21}] tricosa-1(20),2,4,6(23),15,17,21-heptaen-9-one

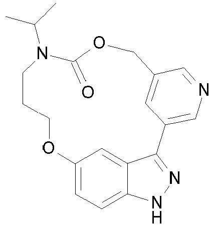

Example 6 is prepared according to the synthesis route described in general Scheme A.

Example 6 is made using analog conditions as for example 2. 2-Iodopropane is used for the alkylation step of the carbamate to yield 10-(propan-2-yl)-8,14-dioxa-4,10,19,20-tetraazatetracyclo[13.5.2.1^{2,6}.01^{8,21}]tricosa-1(20),2,4,6(23),15,17,21-heptaen-9-one **example 6.**
LCMS method E: [M+H]⁺ = 367.2, t_{R} = 2.829 min
LCMS method D: [M+H]⁺ = 367.2, t_{R} = 3.832 min
¹H NMR (300 MHz, 100°C, *d*6-DMSO) δ 12.96 (s, 1H), 9.01 (s, 1H), 8.54 (s, 1H), 8.39 (t, J = 2.1 Hz, 1H), 7.50 (d, J = 9.0 Hz, 1H), 7.23 (s, 1H), 6.99 (dd, J = 9.0, 2.3 Hz, 1H), 5.36 (brs, 2H), 4.28 (t, J = 8.6 Hz, 2H), 4.20 - 4.04 (m, 1H), 3.32 (brt, J = 7.3 Hz, 2H), 2.19 (brs, 2H), 1.18 (s, 3H), 1.15 (s, 3H) ppm.

### Example 7: 8,14-dioxa-5,10,19,20-tetraazatetracyclo[13.5.2.1^{2,6}.0^{18,21}]tricosa-1(20),2,4, 6(23),15,17,21-heptaen-9-one

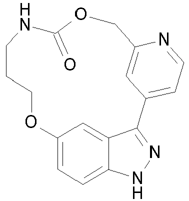

Example 7 is prepared according to the synthesis route described in general Scheme B.

Example 7 is made using analog conditions as for example 4. Methyl 4-bromopyridine-2-carboxylate is used for the Suzuki reaction to give 8,14-dioxa-5,10,19,20-tetraazatetracyclo[13.5.2.1^{2,6}.0^{18,21}]tricosa-1(20),2,4,6(23),15,17,21-heptaen-9-one **example 7.**
LCMS method F: [M+H]⁺ = 325.1, t_{R} = 1.58 min
LCMS method G: [M+H]⁺ = 325.2, t_{R} = 1.83 min
¹H NMR (400 MHz, *d*6-DMSO) δ 13.28 (1H, s), 8.59 - 8.57 (1H, m), 7.86 (2H, m), 7.83 (1H, dd, J = 2.1, 5.5 Hz), 7.55 (1H, d, J = 9.0 Hz), 7.44 (1H, d, J = 2.1 Hz), 7.03 (1H, dd, J = 2.1, 9.0 Hz), 5.32 - 5.31 (2H, m), 4.37 (2H, dd, J = 8.3, 8.6 Hz), 3.19 - 3.18 (2H, m), 2.10 - 2.05 (2H, m) ppm.

### Example 8: 4-methoxy-8,14-dioxa-10,19,20-triazatetracyclo[13.5.2.1^{2,6}.0^{18,21}]tricosa-1(20),2,4,6(23),15,17,21-heptaen-9-one

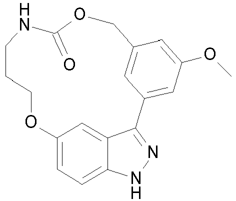

Example 8 is prepared according to the synthesis route described in general Scheme C.

### Preparation of intermediate 26: benzyl N-[3-(3-iodo-1-tetrahydropyran-2-yl-indazol-5-yl) oxypropyl]carbamate

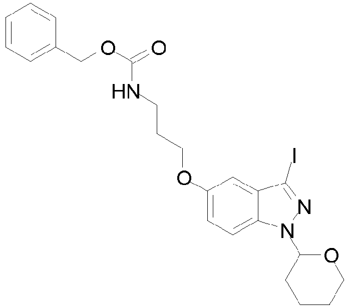

A suspension of 3-iodo-1-tetrahydropyran-2-yl-indazol-5-ol **4** (17.012 g, 49.453 mmol), cesium carbonate (32.144 g, 98.906 mmol) and benzyl N-(3-bromopropyl)carbamate 20 (10.6 mL, 54.398 mmol) in *N,N*-dimethylformamide (250 mL) was heated at 60°C for 20 h. The reaction mixture was filtered and rinsed with acetonitrile. The filtrate crystallized and it was filtered to give a white solid which was rinsed with water (3x). The filtrate was recovered and evaporated under reduced pressure to give a pink solid. It was solubilized with DCM and water was added. It was extracted with DCM (2x) then the combined organic layers were dried over anhydrous sodium sulfate and concentrated under reduced pressure to give a pale pink solid. The solid was recrystallized from acetonitrile to give benzyl N-[3-(3-iodo-1-tetrahydropyran-2-yl-indazol-5-yl)oxypropyl]carbamate **26** as a white solid.
LCMS method F: [M+H]⁺ = 536.0, t_{R} = 3.11 min

### Preparation of intermediate 27: benzyl N-[3-[3-[3-(hydroxymethyl)-5-methoxy-phenyl]-1-tetrahydropyran-2-yl-indazol-5-yl]oxypropyl]carbamate

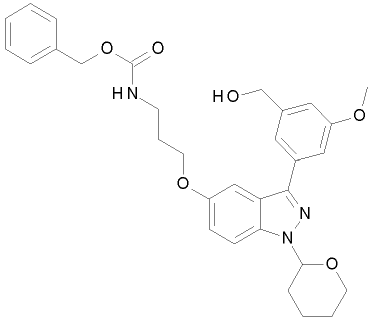

To a degassed solution of benzyl N-[3-(3-iodo-1-tetrahydropyran-2-yl-indazol-5-yl)oxypropyl] carbamate **26** (600 mg, 1.12 mmol), [3-Methoxy-5-(tetramethyl-1,3,2-dioxaborolan-2-yl)phenyl]methanol (444 mg, 1.68 mmol), tripotassium phosphate (713 mg, 3.36 mmol) and xPhos (53 mg, 0.112 mmol) in 1,4-dioxane (7 mL) and water (4.8 mL) was added tetrakis(triphenylphosphine)palladium(0) (65 mg, 0.056 mmol). The reaction mixture was irradiated under µ-waves (Biotage initiator+), absorption level: high at 120°C for 1 h. The reaction mixture was filtered through celite bed then the celite was washed with ethyl acetate. The filtrate was then diluted with water and extracted with ethyl acetate (3x). The organic layer was washed with water then brine, dried over sodium sulfate and concentrated under reduced pressure. The crude was purified by column chromatography eluting with DCM / Ethyl acetate, 100/0 to 70/30 to give benzyl N-[3-[3-[3-(hydroxymethyl)-5-methoxy-phenyl]-1-tetrahydropyran-2-yl-indazol-5-yl]oxypropyl]carbamate **27** as a colorless oil.
LCMS method F: [M+H]⁺ = 546, t_{R} = 2.89 min

### Preparation of intermediate 28: 4-methoxy-19-(oxan-2-yl)-8,14-dioxa-10,19,20-triazatetra cyclo[13.5.2.1^{2,6}.0^{18,21}]tricosa-1(20),2,4,6(23),15,17,21-heptaen-9-one

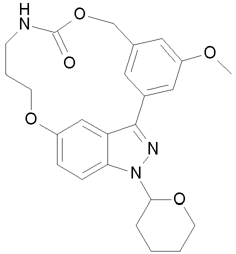

To a suspension of potassium carbonate (80 mg, 0.582 mmol) in acetonitrile (12 mL) was dropwise added a solution of benzyl N-[3-[3-[3-(hydroxymethyl)-5-methoxy-phenyl]-1-tetrahydropyran-2-yl-indazol-5-yl]oxypropyl]carbamate **27** (53 mg, 0.097 mmol) in acetonitrile (7 mL) at RT. The reaction mixture was heated under microwave conditions at 140°C for 6 h. The reaction mixture was filtered and directly purified by column chromatography eluting with DCM / Ethyl acetate, 100/0 to 80/20 to 4-methoxy-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **28** as a colorless oil.
LCMS method F: [M+H]⁺ = 438, t_{R} = 2.76 min

### Preparation of Example 8: 4-methoxy-8,14-dioxa-10,19,20-triazatetracyclo [13.5.2.1^{2,6}.0^{18,21}]tricosa-1(20),2,4,6(23),15,17,21-heptaen-9-one

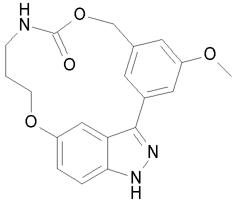

To a solution of 4-methoxy-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **28** (23 mg, 0.053 mmol) in DCM (4 mL) was added trifluoro acetic acid (80 µL, 1.06 mmol) at RT. The reaction mixture was irradiated under µ-waves (Biotage initiator+), absorption level: high at 80°C for 1h30. The crude reaction mixture was purified by flash-column chromatography eluting with DCM / Ethyl acetate : 100/0 to 80/20, to give 4-methoxy-8,14-dioxa-10,19,20-triazatetracyclo [13.5.2.1^{2,6}.0^{18,21}]tricosa-1(20),2,4,6(23),15,17,21-heptaen-9-one **example 8** as a white solid.
LCMS method F: [M+H]⁺ = 354, t_{R} = 2.07 min
LCMS method G: [M+H]⁺ = 354, t_{R} = 2.09 min
¹H NMR (400 MHz, *d*6-DMSO) δ 12.89 (1H, s), 7.67 (1H, m), 7.52 - 7.47 (2H, m), 7.42 - 7.34 (2H, m), 6.99 - 6.96 (1H, m), 6.88 (1H, m), 5.25 (2H, m), 4.31 (2H, t), 3.86 (3H, s), 3.17 (2H, m), 2.03 (2H, m) ppm.

### Example 9: 4-bromo-8,14-dioxa-10,19,20-triazatetracyclo[13.5.2.1^{2,6}.0^{18,21}]tricosa-1(20), 2,4,6(23),15,17,21-heptaen-9-one

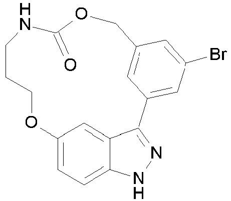

Example 9 can be prepared according to the synthesis route described in general Scheme A, C and D.

### Preparation of intermediate 29: 1-tetrahydropyran-2-ylindazol-5-ol

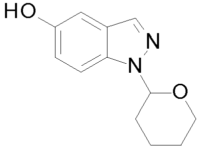

To a solution of tert-butyl-dimethyl-(1-tetrahydropyran-2-ylindazol-5-yl)oxy-silane 17 (12.58 g, 37.8 mmol) in tetrahydrofuran (100 mL) was added by portions tetra-n-butylammonium fluoride 1.0 M in THF (47.58 mL, 47.58 mmol) at RT. The reaction mixture was stirred at RT for 1 h. The reaction mixture was poured into ice water (300 mL) and stirred for 1 h. The aqueous phase was extracted with ethyl acetate (2x150 mL). The combined organic layers were washed with brine (150 mL), dried over magnesium sulfate anhydrous and concentrated under reduced pressure. Purification on silica column (RS SiOH 80 g) using cyclohexane/ ethyl acetate as eluent from 90/10 to 80/20 gave 1-tetrahydropyran-2-ylindazol-5-ol **29** as a colorless oil.
LCMS method F: [M+H]⁺ = 219, t_{R} = 1.81 min

### Preparation of intermediate 30: benzyl N-[3-(1-tetrahydropyran-2-ylindazol-5-yl)oxypropyll carbamate

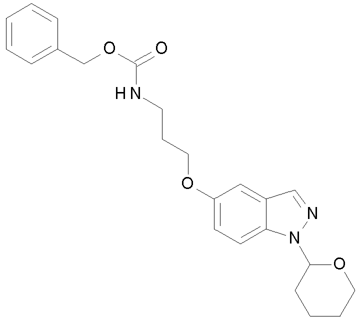

To a solution of 1-tetrahydropyran-2-ylindazol-5-ol **29** (7.06 g, 32.3 mmol) in *N,N-*dimethylformamide (110 mL) was added cesium carbonate (21.0 g, 64.6 mmol) and benzyl N-(3-bromopropyl)carbamate 20 (10.14 g, 37.3 mmol) at RT. The mixture was stirred at 80 °C overnight. The reaction mixture was concentrated under reduced pressure. Water (100 mL) and ethyl acetate (200 mL) were added to the residue. After separation, the aqueous layer was extracted with ethyl acetate (2x 50 mL). The combined organic layers were washed with brine (100 mL), dried over sodium sulfate anhydrous and concentrated under reduced pressure to dryness.

Purification on silica column (RS SiOH 200 g) using Cyclohexane/ Ethyl acetate from 80/20 to 60/40 as eluent gave benzyl N-[3-(1-tetrahydropyran-2-ylindazol-5-yl)oxypropyl]carbamate **30** as a beige solid.
LCMS method F: [M+H]⁺ = 410.2, t_{R} = 2.77 min (current 20V)

### Preparation of intermediate 31: benzyl N-[3-[1-tetrahydropyran-2-yl-3-(4,4,5,5-tetramethyl-1,3,2-dioxaborolan-2-yl)indazol-5-yl]oxypropyl]carbamate

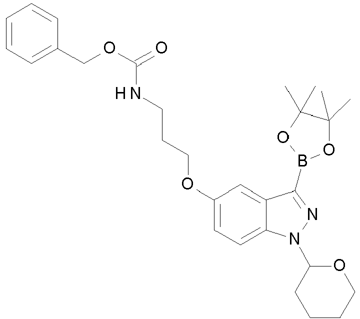

To a solution of benzyl N-[3-(1-tetrahydropyran-2-ylindazol-5-yl)oxypropyl]carbamate **30** (11.42 g, 27.9 mmol) in TBME/THF (500/100 mL) was added 4,4,5,5-tetramethyl-2-(4,4,5,5-tetramethyl-1,3,2-dioxaborolan-2-yl)-1,3,2-dioxaborolane (7.79 g, 30.69 mmol) and 4,4'-di-tert-butyl-2,2'-bipyridine (450 mg, 1.67 mmol). The reaction mixture was degassed by bubbling nitrogen for 15 min and (1,5-cyclooctadiene)(methoxy)iridium(I) dimer (370 mg, 0.56 mmol) was added. The reaction mixture was stirred at 80°C overnight under atmosphere of nitrogen. The solvent was removed under reduced pressure, then the oil was dissolved with ethyl acetate and water. The layers were separated and the aqueous layer was extracted twice with ethyl acetate. The organic layers were combined and the solvent was removed under reduced pressure to give benzyl N-[3-[1-tetrahydropyran-2-yl-3-(4,4,5,5-tetramethyl-1,3,2-dioxaborolan-2-yl)indazol-5-yl]oxypropyl]carbamate **31** as a brown oil. The product was used in next step without further purification.
LCMS method F: [M+H]⁺ = 536.2, t_{R} = 3.18 min (current 20V)

### Preparation of intermediate 32: (3-bromo-5-iodo-phenyl)methanol

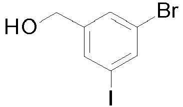

To a solution of 3-bromo-5-iodo-benzoic acid (10.0 g, 30.6 mmol) in THF (450 mL) was slowly added solid sodium borohydride (3.47 g, 91.8 mmol) at 0°C. After the end of the gas release (i.e. 5 min), boron trifluoride diethyl etherate (11.3 mL, 91.8 mmol) was dropwise added at 0°C. The reaction mixture was allowed to warm to RT and stirred at RT overnight. The reaction mixture was cooled to 0 °C and an aqueous 1 M solution of sodium hydroxide (100 mL) was slowly added. The reaction mixture was filtered off under celite pad and eluted with ethyl acetate. The solution was washed with water (100 mL) and with brine (100 mL). The organic layer was dried with sodium sulfate anhydrous, filtered off and the dried under reduced pressure to afford clean (3-bromo-5-iodo-phenyl)methanol **32** as a beige solid.
LCMS method F: [M+H]⁺ = not detected, t_{R} = 2.54 min (current 20V)

### Preparation of intermediate 33: benzyl N-[3-[3-[3-bromo-5-(hydroxymethyl)phenyl]-1-tetrahydropyran-2-yl-indazol-5-yl]oxypropyl]carbamate

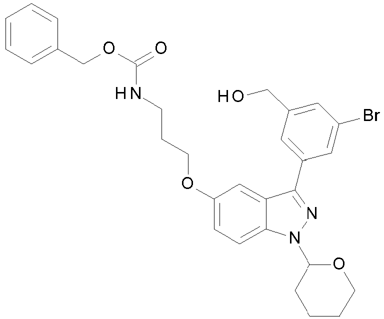

To a solution of benzyl N-[3-[1-tetrahydropyran-2-yl-3-(4,4,5,5-tetramethyl-1,3,2-dioxaborolan-2-yl)indazol-5-yl]oxypropyl]carbamate **32** (1.870 g, 3.50 mmol) in *N,N-*dimethylformamide (15 mL) was added at RT (3-bromo-5-iodo-phenyl)methanol 31 (1.314 g, 4.20 mmol) and Cs₂CO₃ (3.421 g, 10.50 mmol). The reaction mixture was degassed by bubbling nitrogen for 15 min and PdCl₂dppf (0.128 g, 0.18 mmol) was added. The resulting mixture was stirred at 110°C under microwave irradiation for 50 min. The reaction mixture was filtered over celite and washed with ethyl acetate. The solvent was removed under reduced pressure and the oil was dissolved in EtOAc and water. The two layers were separated and the aqueous phase was extracted with ethyl acetate twice. The combined organic layers were dried over anhydrous sodium sulfate and concentrated under reduced pressure. Purification by flash-column chromatography (40 g RS SiOH) chromatography (cyclohexane - ethyl acetate, 100/ 0 to 50/ 50) gave benzyl N-[3-[3-[3-bromo-5-(hydroxymethyl)phenyl]-1-tetrahydropyran-2-yl-indazol-5-yl]oxypropyl]carbamate **33** as an orange oil.
LCMS method F: [M+H]⁺ = 596.1, t_{R} = 3.07 min (current 20V)

### Preparation of intermediate 34: 4-bromo-19-(oxan-2-yl)-8,14-dioxa-10,19,20-triazatetra cyclo[13.5.2.1^{2,6}.0^{18,21}]tricosa-1(20),2,4,6(23),15,17,21-heptaen-9-one

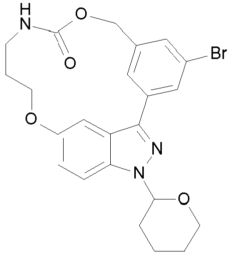

To a solution of benzyl N-[3-[3-[3-bromo-5-(hydroxymethyl)phenyl]-1-tetrahydropyran-2-yl-indazol-5-yl]oxypropyl]carbamate **33** (288 mg, 0.48 mmol) in dry toluene (300 mL) was added sodium hydride 60 % in oil (480 mg, 12 mmol) at RT. The reaction mixture was stirred at 130°C for 1h. The reaction was then stirred at RT overnight and sodium hydride 60 % in oil (192 mg, 4.8 mmol) was added. The reaction mixture was stirred at 130°C for 3h. More sodium hydride 60 % in oil (192 mg, 4.8 mmol) was added and the reaction mixture was stirred at 140 °C for overnight. More sodium hydride 60 % in oil (192 mg, 4.8 mmol) was added and the reaction mixture was stirred at 140 °C for 5 h. Again sodium hydride 60 % in oil (192 mg, 4.8 mmol) was added and the reaction mixture was stirred at 140 °C for 1 h till completion of the reaction. The reaction mixture was allowed to RT and cooled in an ice bath. EtOH (50 mL) was slowly added. The reaction mixture was diluted with ethyl acetate (200 mL) and water was added (200 mL). After separation, the aqueous layer was extracted with ethyl acetate (x3 50 mL). The combined organic layers were washed with brine (150 mL), dried over sodium sulfate, filtered and dried under reduced pressure to afford an orange oil.

Purification on silica column (RS SiOH 80 g) using cyclohexane/ ethyl acetate from 100/ 0 to 0/ 100 as and DCM/MeOH 90/ 10 as eluent gave 60 mg of the intended product. The impure fractions were pooled and the solvent was removed under reduced pressure. The residue was purified on silica column (RS SiOH 40 g) using clyclohexane/ ethyl acetate from 100/ 0 to 50/ 50 as eluent gave 4-bromo-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **34** as a white solid.
LCMS method F: [M+H]⁺ = 487.7, t_{R} = 3.05 min

### Preparation of Example 9: 4-bromo-8,14-dioxa-10,19,20-triazatetracyclo[13.5.2.1^{2,6}.0^{18,21}] tricosa-1(20), 2,4,6(23),15,17,21-heptaen-9-one

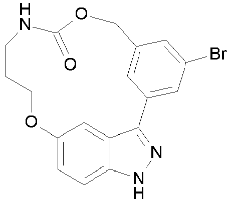

To a solution of 4-bromo-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **34** (30 mg, 0.062 mmol) in DCM (3 mL) was added trifluoroacetic acid (95 µL, 1.24 mmol). The reaction mixture was stirred at 80 °C under microwave irradiation for 2h. The reaction mixture was diluted with DCM (20 mL). Water (50 mL) and ammonium hydroxide 25 % weight aqueous solution (3 mL) were added. After separation, the aqueous layer was extracted with DCM (x3 10 mL). The combined organic layers were washed with saturated sodium carbonate aqueous solution (30 mL) and brine (30 mL). The organic layer was dried over sodium sulfate, filtered and dried under reduced pressure to afford a beige solid. DCM was added to the solid. The precipitate was filtered and the filtrate was purified on preparative TLC using cyclohexane/ ethyl acetate; 50/ 50 as eluent. The resulting product was purified a second time on preparative TLC using cyclohexane/ ethyl acetate ; 50/ 50 as eluent to give 4-bromo-8,14-dioxa-10,19,20-triazatetracyclo [13.5.2.1^{2,6}.0^{18,21}]tricosa-1(20),2,4,6(23),15,17,21-heptaen-9-one **example 9** as a beige solid.
LCMS method F: [M+H]⁺ = 403, t_{R} = 2.40 min
LCMS method G: [M+H]⁺ = 403, t_{R} = 2.38 min
¹H NMR (400 MHz, d6-DMSO) δ 13.07 (1H, s), 8.02 (1H, s), 7.87 (1H, s), 7.74 (1H, s), 7.51 (2H, q, J = 2.8 Hz), 7.32 (1H, d, J = 2.7 Hz), 7.00 (1H, dd, J = 2.3, 8.9 Hz), 5.29 (2H, m), 4.32 (2H, m), 3.18 (2H, m, J = 8.1 Hz), 2.03 (2H, m) ppm.

### Example 10: 5-fluoro-8,14-dioxa-10,19,20-triazatetracyclo[13.5.2.1^{2,6}.0^{18,21}]tricosa-1(20), 2,4,6(23),15,17,21-heptaen-9-one

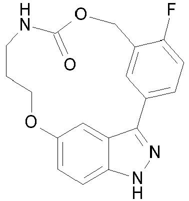

Example 10 is prepared according to the synthesis route described in general Scheme E.

### Preparation of intermediate 35: 2-fluoro-5-[5-hydroxy-1-(oxan-2-yl)-1H-indazol-3-yl] benzoic acid

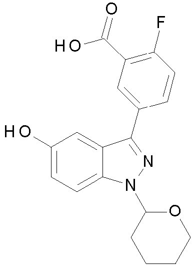

To solution of 3-iodo-1-(oxan-2-yl)-1H-indazol-5-ol **4** (1 g, 2.90 mmol), 2-fluoro-5-(tetramethyl-1,3,2-dioxaborolan-2-yl)benzoic acid (0.925 g, 2.52 mmol) in dioxane/water; 70/30 (12 mL) was added tripotassium phosphate (1.84 g, 8.7 mmol). The mixture was degassed by bubbling nitrogen for 15 minutes. Xphos (0.138 g, 0.29 mmol) and palladium-tetrakis(triphenylphosphine) (0.167 g, 0.145 mmol) were added. The mixture was heated at 120°C for 2 hours under microwaves irradiations (BIOTAGE). The reaction mixture was filtered over celite pad and eluted with ethyl acetate. The solution was washed with water (50 mL) and with brine (50 mL). The organic layer was dried with sodium sulfate and the solvent was removed under reduced pressure to afford a brown oil. Purification on silica column on Biotage using cyclohexane/ethyl acetate from 100/0 to 20/80 as eluent gave 2-fluoro-5-[5-hydroxy-1-(oxan-2-yl)-1H-indazol-3-yl]benzoic acid 35 as a white powder.
LCMS method F: [M+H]⁺ = 357.1, t_{R} = 2.34 min

### Preparation of intermediate 36: 3-[4-fluoro-3-(hydroxymethyl)phenyl]-1-(oxan-2-yl)-1H-indazol-5-ol

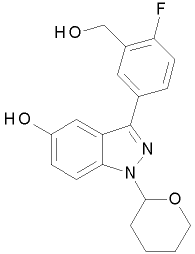

To a solution 2-fluoro-5-[5-hydroxy-1-(oxan-2-yl)-1H-indazol-3-yl]benzoic acid 35 (0.2 g, 0.56 mmol) in THF (25 mL) was added solid sodium borohydride (0.062 g, 1.68 mmol) at RT. After the end of the gas release (i.e. 5 min), the reaction mixture was cooled to 0 °C and neat boron trifluoride diethyl etherate (0.163 mL, 1.68 mmol) was added dropwise over 1 h. The reaction mixture was allowed to warm to RT and stirred at 65 °C for 2 h. The reaction mixture was cooled to 0 °C and an aqueous 1 M solution of sodium hydroxide (50 mL) was added. The mixture was stirred at RT for 2 h. The reaction mixture was filtered over celite and eluted with ethyl acetate. The solution was washed with water (50 mL) and with brine (50 mL). The organic layer was dried with sodium sulfate and the solvent was removed under reduced pressure to afford a brown oil. Purification (Biotage) on silica column using cyclohexane/ethyl acetate from 100/00 to 50/50 as eluent 3-[4-fluoro-3-(hydroxymethyl)phenyl]-1-(oxan-2-yl)-1H-indazol-5-ol **36** as a white powder.
LCMS method F: [M+H]⁺ = 343.1, t_{R} = 2.27 min

### Preparation of intermediate 37: benzyl N-[3-({3-[4-fluoro-3-(hydroxymethyl)phenyl]-1-(oxan-2-yl)-1H-indazol-5-yl}oxy)propyl]carbamate

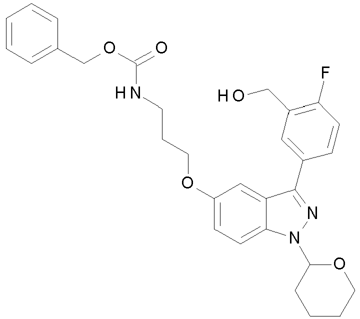

To a solution 3-[4-fluoro-3-(hydroxymethyl)phenyl]-1-(oxan-2-yl)-1H-indazol-5-ol 36 (0.18 g, 0.52 mmol) in N,N-dimethylformamide (10 mL), cesium carbonate (0.338 g, 1.04 mmol) and tert-butyl 3-[(methanesulfonyloxy)methyl]pyrrolidine-1-carboxylate **20** (0.169 gr, 0.624 mmol) was added. The reaction was stirred at 80°C for 16 hours. The mixture was concentrated under reduced pressure. Water (50 mL) was added and the resulting mixture was extracted with EtOAc (4 x 100 mL). Combined organic layers were washed with saturated brine (2 x 50 mL). The organic layer was dried over sodium sulfate and the solvent was removed under reduced pressure to afford brown/orange oil. The residue was purified by flash chromatography on silica gel (Macherey Nagel, 12 g) with gradient elution : cyclohexane/EtOAc 0-70 % to give benzyl N-[3-({3-[4-fluoro-3-(hydroxymethyl)phenyl]-1-(oxan-2-yl)-1H-indazol-5-yl}oxy)propyl] carbamate **37** as a white solid.
LCMS method F: [M+H]⁺ = 534.2, t_{R} = 2.90 min

### Preparation of intermediate 38: 5-fluoro-19-(oxan-2-yl)-8,14-dioxa-10,19,20-triazatetra cyclo[13.5.2.1^{2,6}.0^{18,21}]tricosa-1(20),2,4,6(23),15,17,21-heptaen-9-one

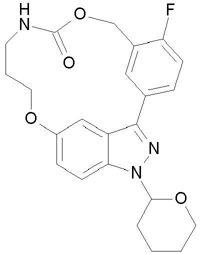

A solution of benzyl N-[3-({3-[4-fluoro-3-(hydroxymethyl)phenyl]-1-(oxan-2-yl)-1H-indazol-5-yl}oxy)propyl]carbamate **37** (0.153 g; 0.28 mmol) in 50 mL of toluene was added to a solution of sodium hydride (60% suspension in paraffin oil) (114 mg, 24 mmol) in 50 mL of toluene at room temperature. The reaction mixture was stirred at RT for 5 min and then one hour at 130°C. The reaction is allowed to cool down and then 10 mL of EtOH is added carefully. 100 mL of water in added. After separation, the aqueous layer was extracted with ethyl acetate (2 x 100 mL). The combined organic layers were washed with a saturated brine, dried over sodium sulfate and the solvent was removed under reduced pressure to give an orange oil. A purification by column chromatography (DCM/MeOH 0-10 %) afforded pure 5-fluoro-19-(oxan-2-yl)-8,14-dioxa-10,19,20-triazatetracyclo[13.5.2.1^{2,6}.0^{18,21}]tricosa-1(20),2,4,6(23),15, 17,21-heptaen-9-one 38 as a whitish solid.
LCMS method F: [M+H]⁺ = 426.2, t_{R} = 2.78 min

### Preparation of Example 10: 5-fluoro-8,14-dioxa-10,19,20-triazatetracyclo [13.5.2.1^{2,6}.0^{18,21}]tricosa-1(20), 2,4,6(23),15,17,21-heptaen-9-one

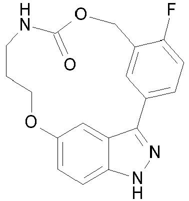

To a solution of 5-fluoro-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **38** (35 mg, 0.082 mmol) in DCM (5 mL) was added trifluoroacetic acid (63 µL, 0.82 mmol). The reaction mixture was stirred at RT for 6 h and at 30 °C overnight. More trifluoroacetic acid (32 µL ,0.41 mmol) was added and the reaction mixture was stirred at 50 °C for 5h. Again more trifluoroacetic acid (32 µL, 0.41 mmol) was added and the reaction mixture was stirred at 50 °C for another 2 h. The reaction mixture was evaporated to dryness and co-evaporated with toluene. DCM (40 mL), water (125 mL) and ammonium hydroxide 25 % weight aqueous solution (3 mL) were added. After separation, the aqueous layer was extracted with DCM (3x 20 mL). The combined organic layers were washed with saturated sodium carbonate solution (100 mL) and brine (100 mL), dried over sodium sulfate anhydrous and the solvent was removed under reduced pressure to afford a beige solid.

Trituration of the residue one time in acetonitrile, five times in DCM and two times in EtOH gave 5-fluoro-8,14-dioxa-10,19,20-triazatetracyclo[13.5.2.1^{2,6}.0^{18,21}]tricosa-1(20),2,4,6(23), 15,17,21-heptaen-9-one **example 10** as a white powder.
LCMS method F: [M+H]⁺ = 342.1, t_{R} = 2.18 min
LCMS method G: [M+H]⁺ = 342.1, t_{R} = 2.36 min
¹H NMR (400 MHz, d6-DMSO) δ 12.95 (1H, s), 7.93 (2H, m), 7.81 (1H, s), 7.89 (1H, d, J = 9.0 Hz), 7.33 (2H, m), 6.99 (1H, dd, J = 9.1 Hz), 5.35 (2H, s), 4.33 (2H, m), 3.19 (2H, m), 2.03 (2H, m) ppm.

### Example 11: 5-methyl-8,14-dioxa-10,19,20-triazatetracyclo[13.5.2.1^{2,6}.0^{18,21}]tricosa-1(20),2, 4,6(23),15,17,21-heptaen-9-one

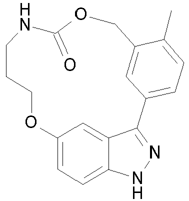

Example 11 is prepared according to the synthesis route described in general Scheme F.

### Preparation of intermediate 39: methyl 5-[5-[3-(benzyloxycarbonylamino)propoxy]-1-tetrahydropyran-2-yl-indazol-3-yl]-2-methyl-benzoate

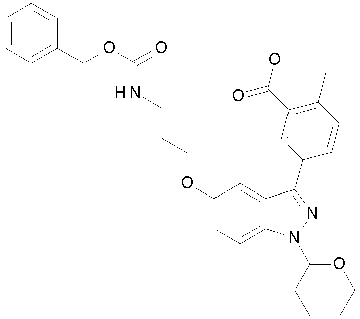

A solution of benzyl N-[3-(3-iodo-1-tetrahydropyran-2-yl-indazol-5-yl)oxypropyl]carbamate **26** (1.2 g, 2.2 mmol postulated), (3-methoxycarbonyl-4-methyl-phenyl)boronic acid (467 mg, 2.42 mmol), potassium phosphate tribasic (1.4 g, 6.6 mmol) and triethylamine (1.4 mL, 9.9 mmol) in THF/H₂O (6.5/3.2 mL) was degassed for 15 minutes. Pd(dppf)Cl₂.DCM (179 mg, 0.22 mmol) was added and the reaction mixture was stirred under nitrogen atmosphere at 100 °C for 17 hours. The reaction mixture was filtered over celite and washed with EtOAc. The filtrate was diluted with water (100 mL) and extracted with EtOAc (2 x 50 mL). The combined organic layers were washed with brine (2 x 50 mL), dried over sodium sulfate, filtered and the solvent was removed under reduced pressure. The residue was purified by column (Macherey Nagel, 40 g) chromatography with eluent cyclohexane/EtOAc (100/0 to 80/20). The desired fractions were collected and the solvent was removed under reduced pressure to give methyl 5-[5-[3-(benzyloxycarbonylamino)propoxy]-1-tetrahydropyran-2-yl-indazol-3-yl]-2-methylbenzoate **39** (1.04 g, 1.87 mmol) as a white solid.
LCMS method F: [M+H]⁺ = 558, t_{R} = 3.33 min

### Preparation of intermediate 40: benzyl N-[3-[3-[3-(hydroxymethyl)-4-methyl-phenyl]-1-tetrahydropyran-2-yl-indazol-5-yl]oxypropyl]carbamate

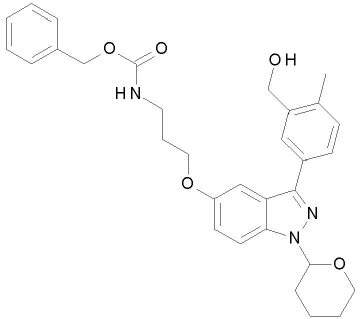

To a solution of methyl 5-[5-[3-(benzyloxycarbonylamino)propoxy]-1-tetrahydropyran-2-yl-indazol-3-yl]-2-methyl-benzoate **39** (1 g, 1.8 mmol) in THF (6 mL) under N₂, LAH 1 M in THF (2.2 mL, 2.2 mmol) was added at 0°C. The reaction was stirred at 0°C for 2 hours and 30 minutes. The mixture was quenched with water (1 mL), NaOH 10 % (0.2 mL) and water (0.5 mL). The mixture was filtered and washed with EtOAc. The filtrate was diluted with water (50 mL) and extracted with EtOAc (3 x 50 mL). The combined organic layers were washed with brine (2 x 50 mL), dried with anhydrous sodium sulfate and the solvent was removed under reduced pressure. The crude was purified by pad of silica with cyclohexane/EtOAc (60/40) as eluent to give benzyl N-[3-[3-[3-(hydroxymethyl)-4-methyl-phenyl]-1-tetrahydropyran-2-yl-indazol-5-yl]oxypropyl]carbamate **40** as a white oil.
LCMS method F: [M+H]⁺ = 530, t_{R} = 2.90 min

### Preparation of intermediate 41: 5-methyl-19-(oxan-2-yl)-8,14-dioxa-10,19,20-triazatetra cyclo[13.5.2.1^{2,6}.0^{18,21}]tricosa-1(20),2,4,6(23),15,17,21-heptaen-9-one

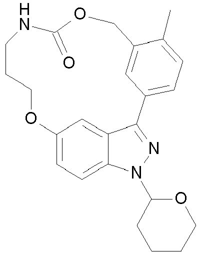

To a solution of benzyl N-[3-[3-[3-(hydroxymethyl)-4-methyl-phenyl]-1-tetrahydropyran-2-yl-indazol-5-yl]oxypropyl]carbamate **40** (120 mg, 0.23 mmol) in acetonitrile (40 mL), potassium carbonate (190 mg, 1.38 mmol) was added. The mixture was divided in two vials then heated in microwaves at 140 °C for 4 hours and 30 minutes. The two vials was heated again in microwaves at 140 °C for 4 hours. The mixture was filtered to removed potassium carbonate and the solvent was evaporated under reduced pressure to give 5-methyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one 41 as a white powder. The crude was used in the next step without further purification.
LCMS method F: [M+H]⁺ = 422, t_{R} = 2.87 min

### Preparation of Example 11: 5-methyl-8,14-dioxa-10,19,20-triazatetracyclo [13.5.2.1^{2,6}.0^{18,21}]tricosa-1(20),2, 4,6(23),15,17,21-heptaen-9-one

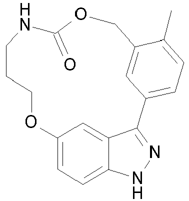

To a solution of 5-methyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **41** (84 mg, 0.2 mmol) in DCM (15 mL) was added trifluoro acetic acid (306 µL, 4 mmol). The mixture was heated in microwaves at 80 °C for 1 hour. The solvent was removed under reduced pressure to afford an oily residue, which was dissolved in DCM (20 mL). A precipitate was formed and filtered. The solid was dissolved in DCM/MeOH (15 mL), then NaHCO₃ saturated was added (15 mL). After separation, the aqueous layer was extracted with DCM (3 x 10 mL). The combined organic layers were washed with brine (20 mL), dried over anhydrous sodium sulfate, filtered and the solvent was removed under reduced pressure to give 5-methyl-8,14-dioxa-10,19,20-triazatetracyclo[13.5.2.1^{2,6}.0^{18,21}]tricosa-1(20),2,4,6(23),15,17,21-heptaen-9-one **example 11** as a white solid.
LCMS method F: [M+H]⁺ = 338, t_{R} = 2.32 min
LCMS method G: [M+H]⁺ = 338, t_{R} = 2.35 min
¹H NMR (400 MHz, d6-DMSO) δ 12.86 - 12.79 (1H, m), 7.84 (1H, m), 7.82 (1H, m), 7.74 (1H, s), 7.46 (1 H, d, J = 8.9 Hz), 7.42 (1H, m), 7.28 (1H, dd, J = 0.6, 8.3 Hz), 6.98 (1H, dd, J = 2.4, 9.0 Hz), 5.28 (2H, s), 4.34 (2H, dd, J = 8.2, 8.5 Hz), 3.2 (2H, m), 2.32 (3H, s), 2.04 - 1.99 (2H, m) ppm.

### Example 12: 4-(pyrrolidin-1-yl)-8,14-dioxa-10,19,20-triazatetracyclo[13.5.2.1^{2,6}.0^{18,21}] tricosa-1(20),2,4,6(23),15,17,21-heptaen-9-one

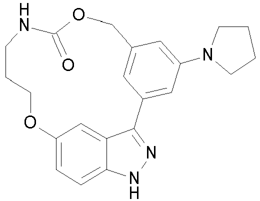

Example 12 is prepared according to the synthesis route described in general Scheme C. Pyrrolidine is used for the Buchwald reaction with the bromide intermediate **34.**

### Preparation of intermediate 42: 19-(oxan-2-yl)-4-(pyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one

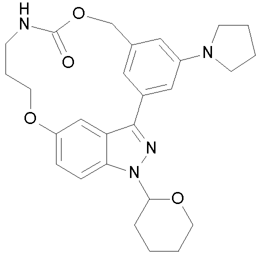

To a degassed solution of 4-bromo-10-methyl-19-(oxan-2-yl)-7-oxa-10,13,19,20-tetraazatetracyclo[13.5.2.12,6.018,21]tricosa-1(20),2,4,6(23),15(22),16,18(21)-heptaen-14-one **example 9** (100 mg, 0.206 mmol), pyrrolidine (19 µl, 0.227 mmol), tBuONa (40 mg, 0.412 mmol) and SPhos (3 mg, 0.008 mmol) in dioxane (2.5 mL) was added Pd₂dba₃ (4 mg, 0.004 mmol) at RT. The reaction mixture was stirred under microwave irradiation for 45 at 60°C. More pyrrolidine (2 µl; 0.021 mmol) was added and the reaction was stirred under microwave irradiation during 20 min at 60°C. After being cooled to RT, the reaction mixture was diluted with water and extracted with ethyl acetate twice. The combined organic layer was washed with brine, dried over anhydrous sodium sulfate and concentrated under reduced pressure. The residue was purified by flash-column (5g SiO₂) chromatography (cyclohexane/Ethyl acetate, 1:0 to 50/50) affording 19-(oxan-2-yl)-4-(pyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one **42** as a white powder.
LCMS method F: [M+H]⁺ = 477.2, t_{R} = 3.00 min

### Preparation of Example 12: 4-(pyrrolidin-1-yl)-8,14-dioxa-10,19,20-triazatetracyclo [13.5.2.1^{2,6}.0^{18,21}] tricosa-1(20),2,4,6(23),15,17,21-heptaen-9-one

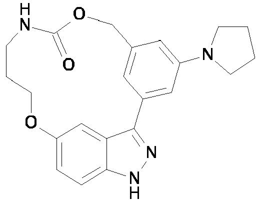

To a mixture of 19-(oxan-2-yl)-4-(pyrrolidin-1-yl)-8,14-dioxa-10,19,20-triazatetracyclo [13.5.2.1^{2,6}.0^{18,21}]tricosa-1(20),2,4,6(23),15,17,21-heptaen-9-one **42** (60mg; 0.126mmol) in DCM (2.5mL) was added TFA (48µl; 0.630mmol). The reaction mixture was stirred under microwaves irradiation at 80°C during 30 min. The solvent was removed under reduced pressure, the mixture was dissolved in EtOAc and washed with 1N NaOH (pH=7), then with water. The organic layer was concentrated under reduced pressure and the product was purified by chromatography using a 4 g SiO2 column eluted with DCM/MeOH 100/0 to 90/10. The desired fractions were combined to give 4-(pyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one **example 12** as a yellow powder.
LCMS method F: [M+H]⁺ = 393.1, t_{R} = 2.39 min (current 20V)
LCMS method G: [M+H]⁺ = 393.1, t_{R} = 2.47 min (pH10 current 20V)
¹H NMR (400 MHz, *d*6-DMSO) δ 7.61 (1H, m), 7.47 - 7.44 (1H, m), 7.36 (1H, d, J = 2.7 Hz), 7.20 (1H, s), 7.04 (1H, t, J = 1.9 Hz), 6.95 (1H, dd, J = 2.4, 9.0 Hz), 6.50 (1H, s), 5.22 - 5.20 (2H, m), 4.30 (2H, d, J = 16.9 Hz),3.32 (4H, m), 3.17 - 3.15 (2H, m), 2.03 - 1.99 (6H, m), 1.07 (1H, d, J = 6.1 Hz) ppm.

### Example 13: 4-[4-(propan-2-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one

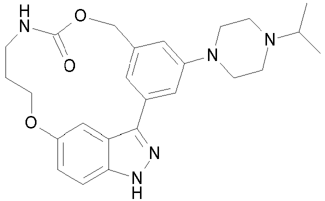

Example 13 is prepared according to the synthesis route described in general Scheme C and procedures analogous to those used to obtain **example 12.** 1-(propan-2-yl)piperazine is used for the Buchwald reaction with the bromide intermediate 34 to give 4-[4-(propan-2-yl)piperazin-1-yl]-8,14-dioxa-10,19,20-triazatetracyclo[13.5.2.1^{2,6}.0^{18,21}]tricosa-1(20),2,4,6(23),15,17,21-heptaen-9-one **example 13.**
LCMS method F: [M+H]⁺ = 450.2, t_{R} = 1.54 min
LCMS method G: [M+H]⁺ = 450.2, t_{R} = 2.26 min
¹H NMR (400 MHz, d6-DMSO, 80°C) δ 12.80 (1H, s), 7.68 - 7.57 (1H, m), 7.46 (1H, d, J = 9.3 Hz), 7.37 - 7.34 (3H, m), 6.96 (1H, dd, J = 2.4, 8.8 Hz), 6.87 (1H, s), 5.23 (2H, s), 4.28 (2H, s), 3.25 - 3.22 (4H, m), 3.17 (2H, s), 2.76 - 2.67 (1H, m), 2.66 - 2.61 (4H, m), 2.02 (2H, s), 1.05 (6H, d, J = 6.5 Hz) ppm.

### Example 14: 4-{2-oxa-6-azaspiro[3.4]octan-6-yl}-8,14-dioxa-10,19,20-triazatetracyclo [13.5.2.1^{2,6}.0^{18,21}]tricosa-1(20),2,4,6(23),15,17,21-heptaen-9-one

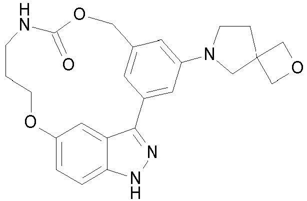

Example 14 is prepared according to the synthesis route described in general Scheme C and procedures analogous to those used to obtain **example 12.** 2-Oxa-6-azaspiro[3.4]octane is used for the Buchwald reaction with the bromide intermediate **34** to give 4-{2-oxa-6-azaspiro[3.4]octan-6-yl}-8,14-dioxa-10,19,20-triazatetracyclo[13.5.2.1^{2,6}.0^{18,21}]tricosa-1(20),2,4,6(23),15,17,21-heptaen-9-one **example 14.**
LCMS method F: [M+H]⁺ = 435, t_{R} = 2.16 min
LCMS method G: [M+H]⁺ = 435, t_{R} = 2.20 min
¹H NMR (400 MHz, *d*6-DMSO, 80°C) δ 12.77 (1H, s), 7.61 (1H, m), 7.46 (1H, d, J = 9.2 Hz), 7.36 (1H, m), 7.22 (1H, m), 7.04 (1H, m), 6.96 (1H, m), 6.51 (1H, m), 5.22 (2H, m), 4.64 - 4.56 (4H, m), 4.30 (2H, m), 3.60 (2H, s), 3.35 (2H, t), 3.16 (2H, m), 2.31 (2H, m), 2.02 (2H, m) ppm.

### Example 15: 4-[4-(oxetan-3-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one

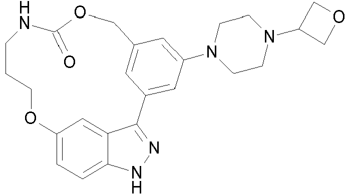

Example 15 is prepared according to the synthesis route described in general Scheme C and and procedures analogous to those used to obtain **example 12.** 1-(oxetan-3-yl)piperazine is used for the Buchwald reaction with the bromide intermediate **34** to give 4-[4-(oxetan-3-yl)piperazin-1-yl]-8,14-dioxa-10,19,20-triazatetracyclo[13.5.2.1^{2,6}.0^{18,21}]tricosa-1(20),2,4, 6(23),15,17,21-heptaen-9-one **example 15.**
LCMS method F: [M+H]⁺ = 464.2, t_{R} = 1.47 min
LCMS method G: [M+H]⁺ = 464.2, t_{R} = 2.00 min
¹H NMR (400 MHz, *d*6-DMSO, 80°C) δ 12.81 (1H, s), 7.64 (1H, s), 7.48 - 7.45 (1H, d, J = 9.0 Hz), 7.39 - 7.34 (3H, m), 6.96 (1H, dd, J = 2.2, 8.8 Hz), 6.89 (1H, m), 5.23 (2H, s), 4.62 - 4.57 (2H, t, J = 6.5 Hz), 4.55 - 4.51 (2H, m), 4.33 - 4.27 (2H, t, J = 8.6 Hz), 3.58 - 3.51 (1H, q, J = 6.2 Hz), 3.30 - 3.26 (4H, m), 3.17 - 3.11 (2H, m), 2.10 - 1.99 (2H, m) ppm. 4 protons were located under the DMSO peak and are not reported here.

### Example 16: 4-(morpholin-4-yl)-8,14-dioxa-10,19,20-triazatetracyclo[13.5.2.1^{2,6}.0^{18,21}] tricosa-1(20),2,4,6(23),15,17,21-heptaen-9-one

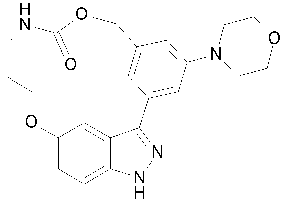

Example 16 is prepared according to the synthesis route described in general Scheme C and procedures analogous to those used to obtain **example 12.** Morpholine is used for the Buchwald reaction with the bromide intermediate **34** to give 4-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one **example 16.**
LCMS method F: [M+H]⁺ = 409.2, t_{R} = 2.13 min
LCMS method G: [M+H]⁺ = 409.2, t_{R} = 2.15 min
¹H NMR (400 MHz, *d*6-DMSO, 80°C) δ 12.82 (1H, s), 7.63 (1H, m), 7.48 - 7.45 (1H, d, J = 9.0 Hz), 7.40 (2H, m), 7.34 (1H, m), 6.97 (1H, dd, J = 2.3, 8.9 Hz), 6.89 (1H, s), 5.23 (2H, s), 4.33 - 4.28 (2H, t, J = 8.32), 3.82 - 3.76 (4H, t, J = 4.8 Hz), 3.23 - 3.20 (4H, t, J = 4.9 Hz), 3.17 (2H, s), 2.02 (2H, s) ppm.

### Example 17: 4-[(2R,6S)-2,6-dimethylmorpholin-4-yl]-8,14-dioxa-10,19,20-triazatetra cyclo[13.5.2.1^{2,6}.0^{18,21}]tricosa-1(20),2,4,6(23),15,17,21-heptaen-9-one

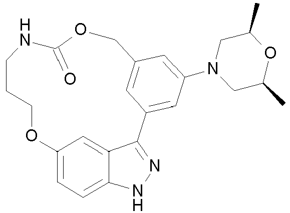

Example 17 is prepared according to the synthesis route described in general Scheme C and procedures analogous to those used to obtain **example 12.** Cis-2,6-dimethylmorpholine is used for the Buchwald reaction with the bromide intermediate **34** to give 4-[(2R,6S)-2,6-dimethylmorpholin-4-yl]-8,14-dioxa-10,19,20-triazatetracyclo[13.5.2.1^{2,6}.0^{18,21}]tricosa-1(20), 2,4,6(23),15,17,21-heptaen-9-one **example 17.**
LCMS method F: [M+H]⁺ = 437.1, t_{R} = 2.30 min
LCMS method G: [M+H]⁺ = 437.2, t_{R} = 2.36 min
¹H NMR (400 MHz, *d*6-DMSO, 80°C) δ 12.81 (1H, s), 7.63 (1H, s), 7.48 - 7.45 (1H, m), 7.39 - 7.34 (3H, m), 6.98 - 6.90 (2H, m), 5.23 (2H, s), 4.30 (2H, m), 3.80 - 3.73 (2H, m), 3.64 (2H, dd, J = 1.5, 12.1 Hz), 3.17 (2H, s), 2.41 - 2.35 (2H, m), 2.06 - 2.05 (2H, m), 1.21 (6H, d, J = 6.3 Hz) ppm.

### Example 18: 4-methyl-8,14-dioxa-10,19,20-triazatetracyclo[13.5.2.1^{2,6}.0^{18,21}]tricosa-1(20),2,4,6(23),15,17,21-heptaen-9-one

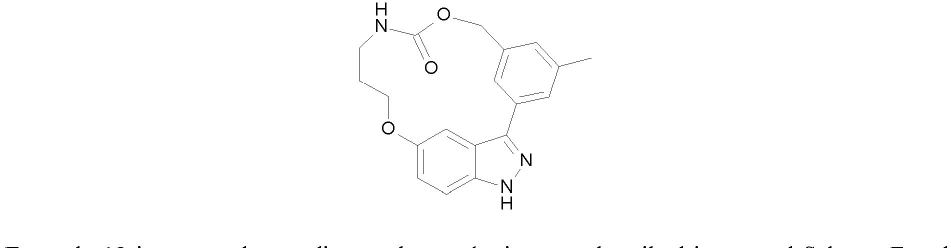

Example 18 is prepared according to the synthesis route described in general Scheme F and procedures analogous to those used to obtain **example 11.** (3-Methoxycarbonyl-5-methylphenyl)boronic acid is used for the Suzuki coupling with intermediate **26** to give 4-methyl-8,14-dioxa-10,19,20-triazatetracyclo[13.5.2.1^{2,6}.0^{18,21}]tricosa-1(20),2,4,6(23),15,17,21-heptaen-9-one **example 18.**
LCMS method F: [M+H]⁺ = 338, t_{R} = 2.25 min
LCMS method G: [M+H]⁺ = 338, t_{R} = 2.30 min
¹H NMR (400 MHz, *d*6-DMSO) δ 7.73 - 7.65 (3H, m), 7.49 - 7.45 (1H, m), 7.34 (1H, d, J = 2.1 Hz), 7.10 - 7.07 (1H, m), 6.97 (1H, dd, J = 2.2, 9.0 Hz), 5.26 - 5.25 (2H, m), 4.34 - 4.28 (2H, m), 3.17 (2H, m), 2.41 (3H, s), 2.04 - 2.01 (2H, m) ppm. The indazole NH proton was not visible in this solvent.

### Example 19: 5-methoxy-8,14-dioxa-10,19,20-triazatetracyclo[13.5.2.1^{2,6}.0^{18,21}]tricosa-1(20),2,4,6(23),15,17,21-heptaen-9-one

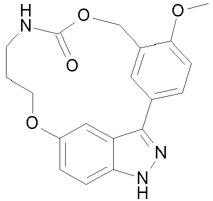

Example 19 is prepared according to the synthesis route described in general Scheme F and procedures analogous to those used to obtain **example 11.** (4-Methoxy-3-methoxycarbonyl-phenyl)boronic acid is used for the Suzuki coupling with intermediate **26** to give 5-methoxy-8,14-dioxa-10,19,20-triazatetracyclo[13.5.2.1^{2,6}.0^{18,21}]tricosa-1(20),2,4,6(23),15,17,21-heptaen-9-one **example 19.**
LCMS method F: [M+H]⁺ = 354, t_{R} = 2.19 min
LCMS method G: [M+H]⁺ = 354, t_{R} = 2.17 min
¹H NMR (400 MHz, *d*6-DMSO) δ 12.75 (1H, s), 7.91 (1H, dd, J = 2.2, 8.6 Hz), 7.83 (1H, m), 7.72 (1H, m), 7.45 (1H, d, J = 8.9 Hz), 7.37 (1H, d, J = 2.2 Hz), 7.15 (1H, d, J = 8.5 Hz), 6.97 (1H, dd, J = 2.4, 9.0 Hz), 5.26 (2H, s), 4.33 (2H, m), 3.90 (3H, s), 3.18 (2H, m), 2.02 (2H, m) ppm.

### Example 20: 4-(4,4-difluoropiperidin-1-yl)-8,14-dioxa-10,19,20-triazatetracyclo [13.5.2.1^{2,6}.0^{18,21}]tricosa-1(20),2,4,6(23),15,17,21-heptaen-9-one

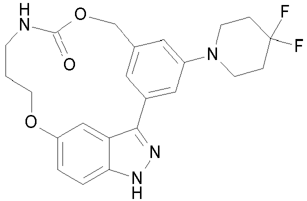

Example 20 is prepared according to the synthesis route described in general Scheme C and procedures analogous to those used to obtain **example 12.** 4,4-Difluoropiperidine is used for the Buchwald reaction with the bromide intermediate **34** to give 4-(4,4-difluoropiperidin-1-yl)-8,14-dioxa-10,19,20-triazatetracyclo[13.5.2.1^{2,6}.0^{18,21}]tricosa-1(20),2,4,6(23),15,17,21-heptaen-9-one **example 20.**
LCMS method F: [M+H]⁺ = 443.1, t_{R} = 2.46 min
LCMS method G: [M+H]⁺ = 443.1, t_{R} = 2.49 min
¹H NMR (400 MHz, d6-DMSO, 80°C) δ 12.83 (1H, s), 7.64 (1H, s), 7.49 - 7.43 (2H, m), 7.39 (1H, s), 7.36 - 7.33 (1H, m), 5.24 (2H, s), 4.36 - 4.27 (2H, m), 3.46 - 3.42 (4H, m), 3.17 (4H, s), 2.17 - 1.98 (6H, m) ppm.

### Example 21: 4-(3,3-difluoropyrrolidin-1-yl)-8,14-dioxa-10,19,20-triazatetracyclo [13.5.2.1^{2,6}.0^{18,21}]tricosa-1(20),2,4,6(23),15,17,21-heptaen-9-one

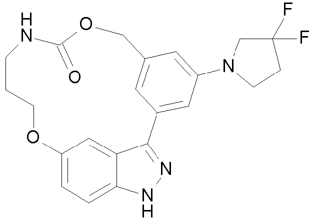

Example 21 is prepared according to the synthesis route described in general Scheme C and procedures analogous to those used to obtain **example 12.** 3,3-Difluoropyrrolidine is used for the Buchwald reaction with the bromide intermediate **34** to give 4-(3,3-difluoropyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one **example 21.**
LCMS method F: [M+H]⁺ = 429.1, t_{R} = 2.46 min
LCMS method G: [M+H]⁺ = 429.1, t_{R} = 2.48 min
¹H NMR (400 MHz, *d*6-DMSO, 80°C) δ 12.82 (1H, s), 7.63 (1H, s,), 7.47 (1H, d, J = 8.9 Hz), 7.36 - 7.31 (2H, m), 7.10 - 7.08 (1H, m), 6.96 (1H, dd, J = 2.4, 9.0 Hz), 6.59 - 6.58 (1H, m), 5.23 (2H, s), 4.33 - 4.27 (2H, m), 3.37 (2H, t, J = 13.7 Hz), 3.58 (2H, t, J = 7.2 Hz), 3.16 (2H, s), 2.63 - 2.53 (2H, m) 2.02 (2H, m) ppm.

### Example 22: 7-methyl-8,14-dioxa-10,19,20-triazatetracyclo[13.5.2.1^{2,6}.0^{18,21}]tricosa-1(20), 2,4,6(23),15,17,21-heptaen-9-one

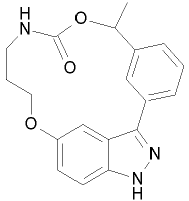

Example 22 is prepared according to the synthesis route described in general Scheme C and procedures analogous to those used to obtain **example 8.** (3-(1-Hydroxyethyl)phenyl)boronic acid is used for the Suzuki coupling to give 7-methyl-8,14-dioxa-10,19,20-triazatetracyclo[13.5.2.1^{2,6}.0^{18,21}]tricosa-1(20),2,4,6(23),15,17,21-heptaen-9-one **example 22.**
LCMS method F: [M+H]⁺ = 338, t_{R} = 2.22 min
LCMS method G: [M+H]⁺ = 338, t_{R} = 2.25 min
¹H NMR (400 MHz, *d*6-DMSO) δ 13.12 (1H, s), 7.95 - 7.92 (1H, m), 7.86 - 7.83 (2H, m), 7.50 - 7.46 (2H, m), 7.35 (1H, m), 7.31 - 7.29 (1H, m), 7.00 - 6.97 (1H, m), 5.95 - 5.90 (1H, m), 4.37 - 4.25 (2H, m), 3.56 - 3.49 (1H, m), 2.77 - 2.68 (1H, m), 2.24 - 2.15 (1H, m), 1.77 - 1.69 (1H, m), 1.59 (3H, d, J = 6.7 Hz) ppm.

### Example 23: 4-[4-(2-methoxyethyl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one

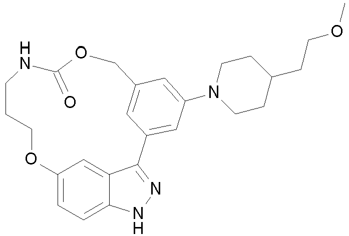

Example 23 is prepared according to the synthesis route described in general Scheme C and procedures analogous to those used to obtain **example 12.** 4-(2-Methoxyethyl)piperidine is used for the Buchwald reaction with the bromide intermediate **34** to give 4-[4-(2-methoxyethyl)piperidin-1-yl]-8,14-dioxa-10,19,20-triazatetracyclo[13.5.2.1^{2,6}.0^{18,21}]tricosa-1(20),2,4,6(23),15,17,21-heptaen-9-one **example 23.**
LCMS method F: [M+H]⁺ = 465.2, t_{R} = 1.81 min
LCMS method G: [M+H]⁺ = 465.2, t_{R} = 2.53 min
¹H NMR (400 MHz, *d*6-DMSO, 80 °C) δ 12.79 (1H, br. s), 7.63 - 7.59 (1H, m), 7.46 (1H, d, *J* = 9.2 Hz), 7.37 (1H, d, *J=* 2.0 Hz), 7.34 (1H, d, *J =* 2.0 Hz), 7.32 (1H, s), 6.96 (1H, dd, *J =* 2.3, 8.9 Hz), 6.86 (1H, s), 5.22 - 5.19 (2H, m), 4.33 - 4.28 (2H, m), 3.77 - 3.73 (2H, m), 3.43 (2H, t, *J =* 8.0 Hz), 3.21 - 3.16 (2H, m), 3.09 - 3.06 (3H, br. s), 2.78 (2H, dt, *J=* 4.0, 11.2 Hz), 2.05 - 2.01 (2H, m), 1.82 - 1.77 (2H, m), 1.58 - 1.49 (3H, m), 1.38 - 1.27 (2H, m) ppm.

### Example 24: 9,14-dioxa-11,19,20-triazatetracyclo[13.5.2.1^{2,6}.0^{18,21}]tricosa-1(20),2,4, 6(23),15,17,21-heptaen-10-one

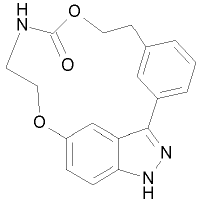

Example 24 is prepared according to the synthesis route described in general Scheme C and procedures analogous to those used to obtain **example 8.** 2-[3-(4,4,5,5-tetramethyl-1,3,2-dioxaborolan-2-yl)phenyl]ethan-1-ol is used for the Suzuki coupling to give 9,14-dioxa-11,19,20-triazatetracyclo[13.5.2.1^{2,6}.0^{18,21}]tricosa-1(20),2,4,6(23),15,17,21-heptaen-10-one **example 24.**
LCMS method F: [M+H]⁺ = 324.1, t_{R} = 2.14 min
LCMS method G: [M+H]⁺ = 324.1, t_{R} = 2.19 min
¹H NMR (400 MHz, *d*6-DMSO) δ 13.05 - 13.03 (1H, m), 7.99 (1H, t, J = 5.9 Hz), 7.82 (1H, s), 7.68 (1H, d, J = 7.6 Hz), 7.58 (1H, d, J = 1.9 Hz), 7.46 - 7.41 (2H, m), 7.28 - 7.25 (1H, m), 7.04 (1H, dd, J = 2.2, 9.0 Hz), 4.33 - 4.21 (4H, m), 3.40 - 3.3 (2H, m), 3.01 (2H, t, J = 5.0 Hz) ppm.

### Example 25: 4-[(3R)-3-hydroxypyrrolidin-1-yl]-8,14-dioxa-10,19,20-triazatetracyclo [13.5.2.1^{2,6}.0^{18,21}]tricosa-1(20),2,4,6(23),15,17,21-heptaen-9-one

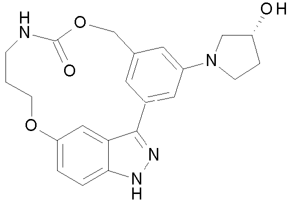

Example 25 is prepared according to the synthesis route described in general Scheme C and procedures analogous to those used to obtain **example 12.** (3R)-Pyrrolidin-3-ol is used for the Buchwald reaction with the bromide intermediate **34** to give 4-[(3R)-3-hydroxypyrrolidin-1-yl]-8,14-dioxa-10,19,20-triazatetracyclo[13.5.2.1^{2,6}.0^{18,21}]tricosa-1(20),2,4,6(23),15,17,21-heptaen-9-one **example 25.**
LCMS method F: [M+H]⁺ = 409.1, t_{R} = 1.96 min
LCMS method G: [M+H]⁺ = 409.2, t_{R} = 2.04 min
¹H NMR (400 MHz, *d*6-DMSO) δ 7.65- 7.56 (1H, m), 7.48 - 7.45 (1H, m), 7.36 (1H, d, J = 2.5 Hz), 7.19 (1H, s), 7.01 (1H, s), 6.93 (1H, dd, J = 2.3, 9.1 Hz), 6.46 (1H, s), 5.22 (2H, s), 4.48 - 4.43 (1H, m), 4.32 - 4.27 (2H, m), 3.53 - 3.32 (3H, m), 3.18- 3.13 (2H, m), 3.11- 2.99 (2H, m), 2.15 - 2.07 (1H, m), 2.07 -1.97 (2H, m), 1.97 - 1.92 (1H, m) ppm. The indazole NH proton was not visible in this solvent.

### Example 26: 4-[(2-methoxyethyl)(methyl)amino]-8,14-dioxa-10,19,20-triazatetracyclo [13.5.2.1^{2,6}.0^{18,21}]tricosa-1(20),2,4,6(23),15,17,21-heptaen-9-one

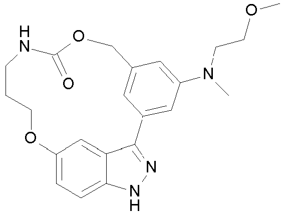

Example 26 is prepared according to the synthesis route described in general Scheme C and procedures analogous to those used to obtain **example 12.** 2-Methoxy-N-methyl-ethanamine is used for the Buchwald reaction with the bromide intermediate **34** to give 4-[(2-methoxyethyl)(methyl)amino]-8,14-dioxa-10,19,20-triazatetracyclo[13.5.2.1^{2,6}.0^{18,21}]tricosa-1(20),2,4,6(23),15,17,21-heptaen-9-one **example 26.**
LCMS method F: [M+H]⁺ = 411.2, t_{R} = 2.07 min
LCMS method G: [M+H]⁺ = 411.2, t_{R} = 2.32 min
¹H NMR (400 MHz, *d*6-DMSO) δ 12.74 (1H, s), 7.48 - 7.45 (1H, m), 7.36 (1H, d, J = 2.3 Hz), 7.23 - 7.19 (2H, m), 6.96 (1H, dd, J = 2.4, 9.0 Hz), 6.67 (1H, dd, J = 1.3, 2.5 Hz), 5.22 (1H, t, J = 9.7 Hz), 4.30 (2H, d, J = 16.7 Hz), 3.31 - 3.31 (3H, m), 3.11 - 3.04 (8H, s), 3.01 (3H, s), 2.01 - 2.02 (2H, m) ppm.

### Example 27: 4-chloro-8,14-dioxa-10,19,20-triazatetracyclo[13.5.2.1^{2,6}.0^{18,21}]tricosa-1(20), 2,4,6(23),15,17,21-heptaen-9-one

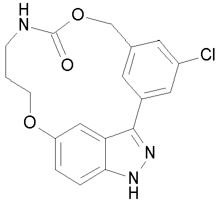

Example 27 is prepared according to the synthesis route described in general Scheme F and procedures analogous to those used to obtain **example 11.** (3-Chloro-5-methoxycarbonyl-phenyl)boronic acid is used for the Suzuki coupling with intermediate **26** to give 4-chloro-8,14-dioxa-10,19,20-triazatetracyclo[13.5.2.1^{2,6}.0^{18,21}]tricosa-1(20),2,4,6(23),15,17,21-heptaen-9-one **example 27.**
LCMS method F: [M+H]⁺ = 358.0, t_{R} = 2.38 min
LCMS method G: [M+H]⁺ = 358.1, t_{R} = 2.52 min
¹H NMR (400 MHz, *d*6-DMSO, 80 °C) δ 13.08 (1H, s), 7.85 (2H, d, *J =* 15.0 Hz), 7.77 - 7.75 (1H, m), 7.50 (1H, d, *J* = 8.0 Hz), 7.36 (1H, s), 7.32 (1H, d, *J* = 2.4 Hz), 7.00 (1H, dd, *J* = 2.3, 8.9 Hz), 5.29 - 5.25 (2H, m), 4.35 - 4.30 (2H, m), 3.23 - 3.12 (2H, m), 2.06 - 2.00 (2H, m) ppm.

### Example 28: 4-fluoro-5-methyl-8,14-dioxa-10,19,20-triazatetracyclo[13.5.2.1^{2,6}.0^{18,21}] tricosa-1(20),2,4,6(23),15,17,21-heptaen-9-one

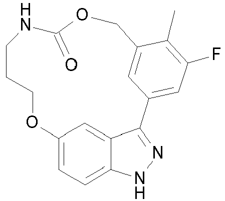

Example 28 is prepared according to the synthesis route described in general Scheme G.

### Preparation of intermediate 43: methyl 5-[5-(3-{[(benzyloxy)carbonyl]amino}lpropoxy)-1-(oxan-2-yl)-1H-indazol-3-yl]-3-fluoro-2-methylbenzoate

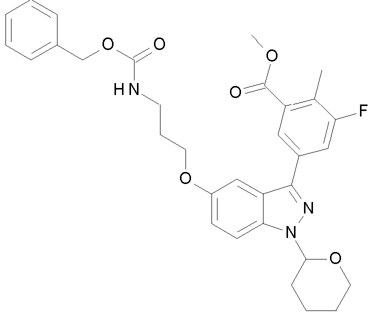

To a solution of benzyl N-(3-{[1-(oxan-2-yl)-3-(tetramethyl-1,3,2-dioxaborolan-2-yl)-1H-indazol-5-yl]oxy}propyl)carbamate **31** (0.6 g, 1.12 mmol) in *N,N*-dimethylformamide (15 mL) was added at RT (methyl 5-bromo-3-fluoro-2-methylbenzoate (0.332 g, 1.35 mmol), Cs₂CO₃ (1.096 g, 3.36 mmol) and PdCl₂(dppf)·DCM (0.041 g, 0.06 mmol). The resulting mixture was degassed by bubbling nitrogen for 10 minutes and stirred at 110°C under microwave irradiation for 50 min. The solvent was removed under reduced pressure and the oil was dissolved in EtOAc and water. The two layers were separated and the aqueous phase was extracted twice with ethyl acetate. The combined organic layers were dried over sodium sulfate and the solvent was removed under reduced pressure. The residue was purified by flash-column chromatography (25 g silica BIOTAGE) chromatography (cyclohexane - ethyl acetate, 100/0 to 50/50) affording methyl 5-[5-(3-{[(benzyloxy)carbonyl]amino}propoxy)-1-(oxan-2-yl)-1H-indazol-3-yl]-3-fluoro-2-methylbenzoate **43** as a yellow powder.
LCMS method F: [M+H]⁺ = 576.2, t_{R} = 3.48 min

### Preparation of intermediate 44: benzyl N-[3-({3-[3-fluoro-5-(hydroxymethyl)-4-methyl phenyl]-1-(oxan-2-yl)-1H-indazol-5-yl}oxy)propyl]carbamate

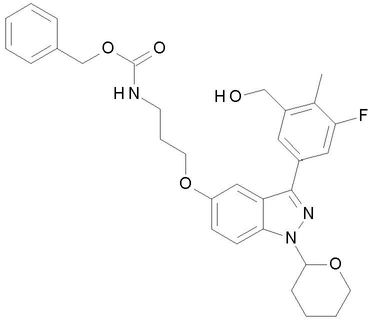

To a methyl 5-[5-(3-{[(benzyloxy)carbonyl]amino}propoxy)-1-(oxan-2-yl)-1H-indazol-3-yl]-3-fluoro-2-methylbenzoate **43** (0.225 g, 0.39 mmol) in THF (50 mL) was added a 1M solution of Lithium aluminium tetrahydride (0.78 mL, 0.78 mmol) at 0°C. The mixture was stirred at 0 °C for 1 hour. To the reaction mixture, EtOAc (10 mL) was added at 0°C and poured in a 10% solution of Rochelle's salt (100 mL) and EtOAc (100 mL). The mixture was stirred at RT for 2 hours. After separation, the aqueous layer was extracted with EtOAc (2x50 mL). The combined organic layers were washed with brine, dried over sodium sulfate and the solvent was removed under reduced pressure to a brown/orange oil. This residue was purified by flash chromatography on silica gel (Macherey Nagel, 25 g) with gradient elution: cyclohexane/EtOAc 0-100 % to give benzyl N-[3-({3-[3-fluoro-5-(hydroxymethyl)-4-methylphenyl]-1-(oxan-2-yl)-1H-indazol-5-yl}oxy)propyl]carbamate 44 as a yellow oil.
LCMS method F: [M+H]⁺ = 548.2, t_{R} = 3.10 min

### Preparation of intermediate 45: 4-fluoro-5-methyl-19-(oxan-2-yl)-8,14-dioxa-10,19,20-triazatetracyclo[13.5.2.1^{2,6}.0^{18,21}]tricosa-1(20),2, 4,6(23),15,17,21-heptaen-9-one

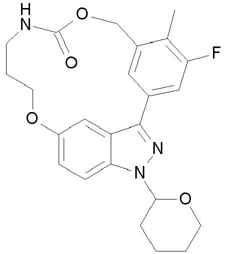

To a solution of benzyl-N-[3-({3-[3-fluoro-5-(hydroxymethyl)-4-methylphenyl]-1-(oxan-2-yl)-1H-indazol-5-yl}oxy)propyl]carbamate **44** (0.125 g, 0.23 mmol) in anhydrous acetonitrile (33 mL) was added at RT cesium carbonate (0.447 g, 1.37 mmol). The resulting reaction mixture was stirred at 90°C for 1h30. The reaction mixture was filtered, the solvent was removed under reduced pressure and the residue was purified by flash-column (15g silica Macherey Nagel) chromatography (DCM - ethyl acetate, 1:0 to 8:2) affording 4-fluoro-5-methyl-19-(oxan-2-yl)-8,14-dioxa-10,19,20-triazatetracyclo[13.5.2.1^{2,6}.0^{18,21}]tricosa-1(20),2, 4,6(23),15,17,21-heptaen-9-one **45** as a white foam.
LCMS method F: [M+H]⁺ = 440.2, t_{R} = 3.03 min

### Preparation of Example 28: 4-fluoro-5-methyl-8,14-dioxa-10,19,20-triazatetracyclo [13.5.2.1^{2,6}.0^{18,21}]tricosa-1(20),2,4,6(23),15,17,21-heptaen-9-one

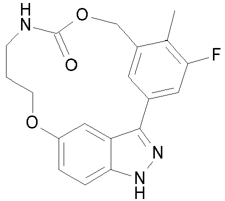

To a solution of 4-fluoro-5-methyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **45** (0.066 g, 0.15 mmol) in DCM (3 mL) was added at RT TFA (0.143 mL, 1.92 mmol). The resulting reaction mixture was stirred under microwave irradiation at 80°C for 1h30. The reaction mixture was concentrated under reduced pressure, diluted with saturated sodium bicarbonate solution and extracted twice with ethyl acetate. The combined organic layers were dried over anhydrous sodium sulfate and the solvent was removed under reduced pressure. The residue was purified by flash-column (5g silica Macherey Nagel) chromatography (DCM - ethyl acetate, 1:0 to 4:6) to give a solid, which was triturated in acetonitrile and filtered affording 4-fluoro-5-methyl-8,14-dioxa-10,19,20-triazatetracyclo[13.5.2.1^{2,6}.0^{18,21}]tricosa-1(20),2,4,6(23),15,17,21-heptaen-9-one **example 28** as a white solid.
LCMS method F: [M+H]⁺ = 356.2, t_{R} = 2.36 min
LCMS method G: [M+H]⁺ = 356.2, t_{R} = 2.39 min
¹H NMR (400 MHz, d6-DMSO) δ 7.80 (1H, s), 7.67 (1H, s), 7.63 (1H, d, J = 11.2 Hz), 7.48 (1H, dd, J = 0.6, 9.1 Hz), 7.40 (1H, d, J = 2.4 Hz), 6.96 (1H, dd, J = 2.3, 8.9 Hz), 5.29 (2H, s), 4.35 (2H, t, J = 8.1 Hz), 3.24 - 3.17 (2H, m), 2.22 (3H, d, J = 1.7 Hz), 2.06 - 2.05 (2H, m) ppm. The indazole NH proton was not visible in this solvent.

### Example 29: 4,5-difluoro-8,14-dioxa-10,19,20-triazatetracyclo[13.5.2.1^{2,6}.0^{18,21}]tricosa-1(20),2,4,6(23),15,17,21-heptaen-9-one

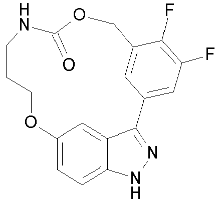

Example 29 is prepared according to the synthesis route described in general Scheme F and procedures analogous to those used to obtain **example 11.** Methyl 2,3-difluoro-5-(4,4,5,5-tetramethyl-1,3,2-dioxaborolan-2-yl)benzoate is used for the Suzuki coupling with intermediate **26** to give 4,5-difluoro-8,14-dioxa-10,19,20-triazatetracyclo[13.5.2.1^{2,6}.0^{18,21}]tricosa-1(20),2, 4,6(23),15,17,21-heptaen-9-one **example 29.**
LCMS method F: [M+H]⁺ = 360, t_{R} = 2.47 min
LCMS method G: [M+H]⁺ = 360, t_{R} = 2.52 min
¹H NMR (400 MHz, d6-DMSO, 80 °C) δ 13.06 (1H, s), 7.84 - 7.78 (2H, m), 7.71 - 7.69 (1H, m), 7.51 (1H, d, J = 9.1 Hz), 7.31 (1H, d, J = 2.1 Hz), 7.01 (1H, dd, J = 2.4, 9.0 Hz), 5.38 (2H, m), 4.34 (2H, dd, J = 8.1, 8.8 Hz), 3.18 (2H, m), 2.03 (2H, m) ppm.

### Example 30: 5-bromo-8,14-dioxa-10,19,20-triazatetracyclo[13.5.2.1^{2,6}.0^{18,21}]tricosa-1(20),2,4,6(23),15,17,21-heptaen-9-one

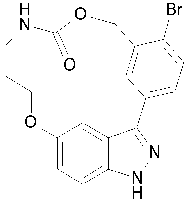

Example 30 is prepared according to the synthesis route described in general Scheme C and procedures analogous to those used to obtain **example 8.**

### Preparation of intermediate 46: [3-bromo-5-(hydroxymethyl)phenyl]boronic acid

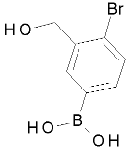

A solution of borane tetrahydrofuran complex (1.0 M in THF, 8.2 mL, 8.2 mmol) was slowly added to a solution of 3-borono-6-bromo-benzoic acid (500 mg, 2.05 mmol) in THF (30 mL) at 0 °C. The reaction mixture was allowed to reach room temperature and stirred for 16 hours. MeOH (25 mL) was added at 0°C to quench the reaction until no gas was produced. The solvent was evaporated, and the residue was partitioned between ethyl acetate (50 mL) and water (50 mL). After separation, the aqueous layer was extracted with ethyl acetate (2 x 50 mL). The combined organic layers were washed with brine (50 mL), dried over anhydrous sodium magnesium sulfate, filtered and concentrated under reduced pressure to give [3-bromo-5-(hydroxymethyl)phenyl]boronic acid **46** as a white solid.
LCMS method F: no m/z detected, t_{R} = 1.58 min

### Preparation of intermediate 47: benzyl N-[3-[3-[4-bromo-3-(hydroxymethyl)phenyl]-1-tetrahydropyran-2-yl-indazol-5-yl]oxypropyl] carbamate

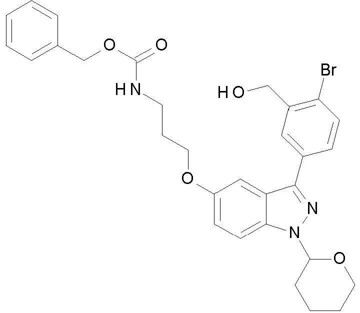

To a solution of benzyl-N-[3-(3-iodo-1-tetrahydropyran-2-yl-indazol-5-yl)oxypropyl] carbamate **26** (692 mg, 1.29 mmol), [4-bromo-3-(hydroxymethyl)phenyl]boronic acid **46** (357 mg, 1.55 mmol) and a 1M solution of Na₂CO₃ (3.9 mL, 3.87 mmol) in DME (13 mL) was added palladium-tetrakis(triphenylphosphine) (75 mg, 0.065 mmol, 5 mol %). The reaction mixture was stirred at 80 °C for 16 hours. After being cooled to room temperature, the reaction mixture was diluted with water (20 mL), extracted twice with ethyl acetate (2x50 mL). The combined organic layer was washed with brine, dried over anhydrous sodium sulfate, filtered and concentrated under reduced pressure to afford a yellow solid. The crude was purified by flash chromatography (CyH/EtOAc 0 to 100% EtOAc) with a 24 g Redisep to afford benzyl N-[3-[3-[4-bromo-3-(hydroxymethyl)phenyl]-1-tetrahydropyran-2-yl-indazol-5-yl]oxypropyl] carbamate **47** as a white solid.
LCMS method F: [M+H]⁺ = 594, t_{R} = 3.12 min

### Preparation of intermediate 48: 5-bromo-19-(oxan-2-yl)-8,14-dioxa-10,19,20-triaza tetracyclo[13.5.2.1^{2,6}.0^{18,21}]tricosa-1(20),2,4,6(23),15,17,21-heptaen-9-one

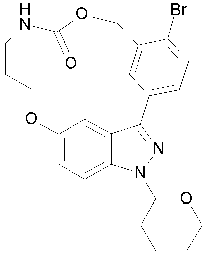

A suspension of benzyl N-[3-[3-[4-bromo-5-(hydroxymethyl)phenyl]-1-tetrahydropyran-2-yl-indazol-5-yl]oxypropyl]carbamate **47** (590 mg, 0.99 mmol) and cesium carbonate (1.94 g, 5.96 mmol) in acetonitrile (200 mL) was heated to 90°C for 2 h. The reaction mixture was cooled to RT then filtered and concentrated under reduced pressure. The obtained solid was triturated with acetonitrile to give 5-bromo-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **48** as a white solid.
LCMS method F: [M+H]⁺ = 486/488, t_{R} = 3.25 min

### Preparation of Example 30: 5-bromo-8,14-dioxa-10,19,20-triazatetracyclo [13.5.2.1^{2,6}.0^{18,21}] tricosa-1(20),2,4,6(23),15,17,21-heptaen-9-one

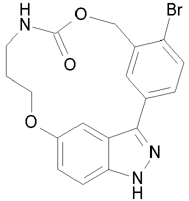

To a solution of 5-bromo-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **48** (50 mg, 0.10 mmol) in DCM (3 mL) was added trifluoroacetic acid (157 µL, 2.05 mmol). The reaction mixture was stirred at RT for 4 h. The reaction mixture was diluted with DCM (20 mL). Water (20 mL) and ammonium hydroxide 25 % weight aqueous solution (3 mL) were added. A white precipitate was presented in organic layer, and not soluble in DCM. The solid was filtered and dried under reduced pressure to afford 5-bromo-8,14-dioxa-10,19,20-triazatetracyclo[13.5.2.1^{2,6}.0^{18,21}] tricosa-1(20),2,4,6(23),15,17,21-heptaen-9-one **example 30** as a white solid.
LCMS method F: [M+H]⁺ = 403, t_{R} = 2.58 min
LCMS method G: [M+H]⁺ = 403, t_{R} = 2.48 min
¹H NMR (400 MHz, d6-DMSO) δ 13.05 (1H, s), 7.91 - 7.87 (3H, m), 7.73 - 7.69 (1H, m), 7.52 - 7.49 (1H, m), 7.37 (1H, d, J = 1.7 Hz), 7.01 (1H, dd, J = 2.3, 8.9 Hz), 5.27 (2H, s), 4.37 - 4.33 (2H, m), 3.19 (2H, m), 2.02 - 1.99 (2H, m) ppm.

### Example 31: 4-(4-methylpiperazin-1-yl)-8,14-dioxa-10,19,20-triazatetracyclo [13.5.2.1^{2,6}.0^{18,21}]tricosa-1(20),2,4,6(23),15,17,21-heptaen-9-one

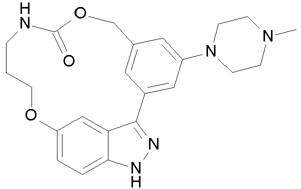

Example 31 is prepared according to the synthesis route described in general Scheme C and procedures analogous to those used to obtain **example 12.** 1-Methylpiperazine is used for the Buchwald reaction with the bromide intermediate **34** to give 4-(4-methylpiperazin-1-yl)-8,14-dioxa-10,19,20-triazatetracyclo[13.5.2.1^{2,6}.0^{18,21}]tricosa-1(20),2,4,6(23),15,17,21-heptaen-9-one **example 31.**
LCMS method F: [M+H]⁺ = 422, t_{R} = 1.44 min
LCMS method G: [M+H]⁺ = 422, t_{R} = 2.02 min
¹H NMR (400 MHz, d6-DMSO) δ 12.80 (1H, s), 7.62 (1H, m), 7.46 (1H, d, J = 9.2 Hz), 7.38 (1H, m), 7.35 (2H, m), 6.98 - 6.95 (1H, m), 6.88 (1H, m), 5.23 (2H, m), 4.32 - 4.28 (2H, m), 3.25 (4H, m), 3.16 (2H, m), 2.53 (4H, m), 2.28 (3H, s), 2.04 (2H, m) ppm.

### Example 32: 4-(3-methoxyazetidin-1-yl)-8,14-dioxa-10,19,20-triazatetracyclo [13.5.2.1^{2,6}.0^{18,21}]tricosa-1(20),2,4,6(23),15,17,21-heptaen-9-one

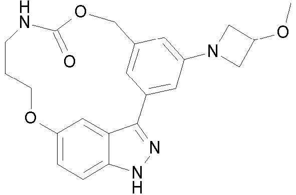

Example 32 is prepared according to the synthesis route described in general Scheme C and procedures analogous to those used to obtain **example 12.** 3-methoxyazetidine hydrochloride is used for the Buchwald reaction with the bromide intermediate 34 to give 4-(3-methoxyazetidin-1-yl)-8,14-dioxa-10,19,20-triazatetracyclo[13.5.2.1^{2,6}.0^{18,21}]tricosa-1(20),2, 4,6(23),15,17,21-heptaen-9-one **example 32.**
LCMS method F: [M+H]⁺ = 409.2, t_{R} = 2.15 min
LCMS method G: [M+H]⁺ = 409.1, t_{R} = 2.13 min
¹H NMR (400 MHz, d6-DMSO, 80 °C) δ 12.85 (1H, br s), 7.61 (1H, br s), 7.48 - 7.45 (1H, m), 7.36 - 7.34 (1H, m), 7.27 (1H, s), 6.95 (1H, dd, J = 2.4, 9.2 Hz), 6.90 (1H, t, J = 2.0 Hz), 6.37 (1H, dd, J = 1.5, 2.1 Hz), 5.20 (2H, s), 4.40 - 4.27 (3H, m), 4.15 - 4.11 (2H, m), 3.69 (2H, dd, J = 4.3, 8.6 Hz), 3.30 (3H, s), 3.22 - 3.12 (2H, m), 2.09 - 1.96 (2H, m) ppm.

### Example 33: 1-{9-oxo-8,14-dioxa-10,19,20-triazatetracyclo[13.5.2.1^{2,6}.0^{18,21}]tricosa-1(20),2,4,6(23),15,17,21-heptaen-4-yl} piperidine-4-carbonitrile

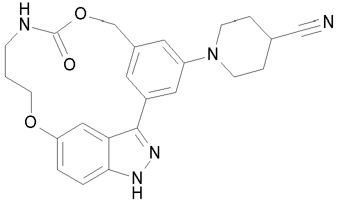

Example 33 is prepared according to the synthesis route described in general Scheme C and procedures analogous to those used to obtain **example 12.** Piperidine-4-carbonitrile is used for the Buchwald reaction with the bromide intermediate **34** to give 1-{9-oxo-8,14-dioxa-10,19,20-triazatetracyclo[13.5.2.1^{2,6}.0^{18,21}]tricosa-1(20),2,4,6(23),15,17,21-heptaen-4-yl}piperidine-4-carbonitrile **example 33.**
LCMS method F: [M+H]⁺ = 432, t_{R} = 2.15 min
LCMS method G: [M+H]⁺ = 432, t_{R} = 2.21 min
¹H NMR (400 MHz, *d*6-DMSO) δ 12.82 (1H, s), 7.63 (1H, m), 7.48 - 7.46 (1H, m), 7.40 - 7.35 (3H, m), 7.98 - 7.95 (1H, m), 7.90 (1H, m), 5.23 (2H, m), 4.30 (2H, m), 3.50 - 3.44 (2H, m), 3.22 - 3.15 (4H, m), 2.08 - 2.00 (4H, m), 1.92 - 1.84 (2H, m), 1.07 (1H, d, J = 5.9 Hz) ppm.

### Example 34: 4-[4-(pyrrolidin-1-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one

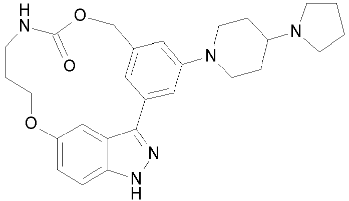

Example 34 is prepared according to the synthesis route described in general Scheme C and procedures analogous to those used to obtain **example 12.** 4-Pyrrolidin-1-ylpiperidine is used for the Buchwald reaction with the bromide intermediate **34** to give 4-[4-(pyrrolidin-1-yl)piperidin-1-yl]-8,14-dioxa-10,19,20-triazatetracyclo[13.5.2.1^{2,6}.0^{18,21}]tricosa-1(20),2,4, 6(23),15,17,21-heptaen-9-one **example 34.**
LCMS method F: [M+H]⁺ = 476, t_{R} = 1.59 min
LCMS method G: [M+H]⁺ = 476, t_{R} = 1.51 min
¹H NMR (400 MHz, DMSO) δ 12.85 (1H, m), 7.47 (1H, d, J = 8.7 Hz), 7.37 (3H, t, J = 13.0 Hz), 6.98 - 6.92 (2H, m), 5.28 (2H, m), 4.30 (2H, s), 3.85 (2H, m), 3.42 (1H, q, J = 7.0 Hz), 3.18 (3H, s), 2.88 - 2.82 (2H, m), 2.14 (2H, s), 2.04 (10H, m) ppm. Two protons were located under the DMSO peak and are not reported here.

### Example 35: 4-(azetidin-1-yl)-8,14-dioxa-10,19,20-triazatetracyclo[13.5.2.1^{2,6}.0^{18,21}] tricosa-1(20),2,4,6(23),15,17,21-heptaen-9-one

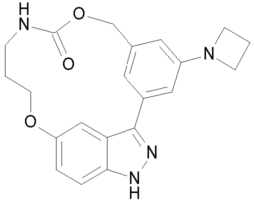

Example 35 is prepared according to the synthesis route described in general Scheme C and procedures analogous to those used to obtain **example 12.** Azetidine is used for the Buchwald reaction with the bromide intermediate **34** to give 4-(azetidin-1-yl)-8,14-dioxa-10,19,20-triazatetracyclo[13.5.2.1^{2,6}.0^{18,21}]tricosa-1(20),2,4,6(23),15,17,21-heptaen-9-one **example 35.**
LCMS method F: [M+H]⁺ = 379, t_{R} = 2.08 min
LCMS method G: [M+H]⁺ = 379, t_{R} = 2.23 min
¹H NMR (400 MHz, *d*6-DMSO) δ 12.78 (1H, m), 7.59 (1H, m), 7.48 - 7.44 (1H, m), 7.35 (1H, s), 7.25 (1H, s), 6.98 - 6.94 (1H, m), 6.88 - 6.87 (1H, m), 6.34 (1H, s), 5.20 (2H, s), 4.32 - 4.27 (2H, m), 3.90 (3H, t, J = 7.2 Hz), 3.15 (3H, m), 2.39 - 2.32 (2H, m), 2.06 (2H, s) ppm.

### Example 36: 4-(piperidin-1-yl)-8,14-dioxa-10,19,20-triazatetracyclo[13.5.2.1^{2,6}.0^{18,21}] tricosa-1(20),2,4,6(23),15,17,21-heptaen-9-one

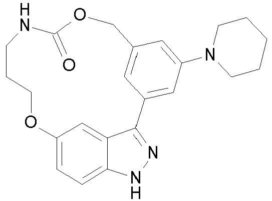

Example 36 is prepared according to the synthesis route described in general Scheme A. Piperidine is used for the Buchwald reaction with the bromide intermediate **34** to give 4-(piperidin-1-yl)-8,14-dioxa-10,19,20-triazatetracyclo[13.5.2.1^{2,6}.0^{18,21}]tricosa-1(20),2,4,6(23), 15,17,21-heptaen-9-one **example 36.**
LCMS method F: [M+H]⁺ = 407.2, t_{R} = 1.65 min
LCMS method G: [M+H]⁺ = 407.2, t_{R} = 2.48 min
¹H NMR (400 MHz, d6-DMSO, 80 °C) δ 12.79 (1H, s), 7.64 - 7.62 (1H, m), 7.48 - 7.44 (1H, d, *J* = 8.4 Hz), 7.38 - 7.32 (3H, m), 6.96 (1H, dd, *J =* 2.3, 9.1 Hz), 6.87 - 6.86 (1H, m), 5.22 (2H, s), 4.30 (2H, dd, *J =* 7.6, 10.0 Hz), 3.27 - 3.21 (4H, m), 3.20 - 3.11 (2H, m), 2.06 - 1.97 (2H, m), 1.71 -1.64 (4H, m), 1.63 - 1.58 (2H, m) ppm.

### Example 37: 4-(2,5-dihydrofuran-3-yl)-8,14-dioxa-10,19,20-triazatetracyclo [13.5.2.1^{2,6}.0^{18,21}]tricosa-1(20),2,4,6(23),15,17,21-heptaen-9-one

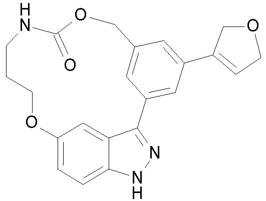

Example 37 is prepared according to the synthesis route described in general Scheme A. 2-(2,5-Dihydrofuran-3-yl)-4,4,5,5-tetramethyl-1,3,2-dioxaborolane is used for the Suzuki reaction with the bromide intermediate **34** to give 4-(2,5-dihydrofuran-3-yl)-8,14-dioxa-10,19,20-triazatetracyclo[13.5.2.1^{2,6}.0^{18,21}]tricosa-1(20),2,4,6(23),15,17,21-heptaen-9-one **example 37.**
LCMS method F: [M+H]⁺ = 392.2, t_{R} = 2.19 min
LCMS method G: [M+H]⁺ = 392.2, t_{R} = 2.19 min
¹H NMR (400 MHz, d6-DMSO, 80 °C) δ 12.94 (1H, s), 7.85 (2H, d, *J =* 6.3 Hz), 7.69 (1H, s), 7.50 (1H, d, *J =* 9.6 Hz), 7.37 (1H, s), 7.33 (1H, d, *J =* 2.0 Hz), 6.99 (1H, dd, *J =* 2.3, 8.9 Hz), 6.55 - 6.52 (1H, m), 5.33 - 5.30 (2H, m), 5.00 - 4.96 (2H, m), 4.80 - 4.77 (2H, m), 4.35 - 4.29 (2H, m), 3.19 - 3.17 (2H, m), 1.99 (2H, s) ppm.

### Example 38: 4-[4-(morpholin-4-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one

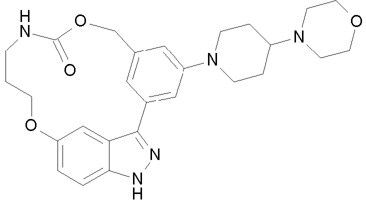

Example 38 is prepared according to the synthesis route described in general Scheme C and procedures analogous to those used to obtain **example 12.** 4-(4-Piperidyl)morpholine is used for the Buchwald reaction with the bromide intermediate **34** to give 4-[4-(morpholin-4-yl)piperidin-1-yl]-8,14-dioxa-10,19,20-triazatetracyclo[13.5.2.1^{2,6}.0^{18,21}]tricosa-1(20),2,4, 6(23),15,17,21-heptaen-9-one **example 38.**
LCMS method F: [M+H]⁺ = 492.2, t_{R} = 1.48 min
LCMS method G: [M+H]⁺ = 492.2, t_{R} = 2.07 min
¹H NMR (400 MHz, *d*6-DMSO) δ 12.79 (1H, s), 7.61 (1H, s), 7.47 (1H, d, J = 5.8 Hz),7.39-7.29 (3H, m), 6.97 (1H, dd, J = 2.2, 9.1 Hz), 6.89 (1H, s), 5.22 (2H, s), 4.36 - 4.26 (2H, m), 3.87 - 3.75 (2H, m), 3.64 - 3.54 (4H, m), 3.23- 3.12 (2H, m), 2.88- 2.76 (2H, m), 2.57- 2.52 (4H, m), 2.41 - 2.29 (1H, m), 2.09 -1.88 (4H, m), 1.63 - 1.5 (2H, m) ppm.

### Example 39: 4-(1-methyl-1,2,3,6-tetrahydropyridin-4-yl)-8,14-dioxa-10,19,20-triazatetra cyclo[13.5.2.1^{2,6}.0^{18,21}]tricosa-1(20),2,4,6(23),15,17,21-heptaen-9-one

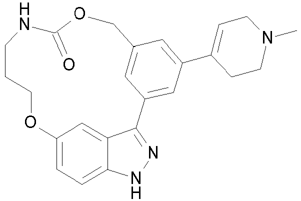

Example 39 is prepared according to the synthesis route described in general Scheme A. 1-methyl-4-(4,4,5,5-tetramethyl-1,3,2-dioxaborolan-2-yl)-3,6-dihydro-2H-pyridine is used for the Suzuki reaction with the bromide intermediate **34** to give 4-(1-methyl-1,2,3,6-tetrahydropyridin-4-yl)-8,14-dioxa-10,19,20-triazatetracyclo[13.5.2.1^{2,6}.0^{18,21}]tricosa-1(20),2, 4,6(23),15,17,21-heptaen-9-one **example 39.**
LCMS method F: [M+H]⁺ = 419.2, t_{R} = 1.49 min
LCMS method G: [M+H]⁺ = 419.2, t_{R} = 2.16 min
¹H NMR (400 MHz, *d*6-DMSO, 80 °C) δ 12.95 (1H, s), 7.97 (1H, s), 7.87 (1H, s), 7.71 - 7.69 (1H, m), 7.50 (1H, d, *J =* 8.0 Hz), 7.40 (1H, s), 7.34 (1H, d, *J =* 1.5 Hz), 7.00 (1H, dd, *J =* 2.3, 8.9 Hz), 6.28 - 6.25 (1H, m), 5.35 - 5.32 (2H, m), 4.32 (2H, dd, *J* = 8.1, 9.0 Hz), 3.94 - 3.91 (2H, m), 3.55 - 3.41 (2H, m), 3.25 - 3.17 (2H, m), 2.92 (3H, s), 2.90 - 2.84 (2H, m), 2.10 - 1.99 (2H, m) ppm.

### Example 40: 4-[(2S,5S)-2,5-dimethylmorpholin-4-yl]-8,14-dioxa-10,19,20-triazatetracyclo [13.5.2.1^{2,6}.0^{18,21}]tricosa-1(20),2,4,6(23),15,17,21-heptaen-9-one

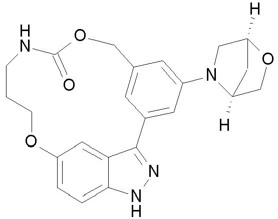

Example 40 is prepared according to the synthesis route described in general Scheme C and procedures analogous to those used to obtain **example 12.** (1S,4S)-2-Oxa-5-azabicyclo[2.2.1] heptane hydrochloride is used for the Buchwald reaction with the bromide intermediate **34** to give 4-[(2S,5S)-2,5-dimethylmorpholin-4-yl]-8,14-dioxa-10,19,20-triazatetracyclo[13.5.2. 1^{2,6}.0^{18,21}]tricosa-1(20),2,4,6(23),15,17,21-heptaen-9-one **example 40.**
LCMS method F: [M+H]⁺ = 421.1, t_{R} = 2.06 min
LCMS method G: [M+H]⁺ = 421.2, t_{R} = 2.06 min
¹H NMR (400 MHz, *d*6-DMSO) δ 12.78 (1H, s), 7.62 (1H, m), 7.46 (1H, d, J = 9.1 Hz), 7.36 (1H, s), 7.23 (1H, s), 7.06 (1H, s), 6.97 - 6.95 (1H, m), 6.58 (1H, s), 5.21 (2H, m), 4.63 (2H, d, J = 17.5 Hz), 4.32 - 4.28 (2H, m), 3.82 (1H, m), 3.76 (1H, m), 3.58 - 3.56 (1H, m), 3.16 (2H, m), 3.10 (1H, m), 2.03 (2H, m), 1.98 - 1.95 (1H, m),1.90 - 1.88 (1H, m) ppm.

### Example 41: 4-[(morpholin-4-yl)methyl]-8,14-dioxa-10,19,20-triazatetracyclo [13.5.2.1^{2,6}.0^{18,21}]tricosa-1(20),2,4,6(23),15,17,21-heptaen-9-one

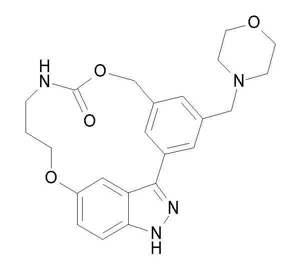

Example 41 is prepared according to the synthesis route described in general Scheme C.

### Preparation of intermediate 49: 4-[(morpholin-4-yl)methyl]-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one

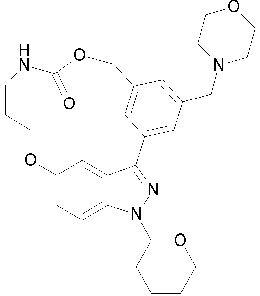

In a sealed tube, to a solution of 4-bromo-19-(oxan-2-yl)-8,14-dioxa-10,19,20 triazatetracyclo[13.5.2.12,6.018,21]tricosa-1(20),2(23),3,5,15(22),16,18(21)-heptaen-9-one **example 12** (100 mg, 0.21 mmol) in THF/H2O 9/1 (4 mL) was added potassium 1-trifluoroboratomethylmorpholine (87 mg, 0.42 mmol) and cesium carbonate (205 mg, 0.63 mmol) at RT. The reaction mixture was degassed for 15 min by bubbling nitrogen gas through the solution, then palladium acetate (2 mg, 0.01 mmol) and Xphos (10 mg, 0.02 mmol) were added and the reaction mixture was stirred at 100°C for 18 hours. The reaction mixture was allowed to cool to RT and the solvent was removed under reduced pressure. EtOAc (50 mL) was added to the residue and the suspension was filtered over celite. The filtrate was extracted with EtOAc (2x20 mL), washed with brine, dried over sodium sulfate and the solvent was removed under reduced pressure to afford a yellow oil. The oil was triturated with acetonitrile and diethyl ether to afford 4-[(morpholin-4-yl)methyl]-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **49** as a beige powder.
LCMS method F: [M+H]⁺ = 507, t_{R} = 1.74 min

### Preparation of example 41: 4-[(morpholin-4-yl)methyl]-8,14-dioxa-10,19,20-triazatetracyclo[13.5.2.1^{2,6}.0^{18,21}]tricosa-1(20),2,4,6(23),15,17,21-heptaen-9-one

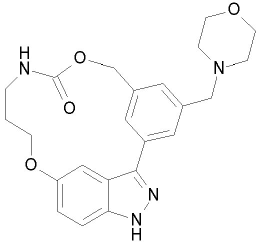

To a solution of 4-[(morpholin-4-yl)methyl]-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **49** (60 mg, 0.13 mmol) in DCM (2 mL) was stirred at RT for 6 hours. The reaction mixture was evaporated under reduced pressure to give a brown oil. dDCM (20 mL) and a saturated solution of bicarbonate (10 mL) was added to the residue, after separation, the organic layer was extracted with DCM (2x10 mL), washed with brine, dried over sodium sulfate and evaporated under reduced pressure to give an yellow oil. Some acetonitrile and diethyl ether was added to the oil, the precipitate formed was filtered to afford 4-[(morpholin-4-yl)methyl]-8,14-dioxa-10,19,20-triazatetracyclo[13.5.2.1^{2,6}.0^{18,21}]tricosa-1(20),2,4,6(23),15,17,21-heptaen-9-one **example 41** as a beige solid.
LCMS method F: [M+H]⁺ = 423, t_{R} = 1.42 min
LCMS method G: [M+H]⁺ = 423, t_{R} = 2.03 min
¹H NMR (400 MHz, DMSO) δ 12.89 (1H, s), 7.81 (2H, d, J = 11.8 Hz), 7.66 (1H, s), 7.50 - 7.47 (1H, m), 7.35 (1H, d, J = 1.9 Hz), 7.22 (1H, s), 6.98 (1H, dd, J = 2.4, 9.0 Hz), 5.29 - 5.26 (2H, m), 4.34 - 4.28 (2H, m), 3.63 (4H, m), 3.56 (2H, s), 3.18 (2H, s), 2.46 (4H, m), 2.06 - 2.03 (2H, m) ppm.

### Example 42: 4-[(pyrrolidin-1-yl)methyl]-8,14-dioxa-10,19,20-triazatetracyclo [13.5.2.1^{2,6}.0^{18,21}]tricosa-1(20),2,4,6(23),15,17,21-heptaen-9-one

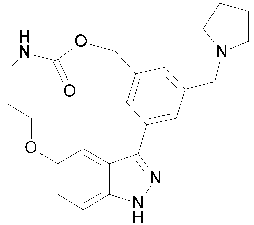

Example 42 is prepared according to the synthesis route described in general Scheme C and procedures analogous to those used to obtain **example 41.** Potassium trifluoro[(pyrrolidin-1-yl)methyl]borate was used for the Suzuki coupling with the bromide intermediate **34** to give 4-[(pyrrolidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one **example 42.**
LCMS method F: [M+H]⁺ = 407, t_{R} = 1.44 min
LCMS method G: [M+H]⁺ = 407, t_{R} = 2.12 min
¹H NMR (400 MHz, *d*6-DMSO) δ 12.88 (1H, s), 7.84 (1H, s), 7.79 (1H, s), 7.66 (1H, m), 7.48 (1H, d, J = 8.8 Hz), 7.35 (1H, d, J = 1.7 Hz), 7.22 (1H, s), 6.98 (1H, dd, J = 2.3, 8.9 Hz), 5.28 (2H, s), 4.32 (2H, dd, J = 8.1, 8.6 Hz), 3.69 (2H, s), 3.17 (2H, m), 2.54 (4H, m), 2.03 (2H, m), 1.75 (4H, m) ppm.

### Example 43: 4-[(piperidin-1-yl)methyl]-8,14-dioxa-10,19,20-triazatetracyclo [13.5.2.1^{2,6}.0^{18,21}]tricosa-1(20),2,4,6(23),15,17,21-heptaen-9-one

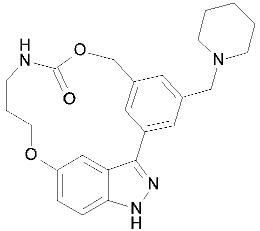

Example 43 is prepared according to the synthesis route described in general Scheme C and procedures analogous to those used to obtain **example 41.** Potassium trifluoro[(piperidin-1-yl)methyl]borate was used for the Suzuki coupling with the bromide intermediate **34** to give 4-[(piperidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one **example 43.**
LCMS method F: [M+H]⁺ = 421, t_{R} = 1.49 min
LCMS method G: [M+H]⁺ = 421, t_{R} = 2.33 min
¹H NMR (400 MHz, *d*6-DMSO, 80°C) δ 12.86 (1H, s), 7.86 (2H, m), 7.59 (1H, m), 7.48 (1H, d, J = 8.4 Hz), 7.37 (1H, d, J = 2.1 Hz), 7.25 (1H, m), 6.99 (1H, dd, J = 2.3, 8.9 Hz), 5.30 (2H, s), 4.32 (2H, m), 3.19 (2H, m), 2.05 (2H, m), 1.62 (4H, m), 1.48 (2H, m) ppm. Some protons are not visible due to different conformations. Structure confirmed by COSY.

### Example 44: 4-[(4-methylpiperazin-1-yl)methyl]-8,14-dioxa-10,19,20-triazatetracyclo [13.5.2.1^{2,6}.0^{18,21}]tricosa-1(20),2,4,6(23),15,17,21-heptaen-9-one

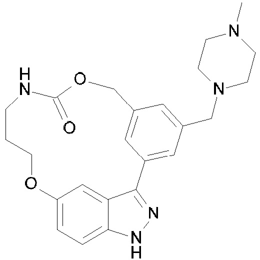

Example 44 is prepared according to the synthesis route described in general Scheme C and procedures analogous to those used to obtain **example 41.** Potassium trifluoro[(4-methylpiperazin-1-yl)methyl]borate was used for the Suzuki coupling with the bromide intermediate **34** to give 4-[(4-methylpiperazin-1-yl)methyl]-8,14-dioxa-10,19,20-triazatetracyclo[13.5.2.1^{2,6}.0^{18,21}]tricosa-1(20),2,4,6(23),15,17,21-heptaen-9-one **example 44.**
LCMS method F: [M+H]⁺ = 436, t_{R} = 1.36 min (current 20V)
LCMS method G: [M+H]⁺ = 436, t_{R} = 1.95 min (pH10 current 20V)
¹H NMR (400 MHz, *d*6-DMSO, 80 °C) δ 12.89 (1H, s), 7.81 (1H, s), 7.79 (1H, s), 7.66 (1H, m), 7.48 (1H, d, J = 8.8 Hz), 7.35 (1H, m), 7.20 (1H, m), 6.98 (1H, dd, J = 2.3, 9.1 Hz), 5.28 (2H, s), 4.31 (2H, m), 3.55 (2H, s), 3.17 (2H, m), 2.46 - 2.37 (8H, m), 2.20 (3H, s), 2.04 (2H, m) ppm.

### Example 45: 5-(morpholin-4-yl)-8,14-dioxa-10,19,20-triazatetracyclo[13.5.2.1^{2,6}.0^{18,21}] tricosa-1(20),2,4,6(23),15,17,21-heptaen-9-one

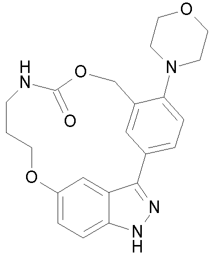

Example 45 is prepared according to the synthesis route described in general Scheme C. Morpholine was used for the Buchwald coupling with the bromide intermediate **48** to give 5-(morpholin-4-yl)-8,14-dioxa-10,19,20-triazatetracyclo[13.5.2.1^{2,6}.0^{18,21}]tricosa-1(20),2,4, 6(23),15,17,21-heptaen-9-one **example 45.**
LCMS method F: [M+H]⁺ = 409, t_{R} = 2.17 min
LCMS method G: [M+H]⁺ = 409, t_{R} = 2.16 min
¹H NMR (400 MHz, *d*6-DMSO) δ 12.80 (1H, s), 7.89 - 7.86 (2H, m), 7.68 (1H, s), 7.49 - 7.45 (1H, m), 7.35 (1H, d, J = 1.3 Hz), 7.29 - 7.25 (1H, m), 6.97 (1H, dd, J = 2.3, 8.9 Hz), 5.37 (2H, s), 4.31 (2H, dd, J = 8.3, 8.6 Hz), 3.78 (4H, m), 3.17 (2H, s), 2.91 (4H, m), 2.05 (2H, s) ppm.

### Example 46: 4-[4-(2-methoxyethyl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one

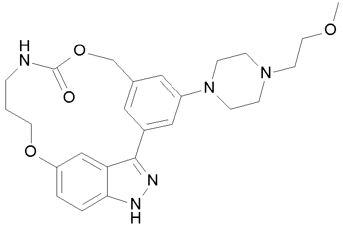

Example 46 is prepared according to the synthesis route described in general Scheme A. 1-(2-methoxyethyl)piperazine is used for the Buchwald reaction with the bromide intermediate **34** to give 4-[4-(2-methoxyethyl)piperazin-1-yl]-8,14-dioxa-10,19,20-triazatetracyclo[13.5.2. 1^{2,6}.0^{18,21}]tricosa-1(20),2,4,6(23),15,17,21-heptaen-9-one **example 46.**
LCMS method F: [M+H]⁺ = 466.2, t_{R} = 1.48 min
LCMS method G: [M+H]⁺ = 466.2, t_{R} = 2.06 min
¹H NMR (400 MHz, *d*6-DMSO, 80 °C) δ 12.83 (1H, s), 7.64 (1H, s), 7.49 - 7.46 (1H, m), 7.40 (2H, s), 7.34 (1H, s), 6.99 - 6.91 (2H, m), 5.24 (2H, s), 4.33 - 4.27 (2H, m), 3.67 - 3.63 (2H, m), 3.17 - 3.08 (15H, m), 2.10 - 1.99 (2H, m) ppm.

### Example 47: 4-(diethylamino)-8,14-dioxa-10,19,20-triazatetracyclo[13.5.2.1^{2,6}.0^{18,21}] tricosa-1(20),2,4,6(23),15,17,21-heptaen-9-one

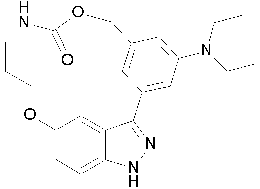

Example 47 is prepared according to the synthesis route described in general Scheme A. Diethylamine is used for the Buchwald reaction with the bromide intermediate **34** to give 4-(diethylamino)-8,14-dioxa-10,19,20-triazatetracyclo[13.5.2.1^{2,6}.0^{18,21}]tricosa-1(20),2,4, 6(23),15,17,21-heptaen-9-one **example 47.**
LCMS method F: [M+H]⁺ = 395.2, t_{R} = 1.57 min
LCMS method G: [M+H]⁺ = 395.2, t_{R} = 2.48 min
¹H NMR (400 MHz, *d*6-DMSO, 80 °C) δ 12.73 (1H, br s), 7.59 (1H, br s), 7.46 (1H, d, J = 9.3 Hz), 7.36 (1H, d, J = 2.1 Hz), 7.18 - 7.16 (2H, m), 6.95 (1H, dd, J = 2.4, 8.8 Hz), 6.63 (1H, s), 5.21 - 5.20 (2H, m), 4.32 - 4.27 (2H, m), 3.42 (4H, q, J = 7.0 Hz), 3.21 - 3.10 (2H, m), 2.08 - 1.96 (2H, m), 1.17 (6H, t, J = 6.9 Hz) ppm.

### Example 48 : 4-cyclopropyl-8,14-dioxa-10,19,20-triazatetracyclo[13.5.2.1^{2,6}.0^{18,21}]tricosa-1(20),2,4,6(23),15,17,21-heptaen-9-one

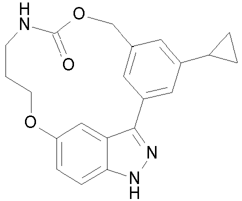

Example 48 is prepared according to the synthesis route described in general Scheme C. Potassium trifluoro[cyclopropyl]borate was used for the Suzuki coupling with the bromide intermediate **34** to give 4-cyclopropyl-8,14-dioxa-10,19,20-triazatetracyclo[13.5.2.1^{2,6}. 0^{18,21}]tricosa-1(20),2,4,6(23),15,17,21-heptaen-9-one **example 48.**
LCMS method F: [M+H]⁺ = 364, t_{R} = 2.40 min
LCMS method G: [M+H]⁺ = 364, t_{R} = 2.39 min
¹H NMR (400 MHz, *d*6-DMSO, 80 °C) δ 12.87 (1H, s), 7.68 (1H, s), 7.64 (1H, m), 7.60 (1H, s), 7.47 (1H, d, J = 9.1 Hz), 7.33 (1H, d, J = 2.1 Hz), 6.99 (1H, s), 6.97 (1H, dd, J = 9.0, 2.3 Hz), 5.24 (2H, m), 4.30 (2H, m), 3.17 (2H, m), 2.03 (3H, m), 1.00 (2H, m), 0.74 (2H, m) ppm.

### Example 49: 5-(4-methylpiperazin-1-yl)-8,14-dioxa-10,19,20-triazatetracyclo [13.5.2.1^{2,6}.0^{18,21}]tricosa-1(20),2,4,6(23),15,17,21-heptaen-9-one

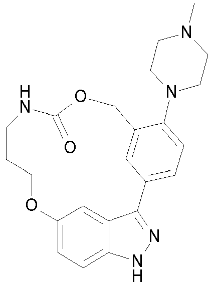

Example 49 is prepared according to the synthesis route described in general Scheme C. 4-Methylpiperazine was used for the Buchwald coupling with the bromide intermediate **48** to give 5-(4-methylpiperazin-1-yl)-8,14-dioxa-10,19,20-triazatetracyclo[13.5.2.1^{2,6}.0^{18,21}]tricosa-1(20),2,4,6(23),15,17,21-heptaen-9-one **example 49.**
LCMS method F: [M+H]⁺ = 422, t_{R} = 1.44 min
LCMS method G: [M+H]⁺ = 422, t_{R} = 2.13 min
¹H NMR (400 MHz, CD₃OD) δ 8.01 - 7.99 (1H, m), 7.92 (1H, dd, J = 2.1, 8.4 Hz), 7.79 (1H, t, J = 6.1 Hz), 7.49 - 7.34 (4H, m), 7.04 (1H, dd, J = 2.3, 9.1 Hz), 5.51 - 5.47 (2H, m), 4.36 (2H, m), 3.74 - 3.63 (2H, m), 3.42 (4H, m), 3.21 (4H, m), 3.03 (3H, s), 2.12 (2H, m) ppm.

### Example 50: 13-methyl-8,14-dioxa-10,19,20-triazatetracyclo[13.5.2.1^{2,6}.0^{18,21}]tricosa-1(20),2,4,6(23),15,17,21-heptaen-9-one

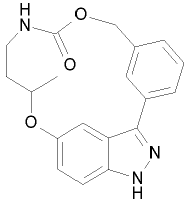

Example 50 is prepared according to the synthesis route described in general scheme C and procedures analogous to those used to obtain **example 8.**

To a solution of 13-methyl-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one (330 mg, 0.78 mmol) in dichloromethane (12 mL) was added trifluoroacetic acid (1.19 mL, 15.65 mmol) at RT. The solution was then irradiated under micro-waves (Biotage initiator+) for 2h. The reaction mixture was concentrated under *vacuo* and the residue was dissolved in EtOAc. The organic phase was washed with a saturated aqueous solution of sodium hydrogen carbonate, with brine, dired over Na₂SO₄, filtered and evaporated under reduced pressure. The solid obtained was triturated in diisopropyl ether and dried to give the expected compound 13-methyl-8,14-dioxa-10,19,20-triazatetracyclo[13.5.2.1^{2,6}.0^{18,21}]tricosa-1(20),2,4,6(23),15,17,21-heptaen-9-one **example 50** as a pale yellow solid.
LCMS method F: [M+H]⁺ = 338, t_{R} = 2.25 min
LCMS method G: [M+H]⁺ = 338, t_{R} = 2.24 min
¹H NMR (400 MHz, *d*6-DMSO) δ 13.12 (1H, s), 7.93 - 7.84 (3H, m), 7.47 (2H, dd, J = 8.5, 15.8 Hz), 7.27 (2H, d, J = 7.0 Hz), 6.97 (1H, dd, J = 2.1, 8.9 Hz), 5.75 (1H, d, J = 12.1 Hz), 4.81 (1H, d, J = 12.5 Hz), 4.57 (1H, dd, J = 6.0, 9.2 Hz), 3.59 - 3.54 (1H, m), 2.93 - 2.86 (1H, m), 2.47-2.33 (1H, m), 1.41 - 1.38 (4H, m) ppm.

### Example 51: 8,14-dioxa-4,5,10,19,20-pentaazatetracyclo[13.5.2.1^{2,5}.0^{18,21}]tricosa-1(20),2(23),3,15(22),16,18(21)-hexaen-9-one

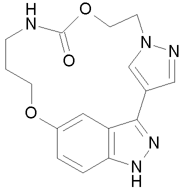

Example 51 is prepared according to the synthesis route described in general Scheme C.

### Preparation of intermediate 50: benzyl N-[3-({3-[1-(2-hydroxyethyl)-1H-pyrazol-4-yl]-1-(oxan-2-yl)-1H-indazol-5-yl}oxy) propyl]carbamate

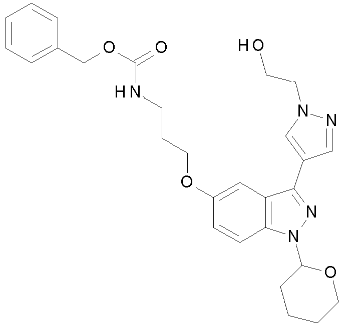

To a solution of benzyl-N-(3-{[3-iodo-1-(oxan-2-yl) -1H-indazol-5-yl]oxy}propyl)carbamate **26** (0.535 g, 1.0 mmol) in dioxane (3 mL) and water (1 mL) was added at RT 1-(2-hydroxyethyl)-1H-pyrazole-4-boronic acid pinacol ester (0.286 g, 1.2 mmol), K₃PO₄ (0.637 g, 3.0 mmol), XPhos (0.048 g, 0.1 mmol) and Pd(PPh₃)₄ (0.058 g, 0.05 mmol). The resulting reaction mixture was stirred under microwave irradiation at 120°C for 1h. The residue was diluted with saturated sodium chloride solution and extracted with ethyl acetate twice. The combined organic layers were dried over anhydrous sodium sulfate and the solvent was removed under reduced pressure. The residue was purified by flash-column (25g silica Macherey Nagel) chromatography (cyclohexane - ethyl acetate 3 / EtOH 1, 1:0 to 1:1) affording benzyl N-[3-({3-[1-(2-hydroxyethyl)-1H-pyrazol-4-yl]-1-(oxan-2-yl)-1H-indazol-5-yl}oxy) propyl]carbamate **50** as a yellow oil.
LCMS method F: [M+H]⁺ = 520.2, t_{R} = 2.56 min

### Preparation of intermediate 51:

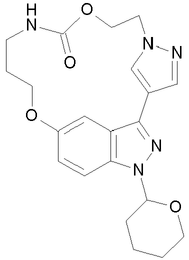

To a solution of benzyl N-[3-({3-[1-(2-hydroxyethyl)-1H-pyrazol-4-yl]-1-(oxan-2-yl)-1H-indazol-5-yl}oxy)propyl]carbamate **50** (0.380 g, 0.73 mmol) in anhydrous acetonitrile (146 mL) was added at RT cesium carbonate (1.430 g, 4.39 mmol). The resulting reaction mixture was stirred at 90°C for 36 h. The reaction mixture was filtered and the filtrate was concentrated under reduced pressure. The residue was purified by flash-column (15g silica Macherey Nagel) chromatography (cyclohexane - ethyl acetate 3 / EtOH 1, 1:0 to 3:7) affording 19-(oxan-2-yl)-8,14-dioxa-4,5,10,19,20-pentaazatetracyclo[13.5.2.1^{2,5}.0^{18,21}]tricosa-1(20),2(23),3,15(22), 16,18(21)-hexaen-9-one **51** as a white solid.
LCMS method F: [M+H]⁺ = 412.2, t_{R} = 2.20 min

### Preparation of Example 51: 8,14-dioxa-4,5,10,19,20-pentaazatetracyclo[13.5.2.1^{2,5}.0^{18,21}] tricosa-1(20),2(23), 3,15(22),16,18(21)-hexaen-9-one

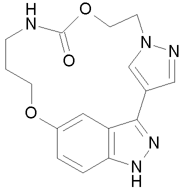

To a solution of 19-(oxan-2-yl)-8,14-dioxa-4,5,10,19,20-pentaazatetracyclo [13.5.2.1^{2,5}.0^{18,21}]tricosa-1(20),2(23),3,15(22),16,18(21)-hexaen-9-one **51** (0.155 g, 0.38 mmol) in DCM (3 mL) was added at RT TFA (0.561 mL, 7.53 mmol). The resulting reaction mixture was stirred under microwave irradiation at 80°C for 1h30. The reaction mixture was concentrated under reduced pressure, diluted with saturated sodium bicarbonate solution and extracted with ethyl acetate twice. The combined organic layers were dried over anhydrous sodium sulfate and concentrated under reduced pressure. The residue was purified by flash-column (15g silica Macherey Nagel) chromatography (cyclohexane - ethyl acetate 3 / EtOH 1, 9:1 to 0:1) to give a solid (70 mg), which was triturated in diisopropyl ether and filtered affording 8,14-dioxa-4,5,10,19,20-pentaazatetracyclo[13.5.2.1^{2,5}.0^{18,21}]tricosa-1(20),2(23), 3,15(22),16, 18(21)-hexaen-9-one **example 51** as a white solid.
LCMS method F: [M+H]⁺ = 328.1, t_{R} = 1.68 min
LCMS method G: [M+H]⁺ = 328.1, t_{R} = 1.68 min
¹H NMR (400 MHz, d6-DMSO) δ 12.82 (1H, s), 8.09 (1H, s), 7.86 (1H, t, J = 6.1 Hz), 7.77 (1H, d, J = 0.6 Hz), 7.44 - 7.41 (1H, m), 7.07 (1H, d, J = 2.3 Hz), 6.94 (1H, dd, J = 2.3, 8.9 Hz), 4.53 - 4.49 (2H, m), 4.38 - 4.28 (4H, m), 3.14 - 3.09 (2H, m), 1.86 (2H, q, J = 8.7 Hz) ppm.

### Example 52: 4-[methyl(oxetan-3-yl)amino]-8,14-dioxa-10,19,20-triazatetracyclo [13.5.2.1^{2,6}.0^{18,21}]tricosa-1(20),2,4,6(23),15,17,21-heptaen-9-one

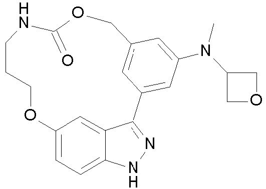

Example 52 is prepared according to the synthesis route described in general Scheme C. N-methyloxetan-3-amine is used for the Buchwald reaction with the bromide intermediate **34** to give 4-[methyl(oxetan-3-yl)amino]-8,14-dioxa-10,19,20-triazatetracyclo[13.5.2.1^{2,6}.0^{18,21}] tricosa-1(20),2,4,6(23),15,17,21-heptaen-9-one **example 52.**
LCMS method F: [M+H]⁺ = 409, t_{R} = 2.04 min
LCMS method G: [M+H]⁺ = 409, t_{R} = 2.06 min
¹H NMR (400 MHz, d6-DMSO) δ 12.81 (1H, s), 7.62 (1H, s), 7.46 (1H, d), 7.34 (2H, s), 7.13 (1H, s), 6.98 - 6.95 (1H, m), 6.64 (1H, s), 5.22 (2H, m), 4.84 - 4.81 (2H, m), 4.77 - 4.74 (1H, m), 4.65 - 4.64 (2H, m), 4.32 - 4.28 (2H, m), 3.16 (2H, m), 2.96 (3H, s), 2.03 (2H, m) ppm.

### Example 53: 4-[(dimethylamino)methyl]-8,14-dioxa-10,19,20-triazatetracyclo [13.5.2.1^{2,6}.0^{18,21}] tricosa-1(20),2,4,6(23),15,17,21-heptaen-9-one

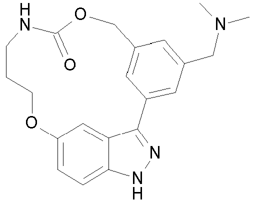

Example 53 is prepared according to the synthesis route described in general Scheme C. Potassium (dimethylamino)methyltrifluoroborate was used for the Suzuki coupling with the bromide intermediate **34** to give 4-[(dimethylamino)methyl]-8,14-dioxa-10,19,20-triazatetracyclo[13.5.2.1^{2,6}.0^{18,21}]tricosa-1(20),2,4,6(23),15,17,21-heptaen-9-one **example 53.**
LCMS method F: [M+H]⁺ = 381, t_{R} = 1.39 min
LCMS method G: [M+H]⁺ = 381, t_{R} = 2.03 min
¹H NMR (400 MHz, d6-DMSO) δ 12.93 (1H, s), 7.87 (2H, m), 7.70 - 7.66 (1H, m), 7.51 - 7.47 (1H, m), 7.36 (1H, d, J = 2.1 Hz), 7.25 (1H, s), 6.99 (1H, dd, J = 2.3, 9.1 Hz), 5.30 - 5.26 (2H, m), 4.34 - 4.30 (2H, m), 3.73 (2H, m), 3.17 (2H, s), 2.40 - 2.33 (6H, m), 2.06 (2H, s) ppm.

### Example 54: 4,10-dimethyl-8,14-dioxa-10,19,20-triazatetracyclo[13.5.2.1^{2,6}.0^{18,21}] tricosa-1(20),2,4,6(23),15,17,21-heptaen-9-one

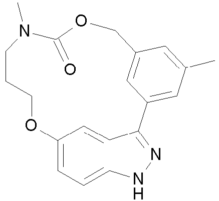

Example 54 is prepared according to the synthesis route described in general Scheme F.

### Preparation of intermediate 52: 4,10-dimethyl-19-(oxan-2-yl)-8,14-dioxa-10,19,20-triazatetracyclo[13.5.2.1²,⁶.0^{18,21}]tricosa-1(20),2,4,6(23),15,17,21-heptaen-9-one

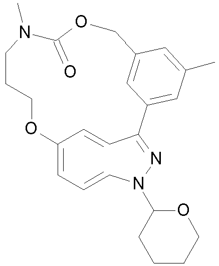

To a mixture of 4-methyl-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one **example 18** (115 mg, 0.273 mmol) in THF (2.5 mL) at 0°C was added NaH 60% in oil dispersion (8 mg, 0.328 mmol) and MeI (20 µL, 0.328 mmol). The reaction mixture was stirred overnight at RT. More NaH 60% in oil dispersion (8 mg, 0.328 mmol) and MeI (20 µL, 0.328 mmol) were added. The reaction mixture was stirred overnight at RT. The solvent was removed under reduced pressure, EtOAc and water were added. The layers were separated, the aqueous one was extracted with ethyl acetate. The organic layers were combined and the solvent was removed under reduced pressure to give 4,10-dimethyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **52** as a colorless oil.
LCMS method F: [M+H]⁺ = 436.2, t_{R} = 3.15 min

### Preparation of Example 54: 4,10-dimethyl-8,14-dioxa-10,19,20-triazatetracyclo [13.5.2.1^{2,6}.0^{18,21}]tricosa-1(20),2,4,6(23),15,17,21-heptaen-9-one

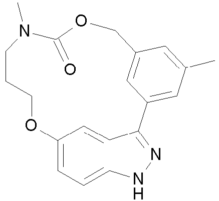

To a mixture of 4,10-dimethyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **52** (150 mg; 0.345 mmol) in DCM (2.5 mL) was added TFA (132 µl, 1.723 mmol). The reaction mixture was stirred under microwave conditions at 80°C during 60 min. The solvent was removed under reduced pressure, the mixture was dissolved in EtOAc and washed with a saturated solution of 1 N NaHCO₃ (pH=7), then with water. The organic layer was concentrated under reduced pressure the oil was purified by chromatography using a 10 g SiO₂ column eluted with DCM / MeOH 100/0 to 95/5. The desired fractions were combined but the product is not enough pure it was re-purified by chromatography using a 10 g SiO₂ column eluted with cyclohexane/Ethyl acetate 70/30 to 50/50. The desired fractions were combined, and the solvent was removed under reduced pressure then the oil was triturated with pentane. The solid was filtered and boiled in hot water, it was filtered and dried under high vacuum to give 4,10-dimethyl-8,14-dioxa-10,19,20-triazatetracyclo[13.5.2.1^{2,6}.0^{18,21}]tricosa-1(20),2,4,6(23),15,17,21-heptaen-9-one **example 54** as a white powder.
LCMS method F: [M+H]⁺ = 352.2, t_{R} = 2.49 min
LCMS method G: [M+H]⁺ = 352.2, t_{R} = 2.49 min

The ¹H NMR analysis showed the presence of rotamers.
¹H NMR (400 MHz, d6-DMSO) δ 13.11 - 13.05 (1H, m), 7.68 (2H, d, J = 13.7 Hz), 7.51 - 7.47 (1H, m), 7.20 - 7.12 (2H, m), 6.99 (1H, dd, J = 2.2, 9.0 Hz), 5.82 (0.75H, d, J = 13.3 Hz), 5.15 (0.25H, s), 4.78 (0.75H, d, J = 13.5 Hz), 4.43 - 4.35 (0.75H, m), 4.28 - 4.12 (1.25H, m), 3.94 - 3.84 (0.75H, m), 3.47 - 3.39 (0.25H, m), 3.04 - 3.03 (3H, m), 2.91 - 2.82 (1.25H, m), 2.41 - 2.39 (4H, m), 2.27 - 2.16 (0.25H,m), 1.77 - 1.70 (0.75H, m) ppm.

### Example 55: 4-(propan-2-yloxy)-8,14-dioxa-10,19,20-triazatetracyclo[13.5.2.1^{2,6}.0^{18,21}] tricosa-1(20),2,4,6(23),15,17,21-heptaen-9-one

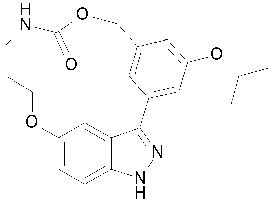

Example 54 is prepared according to the synthesis route described in general Scheme F.

### Preparation of intermediate 53: methyl 3-[5-[3-(benzyloxycarbonylamino)propoxy]-1-tetrahydropyran-2-yl-indazol-3-yl]-5-hydroxy-benzoate

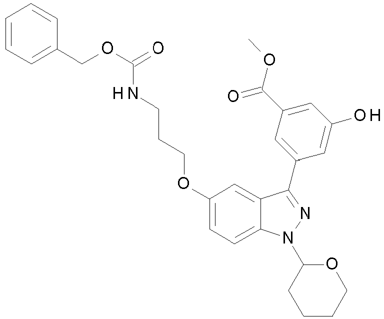

To a degassed solution of benzyl N-[3-(3-iodo-1-tetrahydropyran-2-yl-indazol-5-yl)oxypropyl]carbamate **26** (2.876 g, 5.372 mmol), methyl 3-hydroxy-5-(4,4,5,5-tetramethyl-1,3,2-dioxaborolan-2-yl)benzoate (2.988 g, 10.473 mmol), XPhos (256 mg, 0.537 mmol) and K₃PO₄(3.421 g, 16.116 mmol) in dioxane (40.0 mL) and water (10.0 mL) was added Pd(PPh₃)₄ (311 mg, 0.269 mmol). The resulting cloudy brown solution was degassed with nitrogen gas for 5 minutes and separated in three batches, sealed and heated to 120°C under microwave irradiation for 1 h each. The mixture was poured into water (50 mL), EtOAc (100 mL) was added and the phases were separated. The aqueous layer was extracted with EtOAc (3 x 100 mL) and the combined organic extracts were washed with a saturated aqueous NaCl solution (1 x 50 mL), dried over anhydrous Na₂SO₄, filtered and concentrated under reduced pressure. The resulting crude material (brown oil, 4.1 g) was purified by column chromatography (220 g Macherey Nagel SiO₂, 100 mL/min, CyH/EtOAc 100:0 to 60:40) to afford methyl 3-[5-[3-(benzyloxycarbonylamino)propoxy]-1-tetrahydropyran-2-yl-indazol-3-yl]-5-hydroxybenzoate **53** as a brown oil.
LCMS method F: [M+H]⁺ = 560.1, t_{R} = 2.97 min

### Preparation of intermediate 54: methyl 3-[5-[3-(benzyloxycarbonylamino)propoxy]-1-tetrahydropyran-2-yl-indazol-3-yl]-5-isopropoxy-benzoate

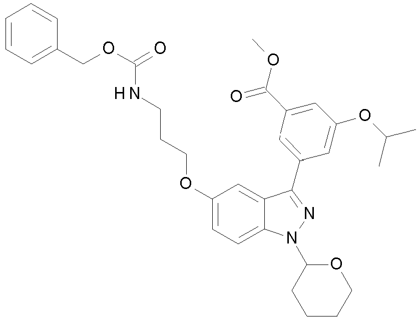

To a solution of methyl 3-[5-[3-(benzyloxycarbonylamino)propoxy]-1-tetrahydropyran-2-yl-indazol-3-yl]-5-hydroxy-benzoate **53** (2.800 g, 5.004 mmol) and K₂CO₃ (1.729 g, 12.510 mmol) in N,N-dimethylformamide (25.0 mL) was added 2-bromopropane (940 µL, 1.231 mg, 10.008 mmol). The resulting cloudy brown solution was heated to 70°C for 2 h. The reaction was quenched with water (20 mL), EtOAc (50 mL) was added and phases were separated. The aqueous layer was extracted with EtOAc (3 x 50 mL) and the combined organic extracts were washed with saturated aqueous NaCl solution (1 x 20 mL), dried over anhydrous Na₂SO₄, filtered and the solvent was removed under reduced pressure. The resulting crude material (brown solid, 3.5 g) was purified by column chromatography (120 g Macherey Nagel SiO₂, CyH/EtOAc 100;0 to 70;30) to afford methyl 3-[5-[3-(benzyloxycarbonylamino)propoxy]-1-tetrahydropyran-2-yl-indazol-3-yl]-5-isopropoxy-benzoate 54 as a brown solid.
LCMS method F: [M+H]⁺ = 602.3, t_{R} = 3.48 min

### Preparation of intermediate 55: benzyl N-[3-[3-[3-(hydroxymethyl)-5-isopropoxy-phenyl]-1-tetrahydropyran-2-yl-indazol-5-yl]oxypropyl]carbamate

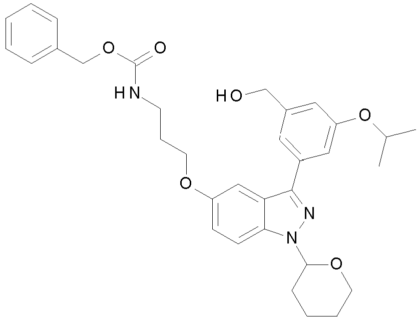

To a solution of methyl 3-[5-[3-(benzyloxycarbonylamino)propoxy]-1-tetrahydropyran-2-yl-indazol-3-yl]-5-isopropoxy-benzoate **54** (3.000 g, 4.986 mmol) in THF (50.0 mL) at 0°C was added dropwise LiAlH₄ (1.0 M in THF, 9.97 mL, 9.972 mmol). The resulting brown solution was stirred at 0°C for 15 minutes, then at room temperature for 1 h. The reaction was carefully quenched with saturated aqueous Rochelle's salt (20 mL), EtOAc (50 mL) was added and the phases were separated. The aqueous layer was extracted with EtOAc (3 x 50 mL) and the combined organic extracts were washed with a saturated aqueous NaCl solution (1 x 50 mL), dried over anhydrous Na₂SO₄, filtered and concentrated under reduced pressure to afford crude benzyl N-[3-[3-[3-(hydroxymethyl)-5-isopropoxy-phenyl]-1-tetrahydropyran-2-yl-indazol-5-yl]oxypropyl]carbamate **55** as a brown oil which was used in the next step without further purification.
LCMS method F: [M+H]⁺ = 574.2, t_{R} = 3.06 min

### Preparation of intermediate 56: 19-(oxan-2-yl)-4-(propan-2-yloxy)-8,14-dioxa-10,19,20-triazatetracyclo[13.5.2.1^{2,6}.0^{18,21}]tricosa-1(20),2,4,6(23),15,17,21-heptaen-9-one

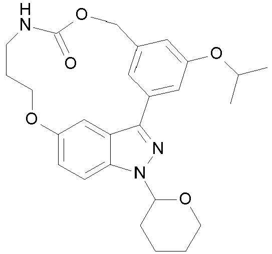

To a solution of benzyl N-[3-[3-[3-(hydroxymethyl)-5-isopropoxy-phenyl]-1-tetrahydropyran-2-yl-indazol-5-yl]oxypropyl]carbamate **55** (100 mg, 0.174 mmol) in MeCN (18.0 mL) was added Cs₂CO₃ (341 mg, 1.046 mmol). The resulting cloudy yellow mixture was heated under reflux for 5 h. The mixture was cooled to room temperature, filtered and concentrated under reduced pressure. The resulting crude material (yellow oil, 100 mg) was purified by column chromatography (4 g Macherey Nagel SiO₂, 15 mL/min, CH₂Cl₂/MeOH 100:0 to 98:2) to afford 19-(oxan-2-yl)-4-(propan-2-yloxy)-8,14-dioxa-10,19,20-triazatetracyclo[13.5.2.1^{2,6}.0^{18,21}] tricosa-1(20),2,4,6(23),15,17,21-heptaen-9-one **56** as a transparent oil.
LCMS method F: [M+H]⁺ = 466.2, t_{R} = 3.03 min

### Preparation of Example 55: 4-(propan-2-yloxy)-8,14-dioxa-10,19,20-triazatetracyclo [13.5.2.1^{2,6}.0^{18,21}]tricosa-1(20),2,4,6(23),15,17,21-heptaen-9-one

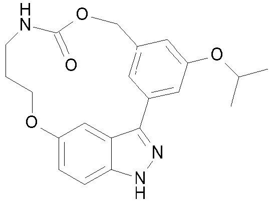

To a solution of 19-(oxan-2-yl)-4-(propan-2-yloxy)-8,14-dioxa-10,19,20-triazatetracyclo [13.5.2.1^{2,6}.0^{18,21}]tricosa-1(20),2,4,6(23),15,17,21-heptaen-9-one **56** (54 mg, 0.116 mmol) in CH₂Cl₂ (5.0 mL) was added TFA (300 µL, 447 mg, 3.920 mmol). The vial containing the resulting clear yellow solution was sealed and heated to 50°C under microwave irradiation for 3 h. Saturated aqueous NaHCO₃ (1 mL) was added and phases were separated. The aqueous layer was extracted with CH₂Cl₂ (3 x 5 mL) and the combined organic extracts were washed with water (1 x 5 mL), dried over anhydrous Na₂SO₄, filtered and concentrated under reduced pressure. The resulting crude material (pale yellow oil, 49 mg) was triturated with *i*Pr₂O to afford 4-(propan-2-yloxy)-8,14-dioxa-10,19,20-triazatetracyclo[13.5.2. 1^{2,6}.0^{18,21}]tricosa-1(20),2,4,6(23),15,17,21-heptaen-9-one example 55 as a white amorphous solid.
LCMS method F: [M+H]⁺ = 382.1, t_{R} = 2.41 min
LCMS method G: [M+H]⁺ = 382.2, t_{R} = 2.40 min
¹H NMR (400 MHz, d6-DMSO, 80°C) δ 7.66 (brs, 1H), 7.49 - 7.46 (m, 2H), 7.39 - 7.34 (m, 2H), 6.98 (dd, *J =* 2.4, 9.0 Hz, 1H), 6.85 (brs, 1H), 5.24 (brs, 1H), 4.72 - 4.63 (sept, *J* = 5.9 Hz, 1H), 4.33 - 4.29 (m, 2H), 3.19 - 3.15 (m, 2H), 2.04 - 2.02 (m, 2H), 1.35 - 1.33 (m, 6H) ppm. Two labile protons were not visible in this solvent.

### Example 56: 4-fluoro-7-methyl-8,14-dioxa-10,19,20-triazatetracyclo[13.5.2.1^{2,6}.0^{18,21}] tricosa-1(20),2,4,6(23),15,17,21-heptaen-9-one

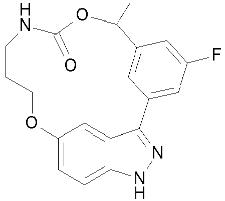

Example 56 is prepared according to the synthesis route described in general Scheme A.

### Preparation of intermediate 57: 1-(3-bromo-5-fluoro-phenyl)ethanol

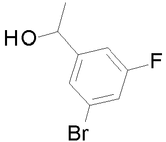

To a cooled solution of 3-bromo-5-fluorobenzaldehyde (1.5 g, 7.389 mmol) in dry tetrahydrofuran (19 mL) was added dropwise methylmagnesium bromide solution 3M in diethyl ether (4.93 mL, 14.778 mmol) at 0°C. The reaction mixture was stirred at 0°C for 20 min then RT for 16 h. The reaction mixture was quenched with a saturated aqueous solution of NH₄Cl then extracted with ethyl acetate (2 x). The combined organic layers were washed with water then brine, dried over sodium sulfate and concentrated under reduced pressure. The crude product was purified by flash-column chromatography eluting with Cyclohexane/Ethyl acetate-EtOH (3-1): 100 / 0 to 80 / 20, to give 1-(3-bromo-5-fluoro-phenyl)ethanol 57 as a colorless oil.
LCMS method F: [M+H]⁺ = mass not detected, t_{R} = 2.32 min

### Preparation of intermediate 58: 1-[3-fluoro-5-(4,4,5,5-tetramethyl-1,3,2-dioxaborolan-2-yl)phenyl]ethanol

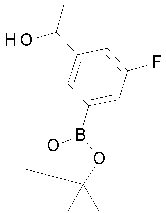

To a degassed solution in a sealed tube of 1-(3-bromo-5-fluoro-phenyl)ethanol **57** (1.196 g, 5.461 mmol), bis(pinacolato)diboron (2.080 g, 8.192 mmol) and potassium acetate (2.144 g, 21.844 mmol) in dioxane (17 mL) was added PdCl₂(dppf) CH₂Cl₂ (0.446 g, 0.546 mmol). The reaction mixture was heated at 90°C for 24h. The reaction mixture was filtered over celite on Whatman paper and rinsed with ethyl acetate. The reaction mixture was diluted with water and extracted with ethyl acetate (3 x). The combined organic layers were washed with water and brine, dried over anhydrous sodium sulfate and concentrated under reduced pressure to give 1-[3-fluoro-5-(4,4,5,5-tetramethyl-1,3,2-dioxaborolan-2-yl)phenyl]ethanol **58** as a black oil.
LCMS method F: no m/z detected, t_{R} = 2.65 min.

### Preparation of intermediate 59: benzyl N-[3-[3-[3-fluoro-5-(1-hydroxyethyl)phenyl]-1-tetrahydropyran-2-yl-indazol-5-yl]oxypropyl]carbamate

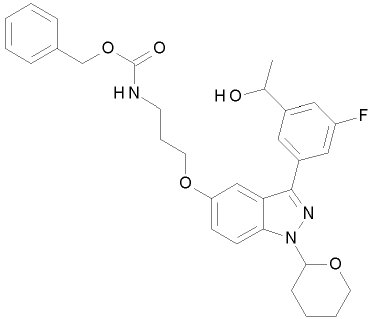

To a degassed solution of benzyl N-[3-(3-iodo-1-tetrahydropyran-2-yl-indazol-5-yl)oxypropyl]carbamate **26** (1.462 g, 2.734 mmol), 1-[3-fluoro-5-(4,4,5,5-tetramethyl-1,3,2-dioxaborolan-2-yl)phenyl]ethanol **58** (1.453 g, 5.466 mmol), tripotassium phosphate (1.742 g, 8.202 mmol) and xPhos (0.130 g, 0.274 mmol) in dioxane (14.6 mL) and water (8.8 mL) was added tetrakis(triphenylphosphine)palladium(0) (0.158 g, 0.137 mmol). The reaction mixture was irradiated under microwaves (Biotage initiator+) at 120°C for 1h. The reaction mixture was filtered over celite and the celite was washed with ethyl acetate. The filtrate was then diluted with water and extracted with ethyl acetate (3x). The combined organic layers were washed with water and brine, dried over sodium sulfate and concentrated under reduced pressure. The crude was purified by column chromatography eluting with DCM / ethyl acetate, 100/0 to 80/20 to give benzyl N-[3-[3-[3-fluoro-5-(1-hydroxyethyl)phenyl]-1-tetrahydropyran-2-yl-indazol-5-yl]oxypropyl]carbamate **59** as a cream solid.
Yield: 780 mg of intermediate 59 (50%)
LCMS method F: [M+H]⁺ = 548, t_{R} = 3.07 min

### Preparation of intermediate 60: 1-[3-[5-(3-aminopropoxy)-1-tetrahydropyran-2-yl-indazol-3-yl]-5-fluoro-phenyl]ethanol and 4-fluoro-7-methyl-19-(oxan-2-yl)-8,14-dioxa-10,19,20-triazatetracyclo[13.5.2.1²,⁶.0^{18,21}]tricosa-1(20),2(23),3,5,15(22),16,18(21)-heptaen-9-one

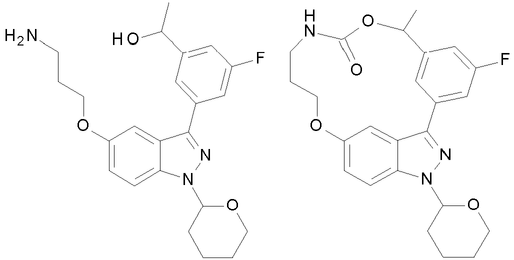

A suspension of benzyl N-[3-[3-[3-fluoro-5-(1-hydroxyethyl)phenyl]-1-tetrahydropyran-2-yl-indazol-5-yl]oxypropyl]carbamate **59** (0.780 g, 1.426 mmol) and cesium carbonate (2.781 g, 8.556 mmol) in acetonitrile (300 mL) was heated to 90°C for 16h. LCMS analysis showed formation of the desired product but starting material remained and the formation of 1-[3-[5-(3-aminopropoxy)-1-tetrahydropyran-2-yl-indazol-3-yl]-5-fluoro-phenyl]ethanol was observed. The reaction mixture was heated to 90°C for 16h. The reaction mixture was cooled to RT then filtered and concentrated under reduced pressure to give a mix of 1-[3-[5-(3-aminopropoxy)-1-tetrahydropyran-2-yl-indazol-3-yl]-5-fluoro-phenyl]ethanol (66 %) and 4-fluoro-7-methyl-19-(oxan-2-yl)-8,14-dioxa-10,19,20-tiiazatetracyclo[13.5.2.1^{2,6}.0^{18,21}]tricosa-1(20),2(23),3,5,15(22),16,18(21)-heptaen-9-one (26 %) (0.667 g, 1.426 mmol (postulated)) as an orange oil. The crude product was not purified, it was engaged in the next step without further purification.

### Preparation of intermediate 61: 4-fluoro-7-methyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one

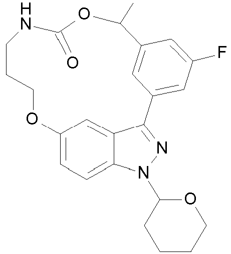

To a solution of 1-[3-[5-(3-aminopropoxy)-1-tetrahydropyran-2-yl-indazol-3-yl]-5-fluorophenyl]ethanol and 4-fluoro-7-methyl-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one **60** (0.567 g, 1.373 mmol) in DMA (350 mL) was added 1,1'-carbonyldiimidazole (0.245 g, 1.510 mmol). The reaction mixture was stirred at RT for 2h then 90°C for 22h. The reaction mixture was concentrated under reduced pressure and ethyl acetate and a saturated aqueous solution of NaHCO₃ were added. The mixture was extracted with ethyl acetate (2 x). The combined organic layers were washed with water and brine, dried over sodium sulfate, filtered and concentrated under reduced pressure.

The crude product was purified by column chromatography eluting with Cyclohexane / Ethyl acetate - EtOH (3-1) : 100/0 to 70/30 to give a cream solid. The solid was triturated from diisopropyl ether to give 4-fluoro-7-methyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **61** as a white solid.
Yield: 100 mg of intermediate 61 (14%)
LCMS method F: [M+H]⁺ = 440, t_{R} = 2.96 min

### Preparation of example 56: 4-fluoro-7-methyl-8,14-dioxa-10,19,20-triazatetracyclo [13.5.2.1^{2,6}.0^{18,21}]tricosa-1(20),2,4,6(23),15,17,21-heptaen-9-one

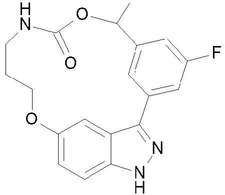

To a solution of 4-fluoro-7-methyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one 61 (100 mg, 0.228 mmol) in DCM (16 mL) was added trifluoro acetic acid (350 µL, 4.560 mmol) at RT. The reaction mixture was irradiated under microwave conditions (Biotage initiator). The solid was triturated from diisopropyl ether to give 4-fluoro-7-methyl-8,14-dioxa-10,19,20-triazatetracyclo [13.5.2.1^{2,6}.0^{18,21}]tricosa-1(20),2,4,6(23),15,17,21-heptaen-9-one **example 56** as a cream solid.
LCMS method F: [M+H]⁺ = 356, t_{R} = 2.33 min
LCMS method G: [M+H]⁺ = 356, t_{R} = 2.32 min
¹H NMR (400 MHz, d6-DMSO) δ 13.26 (1H, s), 8.01 - 7.98 (1H, m), 7.69 (1H, s), 7.59 - 7.56 (1H, m), 7.53 - 7.50 (1H, m), 7.33 (1H, m), 7.22 - 7.18 (1H, m), 7.02 - 6.99 (1H, m), 5.91 - 5.86 (1H, m), 4.35 - 4.28 (2H, m), 3.56 - 3.49 (1H, m), 2.79 - 2.72 (1H, m), 2.21 - 2.16 (1H, m), 1.77 - 1.71 (1H, m), 1.61 - 1.58 (3H, d) ppm.

### Example 57: 4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one

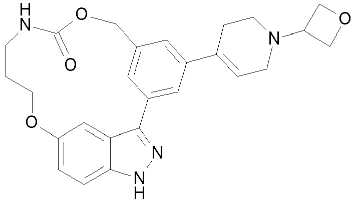

Example 57 is prepared according to the synthesis route described in general Scheme C.

### Preparation of intermediate 62: 1-(oxetan-3-yl)-4-(4,4,5,5-tetramethyl-1,3,2-dioxaborolan-2-yl)-3,6-dihydro-2H-pyridine

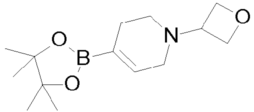

To a solution of 4-(4,4,5,5-tetramethyl-1,3,2-dioxaborolan-2-yl)-1,2,3,6-tetrahydropyridine (370 mg, 1.77 mmol) in DCM (9 mL), triethylamine (245 µL, 1.77 mmol) and 3-bromooxetane (750 mg, 5.5 mmol) were added. The resulting mixture was stirred at room temperature for 2 days. The reaction mixture was evaporated under reduced pressure to give 1-(oxetan-3-yl)-4-(4,4,5,5-tetramethyl-1,3,2-dioxaborolan-2-yl)-3,6-dihydro-2H-pyridine **62** (500 mg, 1.77 postulated) as an orange oil. The compound was used without further purification in the next step.

### Preparation of intermediate 63: 19-(oxan-2-yl)-4-(1,2,3,6-tetrahydropyridin-4-yl)-8,14-dioxa-10,19,20-triazatetracyclo[13.5.2.1^{2,6}.0^{18,21}]tricosa-1(20),2,4,6(23),15,17,21-heptaen-9-one

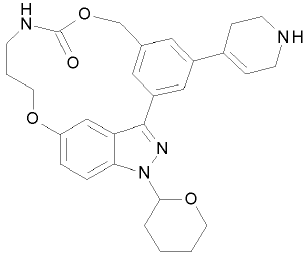

To a solution of 4-bromo-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one intermediate **34** (260 mg, 0.53 mmol) in dioxane/water (15/1.5 mL), 1-(oxetan-3-yl)-4-(4,4,5,5-tetramethyl-1,3,2-dioxaborolan-2-yl)-3,6-dihydro-2H-pyridine **62** (300 mg, 1.06 mmol postulated) and K₃PO₄ (337 mg, 1.59 mmol) were added. The mixture was degassed during 10 minutes, then Pd(dppf)Cl₂. DCM (17 mg, 0.021 mmol) was added. The mixture was heated at 90 °C for 20 hours. Monitoring by LCMS analysis showed formation of the expected product without oxetane. The reaction mixture was cooled to room temperature, then more 1-(oxetan-3-yl)-4-(4,4,5,5-tetramethyl-1,3,2-dioxaborolan-2-yl)-3,6-dihydro-2H-pyridine (200 mg, 0.71 mmol postulated) and K₃PO₄ (168 mg, 0.79 mmol) were added. The mixture was degassed during 10 minutes and more Pd(dppf)Cl₂. DCM (8 mg, 0.0098 mmol) was added. The mixture was heated at 90 °C for 1 days. The reaction mixture was filtered over celite, diluted with EtOAc (50 mL) and water (50 mL). After separation, the aqueous layer was extracted with EtOAc (2 x 50 mL). The combined organic layers were washed with brine (50 mL), dried over anhydrous sodium sulfate, filtered and concentrated under reduced pressure. The crude was purified by column (Macherey Nagel, 25 g) chromatography with DCM/(MeOH/NH3) (100/0 to 90/10). The desired fractions were collected, combined and evaporated to give 19-(oxan-2-yl)-4-(1,2,3,6-tetrahydropyridin-4-yl)-8,14-dioxa-10,19,20-triazatetracyclo[13.5.2.1^{2,6}.0^{18,21}]tricosa-1(20),2, 4,6(23),15,17,21-heptaen-9-one **63** as an orange solid.
LCMS method F: [M+H]⁺ = 489, t_{R} = 1.81 min

### Preparation of intermediate 64: 19-(oxan-2-yl)-4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one

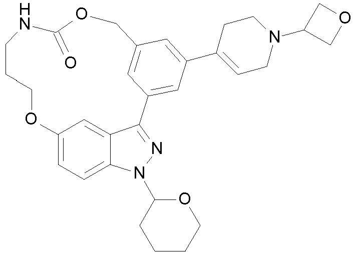

To a solution of 19-(oxan-2-yl)-4-(1,2,3,6-tetrahydropyridin-4-yl)-8,14-dioxa-10,19,20-triazatetracyclo[13.5.2.1^{2,6}.0^{18,21}]tricosa-1(20),2,4,6(23),15,17,21-heptaen-9-one **63** (289 mg, 0.59 mmol) in dry THF (15 mL), oxetan-3-one (212 mg, 2.95 mmol) was added. The mixture was cooled to 0 °C then sodium tris(acetoxy)borohydride (248 mg, 1.18 mmol) was added. The reaction mixture was stirred at room temperature for 18 hours. The reaction mixture was quenched with Na₂CO₃ 1M (~ 7 mL, pH = 8), then the mixture was diluted with EtOAc (50 mL). After separation, the aqueous layer was extracted with EtOAc (3 x 50 mL). The combined organic layers were washed with brine (50 mL), dried over anhydrous sodium sulfate, filtered and concentrated under reduced pressure. The crude was purified by column (Macherey Nagel, 15 g) flash chromatography with DCM/MeoH (100/0 to 97/3) as eluent, to give 19-(oxan-2-yl)-4-[1-(oxetan-3-yl)-1,2,3,6-tetrahydropyridin-4-yl]-8,14-dioxa-10, 19,20-triazatetracyclo [13.5.2.1^{2,6}.0^{18,21}]tricosa-1(20),2,4,6(23),15,17,21-heptaen-9-one **64** as a white crystals.
LCMS method F: [M+H]⁺ = 545, t_{R} = 1.84 min

### Preparation of example 57: 4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one

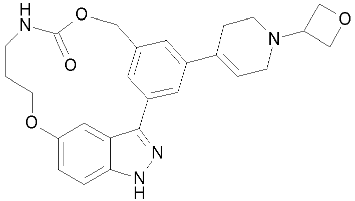

To a solution of 19-(oxan-2-yl)-4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one **64** (82 mg, 0.15 mmol) in DCM (4 mL) was added trifluoro acetic acid (107 µL, 1.4 mmol). The mixture was stirred at room temperature for 24 hours. The reaction mixture was then heated at 40°C for 4 hours. More trifluoro acetic acid (26 µL, 0.35 mmol) was added and the reaction mixture was heated at 40 °C for 3 hours and at room temperature overnight. The reaction mixture was diluted with DCM (25 mL) and a saturated NaHCO₃ solution (25 mL). After separation, the aqueous layer was extracted with DCM (3 x 20 mL). The combined organic layers were washed with brine (25 mL), dried over anhydrous sodium sulfate, filtered and the solvent was removed under reduced pressure. The crude was triturated in acetonitrile, filtered and the solid was washed several times with acetonitrile to give 4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one **example 57** as a cream powder.
LCMS method F: [M+H]⁺ = 461, t_{R} = 1.49 min
LCMS method G: [M+H]⁺ = 461, t_{R} = 2.19 min
¹H NMR (400 MHz, d6-DMSO, 80 °C) δ 12.89 (1H, m), 7.90 (1H, s), 7.80 (1H, s), 7.67 (1H, m), 7.48 (1H, d, J = 9.5 Hz), 7.35 (2H, m), 6.98 (1H, dd, J = 1.5, 8.9 Hz), 6.23 (1H, m), 5.29 (2H, m), 4.61 (2H, t, J = 6.5 Hz), 4.55 (2H, t, J = 5.9 Hz), 4.31 (2H, t, J = 9.3 Hz), 3.65 (1H, t, J = 6.1 Hz), 3.18 (2H, m), 3.09 (2H, m), 2.61 (2H, m), 2.57 (2H, m), 2.04 (2H, m) ppm.

### Example 58: 4-(3-methylpiperidin-1-yl)-8,14-dioxa-10,19,20-triazatetracyclo [13.5.2.1^{2,6}.0^{18,21}]tricosa-1(20),2,4,6(23),15,17,21-heptaen-9-one

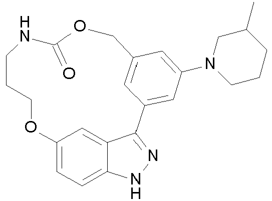

Example 58 is prepared according to the synthesis route described in general Scheme C. 3-Methylpiperidine is used for the Buchwald reaction with the bromide intermediate 34 to give 4-(3-methylpiperidin-1-yl)-8,14-dioxa-10,19,20-triazatetracyclo[13.5.2.1^{2,6}.0^{18,21}]tricosa-1(20),2,4,6(23),15,17,21-heptaen-9-one **example 58.**
LCMS method F: [M+H]⁺ = 421.2, t_{R} = 1.88 min
LCMS method G: [M+H]⁺ = 421.2, t_{R} = 2.66 min
¹H NMR (400 MHz, d6-DMSO) δ 7.69 - 7.56 (1H , m), 7.48 - 7.45 (1H, m), 7.38 - 7.31 (3H, m), 6.95 (1H, dd, J = 2.4, 9.0 Hz), 6.87 - 6.86 (1H, m), 5.24 - 5.20 (2H, m), 4.30 (2H, dd, J = 8.0, 9.1 Hz), 3.22 - 3.1 (2H, m), 3.07 (6H, s), 2.79 - 2.68 (1H, m), 2.07 - 1.98 (2H, m) 1.83 - 1.74 (3H, m), 1.7 - 1.55 (1H, m), 1.19 - 1.05 (1H, m) ppm. The indazole NH proton was not visible in this solvent.

### Example 59: 4-[(3S)-3-hydroxypyrrolidin-1-yl]-8,14-dioxa-10,19,20-triazatetracyclo [13.5.2.1^{2,6}.0^{18,21}]tricosa-1(20),2,4,6(23),15,17,21-heptaen-9-one

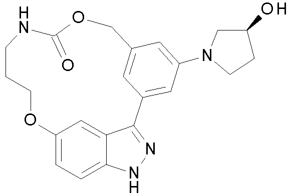

Example 59 is prepared according to the synthesis route described in general Scheme C. (3S)-pyrrolidin-3-ol is used for the Buchwald reaction with the bromide intermediate **34** to give 4-[(3S)-3-hydroxypyrrolidin-1-yl]-8,14-dioxa-10,19,20-triazatetracyclo[13.5.2.1^{2,6}.0^{18,21}]tricosa-1(20),2,4,6(23),15,17,21-heptaen-9-one **example 59.**
LCMS method F: [M+H]⁺ = 409.2, t_{R} = 1.98 min
LCMS method G: [M+H]⁺ = 409.2, t_{R} = 1.96 min
¹H NMR (400 MHz, d6-DMSO) δ 12.84 (1H, m), 7.61 - 7.60 (1H, m), 7.48 - 7.45 (1H, m), 7.36 (1H, d, J = 2.1 Hz), 7.21 - 6.93 (3H, m), 6.47 (1H, s), 5.25 - 5.21 (2H, m), 4.88 - 4.66 (1H, m) 4.48 - 4.45 (1H, m), 4.32 - 4.27 (2H, m), 3.53 - 3.32 (3H, m), 3.20 - 3.16 (3H, m), 2.16 - 2.07 (1H, m), 2.02 - 1.94 (3H, m) ppm.

### Example 60: 4-fluoro-8,14-dioxa-10,19,20-triazapentacyclo[13.5.2.1^{2,6}.1^{7,10}.0^{18,21}] tetracosa-1(20),2(24),3,5,15(22),16,18(21)-heptaen-9-one

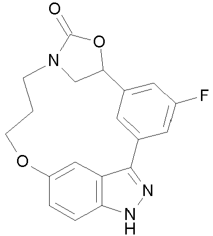

Example 60 is prepared according to the synthesis route described below.

### Preparation of intermediate 65: 1-(3-bromo-5-fluorophenyl)-2-nitroethan-1-ol

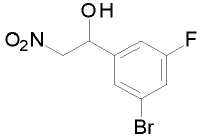

To a stirred solution of 3-bromo-5-fluorobenzaldehyde (2 g, 10 mmol) in THF (20 mL) was added dropwise at 0°C, nitromethane (0.536 mL, 10 mmol) and then dropwise sodium hydroxide solution 1N (10 mL, 10 mmol). The solution was stirred at 0°C for 15 min. The solution was quenched with a solution of acetic acid (12 mL). To the resulting mixture was added water (25 mL). The water layer was extracted with EtOAc (4 x 50 mL). The combined organic layers were washed with saturated brine (2 x 50 mL). The organic layer was dried over sodium sulfate, filtered and the solvent was removed under reduced pressure to afford brown/orange oil. This residue was purified by flash chromatography on silica gel (Macherey Nagel, 120 g) with gradient elution : cyclohexane/EtOAc 0-30 % to give 1-(3-bromo-5-fluorophenyl)-2-nitroethan-1-ol 65 as a white solid.
LCMS method F: [M-H]⁻ = 262.2, t_{R} = 2.28 min

### Preparation of intermediate 66: 2-amino-1-(3-bromo-5-fluorophenyl)ethan-1-ol

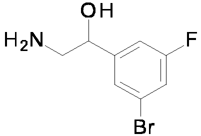

To a solution of 1-(3-bromo-5-fluorophenyl)-2-nitroethan-1-ol **65** (6.2 g, 15.2 mmol) in EtOH (100 mL) was added Raney^{®}-Nickel (2 g) and 0.5 mL of acetic acid. Dihydrogen was bubbled in the mixture for 5 min. The reaction mixture was stirred for 16 h under dihydrogen atmosphere. The reaction mixture was filtered over celite and the solvent of the filtrate was removed under reduced pressure to give a yellow solid as 2-amino-1-(3-bromo-5-fluorophenyl)ethan-1-ol 66 as a transparent oil used which is directly used in the next step without purification.
LCMS method F: [M+H]⁺ = 236, t_{R} = 1.12 min

### Preparation of intermediate 67: 5-(3-bromo-5-fluorophenyl)-1,3-oxazolidin-2-one

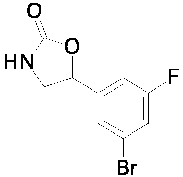

To a solution of 2-amino-1-(3-bromo-5-fluorophenyl)ethan-1-ol **66** (1.75 g , 1.75 mmol) in THF (100 mL) were added 1,1'-carbonyldiimidazole (1.34 g, 8.25 mmol) and imidazole (0.561 g, 8.25 mmol). The reaction mixture was stirred at RT for 16h. To the reaction mixture was added a saturated aqueous solution of NH₄Cl (100 mL). The mixture was extracted with ethyl acetate (2 x 50 mL). The combined organic layers were washed with water and brine, dried over sodium sulfate, filtered and concentrated under reduced pressure. The crude product was purified by column chromatography on a Biotage eluting with cyclohexane / ethyl acetate (3-1) : 100/0 to 70/30 to give a white solid as 5-(3-bromo-5-fluorophenyl)-1,3-oxazolidin-2-one **67.**
LCMS method F: [M+H]⁺ = 262.0, t_{R} = 2.07 min

### Preparation of intermediate 68: 5-(3-bromo-5-fluorophenyl)-3-{3-[(tert-butyldimethylsilyl) oxy]propyl}-1,3-oxazolidin-2-one

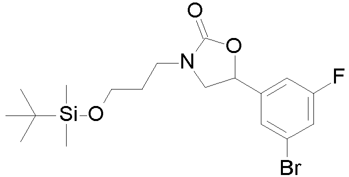

To a stirred solution of 5-(3-bromo-5-fluorophenyl)-1,3-oxazolidin-2-one **67** (1.6 g, 6.1 mmol) in THF (10 mL) was added sodium hydride (0.366 g, 9.1 mmol) at 0°C. The solution was stirred at 0°C for 10 min. Then a solution of (3-bromopropoxy)(tert-butyl)dimethylsilane (1.5 g, 6.1 mmol) in THF (10 mL) was added to the mixture. The mixture was stirred at room temperature for 16 h. The solution is quenched with a solution of saturated chloride ammonium (25 mL). The resulting mixture was extracted with EtOAc (4 x 100 mL). Combined organic layers were washed with saturated brine (2 x 50 mL). The organic layer was dried over sodium sulfate, filtered and the solvent was removed under reduced pressure to afford a brown/orange oil. This residue was purified by flash chromatography on silica gel (Macherey Nagel, 24 g) with gradient elution : cyclohexane/EtOAc 0-50 % to give 5-(3-bromo-5-fluorophenyl)-3-{3-[(tertbutyldimethylsilyl)oxy]propyl}-1,3-oxazolidin-2-one **68** as a yellow oil.
LCMS method F: [M+H]⁺ = 434.0, t_{R} = 3.42 min

### Preparation of intermediate 69: 5-(3-bromo-5-fluorophenyl)-3-(3-hydroxypropyl)-1,3-oxazolidin-2-one

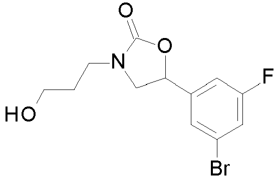

To a solution of 5-(3-bromo-5-fluorophenyl)-3-{3-[(tert-butyldimethylsilyl)oxy]propyl}-1,3-oxazolidin-2-one **68** (0.8 g, 1.85 mmol postulated) in tetrahydrofuran (50 mL) was added portion wise tetra-n-butylammonium fluoride 1.0 M in THF (1.85 mL, 1.85 mmol) at RT. The reaction mixture was stirred at RT for 3 h. The reaction mixture was poured into ice water (100 mL) and stirred for 10 min. The aqueous phase was extracted with ethyl acetate (2x100 mL). The combined organic layers were washed with brine (100 mL), dried over magnesium sulfate and concentrated under reduces pressure. The residue was purified by flash-column chromatography (24 g silica BIOTAGE) chromatography (cyclohexane - ethyl acetate, 100/0 to 50/50) affording 5-(3-bromo-5-fluorophenyl)-3-(3-hydroxypropyl)-1,3-oxazolidin-2-one 69 a beige powder.
LCMS method F: [M+H]⁺ = 320.0, t_{R} = 2.02 min

### Preparation of intermediate 70: 3-[5-(3-bromo-5-fluorophenyl)-2-oxo-1,3-oxazolidin-3-yl]propyl methane sulfonate

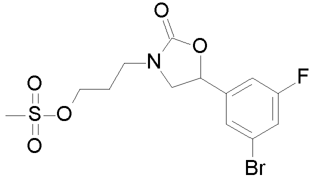

To a solution of 5-(3-bromo-5-fluorophenyl)-3-(3-hydroxypropyl)-1,3-oxazolidin-2-one **69** (0.5 g, 1.57 mmol) and diisopropylethylamine (0.545 mL, 3.14 mmol) in dichoromethane (50 mL) at 0 °C, was added dropwise methanesulfonyl chloride (0.145 mL,1.88 mmol). The reaction mixture was stirred at room temperature for 4 hours. The reaction mixture was washed with a saturated solution of ammonium chloride, with a saturated solution of sodium bicarbonate and brine, filtered and the solvent was removed under reduced pressure to give 3-[5-(3-bromo-5-fluorophenyl)-2-oxo-1,3-oxazolidin-3-yl]propyl methane sulfonate **70** as a colorless oil.
LCMS method F: [M+H]⁺ = 397.9, t_{R} = 2.36 min

### Preparation of intermediate 71: 5-(3-bromo-5-fluorophenyl)-3-(3-{[1-(oxan-2-yl)-1H-indazol-5-yl]oxy}propyl)-1,3-oxazolidin-2-one

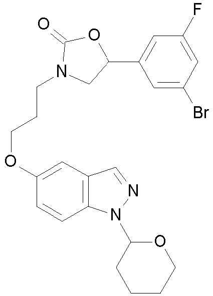

To a solution of 3-[5-(3-bromo-5-fluorophenyl)-2-oxo-1,3-oxazolidin-3-yl]propyl methane sulfonate **70** (0.618 g, 1.57 mmol) in *N,N*-dimethylformamide (100 mL), cesium carbonate (1.02 g, 3.14 mmol) and 1-(oxan-2-yl)-1H-indazol-5-ol 29 (0.343 g, 1.57 mmol) were added. The reaction was stirred at 80°C for 16 hours. The mixture was concentrated under reduced pressure. Water (200 mL) was added and the resulting mixture was extracted with EtOAc (4 x 100 mL). The combined organic layers were washed with brine (2 x 50 mL). The organic layers were dried over sodium sulfate, filtered and concentrated under reduced pressure to afford a brown/orange oil. This residue was purified by flash chromatography on silica gel (Macherey Nagel, 120 g) with gradient elution : cyclohexane/EtOAc 0-70 % to give 5-(3-bromo-5-fluorophenyl)-3-(3-{[1-(oxan-2-yl)-1H-indazol-5-yl]oxy}propyl)-1,3-oxazolidin-2-one 71 as a white solid.
LCMS method F: [M+H]⁺ = 520.0, t_{R} = 2.91 min

### Preparation of intermediate 72: 4-fluoro-19-(oxan-2-yl)-8,14-dioxa-10,19,20-triazapenta cyclo[13.5.2.1^{2,6}.1^{7,10}.0^{18,21}]tetracosa-1(20),2(24),3,5,15(22),16,18(21)-heptaen-9-one

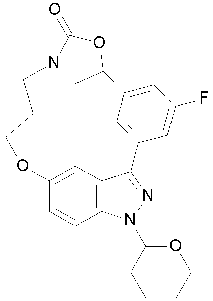

To a solution of 5-(3-bromo-5-fluorophenyl)-3-(3-{[1-(oxan-2-yl)-1H-indazol-5-yl]oxy} propyl)-1,3-oxazolidin-2-one 71 (50 mg, 0.0963 mmol) in 15 mL of toluene was added reagent potassium acetate (113 mg, 1.156 mmol) 2 eq) at room temperature. The mixture was degassed by bubbling nitrogen for 15 minutes. Palladium acetate (25 mg, 0.115 mmol, 0.2 eq) and cataxium (41 mg, 0.115 mmol, 0.2 eq) were added. The mixture was heated at 140°C for 2 hours under microwaves conditions. The reaction mixture was filtered over celite and 20 mL of water was added to the filtrate. The aqueous layer was extracted with ethyl acetate (2x20 mL). The combined organic layers were washed with a brine, dried over sodium sulfate and concentrated under reduced pressure to an orange oil. A purification by column chromatography on a Biotage (cyclohexane /ethyl acetate 0-100%) afforded pure 4-fluoro-19-(oxan-2-yl)-8,14-dioxa-10,19,20-triazapentacyclo[13.5.2.1^{2,6}.1^{7,10}.0^{18,21}]tetracosa-1(20),2(24), 3,5,15(22),16,18(21)-heptaen-9-one **72** as a whitish solid.
LCMS method F: [M+H]⁺ = 438.1, t_{R} = 2.77 min

### Preparation of example 60: 4-fluoro-8,14-dioxa-10,19,20-triazapentacyclo [13.5.2.1^{2,6}.1^{7,10}.0^{18,21}]tetracosa-1(20),2(24),3,5,15(22),16,18(21)-heptaen-9-one

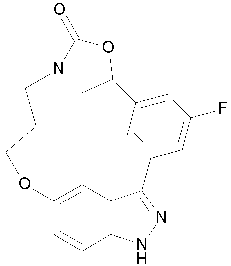

To a solution of 4-fluoro-19-(oxan-2-yl)-8,14-dioxa-10,19,20-triazapentacyclo[13.5.2.1^{2,6}. 1^{7,10}.0^{18,21}]tetracosa-1(20),2(24),3,5,15(22),16,18(21)-heptaen-9-one **72** (0.113g; 0.258 mmol) in DCM (20 mL) was added trifluoroacetic acid (0.2 mL, 2.58 mmol) at room temperature. The mixture was stirred at room temperature for 24 h. The reaction is allowed to cool down to room temperature and toluene (50 mL) was added. The reaction mixture was concentrated under reduced pressure to give an orange oil. Water (25 mL), DCM (25 mL) and a 25 wt% aqueous solution of ammonia (1.5 mL) were added. After separation, the aqueous layer was extracted with DCM (2x20 mL). The combined organic layers were washed with brine, dried over sodium sulfate and concentrated under reduced pressure to an orange oil. A purification by column chromatography on a Biotage (DCM/MeOH 0-5 %) afforded pure 4-fluoro-8,14-dioxa-10,19,20-triazapentacyclo[13.5.2.1^{2,6}.1^{7,10}.0^{18,21}]tetracosa-1(20),2(24),3,5, 15(22),16,18(21)-heptaen-9-one **example 60** as a whitish solid.
LCMS method F: [M+H]⁺ = 354.1, t_{R} = 2.22 min
LCMS method G: [M+H]⁺ = 354.2, t_{R} = 2.22 min
¹H NMR (400 MHz, d6-DMSO) δ 13.26 - 13.24 (1H, m), 8.22 (1H, s), 7.59 (1H, ddd, J = 1.5, 2.5, 9.9 Hz), 7.52 (1H, d, J = 8.9 Hz), 7.49 (1H, d, J = 2.1 Hz), 7.36 (1H, td, J = 1.8, 9.4 Hz), 7.02 (1H, dd, J = 2.3, 9.3 Hz), 5.69 (1H, dd, J = 2.8, 9.0 Hz), 4.47 - 4.38 (1H, m), 4.22 (1H, q, J = 3.9 Hz), 4.17 (1H, t, J = 6.0 Hz), 4.02 - 3.96 (1H, m), 3.57 - 3.46 (1H, m), 3.08 (1H, dd, J = 4.6, 14.4 Hz), 2.36 - 2.25 (1H, m), 1.98 - 1.88 (1H, m) ppm.

### Example 61: 4-(oxolan-3-yl)-8,14-dioxa-10,19,20-triazatetracyclo[13.5.2.1^{2,6}.0^{18,21}]tricosa-1(20),2,4,6(23),15,17,21-heptaen-9-one

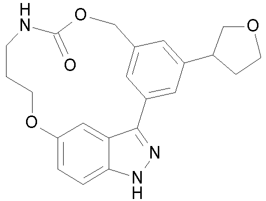

Example 61 is prepared according to the synthesis route described in general Scheme A.

### Preparation of intermediate 73: 4-(2,5-dihydrofuran-3-yl)-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one

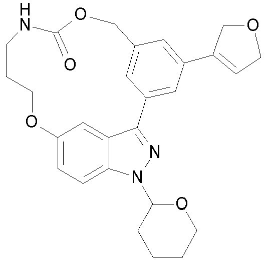

To a degassed solution of 4-bromo-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one **34** (125 mg, 0.25 mmol), 2-(2,5-dihydrofuran-3-yl)-4,4,5,5-tetramethyl-1,3,2-dioxaborolane (100 mg, 0.50 mmol), potassium phosphate tribasic (160 mg, 0.765 mmol) in dioxane / water (9/1, 5.0 mL) was added [1,1'-bis(diphenylphosphino)ferrocene]dichloropalladium(II) (18.5 mg, 0.025 mmol, 10 mol%) under argon at room temperature. The reaction mixture was stirred for 5 hours at 90 °C. The reaction mixture was diluted with water (10 mL) and extracted with ethyl acetate (3 x 15 mL). The combined organic layers were washed with brine (15 mL), dried over anhydrous sodium magnesium sulfate, filtered and the solvent was removed under reduced pressure to afford 4-(2,5-dihydrofuran-3-yl)-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **73** as an orange foam. The crude product was used in the next step without any further purification.
LCMS method F: [M+H]⁺ = 476.1, t_{R} = 2.74 min

### Preparation of intermediate 74: 19-(oxan-2-yl)-4-(oxolan-3-yl)-8,14-dioxa-10,19,20-triazatetracyclo[13.5.2.1^{2,6}.0^{18,21}]tricosa-1(20),2,4,6(23),15,17,21-heptaen-9-one

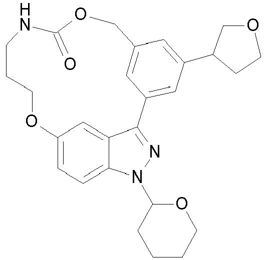

To a stirred solution of 4-(2,5-dihydrofuran-3-yl)-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one **73** (59 mg, 0.125 mmol) in 1.75 mL MeOH and 0.2 mL acetic acid was added 10 % palladium on charcoal (1.5 mg, 0.0013 mmol, 10 mol %) and the mixture was stirred for 20 minutes at room temperature under an atmosphere of hydrogen. The mixture was then filtered, washing with DCM, and the solvent of the filtrate was removed under reduced pressure to afford 19-(oxan-2-yl)-4-(oxolan-3-yl)-8,14-dioxa-10,19,20-triazatetracyclo[13.5.2.1^{2,6}.0^{18,21}]tricosa-1(20),2,4,6(23),15,17,21-heptaen-9-one **74** as a colorless oil.
LCMS method F: [M+H]⁺ = 478.1, t_{R} = 2.71 min

### Preparation of Example 61: 4-(oxolan-3-yl)-8,14-dioxa-10,19,20-triazatetracyclo [13.5.2.1^{2,6}.0^{18,21}] tricosa-1(20),2,4,6(23),15,17,21-heptaen-9-one

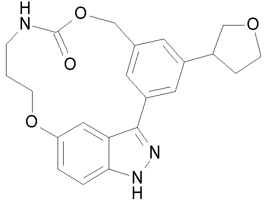

To a solution of 19-(oxan-2-yl)-4-oxolan-3-yl)-8,14-dioxa-10,19,20-triazatetracyclo [13.5.2.1^{2,6}.0^{18,21}]tricosa-1(20),2,4,6(23),15,17,21-heptaen-9-one **74** (0.019 g, 0.039 mmol) in DCM (3 mL) was added trifluoro acetic acid (0.06 mL, 0.78 mmol). The mixture was heated under microwave conditions at 80 °C for 1 h. The solvent was removed under reduced pressure. The crude residue was purified on preparative TLC (DCM/MeOH/NH₃ : 90/9/1) to afford 4-(oxolan-3-yl)-8,14-dioxa-10,19,20-triazatetracyclo[13.5.2.1^{2,6}.0^{18,21}]tricosa-1(20),2,4,6(23), 15, 17,21-heptaen-9-one **example 61** as a white solid.
LCMS method F: [M+H]⁺ = 394.1, t_{R} = 2.13 min
LCMS method G: [M+H]⁺ = 394.2, t_{R} = 2.13 min
¹H NMR (400 MHz, *d*6-DMSO, 80 °C) δ 12.88 (1H, br. s), 7.77 (2H, m), 7.65 (1H, br. s), 7.50 - 7.47 (1H, m), 7.35 - 7.33 (1H, m), 7.19 (1H, s), 6.99 - 6.96 (1H, dd, *J =* 2.0, 8.8 Hz), 5.28 (2H, s), 4.34 - 4.28 (2H, m), 4.12 - 4.07 (1H, m), 4.03 - 3.97 (1H, m), 3.88 - 3.82 (1H, m), 3.67 (1H, t, *J =* 5.2 Hz), 3.53 - 3.44 (1H, m), 3.22 - 3.11 (2H, m), 2.43 - 2.33 (1H, m), 2.06 - 1.96 (3H, m) ppm.

### Example 62: (13S)-13-methyl-8,14-dioxa-10,19,20-triazatetracyclo[13.5.2.1^{2,6}.0^{18,21}] tricosa-1(20),2(23),3,5,15(22),16,18(21)-heptaen-9-one

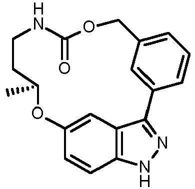

Example 62 is prepared according to the synthesis route described in general Scheme C and by chiral HPLC separation of **example 50.** The chiral separation is done on a Chiralpak IA column 20x250mm 5 µm, eluent [C7/EtOH]+0.1%DEA [90/10] run time 40min, 19 mL/min RT to give (13S)-13-methyl-8,14-dioxa-10,19,20-triazatetracyclo[13.5.2.1^{2,6}.0^{18,21}]tricosa-1(20),2(23), 3,5,15(22),16,18(21)-heptaen-9-one **example 62.**
LCMS method F: [M+H]⁺ = 338.1, t_{R} = 2.24 min
LCMS method G: [M+H]⁺ = 338.1, t_{R} = 2.25 min
¹H NMR (400 MHz, d6-DMSO) δ 13.11 (1H, s), 7.93 - 7.84 (3H, m), 7.47 (2H, dd, J = 8.4, 15.7 Hz), 7.29 - 7.25 (2H, m), 6.97 (1H, dd, J = 2.2, 9.0 Hz), 5.77 - 5.71 (1H, m), 4.83 - 4.79 (1H, m), 4.59 - 4.53 (1H, m), 3.58 (1H, m), 2.94 - 2.85 (1H, m), 1.41 - 1.38 (4H, m), 1.25 - 1.14 (1H, m) ppm.
Chiral HPLC e.e. 98.2%

### Example 63: (13R)-13-methyl-8,14-dioxa-10,19,20-triazatetracyclo[13.5.2.1^{2,6}.0^{18,21}] tricosa-1(20),2(23),3,5,15(22),16,18(21)-heptaen-9-one

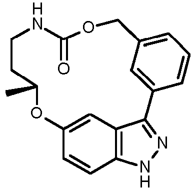

Example 63 is prepared according to the synthesis route described in general Scheme C and by chiral HPLC separation of **example 50.** The chiral separation is done on a Chiralpak IA column 20x250mm 5 µm, eluent [C7/EtOH]+0.1%DEA [90/10] run time 40min, 19 mL/min RT to give (13R)-13-methyl-8,14-dioxa-10,19,20-triazatetracyclo[13.5.2.1^{2,6}.0^{18,21}]tricosa-1(20),2(23), 3,5,15(22),16,18(21)-heptaen-9-one **example 63.**
LCMS method F: [M+H]⁺ = 338.2, t_{R} = 2.25 min
LCMS method G: [M+H]⁺ = 338.2, t_{R} = 2.23 min
¹H NMR (400 MHz, d6-DMSO) δ 13.11 (1H, s), 7.93 - 7.84 (3H, m), 7.47 (2H, dd, J = 8.6, 15.1 Hz), 7.29 - 7.25 (2H, m), 6.97 (1H, dd, J = 2.2, 9.0 Hz), 5.77 - 5.74 (1H, m), 4.83 - 4.79 (1H, m), 4.59 - 4.53 (1H, m), 3.59 - 3.54 (1H, m), 2.96 - 2.86 (1H, m), 1.42 - 1.38 (4H, m), 1.25 - 1.14 (1H, m) ppm.
Chiral HPLC e.e. 98.8%

### Example 64: 4-(1-methyl-1H-pyrazol-3-yl)-8,14-dioxa-10,19,20-triazatetracyclo [13.5.2.1^{2,6}.0^{18,21}]tricosa-1(20),2(23),3,5,15(22),16,18(21)-heptaen-9-one

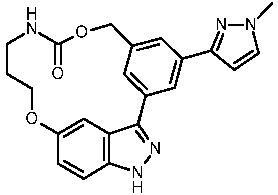

Example 64 is prepared according to the synthesis route described in general Scheme C. 1-Methyl-1H-pyrazole-3-boronic acid pinacol ester was used for the Suzuki coupling with the bromide intermediate **34** to give 4-(1-methyl-1H-pyrazol-3-yl)-8,14-dioxa-10,19,20-triazatetracyclo[13.5.2.1^{2,6}.0^{18,21}]tricosa-1(20),2(23),3,5,15(22),16,18(21)-heptaen-9-one **example 64.**
LCMS method F: [M+H]⁺ = 404, t_{R} = 2.12 min
LCMS method G: [M+H]⁺ = 404, t_{R} = 2.12 min
¹H NMR (400 MHz, d6-DMSO) δ 12.93 (1H, s), 8.30 (1H, s), 7.82 (1H, s), 7.73 - 7.68 (3H, m), 7.51 - 7.49 (1H, d), 7.38 (1H, m), 7.01 - 6.98 (1H, dd), 6.71 (1H, d, J = 2.3 Hz), 5.34 (2H, m), 4.35 - 4.31 (2H, m), 3.93 (3H, s), 3.19 (2H, m), 2.05 (2H, m) ppm.

### Example 65: (7S)-7-methyl-8,14-dioxa-10,19,20-triazatetracyclo[13.5.2.1^{2,6}.0^{18,21}]tricosa-1(20),2(23),3,5,15(22),16,18(21)-heptaen-9-one

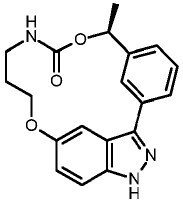

Example 65 is prepared according to the synthesis route described in general Scheme C. (1S)-1-[3-(4,4,5,5-tetramethyl-1,3,2-dioxaborolan-2-yl)phenyl]ethanol is used for the Suzuki coupling with intermediate **26** to give (7S)-7-methyl-8,14-dioxa-10,19,20-triazatetracyclo[13.5.2.1^{2,6}.0^{18,21}]tricosa-1(20),2(23),3,5,15(22),16,18(21)-heptaen-9-one **example 65.**
LCMS method F: [M+H]⁺ = 338, t_{R} = 2.19 min
LCMS method G: [M+H]⁺ = 338, t_{R} = 2.19 min
¹H NMR (400 MHz, d6-DMSO) δ 13.12 (1H, s), 7.94 (1H, m), 7.86 - 7.84 (2H, m), 7.50 - 7.46 (2H, m), 7.35 - 7.29 (2H, m), 7.00 - 6.98 (1H, dd), 5.95 - 5.90 (1H, q), 4.38 - 4.23 (2H, m), 3.56 -3.49 (1H, m), 2.78 - 2.70 (1H, m), 2.25 - 2.15 (1H, m), 1.77 - 1.69 (1H, m), 1.59 (3H, d) ppm.

### Example 66: 4-[2-(morpholin-4-yl)ethoxy]-8,14-dioxa-10,19,20-triazatetracyclo [13.5.2.1^{2,6}.0^{18,21}]tricosa-1(20),2(23),3,5,15(22),16,18(21)-heptaen-9-one

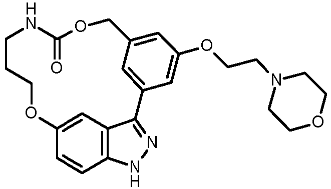

Example 66 is prepared according to the synthesis route described in general Scheme F.

### Preparation of intermediate 75: methyl 3-[5-[3-(benzyloxycarbonylamino)propoxy]-1-tetrahydropyran-2-yl-indazol-3-yl]-5-(2-morpholinoethoxy)benzoate

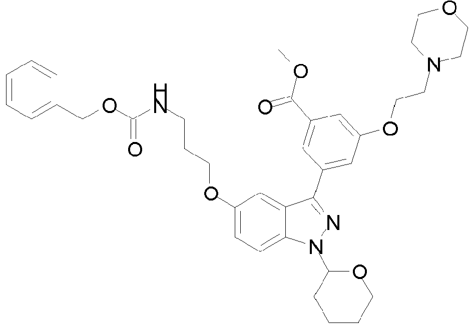

To a solution of methyl 3-[5-[3-(benzyloxycarbonylamino)propoxy]-1-tetrahydropyran-2-yl-indazol-3-yl]-5-hydroxy-benzoate **53** (400 mg, 0.715 mmol), 2-morpholinoethanol (131 µL, 141 mg, 1.073 mmol) and PPh₃ (281 mg, 1.073 mmol) in THF (5.0 mL) was added dropwise a solution of DMEAD (251 mg, 1.073 mmol) in THF (2.5 mL). The resulting clear yellow solution was stirred at room temperature for 16 h. The solvents were evaporated under reduced pressure and the residue was partitioned between EtOAc (20 mL) and water (20 mL). The aqueous phase was extracted with EtOAc (3 x 20 mL) and the combined organic layers were washed with brine (1 x 20 mL), dried over anhydrous Na₂SO₄, filtered and concentrated under reduced pressure. The resulting crude material (yellow oil, 700 mg) was purified by column chromatography (40 g Macherey Nagel SiO₂, 30 mL/min, CyH/EtOAc 100:0 to 80:20 then CH₂Cl₂/MeOH 95:5) to afford methyl 3-[5-[3-(benzyloxycarbonylamino)propoxy]-1-tetrahydropyran-2-yl-indazol-3-yl]-5-(2-morpholinoethoxy)benzoate 75 as a pale yellow oil.
LCMS method F: [M+H]⁺ = 673.3, t_{R} = 2.24 min

### Preparation of intermediate 76: benzyl N-[3-[3-[3-(hydroxymethyl)-5-(2-morpholinoethoxy) phenyl]-1-tetrahydropyran-2-yl-indazol-5-yl]oxypropyl]carbamate

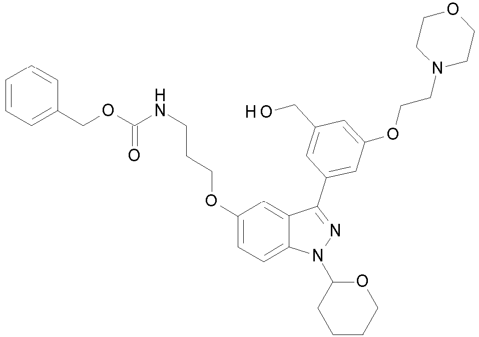

To a solution of methyl 3-[5-[3-(benzyloxycarbonylamino)propoxy]-1-tetrahydropyran-2-yl-indazol-3-yl]-5-(2-morpholinoethoxy)benzoate **75** (451 mg, 0.670 mmol) in THF (6.5 mL) at 0°C was added dropwise LiAlH₄ (1.0 M solution in THF, 1.34 mL, 1.340 mmol). The resulting cloudy white solution was stirred at 0°C for 1 h. The reaction was quenched with saturated aqueous Rochelle's salt (5 mL), EtOAc (20 mL) was added and the two layers were separated. The aqueous layer was extracted with EtOAc (3 x 10 mL) and the combined organic layers were washed with brine (1 x 5 mL), dried over anhydrous Na₂SO₄, filtered and the solvent was removed under reduced pressure to afford crude benzyl N-[3-[3-[3-(hydroxymethyl)-5-(2-morpholinoethoxy)phenyl]-1-tetrahydropyran-2-yl-indazol-5-yl]oxypropyl]carbamate **76** as a transparent oil which was used in the next step without further purification.
LCMS method F: [M+H]⁺ = 645.3, t_{R} = 2.07 min

### Preparation of intermediate 77: 4-[2-(morpholin-4-yl)ethoxy]-19-(oxan-2-yl)-8,14-dioxa-10,19,20-triazatetracyclo[13.5.2.1^{2,6}.0^{18,2}]tricosa-1(20),2(23),3,5,15(22),16,18(21)-heptaen-9-one

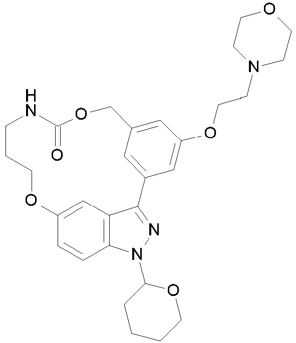

To a solution of benzyl N-[3-[3-[3-(hydroxymethyl)-5-(2-morpholinoethoxy)phenyl]-1-tetrahydropyran-2-yl-indazol-5-yl]oxypropyl]carbamate **76** (200 mg, 0.310 mmol) in degassed MeCN (30.0 mL) was added Cs₂CO₃ (606 mg, 1.860 mmol). The resulting cloudy yellow solution was heated under reflux for 5 h. The mixture was cooled to room temperature, filtered and the solvent was removed under reduced pressure to afford crude 4-[2-(morpholin-4-yl)ethoxy]-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one 77 as a brown solid which was used in the next step without further purification.
LCMS method F: [M+H]⁺ = 537.2, t_{R} = 1.82 min

### Preparation of Example 66: 4-[2-(morpholin-4-yl)ethoxy]-8,14-dioxa-10,19,20-triazatetracyclo[13.5.2.1^{2,6}.0^{18,21}]tricosa-1(20),2(23),3,5,15(22),16,18(21)-heptaen-9-one

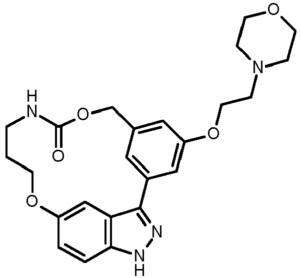

To a solution of crude 4-[2-(morpholin-4-yl)ethoxy]-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one 77 (189 mg, 0.310 mmol postulated) in CH₂Cl₂ (6.0 mL) was added TFA (475 µL, 707 mg, 6.200 mmol). The resulting clear yellow solution was stirred at room temperature for 6 h. Saturated aqueous NaHCO₃ (5 mL) was added and the two layers were separated. The aqueous layer was extracted with CH₂Cl₂ (3 x 5 mL) and the combined organic layers were washed with water (1 x 5 mL), dried over anhydrous Na₂SO₄, filtered and concentrated under reduced pressure. The residue was purified by preparative reverse-phase chromatography (Column XSELECT PHENYL-HEXYL 19*100mm 5µm, [(NH₄)₂CO₃ aq. 2 g/L / ACN] 35%B to 55%B in 7 min, 19 mL/min RT) to afford 4-[2-(morpholin-4-yl)ethoxy]-8,14-dioxa-10,19,20-triazatetracyclo[13.5.2.1^{2,6}.0^{18,21}]tricosa-1(20),2(23),3,5,15(22),16,18(21)-heptaen-9-one **example 66** as a pale orange solid.
LCMS method F: [M+H]⁺ = 453.3, t_{R} = 1.79 min
LCMS method G: [M+H]⁺ = 453.3, t_{R} = 2.80 min
¹H NMR (400 MHz, d6-DMSO, 80°C) δ 12.90 (brs, 1H), 7.67 (brs, 1H), 7.51 (brs, 1H), 7.48 (d, *J =* 8.6 Hz, 1H), 7.42 - 7.40 (m, 1H), 7.36 - 7.34 (m, 1H), 6.98 (dd, *J =* 8.6, 2.0 Hz, 1H), 6.90 - 6.88 (m, 1H), 5.25 (brs, 1H), 4.34 - 4.31 (m, 2H), 4.20 (t, *J =* 5.7 Hz, 2H), 3.63 - 3.59 (m, 4H), 3.17 (brs, 2H), 2.77 (t, *J =* 5.7 Hz, 2H), 2.55 - 2.52 (m, 4H), 2.03 (brs, 2H) ppm.

### Example 67: 4-(2-methoxyethyl)-8,14-dioxa-10,19,20-triazatetracyclo[13.5.2.1^{2,6}.0^{18,21}] tricosa-1(20),2(23),3,5,15(22),16,18(21)-heptaen-9-one

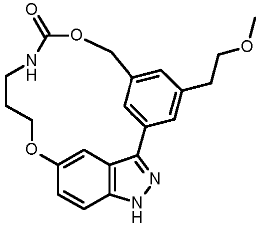

Example 67 is prepared according to the synthesis route described in general Scheme A. Potassium (2-methoxyethyl) trifluoroborate is used for the Suzuki coupling with intermediate 26 to give 4-(2-methoxyethyl)-8,14-dioxa-10,19,20-triazatetracyclo[13.5.2.1^{2,6}.0^{18,21}]tricosa-1(20),2(23),3,5,15(22),16,18(21)-heptaen-9-one **example 67.**
LCMS method F: [M+H]⁺ = 382.2, t_{R} = 2.17 min
LCMS method G: [M+H]⁺ = 382.2, t_{R} = 2.18 min
¹H NMR analysis indicated the presence of two rotamers.
¹H NMR (400 MHz, *d*6-DMSO, 80 °C) δ 12.87 (1H, br. s), 7.82 (0.4H, d, *J =* 1.6 Hz, rot. 2), 7.74 (1.6H, d, *J =* 4.9 Hz, rot. 1), 7.67 - 7.63 (1H, m), 7.50 - 7.46 (1H, m), 7.34 (1H, d, *J =* 1.9 Hz), 7.22 (0.2H, s, rot. 2), 7.14 (0.8H, s, rot. 1), 6.99 - 6.96 (1H, m), 5.31 - 5.26 (2H, m), 4.34 - 4.28 (2H, m), 3.65 (2H, t, *J =* 6.80 Hz), 3.30 (3H, s), 3.20 - 3.11 (2H, m), 2.91 (2H, t, *J =* 6.7 Hz), 2.07 - 1.99 (3H, m) ppm.

### Example 68: (7R)-7-methyl-8,14-dioxa-10,19,20-triazatetracyclo[13.5.2.1^{2,6}.0^{18,21}]tricosa-1(20),2(23),3,5,15(22),16,18(21)-heptaen-9-one

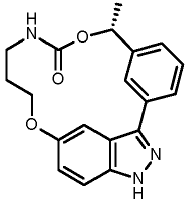

Example 68 is prepared according to the synthesis route described in general Scheme C. (1R)-1-[3-(4,4,5,5-tetramethyl-1,3,2-dioxaborolan-2-yl)phenyl]ethanol is used for the Suzuki coupling with intermediate **26** to give (7R)-7-methyl-8,14-dioxa-10,19,20-triazatetracyclo [13.5.2.1^{2,6}.0^{18,21}]tricosa-1(20),2(23),3,5,15(22),16,18(21)-heptaen-9-one **example 68.**
LCMS method F: [M+H]⁺ = 338.2, t_{R} = 2.18 min
LCMS method G: [M+H]⁺ = 338.2, t_{R} = 2.19 min
¹H NMR (400 MHz, d6-DMSO) δ 13.13 - 13.11 (1H, m), 7.94 (1H, dd, J = 4.7, 7.4 Hz), 7.87 - 7.83 (2H, m), 7.51 - 7.46 (2H, m), 7.36 - 7.29 (2H, m), 6.99 (1H, dd, J = 2.3, 9.1 Hz), 5.93 (1H, q, J = 6.8 Hz), 4.38 - 4.23 (2H, m), 3.56 - 3.49 (1H, m), 2.78 - 2.70 (1H, m), 2.25 - 2.15 (1H, m), 1.78 - 1.69 (1H, m), 1.61 - 1.58 (3H, m) ppm.

### Example 69: 5-cyclopropyl-8,14-dioxa-10,19,20-triazatetracyclo[13.5.2.1^{2,6}.0^{18,21}]tricosa-1(20),2,4,6(23),15,17,21-heptaen-9-one

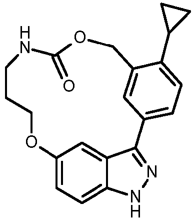

Example 69 is prepared according to the synthesis route described in general Scheme C. Potassium cyclopropyl(trifluoro)borate was used for the Suzuki coupling with the bromide intermediate **48** to give 5-cyclopropyl-8,14-dioxa-10,19,20-triazatetracyclo[13.5.2.1^{2,6}.0^{18,21}] tricosa-1(20),2,4,6(23),15,17,21-heptaen-9-one **example 69.**
LCMS method F: [M+H]⁺ = 364, t_{R} = 2.40 min
LCMS method G: [M+H]⁺ = 364, t_{R} = 2.40 min
¹H NMR (400 MHz, *d*6-DMSO) δ 7.86 - 7.83 (2H, m), 7.74 (1H, s), 7.50 - 7.37 (3H, m), 7.16 - 7.13 (1H, m), 6.99 - 6.96 (1H, m), 5.48 (2H, s), 4.37 - 4.32 (2H, m), 3.20 (2H, s), 2.00 (1H, m), 1.92 (1H, m), 1.16 (1H, m), 1.02 - 0.97 (2H, m), 0.73 - 0.68 (2H, m) ppm.

### Example 70: 4-(2-methoxyethoxy)-8,14-dioxa-10,19,20-triazatetracyclo[13.5.2.1^{2,6}.0^{18,21}] tricosa-1(20),2,4,6(23),15,17,21-heptaen-9-one

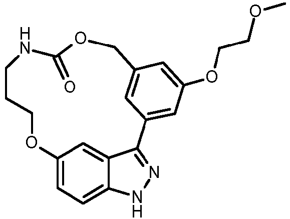

Example 70 is prepared according to the synthesis route described in general Scheme F and procedures analogous to those used to obtain **example 66** to give 4-(2-methoxyethoxy)-8,14-dioxa-10,19,20-triazatetracyclo[13.5.2.1^{2,6}.0^{18,21}]tricosa-1(20),2,4,6(23),15,17,21-heptaen-9-one **example 70.**
LCMS method F: [M+H]⁺ = 398.2, t_{R} = 2.89 min
LCMS method G: [M+H]⁺ = 398.2, t_{R} = 2.13 min
¹H NMR (400 MHz, *d*6-DMSO, 80°C) δ 12.89 (brs, 1H), 7.67 (brs, 1H), 7.52 (s, 1H), 7.48 (d, *J* = 8.9 Hz, 1H), 7.42 - 7.41 (m, 1H), 7.36 (d, *J =* 1.9 Hz, 1H), 6.98 (dd, *J =* 8.9, 1.9 Hz, 1H), 6.89 (s, 1H), 5.25 (brs, 2H), 4.34 - 4.29 (m, 2H), 4.21 (t, *J =* 4.5 Hz, 2H), 3.72 (t, *J =* 4.5 Hz, 2H), 3.37 (s, 3H), 3.22 - 3.12 (m, 2H), 2.08 - 1.98 (m, 2H) ppm.

### Example 71: 4-fluoro-13-methyl-8,14-dioxa-10,19,20-triazatetracyclo[13.5.2.1^{2,6}.0^{18,21}] tricosa-1(20),2,4,6(23),15,17,21-heptaen-9-one

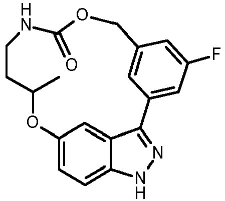

Example 71 is prepared according to the synthesis route described in general Scheme C to give 4-fluoro-13-methyl-8,14-dioxa-10,19,20-triazatetracyclo[13.5.2.1^{2,6.}0^{18,21}]tricosa-1(20),2,4, 6(23),15,17,21-heptaen-9-one **example 71.**
LCMS method F: [M+H]⁺ = 356.1, t_{R} = 2.37 min
LCMS method G: [M+H]⁺ = 356.2, t_{R} = 2.33 min
¹H NMR (400 MHz, d6-DMSO) δ 13.26 (1H, m), 8.01 - 7.96 (1H, m), 7.68 (1H, s), 7.61 - 7.50 (2H, m), 7.27 (1H, d, *J =* 1.9 Hz), 7.17 (1H, ddd, *J =* 1.4, 2.4, 9.5 Hz), 6.99 (1H, dd, J = 2.2, 9.0 Hz), 5.71 (1H, d, J = 13.7 Hz), 4.89 - 4.83 (1H, m), 4.62 - 4.54 (1H, m), 3.61-3.50 (1H, m), 2.94 - 2.86 (1H, m), 2.43 - 2.36 (1H, m), 1.42-1.39 (4H, m) ppm.

### Example 72: 11-methyl-8,14-dioxa-10,19,20-triazatetracyclo[13.5.2.1^{2,6}.0^{18,21}]tricosa-1(20),2,4,6(23),15,17,21-heptaen-9-one

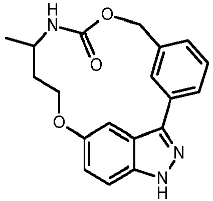

Example 72 is prepared according to the synthesis route described in general Scheme C to give 11-methyl-8,14-dioxa- 0,19,20-triazatetracyclo[13.5.2.1^{2,6}.0^{18,21}]tricosa-1(20),2,4,6(23),15, 17,21-heptaen-9-one **example 72.**
LCMS method F: [M+H]⁺ = 338, t_{R} = 2.26 min
LCMS method G: [M+H]⁺ = 338, t_{R} = 2.25 min
¹H NMR (400 MHz, d6-DMSO) δ 12.92 - 12.85 (1H, m), 7.95 (1H, s), 7.86 (1H, d, J = 7.4 Hz), 7.56 (1H, s), 7.50 - 7.43 (2H, m), 7.35 (1H, d, J = 2.1 Hz), 7.28 - 7.26 (1H, m), 6.99 (1H, dd, J = 2.3, 8.9 Hz), 5.68 (1H, s), 4.94 - 4.89 (1H, m), 4.41 - 4.34 (2H, m), 3.88 - 3.81 (1H, m), 2.19 (1H, s), 1.97 - 1.95 (1H, m), 1.25 - 1.22 (3H, m) ppm.

### Example 73: 4-(3-oxomorpholin-4-yl)-8,14-dioxa-10,19,20-triazatetracyclo[13.5.2. 1^{2,6}.0^{18,21}]tricosa-1(20),2(23),3,5,15(22),16,18(21)-heptaen-9-one

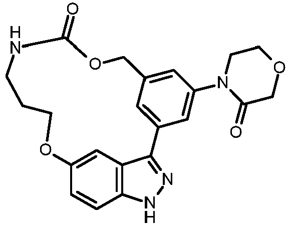

Example 73 is prepared according to the synthesis route described in general Scheme C and analogues procedures have been used as to obtain **example 12.** Morpholin-3-one is used for the Buchwald reaction with the bromide intermediate **34** to give 4-(3-oxomorpholin-4-yl)-8,14-dioxa-10,19,20-triazatetracyclo[13.5.21^{2,6}.0^{18,21}]tricosa-1(20),2(23),3,5,15(22),16,18(21)-heptaen-9-one **example 73.**
LCMS method F: [M+H]⁺ = 423, t_{R} = 1.88 min
LCMS method G: [M+H]⁺ = 423, t_{R} = 1.87 min
¹H NMR (400 MHz, d6-DMSO) δ 12.99 (1H, s), 7.87 (1H, s), 7.80 (1H, s), 7.72 (1H, s), 7.48 (1H, s), 7.34 (2H, d, J = 10.8 Hz), 6.99 (1H, dd, J = 2.1, 8.9 Hz), 5.31 (2H, s), 4.35 - 4.30 (2H, m), 4.24 (2H, s), 4.03 (2H, t, J = 5.1 Hz), 3.82 (2H, t, J = 5.0 Hz), 3.18 (2H, s), 2.06 (2H, s) ppm.

### Example 74: 4-(2-oxopyrrolidin-1-yl)-8,14-dioxa-10,19,20-triazatetracyclo[13.5.2. 1^{2,6}.0^{18,21}]tricosa-1(20),2(23),3,5,15(22),16,18(21)-heptaen-9-one

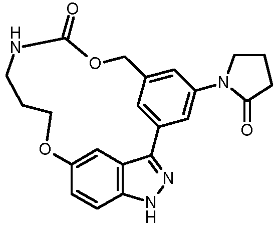

Example 74 is prepared according to the synthesis route described in general Scheme C and procedures analogous to those used to obtain **example 12.** Pyrrolidin-2-one is used for the Buchwald reaction with the bromide intermediate **34** to give 4-(2-oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one **example 74.**
LCMS method F: [M+H]⁺ = 407, t_{R} = 2.02 min
LCMS method G: [M+H]⁺ = 407, t_{R} = 2.01 min
¹H NMR (400 MHz, d6-DMSO) δ 12.93 (1H, s), 8.14 (1H, s), 7.69 (2H, s), 7.59 (1H, s), 7.48 (1H, d, J = 9.54 Hz), 7.35 (1H, s), 6.98 (1H, dd, J = 2.4, 9.0 Hz), 5.28 (2H, s), 4.32 (2H, m), 3.93 (2H, m), 3.17 (2H, m), 3.53 (2H, m), 3.13 (2H, m), 2.03 (2H, m) ppm.

### Example 75: 5-(2-oxopyrrolidin-1-yl)-8,14-dioxa-10,19,20-triazatetracyclo[13.5.2. 1^{2,6}.0^{18,21}]tricosa-1(20),2(23),3,5,15(22),16,18(21)-heptaen-9-one

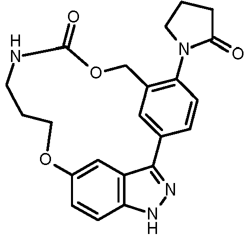

Example 75 is prepared according to the synthesis route described in general Scheme C and procedures analogous to those used to obtain **example 12.** Pyrrolidin-2-one is used for the Buchwald reaction with the bromide intermediate **48** to give 5-(2-oxopyrrolidin-1-yl)-8,14-dioxa- 0,19,20-triazatetracyclo[13.5.2.1^{2,6}.0^{18,21}]tricosa-1(20),2(23),3,5,15(22),16,18(21)-heptaen-9-one **example 75.**
LCMS method F: [M+H]⁺ = 407, t_{R} = 1.92 min
LCMS method G: [M+H]⁺ = 407, t_{R} = 1.92 min
¹H NMR (400 MHz, *d*6-DMSO) δ 12.95 (1H, s), 7.97 - 7.93 (2H, m), 7.71 (1H, s), 7.48 (1H, s), 7.40 - 7.37 (2H, m), 6.99 (1H, dd, J = 2.2, 8.8 Hz), 5.11 (2H, s), 4.36 - 4.31 (2H, m), 3.81 (2H, t, J = 6.9 Hz), 3.17 (2H, s), 3.06 (2H, m hidden), 2.23 - 2.18 (2H, m), 2.03 (2H, m) ppm.

### Example 76: 4-(2-methylpyrrolidin-1-yl)-8,14-dioxa-10,19,20-triazatetracyclo [13.5.2.1^{2,6}.0^{18,21}]tricosa-1(20),2(23),3,5,15(22),16,18(21)-heptaen-9-one

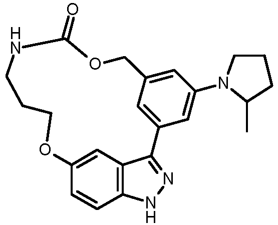

Example 76 is prepared according to the synthesis route described in general Scheme C and procedures analogous to those used to obtain **example 12.** 2-Methylpyrrolidine is used for the Buchwald reaction with the bromide intermediate **34** to give 4-(2-methylpyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one **example 76.**
LCMS method F: [M+H]⁺ = 407, t_{R} = 2.27 min
LCMS method G: [M+H]⁺ = 407, t_{R} = 2.56 min
¹H NMR (400 MHz, d6-DMSO) δ 12.75 (1H, m), 7.61 - 7.58 (1H, m), 7.47 - 7.44 (1H, m), 7.36 (1H, d, J = 2.3 Hz), 7.18 (1H, s), 7.05 (1H, s), 6.95 (1H, dd, J = 2.4, 9.0 Hz), 6.50 (1H, s), 5.24 - 5.21 (2H, m), 4.32 - 4.27 (2H, m), 3.98 - 3.95 (1H, m), 3.48 - 3.43 (1H, m), 3.19 (2H, m), 2.12 - 1.97 (6H, m), 1.73 - 1.70 (1H, m), 1.19 (3H, d, J = 6.1 Hz) ppm.

### Example 77: 2-{9-oxo-8,14-dioxa-10,19,20-triazatetracyclo[13.5.2.1^{2,6}.0^{18,21}]tricosa-1(20),2(23),3,5,15(22),16,18(21)-heptaen-4-yl} acetonitrile

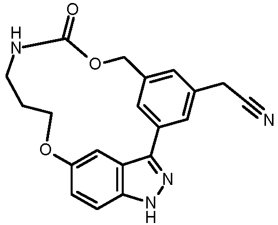

Example 77 is prepared according to the synthesis route described in general Scheme C.

### Preparation of intermediate 78: 2-[19-(oxan-2-yl)-9-oxo-8,14-dioxa-10,19,20-triazatetra cyclo[13.5.2.1^{2,6}.0^{18,21}]tricosa-1(20),2(23),3,5,15(22),16,18(21)-heptaen-4-yl]acetonitrile

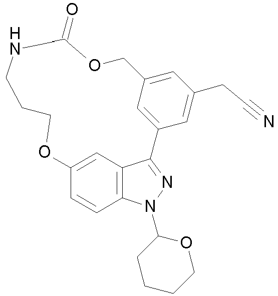

To a solution of 4-bromo-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one **34** (100 mg, 0.21 mmol) in DMSO (2 mL) was added isoxazol-3-ylboronic acid (49 mg, 0.25 mmol) and a 1 M solution of potassium fluoride (0.63 mL, 0.63 mmol) at RT. The reaction mixture was degassed for 15 min by bubbling nitrogen gas, then [1,1'-Bis(diphenylphosphino)ferrocene]dichloropalladium(II) (15 mg, 0.02 mmol) was added the reaction mixture was stirred at 130°C for 18 hours. The reaction mixture was filtered over celite, washed with ethyl acetate and concentrated under reduced pressure. EtOAc (30 mL) and water (10 mL) were added, the mixture was filtered over celite. The two layers were separated, the organic layer was extracted with EtOAc (2x20 mL), washed with brine, dried over sodium sulfate, filtered and concentrated under reduced pressure to give a black oil. The residue was purified by flash chromatography (CyH/AE 5/5) to afford 2-[19-(oxan-2-yl)-9-oxo-8,14-dioxa-10,19,20-triazatetracyclo[13.5.2.1^{2,6}.0^{18,21}]tricosa-1(20),2(23),3,5,15(22),16,18(21)-heptaen-4-yl]acetonitrile 78 as a white solid.
LCMS method F: [M+H]⁺ = 447, t_{R} = 2.64 min

### Preparation of Example 77: 2-{9-oxo-8,14-dioxa-10,19,20-triazatetracyclo [13.5.2.1^{2,6}.0^{18,21}]tricosa-1(20),2(23),3,5,15(22),16,18(21)-heptaen-4-yl}acetonitrile

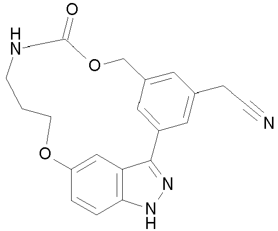

To a solution of 2-[19-(oxan-2-yl)-9-oxo-8,14-dioxa-10,19,20-triazatetracyclo [13.5.2.1^{2,6}.0^{18,21}]tricosa-1(20),2(23),3,5,15(22),16,18(21)-heptaen-4-yl]acetonitrile **78** (38 mg, 0.085 mmol) in DCM (2 mL) was added TFA (130 µL, 1.70 mmol) and the reaction mixture was stirred at RT for 12 hours. To the reaction mixture was added a saturated solution sodium bicarbonate (10 mL) and DCM (10 mL). The two layers were separated, the organic layer was extracted twice with DCM (10 mL), washed with brine, dried over sodium sulfate, filtered and concentrated under reduced pressure to give a yellow oil. The residue was put in diethyl ether and water, the precipitate was filtered to afford 2-{9-oxo-8,14-dioxa-10,19,20-triazatetracyclo[13.5.2.1^{2,6}.0^{18,21}]tricosa-1(20),2(23),3,5,15(22),16,18(21)-heptaen-4-yl } acetonitrile **example 77** as a white solid.
LCMS method F: [M+H]⁺ = 363, t_{R} = 2.08 min
LCMS method G: [M+H]⁺ = 363, t_{R} = 2.08 min
¹H NMR (400 MHz, d6-DMSO) δ 12.97 (1H, s), 7.90 - 7.85 (2H, m), 7.73 - 7.69 (1H, m), 7.52 -7.48 (1H, m), 7.35 (1H, d, J = 1.9 Hz), 7.25 (1H, s), 6.99 (1H, dd, J = 2.3, 8.9 Hz), 5.30 (2H, s), 4.32 (2H, dd, J = 7.6, 8.7 Hz), 4.10 (2H, s), 3.17 (2H, s), 2.02 (2H, s) ppm.

### Example 78: (11R) or (11S)-11-methyl-8,14-dioxa-10,19,20-triazatetracyclo [13.5.2.1^{2,6}.0^{18,21}]tricosa-1(20),2(23),3,5,15(22),16,18(21)-heptaen-9-one

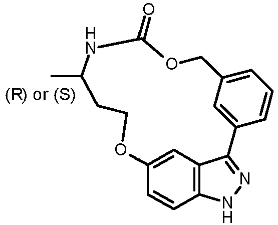

Example 78 is prepared according to the synthesis route described in general Scheme C and by chiral HPLC separation of the two enantiomers of **example 72.** The chiral separation is done on a Chiralpak IA column 20x250mm 5 µm, eluent [C7/EtOH]+0.1%DEA [90/10] run time 40 min, 19 mL/min RT to give (11R) or (11S)-11-methyl-8,14-dioxa-10,19,20-triazatetracyclo [13.5.2.1^{2,6}.0¹⁸_{'}²¹]tricosa-1(20),2(23),3,5,15(22),16,18(21)-heptaen-9-one **example 78.**
LCMS method F: [M+H]⁺ = 338, t_{R} = 2.27 min
LCMS method G: [M+H]⁺ = 338, t_{R} = 2.27 min
¹H NMR (400 MHz, d6-DMSO) δ 12.90 (1H, s), 7.94 (1H, s), 7.84 (1H, d, J = 7.8 Hz), 7.57 (1H, s), 7.50 - 7.43 (2H, m), 7.36 (1H, s), 7.28 - 7.26 (1H, m), 6.99 (1H, dd, J = 2.3, 8.9 Hz), 5.67 (1H, m), 4.90 (1H, m), 4.39 - 4.34 (2H, m), 3.82 (1H, s), 2.20 (1H, m), 1.93 (1H, m), 1.25 - 1.22 (3H, m) ppm.
Chiral HPLC e.e. 98.2%

The compound is a pure enantiomer, but the absolute stereochemistry of the chiral center is unknown.

### Example 79: (11R) or (11S)-11-methyl-8,14-dioxa-10,19,20-triazatetracyclo [13.5.2.1^{2,6}.0^{18,21}]tricosa-1(20),2(23),3,5,15(22),16,18(21)-heptaen-9-one

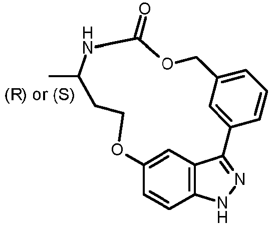

Example 79 is prepared according to the synthesis route described in general Scheme C and by chiral HPLC separation of the two enantiomers. The chiral separation is done on a Chiralpak IA column 20x250mm 5 µm, eluent [C7/EtOH]+0.1%DEA [90/10] run time 40 min, 19 mL/min RT to give (11R) or (11S)-11-methyl-8,14-dioxa-10,19,20-triazatetracyclo [13.5.2.1^{2,6}.0¹⁸_{'}²¹]tricosa-1(20),2(23),3,5,15(22),16,18(21)-heptaen-9-one **example 79.**
LCMS method F: [M+H]⁺ = 338, t_{R} = 2.27 min
LCMS method G: [M+H]⁺ = 338, t_{R} = 2.26 min
¹H NMR (400 MHz, DMSO) δ 12.89 (1H, s), 7.94 (1H, s), 7.87 - 7.84 (1H, m), 7.55 (1H, m), - 7.47 (2H, m), 7.35 (1H, m), 7.28 - 7.26 (1H, m), 7.01 - 6.97 (1H, m), 5.66 (1H, m), 4.90 (1H, m), 4.41 - 4.34 (2H, m), 3.87 - 3.81 (1H, m), 2.22 (1H, m), 1.93 (1H, m), 1.25 - 1.22 (3H, m) ppm.
Chiral HPLC e.e. 98.5%

The compound is a pure enantiomer, but the absolute stereochemistry of the chiral center is unknown.

### Example 80:4-ethynyl-8,14-dioxa-10,19,20-triazatetracyclo[13.5.2.1^{2,6}.0^{18,21}]tricosa-1(20),2(23),3,5,15(22),16,18(21)-heptaen-9-one

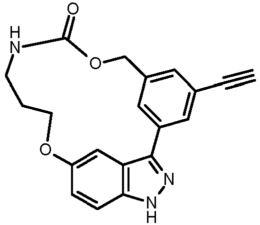

Example 80 is prepared according to the synthesis route described in general Scheme C.

### Preparation of intermediate 79: 19-(oxan-2-yl)-4-[2-(triethylsilyl)ethynyl]-8,14-dioxa-10,19,20-triazatetracyclo[13.5.2.1^{2,6}.0^{18,21}]tricosa-1(20),2(23),3,5,15(22),16,18(21)-heptaen-9-one

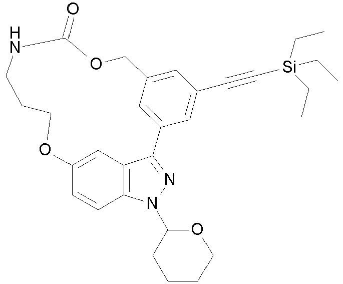

To a solution of intermediate **34** (100 mg, 0.21 mmol) in THF( 2 mL) in a sealed tube were added triethyl(ethynyl)silane (44.1 mg, 0.31 mmol) and triethylamine (63.63 mg, 0.63 mmol). The reaction mixture was degassed for 15 min by bubbling nitrogen gas and Pd(PPh₃)₄ (12 mg, 0.01 mmol) and copper iodide (0.4 mg, 0.0021 mmol) were added. The reaction mixture was stirred at 80 °C for 12 hours. The reaction mixture was concentrated under reduced pressure to give a yellow oil. To the residue were added EtOAc (20 mL) and water (10 mL), after separation of the two layers, the organic layer was extracted twice with EtOAc (10 mL), washed with brine, dried over sodium sulfate, filtered and the solvent was removed under reduced pressure to give an off-white solid. Diethyl ether was added to the residue, the beige precipitate was filtered, washed with water, to afford 19-(oxan-2-yl)-4-[2-(triethylsilyl)ethynyl]-8,14-dioxa-10,19,20-triazatetracyclo[13.5.2.1^{2,6}.0^{18,21}]tricosa-1(20),2(23),3,5,15(22),16,18(21)-heptaen-9-one 79 as a beige solid.
LCMS method F: [M+H]⁺ = 546, t_{R} = 3.88 min

### Preparation of intermediate 80: 4-ethynyl-19-(oxan-2-yl)-8,14-dioxa-10,19,20-triazatetracyclo[13.5.2.1²,⁶.0^{18,21}]tricosa-1(20),2(23),3,5,15(22),16,18(21)-heptaen-9-one

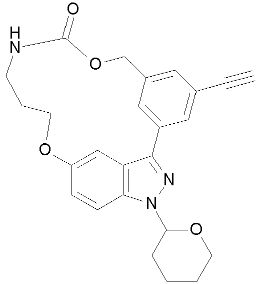

To a solution of 19-(oxan-2-yl)-4-[2-(triethylsilyl)ethynyl]-8,14-dioxa-10,19,20-triazatetra cyclo[13.5.2.1^{2,6}.0^{18,21}]tricosa-1(20),2(23),3,5,15(22),16,18(21)-heptaen-9-one 79 (97 mg, 0.21 mmol) in MeOH (2 mL) was added potassium carbonate (87 mg, 0.63 mmol). The resulting reaction mixture was stirred at 60°C for 3h. The reaction mixture was filtered and washed with water to afford 4-ethynyl-19-(oxan-2-yl)-8,14-dioxa-10,19,20-triazatetracyclo[13.5.2.1^{2,6}. 0^{18,21}]tricosa-1(20),2(23),3,5,15(22),16,18(21)-heptaen-9-one **80** as a white solid.
LCMS method F: [M+H]⁺ = 432, t_{R} = 2.93 min (current 20V)

### Preparation of Example 80: 4-ethynyl-8,14-dioxa-10,19,20-triazatetracyclo [13.5.2.1^{2,6}.0^{18,21}]tricosa-1(20),2(23),3,5,15(22),16,18(21)-heptaen-9-one

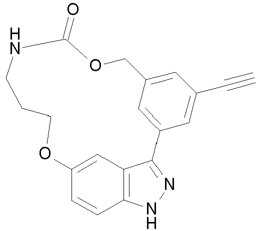

To a solution of 4-ethynyl-19-(oxan-2-yl)-8,14-dioxa-10,19,20-triazatetracyclo[13.5.2. 1^{2,6}.0^{18,21}]tricosa-1(20),2(23),3,5,15(22),16,18(21)-heptaen-9-one **80** (45 mg, 0.10 mmol) in DCM (2 mL) was added TFA (147 µL, 2.00 mmol) and stirred at RT for 12 hours. To the reaction mixture was added a saturated solution sodium bicarbonate (10 mL) and DCM (10 mL). The two layers were separated and the water layer was extracted twice with DCM (10 mL). The combined organic layers were washed with brine, dried over sodium sulfate, filtered and concentrated under reduced pressure to give a yellow oil. The residue was purified by flash chromatography (CyH/AE 5/5) to afford 4-ethynyl-8,14-dioxa-10,19,20-triazatetracyclo [13.5.2.1^{2,6}.0^{18,21}]tricosa-1(20),2(23),3,5,15(22),16,18(21)-heptaen-9-one **example 80** as a white solid.
LCMS method F: [M+H]⁺ = 348, t_{R} = 2.30 min
LCMS method G: [M+H]⁺ = 348, t_{R} = 2.30 min
¹H NMR (400 MHz, *d*6-DMSO) δ 13.02 (1H, s), 7.93 (2H, d, J = 16.3 Hz), 7.74 - 7.72 (1H, m), 7.52 - 7.48 (1H, m), 7.39 (1H, s), 7.32 (1H, d, J = 1.9 Hz), 7.00 (1H, dd, J = 2.3, 9.1 Hz), 5.29 - 5.27 (2H, m), 4.35 - 4.29 (2H, m), 4.08 - 4.07 (1H, m), 3.18 (2H, s), 2.02 (2H, s) ppm.

### Example 81: 4-(piperazin-1-yl)-8,14-dioxa-10,19,20-triazatetracyclo[13.5.2.1^{2,6}.0^{18,21}] tricosa-1(20),2(23),3,5,15(22),16,18(21)-heptaen-9-one

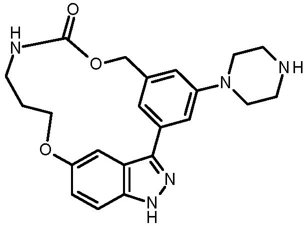

Example 81 is prepared according to the synthesis route described in general Scheme A. Tert-butyl piperazine-1-carboxylate is used for the Buchwald reaction with the bromide intermediate **34** to give 4-(piperazin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one **example 81.**
LCMS method F: [M+H]⁺ = 408.2, t_{R} = 1.45 min
LCMS method G: [M+H]⁺ = 408.2, t_{R} = 1.85 min
¹H NMR (400 MHz, d6-DMSO, 80 °C) δ 12.82 (1H, s), 7.62 (1H, s), 7.47 (1H, d, *J =* 9.3 Hz), 7.39 - 7.33 (3H, m), 6.96 (1H, dd, *J =* 2.3, 8.9 Hz), 6.88 - 6.86 (1H, m), 5.23 - 5.22 (2H, m), 4.33 - 4.27 (2H, m), 3.21 - 3.16 (7H, m), 2.96 - 2.92 (4H, m), 2.06 - 1.99 (2H, m) ppm.

### Example 82: 4-(1,2,3,6-tetrahydropyridin-4-yl)-8,14-dioxa-10,19,20-triazatetracyclo [13.5.2.1^{2,6}.0^{18,21}]tricosa-1(20),2(23),3,5,15(22),16,18(21)-heptaen-9-one

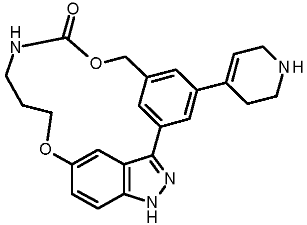

Example 82 is prepared according to the synthesis route described in general Scheme A. Tert-butyl 4-(4,4,5,5-tetramethyl-1,3,2-dioxaborolan-2-yl)-3,6-dihydro-2H-pyridine-1-carboxylate is used for the Suzuki coupling with the bromide intermediate **34** to give 4-(1,2,3,6-tetrahydropyridin-4-yl)-8,14-dioxa-10,19,20-triazatetracyclo[13.5.2.1^{2,6}.0^{18,21}]tricosa-1(20),2(23),3,5,15(22),16,18(21)-heptaen-9-one **example 82.**
LCMS method F: [M+H]⁺ = 405.2, t_{R} = 1.49 min
LCMS method G: [M+H]⁺ = 405.2, t_{R} = 1.95 min
¹H NMR (400 MHz, d6-DMSO, 80 °C) δ 12.95 (1H, s), 7.89 (1H, s), 7.79 (1H, s), 7.70 - 7.65 (1H, m), 7.50 - 7.47 (1H, m), 7.35 - 7.32 (2H, m), 6.98 (1H, dd, *J =* 2.4, 9.2 Hz), 6.29 - 6.25 (1H, m), 5.37 - 5.30 (2H, m), 4.34 - 4.28 (2H, m), 3.50 - 3.44 (2H, m), 3.14 - 3.00 (5H, m), 2.48 - 2.41 (2H, m), 2.06 - 2.03 (2H, m) ppm.

### Example 83: 11-(methoxymethyl)-8,14-dioxa-10,19,20-triazatetracyclo[13.5.2.1^{2,6}.0^{18,21}] tricosa-1(20),2(23),3,5,15(22),16,18(21)-heptaen-9-one

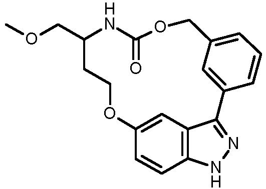

Example 83 is prepared according to the synthesis route described in general Scheme C.

### Preparation of intermediate 81: 3-benzylamino-4-methoxy-but-2-enoic acid methyl ester

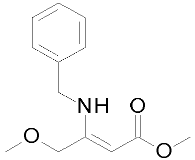

A solution of methyl-4-methoxyacetoacetate (5.00 g, 34.24 mmol), benzylamine (3.73 mL, 34.24 mmol) and acetic acid (0.097 mL, 1.71 mmol) in toluene (24 mL) was heated at 60°C for 6 hours. The mixture was concentrated under reduced pressure to give 3-benzylamino-4-methoxy-but-2-enoic acid methyl ester **81** as an orange oil. The crude was used in the next step without purification.

### Preparation of intermediate 82: methyl 3-(benzylamino)-4-methoxy-butanoate

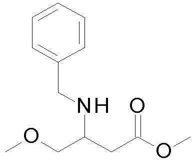

To a solution of 3-benzylamino-4-methoxy-but-2-enoic acid methyl ester **81** (8.66 g, 34.24 mmol postulated) in DCE (80 mL) were added at 0°C, acetic acid (9.77 mL, 171 mmol) and sodium tris(acetoxy)borohydride (21.67 g, 102.72 mmol). The reaction mixture was allowed to warm up to room temperature, then stirred at room temperature for 17 hours. The mixture was diluted with DCM (30 mL), quenched with a saturated solution of NaHCO₃ (40 mL). Water (50 mL) was added and the aqueous layer was extracted with DCM (2 x 70 mL). The combined organic layers were washed with brine (100 mL), dried over anhydrous sodium sulfate, filtered and concentrated under reduced pressure. The crude was purified by column (Macherey Nagel, 330 g) chromatography with cyclohexane/EtOAc (100/0 to 0/100) as eluent. DCM/MeOH (90/10) was used to elute the product. The desired fractions were combined and evaporated under reduced pressure to give methyl 3-(benzylamino)-4-methoxy-butanoate 82 as a brown oil.
LCMS method F: [M+H]⁺ = 238, t_{R} = 0.94 min

### Preparation of intermediate 83: 3-(benzylamino)-4-methoxy-butan-1-ol

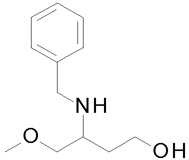

To a solution of methyl 3-(benzylamino)-4-methoxy-butanoate **82** (3.97 g, 16.8 mmol) in THF (70 mL) under N₂, LAH 1 M in THF (20.2 mL, 20.2 mmol) was added at 0°C. The reaction was stirred at 0°C for 18 hours. The mixture was quenched with water (1.8 mL), NaOH 10 % (1.8 mL) and water (1.8 mL). The mixture was filtered with EtOAc as eluent. The filtrate was diluted with water (50 mL) and extracted with EtOAc (3 x 50 mL). Combined organic layers were washed with brine (100 mL), dried with anhydrous sodium sulfate, filtered and evaporated under reduced pressure to give 3-(benzylamino)-4-methoxy-butan-1-ol **83** as a brown oil. The crude was used in the next step without further purification.
LCMS method H: [M+H]⁺ = 210, t_{R} = 0.48 min

### Preparation of intermediate 84: 3-amino-4-methoxy-butan-1-ol;2,2,2-trifluoroacetic acid

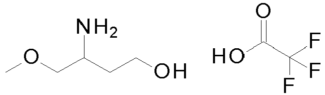

To a solution of 3-(benzylamino)-4-methoxy-butan-1-ol **83** (2.44 g, 11.7 mmol) in MeOH (200 mL), TFA (2.42 mL, 31.59 mmol) was added. The resulting mixture was degassed under N₂ during 15 min. Pd(OH)₂ (819 mg, 5.85 mmol) was added and the reaction mixture was heated at 50 °C under H₂ for 19 hours. The mixture was filtered over celite and the solvent was removed under reduced pressure to give 3-amino-4-methoxy-butan-1-ol;2,2,2-trifluoroacetic acid **84** as a yellow oil. The crude was used in the next step without further purification.

### Preparation of intermediate 85: benzyl N-[3-hydroxy-1-(methoxymethyl)propyl]carbamate

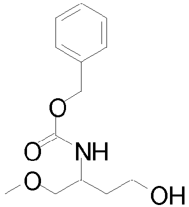

To a solution of 3-amino-4-methoxy-butan-1-ol;2,2,2-trifluoroacetic acid **84** (3.32 g, 13 mmol postulated) in THF (18 mL)/water (18 mL), was added at room temperature NaHCO₃ (3.28 g, 39 mmol). The reaction was stirred at room temperature during 15 minutes, then at 0 °C was slowly added CbzCl (1.85 mL, 13 mmol). The resulting mixture was stirred at room temperature overnight. The mixture was diluted with water (50 mL) and EtOAc (50 mL). After separation, the aqueous layer was extracted with EtOAc (3 x 50 mL). The combined organic layers were washed with brine (100 mL), dried over anhydrous sodium sulfate, filtered and concentrated under reduced pressure. The crude was purified by column (Macherey Nagel, 120 g) chromatography with cyclohexane/EtOAc (100/0 to 0/100), then DCM/MeOH (90/10) as eluent. The desired fractions were collected, combined and the solvent was removed under reduced pressure to give benzyl N-[3-hydroxy-1-(methoxymethyl)propyl]carbamate 85 as a colorless oil.
LCMS method F: [M+H]⁺ = 254, t_{R} = 1.86 min

### Preparation of intermediate 86: [3-(benzyloxycarbonylamino)-4-methoxy-butyl] methane sulfonate

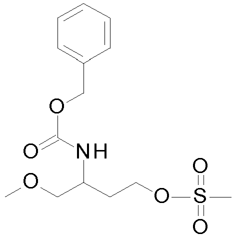

To a solution of benzyl N-[3-hydroxy-1-(methoxymethyl)propyl]carbamate **85** (1.94 g, 7.67 mmol) and diisopropylethylamine (2.66 mL, 15.34 mmol) in dichoromethane (70 mL) at 0 °C, was added dropwise methanesulfonyl chloride (0.71 mL, 9.20 mmol). The reaction mixture was stirred at room temperature for 2 hours. The mixture was diluted with water (100 mL) and DCM (100 mL). The water layer was extracted with DCM (2 x 100 mL). The combined organic layers were washed with brine (150 mL), dried over anhydrous sodium sulfate, filtered and the solvent was removed under reduced pressure to afford [3-(benzyloxycarbonylamino)-4-methoxy-butyl] methane sulfonate **86** as an orange viscous oil. The crude was used in the next step without further purification.
LCMS method F: [M+H]⁺ = 332, t_{R} = 2.18 min

### Preparation of intermediate 87: benzyl N-[3-(3-iodo-1-tetrahydropyran-2-yl-indazol-5-yl)oxy-1-(methoxymethyl)propyl]carbamate

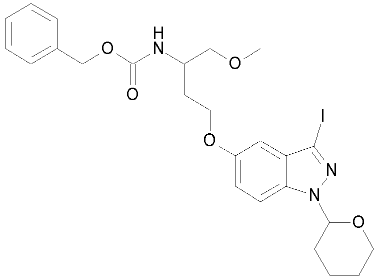

To a solution of 3-iodo-1-tetrahydropyran-2-yl-indazol-5-ol **86** (2.33 g, 6.77 mmol) in *N,N-*dimethylformamide (60 mL) were added cesium carbonate (6.21 g, 19.1 mmol) and [3-(benzyloxycarbonylamino)-4-methoxy-butyl] methane sulfonate (2.53 g, 7.64 mmol). The resulting mixture was heated at 60 °C overnight. The mixture was filtered and concentrated under reduced pressure. The residue was diluted with water (100 mL) and EtOAc (100 mL). After separation, the aqueous layer was extracted with EtOAc (2 x 100 mL). The combined organic layers were washed with brine (150 mL), dried over anhydrous sodium sulfate, filtered and evaporated under reduced pressure to afford a red oil. The crude was purified by column (Macherey Nagel, 120 g) flash chromatography with cyclohexane/EtOAc (100/0 to 80/20) as eluent. The desired fractions were collected, combined and the solvent was removed under reduced pressure to afford benzyl N-[3-(3-iodo-1-tetrahydropyran-2-yl-indazol-5-yl)oxy-1-(methoxymethyl)propyl]carbamate **87** as a white oil.
LCMS method F: [M+H]⁺ = 580, t_{R} = 3.13 min

### Preparation of intermediate 88: benzyl N-[3-[3-[3-(hydroxymethyl)phenyl]-1-tetrahydropyran-2-yl-indazol-5-yl]oxy-1-( methoxymethyl)propyl] carbamate

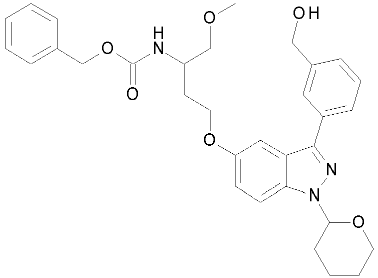

To a degassed solution of benzyl N-[3-(3-iodo-1-tetrahydropyran-2-yl-indazol-5-yl)oxy-1-(methoxymethyl)propyl]carbamate **87** (2.81 g, 4.85 mmol), [3-(hydroxymethyl)phenyl]boronic acid (1.11 g, 7.28 mmol) and a 1M solution of Na₂CO₃ (14.55 mL, 14.55 mmol) in DME (50 mL) was added palladium-tetrakis(triphenylphosphine) (277 mg, 0.24 mmol, 5 mol %). The reaction mixture was stirred at 80 °C for 2 days. More [3-(hydroxymethyl)phenyl]boronic acid (72 mg, 0.48 mmol), 1M solution of Na₂CO₃ (1.45 mL, 1.45 mmol) and palladium-tetrakis(triphenylphosphine) (56 mg, 0.049 mmol, 1 mol %) were added and the reaction mixture was stirred at 80 °C for 4 hours. After being cooled to room temperature, the reaction mixture was filtered over celite and the filtrate was diluted with water (100 mL) and extracted with EtOAc (3 x 100 mL). The combined organic layers were washed with brine (150 mL), dried over anhydrous sodium sulfate, filtered and concentrated under reduced pressure. The crude was purified by column (Macherey Nagel, 120 g) flash chromatography with cyclohexane/EtOAc (100/0 to 60/40) as eluent. The desired fractions were collected, combined and the solvent was removed under reduced pressure to afford benzyl N-[3-[3-[3-(hydroxymethyl)phenyl]-1-tetrahydropyran-2-yl-indazol-5-yl]oxy-1-(methoxymethyl)propyl] carbamate **88** as a yellow viscous oil.
LCMS method F: [M+H]⁺ = 560, t_{R} = 2.90 min

### Preparation of intermediate 89: 11-(methoxymethyl)-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one

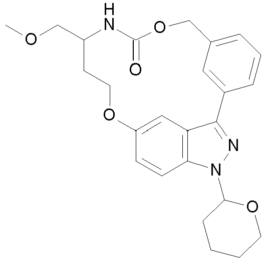

A suspension of benzyl N-[3-[3-[3-(hydroxymethyl)phenyl]-1-tetrahydropyran-2-yl-indazol-5-yl]oxy-1-(methoxymethyl)propyl]carbamate **88** (1.5 g, 2.68 mmol) and cesium carbonate (5.23 g, 16.08 mmol) in acetonitrile (600 mL) was heated to 90°C for 6 hours. The reaction mixture was filtered at 90°C, cooled to room temperature and concentrated under reduced pressure. The crude was purified by column (Macherey Nagel, 80 g) chromatography with cyclohexane/EtOAc (100/0 to 60/40) as eluent. The desired fractions were collected, combined and the solvent was removed under reduced pressure to give 11-(methoxymethyl)-19-(oxan-2-yl)-8,14-dioxa-10,19,20-triazatetracyclo[13.5.2.1^{2,6}.0^{18,21}]tricosa-1(20),2(23),3,5,15(22),16, 18(21)-heptaen-9-one **89** (757 mg, 1.67 mmol) as a white powder.
LCMS method F: [M+H]⁺ = 452, t_{R} = 2.87 min

### Preparation of Example 83: 11-(methoxymethyl)-8,14-dioxa-10,19,20-triazatetracyclo [13.5.2.1^{2,6}.0^{18,21}]tricosa-1(20),2(23),3,5,15(22),16,18(21)-heptaen-9-one

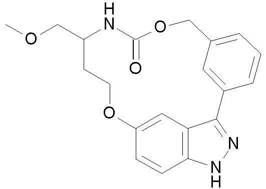

To a solution of 11-(methoxymethyl)-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one **89** (100 mg, 0.22 mmol) in DCM (15 mL) was added trifluoro acetic acid (0.34 mL, 4.4 mmol). The mixture was heated under microwave conditions at 80 °C for 1 hour. The solvent was removed under reduced pressure to afford an oily residue, which was dissolved in DCM (20 mL) and a saturated solution of NaHCO₃ (20 mL) was added. After separation, the aqueous layer was extracted with DCM (3 x 10 mL). The combined organic layers were washed with brine (20 mL), dried over anhydrous sodium sulfate, filtered and evaporated under reduced pressure. The crude was triturated in acetonitrile but the resulting solid was not clean. The crude was solubilized in DCM and combined with the filtrate. The solvent was removed under reduced pressure. The crude was purified by column (Macherey Nagel, 15 g) flash chromatography with cyclohexane/EtOAc (100/0 to 60/40) as eluent. The desired fractions was collected, combined and the solvent was removed under reduced pressure under reduced pressure to give 11-(methoxymethyl)-8,14-dioxa-10,19,20-triazatetracyclo[13.5.2.1^{2,6}.0^{18,21}] tricosa-1(20),2(23),3,5,15(22),16,18(21)-heptaen-9-one **example 83** as a pale yellow powder.
LCMS method F: [M+H]⁺ = 368, t_{R} = 2.21 min
LCMS method G: [M+H]⁺ = 368, t_{R} = 2.20 min
¹H NMR (400 MHz, d6-DMSO, 80 °C) δ 12.9 (1H, m), 7.94 (1H, s), 7.86 (1H, m), 7.58 (1H, m), 7.47 (2H, m), 7.37 (1H, d, J = 2.1 Hz), 7.27 (1H, m), 6.99 (1H, dd, J = 2.3, 8.9 Hz), 5.61 (1H, m), 4.96 (1H, m), 4.33 (2H, m), 3.81 (1H, m), 3.51 (1H, m), 3.43 (1 H, dd, J = 6.6, 10.0 Hz), 3.32 (3H, s), 2.15 (1H, m), 2.0 (1H, m) ppm.

### Example 84: 8,14-dioxa-5,10,19,20,23-pentaazatetracyclo[13.5.2.1^{2,5}.0^{18,21}]tricosa-1(20),2(23),3,15(22),16,18(21)-hexaen-9-one

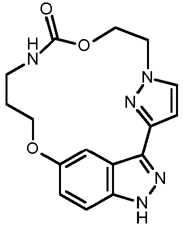

Example 84 is prepared according to the synthesis route described in general Scheme K.

### Preparation of intermediate 90: 2-(3-bromopyrazol-1-yl)ethanol

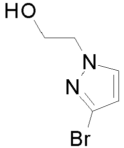

To a solution of 3-bromopyrazole (1 g, 6.807 mmol) in *N,N-*dimethylformamide (60 mL) was added potassium tert-butoxide solution (1M in THF) (10.2 mL, 10.211 mmol) at RT. The reaction mixture was stirred at RT for 10min then 1,3,2-Dioxathiolane 2,2-dioxide (1.267 g, 10.211 mmol) was added. The reaction mixture was stirred at RT for 4h30. Concentrated hydrochloric acid (6 mL) was added to the reaction mixture and it was stirred at RT for 16h. The reaction mixture was concentrated under reduced pressure and diluted with ethyl acetate. A saturated solution of NaHCO₃ was added and it was extracted with ethyl acetate (3 x). The combined organic layers were washed with water then brine, dried over sodium sulfate, filtered and concentrated under reduced pressure. The crude product was purified by flash column chromatography eluting with Cyclohexane / Ethyl acetate - EtOH (3-1), 100/0 to 60/40 to give 2-(3-bromopyrazol-1-yl)ethanol **90** as a colorless oil.
LCMS method F: [M+H]⁺ = 193, t_{R} = 1.25 min

### Preparation of intermediate 91: 2-[3-[5-[tert-butyl(dimethyl)silyl]oxy-1-tetrahydropyran-2-yl-indazol-3-yljpyrazol-1-yljethanol

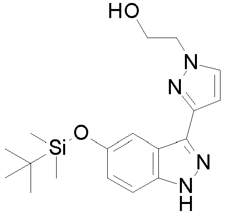

To a degassed solution of [5-[tert-butyl(dimethyl)silyl]oxy-1-tetrahydropyran-2-yl-indazol-3-yl]boronic acid (563 mg, 1.496 mmol), 2-(3-bromopyrazol-1-yl)ethanol (300 mg, 1.571 mmol), tripotassium phosphate (953 mg, 4.488 mmol), xPhos (71.5 mg, 0.150 mmol) in dioxane (4.52 mL) and water (1.51 mL) was added tetrakis(triphenylphosphine)palladium(0) (86.7 mg, 0.075 mmol). The reaction mixture was irradiated under microwave conditions (Biotage initiator+) at 100°C for 1h.The reaction mixture was filtered over celite and washed with ethyl acetate. The filtrate was diluted with water and extracted with ethyl acetate (3x). The combined organic layers were washed with water and brine, dried over sodium sulfate and concentrated under reduced pressure. The crude product was purified by column chromatography eluting with Cyclohexane / Ethyl acetate - EtOH (3-1), 100/0 to 70/30 to give 2-[3-[5-[tert-butyl(dimethyl)silyl]oxy-1-tetrahydropyran-2-yl-indazol-3-yl]pyrazol-1-yl]ethanol **91** as an orange oil.
LCMS method F: [M+H]⁺ = 443, t_{R} = 3.25 min

### Preparation of intermediate 92: 3-[1-(2-hydroxyethyl)pyrazol-3-yl]-1-tetrahydropyran-2-yl-indazol-5-ol

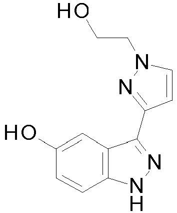

To a solution of 2-[3-[5-[tert-butyl(dimethyl)silyl]oxy-1-tetrahydropyran-2-yl-indazol-3-yl]pyrazol-1-yl]ethanol **91** (406 mg, 0.919 mmol) in THF (2 mL) was added a solution of tetrabutylammonium fluoride 1M in THF (1 mL, 1.012 mmol). The reaction mixture was stirred at room temperature for 16h. Ice water was added and the reaction mixture was stirred for 20 min. The aqueous phase was extracted with EtOAc (3x) and the combined organic layers were washed with brine, dried over Na₂SO₄, filtered and evaporated under reduced pressure. The crude product was purified by flash column chromatography eluting with Cyclohexane / Ethyl acetate - EtOH (3-1), 100/0 to 60/40 to give 3-[1-(2-hydroxyethyl)pyrazol-3-yl]-1-tetrahydropyran-2-yl-indazol-5-ol **92** as a yellow oil.
LCMS method F: [M+H]⁺ = 329, t_{R} = 1.92 min

### Preparation of intermediate 93: benzyl N-[3-[3-[1-(2-hydroxyethyl)pyrazol-3-yl]-1-tetrahydropyran-2-yl-indazol-5-ylloxypropyl]carbamate

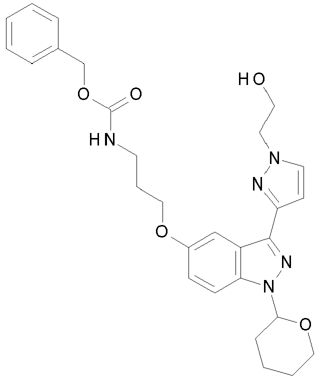

A suspension of 3-[1-(2-hydroxyethyl)pyrazol-3-yl]-1-tetrahydropyran-2-yl-indazol-5-ol **92** (150 mg, 0.457 mmol), cesium carbonate (297 mg, 0.914 mmol) and benzyl N-(3-bromopropyl)carbamate (98 µL, 0.503 mmol) in dry acetonitrile (4 mL) was stirred at RT for 16 h. More benzyl N-(3-bromopropyl)carbamate (25 µL, 0.091 mmol, 0.2 eq) in dry acetonitrile (1 mL) was added and the reaction mixture was stirred at RT for 32h. The reaction mixture was filtered and rinsed with ethyl acetate. Water was added and it was extracted with ethyl acetate (3 x). The combined organic layers were washed with water and brine, dried over sodium sulfate, filtered and concentrated under reduced pressure. The crude product was purified by flash column chromatography eluting with Cyclohexane / Ethyl acetate - EtOH (3-1), 100/0 to 60/40, to give benzyl N-[3-[3-[1-(2-hydroxyethyl)pyrazol-3-yl]-1-tetrahydropyran-2-yl-indazol-5-yl]oxypropyl]carbamate **93** as a white solid.
LCMS method F: [M+H]⁺ = 520, t_{R} = 2.64 min

### Preparation of intermediate 94: 19-(oxan-2-yl)-8,14-dioxa-5,10,19,20,23-pentaazatetracyclo [13.5.2.1^{2,5}.0^{18,21}]tricosa-1(20),2(23),3,15(22),16,18(21)-hexaen-9-one

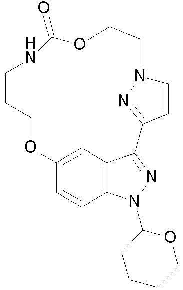

A solution of benzyl N-[3-[3-[1-(2-hydroxyethyl)pyrazol-3-yl]-1-tetrahydropyran-2-yl-indazol-5-yl]oxypropyl]carbamate **93** (187 mg, 0.360 mmol, 1eq) and cesium carbonate (702 mg, 2.160 mmol, 6 eq) in acetonitrile (54 mL) was stirred at 85°C for 16 h. The reaction mixture was cooled to RT, filtered, rinsed with ethyl acetate and evaporated under reduced pressure. The crude product was purified by column chromatography eluting with DCM / Ethyl acetate : 100/0 to 60/40 to give the expected product 19-(oxan-2-yl)-8,14-dioxa-5,10,19,20,23-pentaazatetracyclo[13.5.2.1^{2,5}.0^{18,21}]tricosa-1(20),2(23),3,15(22),16,18(21)-hexaen-9-one **94** as a white solid.
LCMS method F: [M+H]⁺ = 412, t_{R} = 2.28 min

### Preparation of Example 84: 8,14-dioxa-5,10,19,20,23-pentaazatetracyclo[13.5.2.1^{2,5}.0^{18,21}] tricosa-1(20),2(23),3,15(22),16,18(21)-hexaen-9-one

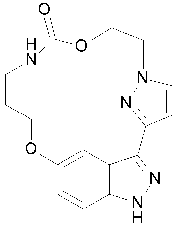

To a solution of 19-(oxan-2-yl)-8,14-dioxa-5,10,19,20,23-pentaazatetracyclo[13.5.2.1^{2,5}.0^{18,21}] tricosa-1(20),2(23),3,15(22),16,18(21)-hexaen-9-one **94** (51 mg, 0.124 mmol, 1 eq) in DCM (5 mL) was added trifluoroacetic acid (190 µL, 2.480 mmol, 20 eq) at RT. The solution was heated under microwave conditions at 80°C for 2 h. The reaction mixture was concentrated under reduced pressure and the brown residue was dissolved in ethyl acetate. A saturated aqueous solution of sodium hydrogen carbonate was added and it was extracted with ethyl acetate (3 x). The combined organic layers were washed with water and brine, dried over sodium sulfate, filtered and the solvent was removed under reduced pressure. The crude product was purified by flash column chromatography eluting with Cyclohexane / Ethyl acetate - EtOH (3-1), 100/0 to 50/50. The desired fractions were collected and the solvent was removed under reduced pressure. The compound was triturated with diisopropyl ether to give 8,14-dioxa-5,10,19,20,23-pentaazatetracyclo[13.5.2.1^{2,5}.0^{18,21}]tricosa-1(20),2(23),3,15(22),16,18(21)-hexaen-9-one **example 84** as a white solid.
LCMS method F: [M+H]⁺ = 328, t_{R} = 1.69 min
LCMS method G: [M+H]⁺ = 328, t_{R} = 1.66 min
¹H NMR (400 MHz, *d*6-DMSO) δ 12.83 (1H, s), 7.82 (1H, d), 7.65 (1H, m), 7.61 - 7.58 (1H, m), 7.40 (1H, d), 6.95 - 6.92 (1H, dd), 6.63 (1H, d), 4.52 - 4.50 (2H, m), 4.42 - 4.40 (2H, m), 4.28 - 4.24 (2H, m), 3.11 - 3.07 (2H, m), 1.90 - 1.84 (2H, m) ppm.

### Example 85: 11-methyl-8,14-dioxa-4,5,10,19,20-pentaazatetracyclo[13.5.2.1^{2,5}.0^{18,21}] tricosa-1(20),2(23),3,15(22),16,18(21)-hexaen-9-one

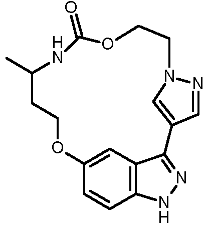

Example 85 is prepared according to the synthesis route described in general Scheme C to give 11-methyl-8,14-dioxa-4,5,10,19,20-pentaazatetracyclo[13.5.2.1^{2,5}.0^{18,21}]tricosa-1(20),2(23), 3,15(22),16,18(21)-hexaen-9-one **example 85.**
LCMS method F: [M+H]⁺ = 342, t_{R} = 1.83 min
LCMS method G: [M+H]⁺ = 342, t_{R} = 1.83 min
¹H NMR (400 MHz, *d*6-DMSO) δ 12.82 - 12.79 (1H, m), 8.10 (1H, s), 7.76 - 7.71 (2H, m), 7.44 - 7.39 (1H, m), 7.08 (1H, d, J = 0.9 Hz), 6.94 (1H, dd, J = 1.7, 8.9 Hz), 4.68 - 4.62 (1H, m), 4.55 - 4.41 (3H, m), 4.35 - 4.26 (1H, m), 4.08 - 4.03 (1H, m), 3.82 - 3.76 (1H, m), 1.98 (1H, s), 1.80 (1H, s), 1.14 - 1.04 (3H, m) ppm.

### Example 86: 12-methyl-8,14-dioxa-10,19,20-triazatetracyclo[13.5.2.1^{2,6}.0^{18,21}]tricosa-1(20),2(23),3,5,15(22),16,18(21)-heptaen-9-one

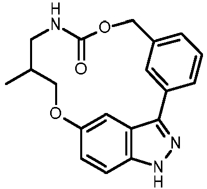

Example 86 is prepared according to the synthesis route described in general Scheme E to give 12-methyl-8,14-dioxa-10,19,20-triazatetracyclo[13.5.2.1^{2,6}.0^{18,21}]tricosa-1(20),2(23),3,5, 15(22),16,18(21)-heptaen-9-one **example 86.**
LCMS method F: [M+H]⁺ = 338.2, t_{R} = 3.12 min
LCMS method G: [M+H]⁺ = 338.2, t_{R} = 3.09 min
¹H NMR (400 MHz, *d*6-DMSO) δ 13.13 (brs, 1H), 8.04 - 7.99 (m, 1H), 7.88 - 7.83 (m, 2H), 7.52 - 7.44 (m, 2H), 7.31 - 7.27 (m, 2H), 7.02 (dd, J = 2.5, 9.0 Hz, 1H), 5.78 (d, J = 14.3 Hz, 1H), 4.80 (d, J = 14.3 Hz, 1H), 4.53 (d, J = 12.0 Hz, 1H), 3.75 (t, J = 12.0 Hz, 1H), 3.32 - 3.30 (m, 1H), 0.96 (d, J = 6.5 Hz, 3H) ppm. Two protons were located under the DMSO peak and are not reported here.

### Example 87: 11-ethyl-8,14-dioxa-10,19,20-triazatetracyclo[13.5.2.1^{2,6}.0^{18,21}]tricosa-1(20),2(23),3,5,15(22),16,18(21)-heptaen-9-one

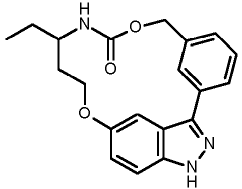

Example 87 is prepared according to the synthesis route described in general Scheme E to give 11-ethyl-8,14-dioxa-10,19,20-triazatetracyclo[13.5.2.1^{2,6}.0^{18,21}]tricosa-1(20),2(23),3,5, 15(22),16,18(21)-heptaen-9-one **example 87.**
LCMS method F: [M+H]⁺ = 352, t_{R} = 2.44 min
LCMS method G: [M+H]⁺ = 352, t_{R} = 2.38 min
¹H NMR (400 MHz, *d*6-DMSO, 80 °C) δ 12.87 (1H, m), 7.95 (1H, m), 7.86 (1H, m), 7.46 (3H, m), 7.37 (1H, d, J = 2.1 Hz), 7.27 (1H, dd, J = 0.7, 7.5 Hz), 6.98 (1H, dd, J = 2.3, 8.9 Hz), 5.67 (1H, m), 4.91 (1H, m), 4.35 (2H, m), 3.55 (1H, m), 2.21 (1H, m), 1.96 (1H, m), 1.57 (2H, m), 0.95 (3H, t, J = 7.5 Hz) ppm.

### Example 88: 4-fluoro-5,7-dimethyl-8,14-dioxa-10,19,20-triazatetracyclo[13.5.2.1^{2,6}.0^{18,21}] tricosa-1(20),2(23),3,5,15(22),16,18(21)-heptaen-9-one

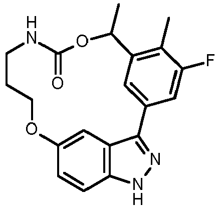

Example 88 is prepared according to the synthesis route described in general Scheme A.

### Preparation of intermediate 95: (5-bromo-3-fluoro-2-methyl-phenyl)methanol

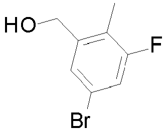

To a mixture of methyl 5-bromo-3-fluoro-2-methyl-benzoate (3.00 g, 12.14 mmol) in THF (35 mL) at 0 °C, was added a solution of DIBAL-H (30.4 mL, 30.36 mmol, 1.0 M solution in THF). The reaction mixture was stirred at room temperature for 16 hours. The reaction mixture was diluted with ethyl acetate and cold 1 N HCl was added. The organic layer was washed with 1 N HCl and brine, dried over MgSO₄, filtered and the solvent was removed under reduced pressure to afford (5-bromo-3-fluoro-2-methyl-phenyl)methanol **95** as a white solid. The crude product was used in the next step without any further purification.
LCMS method F: [M-H₂O+H]⁺ = 201.0, t_{R} = 2.35 min

### Preparation of intermediate 96: 5-bromo-3-fluoro-2-methyl-benzaldehyde

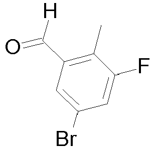

To a solution of (5-bromo-3-fluoro-2-methyl-phenyl)methanol **95** (2.66 g, 12.14 mmol) in DCM (160 mL) was added portion wise manganese dioxide (10.56 g, 121.4 mmol). After stirring for 18 h at room temperature, the suspension was filtered through a sinter funnel. The filtrate was dried over anhydrous magnesium sulfate, filtered and the solvent was removed under reduced pressure to give 5-bromo-3-fluoro-2-methyl-benzaldehyde **96** as a slightly yellow. The crude product was used in the next step without any further purification.
LCMS method F: no m/z detected, t_{R} = 2.67 min (current 20V)

### Preparation of intermediate 97: 1-(5-bromo-3-fluoro-2-methyl-phenyl)ethanol

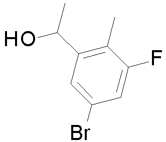

To a cooled solution of 5-bromo-3-fluoro-2-methyl-benzaldehyde **96** (2.43 g, 11.20 mmol) in dry tetrahydrofuran (30 mL) was added dropwise at 0 °C a 3M methylmagnesium bromide solution in diethyl ether (7.5 mL, 22.40 mmol). The reaction mixture was stirred at 0 °C for 20 min and allowed to reach room temperature for 16 hours. The reaction mixture was quenched with a saturated aqueous solution of NH₄Cl then extracted with ethyl acetate (2 x 100 mL). The organic layer was washed with water the brine, dried over magnesium sulfate, filtered and concentrated under reduced pressure. The crude product was purified by flash-column chromatography eluting with Cyclohexane / Ethyl acetate - EtOH (3-1): 100 / 0 to 80 / 20, to give 1-(5-bromo-3-fluoro-2-methyl-phenyl)ethanol **97** as a colorless oil.
LCMS method F: [M-H₂O+H]⁺ = 217.0, t_{R} = 2.50 min

### Preparation of intermediate 98: 1-[3-fluoro-2-methyl-5-(4,4,5,5-tetramethyl-1,3,2-dioxaborolan-2-yl)phenyl]ethanol

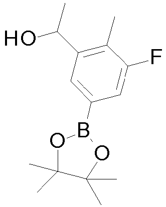

To a degassed solution in a sealed tube of 1-(5-bromo-3-fluoro-2-methyl-phenyl)ethanol **97** (2.49 g, 10.66 mmol), bis(pinacolato)diboron (4.06 g, 15.99 mmol) and potassium acetate (4.18 g, 42.64 mmol) in dioxane (30 mL) was added [1,1'-bis(diphenylphosphino)ferrocene] dichloropalladium(II), complex with DCM (0.874 g, 1.07 mmol). The reaction mixture was stirred under argon atmosphere at 100 °C for 16 hours. The reaction mixture was filtered over celite on Whatman paper and rinsed with ethyl acetate. The reaction mixture was diluted with water and extracted with ethyl acetate (3 x 100 mL). The combined organic layers were washed with water then brine, dried over anhydrous magnesium sulfate and concentrated under reduced pressure to afford 1-[3-fluoro-2-methyl-5-(4,4,5,5-tetramethyl-1,3,2-dioxaborolan-2-yl)phenyl]ethanol **98** as a dark brown oil. The crude product was used in the next step without any purification.
LCMS method F: [M-H₂O+H]⁺ = 263.2, t_{R} = 2.77 min

### Preparation of intermediate 99: benzyl N-[3-[3-[3-fluoro-5-(1-hydroxyethyl)-4-methylphenyl]-1-tetrahydropyran-2-yl-indazol-5-yl]oxypropyl]carbamate

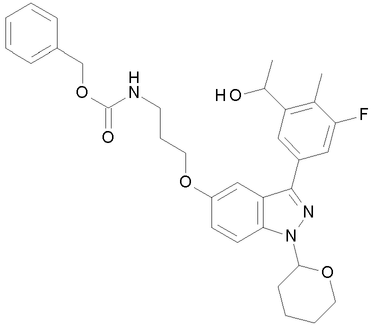

A solution of benzyl N-[3-(3-iodo-1-tetrahydropyran-2-yl-indazol-5-yl)oxypropyl]carbamate **26** (700 mg, 1.31 mmol), 1-[3-fluoro-2-methyl-5-(4,4,5,5-tetramethyl-1,3,2-dioxaborolan-2-yl)phenyl]ethanol **98** (403 mg, 1.44 mmol), potassium phosphate tribasic (833 mg, 3.93 mmol), Xphos (62 mg, 0.13 mmol) and palladium-tetrakis(triphenylphosphine) (75 mg, 0.065 mmol, 5 mol %) in a mixture of dioxane (22 mL) and water (5 mL) was heated at 110 °C for 2 days. The solution was diluted with water and extracted twice with EtOAc. The combined organic layers were washed with brine, dried over magnesium sulfate, filtered and concentrated under reduced pressure. The crude was purified by chromatography on silica gel (DCM/MeOH : 100/0 to 95/5) to give benzyl N-[3-[3-[3-fluoro-5-(1-hydroxyethyl)-4-methyl-phenyl]-1-tetrahydropyran-2-yl-indazol-5-yl]oxypropyl]carbamate **99** as a slightly yellow oil.
LCMS method F: [M+H]⁺ = 562.3, t_{R} = 3.15 min

### Preparation of intermediate 100: 4-fluoro-5,7-dimethyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one and 1-[5-[5-(3-aminopropoxy)-1-tetrahydropyran-2-yl-indazol-3-yl]-3-fluoro-2-methyl-phenyl]

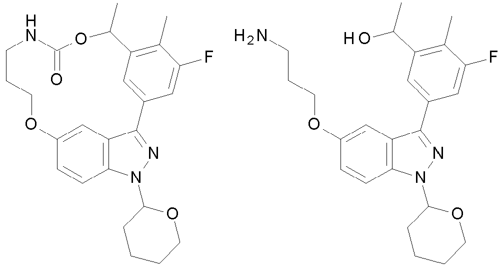

To a solution of benzyl N-[3-[3-[3-fluoro-5-(1-hydroxyethyl)-4-methyl-phenyl]-1-tetrahydropyran-2-yl-indazol-5-yl]oxypropyl]carbamate **99** (594 mg, 1.06 mmol) in anhydrous acetonitrile (200 mL) at room temperature was added cesium carbonate (861 mg, 2.65 mmol). The resulting reaction mixture was stirred at 90 °C for 17h30. LCMS showed the formation the expected macrocycle (40 % by LCMS) and a side-product arising from the carbamate hydrolysis (42 % by LCMS). The reaction mixture was filtered and concentrated under reduced pressure to afford a mixture of 4-fluoro-5,7-dimethyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one and 1-[5-[5-(3-aminopropoxy)-1-tetrahydropyran-2-yl-indazol-3-yl]-3-fluoro-2-methyl-phenyl] ethanol **100** as a yellow solid. The crude mixture was used in the next step (CDI, DMA, 90 °C) without any purification.
LCMS method F: expected macrocycle [M+H]⁺ = 454.2, t_{R} = 3.01 min
LCMS method F: hydrolyzed product [M+H]⁺ = 428.2, t_{R} = 1.94 min

### Preparation of intermediate 101: 4-fluoro-5,7-dimethyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one

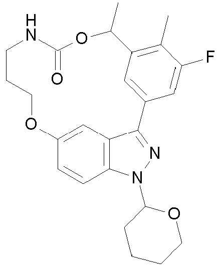

To a solution of 4-fluoro-5,7-dimethyl-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one and 1-[5-[5-(3-aminopropoxy)-1-tetrahydropyran-2-yl-indazol-3-yl]-3-fluoro-2-methyl-phenyl]ethanol **100** (0.452 g, 1.06 mmol) in dimethylacetamide (350 mL) was added 1,1'-carbonyldiimidazole (0.188 g, 1.16 mmol). The reaction mixture was stirred at room temperature for 16 hours. The mixture was diluted in water and extracted three times with ethyl acetate (3 x 100 mL). The combined organic layers were washed with water, then brine, dried over anhydrous magnesium sulfate, filtered and concentrated under reduced pressure. The crude product was purified by silica gel column chromatography (eluent: DCM/MeOH from 100/0 to 95/5) to afford 4-fluoro-5,7-dimethyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one **101** as a colorless oil.
LCMS method F: [M+H]⁺ = 454.1, t_{R} = 2.99 min (current 20V)

### Preparation of Example 88: 4-fluoro-5,7-dimethyl-8,14-dioxa-10,19,20-triazatetracyclo [13.5.2.1^{2,6}.0^{18,21}]tricosa-1(20),2(23),3,5,15(22),16,18(21)-heptaen-9-one

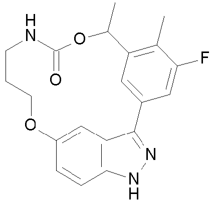

To a solution of 4-fluoro-5,7-dimethyl-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one **101** (0.080 g, 0.176 mmol) in DCM (15 mL) was added trifluoroacetic acid (0.27 mL, 3.52 mmol). The mixture was heated under microwave conditions at 80°C for 1h15. The solvent was removed under reduced pressure to give a mixture of a yellow solid in an oily residue. The solid residue appeared to be the expected product, which was not soluble enough for column chromatography purification. In order to remove impurities from this solid, the crude mixture was suspended in MeOH (3 mL) and refluxed for 3 hours. The suspension was filtered and the filter cake was rinsed with MeOH. This filter cake was suspended again in MeOH (3 mL) and refluxed for 3 hours. After filtration, the residual solid was suspended in water (3 mL) and reflux for 2 hours. After filtration of the resulting suspension, the solid was collected and dried under reduced pressure at 60°C to afford 4-fluoro-5,7-dimethyl-8,14-dioxa-10,19,20-triazatetracyclo[13.5.2.1^{2,6}.0^{18,21}] tricosa-1(20),2(23),3,5,15(22),16,18(21)-heptaen-9-one **example 88** as a white solid.
LCMS method F: [M+H]⁺ = 370.2, t_{R} = 2.38 min
LCMS method G: [M+H]⁺ = 370.2, t_{R} = 2.35 min
¹H NMR (400 MHz, *d*6-DMSO, 80 °C) δ 12.89 (1H, br. s), 7.70 - 7.67 (2H, m), 7.49 - 7.42 (2H, m), 7.36 (1H, s), 7.00 - 6.97 (1H, m), 5.81 - 5.68 (1H, m), 4.35 - 4.25 (2H, m), 3.52 (1H, br. s), 2.77 (1H, br. s), 2.27 (3H, d, *J =* 1.9 Hz), 2.12 (1H, br. s), 1.75 (1H, br. s), 1.50 (3H, d, *J* = 7.2 Hz) ppm.

### Example 89: 4-fluoro-5-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-one

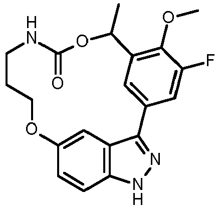

Example 89 is prepared according to the synthesis route described in general Scheme A and according to the procedures described to obtain **example 88** to give 4-fluoro-5-methoxy-7-methyl-8,14-dioxa-10,19,20-triazatetracyclo[13.5.2.1^{2,6}.0^{18,21}]tricosa-1(20),2(23),3,5,15(22), 16,18(21)-heptaen-9-one **example 89.**
LCMS method F: [M+H]⁺ = 386.2, t_{R} = 2.40 min
LCMS method G: [M+H]⁺ = 386.2, t_{R} = 2.37 min
¹H NMR (400 MHz, *d*6-DMSO, 80 °C) δ 12.95 (1H, br. s), 7.74 (1H, s), 7.64 (1H, s), 7.55 (1H, d, *J =* 13.1 Hz), 7.49 - 7.46 (1H, m), 7.34 (1H, s), 7.01 - 6.97 (1H, m), 5.80 (1H, s), 4.38 - 4.27 (2H, m), 4.00 - 3.99 (3H, m), 3.53 (1H, br. s), 2.78 (1H, br. s), 2.15 (1H, br. s), 1.76 (1H, br. s), 1.54 (3H, d, *J =* 6.6 Hz) ppm.

### Example 90: 5-fluoro-4,7-dimethyl-8,14-dioxa-10,19,20-triazatetracyclo[13.5.2.1^{2,6}.0^{18,21}] tricosa-1(20),2(23),3,5,15(22),16,18(21)-heptaen-9-one

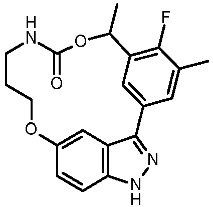

Example 90 is prepared according to the synthesis route described in general Scheme A and according to the procedures described to obtain **example 88** to give 5-fluoro-4,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-one **example 90.**
LCMS method F: [M+H]⁺ = 370.2, t_{R} = 2.44 min
LCMS method G: [M+H]⁺ = 370.2, t_{R} = 2.43 min
¹H NMR (400 MHz, *d*6-DMSO, 80 °C) δ 12.83 (1H, s), 7.73 - 7.69 (3H, m), 7.48 - 7.44 (1H, m), 7.37 - 7.35 (1H, m), 6.98 (1H, dd, *J =* 2.3, 8.9 Hz), 5.88 - 5.82 (1H, m), 4.38 - 4.25 (2H, m), 3.56 (1H, br. s), 2.81 - 2.76 (1H, m), 2.36 - 2.33 (3H, m), 2.19 - 2.14 (1H, m), 1.80 - 1.73 (1H, m), 1.58 (3H, d, *J =* 6.8 Hz) ppm.

### Example 91: 8,14-dioxa-10,19,20-triazapentacyclo[13.5.2.1^{2,6}.1^{7,10}.0^{18,21}]tetracosa-1(20),2(24),3,5,15(22),16,18(21)-heptaen-9-one

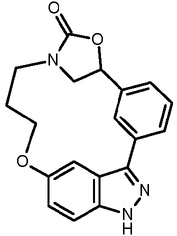

Example 91 is prepared according to the synthesis route described below.

### Preparation of intermediate 102: 1-(3-bromophenyl)-2-nitro-ethanol

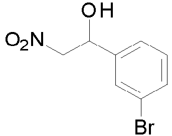

In a round bottom flask, to a stirred solution of 3-bromo-benzaldehyde (1.850 g, 10.00 mmol) in THF (50.0 ml) was added dropwise at 0°C, nitromethane (535 µL, 10.00 mmol) and then sodium hydroxide solution 1N (10.00 ml, 10.00 mmol). The mixture was stirred during 1 h. LC/MS analysis indicated 70% formation of required product and 50% starting material. The orange mixture was stirred during 3h at room temperature. The solution was carrefuly quenched with a solution of acetic acid (10 ml) and water (20 ml). Phases were separated and the aqueous layer was extracted with AcOEt (3 x 35 ml). Combined organic layer were washed with brine (30 ml), and dried over MgSO4, filtered and concentrated under reduce pressure to afford a crude material (1.720 g). The crude was purified by chromatography column by solid deposit (Macherey Nagel, 4 g, Cyclohexane/AcOEt :90/10 to 70/30). Solvent was evaporated to afford 1-(3-bromophenyl)-2-nitro-ethanol **102** as a yellow pale oil.
LCMS method F: [M-H]⁻ = 246.1, t_{R} = 2.24 min

### Preparation of intermediate 103: 2-amino-1-(3-bromophenyl)ethanol

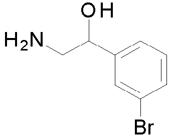

The reaction was divided in 2 batches of 730 mg (2.97 mmol) of 1-(3-bromophenyl)-2-nitro-ethanol **102.** To a solution of 1-(3-bromophenyl)-2-nitro-ethanol (0.730 g, 2.97 mmol) in EtOH/water (2:1 v:v) were added iron powder (0.829 g, 29.67 mmol) and ammonium chloride (4.758 g, 177.90 mmol). The resulting brown mixture was stirred during 16 h at room temperature. The solution was filtered to remove iron. Solvent was concentrated and to the residue was added water and EtOAc (50 ml) and phases were separated and extracted with EtOAc (3 x 50 ml). Organic phases were gathered and washed with brine, dried with MgSO₄, filtered and concentrated under reduc pressure to afford 2-amino-1-(3-bromophenyl)ethanol **103** as a pale yellow oil which was used in the next step without further purification.
LCMS method H: [M+H]⁺ = 216.0, t_{R} = 1.02 min

### Preparation of intermediate 104: 5-(3-bromophenyl)oxazolidin-2-one

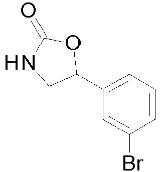

To a solution of 2-amino-1-(3-bromophenyl)ethanol **103** (0.710 g, 3.29 mmol) in THF (33.0 mL) was added 1,1'-Carbonyldiimidazole (0.587 g, 3.62 mmol) and imidazole (0.246 g, 3.62 mmol). The reaction mixture was stirred at RT for 16h. To the reaction mixture was added saturated aqueous solution of NH₄Cl (30 ml). The mixture was extracted with ethyl acetate (3 x 30 ml). The combined organic layer was washed with water then brine, dried over sodium sulfate, filtered and concentrated under reduced pressure. The crude product was purified by column chromatography on a biotage eluting with cyclohexane / ethyl acetate: 100/0 to 70/30 to give 5-(3-bromophenyl)oxazolidin-2-one **104.**

### Preparation of intermediate 105: 5-(3-bromophenyl)-3-[3-[tert-butyl(dimethyl)silyl] oxypropyl]oxazolidin-2-one

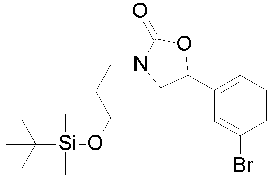

To a solution of 5-(3-bromophenyl)oxazolidin-2-one **104** (0.580 g, 2.40 mmol) in dry THF (25.0 ml) at 0°C was added sodium hydride (0.115 g, 4.80 mmol). The reaction was stirred 30 min at 0°C, then tetrabutylammonium iodide (0.044 g, 0.120 mmol) and 3-bromopropoxy-tert-butyl-dimethyl-silane (0.669 g, 612 µL, 2.64 mmol) were added. The resulting yellow mixture was stirred at 60°C during 2 days. It was quenched with a saturated solution of NaHCO₃ (25 ml) and extracted with EtOAc (3 x 50 ml). Organic phase was washed with brine (25 ml), then dried with MgSO₄, filtered and concentrated under reduce pressure to afford crude (1.200 g) which was purified by chromatography column by solid deposit (Macherey Nagel 24g, Cyclohexane/AcOEt 90/10 to 70/30). Solvent was evaporated to afford 5-(3-bromophenyl)-3-[3-[tert-butyl(dimethyl)silyl]oxypropyl]oxazolidin-2-one **105** as a pale yellow oil.
LCMS method F: [M+H]⁺ = 416.1, t_{R} = 3.41 min

### Preparation of intermediate 106: 5-(3-bromophenyl)-3-(3-hydroxypropyl)oxazolidin-2-one

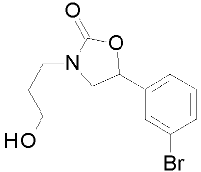

To a solution of 5-(3-bromophenyl)-3-[3-[tert-butyl(dimethyl)silyl]oxypropyl]oxazolidin-2-one **105** (0.650 g, 1.57 mmol) in dry THF (31.0 ml) at room temperature was added tetra-n-butylammonium fluoride (1.57 ml, 1.57 mmol, 1.0 M in THF). The reaction was stirred at room temperature during 16 h. The mixture was poured into ice water (20 ml) and stirred for 15 min. The aqueous phase was extracted with ethyl acetate (3 x 25 ml). The combined organic layers were washe with brine (25 ml), dried over MgSO₄, filtered and concentrated under reduce pressure to afford 5-(3-bromophenyl)-3-(3-hydroxypropyl)oxazolidin-2-one **106** as pale yellow oil.
LCMS method F: [M+H]⁺ = 302.0, t_{R} = 2.00 min

### Preparation of intermediate 107: 3-[5-(3-bromophenyl)-2-oxo-oxazolidin-3-yl]propyl methanesulfonate

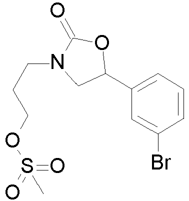

To a solution of 5-(3-bromophenyl)-3-(3-hydroxypropyl)oxazolidin-2-one **106** (0.420 g, 1.40 mmol) and diisopropylethylamine (0.487 mL, 2.80 mmol) in DCM (15.0 mL) at 0 °C, was added dropwise methanesulfonyl chloride (0.130 mL, 1.68 mmol). The reaction mixture was stirred at room temperature for 3 h. LC/MS analysis indicated the reaction was completed. The organic phase was washed with a saturated solution of ammonium chloride, with a saturated solution of sodium bicarbonate and brine, dried with MgSO₄, filtered and evaporated under reduced pressure to afford 3-[5-(3-bromophenyl)-2-oxo-oxazolidin-3-yl]propyl methanesulfonate **107** as a pale yellow oil, which was used in the next step without further purification.
LCMS method F: [M+H]⁺ = 380.1, t_{R} = 2.28 min

### Preparation of intermediate 108: 5-(3-bromophenyl)-3-[3-(1-tetrahydropyran-2-ylindazol-5-yl)oxypropyl]oxazolidin-2-one

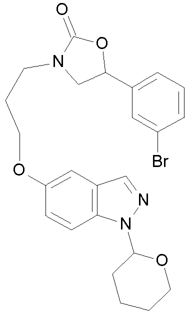

To a solution of 3-[5-(3-bromophenyl)-2-oxo-oxazolidin-3-yl]propyl methanesulfonate **107** (0.912 g, 1.40 mmol) in DMF (28.0 mL), cesium carbonate (0.913 g, 2.80 mmol) and 1-tetrahydropyran-2-ylindazol-5-ol (0.305 g, 1.40 mmol) were added. The reaction was stirred at 60°C during 1h30. The mixture was concentrated under reduced pressure. Water (50 mL) was added and the resulting mixture was extracted with AcOEt (4 x 30 mL). Combined organic layers were washed with saturated brine (30 mL). The organic layer was dried over sodium sulfate, filtered off and evaporated under reduced pressure to afford brown oil. This resiude was purified by flash chromatography on silica gel (Macherey Nagel, 24 g, with gradient elution: Cyclohexane/AcOEt: 100/0 to 70/30) to give 5-(3-bromophenyl)-3-[3-(1-tetrahydropyran-2-ylindazol-5-yl)oxypropyl]oxazolidin-2-one **108** as a yellow oil.
LCMS method F: [M+H]+ = 502.0, t_{R} = 2.45 min

### Preparation of intermediate 109: 19-(oxan-2-yl)-8,14-dioxa-10,19,20-triazapentacyclo [13.5.2.1^{2,6}.1^{7,10}.0^{18,2}]tetracosa-1(20),2(24),3,5,15(22),16,18(21)-heptaen-9-one

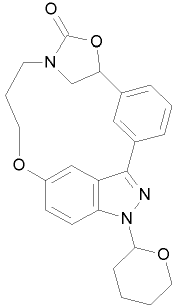

To a solution of 5-(3-bromophenyl)-3-[3-(1-tetrahydropyran-2-ylindazol-5-yl)oxypropyl]oxazolidin-2-one **108** (0.300 g, 0.600 mmol) in 10.0 ml of toluene was added reagent potassium acetate (0.118 g, 1.200 mmol) at room temperature. The mixture was degassed by bubbling nitrogen for 15 minutes. Palladium acetate (0.027 g, 0.120 mmol) and cataCXium A (0.043 g, 0.120 mmol) were then added. The mixture was heated at 120°C for 1h under microwaves irradiations (BIOTAGE), then 1h30 at 130°C and 45 min at 140°C. The reaction mixture was filtered over celite and 20 ml of water were added to the filtrate. The aqueous layer was extracted with ethyl acetate (2 x 20 ml). The combined organic layer was washed with a saturated brine, dried over MgSO₄ and evaporated in vacuo to give crude (0.280 g), which was purified by column chromatography (Macherey Nagel, 12g, DCM/MeOH ammoniac : 100/0 to 95/5). Solvents were evaporated to afford 19-(oxan-2-yl)-8,14-dioxa-10,19,20-triazapentacyclo[13.5.2.1^{2,6}.1^{7,10}.0^{18,21}]tetracosa-1(20),2(24),3,5,15(22),16,18(21)-heptaen-9-one **109** as a yellow powder.
LCMS method F: [M+H]⁺ = 420.2, t_{R} = 2.57 min

### Preparation of Example 91: 8,14-dioxa-10,19,20-triazapentacyclo[13.5.2.1^{2,6}.1^{7,10}.0^{18,21}] tetracosa-1(20),2(24),3,5,15(22),16,18(21)-heptaen-9-one

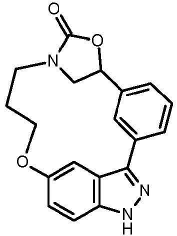

In a vial, 19-(oxan-2-yl)-8,14-dioxa-10,19,20-triazapentacyclo[13.5.2.1^{2,6}.1^{7,10}.0^{11,21}]tetracosa-1(20),2(24),3,5,15(22),16,18(21)-heptaen-9-one **109** (0.195 g, 0.465 mmol) was dissolved in CH₂Cl₂ (9.0 mL) and TFA (0.1 M in CH₂Cl₂, 80 µL) was added. The resulting clear yellow solution was stirred at room temperature for 3 days. The reaction was quenched with saturated aqueous NaHCO₃ (10 mL) and EtOAc (10 mL) was added. The aqueous phase was extracted with EtOAc (3 x 15 mL) and the combined organic extracts were washed with saturated aqueous NaHCO₃ (15 mL) and saturated aqueous NaCl (15 mL), dried over anhydrous MgSO₄, filtered and concentrated under reduced pressure to afford crude (0.195 g) which purified by preparative reverse-phase chromatography (Column XSELECT PHENYL-HEXYL 19*100mm 5µm [(NH4)2CO3 aq 2g/LACN] 30%B to 40%B in 7min 19mL/min R.T.). Solvent was removed and an other purification was done (0.015 g crude) by chromatography column (Macherey Nagel 4g, DCM/MeOH: 100/0 to 96/4) to give 8,14-dioxa-10,19,20-triazapentacyclo[13.5.2.1^{2,6}.1^{7,10}.0^{18,21}]tetracosa-1(20),2(24),3,5,15(22),16,18(21)-heptaen-9-one **example 91.**
LCMS method F: [M+H]⁺ = 336.2, t_{R} = 1.97 min
LCMS method G: [M+H]⁺ = 336.2, t_{R} = 1.96 min
¹H NMR (400 MHz, *d*6-DMSO) δ 12.86 (1H, s), 8.39 (1H, t, J = 1.7 Hz), 7.92 - 7.86 (1H, m), 7.58 - 7.41 (4H, m), 7.01 - 6.97 (1H, m), 5.69 - 5.69 (2H, m), 4.46 - 4.37 (1H, m), 4.30 - 4.23 (1H, m), 4.12 - 3.99 (2H, m), 3.61 - 3.40 (1H, m), 2.35 - 2.24 (1H, m), 2.04 - 1.92 (1H, m) ppm.

### Example 92: 13-methyl-8,14-dioxa-10,19,20,23-tetraazatetracyclo[13.5.2.1^{2,6}.0^{18,21}] tricosa-1(20),2(23),3,5,15(22),16,18(21)-heptaen-9-one

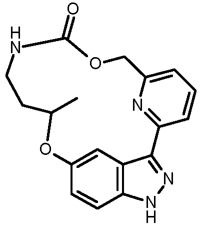

Example 92 is prepared according to the synthesis route described in general Scheme B to give 13-methyl-8,14-dioxa-10,19,20,23-tetraazatetracyclo[13.5.2.1^{2,6}.0^{18,21}]tricosa-1(20),2(23),3,5, 15(22),16,18(21)-heptaen-9-one **example 92.**
LCMS method F: [M+H]⁺ = 339.2, t_{R} = 2.09 min
LCMS method G: [M+H]⁺ = 339.2, t_{R} = 2.07 min
¹H NMR (400 MHz, *d*6-DMSO) δ 13.22 (1H, s), 8.09 - 8.06 (1H, m), 7.90 - 7.81 (2H, m), 7.74 - 7.70 (1H, m), 7.48 - 7.44 (1H, m), 7.26 - 7.23 (1H, m), 6.95 (1H, dd, J=2.5, 8.9 Hz), 5.58 (1H, s), 5.08 - 5.04 (1H, m), 4.62 (1H, s), 3.45 (1H, m), 2.92 (1H, s), 2.29 - 2.25 (1H, m), 1.38 - 1.35 (4H, m) ppm.

### Example 93: 12-methyl-8,14-dioxa-4,5,10,19,20-pentaazatetracyclo[13.5.2. 1^{2,5}.0^{18,21}] tricosa-1(20),2(23),3,15(22),16,18(21)-hexaen-9-one

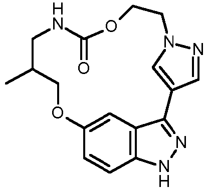

Example 93 is prepared according to the synthesis route described in general Scheme C to give 12-methyl-8,14-dioxa-4,5,10,19,20-pentaazatetracyclo[13.5.2.1^{2,5 18,21}]tricosa-1(20),2(23),3, 15(22),16,18(21)-hexaen-9-one **example 93.**
LCMS method F: [M+H]⁺ = 342.1, t_{R} = 2.45 min
LCMS method G: [M+H]⁺ = 342.2, t_{R} = 2.40 min
¹H NMR (400 MHz, *d*6-DMSO) δ 12.80 (brs, 1H), 8.08 (s, 1H), 7.95 - 7.91 (m, 1H), 7.77 (s, 1H), 7.42 (d, *J =* 9.6 Hz, 1H), 7.07 (d, *J =* 2.5 Hz, 1H), 6.99 - 6.96 (m, 1H), 4.67 - 4.43 (m, 4H), 4.07 (ddt, *J =* 2.4, 5.6, 6.1 Hz, 1H), 3.70 (dd, *J =* 10.1, 12.7 Hz, 1H), 3.36 - 3.26 (m, 1H), 2.58 - 2.53 (m, 1H), 2.22 - 2.10 (m, 1H), 0.93 (d, *J =* 6.6 Hz, 3H) ppm.

### Example 94: 7-methyl-8,14-dioxa-4,5,10,19,20-pentaazatetracyclo[13.5.2.1^{2,5}.0^{18,21}] tricosa-1(20),2(23),3,15(22),16,18(21)-hexaen-9-one

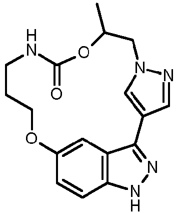

Example 94 is prepared according to the synthesis route described in general Scheme C.

### Preparation of intermediate 110: 1-[4-(4,4,5,5-tetramethyl-1,3,2-dioxaborolan-2-yl)pyrazol-1-yl]propan-2-ol

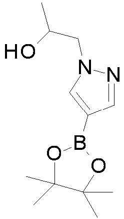

In a microwave vial, NaH (60% in mineral oil, 480 mg, 12.000 mmol) was suspended in *N,N-*dimethylformamide (10.0 mL) and a solution of 4-(4,4,5,5-tetramethyl-1,3,2-dioxaborolan-2-yl)-1H-pyrazole (776 mg, 4.000 mmol) in *N,N*-dimethylformamide (10.0 mL) was added. The resulting cloudy white solution was stirred at room temperature for 15 minutes and *rac-*propylene oxide (839 µL, 697 mg, 12.000 mmol) was added. The vial containing the resulting cloudy yellow solution was sealed and heated to 80°C for 2 h. The solvents were evaporated under reduced pressure and the residue was dissolved in CH₂Cl₂ (50 mL), filtered through a silica pad and concentrated under reduced pressure to afford crude 1-[4-(4,4,5,5-tetramethyl-1,3,2-dioxaborolan-2-yl)pyrazol-1-yl]propan-2-ol **110** as a brown solid which was used in the next step without further purification.
LCMS method F: [M+H]⁺ = 253.2, t_{R} = 1.93 min

### Preparation of intermediate 111: benzyl N-[3-[3-[1-(2-hydroxypropyl)pyrazol-4-yl]-1-tetrahydropyran-2-yl-indazol-5-ylloxypropyl]carbamate

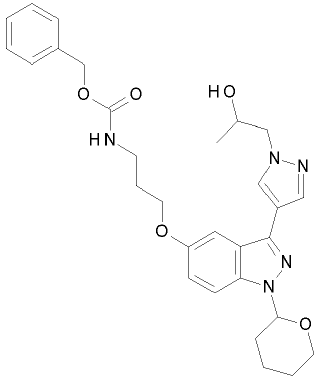

In a microwave vial, benzyl N-[3-(3-iodo-1-tetrahydropyran-2-yl-indazol-5-yl)oxypropyl]carbamate **26** (803 mg, 1.500 mmol), 1-[4-(4,4,5,5-tetramethyl-1,3,2-dioxaborolan-2-yl)pyrazol-1-yl]propan-2-ol **110** (567 mg, 2.250 mmol), XPhos (72 mg, 0.150 mmol) and K₃PO₄ (955 mg, 4.500 mmol) were suspended in dioxane (6.0 mL) and water (1.5 mL) and the mixture was degassed with N₂ for 15 minutes. Pd(PPh₃)₄ (87 mg, 0.075 mmol) was added and the resulting cloudy yellow solution was sealed and heated to 120°C under microwave conditions for 2 h. The mixture was cooled to room temperature and poured in EtOAc (25 mL) and water (25 mL) and the two layers were separated. The aqueous layer was extracted with EtOAc (3 x 25 mL) and the combined organic layers were washed with saturated aqueous NaCl (1 x 25 mL), dried over anhydrous Na₂SO₄, filtered and concentrated under reduced pressure. The resulting crude material (yellow oil, 900 mg) was purified by column chromatography (40 g Macherey Nagel SiO₂, CH₂Cl₂/MeOH 100:0 to 95:5) to afford benzyl N-[3-[3-[1-(2-hydroxypropyl)pyrazol-4-yl]-1-tetrahydropyran-2-yl-indazol-5-yl]oxypropyl]carbamate **111** as a yellow solid.
LCMS method F: [M+H]⁺ = 534.3, t_{R} = 2.66 min

### Preparation of intermediate 112: 7-methyl-19-(oxan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one

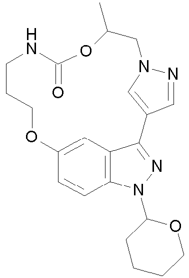

To a solution of benzyl N-[3-[3-[1-(2-hydroxypropyl)pyrazol-4-yl]-1-tetrahydropyran-2-yl-indazol-5-yl]oxypropyl]carbamate **111** (180 mg, 0.334 mmol) in MeCN (18.0 mL) was added Cs₂CO₃ (659 mg, 2.024 mmol). The resulting cloudy white solution was heated to reflux for 6 h. LC/MS analysis indicated that the reaction was complete. The mixture was cooled to room temperature, filtered and concentrated under reduced pressure. The resulting crude material (pale yellow oil, 180 mg) was purified by column chromatography (4 g Macherey Nagel SiO₂, 15 mL/min, CyH/EtOAc 100:0 to 0:100) to afford 7-methyl-19-(oxan-2-yl)-8,14-dioxa-4,5,10,19,20-pentaazatetracyclo[13 .5.2.1^{2,5}.0^{18,21}]tricosa-1(20),2(23),3,15(22),16,18(21)-hexaen-9-one **112** as a white solid.
LCMS method F: [M+H]⁺ = 426.2, t_{R} = 2.31 min

### Preparation of Example 94: 7-methyl-8,14-dioxa-4,5,10,19,20-pentaazatetracyclo [13.5.2.1^{2,5}.0^{18,21}]tricosa-1(20),2(23),3,15(22),16,18(21)-hexaen-9-one

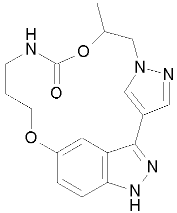

To a solution of 7-methyl-19-(oxan-2-yl)-8,14-dioxa-4,5,10,19,20-pentaazatetracyclo [13.5.2.1^{2,5}.0^{11,21}]tricosa-1(20),2(23),3,15(22),16,18(21)-hexaen-9-one **112** (43 mg, 0.101 mmol) in CH₂Cl₂ (2.5 mL) was added TFA (0.39 mL, 0.576 mg, 5.050 mmol). The resulting transparent solution was stirred at room temperature for 6 h. The solvent was removed under reduced pressure and the residue was triturated with MeCN and dried (50°C, 5 mbar) for 3 h to afford 7-methyl-8,14-dioxa-4,5,10,19,20-pentaazatetracyclo[13.5.2.1^{2,5}.0^{18,21}]tricosa-1(20),2(23),3,15(22),16,18(21)-hexaen-9-one **example 94** as a white solid.
LCMS method F: [M+H]⁺ = 342.2, t_{R} = 2.42 min
LCMS method G: [M+H]⁺ = 342.2, t_{R} = 2.39 min
¹H NMR (400 MHz, *d*6-DMSO) δ 12.81 (s, 1H), 8.14 (s, 1H), 7.86 - 7.82 (m, 1H), 7.77 (s, 1H), 7.42 (d, *J =* 9.0 Hz, 1H), 7.08 (d, *J =* 2.5 Hz, 1H), 6.94 (dd, *J =* 2.7, 9.1 Hz, 1H), 4.96 - 4.89 (m, 1H), 4.49 (dd, *J =* 2.3, 14.9 Hz, 1H), 4.42 (dd, *J =* 3.6, 12.2 Hz, 1H), 4.30 (dd, *J =* 10.2, 14.5 Hz, 1H), 4.24 - 4.16 (m, 1H), 3.53 - 3.47 (m, 1H), 2.78 - 2.71 (m, 1H), 1.97 - 1.88 (m, 1H), 1.78 - 1.68 (m, 1H), 1.31 (d, *J =* 6.3 Hz, 3H) ppm.

### Example 95: 5-fluoro-4-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-one

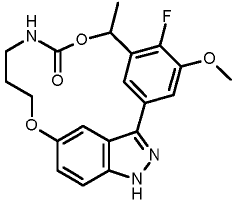

Example 95 is prepared according to the synthesis route described in general Scheme A and according to the procedures described to obtain **example 88** to give 5-fluoro-4-methoxy-7-methyl-8,14-dioxa-10,19,20-triazatetracyclo[13.5.2.1^{2,6}.0^{18,21}]tricosa-1(20),2(23),3,5,15(22), 16,18(21)-heptaen-9-one **example 95.**
LCMS method F: [M+H]⁺ = 386.2, t_{R} = 2.41 min
LCMS method G: [M+H]⁺ = 386.2, t_{R} = 2.40 min
¹H NMR (400 MHz, *d*6-DMSO) δ 13.14 (1H, br. s), 8.07 (1H, dd, *J =* 4.2, 7.8 Hz), 7.52 - 7.47 (2H, m), 7.43 - 7.40 (1H, m), 7.33 (1H, d, *J =* 2.1 Hz), 6.99 (1H, dd, *J =* 2.2, 9.0 Hz), 5.84 - 5.77 (1H, m), 4.37 - 4.27 (2H, m), 3.93 (3H, s), 3.55 - 3.48 (1H, m), 2.77 - 2.67 (1H, m), 2.15 - 2.07 (1H, m), 1.78 - 1.69 (1H, m), 1.56 - 1.53 (3H, d, *J =* 6.4 Hz) ppm.

### Example 96: (7R,13R)-7,13-dimethyl-8,14-dioxa-10,19,20-triazatetracyclo [13.5.2.1^{2,6}.0^{18,21}]tricosa-1(20),2(23),3,5,15(22),16,18(21)-heptaen-9-one

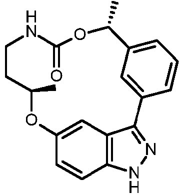

Example 96 is prepared according to the synthesis route described in general Scheme C and by chiral HPLC purification. The chiral purification is done on a Chiralpak IA column 250x4.6mm 5 µm, eluent [heptane/EtOH]+0.1%DEA [80/0], 1 mL/min RT to give (7R,13R)-7,13-dimethyl-8,14-dioxa-10,19,20-triazatetracyclo[13.5.2.1^{2,6}.0^{18,21}]tricosa-1(20),2(23),3,5,15(22), 16,18(21)-heptaen-9-one **example 96.**
LCMS method F: [M+H]⁺ = 352.2, t_{R} = 2.47 min
LCMS method G: [M+H]⁺ = 352.2, t_{R} = 2.44 min
¹H NMR (400 MHz, *d*6-DMSO) δ 12.81 - 12.77 (1H, m), 7.92 (1H, s), 7.76 (1H, d, J = 7.6 Hz), 7.53 - 7.36 (3H, m), 7.31 - 7.27 (2H, m), 6.96 (1H, dd, J = 2.3, 8.9 Hz), 5.92 (1H, s), 4.64 (1H, s), 3.26 - 3.22 (1H, m), 3.15 - 3.05 (1H, m), 2.1-1.86 (1H, m), 1.62 (4H, s), 1.41 - 1.37 (3H, m) ppm.
Chiral HPLC e.e. 100%

### Example 97: (13R)-13-methyl-8,14-dioxa-4,5,10,19,20-pentaazatetracyclo [13.5.2.1^{2,5}.0^{18,21}]tricosa-1(20),2(23),3,15,17,21-hexaen-9-one

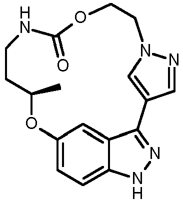

Example 97 is prepared according to the synthesis route described in general Scheme C and by chiral HPLC purification. The chiral purification is done on a Chiralpak IB N-5 column 20x250mm 5 µm, eluent [heptane/EtOH]+0.1%DEA [85/15] run time 20 min, 19 mL/min RT to give (13R)-13-methyl-8,14-dioxa-4,5,10,19,20-pentaazatetracyclo[13.5.2.1^{2,5}.0^{18,21}]tricosa-1(20),2(23),3,15,17,21-hexaen-9-one **example 97.**
LCMS method F: [M+H]⁺ = 342.3, t_{R} = 1.91 min
LCMS method G: [M+H]⁺ = 342.2, t_{R} = 1.92 min
¹H NMR (400 MHz, *d*6-DMSO) δ 12.80 (1H, s), 8.08 (1H, s), 7.77 - 7.76 (2H, m), 7.43 - 7.39 (1H, m), 7.07 (1H, d, J = 1.9 Hz), 6.92 (1H, dd, J = 2.2, 9.0 Hz), 4.68 - 4.42 (4H, m), 4.11 - 4.05 (1H, m), 3.58 - 3.46 (1H, mm), 2.94 - 2.86 (1H, m), 2.16 - 2.08 (1H, m), 1.39 - 1.36 (4H, m) ppm.
Chiral HPLC e.e. >99%

### Example 98: 8,15-dioxa-4,10,20,21-tetraazapentacyclo[14.5.2.1^{2,6}.1^{10,13}.0^{19,22}]pentacosa-1(21),2(25),3,5,16(23),17,19(22)-heptaen-9-one

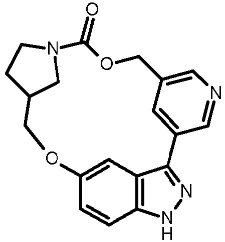

Example 98 is prepared according to the synthesis route described in general Scheme N.

### Preparation of intermediate 113: benzyl 3-(hydroxymethyl)pyrrolidine-1-carboxylate

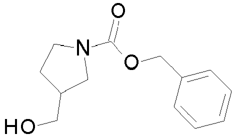

To a solution of 3-bromopropylamine hydrobromide (2.0 g, 19.8 mmol) in aq.NaOH 10 % (60 mL) at 0 °C was slowly added benzyl chlorformate (3.1 mL, 21.8 mmol) and the mixture was stirred at RT for 1 hour. The reaction mixture was diluted with DCM (100 mL). The aqueous layer was extracted two times with DCM (50mL). The combined organic layers were washed with brine (50 mL), dried over sodium sulfate, filtered and concentrated under reduced pressure to give a yellow oil. The residue was purified by flash chromatography (CyH/AE 0 to 100% EtOAc) to afford benzyl 3-(hydroxymethyl)pyrrolidine-1-carboxylate **113** as an yellow oil.
LCMS method F: [M+H]⁺ = 236, t_{R} = 1.97 min

### Preparation of intermediate 114: benzyl 3-(methylsulfonyloxymethyl)pyrrolidine-1-carboxylate

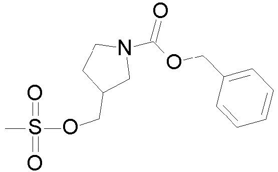

To a solution of benzyl 3-(hydroxymethyl)pyrrolidine-1-carboxylate **113** (2.90 g, 12.3 mmol) and diisopropylethylamine (4.28 mL, 24.6 mmol) in dichoromethane (20 mL) at 0 °C, was added dropwise methanesulfonyl chloride (1.13 mL, 14.8 mmol). The reaction mixture was stirred at room temperature for 4 hours. The organic layer was washed with a saturated solution of ammonium chloride (50 mL), with a saturated solution of sodium bicarbonate (50 mL) and brine, filtered and the solvent was removed under reduced pressure to give benzyl 3-(methylsulfonyloxymethyl)pyrrolidine-1-carboxylate **114** as a yellow oil. The crude product was used in the next step without further purification.
LCMS method F: [M+H]⁺ = 314, t_{R} = 2.29 min

### Preparation of intermediate 115: benzyl 3-(methylsulfonyloxymethyl)pyrrolidine-1-carboxylate

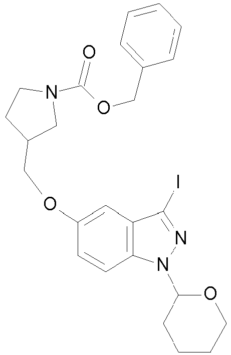

To a solution of 3-iodo-1-tetrahydropyran-2-yl-indazol-5-ol **4** (4.33 g, 12.6 mmol) in *N,N-*dimethylformamide (100 mL) was added cesium carbonate (10.27 g, 31.5 mmol). The resulting green solution was stirred at room temperature for 10 minutes. tert-butyl 3-(methylsulfonyloxymethyl)azetidine-1-carboxylate **114** (3.94 g, 12.6 mmol) was added and the mixture was stirred at 60 °C for 12 h. The mixture was cooled to room temperature and concentrated under reduced pressure. The residue was diluted with water (50 mL) and ethyl acetate (100 mL). After separation, the aqueous layer was extracted with ethyl acetate (2 x 100 mL). The combined organic layers were washed with brine, dried over anhydrous magnesium sulfate, filtered and evaporated under reduced pressure to afford a yellow oil. The oily residue was purified by flash chromatography (CyH/EtOAc 7/3) to afford benzyl 3-(methylsulfonyloxymethyl)pyrrolidine-1-carboxylate **115** as a colorless oil.
LCMS method F: [M+H]⁺ = 562, t_{R} = 3.32 min

### Preparation of intermediate 116: 5-(pyrrolidin-3-ylmethoxy)-1-tetrahydropyran-2-yl-indazole

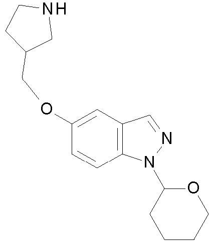

To a solution of benzyl 3-[(3-iodo-1-tetrahydropyran-2-yl-indazol-5-yl)oxymethyl]pyrrolidine-1-carboxylate **115** (4.00 g, 7.13 mmol) in MeOH (200 mL) were added triethylamine (2.4 mL) and 10 % Pd/C (75 mg). The reaction vessel was pressurized to 50 psi (approx. 3-4 bar) for 24 hours in a parr shaker. The mixture was filtered over celite. The filtrate was concentrated under reduced pressure to give 5-(pyrrolidin-3-ylmethoxy)-1-tetrahydropyran-2-yl-indazole **116** as a slightly yellow foam. The crude product was used in the next step without any further purification.
LCMS method F: [M+H]⁺ = 302.2, t_{R} = 1.43 min

### Preparation of intermediate 117: (5-bromo-3-pyridyl)methyl 3-[(1-tetrahydropyran-2-ylindazol-5-yl)oxymethyljpyrrolidine-1-carboxylate

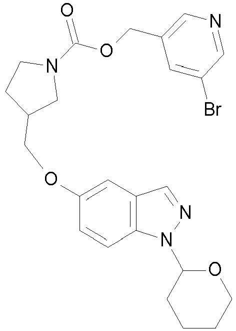

To a solution of 5-(pyrrolidin-3-ylmethoxy)-1-tetrahydropyran-2-yl-indazole **116** (0.890 g, 2.95 mmol) in dimethylacetamide (200 mL) was added 1,1'-carbonyldiimidazole (0.526 g, 3.25 mmol). The reaction mixture was stirred at room temperature for 2 hours. The reaction mixture was then added dropwise to a solution of (5-bromo-3-pyridyl)methanol (0.830 g, 4.42 mmol) and cesium carbonate (4.79 g, 14.75 mmol) in DMA (50 mL) at 90 °C and the mixture was stirred at 90°C for 16 hours. The reaction mixture was allowed to cool down to room temperature and filtered. The filtrate was diluted with water and extracted three times with ethyl acetate (3 x 100 mL). The combined organic layers were washed with water, brine, dried over anhydrous magnesium sulfate, filtered and concentrated under reduced pressure. The crude product was purified by silica gel column chromatography (eluent: Cyclohexane/EtOAc from 95/5 to 50/50) to afford (5-bromo-3-pyridyl)methyl 3-[(1-tetrahydropyran-2-ylindazol-5-yl)oxymethyl]pyrrolidine-1-carboxylate **117** as a colorless oil.
LCMS method F: [M+H]⁺ = 517.1, t_{R} = 2.81 min

### Preparation of intermediate 118: 20-(oxan-2-yl)-8,15-dioxa-4,10,20,21-tetraazapentacyclo [14.5.2.1^{2,6}.1^{10,13}.0^{19,22}]pentacosa-1(21),2(25),3,5,16(23),17,19(22)-heptaen-9-one

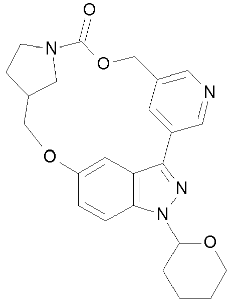

To a solution of (5-bromo-3-pyridyl)methyl 3-[(1-tetrahydropyran-2-ylindazol-5-yl)oxymethyl]pyrrolidine-1-carboxylate **117** (0.530 g, 1.03 mmol) in toluene (60 mL) was added potassium acetate (0.202 g, 2.06 mmol) at room temperature. The mixture was degassed by bubbling nitrogen for 15 minutes, then, palladium acetate (0.047 g, 0.21 mmol,) and tricyclohexylphosphine (0.059 g, 0.21 mmol) were added. The mixture was heated under microwave conditions at 150 °C for 1 hour and 30 minutes. The reaction mixture was filtered over celite, concentrated under reduced pressure, diluted with DCM, extracted with water, dried over anhydrous magnesium sulfate, filtered and concentrated under reduced pressure. The crude was purified by column chromatography on silica gel (DCM/MeOH : from 10/0 to 9/1) to afford 20-(oxan-2-yl)-8,15-dioxa-4,10,20,21-tetraazapentacyclo[14.5.2.1^{2,6}.1^{10,13}.0^{19,22}]pentacosa-1(21),2(25),3,5,16(23),17,19(22)-heptaen-9-one **118** as a colorless oil.
LCMS method F: [M+H]⁺ = 435.3, t_{R} = 2.48 min

### Preparation of Example 98: 8,15-dioxa-4,10,20,21-tetraazapentacyclo [14.5.2.1^{2,6}.1^{10,13}.0^{19,22}]pentacosa-1(21),2(25),3,5,16(23),17,19(22)-heptaen-9-one

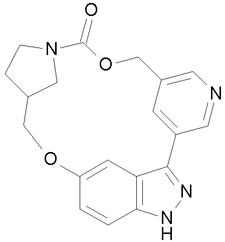

To a solution of 20-(oxan-2-yl)-8,15-dioxa-4,10,20,21-tetraazapentacyclo [14.5.2.1^{2,6}.1^{10,13}.0^{19,22}]pentacosa-1(21),2(25),3,5,16(23),17,19(22)-heptaen-9-one **118** (0.043 g, 0.10 mmol) in DCM (8 mL) was added trifluoroacetic acid (0.15 mL, 2.00 mmol). The mixture was heated under microwave conditions at 80 °C for 1 hour. More trifluoroacetic acid (0.3 mL, 4.00 mmol) was added. The mixture was heated under microwave conditions at 90 °C for 2 more hours. The solvent was evaporated under reduced pressure to give a yellow oily residue. The residue was recrystallized in DCM, filtered and dried under reduced pressure to afford 8,15-dioxa-4,10,20,21-tetraazapentacyclo[14.5.2. 1^{2,6}.1^{10,13}.0^{19,22}]pentacosa-1(21),2(25), 3,5,16(23),17,19(22)-heptaen-9-one **example 98** as a slightly brown beige solid.
LCMS method F: [M+H]⁺ = 351.2, t_{R} = 1.75 min
LCMS method G: [M+H]⁺ = 351.2, t_{R} = 1.96 min

The NMR showed a mixture of rotamers, reported as rot.1 and rot.2 in the NMR description below.
¹H NMR (400 MHz, *d*6-DMSO) δ 13.33 (0.7H, rot.1, br. s), 13.30 (0.3H, rot.2, br. s), 8.95 (0.7H, rot.1, s), 8.89 (0.3H, rot.2, s), 8.60 (0.3H, rot.2, s), 8.56 (0.7H, rot.1, s), 8.29 (0.7H, rot.1, s), 8.26 (0.3H, rot.2, s), 7.52 (0.7H, rot.1, d, J = 9.4 Hz), 7.51 (0.3H, rot.2, d, J = 9.4 Hz), 7.23 - 7.05 (2H, rot.1 + rot.2, m), 5.75 (0.7H, rot.1, d, J = 13.6 Hz), 5.67 (0.3H, rot.2, d, J = 14.0 Hz), 5.04 (0.7H, rot.1, d, J = 13.6 Hz), 5.02 (0.3H, rot.2, d, J = 13.6 Hz), 4.27 - 4.20 (1H, rot.1 + rot.2, m), 4.15 - 4.01 (1H, rot.1 + rot.2, m), 3.89 (1H, rot.1 + rot.2, t, J = 12.4 Hz), 3.72 - 3.61 (1H, rot.1 + rot.2, m), 3.57 - 3.47 (2H, rot.1 + rot.2, m), 2.80 - 2.70 (1H, rot.1 + rot.2, m), 2.16 - 1.98 (1H, rot.1 + rot.2, m), 1.74 - 1.64 (1H, rot.1 + rot.2, m) ppm.

### Example 99: 8,14-dioxa-5,10,19,20-tetraazatetracyclo[13.5.2.1^{2,5}.0^{18,21}]tricosa-1(20),2(23),3,15(22),16,18(21)-hexaen-9-one

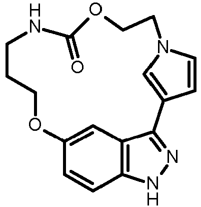

Example 99 is prepared according to the synthesis route described in general Scheme J.

### Preparation of intermediate 119: 5-(benzyloxy)-3-iodo-1-(oxan-2-yl)-1H-indazole

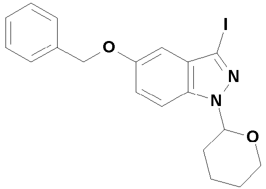

To a solution of 3-iodo-1-(oxan-2-yl)-1H-indazol-5-ol (3.442 g, 10.0 mmol) in acetonitrile (100 mL) were added at RT cesium carbonate (4.235 g, 13.0 mmol) and benzyl bromide (1.308 mL, 11.0 mmol). The resulting reaction mixture was stirred at RT overnight. The reaction mixture was concentrated under reduced pressure. The residue was diluted with water and brine and extracted twice with ethyl acetate. The combined organic layers were dried over anhydrous sodium sulfate and the solvent was removed under reduced pressure. The residue was triturated in acetonitrile and filtered affording 5-(benzyloxy)-3-iodo-1-(oxan-2-yl)-1H-indazole **119** as a white solid.
LCMS method F: [M+H]⁺ = 435.1, t_{R} = 3.33 min

### Preparation of intermediate 120: 5-(benzyloxy)-1-(oxan-2-yl)-3-(1H-pyrrol-3-yl)-1H-indazole

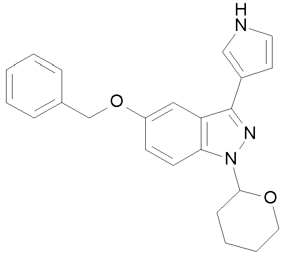

To a solution of 5-(benzyloxy)-3-iodo-1-(oxan-2-yl)-1H-indazole **119** (1.000 g, 2.3 mmol) in dioxane (6.9 mL) and water (2.3 mL) was added at RT 3-(4,4,5,5-tetramethyl-1,3,2-dioxaborolan-2-yl)-1H-pyrrole (0.534 g, 2.76 mmol), K₃PO₄ (1.466 g, 6.91 mmol), XPhos (0.110 g, 0.23 mmol) and Pd(PPh₃)₄ (0.133 g, 0.12 mmol). The resulting reaction mixture was stirred under microwave conditions at 120°C for 1h. The residue was diluted with brine and extracted twice with ethyl acetate. The combined organic layers were dried over anhydrous sodium sulfate and concentrated under reduced pressure. The residue was purified by flash-column (25g silica Macherey Nagel) chromatography (cyclohexane - ethyl acetate, 1:0 to 6:4) affording 5-(benzyloxy)-1-(oxan-2-yl)-3-(1H-pyrrol-3-yl)-1H-indazole **120** as a yellow oil.
LCMS method F: [M+H]⁺ = 374.2, t_{R} = 2.95 min

### Preparation of intermediate 121: 5-(benzyloxy)-3-(1-{2-[(tert-butyldimethylsilyl)oxy]ethyl}-1H-pyrrol-3-yl)-1-(oxan-2-yl)-1H-indazole

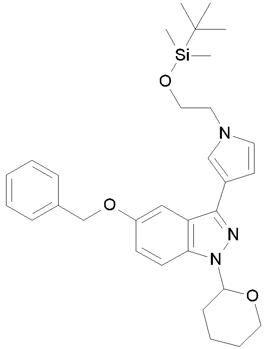

To a solution of 5-(benzyloxy)-1-(oxan-2-yl)-3-(1H-pyrrol-3-yl)-1H-indazole **120** (0.740 g, 1.98 mmol) in *N,N*-dimethylformamide (8 mL) at 0°C was added portion wise NaH (0.119 g, 2.97 mmol). After 20 min (2-bromoethoxy)(tert-butyl)dimethylsilane (0.850 mL, 3.96 mmol) in *N,N*-dimethylformamide (2 mL) was added dropwise at 0°C. The resulting reaction mixture was stirred at 0°C for 10 min and at RT for 2h. The reaction mixture was quenched by addition of MeOH and it was concentrated under reduced pressure. The residue was diluted with brine and extracted twice with ethyl acetate. The combined organic layers were dried over anhydrous sodium sulfate and concentrated under reduced pressure. The residue was purified by flash-column (25g silica Macherey Nagel) chromatography (cyclohexane - ethyl acetate, 1:0 to 9:1) affording 5-(benzyloxy)-3-(1-{2-[(tert-butyldimethylsilyl)oxy]ethyl}-1H-pyrrol-3-yl)-1-(oxan-2-yl)-1H-indazole **121** as a yellow oil.
LCMS method F: [M+H]⁺ = 532.4, t_{R} = 3.80 min

### Preparation of intermediate 122: 3-(1-{2-[(tert-butyldimethylsilyl)oxy]ethyl}-1H-pyrrol-3-yl)-1-(oxan-2-yl)-1H-indazol-5-ol

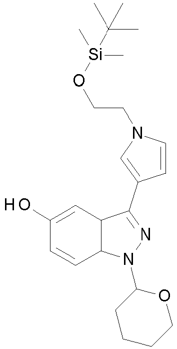

To a solution of 5-(benzyloxy)-3-(1-{2-[(tert-butyldimethylsilyl)oxy]ethyl}-1H-pyrrol-3-yl)-1-(oxan-2-yl)-1H-indazole **121** (0.900 g, 1.69 mmol) in EtOH (15 mL) was added at RT palladium 10% on carbon (90 mg). The reaction mixture was stirred under hydrogen atmosphere at RT overnight. The reaction mixture was filtered and the filtrate was concentrated under reduced pressure. The residue was purified by flash-column (25g silica Macherey Nagel) chromatography (cyclohexane - ethyl acetate, 1:0 to 8:2) affording 3-(1-{2-[(tertbutyldimethylsilyl)oxy]ethyl}-1H-pyrrol-3-yl)-1-(oxan-2-yl)-1H-indazol-5-ol **122** as a colorless oil.
LCMS method F: [M+H]⁺ = 442.2, t_{R} = 3.27 min

### Preparation of intermediate 123: benzyl N-(3-{[3-(1-{2-[(tert-butyldimethylsilyl)oxy]ethyl}-1H-pyrrol-3-yl)-1-(oxan-2-yl)-1H-indazol-5-yl]oxy}propyl)carbamate

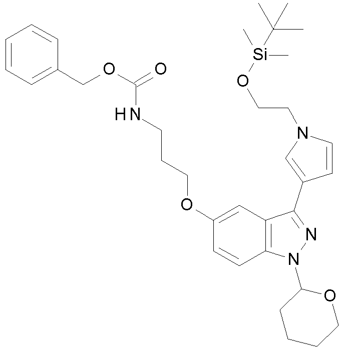

To a solution of 3-(1-{2-[(tert-butyldimethylsilyl)oxy]ethyl}-1H-pyrrol-3-yl)-1-(oxan-2-yl)-1H-indazol-5-ol **122** (0.310 g, 0.70 mmol) in acetonitrile (5 mL) were added at RT cesium carbonate (0.297 g, 0.91 mmol) and benzyl N-(3-bromopropyl)carbamate (0.150 mL, 0.77 mmol). The resulting reaction mixture was stirred at RT overnight. The reaction mixture was concentrated under reduced pressure. The residue was diluted with water and brine and extracted with ethyl acetate twice. The combined organic layers were dried over anhydrous sodium sulfate and concentrated under reduced pressure affording benzyl N-(3-{[3-(1-{2-[(tertbutyldimethylsilyl)oxy]ethyl}-1H-pyrrol-3-yl)-1-(oxan-2-yl)-1H-indazol-5-yloxy}propyl) carbamate **123** as a yellow oil. The product was used in the next step without further purification.
LCMS method F: [M+H]⁺ = 633.3, t_{R} = 3.64 min

### Preparation of intermediate 124: benzyl N-[3-({3-[1-(2-hydroxyethyl)-1H-pyrrol-3-yl]-1-(oxan-2-yl)-1H-indazol-5-yl}oxy)propyl]carbamate

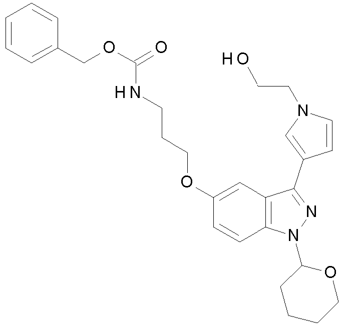

To a solution of benzyl N-(3-{[3-(1-{2-[(tert-butyldimethylsilyl)oxy]ethyl }-1H-pyrrol-3-yl)-1-(oxan-2-yl)-1H-indazol-5-yl]oxy}propyl)carbamate **123** (0.444 g, 0.70 mmol) in THF (5 mL) was added at RT tetrabutylammonium fluoride 1M in THF (1.4 mL, 1.40 mmol). The resulting reaction mixture was stirred at RT for 2h. The reaction mixture was diluted with brine and extracted with ethyl acetate twice. The combined organic layers were dried over anhydrous sodium sulfate and concentrated under reduced pressure. The residue was purified by flash-column (15g silica Macherey Nagel) chromatography (cyclohexane - ethyl acetate 3 / EtOH 1, 1:0 to 7:3) affording benzyl N-[3-({3-[1-(2-hydroxyethyl)-1H-pyrrol-3-yl]-1-(oxan-2-yl)-1H-indazol-5-yl }oxy)propyl]carbamate **124** as a colorless oil.
LCMS method F: [M+H]⁺ = 519.2, t_{R} = 2.72 min

### Preparation of intermediate 125: 19-(oxan-2-yl)-8,14-dioxa-5,10,19,20-tetraazatetracyclo [13.5.2.1^{2,5}.0^{18,21}]tricosa-1(20),2(23),3,15(22), 16,18(21)-hexaen-9-one

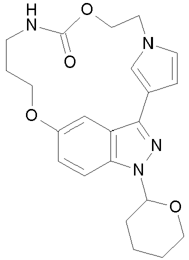

To a solution of benzyl N-[3-({3-[1-(2-hydroxyethyl)-1H-pyrrol-3-yl]-1-(oxan-2-yl)-1H-indazol-5-yl}oxy)propyl]carbamate **124** (0.230 g, 0.44 mmol) in anhydrous acetonitrile (88 mL) was added at RT cesium carbonate (0.867 g, 2.66 mmol). The resulting reaction mixture was stirred at 90°C for 48h. The reaction mixture was filtered and the filtrate was concentrated under reduced pressure. The residue was purified by flash-column (15g silica Macherey Nagel) chromatography (cyclohexane - ethyl acetate 3 / EtOH 1, 1:0 to 8:2) affording 19-(oxan-2-yl)-8,14-dioxa-5,10,19,20-tetraazatetracyclo[13.5.2.1^{2,5}.0^{18,21}]tricosa-1(20),2(23),3,15(22), 16,18(21)-hexaen-9-one **125** as a colorless oil.
LCMS method F: [M+H]⁺ = 411.2, t_{R} = 2.42 min

### Preparation of Example 99: 8,14-dioxa-5,10,19,20-tetraazatetracyclo[13.5.2.1^{2,5}.0^{18,21}] tricosa-1(20),2(23),3,15(22),16,18(21)-hexaen-9-one

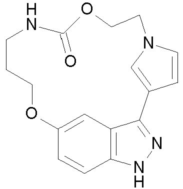

To a solution of 19-(oxan-2-yl)-8,14-dioxa-5,10,19,20-tetraazatetracyclo[13.5.2.1^{2,5}.0^{18,21}] tricosa-1(20),2(23),3,15(22),16,18(21)-hexaen-9-one **125** (0.100 g, 0.24 mmol) in DCM (3 mL) was added at RT TFA (0.363 mL, 4.87 mmol). The resulting reaction mixture was stirred under microwave conditions at 80°C for 20 min. The reaction mixture was concentrated under reduced pressure, diluted with a saturated sodium bicarbonate solution and extracted with ethyl acetate twice. The combined organic layers were dried over anhydrous sodium sulfate and concentrated under reduced pressure. The residue was purified by flash-column (15g silica Macherey Nagel) chromatography (cyclohexane - ethyl acetate 3 / EtOH 1, 1:0 to 7:3) to give the product with some impurities. The product was purified twice by flash-column (2*4g silica Macherey Nagel) chromatography (DCM - MeOH, 1:0 to 98:2) to give a solid (10 mg), which was triturated in diisopropyl ether and filtered affording 8,14-dioxa-5,10,19,20-tetraazatetracyclo [13.5.2.1^{2,5}.0^{18,21}]tricosa-1(20),2(23),3,15(22),16,18(21)-hexaen-9-one **example 99** as a cream solid.
LCMS method F: [M+H]⁺ = 327.2, t_{R} = 1.82 min
LCMS method G: [M+H]⁺ = 327.3, t_{R} = 1.90 min
¹¹H NMR (400 MHz, *d*6-DMSO) δ 12.56 (1H, s); 7.90 - 7.86 (1H, m), 7.39 - 7.36 (1H, m), 7.26 - 7.24 (1H, m), 7.15 (1H, d, J = 1.9 Hz), 6.92 - 6.87 (2H, m), 6.39 (1H, dd, J = 1.7, 2.5 Hz), 4.35 - 4.23 (6H, m), 3.18 - 3.11 (2H, m), 1.92 - 1.83 (2H, m) ppm.

### Example 100: (13R) or (13S)-4-fluoro-13-methyl-8,14-dioxa-10,19,20-triazatetracyclo [13.5.2.1^{2,6}.0^{18,21}]tricosa-1(20),2(23),3,5,15(22),16,18(21)-heptaen-9-one

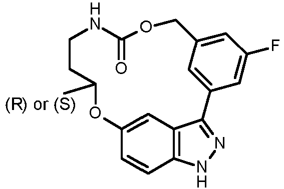

Example 100 is prepared according to the synthesis route described in general Scheme C and by chiral SFC separation of **example 71** to give (13R) or (13 S)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one **example 100.**
LCMS method F: [M+H]⁺ = 356.2, t_{R} = 2.46 min
LCMS method G: [M+H]⁺ = 356.2, t_{R} = 2.46 min
¹H NMR (400 MHz, d6-DMSO) δ 13.26 (1H, s), 7.99 (1H, dd, J = 5.0, 7.3 Hz), 7.67 (1H, s), 7.61 - 7.50 (2H, m), 7.26 (1H, d, J = 1.7 Hz), 7.17 (1H, d, J=9.5 Hz), 6.98 (1H, dd, J = 2.3, 8.9 Hz), 5.73 (1H, s), 4.89 - 4.83 (1H, m), 4.61 - 4.54 (1H, m), 3.61 - 3.58 (1H, m), 2.95 - 2.87 (1H, m), 2.40 - 2.33 (1H, m), 1.42 - 1.39 (4H, m) ppm.
Chiral HPLC e.e. >98%

The compound is a pure enantiomer, but the absolute stereochemistry of the chiral center is unknown.

### Example 101: (13R) or (13S)-4-fluoro-13-methyl-8,14-dioxa-10,19,20-triazatetracyclo [13.5.2.1^{2,6}.0^{18,21}] tricosa-1(20),2(23),3,5,15(22),16,18(21)-heptaen-9-one

Example 101 is prepared according to the synthesis route described in general Scheme C and by chiral SFC separation of **example 71** to give (13R) or (13 S)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one **example 101.** 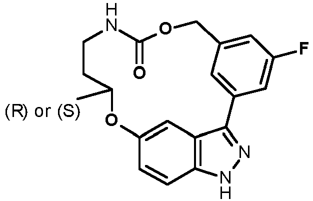
LCMS method F: [M+H]⁺ = 356.2, t_{R} = 2.47 min
LCMS method G: [M+H]⁺ = 356.2, t_{R} = 2.46 min
¹H NMR (400 MHz, *d*6-DMSO) δ 13.28 - 13.26 (1H, m), 7.99 (1H, dd, J = 5.2, 6.7 Hz), 7.67 (1H, s), 7.61 - 7.50 (2H, m), 7.26 (1H, d, J = 1.9 Hz), 7.19 - 7.15 (1H, m), 6.98 (1H, dd, J = 2.3, 8.9 Hz), 5.74 - 5.70 (1H, m), 4.89-4.79 (1H, m), 4.61 - 4.53 (1H, m), 3.59 (1H, s), 2.95 - 2.86 (1H, m), 2.40 - 2.33 (1H, m), 1.42 - 1.39 (4H, m) ppm.
Chiral HPLC e.e. >98%
The compound is a pure enantiomer, but the absolute stereochemistry of the chiral center is unknown

### Example 102: (13R)-13-methyl-8,14-dioxa-4,10,19,20-tetraazatetracyclo[13.5.2.1^{2,6}.0^{18,21}] tricosa-1(20),2(23),3,5,15(22),16,18(21)-heptaen-9-one

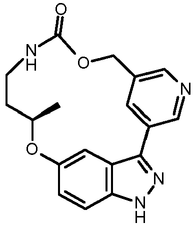

Example 102 is prepared according to the synthesis route described in general Scheme C and by chiral HPLC purification. The chiral purification is done on a Chiralpak IB N-5 column 250x4.6mm 5 µm, eluent [C7/EtOH]+0.1%DEA [80/20], 1 mL/min RT to give (13R)-13-methyl-8,14-dioxa-4,10,19,20-tetraazatetracyclo[13.5.2.1^{2,6}.0^{18,21}]tricosa-1(20),2(23),3,5, 15(22),16,18(21)-heptaen-9-one **example 102.**
LCMS method F: [M+H]⁺ = 339, t_{R} = 1.80 min
LCMS method G: [M+H]⁺ = 339, t_{R} = 2.05 min
¹H NMR (400 MHz, *d*6-DMSO) δ 13.32 (1H, s), 9.04 (1H, d), 8.53 (1H, d), 8.15 (1H, m), 8.02 - 7.99 (1H, m), 7.54 (1H, d), 7.19 (1H, m), 7.01 - 6.98 (1H, dd, J = 2.2, 9.0 Hz), 5.77 - 5.74 (1H, m), 4.95 - 4.92 (1H, m), 4.59 - 4.52 (1H, m), 3.60 - 3.53 (1H, m), 2.95 - 2.87 (1H, m), 2.45 - 2.38 (1H, m), 1.41 (3H, d), 1.38 - 1.34 (1H, m) ppm.
Chiral HPLC e.e. >99%

### Example 103: 6-cyclopropyl-8,14-dioxa-4,5,10,19,20-pentaazatetracyclo[13.5.2.1^{2,5}.0^{18,21}] tricosa-1(20),2(23),3,15(22),16,18(21)-hexaen-9-one

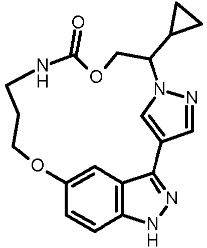

Example 103 is prepared according to the synthesis route described in general Scheme B.

### Preparation of intermediate 126: ethyl 2-(4-bromopyrazol-1-yl)-2-cyclopropyl-acetate

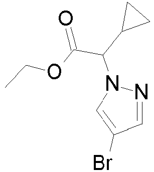

To a solution of 4-bromo-1H-pyrazole (588 mg, 4 mmol) in *N,N*-dimethylformamide (2 mL) were added ethyl 2-bromo-2-cyclopropyl-acetate (1 g, 4.8 mmol), and potassium carbonate (1.11 g, 8 mmol). The mixture was stirred for 4 hours at 80 °C. The reaction was quenched with water (15 mL) and the resulting solution was extracted with EtOAc (20 mL x 3). The combined organic layers were washed with brine, dried over anhydrous sodium sulfate, filtered, and concentrated under reduced pressure to give ethyl 2-(4-bromopyrazol-1-yl)-2-cyclopropyl-acetate **126** as a yellow liquid. The crude was used in the next step without any purification.
LCMS method F: [M+H]⁺ = 274, t_{R} = 2.53 min

### Preparation of intermediate 127: ethyl 2-[4-[5-[tert-butyl(dimethyl)silyl]oxy-1-tetrahydropyran-2-yl-indazol-3-yl[pyrazol-1-yl]-2-cyclopropyl-acetate

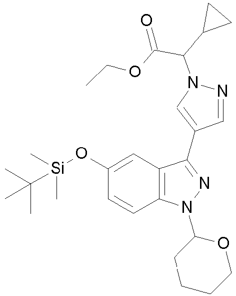

To a solution of [5-[tert-butyl(dimethyl)silyl]oxy-1-tetrahydropyran-2-yl-indazol-3-yl]boronic acid **18** (1.63 g, 4.35 mmol), ethyl 2-(4-bromopyrazol-1-yl)-2-cyclopropyl-acetate **126** (700 mg, 2.56 mmol), tripotassium phosphate (1.63 g, 7.68 mmol) in dioxane (7.7 mL) and water (2.6 mL) were added XPhos (122 mg, 0.25 mmol) and tetrakis(triphenylphosphine)palladium(0) (147 mg, 0.13 mmol). The reaction mixture was heated at 100°C under microwave conditions for 1 hour and 30 minutes. The reaction mixture was filtered over celite and the celite was washed with EtOAc. The filtrate was then diluted with water and extracted with EtOAc (3 x). The combined organic layers were washed with brine, dried over anhydrous sodium sulfate and concentrated under reduced pressure. The crude product was purified by column (Macherey Nagel, 40 g) chromatography with cyclohexane/EtOAc (100/0 to 80/20) as eluent. The desired fractions were combined and evaporated under reduced pressure to give ethyl 2-[4-[5-[tert-butyl(dimethyl)silyl]oxy-1-tetrahydropyran-2-yl-indazol-3-yl]pyrazol-1-yl]-2-cyclopropyl-acetate **127** as a yellow oil.
LCMS method F: [M+H]⁺ = 525, t_{R} = 3.76 min

### Preparation of intermediate 128: ethyl 2-cyclopropyl-2-[4-(5-hydroxy-1-tetrahydropyran-2-yl-indazol-3-yl)pyrazol-1-yljacetate

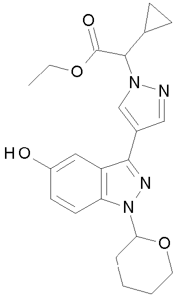

To a solution of ethyl 2-[4-[5-[tert-butyl(dimethyl)silyl]oxy-1-tetrahydropyran-2-yl-indazol-3-yl]pyrazol-1-yl]-2-cyclopropyl-acetate **127** (238 mg, 0.45 mmol) in THF (1.8 mL) was added dropwise at room temperature tetrabutylammonium fluoride 1M in THF (0.5 mL, 0.5 mmol). The resulting reaction mixture was stirred at room temperature overnight. The reaction mixture was poured into ice water and stirred for 20 min. The aqueous phase was extracted twice with EtOAc and the combined organic layers were washed with brine, dried over anhydrous sodium sulfate and concentrated under reduced pressure to give ethyl 2-cyclopropyl-2-[4-(5-hydroxy-1-tetrahydropyran-2-yl-indazol-3-yl)pyrazol-1-yl]acetate **128** as an orange solid.
LCMS method F: [M+H]⁺ = 411, t_{R} = 2.53 min

### Preparation of intermediate 129: ethyl 2-[4-[5-[3-(benzyloxycarbonylamino)propoxy]-1-tetrahydropyran-2-yl-indazol-3-yl[pyrazol-1-yl]-2-cyclopropyl-acetate

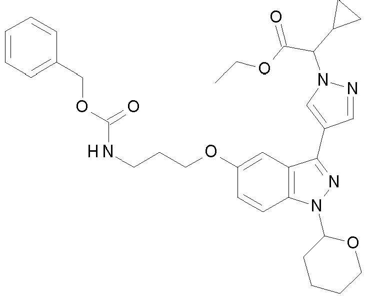

To a solution of ethyl 2-cyclopropyl-2-[4-(5-hydroxy-1-tetrahydropyran-2-yl-indazol-3-yl)pyrazol-1-yl]acetate **128** (232 mg, 0.57 mmol,) in acetonitrile (6 mL), were added cesium carbonate (370 mg, 1.14 mmol) and benzyl N-(3-bromopropyl)carbamate (169 mg, 0.62 mmol). The mixture was stirred at room temperature overnight. The reaction mixture was filtered and rinsed with EtOAc. Water was added and the water layer was extracted with EtOAc (3 x). The combined organic layers were washed with brine, dried over sodium sulfate, filtered and concentrated under reduced pressure to afford ethyl 2-[4-[5-[3-(benzyloxycarbonyl amino)propoxy]-1-tetrahydropyran-2-yl-indazol-3-yl]pyrazol-1-yl]-2-cyclopropyl-acetate **129** as a brown oil.
LCMS method F: [M+H]⁺ = 602, t_{R} = 3.18 min

### Preparation of intermediate 130: benzyl N-[3-[3-[1-(1-cyclopropyl-2-hydroxy-ethyl)pyrazol-4-yl]-1-tetrahydropyran-2-yl-indazol-5-ylloxypropyl]carbamate

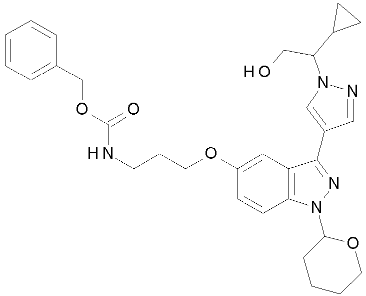

A solution of ethyl 2-[4-[5-[3-(benzyloxycarbonylamino)propoxy]-1-tetrahydropyran-2-yl-indazol-3-yl]pyrazol-1-yl]-2-cyclopropyl-acetate **129** (324 mg, 0.54 mmol) in THF (2.3 mL) was degassed with N₂ during 10 minutes, LAH 1 M in THF (0.65 mL, 0.65 mmol) was added at 0°C and the reaction was stirred at 0°C for 2 hours and 30 minutes. The mixture was quenched with water (0.2 mL), 10% NaOH (0.2 mL) and water (0.2 mL). The mixture was filtered with EtOAc as eluent. The filtrate was diluted with water and extracted with EtOAc (3 x). The combined organic layers were washed with brine, dried with anhydrous sodium sulfate and the solvent was removed under reduced pressure to afford benzyl N-[3-[3-[1-(1-cyclopropyl-2-hydroxy-ethyl)pyrazol-4-yl]-1-tetrahydropyran-2-yl-indazol-5-yl]oxypropyl]carbamate **130** as a colorless oil.
LCMS method F: [M+H]⁺ = 560, t_{R} = 2.76 min

### Preparation of intermediate 131: 6-cyclopropyl-19-(oxan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one

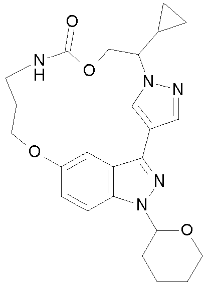

A suspension of benzyl N-[3-[3-[1-(1-cyclopropyl-2-hydroxy-ethyl)pyrazol-4-yl]-1-tetrahydropyran-2-yl-indazol-5-yl]oxypropyl]carbamate **130** (112 mg, 0.2 mmol) and cesium carbonate (390 mg, 1.2 mmol) in acetonitrile (44 mL) was heated to 80°C for 5 hours. The reaction mixture was filtered at 80°C, cooled to room temperature and concentrated under reduced pressure. The crude was purified by column (Macherey Nagel, 15 g) chromatography with DCM/MeOH (100/0 to 97/3) as eluent. The desired fractions were combined and the solvent was removed under reduced pressure to give 6-cyclopropyl-19-(oxan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one **131** as a colorless solid.
LCMS method F: [M+H]⁺ = 452, t_{R} = 2.47 min

### Preparation of Example 103: 6-cyclopropyl-8,14-dioxa-4,5,10,19,20-pentaazatetracyclo [13.5.2.1^{2,5}.0^{18,21}]tricosa-1(20),2(23),3,15(22), 16,18(21)-hexaen-9-one

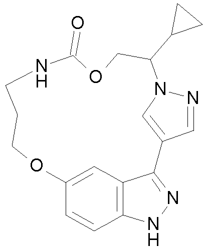

To a solution of 6-cyclopropyl-19-(oxan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one **131** (46 mg, 0.1 mmol) in DCM (11 mL) was added trifluoro acetic acid (0.16 mL, 2.03 mmol). The mixture was heated at 80°C under microwave conditions for 1 hour. The reaction mixture was diluted with DCM (25 mL) and a saturated sodium bicarbonate solution (25 mL). After separation, the aqueous layer was extracted with DCM (3 x 20 mL). The combined organic layers were washed with brine (25 mL), dried over anhydrous sodium sulfate, filtered and concentrated under reduced pressure. The crude was purified by column (Macherey Nagel, 15 g) chromatography with DCM/EtOAc (100/0 to 25/75) as eluent. The desired fractions were combined and the solvent was removed under reduced pressure to afford 6-cyclopropyl-8,14-dioxa-4,5,10,19,20-pentaazatetracyclo[13.5.2.1^{2,5}.0^{18,21}]tricosa-1(20),2(23),3,15(22), 16,18(21)-hexaen-9-one **example 103** as a white solid.
LCMS method F: [M+H]⁺ = 368, t_{R} = 2.02 min
LCMS method G: [M+H]⁺ = 368, t_{R} = 2.03 min
¹H NMR (400 MHz, *d*6-DMSO) δ 12.80 (1H, s), 8.17 (1H, s), 7.82 (1H, dd, J = 4.6, 7.3 Hz), 7.75 (1H, s), 7.42 (1H, d, J = 9 Hz), 7.09 (1H, d, J = 2.3 Hz), 6.94 (1H, dd, J = 2.3, 8.9 Hz), 4.55 (1H, dd, J = 2.5, 11.6 Hz), 4.40 (1H, m), 4.23 (2H, m), 3.90 (1H, m), 2.89 (1H, m), 1.85 (2H, m), 1.58 (1H, m), 0.67 (1H, m), 0.55 (2H, m), 0.44 (1H, m) ppm. One proton was located under the residual water peak and was not reported here.

### Example 104: 7-ethyl-8,14-dioxa-10,19,20-triazatetracyclo[13.5.2.1^{2,6}.0^{18,21}]tricosa-1(20),2,4,6(23),15,17,21-heptaen-9-one

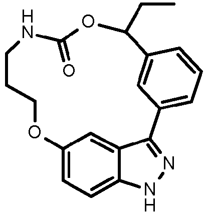

Example 104 is prepared according to the synthesis route described in general Scheme G.

### Preparation of intermediate 132: 1-[3-(4,4,5,5-tetramethyl-1,3,2-dioxaborolan-2-yl)phenyl] propan-1-one

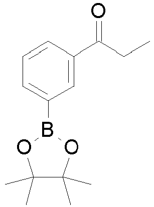

To a mixture of 1-(3-bromophenyl)propan-1-one (2 g, 9.4 mmol) in dioxane (30 mL) was added 4,4,5,5-tetramethyl-2-(tetramethyl-1,3,2-dioxaborolan-2-yl)-1,3,2-dioxaborolane (2.63 g, 10.3 mmol), Pd₂dba₃ (431 mg, 0.471 mmol), KOAc (1.48 g, 15 mmol) and tricyclohexylphosphine (264 mg, 0.94 mmol). The reaction mixture was stirred at 100°C for 2 h. The solvent was removed under reduced pressure,then it was dissolved in EtOAc and washed with water (x3). The organic layer was filtered on a Guanidine(SPE) pad. Then the solvant was removed under reduced pressure to give 1-[3-(4,4,5,5-tetramethyl-1,3,2-dioxaborolan-2-yl)phenyl]propan-1-one **132** as a yellow oil.
LCMS method F: [M+H]⁺= 261, t_{R} = 2.99 min

### Preparation of example 104: 7-ethyl-8,14-dioxa-10,19,20-triazatetracyclo[13.5.2.1^{2,6}.0^{18,21}] tricosa-1(20),2,4,6(23),15,17,21-heptaen-9-one

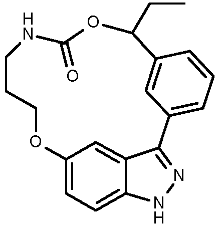

To a solution of 7-ethyl-19-(oxan-2-yl)-8,14-dioxa-10,19,20-triazatetracyclo [13.5.2.1^{2,6}.0^{18,21}] tricosa-1(20),2(23),3,5,15(22),16,18(21)-heptaen-9-one **124** (220 mg, 0.51 mmol) in DCM (10 mL) was added trifluoroacetic acid (775 µL, 10.11 mmol). Stirred at 50°C during 2h. The reaction mixture was diluted with EtOAc (30 mL) and water (20 mL) After separation, the aqueous layer was extracted with EtOAc (3 x 10 mL). The combined organic layers were washed with saturated sodium carbonate aqueous solution (30 mL) and brine (30 mL). The organic layer was dried over sodium sulfate anhydrous, filtered off and concentrated to dryness. The oil was triturated in DCM and the solid was filtered and dried under reduced pressure to give 7-ethyl-8,14-dioxa-10,19,20-triazatetracyclo[13.5.2.1^{2,6}.0^{18,21}]tricosa-1(20),2,4,6(23),15, 17,21-heptaen-9-one **104** as a white powder.
LCMS method F: [M+H]⁺ = 352.2, t_{R} = 2.41 min (current 20V)
LCMS method G: [M+H]⁺ = 352.2, t_{R} = 2.38 min (pH10 current 20V)
¹H NMR (400 MHz, *d*6-DMSO) δ 7.96 (1H, dd, J = 4.7, 7.6 Hz), 7.85 - 7.80 (2H, m), 7.50 - 7.45 (2H, m), 7.37 - 7.28 (2H, m), 6.99 (1H, dd, J = 2.3, 8.9 Hz), 5.68 (1H, dd, J = 3.6, 8.5 Hz),4.41 - 4.21 (2H, m), 3.56 - 3.49 (1H, m), 2.78 - 2.67 (1H, m), 2.23 - 2.15 (1H, m), 2.10 - 2.01 (1H, m), 1.89 - 1.69 (2H, m), 1.00 (3H, t, J = 7.3 Hz) ppm.

### Example 105: (13R)-13-methyl-8,14-dioxa-5,10,19,20,23-pentaazatetracyclo [13.5.2.1^{2,6}.0^{18,21}]tricosa-1(20),2,4,6(23),15,17,21-heptaen-9-one

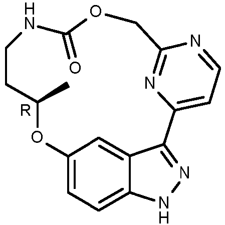

Example 105 is prepared according to the synthesis route described in general Scheme B.

To a solution of (13R)-13-methyl-19-(oxan-2-yl)-8,14-dioxa-5,10,19,20,23-pentaazatetracyclo[13.5.2.1^{2,6}.0^{18,21}]tricosa-1(20),2,4,6(23),15,17,21-heptaen-9-one (100 mg, 0.24 mmol) in DCM (2 mL) was added trifluoroacetic acid (362 µL, 4.73 mmol). The mixture was heated under microwaves irradiation at 80 °C for 45 min. The reaction mixture was diluted with DCM (25 mL) and saturated NaHCO₃ (25 mL). A yellow precipitate was appeared and filtered to afford, after dried under vacuum at 60 °C for 12 hours (13R)-13-methyl-8,14-dioxa-5,10,19,20,23-pentaazatetracyclo[13.5.2.1^{2,6}.0^{18,21}]tricosa-1(20),2,4,6(23),15,17,21-heptaen-9-one **example 105** as a yellow solid.
LCMS method F: [M+H]⁺ = 340, t_{R} = 2.00 min
LCMS method G: [M+H]⁺ = 340, t_{R} = 2.02 min
¹H NMR (400 MHz, *d*6-DMSO) δ 13.68 - 13.67 (1H, m), 8.77 - 8.74 (1H, m), 8.05 (1H, d, J = 5.3 Hz), 7.92 - 7.87 (2H, m), 7.55 - 7.52 (1H, m), 7.00 (1H, dd, J = 2.3, 9.1 Hz), 5.61 - 5.55 (1H, m), 5.03 - 4.97 (1H, m), 4.61 (1H, t, J = 6.8 Hz), 3.50 (1H, m), 2.94 - 2.86 (1H, m), 2.38 - 2.31 (1H, m), 1.42 - 1.38 (4H, m) ppm.

### Example 106: (7R,13R)-4-fluoro-7,13-dimethyl-8,14-dioxa-10,19,20-triazatetracyclo [13.5.2.1^{2,6}.0^{18,21}]tricosa-1(20),2,4,6(23),15,17,21-heptaen-9-one

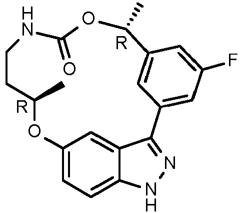

Example 106 is prepared according to the synthesis route described in general Scheme C.

### Preparation of intermediate 133: (1R)-1-[3-fluoro-5-(4,4,5,5-tetramethyl-1,3,2-dioxaborolan-2-yl)phenyl]ethan-1-ol

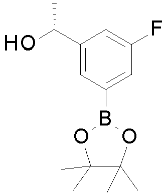

To a degassed solution of (1R)-1-(3-bromo-5-fluorophenyl)ethan-1-ol (0.500 g, 2.28 mmol) in dioxane (3 mL) was added at RT bis(pinacolato)diboron (0.695 g, 2.74 mmol), KOAc (0.672 g, 6.85 mmol) and PdCl₂(dppf)·DCM (0.093 g, 0.11 mmol). The resulting reaction mixture was stirred under microwave irradiation at 100°C for 2 h. The residue was filtered on celite, diluted with water and extracted with ethyl acetate twice. The combined organic layer was dried over anhydrous sodium sulfate and concentrated in vacuo affording (1R)-1-[3-fluoro-5-(4,4,5,5-tetramethyl-1,3,2-dioxaborolan-2-yl)phenyl]ethan-1-ol **133** as a black oil, used without further purification in the next step.
LCMS method F: [M-H₂O+H]⁺= 249.1, t_{R} = 2.68 min

### Preparation of Example 106: (7R,13R)-4-fluoro-7,13-dimethyl-8,14-dioxa-10,19,20-triazatetracyclo[13.5.2.1^{2,6}.0^{18,21}]tricosa-1(20),2,4,6(23),15,17,21-heptaen-9-one

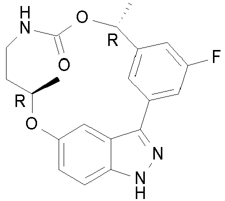

To a solution of (7R,13R)-4-fluoro-7,13-dimethyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one (0.530 g, 1.17 mmol) in DCM (10 mL) was added at RT TFA (1.740 mL, 23.37 mmol). The resulting reaction mixture was stirred under microwave irradiation at 80°C for 1 h. The reaction mixture was concentrated in vacuo, diluted with saturated sodium bicarbonate solution and extracted with ethyl acetate twice. The combined organic layer was dried over anhydrous sodium sulfate and concentrated in vacuo. The residue was purified by flash-column (15g silica Macherey Nagel) chromatography (cyclohexane - ethyl acetate 3 / EtOH 1, 1:0 to 6:4) to give a solid (0.380 g), which was triturated in acetonitrile and filtered affording (7R,13R)-4-fluoro-7,13-dimethyl-8,14-dioxa-10,19,20-triazatetracyclo[13.5.2.1^{2,6}.0^{18,21}]tricosa-1(20),2,4,6(23),15,17,21-heptaen-9-one **example 106** as a white solid.
LCMS method F: [M+H]⁺ = 370.1, [M-H]- = 368.3, t_{R} = 2.51 min
LCMS method G: [M+H]⁺ = 370.2, [M-H]- = 368.4, t_{R} = 2.58 min
¹H NMR (400 MHz, *d*6-DMSO, 80°C) δ 13.22 - 13.20 (1H, m), 7.78 (1H, dd, J = 4.6, 8.0 Hz), 7.65 (1H, d, J = 0.8 Hz), 7.52 - 7.44 (2H, m), 7.23 - 7.19 (2H, m), 7.00 - 6.96 (1H, m), 5.88 (1H, q, J = 6.6 Hz), 4.65 - 4.57 (1H, m), 3.24 - 3.17 (1H, m), 3.10 - 3.04 (1H, m), 1.83 (1H, dd, J = 8.4, 13.1 Hz), 1.72 - 1.64 (1H, m), 1.62 (3H, d, J = 6.6 Hz), 1.38 - 1.35 (3H, m) ppm.

### Example 107: 7-methyl-8,14-dioxa-4,10,19,20-tetraazatetracyclo[13.5.2.1^{2,6}.0^{18,21}]tricosa-1(20),2,4,6(23),15,17,21-heptaen-9-one

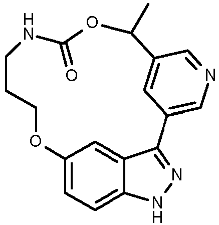

Example 107 is prepared according to the synthesis route described in general Scheme H.

To a solution of 7-methyl-19-(oxan-2-yl)-8,14-dioxa-4,10,19,20-tetraazatetracyclo [13.5.2.1^{2,6}.0^{18,21}]tricosa-1(20),2,4,6(23),15,17,21-heptaen-9-one (32 mg, 0.076 mmol, 1 eq) in DCM (1.8 mL) was added trifluoroacetic acid (233 µL, 3.040 mmol, 40 eq) at RT. The solution was heated under microwave conditions at 80°C for 2 h 40 min. The reaction mixture (brown solution) was evaporated under vacuo, the brown residue was dissolved in EtOAc then a saturated aqueous solution of sodium hydrogen carbonate was added. After separation, the aqueous layer was extracted with ethyl acetate (2 x). The combined organic layer was washed with water then brine, dried over sodium sulfate, filtered and evaporated under reduced pressure to give an orange oil. The crude product was purified by column chromatography eluting with Cyclohexane / Ethyl acetate - EtOH (3-1), 100/0 to 60/40 to give the expected product. It was triturated from diisopropyl ether and directly transferred into the brown vial (without filtration due to the low mass), dried under vacuo to give 7-methyl-8,14-dioxa-4,10,19,20-tetraazatetracyclo[13.5.2.1^{2,6}.0^{18,21}]tricosa-1(20),2,4,6(23),15,17,21-heptaen-9-one **example 107** as a white solid.
LCMS method F: [M+H]⁺ = 339, t_{R} = 1.69 min
LCMS method G: [M+H]⁺ = 339, t_{R} = 1.98 min
¹H NMR (400 MHz, *d*6-DMSO) δ 13.33 (1H, s), 9.03 (1H, d), 8.57 (1H, d), 8.15 (1H, m), 8.04 - 8.01 (1H, m), 7.55 - 7.53 (1H, d), 7.28 (1H, m), 7.03 - 7.01 (1H, dd), 5.98 - 5.93 (1H, q), 4.37 - 4.27 (2H, m), 3.55 - 3.48 (1H, m), 2.80 - 2.71 (1H, m), 2.23 - 2.14 (1H, m), 1.78 - 1.70 (1H, m), 1.65 (3H, d) ppm.

### Example 108: (7R)- or (7S)-4-fluoro-7-methyl-8,14-dioxa-10,19,20-triazatetracyclo [13.5.2.1^{2,6}.0^{18,21}]tricosa-1(20),2,4,6(23),15,17,21-heptaen-9-one

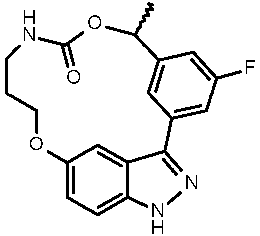

Example 108 is prepared according to the synthesis route described in general Scheme A and by chiral SFC separation to give (7R)- or (7S)-4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one **example 108.**
LCMS method F: [M+H]+ = 356, t_{R} = 2.39 min
LCMS method G: [M+H]⁺ = 356, t_{R} = 2.42 min
¹H NMR (400 MHz, *d*6-DMSO) δ 13.28 (1H, s), 8.01 - 7.97 (1H, m), 7.69 (1H, s), 7.59 - 7.56 (1H, m), 7.53 - 7.50 (1H, m), 7.33 (1H, d), 7.21 - 7.18 (1H, m), 7.02 - 6.99 (1H, dd), 5.91 - 5.86 (1H, q), 4.37 - 4.26 (2H, m), 3.55 - 3.49 (1H, m), 2.78 - 2.71 (1H, m), 2.23 - 2.14 (1H, m), 1.78 - 1.70 (1H, m), 1.59 (3H, d) ppm.
Chiral HPLC : ee > 97.5 %

The compound is a pure enantiomer, but the absolute stereochemistry of the chiral center is unknown.

### Example 109: (7R)- or (7S)-4-fluoro-7-methyl-8,14-dioxa-10,19,20-triazatetracyclo [13.5.2.1^{2,6}.0^{18,21}]tricosa-1(20),2,4,6(23),15,17,21-heptaen-9-one

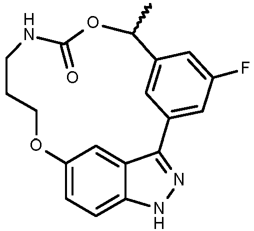

Example 109 is prepared according to the synthesis route described in general Scheme A and by chiral SFC separation to give (7R)- or (7S)-4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one **example 109.**
LCMS method F: [M+H]⁺ = 356, t_{R} = 2.39 min
LCMS method G: [M+H]⁺ = 356, t_{R} = 2.41 min
¹H NMR (400 MHz, *d*6-DMSO) δ 13.27 (1H, s), 8.01 - 7.98 (1H, m), 7.69 (1H, s), 7.59 - 7.56 (1H, m), 7.53 - 7.51 (1H, m), 7.33 (1H, d), 7.21 - 7.18 (1H, m), 7.01 - 6.99 (1H, dd), 5.91 - 5.86 (1H, q), 4.37 - 4.24 (2H, m), 3.56 - 3.49 (1H, m), 2.78 - 2.71 (1H, m), 2.23 - 2.14 (1H, m), 1.78 - 1.70 (1H, m), 1.59 (3H, d) ppm.
Chiral HPLC : ee > 98.0 %

The compound is a pure enantiomer, but the absolute stereochemistry of the chiral center is unknown.

### Example 110: 6-methyl-8,14-dioxa-4,5,10,19,20-pentaazatetracyclo[13.5.2.1^{2,5}.0^{18,21}] tricosa-1(20),2(23),3,15,17,21-hexaen-9-one

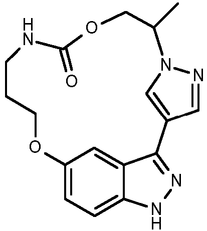

Example 110 is prepared according to the synthesis route described in general Scheme B.

### Preparation of intermediate 134: ethyl 2-(4-bromopyrazol-1-yl)propanoate

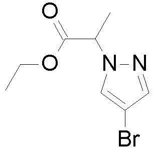

A suspension of 4-bromo-1H-pyrazole (2.00 g, 13.60 mmol), ethyl 2-bromopropanoate (2.1 mL, 16.32 mmol), and potassium carbonate (3.78 g, 27.20 mmol) in N,N-dimethylformamide (8.0 mL) was stirred for 3 h at 80 °C. The reaction was quenched by water (30 mL), and the resulting solution was extracted twice with ethyl acetate (50 mL x 2). The combined organic layers were dried over anhydrous magnesium sulfate, filtered, and concentrated under reduced pressure to give ethyl 2-(4-bromopyrazol-1-yl)propanoate **134** as a yellow oil.
Yield: 4.58 g of intermediate 134 (quantitative)
LCMS method F: [M+H]⁺ = 249, t_{R} = 2.29 min

### Preparation of Example 110: 6-methyl-8,14-dioxa-4,5,10,19,20-pentaazatetracyclo [13.5.2.1^{2,5}.0^{18,21}]tricosa-1(20),2(23),3,15,17,21-hexaen-9-one

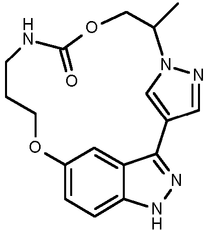

To a solution of 6-methyl-19-(oxan-2-yl)-8,14-dioxa-4,5,10,19,20-pentaazatetracyclo [13.5.2.1^{2,5}.0^{11,21}]tricosa-1(20),2(23),3,15,17,21-hexaen-9-one (68 mg, 0.16 mmol) in DCM (2 mL) was added trifluoro acetic acid (245 µL, 3.20 mmol). The mixture was heated in microwaves at 80 °C for 30 min. The reaction mixture was diluted with DCM (25 mL) and NaHCO₃ saturated (25 mL). After separation, aqueous layer was extracted with DCM (3 x 20 mL). The combined organic layer was washed with brine (25 mL), dried over anhydrous sodium sulfate, filtered and evaporated under reduced pressure. The crude was purified by flash chromatography (DCM/MeOH 95/5) to afford 6-methyl-8,14-dioxa-4,5,10,19,20-pentaazatetracyclo[13.5.2.1^{2,5}.0^{18,21}]tricosa-1(20),2(23),3,15,17,21-hexaen-9-one **example 110** as an off-white solid.
LCMS method F: [M+H]⁺ = 342, t_{R} = 1.86 min
LCMS method G: [M+H]⁺ = 342, t_{R} = 1.89 min
¹H NMR (400 MHz, *d*6-DMSO) δ 12.80 - 12.79 (1H, m), 8.07 (1H, s), 7.83 - 7.76 (2H, m), 7.43 - 7.40 (1H, m), 7.06 (1H, d, J = 2.1 Hz), 6.94 (1H, dd, J = 2.3, 8.9 Hz), 4.82 - 4.75 (1H, m), 4.44 - 4.15 (4H, m), 3.23 (1H, t, J = 7.1 Hz), 3.03 - 2.98 (1H, m), 1.91 - 1.82 (2H, m), 1.59 (3H, d, J = 7.0 Hz) ppm.

### Example 111: 7-methyl-8,14-dioxa-10,19,20,23-tetraazatetracyclo[13.5.2.1^{2,6}.0^{18,21}] tricosa-1(20),2,4,6(23),15,17,21-heptaen-9-one

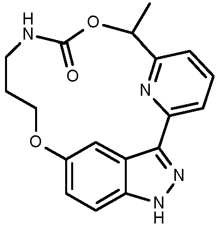

Example 111 is prepared according to the synthesis route described in general Scheme H using SEM as indazole protecting group.

### Preparation of intermediate 135: 1-(6-bromo-2-pyridyl)ethanol

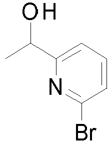

To a cooled solution of 6-bromopyridine-2-carbaldehyde (2.0 g, 10.81 mmol) in dry tetrahydrofuran (28 mL) was dropwise added methylmagnesium bromide solution 3 M in diethyl ether (7.2 mL, 21.62 mmol) at 0 °C. The reaction mixture was stirred at 0 °C for 20 min then allowed to reach room temperature for 16 hours. TLC analysis showed total consumption of starting material. The reaction mixture was quenched with a saturated aqueous solution of NH₄Cl then extracted with ethyl acetate (2 x 100 mL). The organic layer was washed with water the brine, dried over magnesium sulfate, filtered and concentrated under reduced pressure. The crude product was purified by flash-column chromatography eluting with Cyclohexane / Ethyl acetate - EtOH (3-1): 100 / 0 to 80 / 20, to give 1-(6-bromo-2-pyridyl)ethanol **135** as a colorless oil.
LCMS method F: [M+H]⁺ = 202.0, t_{R} = 1.65 min

### Preparation of Example 111: 7-methyl-8,14-dioxa-10,19,20,23-tetraazatetracyclo [13.5.2.1^{2,6}.0^{18,21}]tricosa-1(20),2,4,6(23),15,17,21-heptaen-9-one

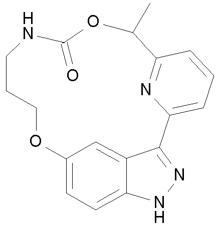

To a solution of 7-methyl-20-{[2-(trimethylsilyl)ethoxy]methyl}-8,14-dioxa-10,19,20,23-tetraazatetracyclo[13.5.2.1^{2,6}.0^{18,21}]tricosa-1(21),2(23),3,5,15(22),16,18-heptaen-9-one (0.037 g, 0.079 mmol) in dichloroethane (2.0 mL) was added an aqueous solution of HCl (6 N, 0.15 mL, 0.95 mmol). The mixture was heated at 70 °C for 16 hours. The mixture was concentrated under reduced pressure and 1,4-dioxane (2.0 mL) and NH₄OH solution (0.4 mL) were added. The mixture was heated at 75 °C for 3 hours, concentrated under reduced pressure and the residue was suspended in MeOH (0.4 mL) and filtered. The crude product was purified with two successive preparative TLC on silica gel (first using (DCM/MeOH : 95/5) as an eluent, then using Cyclohexane / EtOAc-EtOH : 4 / 6 (3-1)) to afford 7-methyl-8,14-dioxa-10,19,20,23-tetraazatetracyclo[13.5.2.1^{2,6}.0^{18,21}]tricosa-1(20),2,4,6(23),15,17,21-heptaen-9-one **example 111** as a white solid.
LCMS method F: [M+H]⁺ = 339.2, t_{R} = 2.02 min
LCMS method G: [M+H]⁺ = 339.3, t_{R} = 2.07 min
¹H NMR (400 MHz, *d*6-DMSO) δ 13.27 (1H, s), 8.04 (1H, d, *J =* 8.4 Hz), 7.88 - 7.84 (2H, m), 7.71 (1H, dd, *J =* 4.5, 7.7 Hz), 7.47 (1H, d, *J =* 8.0 Hz), 7.33 (1H, d, *J =* 7.2 Hz), 6.97 (1H, dd, *J =* 2.8, 9.6 Hz), 5.93 (1H, q, *J =* 6.8 Hz), 4.37 - 4.24 (2H, m), 3.52 - 3.40 (1H, m), 2.78 - 2.67 (1H, m), 2.22 - 2.12 (1H, m), 1.64 - 1.61 (4H, m) ppm.

### Example 112: 6-(propan-2-yl)-8,14-dioxa-4,5,10,19,20-pentaazatetracyclo [13.5.2.1^{2,5}.0^{18,21}]tricosa-1(20),2(23),3,15(22),16,18(21)-hexaen-9-one

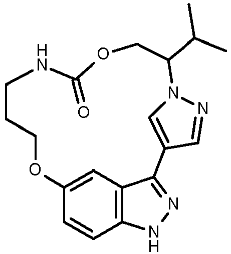

Example 112 is prepared according to the synthesis route described in general Scheme B.

### Preparation of intermediate 136: ethyl 2-(4-bromopyrazol-1-yl)-3-methyl-butanoate

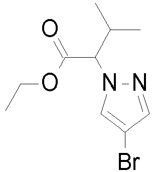

A suspension of 4-Bromo-1H-pyrazole (2.00 g, 13.60 mmol), ethyl 2-bromo-3-methylbutanoate (2.5 mL, 16.32 mmol), and potassium carbonate (3.78 g, 27.20 mmol) in *N,N-*dimethylformamide (4.0 mL) was stirred for 3 h at 80°C. The reaction was quenched by water (30 mL), and the resulting solution was extracted twice with ethyl acetate (50 mL x 2). The combined organic layers were dried over anhydrous magnesium sulfate, filtered, and concentrated under reduced pressure to give ethyl 2-(4-bromopyrazol-1-yl)-3-methyl-butanoate **136** as a colorless oil.
LCMS method F: [M+H]⁺ = 277.1, t_{R} = 2.72 min

### Preparation of Example 112: 6-(propan-2-yl)-8,14-dioxa-4,5,10,19,20-pentaaza tetracyclo[13.5.2.1^{2,5}.0^{18,21}]tricosa-1(20),2(23),3,15(22),16,18(21)-hexaen-9-one

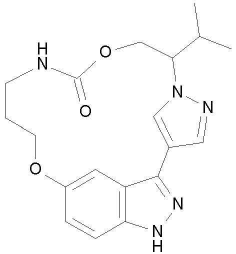

To a solution of 19-(oxan-2-yl)-6-(propan-2-yl)-8,14-dioxa-4,5,10,19,20-pentaazatetracyclo [13.5.2.1^{2,5}.0^{18,21}]tricosa-1(20),2(23),3,15(22),16,18(21)-hexaen-9-one (0.409 g, 0.90 mmol) in DCM (22 mL) was added trifluoroacetic acid (1.37 mL, 18.0 mmol). The reaction mixture was directly heated to 50 °C for 3 hours. LCMS analysis showed formation of the expected product and total consumption of starting material. The reaction mixture (brown solution) was evaporated under reduced pressure. The brown residue was dissolved in EtOAc then a saturated aqueous solution of sodium hydrogen carbonate was added. After separation, the aqueous layer was extracted with ethyl acetate (2 x). The combined organic layer was washed with water then brine, dried over sodium sulfate, filtered and evaporated under reduced pressure to give an orange oil. The crude product was purified by column chromatography eluting with Cyclohexane / Ethyl acetate - EtOH (3-1), 100/0 to 70/30. The pure fractions were combined and evaporated under reduced pressure. After evaporation of residual solvent, the product turned out to be less soluble in DCM than before, allowing for recrystallization trials in this solvent. In order to do so, the solid (approx. 160 mg) was stirred in DCM (5 mL) for several minutes, while heating the suspension up to 40 °C. After cooling down to room temperature, the solid was isolated by filtration, then recovered and further dried under high vaccum at 60°C to afford 6-(propan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one **example 112** as a white solid.
LCMS method F: [M+H]⁺ = 370.3, t_{R} = 2.05 min
LCMS method G: [M+H]⁺ = 370.3, t_{R} = 2.12 min
¹H NMR (400 MHz, *d*6-DMSO) δ 12.80 (1H, s), 8.17 (1H, s), 7.76 - 7.75 (2H, m), 7.43 - 7.40 (1H, m), 7.09 (1H, d, *J =* 1.9 Hz), 6.94 (1H, dd, *J =* 2.3, 8.9 Hz), 4.53 (1H, dd, *J =* 1.6, 11.2 Hz), 4.43 (1H, dt, *J =* 4.4, 12 Hz), 4.29 - 4.19 (3H, m), 3.44 - 3.35 (1H, m), 3.30 (1H, s), 2.88 - 2.81 (1H, m), 1.97- 1.85 (1H, m), 1.80 - 1.75 (1H, m), 1.10 (3H, d, *J =* 6.6 Hz), 0.78 (3H, d, *J* = 6.6 Hz) ppm.

### Example 113: (13R)-7,13-dimethyl-8,14-dioxa-4,5,10,19,20-pentaazatetracyclo [13.5.2.1^{2,5}.0^{18,21}]tricosa-1(20),2(23),3,15(22),16,18(21)-hexaen-9-one

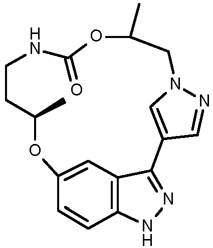

Example 113 is prepared according to the synthesis route described in general Scheme C.

### Preparation of intermediate 137: 1-[4-(4,4,5,5-tetramethyl-1,3,2-dioxaborolan-2-yl)pyrazol-1-yl]propan-2-ol

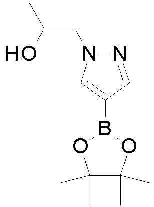

In a scelled round bottom flask, NaH (60% in mineral oil, 0.990 g, 41.24 mmol) was suspended in *N,N*-dimethylformamide (100.0 mL) and a solution of 4-(4,4,5,5-tetramethyl-1,3,2-dioxaborolan-2-yl)-1H-pyrazole (4.00 g, 20.62 mmol) was added. The reaction was stirred at room temperature for 15 minutes, then *rac*-propylene oxide (4.33 mL, 2.40 g, 41.24 mmol) was added. The round bottom flask containing the resulting cloudy brown solution was sealed and heated to 50°C for 4 h. LC/MS analysis indicated that the reaction was completed. Solvents were evaporated under reduced pressure to afford crude 1-[4-(4,4,5,5-tetramethyl-1,3,2-dioxaborolan-2-yl)pyrazol-1-yl]propan-2-ol **137** as a brown solid which was used in the next step without further purification.
LCMS method F: [M+H]⁺ = 253.2, t_{R} = 1.92 min

### Preparation of Example 113: (13R)-7,13-dimethyl-8,14-dioxa-4,5,10,19,20-pentaazatetra cyclo[13.5.2.1^{2,5}.0^{18,21}]tricosa-1(20),2(23), 3,15(22),16,18(21)-hexaen-9-one

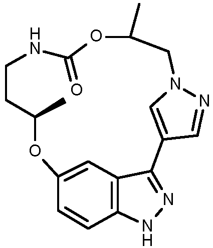

To a solution of (13R)-7,13-dimethyl-19-(oxan-2-yl)-8,14-dioxa-4,5,10,19,20-pentaazatetra cyclo[13.5.2.1^{2,5}.0^{18,21}]tricosa-1(20),2(23),3,15(22),16,18(21)-hexaen-9-one (0.090 g, 0.183 mmol) in DCM (3.0 mL) was added trifluoroacetic acid (140 µL, 1.83 mmol). The reaction mixture was stirred at room temperature during 4 h. LCMS analysis showed formation of the expected product. A saturated solution of NaHCO₃ (10 mL) was added and phases were separated. The aqueous phase was extracted with DCM (3 x 10 mL), and organic phase was washed with brine (10 mL), dried with MgSO₄, filtered and concentrated under reduce pressure. The crude (120 mg) was purified by chromatography column (Macherey Nagel, 4g, DCM/MeOH : 100/0 to 90/10). Solvents was evaporated to afford (13R)-7,13-dimethyl-8,14-dioxa-4,5,10,19,20-pentaazatetracyclo[13.5.2.1^{2,5}.0^{18,21}]tricosa-1(20),2(23), 3,15(22),16,18(21)-hexaen-9-one **example 113** as a white powder.
LCMS method F: [M+H]⁺ = 356.3, t_{R} = 1.96 and 2.02 min, diastereomers
LCMS method G: [M+H]⁺ = 356.3, t_{R} = 2.03 and 2.08 min, diastereomers
¹H NMR (400 MHz, *d*6-DMSO) (mixture of both diastereomers) δ 12.79 (s, 1H), 8.13 - 8.12 (2H, m), 7.84 (dd, J = 3.3, 8.8 Hz, 1H), 7.74 (dd, J = 0.7, 16.4 Hz, 2H), 7.61 - 7.56 (1H, m), 7.43 - 7.39 (2H, m), 7.08 (d, J = 2.5 Hz, 1H), 7.00 (d, J = 2.1 Hz, 1H), 6.94 - 6.90 (2H, m), 5.12 - 5.04 (1H, m), 4.91 - 4.86 (1H, m), 4.72 - 4.64 (1H, m), 4.50 - 4.43 (2H, m), 4.38 - 4.25 (2H, m), 3.61 - 3.51 (2H, m), 3.25 - 3.18 (1H, m), 2.86 (1H, t, J = 11.0 Hz), 2.08 (2H, t, J = 12.1 Hz), 1.86 (1H, s), 1.62 - 1.52 (1H, m), 1.39 - 1.35 (9H, m), 1.32 - 1.28 (3H, m) ppm .

### Example 114: (13R)-13-methyl-8,14-dioxa-10,19,20,23-tetraazatetracyclo [13.5.2.1^{2,6}.0^{18,21}]tricosa-1(20),2,4,6(23),15,17,21-heptaen-9-one

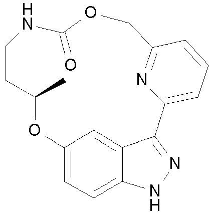

Example 114 is prepared according to the synthesis route described in general Scheme K.

In 2 µwaves tubes were put a solution of (13R)-13-methyl-19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one (300 mg, 0.71 mmol) in DCM (10 mL). To this solution was added trifluoroacetic acid (4.3 mL, 56.84 mmol). The reaction mixture was stirred at 80 °C under microwaves irradiations for 1 h. The solvant was removed under reduced pressure, the oil was dissolved in EtOAc and washed with a saturated solution of NaHCO₃. The phase were separated and the organic one was dried with Na₂SO₄. The solvant was removed under reduced pressure. The crude was purified by chromatography using a 10 g SiO₂ column eluted with cyclohexane/Ethyl acetate 70/30 to 50/50. The good fractions were combined and the solvant was removed under reduced pressure. The oil was triturated in DCM and the solid formed was filtered and dried under reduced pressure to give (13R)-13-methyl-8,14-dioxa-10,19,20,23-tetraazatetracyclo [13.5.2.1^{2,6}.0^{18,21}]tricosa-1(20),2,4,6(23),15,17,21-heptaen-9-one example 114 as a light yellow powder.
LCMS method F: [M+H]⁺ = 339.3, t_{R} = 2.17 min
LCMS method G: [M+H]⁺ = 339.3, t_{R} = 2.16 min
¹H NMR (400 MHz, *d*6-DMSO) δ 13.24 - 13.21 (1H, m), 8.10 - 8.06 (1H, m), 7.90 - 7.80 (2H, m), 7.75 - 7.71 (1H, m), 7.48 - 7.45 (1H, m), 7.26 - 7.23 (1H, m), 6.96 (1H, dd, J = 2.3, 8.9 Hz), 5.59 (1H, d, J = 14.0 Hz), 5.06 (1H, d, J = 14.6 Hz), 4.66 - 4.59 (1H, m), 3.5 - 3.42 (1H, m), 2.97 - 2.89 (1H, m), 2.29 - 2.22 (1H, m), 1.39 - 1.35 (4H, m) ppm.

### Example 115: (7R)- or (7S)-7-ethyl-8,14-dioxa-10,19,20-triazatetracyclo[13.5.2.1^{2,6}.0^{18,21}] tricosa-1(20),2,4,6(23),15,17,21-heptaen-9-one

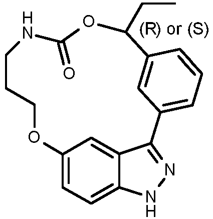

Example 115 is prepared according to the synthesis route described in general Scheme G and by chiral HPLC separation of **example 104** using Chiralpak IB N-5 20x250mm 5 µm [C7/EtOH]+0.1%DEA [80/20] run 20 min, 19 mL/min RT to give (7R)- or (7S)-7-ethyl-8,14-dioxa-10,19,20-triazatetracyclo[13.5.2.1^{2,6}.0^{18,21}]tricosa-1(20),2,4,6(23),15,17,21-heptaen-9-one **example 115.**
LCMS method F: [M+H]⁺ = 352.3, t_{R} = 2.42 min
LCMS method G: [M+H]⁺ = 352.3, t_{R} = 2.41 min
¹H NMR (400 MHz, *d*6-DMSO) δ 7.96 (1H, dd, J = 5.0, 7.7 Hz), 7.85 - 7.80 (2H, m), 7.52 - 7.45 (2H, m), 7.37 - 7.27 (2H, m), 6.98 (1H, dd, J = 2.3, 8.9 Hz), 5.68 (1H, dd, J = 3.5, 8.8 Hz), 4.38 - 4.24 (2H, m), 3.58 - 3.466 (1H, m), 2.78 - 2.67 (1H, m), 2.22 - 2.16 (1H, m), 2.10 - 2.02 (1H, m), 1.87 - 1.72 (2H, m), 1.08 - 0.98 (3H, m) ppm. The indazole NH proton was not visible in this solvent.
Chiral HPLC: ee 100%
The compound is a pure enantiomer, but the absolute stereochemistry of the chiral center is unknown.

### Example 116: (7R)- or (7S)-7-ethyl-8,14-dioxa-10,19,20-triazatetracyclo[13.5.2.1^{2,6}.0^{18,21}] tricosa-1(20),2,4,6(23),15,17,21-heptaen-9-one

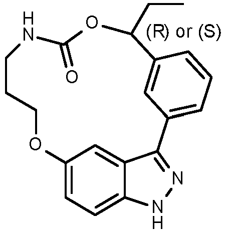

Example 116 is prepared according to the synthesis route described in general Scheme G and by chiral HPLC separation of example 104 using Chiralpak IB N-5 20x250mm 5 µm [C7/EtOH]+0.1%DEA [80/20] run 20min, 19 mL/min RT to give (7R)- or (7S)-7-ethyl-8,14-dioxa-10,19,20-triazatetracyclo[13.5.2.1^{2,6}.0^{18,21}]tricosa-1(20),2,4,6(23),15,17,21-heptaen-9-one **example 116.**
LCMS method F: [M+H]⁺ = 352.2, t_{R} = 2.42 min
LCMS method G: [M+H]⁺ = 352.3, t_{R} = 2.41 min
¹H NMR (400 MHz, *d*6-DMSO) δ 13.12 (1H, s), 7.96 (1H, dd, J = 4.7, 7.8 Hz), 7.85 - 7.80 (2H, m), 7.51 - 7.45 (2H, m), 7.37 - 7.28 (2H, m), 6.99 (1H, dd, J = 2.1, 8.9 Hz), 5.68 (1H, dd, J = 3.6, 8.7 Hz), 4.39 - 4.24 (2H, m), 3.56 - 3.50 (1H, m), 2.78 - 2.67 (1H, m), 2.22 - 2.16 (1H, m), 2.10 - 2.01 (1H, m), 1.87 - 1.72 (2H, m), 1.00 (3H, t, J = 7.2 Hz) ppm.
Chiral HPLC: ee 100%

The compound is a pure enantiomer, but the absolute stereochemistry of the chiral center is unknown.

### Example 117: (13R)-13-methyl-8,14-dioxa-5,10,19,20-tetraazatetracyclo [13.5.2.1^{2,6}.0^{18,21}]tricosa-1(20),2,4,6(23),15,17,21-heptaen-9-one

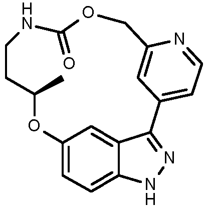

Example 117 is prepared according to the synthesis route described in general Scheme K.

In a round bottom flask was added (13R)-13-methyl-19-(oxan-2-yl)-8,14-dioxa-5,10,19,20-tetraazatetracyclo[13.5.2.1^{2,6}.0^{18,21}]tricosa-1(20),2,4,6(23),15,17,21-heptaen-9-one (0.280 g, 0.663 mmol) in DCM (10.0 mL) and trifluoroacetic acid (2.02 mL, 26.52 mmol). The reaction mixture was stirred at 80 °C under microwaves irradiations during 2 h. LC/MS analysis indicated the reaction was completed. The reaction was quenched with a saturated solution of NaHCO₃ (10 mL). Phases were separated and the aqueous phase was extracted with DCM (3 x 10 mL). Organic phase was washed with a saturated solution of NaCl (10 mL), dried with MgSO₄, filtered and concentrated under reduce pressure to afford crude (0.250 g) which was purified by chromatography column (Macherey Nagel 4g, DCM/MeOH : 100/0 to 90/10). Solvents was removed and the powder was triturated with DCM, to afford (13R)-13-methyl-8,14-dioxa-5,10,19,20-tetraazatetracyclo[13.5.2.1^{2,6}.0^{18,21}]tricosa-1(20),2,4,6(23),15,17,21 - heptaen-9-one **example 117** as a yellow powder.
LCMS method F: [M+H]⁺ = 339.2, t_{R} = 1.83 min
LCMS method G: [M+H]⁺ = 339.2, t_{R} = 2.07 min
¹H NMR (400 MHz, *d*6-DMSO) δ 13.57- 13.48 (1H, s), 8.59 (1H, d, J = 5.3 Hz), 8.14 (1H, s), 7.86 - 7.81 (2H, m), 7.58 - 7.55 (1H, m), 7.37 (1H, d, J = 1.9 Hz), 7.02 (1H, dd, J = 2.2, 9.0 Hz), 5.72 - 5.56 (1H, m), 4.99 - 4.85 (1H, m), 4.62 (1H, dd, J = 6.1, 11.2 Hz), 3.00 - 2.88 (1H, m), 2.70 - 2.66 (1H, m), 2.35 - 2.32 (1H, m), 1.43 (3H, d, J = 5.9 Hz), 1.06 (1H, t, J = 7.0 Hz) ppm.

### Example 118: 6-(oxan-4-yl)-8,14-dioxa-4,5,10,19,20-pentaazatetracyclo[13.5.2.1^{2,5}.0^{18,21}] tricosa-1(20),2(23),3,15,17,21-hexaen-9-one

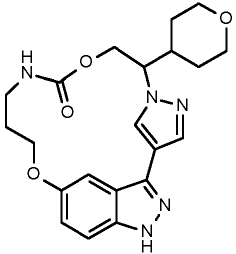

Example 118 is prepared according to the synthesis route described in general Scheme B.

### Preparation of intermediate 138: ethyl 2-tetrahydropyran-4-ylideneacetate

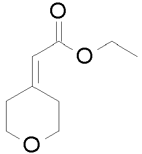

Sodium hydride (60 % dispersion in mineral oil, 0.88 g, 22.0 mmol) was suspended in THF (120 mL) and cooled to 0-5 °C in an ice bath. A solution of triethyl phosphonoacetate (4.4 mL, 22.0 mmol) in THF (120 mL) was added dropwise. The mixture was stirred at room temperature for 30 min and a solution of tetrahydro-4H-pyran-4-one (1.9 mL, 20.0 mmol) in THF (120 mL) was added dropwise. The resulting mixture was stirred for additional 6 hours at room temperature, cooled to 0-5 °C and water (500 mL) was added. The mixture was extracted with diethylether (3 x 250 mL), the combined organic layers were dried over anhydrous magnesium sulfate, filtered and concentrated under reduced pressure to afford ethyl 2-tetrahydropyran-4-ylideneacetate **138** as a colorless oil.
LCMS method F: [M+H]⁺ = 171.1, t_{R} = 2.08 min

### Preparation of intermediate 139: ethyl 2-tetrahydropyran-4-ylacetate

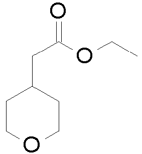

To a solution of ethyl 2-tetrahydropyran-4-ylideneacetate **138** (3.40 g, 20.0 mmol) in MeOH (130 mL) under argon atmosphere, was added 10 % Pd/C (0.40 g, 0.40 mmol). The mixture was hydrogenated under a hydrogen atmosphere for 4 hours. The suspension was filtered through a pad of Celite, rinsed with MeOH (100 mL) and the filtrate was evaporated under reduced pressure to give ethyl 2-tetrahydropyran-4-ylacetate **139** as a colorless oil. The crude product was used in the next step without any purification.

### Preparation of intermediate 140: ethyl 2-bromo-2-tetrahydropyran-4-yl-acetate

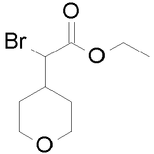

To a solution of lithium diisopropylamide (1.8 M in THF/heptane/ethylbenzene, 10.6 mL, 19.1 mmol) in 30 mL of THF at - 78 °C was added trimethylsilyl chloride (4.2 mL, 33.1 mmol) dropwise. Ethyl 2-tetrahydropyran-4-ylacetate **139** (3.000 g, 17.4 mmol) in 15 mL of THF was then added to the mixture dropwise very slowly. The mixture was stirred at -78°C for 2 hours then *N*-bromosuccinimide (3.258 g, 18.3 mmol) in 30 mL of THF was added dropwise very slowly. The reaction mixture was allowed to warm slowly to room temperature and was stirred for 16 hours. TLC showed complete reaction. The mixture was concentrated under reduced pressure and the residue was dissolved in 120 mL of ethyl acetate, washed 1 x 30 mL of water. The aqueous layer was extracted 3 x 30 mL of ethyl acetate. The combined organic layers were dried over anhydrous magnesium sulfate, filtered and concentrated under reduced pressure to give a brown oily residue. The crude was purified by column chromatography on silica gel (Cyclohexane : EtOAc from 100 : 0 to 80 : 20) to provide ethyl 2-bromo-2-tetrahydropyran-4-yl-acetate **140** as a yellow oil.
LCMS method F: t_{R} = 2.36 min, no m/z detected

### Preparation of intermediate 141: ethyl 2-(4-bromopyrazol-1-yl)-2-tetrahydropyran-4-yl-acetate

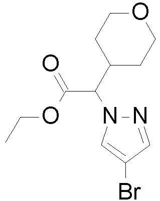

A suspension of 4-bromo-1H-pyrazole (1.50 g, 10.20 mmol), ethyl 2-bromo-2-tetrahydropyran-4-yl-acetate **140** (3.07 g, 12.24 mmol), and potassium carbonate (2.83 g, 20.40 mmol) in *N,N-*dimethylformamide (3.0 mL) was stirred for 3 h at 80°C. The reaction was quenched by water (30 mL), and the resulting solution was extracted twice with ethyl acetate (50 mL x 2). The combined organic layers were dried over anhydrous magnesium sulfate, filtered, and concentrated under reduced pressure to give ethyl 2-(4-bromopyrazol-1-yl)-2-tetrahydropyran-4-yl-acetate **141** as a slightly yellow oil.
LCMS method F: [M+H]⁺ = 317.0, t_{R} = 2.38 min

### Preparation of Example 118: 6-(oxan-4-yl)-8,14-dioxa-4,5,10,19,20-pentaazatetracyclo [13.5.2.1^{2,5}.0^{18,21}]tricosa-1(20),2(23),3,15,17,21-hexaen-9-one

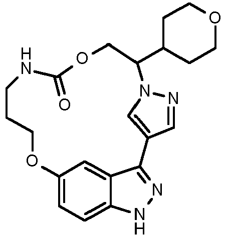

To a solution of 19-(oxan-2-yl)-6-(oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-one (0.115 g, 0.23 mmol) in DCM (6.0 mL) was added trifluoroacetic acid (0.35 mL, 4.60 mmol). The reaction mixture was directly heated to 50 °C for 3 hours. The reaction mixture (brown solution) was evaporated under reduced pressure. The brown residue was dissolved in EtOAc then a saturated aqueous solution of sodium hydrogen carbonate was added. After separation, the aqueous layer was extracted with ethyl acetate (2 x). The combined organic layer was washed with water then brine, dried over sodium sulfate, filtered and evaporated under reduced pressure to give an orange oil. The crude product was purified by preparative TLC plate on silica eluting with Cyclohexane / Ethyl acetate - EtOH (3-1), 100/0 to 70/30 to afford 6-(oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-one **example 118** as a white solid.
LCMS method F: [M+H]⁺ = 412.3, t_{R} = 1.92 min
LCMS method G: [M+H]⁺ = 412.3, t_{R} = 1.92 min
¹H NMR (400 MHz, *d*6-DMSO) δ 12.80 (1H, s), 8.22 (1H, s), 7.83 (1H, dd, *J =* 3.6, 8.0 Hz), 7.77 (1H, s), 7.42 (1H, d, *J =* 9.2 Hz), 7.10 (1H, d, *J =* 1.9 Hz), 6.94 (1H, dd, *J =* 2.3, 8.9 Hz), 4.57 (1H, dd, *J =* 1.8, 12.1 Hz), 4.45 (1H, dt, *J =* 2.8, 12.0 Hz), 4.38 (1H, dq, *J =* 2.0, 10.4 Hz), 4.26 - 4.15 (2H, m), 3.95 (1H, dd, *J =* 2.8, 11.1 Hz), 3.84 - 3.80 (1H, m), 3.48 - 3.39 (1H, m), 3.32 - 3.28 (1H, m), 3.22 (1H, dt, *J =* 2.4, 11.2 Hz), 2.82 (1H, dt, *J* = 2.8, 11.2 Hz), 2.47 - 2.36 (1H, m), 1.95 - 1.88 (1H, m), 1.81 - 1.75 (2H, m), 1.48 (1H, dq, *J =* 4.8, 12.8 Hz), 1.26 (1H, dq, *J =* 4.8, 12.8 Hz), 1.07 (1H, d, *J =* 12.8 Hz) ppm.

### Example 119: 4-ethyl-8,14-dioxa-5,10,19,20,23-pentaazatetracyclo[13.5.2.1^{2,5}.0^{18,21}] tricosa-1(20),2(23),15,17,21-pentaen-9-one

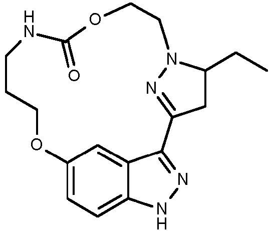

Example 119 is prepared according to the synthesis route described in general Scheme B.

### Preparation of intermediate 142: 5-ethylpyrazolidin-3-one

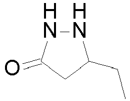

To a solution of hydrazine hydrate 50-60 % (1.00 mL, 15.94 mmol) in isopropanol (13 mL), was added a solution of methyl pent-2-enoate (2.000 g, 17.54 mmol) in isopropanol (10 mL) at room temperature. The reaction mixture was stirred at 80 °C for 16 hours. The mixture was evaporated under reduced pressure. Taken in DCM and again evaporated under reduced pressure twice to remove isopropanol affording the crude 5-ethylpyrazolidin-3-one **142** as a pale yellow oil. The crude was used in the next step without further purification.
Yield: 1.940 g of intermediate 142 (quantitative)

### Preparation of intermediate 143: ethyl 2-(5-ethyl-3-oxo-pyrazolidin-1-yl)acetate

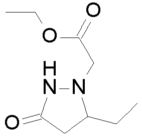

To a solution of 5-ethylpyrazolidin-3-one **142** (1.817 g, 15.94 mmol) and ethyl glyoxylate 50% wt in toluene (3.576 g, 17.53 mmol) in DCM (35 mL) was added at 0 °C sodium triacetoxyborohydride (13.45 g, 63.76 mmol). Gas evolution was observed. The reaction mixture was warmed up to room temperature and stirred for 48 hours. The reaction was quenched by slow addition of an aqueous NaHCO₃ solution (50 mL). Gas evolution was observed. The resulting mixture was diluted with DCM (50 mL), followed by an aqueous NaOH 1 M solution until pH ~ 8/9. After separation, the aqueous layer was extracted with DCM (3 x 50 mL). The combined organic layer was dried over anhydrous magnesium sulfate, filtered and evaporated under reduced pressure to afford ethyl 2-(5-ethyl-3-oxo-pyrazolidin-1-yl)acetate **143** as a brown oil. The crude product was used in the next step without further purification.

### Preparation of intermediate 144: ethyl 2-(5-bromo-3-ethyl-3,4-dihydropyrazol-2-yl)acetate

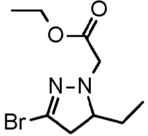

To a mixture of ethyl 2-(5-ethyl-3-oxo-pyrazolidin-1-yl)acetate **143** (1.443 g, 7.21 mmol) and tetraethylammonium bromide (0.453 g, 2.16 mmol) in DCM (3.5 mL) at 20-25 °C was added dropwise triethylamine (0.90 mL, 6.49 mmol) over 2-3 minutes, maintaining the internal temperature below 35 °C. The resulting reaction mixture was then cooled to 0-5 °C. A solution of phosphorus(V) oxybromide (2.680 g, 9.37 mmol) in DCM (3.7 mL) was added dropwise over 5-10 minutes, maintaining the internal temperature below 20 °C. The reaction mixture was warmed to 30 °C and stirred for 5 hours at this temperature. After 5 hours, the reaction mixture was slowly poured into a separate reactor containing a solution of NaOH (1.298 g, 32.44 mmol) in water (26 mL) at 0-5 °C. This slow addition was performed using an addition funnel, maintaining the internal temperature below 20 °C. The addition funnel was rinsed with DCM (1.1 mL). The resulting biphasic mixture was warmed to 20-25 °C, stirred for 3 hours, and the aqueous layer checked by pH. The layers were separated. The organic layer was sequentially washed with a 0.1 M aqueous solution of sodium dihydrogenophosphate (NaH₂PO_{4 (aq)}, 10 mL) and water (10 mL), then concentrated under reduced pressure. The crude oily residue was purified by column chromatography on silica gel (Cyclochexane / EtOAc : from 10/0 to 9/1) to afford ethyl 2-(5-bromo-3-ethyl-3,4-dihydropyrazol-2-yl)acetate **144** as a yellow oil.
LCMS method F: [M+H]⁺ = 265.1, t_{R} = 2.42 min

### Preparation of Example 119: 4-ethyl-8,14-dioxa-5,10,19,20,23-pentaazatetracyclo [13.5.2.1^{2,5}.0^{18,21}]tricosa-1(20),2(23),15,17,21-pentaen-9-one

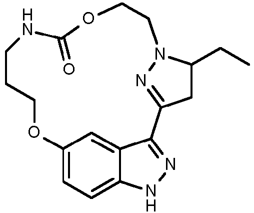

To a solution of 4-ethyl-19-(oxan-2-yl)-8,14-dioxa-5,10,19,20,23-pentaazatetracyclo [13.5.2.1^{2,5}.0^{18,21}]tricosa-1(20),2(23),15,17,21-pentaen-9-one (0.052 g, 0.12 mmol) in DCM (3.0 mL) was added trifluoroacetic acid (0.18 mL, 2.40 mmol). The reaction mixture was directly heated to 50 °C for 3 hours. LCMS analysis showed formation of the expected product and total consumption of starting material. The reaction mixture (brown solution) was evaporated under reduced pressure. The brown residue was dissolved in EtOAc then a saturated aqueous solution of sodium hydrogen carbonate was added. After separation, the aqueous layer was extracted with ethyl acetate (2 x). The combined organic layer was washed with water then brine, dried over sodium sulfate, filtered and evaporated under reduced pressure to give an orange oil. The crude product was purified over preparative TLC plate on silica eluting with Cyclohexane / Ethyl acetate - EtOHormate (3-1), 100/0 to 70/30. The pure fractions were combined and evaporated under reduced pressure to afford 4-ethyl-8,14-dioxa-5,10,19,20,23-pentaazatetracyclo[13.5.2.1^{2,5}.0^{18,21}]tricosa-1(20),2(23),15,17,21-pentaen-9-one **example 119** as a sliglhty yellow solid.
LCMS method F: [M+H]⁺ = 358.2, t_{R} = 2.01 min
LCMS method G: [M+H]⁺ = 358.3, t_{R} = 2.13 min
¹H NMR (400 MHz, d6-DMSO, 80 °C) δ 12.70 (1H, br. s), 7.61 (1H, d, *J =* 1.9 Hz), 7.39 (1H, d, *J* = 8.8 Hz), 7.11 (1H, br. s), 6.94 (1H, dd, *J=* 2.8, 8.8 Hz), 4.45 - 4.17 (4H, m), 3.31 - 3.07 (5H, m), 2.71 - 2.59 (1H, m), 1.92 - 1.80 (3H, m), 1.64 - 1.52 (1H, m), 1.32 - 1.27 (1H, m), 0.98 (3H, t, *J* = 7.6 Hz) ppm.

### Example 120: (13R)-23-fluoro-13-methyl-8,14-dioxa-10,19,20-triazatetracyclo [13.5.2.1^{2,6}.0^{18,21}]tricosa-1(20),2,4,6(23),15,17,21-heptaen-9-one

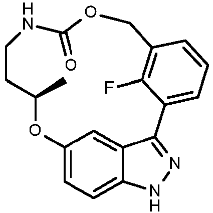

Example 120 is prepared according to the synthesis route described in general Scheme C. In a µwave tube was put a solution of (13R)-23-fluoro-13-methyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one (165 mg, 0.391 mmol) in DCM (10 mL). To this solution was added trifluoroacetic acid (1.2 mL, 15.6 mmol). The reaction mixture was stirred at 80 °C under microwaves irradiations for 1 h. The solvent was removed under reduced pressure, the oil was dissolved in EtOAc and washed with a saturated solution of NaHCO₃. The phase were separated and the organic one was dried with Na₂SO₄. The solvant was removed under reduced pressure. The crude was triturated in DCM, the solid formed was filtered washed with water and dried to give (13R)-23-fluoro-13-methyl-8,14-dioxa-10, 19,20-triazatetracyclo[13.5.2. 1^{2,6}.0^{18,21}]tricosa-1(20),2, 4,6(23),15,17,21-heptaen-9-one **example 120** as an off white powder.
LCMS method F: [M+H]⁺ = 356.2, t_{R} = 2.25 min
LCMS method G: [M+H]⁺ = 356.2, t_{R} = 2.25 min
¹H NMR (400 MHz, *d*6-DMSO) δ 13.42 - 13.11 (1H, s), 7.76 - 7.68 (2H, m), 7.56 - 7.45 (2H, m), 7.30 (1H, t, J = 7.6 Hz), 6.96 - 6.90 (2H, m), 6.07 - 6.02 (1H, m), 4.65 (1H, d, J = 12.5 Hz), 4.58 - 4.54 (1H, m), 3.66 - 3.58 (1H, m), 2.81 - 2.68 (1H, m), 2.23 - 2.16 (1H, m), 1.35 - 1.31 (4H, m) ppm.

### Example 121: 9,14-dioxa-4,5,11,19,20-pentaazatetracyclo[13.5.2.1^{2,5}.0^{18,21}]tricosa-1(20),2(23),3,15,17,21-hexaen-10-one

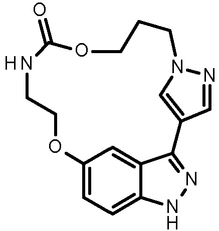

Example 121 is prepared according to the synthesis route described in general Scheme A.

### Preparation of intermediate 145: 3-[4-(4,4,5,5-tetramethyl-1,3,2-dioxaborolan-2-yl)-1H-pyrazol-1-yl]propan-1-ol

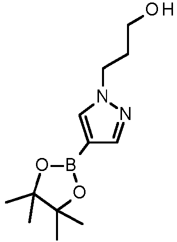

To a solution of 4-bromo-1-(3-hydroxypropyl)-1H-pyrazole (0.410 g, 2.0 mmol) in dioxane (4 mL) was added at RT bis(pinacolato)diboron (0.609 g, 2.4 mmol), KOAc (0.589 g, 6.0 mmol) and PdCl₂(dppf)·DCM (0.082 g, 0.1 mmol). The resulting reaction mixture was stirred under microwave irradiation at 100°C for 2 h. The residue was diluted with water and extracted with ethyl acetate twice. The combined organic layer was dried over anhydrous sodium sulfate affording 3-[4-(4,4,5,5-tetramethyl-1,3,2-dioxaborolan-2-yl)-1H-pyrazol-1-yl]propan-1-ol **145** as a brown oil, which was used in the next step without further purification.
Yield: 0.504 g of intermediate 145 (quantitative)
LCMS method F: [M+H]⁺ = 253.2, t_{R} = 1.91 min

### Preparation of Example 121: 9,14-dioxa-4,5,11,19,20-pentaazatetracyclo [13.5.2.1^{2,5}.0^{18,21}]tricosa-1(20),2(23),3,15,17,21-hexaen-10-one

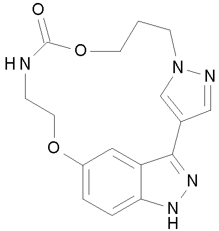

To a solution of 19-(oxan-2-yl)-9,14-dioxa-4,5,11,19,20-pentaazatetracyclo[13.5.2.1^{2,5}.0^{18,21}] tricosa-1(20),2(23),3,15,17,21-hexaen-10-one (0.015 g, 0.04 mmol) in DCM (1 mL) was added at RT TFA (0.054 mL, 0.73 mmol). The resulting reaction mixture was stirred under microwave irradiation at 80°C for 1h. The reaction mixture was concentrated in vacuo, diluted with saturated sodium bicarbonate solution and extracted with ethyl acetate twice. The combined organic layer was dried over anhydrous sodium sulfate and concentrated in vacuo. The residue was purified by flash-column (4 g silica Macherey Nagel) chromatography (cyclohexane - ethyl acetate 3 / EtOH 1, 1:0 to 6:4) affording 9,14-dioxa-4,5,11,19,20-pentaazatetracyclo [13.5.2.1^{2,5}.0^{18,21}]tricosa-1(20),2(23),3,15,17,21-hexaen-10-one **example 121** as a white solid.
LCMS method F: [M+H]⁺ = 328.2, [M-H]⁻ = 326.3, t_{R} = 1.67
LCMS method G: [M+H]⁺ = 328.2, [M-H]⁻ = 326.4, t_{R} = 1.75
¹H NMR (400 MHz, *d*6-DMSO) δ 12.79 (1H, s), 8.31 (1H, s), 7.94 (1H, t, J = 5.5 Hz), 7.80 (1H, s), 7.42 - 7.36 (2H, m), 7.00 (1H, dd, J = 2.3, 8.9 Hz), 4.38 (2H, t, J = 6.0 Hz), 4.29 - 4.25 (2H, m), 3.60 (2H, t, J = 5.6 Hz), 3.41 - 3.35 (2H, m) 2.14 - 2.08 (2H, m) ppm.

### Example 122: 4-ethyl-8,14-dioxa-5,10,19,20,23-pentaazatetracyclo[13.5.2.1^{2,5}.0^{18,21}] tricosa-1(20),2(23),3,15,17,21-hexaen-9-one

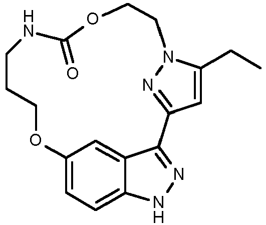

Example 122 is prepared according to the synthesis route described in general Scheme B.

### Preparation of intermediate 146: ethyl 2-[3-[5-[tert-butyl(dimethyl)silyl]oxy-1-tetrahydropyran-2-yl-indazol-3-yl]-5-ethyl-pyrazol-1-yl]acetate

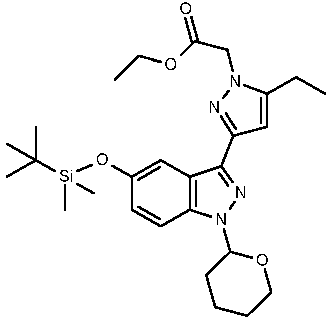

Ethyl 2-[5-[5-[tert-butyl(dimethyl)silyl]oxy-1-tetrahydropyran-2-yl-indazol-3-yl]-3-ethyl-3,4-dihydropyrazol-2-yl]acetate (0.470 g, 0.91 mmol) was diluted in toluene (3.2 mL) and manganese(IV) dioxide (1.193 g, 13.72 mmol) and stirred at 100-105 °C for 24 hours. The reaction mixture was cooled down to room temperature, filtered over a celite pad, rinsed with ethyl acetate, then evaporated under reduced pressure. The crude residue was purified by column chromatography (DCM / EtOAc from 1 : 0 to 7:3) to afford ethyl 2-[3-[5-[tert-butyl(dimethyl)silyl]oxy-1-tetrahydropyran-2-yl-indazol-3-yl]-5-ethyl-pyrazol-1-yl]acetate **146** as yellow solid.
LCMS method F: [M+H]⁺ = 513.4, t_{R} = 3.77 min

### Preparation of Example 122: 4-ethyl-8,14-dioxa-5,10,19,20,23-pentaazatetracyclo [13.5.2.1^{2,5}.0^{18,21}]tricosa-1(20),2(23),3,15,17,21-hexaen-9-one

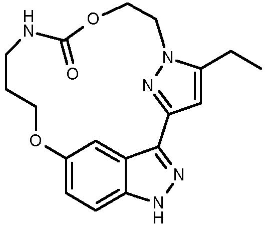

To a solution of 4-ethyl-19-(oxan-2-yl)-8,14-dioxa-5,10,19,20,23-pentaazatetracyclo [13.5.2.1^{2,5}.0^{18,21}]tricosa-1(20),2(23),3,15,17,21-hexaen-9-one (0.024 g, 0.054 mmol) in DCM (1.5 mL) was added trifluoroacetic acid (83 µL, 1.09 mmol). The reaction mixture was directly heated to 50 °C *(sand bath previously heated to 50* °C) for 6 hours. The reaction mixture (brown solution) was evaporated under reduced pressure. The brown residue was dissolved in EtOAc then a saturated aqueous solution of sodium hydrogen carbonate was added. After separation, the aqueous layer was extracted with ethyl acetate (2 x). The combined organic layer was washed with water then brine, dried over sodium sulfate, filtered and evaporated under reduced pressure. The crude product was purified by preparative TLC plate using DCM/MeOH 9/1 as an eluant to give 4-ethyl-8,14-dioxa-5,10,19,20,23-pentaazatetracyclo[13.5.2.1^{2,5}.0^{18,21}]tricosa-1(20),2(23),3,15,17,21-hexaen-9-one **example 122** as an orange solid.
LCMS method F: [M+H]⁺ = 356.3, t_{R} = 1.99 min
LCMS method G: [M+H]⁺ = 356.3, t_{R} = 2.03 min
¹H NMR (400 MHz, d6-DMSO) δ 12.80 (1H, s), 7.64 - 7.58 (2H, m), 7.38 (1H, d, *J =* 9.2 Hz), 6.92 (1H, dd, *J* = 9.2 *,* 3.2 Hz), 6.44 (1H, s), 4.41 (4H, s), 4.27 - 4.22 (2H, m), 3.11 - 3.06 (2H, m), 2.70 (2H, q, *J =* 7.2 Hz), 1.92 - 1.82 (2H, m), 1.27 (3H, t, *J =* 7.2 Hz) ppm.

### Example 123: 3,9,15-trioxa-4,11,20,21-tetraazatetracyclo[14.5.2.1^{2,5}.0^{19,22}]tetracosa-1(21),2(24),4,16,18,22-hexaen-10-one

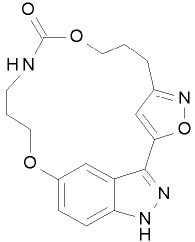

Example 123 is prepared according to the synthesis route described in general Scheme M.

### Preparation of intermediate 147: benzyl N-[3-[1-tetrahydropyran-2-yl-3-(2-triethylsilylethynyl)indazol-5-yl]oxypropyl]carbamate

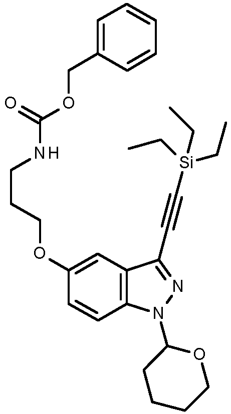

To a degassed solution of benzyl N-[3-(3-iodo-1-tetrahydropyran-2-yl-indazol-5-yl)oxypropyl]carbamate (2.500 g, 4.67 mmol) in THF (15.0 mL), triethylamine (0.754 mL, 5.65 mmol), CuI (0.071 g, 0.374 mmol) and Tetrakis(triphenylphosphine) palladium (0.058 g, 0.050 mmol) was added. Triethylsilylacetylene (1.088 mL, 6.07 mmol) was slowly added and the reaction was heated to 80°C for 4 h. The reaction mixture was diluted with water (30 mL) and EtOAc (30 mL) and extracted with EtOAc (3 x 30 mL). The organic layer was combined, washed with saturated brine (30 mL), dried with MgSO₄, filtered and concentrated under reduced pressure. The crude material (3.0 g) was purified by column (Macherey Nagel, 40 g, CyH/EtOAc : 90/10 to 70/30). Solvents were evaporated to afford benzyl N-[3-[1-tetrahydropyran-2-yl-3-(2-triethylsilylethynyl)indazol-5-yl]oxypropyl]carbamate **147** as an orange oil.
LCMS method F: [M+H]⁺ = 548.4, t_{R} = 3.81 min

### Preparation of intermediate 148: benzyl N-[3-(3-ethynyl-1-tetrahydropyran-2-yl-indazol-5-yl)oxypropyl]carbamate

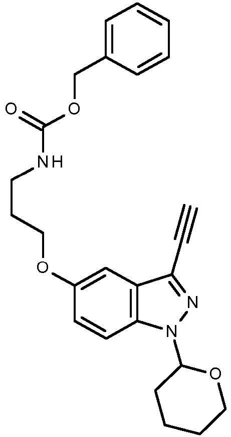

To a solution of benzyl N-[3-[1-tetrahydropyran-2-yl-3-(2-triethylsilylethynyl)indazol-5-yl]oxypropyl]carbamate **147** (2.375 g, 4.36 mmol) in THF (100.0 mL) was added dropwise at room temperature tetrabutylammonium fluoride 1 M in THF (4.77 mL, 4.77 mmol). The resulting mixture was stirred at room temperature during 16 h. Water (35 mL) and EtOAc (35 mL) were added and phases were separated. The aqueous layer was extracted with EtOAc (3 x 25 mL) and the organic phase was washed with brine (35 mL) and dried with MgSO₄, filtered and concentrated. The crude (2.0 g) was purified by chromatography column (Macherey Nagel, 24 g, CyH/EtOAc : 90/10 to 70/30). Solvents were evaporated to afford benzyl N-[3-(3-ethynyl-1-tetrahydropyran-2-yl-indazol-5-yl)oxypropyl]carbamate **148** as a yellow oil.
LCMS method F: [M+H]⁺ = 434.3, t_{R} = 3.05 min

### Preparation of intermediate 149: tert-butyl-(4-nitrobutoxy)-diphenyl-silane

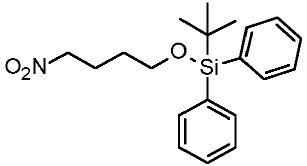

In a round bottom flask, to a solution of 4-nitrobutan-1-ol (1.00 g, 8.39 mmol) and imidazole (0.686 g, 10.07 mmol) in DCM (85.0 mL) was added slowly tert-butyl(chloro)diphenylsilane (2.18 mL, 8.39 mmol). The mixture was stirred at room temperature during 3 h. Water was added (35 mL) and phases were separated. The aqueous layer was extracted with DCM (3 x 30 mL), then the organic layer was washed with a saturated solution of NaCl (25 mL), dried with MgSO₄, filtered and concentrated under reduced pressure to afford crude tert-butyl-(4-nitrobutoxy)-diphenyl-silane **149** as a colorless oil which was used in the next step without further purification.
LCMS method F: [M+H]⁺ = 358.3, t_{R} = 3.67 min

### Preparation of intermediate 150: benzyl N-[3-[3-[3-[2-[tert-butyl(diphenyl) silyl]oxyethyl]isoxazol-5-yl]-1-tetrahydropyran-2-yl-indazol-5-yl]oxypropy]carbamate

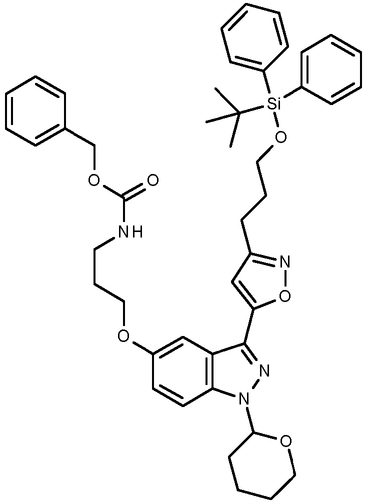

In a round bottom flask, to a solution of tert-butyl-(4-nitrobutoxy)-diphenyl-silane 149 (3.030 g, 8.39 mmol) and benzyl N-[3-(3-ethynyl-1-tetrahydropyran-2-yl-indazol-5-yl)oxypropyl]carbamate **141** (1.000 g, 2.31 mmol) in toluene (25.0 mL), were added phenyl isocyanate (0.276 mL, 2.54 mmol) and triethylamine (0.353 mL, 2.54 mmol). The reaction was stirred during 2 days at 60°C. The precipitate (urea) was filtered and solvent was removed to give crude (4 g) which was purified by chromatography column by solid deposit (Macherey Nagel 40 g, Cyclo/EtOAc : 100/0 to 70/30). Solvents were evaporated to afford benzyl N-[3-[3-[3-[2-[tert-butyl(diphenyl)silyl]oxyethyl]isoxazol-5-yl]-1-tetrahydropyran-2-yl-indazol-5-yl]oxypropyl]carbamate **150** as a yellow solid.
LCMS method I: [M+H]⁺ = 773.5, t_{R} = 3.71 min

### Preparation of intermediate 151: benzyl N-[3-[3-[3-(3-hydroxypropyl)isoxazol-5-yl]-1-tetrahydropyran-2-yl-indazol-5-yl]oxypropyl]carbamate

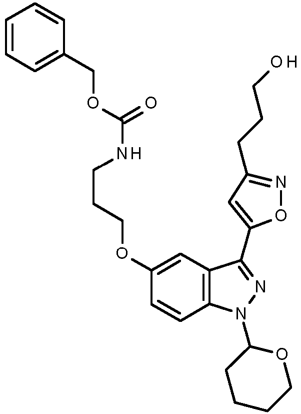

In a round bottom flask, to a solution of benzyl N-[3-[3-[3-[3-[tert-butyl(diphenyl) silyl]oxypropyl]isoxazol-5-yl]-1-tetrahydropyran-2-yl-indazol-5-yl]oxypropyl]carbamate **150** (2.00 g, 2.31 mmol) in THF (25.0 mL), was added TBAF (1.0 M in THF), 2.77 mL, 2.77 mmol). The reaction was stirred during 2 h at room temperature. Water (20 mL) and EtOAc (20 mL) were added. Phases were separated and the aqueous phase was extracted with EtOAc (3 x 25 mL). Then, the organic phase was washed with a saturated solution of NaCl (20 mL), dried with MgSO₄, filtered and concentrated under reduced pressure to give crude (1.8 g) which was purified by chromatography column (RediSep 25 g, CyH/EtOAc : 100/0 to 70/30, then DCM/MeOH : 100/0 to 90/10). Solvents was evaporated to afford benzyl N-[3-[3-[3-(3-hydroxypropyl)isoxazol-5-yl]-1-tetrahydropyran-2-yl-indazol-5-yl]oxypropyl]carbamate **151** as an orange oil.
LCMS method F: [M+H]⁺ = 535.3, t_{R} = 2.85 min

### Preparation of intermediate 152: 20-(oxan-2-yl)-3,9,15-trioxa-4,11,20,21-tetraazatetracyclo [14.5.2.1^{2,5}.0^{19,22}]tetracosa-1(21),2(24), 4,16,18,22-hexaen-10-one

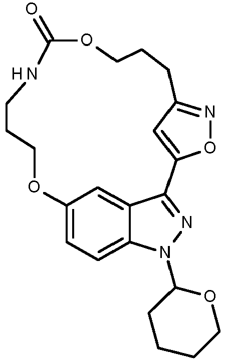

The reaction was divided in 2 batches. In a round bottom flask, benzyl N-[3-[3-[3-(3-hydroxypropyl)isoxazol-5-yl]-1-tetrahydropyran-2-yl-indazol-5-yl]oxypropyl]carbamate **151** (0.430 g, 0.805 mmol) in dry MeCN (300.0 mL) was added cesium carbonate (1.572 g, 4.825 mmol). The reaction was stirred during 48 h at 85°C. The mixture was filtered and the filtrate was concentrated under reduce pressure to afford crude (2 x 400 mg) which were combined and purified by chromatography column by solid deposit (Macherey Nagel 4 g, DCM/MeOH : 100/0 to 95/5). Good fractions were gathered and solvent was removed, to afford 20-(oxan-2-yl)-3,9,15-trioxa-4,11,20,21-tetraazatetracyclo[14.5.2.1^{2,5}.0^{19,22}]tetracosa-1(21),2(24), 4,16,18,22-hexaen-10-one **152** as a white powder.
LCMS method F: [M+H]⁺ = 427.2, t_{R} = 2.51 min

### Preparation of Example 123: 3,9,15-trioxa-4,11,20,21- tetraazatetracyclo[14.5.2.1^{2,5}.0^{19,22}] tetracosa-1(21),2(24),4,16,18,22-hexaen-10-one

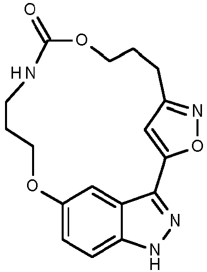

At 0°C, in a round bottom flask, to a solution of 20-(oxan-2-yl)-3,9,15-trioxa-4,11,20,21-tetraazatetracyclo[14.5.2.1^{2,5}.0^{19,22}]tetracosa-1(21),2(24),4,16(23),17,19(22)-hexaen-10-one **152** (0.200 g, 0.470 mmol) in DCM (50.0 mL), was added trifluoroacetic acid (1.08 mL, 14.10 mmol). The reaction was stirred at room temperature during 18h. A saturated solution of NaHCO₃ (50 mL) was added and phases were separated. The aqueous phase was extracted with DCM (3 x 25 mL), dried with MgSO₄, filtered and concentrated to afford crude (90 mg) which was purified by preparative reverse-phase chromatography (Column Waters XSELECT C18 19*100mm, 5 µm (NH₄)₂CO₃ aq. 2 g/L MeCN 19 mL /min, RT 25% B to 55% B in 7 min). Solvents were removed to afford 3,9,15-trioxa-4,11,20,21- tetraazatetracyclo[14.5.2.1^{2,5}.0^{19,22}] tetracosa-1(21),2(24),4,16,18,22-hexaen-10-one **example 123** as a white powder.
LCMS method F: [M+H]⁺ = 343.3, t_{R} = 2.01 min
LCMS method G: [M+H]⁺ = 343.2, t_{R} = 1.99 min
¹H NMR (400 MHz, d6-DMSO) δ 13.59 (1H, s), 7.78 (1H, t, J = 6.0 Hz), 7.59 - 7.56 (1H, m), 7.16 (1H, d, J = 2.3 Hz), 7.04 (1H, dd, J = 2.4, 9.0 Hz), 6.86 (1H, s), 4.39 - 4.33 (2H, m), 3.85 (2H, t, J = 5.6 Hz), 3.08 (2H, dd, J = 6.2, 10.0 Hz), 2.93 (2H, t, J = 6.5 Hz), 2.02 - 1.96 (2H, m), 1.86 - 1.75 (2H, m) ppm.

### Example 124: (13R)-16-fluoro-13-methyl-8,14-dioxa-4,10,19,20-tetraazatetracyclo [13.5.2.1^{2,6}.0^{18,21}]tricosa-1(20),2,4,6(23),15,17,21-heptaen-9-one

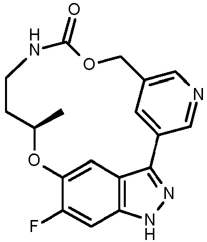

Example 124 is prepared according to the synthesis route described in general Scheme C.

### Preparation of intermediate 153: benzyl N-[(3R)-3-[(6-fluoro-3-iodo-1H-indazol-5-yl)oxy]butyl]carbamate

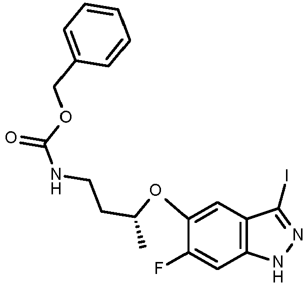

To a solution of benzyl *N*-[(3R)-3-[(6-fluoro-1H-indazol-5-yl)oxy]butyl]carbamate (1.96 g, 5.49 mmol) in acetone (14 mL) was added portionwise *N*-Iodosuccinimide (1.35 g, 6.04 mmol) at 0°C and the reaction mixture was then stirred at room temperature overnight. The reaction mixture was evaporated to dryness and the residue was dissolved in EtOAc (50 mL). The organic phase was washed with a 1 M solution of sodium thiosulfate, with brine, dried over anhydrous sodium sulfate, filtered and evaporated under reduced pressure to give a colorless oil. The residue was purified by flash chromatography (CyH/EtOAc 5/5) to afford benzyl N-[(3R)-3-[(6-fluoro-3-iodo-1H-indazol-5-yl)oxy]butyl]carbamate **153.**
LCMS method F: [M+H]⁺ = 484, t_{R} = 2.82 min

### Preparation of intermediate 154: benzyl N-[(3R)-3-[(6-fluoro-3-iodo-1H-indazol-5-yl)oxy]butyl]carbamate

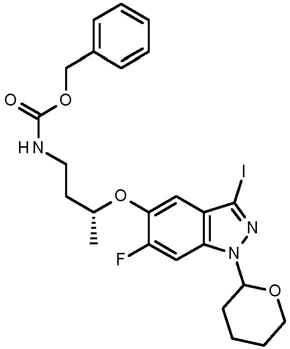

To a mixture of benzyl benzyl *N*-[(3R)-3-[(6-fluoro-3-iodo-1H-indazol-5-yl)oxy]butyl]carbamate and benzyl *N*-[(3R)-3-{[6-fluoro-3-iodo-1-(oxan-2-yl)-1H-indazol-5-yl]oxy}butyl]carbamate (900 mg, 1.59 mmol supposed) in DCM (20 mL) was added trifluoroacetic acid (0.6 mL, 7.95 mmol) and the solution was stirred at room temperature for 4 hours. The residue was diluted with saturated sodium bicarbonate solution (50 mL) and extracted with DCM (3 x 50 mL). The combined organic layer was washed with brine, dried over Na₂SO₄, filtered and evaporated under reduced pressure. The residue was filtered on a silice pad eluting with cyclohexane/EtOAc : 100/0 to 50/50 to afford the expected compound benzyl *N*-[(3R)-3-[(6-fluoro-3-iodo-1H-indazol-5-yl)oxy]butyl]carbamate **154** as a colorless oil.
LCMS method F: [M+H]⁺ = 568, t_{R} = 3.25 min

### Preparation of Example 124: (13R)-16-fluoro-13-methyl-8,14-dioxa-4,10,19,20-tetraazatetracyclo[13.5.2.1^{2,6}.0^{18,21}]tricosa-1(20),2,4, 6(23),15,17,21-heptaen-9-one

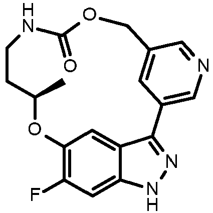

To a solution of (13R)-16-fluoro-13-methyl-19-(oxan-2-yl)-8,14-dioxa-4,10,19,20-tetraazatetracyclo[13.5.2.1^{2,6}.0^{18,21}]tricosa-1(20),2,4,6(23),15,17,21-heptaen-9-one (126 mg, 0.29 mmol,) in DCM (3 mL) was added trifluoroacetic acid (438 µL, 5.73 mmol). The mixture was heated under microwave irradiation at 80 °C for 30 min. The reaction mixture was diluted with DCM (25 mL) and NaHCO₃ saturated (25 mL). A precipitate was formed between the layers after 2 hours at RT. This precipitate was filtered and washed with diethyl ether twice and dried under vaccum at 60 °C for 12 hours to afford (13R)-16-fluoro-13-methyl-8,14-dioxa-4,10,19,20-tetraazatetracyclo[13.5.2.1^{2,6}.0^{18,21}]tricosa-1(20),2,4, 6(23),15,17,21-heptaen-9-one **example 124** as a white solid.
LCMS method F: [M+H]⁺ = 357, t_{R} = 1.94 min
LCMS method G: [M+H]⁺ = 357, t_{R} = 1.93 min
¹H NMR (400 MHz, d6-DMSO) δ 13.43 (1H, s), 9.04 (1H, d, J=1.1 Hz), 8.57 (1H, s), 8.15 (1H, s), 8.03 - 8.00 (1H, m), 7.53 (1H, d, J=10.8 Hz), 7.32 - 7.28 (1H, m), 5.77 - 5.73 (1H, m), 4.98 - 4.94 (1H, m), 4.64 - 4.58 (1H, m), 3.51 (1H, m), 2.93 (1H, dd, J=13.3, 16.9 Hz), 2.41 - 2.39 (1H, m), 1.44 (4H, m) ppm.

### Example 125: (13R)-4-chloro-13-methyl-8,14-dioxa-10,19,20,23-tetraazatetracyclo [13.5.2.1^{2,6}.0^{18,21}]tricosa-1(20),2(23),3,5,15(22),16,18(21)-heptaen-9-one

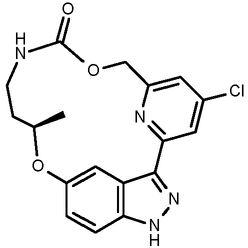

Example 125 is prepared according to the synthesis route described in general Scheme K.

In a round bottom flask, was added (13R)-4-chloro-13-methyl-19-(oxan-2-yl)-8,14-dioxa-10,19,20,23-tetraazatetracyclo[13.5.2.1^{2,6}.0^{18,21}]tricosa-1(20),2(23),3,5,15(22),16,18(21)-heptaen-9-one (0.650 g, 1.42 mmol) in DCM (14.5 mL) and trifluoroacetic acid (3.26 mL, 42.60 mmol). The reaction mixture was stirred at 80°C during 2 h. LC/MS analysis indicated the reaction was completed. The reaction was quenched with a saturated solution of NaHCO₃ (15 mL). Phases were separated and the aqueous phase was extracted with DCM (3 x 10 mL). Organic phase was washed with a saturated solution of NaCl (15 mL), dried with MgSO₄, filtered and concentrated under reduce pressure to afford crude (0.600 g) which was purified by chromatography column (Macherey Nagel 12 g, DCM/MeOH : 100/0 to 95/5). Solvents were removed and the powder was triturated with DCM, to afford (13R)-4-chloro-13-methyl-8,14-dioxa-10,19,20,23-tetraazatetracyclo[13.5.2.1^{2,6}.0^{18,21}]tricosa-1(20),2(23),3,5,15(22), 16,18(21)-heptaen-9-one **example 125** as a white powder.
LCMS method F: [M+H]⁺ = 373.2, t_{R} = 2.32 min
LCMS method G: [M+H]⁺ = 373.2, t_{R} = 2.31 min
¹H NMR (400 MHz, d6-DMSO) δ 13.41 (1H, s), 8.08 (1H, d, J = 1.9 Hz), 7.84 - 7.76 (2H, m), 7.50 - 7.42 (2H, m), 6.97 (1H, dd, J = 2.4, 9.0 Hz), 5.62 - 5.57 (1H, m), 5.09 - 5.04 (1H, m), 4.60 (1H, t, J = 10.2 Hz), 3.50 - 3.44 (1H, m), 2.92 (1H, t, J = 14.8 Hz), 2.27 - 2.23 (1H, m), 1.39 - 1.36 (4H, m) ppm.

### Example 126: 8,14-dioxa-2,4,10,19,20-pentaazatetracyclo[13.5.2.1^{2,5}.0^{18,21}]tricosa-1(20),3,5(23),15(22),16,18(21)-hexaen-9-one

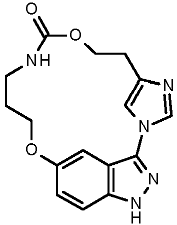

Example 126 is prepared according to the synthesis route described in general Scheme A.

To a solution of 19-(oxan-2-yl)-8,14-dioxa-2,4,10,19,20-pentaazatetracyclo [13.5.2.1^{2,5}.0^{18,21}]tricosa-1(20),3,5(23),15(22),16,18(21)-hexaen-9-one (99 mg, 0.24 mmol) in dioxane (3.8 mL) was added HCl 4 M in dioxane (1.2 mL, 4.82 mmol). The mixture was heated under microwave irradiations at 100 °C for 1 hour and 30 minutes. HCl 4 M in dioxane (0.5 mL, 2 mmol) was added and the reaction was heated under microwave irradiations for 1 hour and 20 minutes. In the reaction mixture a precipitate was formed so it was filtrated and the solid was triturated in acetonitrile. So the solid was dissolved with DCM/MeOH and diluted with NaHCO₃ saturated. After separation, aqueous layer was extracted with DCM (3 x). The combined organic layer was washed with brine, dried over anhydrous sodium sulfate, filtered and evaporated under reduced pressure to give 8,14-dioxa-2,4,10,19,20-pentaazatetracyclo[13.5.2.1^{2,5}.0^{18,21}]tricosa-1(20),3,5(23),15(22),16,18(21)-hexaen-9-one **example 126** as a white powder.
LCMS method F: [M+H]⁺ = 328, t_{R} = 1.15 min
LCMS method G: [M+H]⁺ = 328, t_{R} = 1.62 min
¹H NMR (400 MHz, *d*6-DMSO) δ 13.05 (1H, m), 8.02 (1H, d, J = 1.3 Hz), 7.69 (1H, t, J = 6.1 Hz), 7.50 (1H, m), 7.30 (1H, m), 7.04 (2H, dd, J = 2.3, 9.1 Hz), 6.95 (1H, m), 4.38 (2H, m), 4.25 (2H, m), 3.08 (2H, m), 2.95 (2H, t, J = 5.3 Hz), 1.85 (1H, m) ppm.

### Example 127: (13R)-4-methoxy-13-methyl-8,14-dioxa-10,19,20,23-tetraazatetracyclo [13.5.2.1^{2,6}.0^{18,21}]tricosa-1(20),2,4,6(23),15,17,21-heptaen-9-one

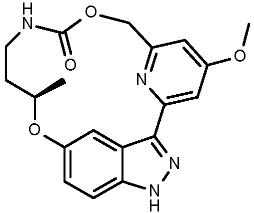

Example 127 is prepared according to the synthesis route described in general Scheme K.

In a microwaves tube was put a solution of (13R)-4-methoxy-13-methyl-19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one (60 mg, 0.133 mmol) in DCM (2.5 mL). To this solution was added trifluoroacetic acid (0.813 mL, 10.6 mmol). The reaction mixture was stirred at 80 °C under microwave conditions for 1 h.

The solvant was removed under reduced pressure, the oil was dissolved in EtOAc and washed with a sol. sat. of NaHCO₃. The phase were separated and the organic one was dried with Na₂SO₄. The solvant was removed under reduced pressure. The crude was purified by chromatography using a 10 g SiO₂ column eluted with cyclohexane/Ethyl acetate 70/30 to 50/50. The good fractions were combined and the solvant was removed under reduced pressure. The oil was triturated in DCM and the solid formed was filtered and dried under reduced pressure to give (13R)-4-methoxy-13-methyl-8,14-dioxa-10,19,20,23-tetraazatetracyclo [13.5.2.1^{2,6}.0^{18,21}]tricosa-1(20),2,4,6(23),15,17,21-heptaen-9-one **example 127** as a white fluffy powder.
LCMS method F: [M+H]⁺ = 369.3, t_{R} = 2.05 min
LCMS method G: [M+H]⁺ = 369.3, t_{R} = 2.06 min
¹H NMR (400 MHz, *d*6-DMSO) δ 13.22 - 13.20 (1H, m), 7.88 (1H, d, J = 1.9 Hz), 7.72 (1H, dd, J = 5.4, 6.9 Hz), 7.60 (1H, d, J=2.5 Hz), 7.47 - 7.44 (1H, m), 6.96 - 6.88 (2H, m), 5.55 (1H, d, J = 14.6 Hz), 5.01 - 4.96 (1H, m), 4.61 (1H, dd, J = 2.9, 3.9 Hz), 3.91 (3H, s), 3.48 - 3.42 (1H, m), 2.96 - 2.88 (1H, m), 2.34 - 2.22 (1H, m), 1.38 - 1.35 (4H, m) ppm.

### Example 128: (13R)-13-methyl-9-oxo-8,14-dioxa-10,19,20-triazatetracyclo [13.5.2.1^{2,6}. 0^{18,21}]tricosa-1(20),2,4,6(23),15,17,21-heptaene-5-carbonitrile

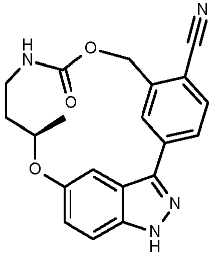

Example 128 is prepared according to the synthesis route described in general Scheme B.

### Preparation of intermediate 155: ethyl 5-bromo-2-cyano-benzoate

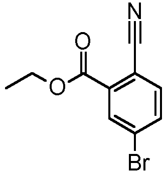

To a solution of 5-bromo-2-cyano-benzoic acid (2.500 g, 11.06 mmol) in ACN (50 mL) were added cesium carbonate (7.207 g, 22.12 mmol) and ethyl iodide (1.33 mL, 16.59 mmol). The mixture was stirred at 90°C for 30 min. Ethyl iodide (1.33 mL, 16.59 mmol) was added and the reaction was heated at 80°C. After 2 h, ethyl iodide (1.33 mL, 16.59 mmol) was added and the reaction was heated at 80°C for 3 additional hours. The solvent was evaporated under reduced pressure. The residue was diluted with water and EtOAc and the layers were separated. The aqueous layer was extracted with EtOAc (3 x 20 mL) and the combined organic layers were dried over anhydrous sodium sulfate, filtered and evaporated under reduced pressure. The residue was purified by silica gel column chromatography (Chromabond Macherey Nagel 80 g) using Cyclohexane/(EtOAc/EtOH 3: 1) 100:0 to 80:20 as eluent. The expected fractions were combined and evaporated under reduced pressure to give ethyl 5-bromo-2-cyano-benzoate **155** as a white solid.
LCMS method F: t_{R} = 2.53 min, no m/z detected

### Preparation of Example 128 : (13R)-13-methyl-9-oxo-8,14-dioxa-10,19,20-triazatetra cyclo [13.5.2.1^{2,6}.0^{18,21}]tricosa-1(20),2,4,6(23),15,17,21-heptaene-5-carbonitrile

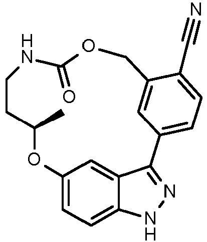

To a suspension of (13R)-13-methyl-19-(oxan-2-yl)-9-oxo-8,14-dioxa-10,19,20-triazatetracyclo[13.5.2.1^{2,6}.0^{18,21}]tricosa-1(20),2,4,6(23),15,17,21-heptaene-5-carbonitrile (102 mg, 0.23 mmol) in DCM (3 mL) was added TFA (0.03 mL, 0.35 mmol). The reaction mixture was stirred at room temperature for 30 min. TFA (0.03 mL, 0.35 mmol) was added and the reaction was stirred for 2 more hours. More TFA (0.03 mL, 0.35 mmol) was added for 3 additional hours. A saturated solution of NaHCO₃ was added and the layers were separated. The aqueous layer was extracted with DCM (3 x 15 mL) and the combined organic layers were dried over anhydrous sodium sulfate, filtered and evaporated under reduced pressure. The residue was purified by preparative HPLC: Column Waters Phenyl-Hexyl C18 19* 100 mm, 5 µm / A: (NH₄)₂CO₃ aq. 2 g/L / B: ACN / 19 mL/min, RT / 40% B to 90% B in 7 min. The solvent was evaporated under reduced pressure to give (13R)-13-methyl-9-oxo-8,14-dioxa-10,19,20-triazatetracyclo[13.5.2.1^{2,6}.0^{18,21}]tricosa-1(20),2,4,6(23),15,17,21-heptaene-5-carbonitrile **example 128** as a white solid.
LCMS method F: [M+H]⁺ = 363.3, t_{R} = 2.28 min
LCMS method G: [M+H]⁺ = 363.3, t_{R} = 2.26 min
¹H NMR (400 MHz, d6-DMSO) δ 13.49 (1H, s), 8.19 - 8.15 (1H, m), 8.11 - 8.07 (1H, m), 7.97 - 7.93 (2H, m), 7.58 - 7.54 (1H, m), 7.29 (1H, s), 7.04 - 6.99 (1H, m), 5.76 - 5.71 (1H, m), 5.16 - 5.10 (1H, m), 4.61 - 4.57 (1H, m), 3.58 - 3.55 (1H, m), 2.99 - 2.90 (1H, m), 2.39 - 2.33 (1H, m), 1.44 - 1.40 (4H, m) ppm.

### Example 129: (13R)-13-methyl-4-(pyrrolidin-1-yl)-8,14-dioxa-5,10,19,20,23-pentaaza tetracyclo[13.5.2.1^{2,6}.0^{18,21}]tricosa-1(20),2,4,6(23),15,17,21-heptaen-9-one

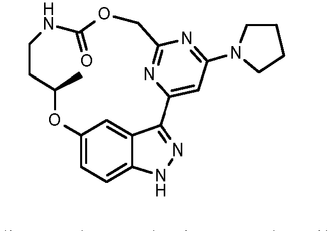

Example 129 is prepared according to the synthesis route described below.

### Preparation of intermediate 156: 4-chloro-2-(chloromethyl)-6-pyrrolidin-1-yl-pyrimidine

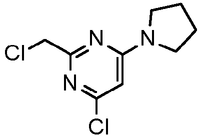

To a solution of 4,6-dichloro-2-(chloromethyl)pyrimidine (0.050 g, 0.253 mmol) in *N,N-*dimethylformamide (3.0 mL) at 0°C was added triethylamine (0.048 mL, 0.304 mmol), and the reaction was stirred during 20 minutes. Then, pyrrolidine (0.021 mL, 0.253 mmol) was added and the mixture was stirred during 2 h. Solvent was evaporated. Water (10 mL) and EtOAc (10 mL) were added. The aqueous phase was extracted with EtOAc (3 x 10 mL), then organic phase was washed with a saturated solution of NaCl (10 mL), dried with MgSO₄, filtered and concentrated. The crude (60 mg) was purified by chromatography column (Macherey Nagel 4g, CyH/EtOAc: 100/0 to 80/20). Solvents were removed to afford 4-chloro-2-(chloromethyl)-6-pyrrolidin-1-yl-pyrimidine **156** as a white powder.
LCMS method F: [M+H]⁺ = 232.2, t_{R} = 2.53 min

### Preparation of intermediate 157: (4-chloro-6-pyrrolidin-1-yl-pyrimidin-2-yl)methyl acetate

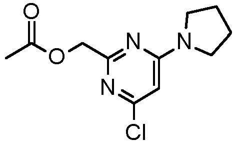

To a solution of 4-chloro-2-(chloromethyl)-6-pyrrolidin-1-yl-pyrimidine **156** (0.041 g, 0.177 mmol) in *N,N*-dimethylformamide (3.0 mL) at 0°C was added sodium iodide (0.030 g, 0.195 mmol), and potassium acetate (0.035 g, 0.354 mmol) was added and the mixture was stirred during 3 days at room temperature. Solvent was evaporated. Water (10 mL) and EtOAc (10 mL) were added. The aqueous phase was extracted with EtOAc (3 x 10 mL), then organic phase was washed with a saturated solution of NaCl (10 mL), dried with MgSO₄, filtered and concentrated to afford (4-chloro-6-pyrrolidin-1-yl-pyrimidin-2-yl)methyl acetate **157** as a white powder which was used in the next step without further purification.
LCMS method F: [M+H]⁺ = 256.1, t_{R} = 2.28 min

### Preparation of intermediate 158: benzyl N-[(3R)-3-[3-[2-(hydroxymethyl)-6-pyrrolidin-1-yl-pyrimidin-4-yl]-1-tetrahydropyran-2-yl-indazol-5-yl]oxybutyl]carbamate

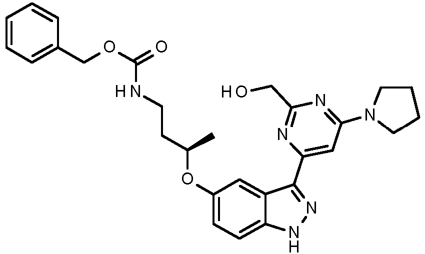

To a suspension of [4-[5-[(1R)-3-(benzyloxycarbonylamino)-1-methyl-propoxy]-1-tetrahydropyran-2-yl-indazol-3-yl]-6-pyrrolidin-1-yl-pyrimidin-2-yl]methyl acetate **157** (0.650 g, 1.011 mmol) in MeOH (20.0 mL) was added potassium carbonate (0.168 g, 1.214 mmol). The reaction was stirred at room temperature during 2 h. The reaction was stopped and filtered and evaporated. The crude (1.0 g) was purified by chromatography column (RediSep, 12 g, DCM/MeOH: 100/0 to 95/5). Solvents was evaporated to afford benzyl N-[(3R)-3-[3-[2-(hydroxymethyl)-6-pyrrolidin-1-yl-pyrimidin-4-yl]-1-tetrahydropyran-2-yl-indazol-5-yl]oxybutyl]carbamate **158** as a yellow solid, which was used in the next step without further purification.
LCMS method F: [M+H]⁺ = 601.4, t_{R} = 2.33 min

### Preparation of Example 129: (13R)-13-methyl-4-(pyrrolidin-1-yl)-8,14-dioxa-5,10,19, 20,23-pentaazatetracyclo[13.5.2.1^{2,6}.0^{18,21}]tricosa-1(20),2,4,6(23),15,17,21-heptaen-9-one

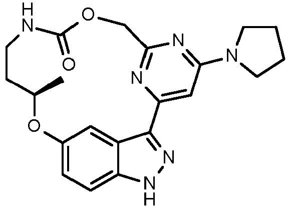

In a round bottom flask, was added (13R)-13-methyl-19-(oxan-2-yl)-4-(pyrrolidin-1-yl)-8,14-dioxa-5,10,19,20,23-pentaazatetracyclo[13.5.2.1^{2,6}.0^{18,21}]tricosa-1(20),2,4,6(23),15, 17,21-heptaen-9-one (0.210 g, 0.426 mmol) in DCM (25.0 mL) and trifluoroacetic acid (1.00 mL, 12.79 mmol). The reaction mixture was stirred at 50 °C during 6 h. A saturated solution of NaHCO₃ (30 mL) was added and phases were separated. The aqueous phase was washed with DCM (3 x 25 mL), and organic phase was washed with a saturated solution of NaCl (30 mL), dried with MgSO₄, filtered and concentrated under reduce pressure to crude which was triturated with MeOH then dried (speedvac), to afford (13R)-13-methyl-4-(pyrrolidin-1-yl)-8,14-dioxa-5,10,19,20,23-pentaazatetracyclo[13.5.2.1^{2,6}.0^{18,21}]tricosa-1(20),2,4,6(23),15, 17,21-heptaen-9-one **example 129** as a white powder.
LCMS method F: [M+H]⁺ = 409.4, t_{R} = 2.03 min
LCMS method G: [M+H]⁺ = 409.4, t_{R} = 2.20 min
¹H NMR (400 MHz, *d*6-DMSO) δ 13.35 (1H, s), 7.93 (1H, d, J = 2.1 Hz), 7.78 (1H, dd, J = 4.4, 7.6 Hz), 7.48 - 7.45 (1H, m), 7.05 (1H, s), 6.95 (1H, dd, J = 2.5, 8.9 Hz), 5.43 - 5.31 (1H, m), 4.80 - 4.53 (2H, m), 3.60 - 3.52 (6H, m), 2.96 - 2.80 (1H, m), 2.34 (1H, dd, J = 1.9, 3.6 Hz), 1.99 (4H, s), 1.37 (3H, d, J = 6.1 Hz) ppm.

### Example 130: (7R,13R)- or (7S,13R)-7,13-dimethyl-8,14-dioxa-5,10,19,20,23-penta azatetracyclo[13.5.2.1^{2,6}.0^{18,21}]tricosa-1(20),2,4,6(23),15,17,21-heptaen-9-one

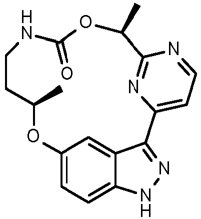

Example 130 is prepared according to the synthesis route described below.

### Preparation of intermediate 159: 4-chloro-2-iodopyrimidine

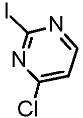

To a mixture of 4-chloropyrimidin-2-amine (50 g, 386.0 mmol), isopentyl nitrite (155.9 mL, 1157.9 mmol) and diiodomethane (139.9 mL, 1736.8 mmol) in 501 mL of THF, was added copper(I) iodide (73.5 g, 386.0 mmol) at room temperature. The mixture was stirred at 85°C for 6 hours. The mixture was filtered over a pad of celite and concentrated under vacuo. The mixture was diluted with EtOAc and washed with a 10% solution of sodium bisulfite (x2). The orgranic layers were dried over MgSO₄, filtered and concentrated udner reduced pressure. The product was purified by flash chromatography on silica gel, using as eluents heptane/ethyl acetate (100:0 to 80:20) to afford 4-chloro-2-iodopyrimidine **159** as a white solid.
LCMS method B: [M+H]⁺ = 240.9, t_{R} = 0.485 min

### Preparation of intermediate 160: 1-(4-chloropyrimidin-2-yl)ethan-1-ol

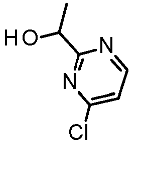

4-chloro-2-iodopyrimidine **159** (6.000 g, 24.955 mmol) was dissolved in 125 mL of dry THF under nitrogen atmosphere. The reaction was cooled at -78°C and methylmagnesium chloride (3 M in THF) (16.630 mL, 49.910 mmol) was added dropwise and the mixture was stirred at - 78°C for 1 hour. Acetaldehyde (4.185 mL, 74.865 mmol) was added to the mixture and the reaction was warmed at 0°C over 2.5 hours. The reaction was diluted with EtOAc and a sat. solution of NH₄Cl was added. The organic layer was separated, dried over MgSO₄, filtered and concentrated under reduced pressure. The crude was purified by flash chromatography on silica gel, using as eluents heptane/ethyl acetate (100:0 to 65:35) to afford 1-(4-chloropyrimidin-2-yl)ethan-1-ol **160** as a yellow oil.
LCMS method E: [M+H]⁺ = 159.0, t_{R} = 1.297 min

### Preparation of Example 130: (7R,13R)- or (7S,13R)-7,13-dimethyl-8,14-dioxa-5,10,19, 20,23-pentaazatetracyclo[13.5.2.1^{2,6}.0^{18,21}]tricosa-1(20),2,4,6(23),15,17,21-heptaen-9-one

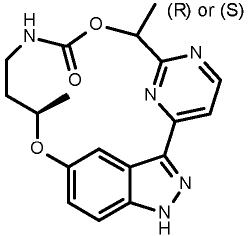

Example 130 is prepared via chiral HPLC separation of the two diastereoisomers. The chiral separation was done on a Column Waters XSELECT C18 19*100 mm, 5 µm, 35% to 40% MeCN in (NH₄)₂CO₃ aq. 2 g/L, 19 mL/min, RT.

To a solution of (13R)-7,13-dimethyl-19-(oxan-2-yl)-8,14-dioxa-5,10,19,20,23-pentaazatetra cyclo[13.5.2.1^{2,6}.0^{18,21}]tricosa-1(20),2,4,6(23),15,17,21-heptaen-9-one (710 mg, 1.62 mmol) in MeOH (27.8 mL) and water (4.6 mL) was added p-toluenesulfonic acid monohydrate (1.54 g, 8.12 mmol) and the reaction mixture was stirred at 80°C for 24 h. Then, to the reaction mixture was added p-toluenesulfonic acid monohydrate (308 mg, 1.62 mmol) and stirred for 2h at 80°C. The reaction mixture was evaporated under vacuo and the residue was dissolved in EtOAc (50 mL) then a saturated aqueous solution of sodium hydrogen carbonate (50 mL) was added. After separation, the aqueous layer was extracted with ethyl acetate (2x). The combined organic layer was washed with brine, dried over sodium sulfate, filtered and evaporated under reduced pressure to give the (7R,13R)- or (7S,13R)-7,13-dimethyl-8,14-dioxa-5,10,19,20,23-pentaazatetracyclo[13.5.2.1^{2,6}.0^{18,21}]tricosa-1(20),2,4, 6(23),15,17,21-heptaen-9-one **example 130** as a white solid.
LCMS method F: [M+H]⁺ = 354, t_{R} = 1.92 min
LCMS method G: [M+H]⁺ = 354, t_{R} = 1.92 min
¹H NMR (400 MHz, *d*6-DMSO) δ 13.63 (1H, m), 8.78 (1H, d, J = 5.3 Hz), 8.03 (1H, dd, J = 0.7, 5.2 Hz), 7.89 - 7.86 (2H, m), 7.55 - 7.52 (1H, m), 7.00 (1H, dd, J = 2.5, 8.9 Hz), 5.77 (1H, q, J = 6.8 Hz), 4.63 - 4.57 (1H, m), 3.58 - 3.50 (1H, m), 2.89 - 2.80 (1H, m), 2.38 - 2.29 (1H, m), 1.67 (3H, d, J = 7.0 Hz), 1.40 - 1.37 (4H, m) ppm.

### Example 131: (7R,13R)- or (7S,13R)-7,13-dimethyl-8,14-dioxa-5,10,19,20,23-penta azatetracyclo[13.5.2.1^{2,6}.0^{18,21}]tricosa-1(20),2,4,6(23),15,17,21-heptaen-9-one

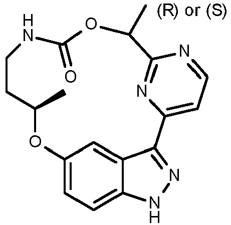

Example 131 is prepared according to the synthesis route described in general Scheme K and chiral HPLC separation of the two diastereoisomers. The chiral separation was done on a Column Waters XSELECT C18 19*100 mm, 5 µm, 35% to 40% MeCN in (NH₄)₂CO₃ aq. 2 g/L, 19 mL/min, RT.

The fractions for the second batch was evaporated in vacuo to afford (7R,13R)- or (7S,13R)-7,13-dimethyl-8,14-dioxa-5,10,19,20,23-pentaazatetracyclo[13.5.2.1^{2,6}.0^{18,21}]tricosa-1(20),2,4,6(23),15,17,21-heptaen-9-one with a 90% of purity (contained 5 % of the other diastereoisomer). So the powder was put in acetonitrile for a recrystallization, the solid obtained was filtered to afford the pure (7R,13R)- or (7S,13R)-7,13-dimethyl-8,14-dioxa-5,10,19,20,23-pentaazatetracyclo[13.5.2.1^{2,6}.0^{18,21}]tricosa-1(20),2,4,6(23),15,17,21-heptaen-9-one **example 131** as a white powder.
LCMS method F: [M+H]⁺ = 354, t_{R} = 2.03 min
LCMS method G: [M+H]⁺ = 354, t_{R} = 2.02 min
¹H NMR (400 MHz, *d*6-DMSO) δ 13.62 (1H, s), 8.81 - 8.78 (1H, m), 8.02 - 7.98 (2H, m), 7.60 - 7.49 (2H, m), 7.04 - 6.97 (1H, m), 6.10 (1H, q, J = 6.7 Hz), 4.93 - 4.85 (1H, m), 3.25 (1H, m), 3.05 - 2.97 (1H, m), 1.85 - 1.77 (1H, m), 1.71 - 1.51 (4H, m), 1.41 - 1.38 (3H, m) ppm.

### Example 132: (13R)-16-fluoro-13-methyl-8,14-dioxa-10,19,20-triazatetracyclo [13.5.2.1^{2,6}.0^{18,21}]tricosa-1(20),2,4,6(23),15,17,21-heptaen-9-one

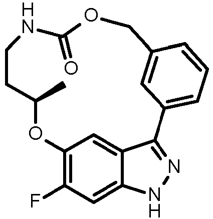

Example 132 is prepared according to the synthesis route described in general Scheme C.

To a solution of (13R)-16-fluoro-13-methyl-19-(oxan-2-yl)-8,14-dioxa-10,19,20-triazatetracyclo[13.5.2.1^{2,6}.0^{18,21}]tricosa-1(20),2,4,6(23),15,17,21-heptaen-9-one (284 mg, 0.65 mmol) in MeOH (11 mL) and water (2 mL) was added p-toluenesulfonic acid monohydrate (615 mg, 3.23 mmol) and the reaction mixture was stirred at 80°C for 12 h. The reaction mixture was evaporated under vacuo and the residue was dissolved in EtOAc (50 mL) then a saturated aqueous solution of sodium hydrogen carbonate (50 mL) was added. After separation, the aqueous layer was extracted with ethyl acetate (2x). The combined organic layer was washed brine, dried over sodium sulfate, filtered and evaporated under reduced pressure to give an oil. The residue was put in acetonitrile, a solid appeared and was filtered to afford (13R)-16-fluoro-13-methyl-8,14-dioxa-10,19,20-triazatetracyclo[13.5.2.1^{2,6}.0^{18,21}]tricosa-1(20),2,4,6(23), 15,17,21-heptaen-9-one **example 132** as a white solid.
LCMS method F: [M+H]⁺ = 356, t_{R} = 2.30 min
LCMS method G: [M+H]⁺ = 356, t_{R} = 2.38 min
¹H NMR (400 MHz, *d*6-DMSO) δ 13.23 - 13.20 (1H, m), 7.93 (1H, dd, J = 4.9, 7.0 Hz), 7.86 - 7.80 (2H, m), 7.50 - 7.45 (2H, m), 7.39 - 7.36 (1H, m), 7.31 - 7.28 (1H, m), 5.75 (1H, d, J = 12.9 Hz), 4.82 (1H, d, J = 12.0 Hz), 4.66 - 4.59 (1H, m), 3.59 - 3.54 (1H, m), 2.95 - 2.87 (1H, m), 2.40 - 2.33 (1H, m), 1.45 - 1.42 (4H, m) ppm.

### Example 133: (13R)-13-methyl-8,14-dioxa-4,10,19,20,23-pentaazatetracyclo [13.5.2.1^{2,6}.0^{18,21}]tricosa-1(20),2,4,6(23),15,17,21-heptaen-9-one

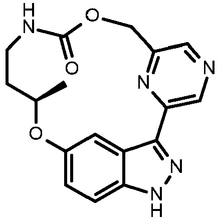

Example 133 is prepared according to the synthesis route described in general Scheme K.

To a solution of (13R)-13-methyl-19-(oxan-2-yl)-8,14-dioxa-4,10,19,20,23-pentaazatetra cyclo[13.5.2.1^{2,6}.0^{18,21}]tricosa-1(20),2,4,6(23),15,17,21-heptaen-9-one (413 mg, 0.98 mmol) in MeOH (16.8 mL) and water (2.8 mL) was added p-toluenesulfonic acid monohydrate (931 mg, 4.88 mmol) and the reaction mixture was stirred at 65°C overnight. The reaction mixture was diluted with DCM and with a saturated aqueous solution of sodium hydrogen carbonate. After separation, the aqueous layer was extracted with DCM (3x). The combined organic layer was washed brine, dried over sodium sulfate, filtered and evaporated under reduced pressur to give an yellow oil. This oil was triturated in ACN and a cream precipitate was observed. The mixture was filtered and washed with ACN (2 x) the cream solid was dried for 1 night to give (13R)-13-methyl-8,14-dioxa-4,10,19,20,23-pentaazatetracyclo[13.5.2.1^{2,6}.0^{18,21}]tricosa-1(20),2,4,6(23),15,17,21-heptaen-9-one **example 133** as a cream solid.
LCMS method F: [M+H]⁺ = 340, t_{R} = 1.92 min
LCMS method G: [M+H]⁺ = 340, t_{R} = 1.98 min
¹H NMR (400 MHz, *d*6-DMSO) δ 13.51 (1H, m), 9.27 (1H, s), 8.52 (1H, s), 7.83 (2H, m), 7.52 (1H, d, J = 8.9 Hz), 6.99 (1H, dd, J = 2.3, 8.9 Hz), 5.69 (1H, d, J = 15.0 Hz), 5.16 (1H, d, J = 14.8 Hz), 4.60 (1H, m), 3.49 (1H, m), 2.91 (1H, t, J = 11.4 Hz), 2.32 (1H, t, J = 11.8 Hz), 1.43 (1H, m), 1.38 (3H, d, J = 5.9 Hz) ppm.

### Example 134: 8,14-dioxa-4-thia-10,19,20,23-tetraazatetracyclo[13.5.2.1^{2,5}.0^{18,21}]tricosa-1(20),2,5(23),15,17,21-hexaen-9-one

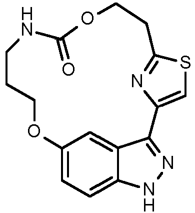

Example 134 is prepared according to the synthesis route described in general Scheme L.

### Preparation of intermediate 161: 2-(4-bromothiazol-2-yl)ethyl N-(3-hydroxypropyl) carbamate

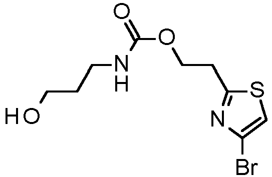

To a solution of 4-nitrophenyl chloroformate (0.426 g, 2.11 mmol) and pyridine (0.311 mL, 3.84 mmol) in DCM (10 mL) was added dropwise at RT 2-(4-bromothiazol-2-yl)ethanol (0.400 g, 1.92 mmol) in DCM (5 mL). After 1 h at RT, a mixture of 3-aminopropan-1-ol (0.159 g, 2.11 mmol) and DIPEA (0.668 mL, 3.84 mmol) in DCM (5 mL) was added. The resulting reaction mixture was stirred at RT overnight. The residue was diluted with 0.5 N NaOH solution and extracted with DCM twice. The combined organic layer was washed once again with 0.5 N NaOH solution and dried over anhydrous sodium sulfate and concentrated in vacuo. The residue was purified by flash-column (25 g silica Macherey Nagel) chromatography (DCM - ethyl acetate 1:0 to 3:7) affording 2-(4-bromothiazol-2-yl)ethyl N-(3-hydroxypropyl)carbamate **161** as a colorless oil.
LCMS method F: [M+H]⁺ = 309, t_{R} = 1.65 min

### Preparation of intermediate 162: 2-[4-[5-[tert-butyl(dimethyl)silyl]oxy-1-tetrahydropyran-2-yl-indazol-3-yl]thiazol-2-yl]ethyl N-(3-hydroxypropyl)carbamate

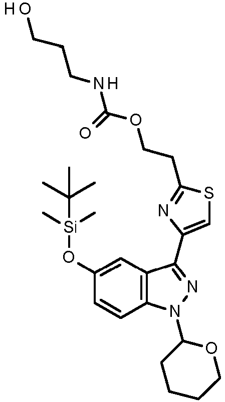

To a solution of tert-butyl-dimethyl-[1-tetrahydropyran-2-yl-3-(4,4,5,5-tetramethyl-1,3,2-dioxaborolan-2-yl)indazol-5-yl]oxy-silane (0.700 g, 1.53 mmol) in dioxane (15 mL) and water (1.5 mL) were added at RT 2-(4-bromothiazol-2-yl)ethyl N-(3-hydroxypropyl)carbamate 161 (0.472 g, 1.53 mmol), K₃PO₄ (0.972 g, 4.58 mmol), XPhos (0.073 g, 0.15 mmol) and Pd(PPh₃)₄ (0.088 g, 0.08 mmol). The resulting reaction mixture was stirred under microwave irradition at 90°C for 1 h. The residue was diluted with saturated sodium chloride solution and extracted with ethyl acetate twice. The combined organic layer was dried over anhydrous sodium sulfate and concentrated in vacuo. The residue was purified by flash-column (25 g silica Macherey Nagel) chromatography (cyclohexane - ethyl acetate, 1:0 to 0:1) affording 2-[4-[5-[tert-butyl(dimethyl)silyl]oxy-1-tetrahydropyran-2-yl-indazol-3-yl]thiazol-2-yl]ethyl N-(3-hydroxy propyl)carbamate **162** as a colorless oil.
LCMS method F: [M+H]⁺ = 561.3, t_{R} = 3.29 min

### Preparation of intermediate 163: 3-[2-[4-[5-[tert-butyl(dimethyl)silyl]oxy-1-tetrahydropyran-2-yl-indazol-3-yl]thiazol-2-yl]ethoxycarbonylamino]propyl methanesulfonate

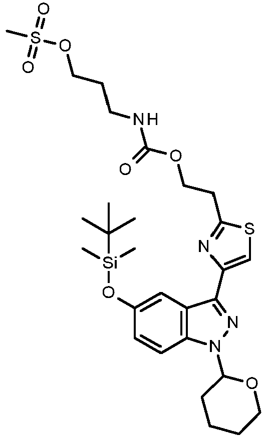

To a solution of 2-[4-[5-[tert-butyl(dimethyl)silyl]oxy-1-tetrahydropyran-2-yl-indazol-3-yl]thiazol-2-yl]ethyl N-(3-hydroxypropyl)carbamate **162** (0.540 g, 0.96 mmol) and triethylamine (0.268 mL, 1.93 mmol) in DCM (18 mL) was added at 0°C methanesulfonyl chloride (0.097 mL, 1.25 mmol) in DCM (2 mL). The reaction mixture was stirred at RT for 2h. The residue was diluted with saturated sodium chloride solution and extracted with DCM twice. The combined organic layer was dried over anhydrous sodium sulfate and concentrated in vacuo affording 3-[2-[4-[5-[tert-butyl(dimethyl)silyl]oxy-1-tetrahydropyran-2-yl-indazol-3-yl]thiazol-2-yl]ethoxycarbonylamino]propyl methanesulfonate **163** as a yellow oil, which was used in the next step without further purification.
LCMS method F: [M+H]⁺ = 639.3, t_{R} = 3.41 min

### Preparation of intermediate 164: 3-[2-[4-(5-hydroxy-1-tetrahydropyran-2-yl-indazol-3-yl)thiazol-2-yl]ethoxycarbonylamino]propyl methanesulfonate

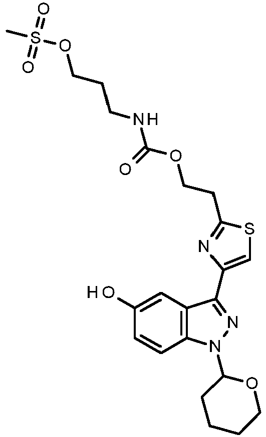

To a solution of 3-[2-[4-[5-[tert-butyl(dimethyl)silyl]oxy-1-tetrahydropyran-2-yl-indazol-3-yl]thiazol-2-yl]ethoxycarbonylamino]propyl methanesulfonate **163** (0.615 g, 0.96 mmol) in THF (10 mL) was added at -10°C tetrabutylammonium fluoride 1 M in THF (1.06 mL, 1.06 mmol). The resulting reaction mixture was stirred at -10°C for 5 min. The reaction mixture was diluted with saturated ammonium chloride solution and extracted with ethyl acetate twice. The combined organic layer was dried over anhydrous sodium sulfate and concentrated in vacuo affording 3-[2-[4-(5-hydroxy-1-tetrahydropyran-2-yl-indazol-3-yl)thiazol-2-yl]ethoxy carbonylamino]propyl methanesulfonate **164** as a yellow oil, which was used in the next step without further purification.
LCMS method F: [M+H]⁺ = 525.1, t_{R} = 2.34 min

### Preparation of intermediate 165: 19-(oxan-2-yl)-8,14-dioxa-4-thia-10,19,20,23-tetraazatetracyclo[13.5.2.1^{2,5}.0^{18,21}]tricosa-1(20),2,5(23),15(22),16,18(21)-hexaen-9-one

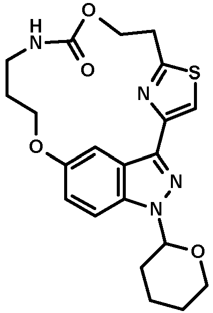

To a suspension of cesium carbonate (0.941 g, 2.89 mmol) in anhydrous *N,N-*dimethylformamide (192 mL) at 80°C was added dropwise (during 2 h) 3-[2-[4-(5-hydroxy-1-tetrahydropyran-2-yl-indazol-3-yl)thiazol-2-yl]ethoxycarbonylamino]propyl methanesulfonate **164** (0.505 g, 0.96 mmol) in *N,N*-dimethylformamide (192 mL). After addition the resulting reaction mixture was stirred at 80°C for 2h. The reaction mixture was filtered and concentrated in vacuo, diluted with saturated sodium chloride solution and extracted with ethyl acetate twice. The combined organic layer was dried over anhydrous sodium sulfate and concentrated in vacuo. The residue was purified by flash-column (25 g silica Macherey Nagel) chromatography (DCM - ethyl acetate, 1:0 to 7:3) affording 19-(oxan-2-yl)-8,14-dioxa-4-thia-10,19,20,23-tetraazatetracyclo[13.5.2.1^{2,5}.0^{18,21}]tricosa-1(20),2,5(23),15(22),16,18(21)-hexaen-9-one **165** as a white solid.
LCMS method F: [M+H]⁺ = 429.4, t_{R} = 2.45 min

### Preparation of Example 134: 8,14-dioxa-4-thia-10,19,20,23-tetraazatetracyclo [13.5.2.1^{2,5}.0^{18,21}]tricosa-1(20),2,5(23),15,17,21-hexaen-9-one

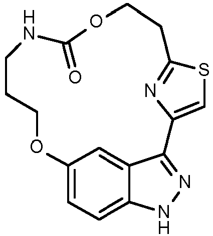

To a suspension of 19-(oxan-2-yl)-8,14-dioxa-4-thia-10,19,20,23-tetraazatetracyclo [13.5.2.1^{2,5}.0^{18,21}]tricosa-1(20),2,5(23),15(22),16,18(21)-hexaen-9-one **165** (0.150 g, 0.35 mmol) in MeOH (17.5 mL) and water (2.5 mL) was added p-toluenesulfonic acid monohydrate (0.333 g, 1.75 mmol) and the reaction mixture was stirred at 80°C for 4 h. The reaction mixture was concentrated under vacuo and the residue was neutralized by addition of saturated aqueous solution of sodium hydrogen carbonate. The residue was diluted with EtOAc. The phases were separated and the aqueous phase was extracted with ethyl acetate. The combined organic layer was washed with sodium hydrogen carbonate, brine, dried over sodium sulfate, filtered and evaporated under reduced pressure. The solid was recrystallized in acetonitrile to give 8,14-dioxa-4-thia-10,19,20,23-tetraazatetracyclo[13.5.2.1^{2,5}.0^{18,21}]tricosa-1(20),2,5(23),15,17,21-hexaen-9-one **example 134** as a white solid.
LCMS method F: [M+H]⁺ = 345.2, t_{R} = 1.79 min
LCMS method G: [M+H]⁺ = 345.2, t_{R} = 1.78 min, [M-H]⁻ = 343.1, t_{R} = 1.78 min
¹H NMR (400 MHz, *d*6-DMSO) δ 13.05 - 13.03 (1H, m), 7.83 (1H, s), 7.59 - 7.51 (2H, m), 7.46 - 7.43 (1H, m), 6.95 (1H, dd, J = 2.4, 9.0 Hz), 4.42 (2H, t, J = 5.2 Hz), 4.27 - 4.22 (2H, m), 3.48 (2H, dd, J = 4.6, 5.8 Hz), 3.10 - 3.05 (2H, m), 1.82 (2H, d, J = 8.2 Hz) ppm.

### Example 135: 8,14-dioxa-3-thia-10,19,20,23-tetraazatetracyclo[13.5.2.1^{2,5}.0^{18,21}]tricosa-1(20),2(23),4,15,17,21-hexaen-9-one

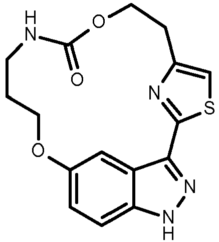

Example 135 is prepared according to the synthesis route described in general Scheme L.

### Preparation of intermediate 166: 2-(2-bromothiazol-4-yl)ethyl N-(3-hydroxypropyl) carbamate

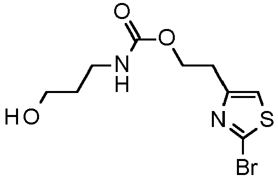

To a solution of 4-nitrophenyl chloroformate (0.533 g, 2.64 mmol) and pyridine (0.388 mL, 4.81 mmol) in DCM (10 mL) was added dropwise at RT 2-(2-bromothiazol-4-yl)ethanol (0.500 g, 2.40 mmol) in DCM (5 mL). After 1 h at RT, a mixture of 3-aminopropan-1-ol (0.198 g, 2.64 mmol) and DIPEA (0.835 mL, 4.81 mmol) in DCM (5 mL) was added. The resulting reaction mixture was stirred at RT overnight. The residue was diluted with 0.5 N NaOH solution and extracted with DCM twice. The combined organic layer was washed once again with 0.5 N NaOH solution and dried over anhydrous sodium sulfate and concentrated in vacuo. The residue was purified by flash-column (25 g silica Macherey Nagel) chromatography (DCM - ethyl acetate 1:0 to 2:8) affording 2-(2-bromothiazol-4-yl)ethyl N-(3-hydroxypropyl)carbamate **166** as a colorless oil.
LCMS method F: [M+H]⁺ = 309, t_{R} = 1.60 min

### Preparation of Example 135: 8,14-dioxa-3-thia-10,19,20,23-tetraazatetracyclo [13.5.2.1^{2,5}.0^{18,21}]tricosa-1(20),2(23),4,15, 17,21-hexaen-9-one

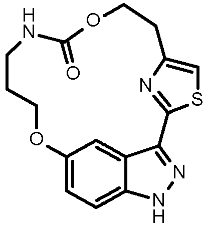

To a solution of 19-(oxan-2-yl)-8,14-dioxa-3-thia-10,19,20,23-tetraazatetracyclo[13 .5.2.1^{2,5}.0^{18,21}]tricosa-1(20),2(23),4,15,17,21-hexaen-9-one (0.360 g, 0.84 mmol) in MeOH (150 mL) and water (5 mL) was added p-toluenesulfonic acid monohydrate (0.799 g, 4.20 mmol) and the reaction mixture was stirred at 80°C for 4 days. The reaction mixture was concentrated under vacuo and the residue was neutralized by addition of saturated aqueous solution of sodium hydrogen carbonate. The residue was diluted with EtOAc. The phases were separated and the aqueous phase was extracted with ethyl acetate. The combined organic layer was washed with sodium hydrogen carbonate, brine, dried over sodium sulfate, filtered and evaporated under reduced pressure. The solid was triturated in acetonitrile to give 8,14-dioxa-3-thia-10,19,20,23-tetraazatetracyclo[13.5.2.1^{2,5}.0^{18,21}]tricosa-1(20),2(23),4,15, 17,21-hexaen-9-one **example 135** as a white solid.
LCMS method F: [M+H]⁺ = 345.2 t_{R} = 1.87 min
LCMS method G: [M+H]⁺ = 345.2, t_{R} = 1.85 min, [M-H]⁻ = 343.1, t_{R} = 1.85 min
¹H NMR (400 MHz, *d*6-DMSO) δ 13.35 (1H, s), 7.69 - 7.67 (1H, m), 7.56 - 7.49 (2H, m), 7.35 (1H, s), 7.01 (1H, dd, J = 2.4, 9.0 Hz), 4.38 - 4.26 (4H, m), 3.18 - 3.05 (4H, m), 1.84 (2H, ddd, J = 0.8, 8.9, 17.4 Hz) ppm.

### Example 136: (7R,13R)-7,13-dimethyl-8,14-dioxa-10,19,20,23-tetraazatetracyclo [13.5.2.1^{2,6}.0^{18,21}]tricosa-1(20),2,4,6(23),15,17,21-heptaen-9-one

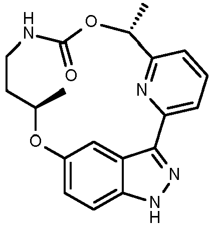

Example 136 is prepared according to the synthesis route described in general Scheme K.

### Preparation of intermediate 167: [(1R)-1-(6-bromo-2-pyridyl)ethyl] acetate

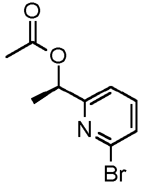

To a stirred solution of 1-(6-bromo-2-pyridyl)ethanol (1 g, 4.94 mmol, 1 eq) in anhydrous diisopropyl ether (120 mL) at 0°C, was added vinyl acetate (2 mL, 21 mmol, 42 eq), 4A molecular sieves (1 g), and Lipase immobilized from *Candida Antarctica* (200 mg) and the reaction mixture was stirred at room temperature for 48 hours. The catalyst and molecular sieves were filtered off and the solvent was concentrated under reduced pressure. The oily residue was purified by column chromatography eluting with cyclohexane/EtOAc : 90/10 to 80/20 to give [(1R)-1-(6-bromo-2-pyridyl)ethyl] acetate **167** as colorless oil.
LCMS method F: [M+H]⁺ = 246, t_{R} = 2.28 min

### Preparation of intermediate 168: (1R)-1-(6-bromo-2-pyridyl)ethanol

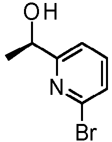

To a solution of [(1R)-1-(6-bromo-2-pyridyl)ethyl] acetate **167** (563 mg, 2.3 mmol, 1 eq) in a mixture of MeOH/water: 1/1 (20 mL) was added potassium carbonate (317 mg, 2.3 mmol, 1 eq) and the solution was stirred at room temperature for 3 hours. MeOH was then evaporated and the aqueous phase was extracted with EtOAc (3x). The combined organic extract was washed with brine, dried over Na₂SO₄, filtered and evaporated uner reduced pressure to give the expected compound (1R)-1-(6-bromo-2-pyridyl)ethanol **168** as a colorless oil.
LCMS method F: [M+H]⁺ = 202, t_{R} = 1.69 min

### Preparation of Example 136: (7R,13R)-7,13-dimethyl-8,14-dioxa-10,19,20,23-tetraazatetracyclo[13.5.2.1^{2,6}.0^{18,21}]tricosa-1(20),2,4, 6(23),15,17,21-heptaen-9-one

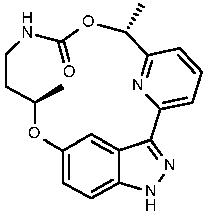

To a suspension of (7R,13R)-7,13-dimethyl-19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one (80 mg, 0.18 mmol, 1 eq) in MeOH/water (14 mL / 2 mL) was added para-toluenesulfonic acid monohydrate (174 mg, 0.91 mmol, 5 eq) and the reaction mixture was heated at 65°C for 24 hours. MeOH was removed partially under reduced pressure and a saturated solution of NaHCO₃ was added. The aqueous phase was extracted with EtOAc (2x) and the organic extract was washed with brine, dried over Na₂SO₄, filtered and evaporated under reduced pressure. The solid residue was triturated in diisopropyl ether, filtered and dried to give (7R,13R)-7,13-dimethyl-8,14-dioxa-10,19,20,23-tetraazatetracyclo[13.5.2.1^{2,6}.0^{18,21}]tricosa-1(20),2,4, 6(23),15,17,21-heptaen-9-one **example 136** as a white solid.
LCMS method F: [M+H]⁺ = 353.2, t_{R} = 2.28 min
LCMS method G: [M+H]⁺ = 353.3, t_{R} = 2.15 min
¹H NMR (400 MHz, *d*6-DMSO, 80°C) δ 12.93 - 12.90 (1H, m), 8.11 - 8.05 (2H, m), 7.85 (1H, t, J = 7.7 Hz), 7.46 - 7.42 (1H, m), 7.34 - 7.29 (1H, m), 7.22-7.08 (1H, m), 6.97 - 6.94 (1H, m), 6.2-5.9 (1H, m), 5.05-4.8 (1H, m), 3.4-3.2 (1H, m), 3-2.95 (1H, m), 1.94-1.73 (1H, m), 1.72-1.51 (4H, m), 1.40 (3H, d, J = 6.1 Hz) ppm.

### Example 137: (13R)-4-[(3R)-3-methoxypyrrolidin-1-yl]-13-methyl-8,14-dioxa-5,10,19,20, 23-pentaazatetracyclo[13.5.2.1^{2,6}.0^{18,21}]tricosa-1(20),2,4,6(23),15,17,21-heptaen-9-one

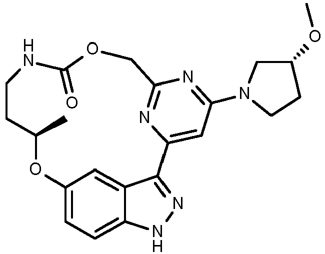

Example 137 is prepared according to the synthesis route described in general Scheme K.

### Preparation of intermediate 169: (4,6-dichloropyrimidin-2-yl)methyl acetate

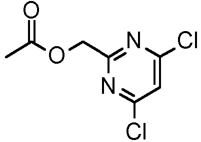

To a solution of 4,6-dichloro-2-(chloromethyl)pyrimidine (0.830 g, 4.23 mmol) in *N,N-*dimethylformamide (50.0 mL) at 0°C was added potassium iodide (0.774 g, 4.66 mmol), and potassium acetate (0.457 g, 4.66 mmol) was added and the mixture was stirred during 12 hours at room temperature. Solvent was evaporated. Water (25 mL) and EtOAc (25 mL) were added. Phases were separated and aqueous phase was extracted with EtOAc (3 x 25 mL), then organic phase was washed with a saturated solution of NaCl (25 mL), dried with Na₂SO₄, filtered and concentrated to afford (4,6-dichloropyrimidin-2-yl)methyl acetate **169** as a white powder.
LCMS method F: [M+H]⁺ = 221, t_{R} = 2.07 min

### Preparation of intermediate 170: [4-[5-[tert-butyl(dimethyl)silyl]oxy-1-tetrahydropyran-2-yl-indazol-3-yl]-6-chloro-pyrimidin-2-yl]methyl acetate

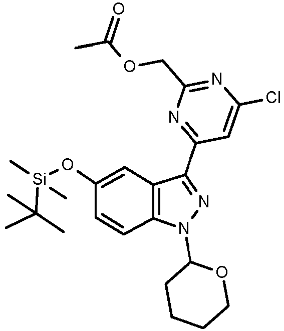

To a solution of tert-butyl-dimethyl-[1-tetrahydropyran-2-yl-3-(4,4,5,5-tetramethyl-1,3,2-dioxaborolan-2-yl)indazol-5-yl]oxy-silane (2.5 g, 5.45 mmol) in dioxane (10 mL) and water (1 mL) was added at RT (4,6-dichloropyrimidin-2-yl)methyl acetate 169 (999 mg, 4.54 mmol), Potassium phosphate tribasic (2.89 g, 13.62 mmol). The reaction mixture was degassed by bubbling nitrogen for 15 min, then XPhos (65 mg, 0.14 mmol) and Pd(PPh₃)₄ (53 mg, 0.045 mmol) were added. The reaction mixture was stirred at 80°C for 45 min under microwave radiations. The reaction mixture was evaporated in vacuo to give a brown oil. To the reaction mixture was added EtOAc (100 mL) and water (50 mL). After separation, the aqueous layer was extracted with EtOAc (2x50 mL), then the organic layer was washed with brine, dried over sodium sulfate, filtered and evaporated in vacuo. The residue was purified by flash chromatography (CyH/EtOAc 100 to 8/2 CyH/EtOAc) to afford [4-[5-[tert-butyl(dimethyl)silyl]oxy-1-tetrahydropyran-2-yl-indazol-3-yl]-6-chloro-pyrimidin-2-yl]methyl acetate **170** as an yellow oil.
LCMS method F: [M+H]⁺ = 517, t_{R} = 3.91 min

### Preparation of intermediate 171: [4-chloro-6-(5-hydroxy-1-tetrahydropyran-2-yl-indazol-3-yl)pyrimidin-2-yl]methyl acetate

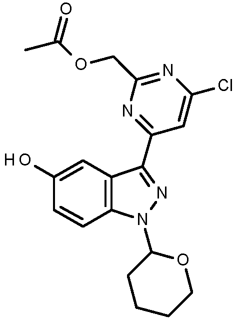

To a solution of [4-[5-[tert-butyl(dimethyl)silyl]oxy-1-tetrahydropyran-2-yl-indazol-3-yl]-6-chloro-pyrimidin-2-yl]methyl acetate **170** (2.34 g, 4.53 mmol) in THF (40 mL) was added dropwise at RT tetrabutylammonium fluoride 1 M in THF (1.3 mL, 4.98 mmol). The resulting reaction mixture was stirred at RT overnight. The reaction mixture was poured into ice water and stirred for 20 min. The aqueous phase was extracted with ethyl acetate (100 mL) twice and the combined organic phase was washed with saturated brine, dried over anhydrous sodium sulfate and concentrated in vacuo to give a brown oil. The residue was purified by flash chromatography CyH/EtOAc 5/5 to afford [4-chloro-6-(5-hydroxy-1-tetrahydropyran-2-yl-indazol-3-yl)pyrimidin-2-yl]methyl acetate **171** as a white solid.
LCMS method F: [M+H]⁺ = 403, t_{R} = 2.82 min

### Preparation of intermediate 172: benzyl [4-[5-[(1R)-3-(benzyloxycarbonylamino)-1-methyl-propoxyl-1-tetrahydropyran-2-yl-indazol-3-yl]-6-chloro-pyrimidin-2-yl]methyl acetate

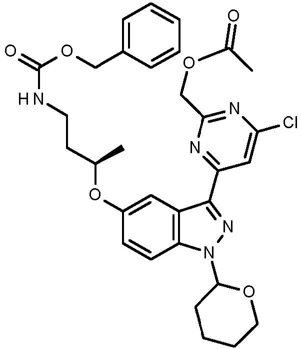

To a mixture of [4-chloro-6-(5-hydroxy-1-tetrahydropyran-2-yl-indazol-3-yl)pyrimidin-2-yl]methyl acetate **171** (945 mg, 2.35 mmol) and cesium carbonate (1.53 g, 4.7 mmol) in acetonitrile (20 mL) and *N,N*-dimethylformamide (5 mL) was added [(1S)-3-(benzyloxycarbonylamino)-1-methyl-propyl] methanesulfonate (778 mg, 2.59 mmol postulated) and the suspension was heated for 8 hours at RT. The reaction mixture was filtered to removed cesium carbonate and the salts were washed with acetonitrile. The filtrate was evaporated in vacuo to give an yellow oil. The oily residue was put in EtOAc (50 mL) and water (30 mL), the aqueous layer was extracted with EtOAc (2x50 mL), washed with brine, dried over sodium sulfate, filtered and evaporated in vacuo to give an oil. The residue was purified by flash chromatography (CyH/AE 5/5) to afford benzyl [4-[5-[(1R)-3-(benzyloxycarbonylamino)-1-methyl-propoxy]-1-tetrahydropyran-2-yl-indazol-3-yl]-6-chloro-pyrimidin-2-yl]methyl acetate **172** as an off-white solid.
LCMS method I: [M+H]⁺ = 608, t_{R} = 2.86 min

### Preparation of intermediate 173: [4-[5-[(1R)-3-(benzyloxycarbonylamino)-1-methyl-propoxy]-1-tetrahydropyran-2-yl-indazol-3-yl]-6-[(3R)-3-methoxypyrrolidin-1-yl]pyrimidin-2-yl]methyl acetate

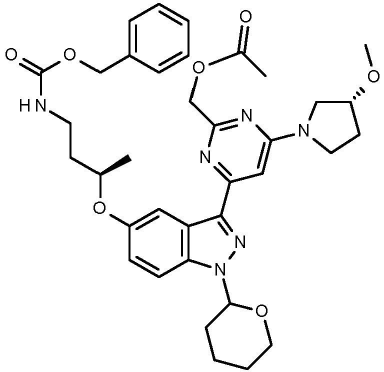

To a mixture of [4-[5-[(1R)-3-(benzyloxycarbonylamino)-1-methyl-propoxy]-1-tetrahydropyran-2-yl-indazol-3-yl]-6-chloro-pyrimidin-2-yl]methyl acetate **172** (103 mg, 0.17 mmol) and potassium carbonate (94 mg, 0.37 mmol) in *N,N-*dimethylformamide (1.5 mL) was added (3R)-3-methoxypyrrolidine hydrochloride (51 mg, 0.37 mmol) and the suspension was stirred for 8 hours at RT. The reaction mixture was evaporated in vacuo to give an yellow oil. Then to the residue was added EtOAc (20 mL) and water (10 mL). After separation of the layers, the aqueous layer was extracted with EtOAc (10 mL) twice, then the organic layer was washed with brine, dried over sodium sulfate, filtered and evaporated in vacuo to afford the crude [4-[5-[(1R)-3-(benzyloxycarbonylamino)-1-methyl-propoxy]-1-tetrahydropyran-2-yl-indazol-3-yl]-6-[(3R)-3-methoxypyrrolidin-1-yl]pyrimidin-2-yl]methyl acetate **173** as an yellow oil.
LCMS method F: [M+H]⁺ = 673, t_{R} = 2.89 min

### Preparation of intermediate 174: benzyl N-[(3R)-3-[3-[2-(hydroxymethyl)-6-pyrrolidin-1-yl-pyrimidin-4-yl]-1-tetrahydropyran-2-yl-indazol-5-yl]oxybutyl[carbamate

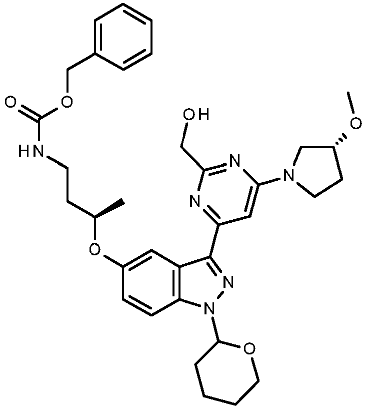

To a suspension of [4-[5-[(1R)-3-(benzyloxycarbonylamino)-1-methyl-propoxy]-1-tetrahydropyran-2-yl-indazol-3-yl]-6-[(3R)-3-methoxypyrrolidin-1-yl]pyrimidin-2-yl]methyl acetate **173** (0.155 g, 0.23 mmol) in MeOH (2.0 mL) was added potassium carbonate (0.038 g, 0.28 mmol). The reaction was stirred at room temperature during 2 h. The reaction was stopped and filtered and evaporated. The crude was purified by chromatography column (RediSep, 4 g, CyH/EtOAc : 0% to 100% EtOAc) to afford benzyl N-[(3R)-3-[3-[2-(hydroxymethyl)-6-pyrrolidin-1-yl-pyrimidin-4-yl]-1-tetrahydropyran-2-yl-indazol-5-yl]oxybutyl]carbamate **174** as a light yellow oil.
LCMS method F: [M+H]⁺ = 631, t_{R} = 2.23 min

### Preparation of intermediate 175: (13R)-4-[(3R)-3-methoxypyrrolidin-1-yl]-13-methyl-19-(oxan-2-yl)-8,14-dioxa-5,10,19,20,23-pentaazatetracyclo[13.5.2.1^{2,6}.0^{18,2}]tricosa-1(20),2(23),3,5,15(22),16,18(21)-heptaen-9-one

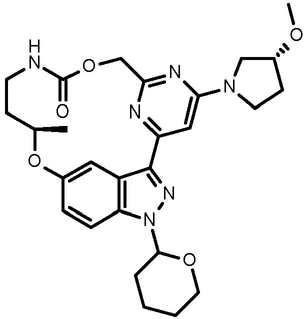

To a solution of benzyl N-[(3R)-3-[3-[2-(hydroxymethyl)-6-[(3R)-3-methoxypyrrolidin-1-yl]pyrimidin-4-yl]-1-tetrahydropyran-2-yl-indazol-5-yl]oxybutyl]carbamate **174** (121 mg, 0.19 mmol) in dry acetonitrile (10 mL) was added at RT finely powdered potassium hydroxide (54 mg, 0.96 mmol) in one portion. The reaction mixture was stirred at RT for 12 h. The reaction mixture was filtered then rinsed with ethyl acetate and evaporated under reduced pressure to give (13R)-4-[(3R)-3-methoxypyrrolidin-1-yl]-13-methyl-19-(oxan-2-yl)-8,14-dioxa-5,10,19,20,23-pentaazatetracyclo[13.5.2.1^{2,6}.0^{18,2 1}]tricosa-1(20),2(23),3,5,15(22),16,18(21)-heptaen-9-one **175** as a light yellow oil.
LCMS method F: [M+H]⁺ = 523, t_{R} = 2.68 min

### Preparation of Example 137 : (13R)-4-[(3R)-3-methoxypyrrolidin-1-yl]-13-methyl-8,14-dioxa-5,10,19,20,23-pentaazatetracyclo[13.5.2.1^{2,6}.0^{18,21}]tricosa-1(20),2,4,6(23),15,17,21-heptaen-9-one

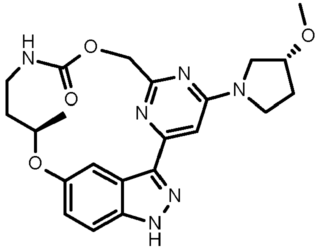

To a solution of (13R)-4-[(3R)-3-methoxypyrrolidin-1-yl]-13-methyl-19-(oxan-2-yl)-8,14-dioxa-5,10,19,20,23-pentaazatetracyclo[13.5.2.1^{2,6}.0^{18,21}]tricosa-1(20),2(23),3,5,15(22),16,18(21)-heptaen-9-one **175** (53 mg, 0.10 mmol) in MeOH/H₂O (2/0.3 mL) was added *p*-toluenesulfonic acid monohydrate (95 mg, 0.5 mmol). The mixture was heated at 80 °C for 5 hours. Then to the reaction mixture was added p-toluenesulfonic acid monohydrate (50 mg, 0.26 mmol) and stirred at 80 °C for 5 hours. The reaction mixture was evaporated in vacuo and a saturated solution of sodium bicarbonate (10 mL) and DCM (10 mL) was added. Then, the aqueous layer was extracted with DCM (2x10 mL), then the organic layer was washed with brine, dried over sodium sulfate, filtered and evaporated in vacuo to give a solid. The solid was purified by flash chromatography (CyH to 100% EtOAc) to afford (13R)-4-[(3R)-3-methoxypyrrolidin-1-yl]-13-methyl-8,14-dioxa-5,10,19,20,23-pentaazatetracyclo [13.5.2.1^{2,6}.0^{18,21}]tricosa-1(20),2,4,6(23),15,17,21-heptaen-9-one **example 137** as a white powder.
LCMS method F: [M+H]⁺ = 439, t_{R} = 2.06 min
LCMS method G: [M+H]⁺ = 439, t_{R} = 2.23 min
¹H NMR (400 MHz, d6-DMSO) δ 13.36 (1H, s), 7.93 (1H, d, J = 1.9 Hz), 7.79 (1H, dd, J = 4.9, 7.4 Hz), 7.49 - 7.46 (1H, m), 7.06 (1H, s), 6.95 (1H, dd, J = 2.4, 9.0 Hz), 5.41 - 5.36 (1H, m), 4.74 (1H, d, J = 15.6 Hz), 4.62 - 4.56 (1H, m), 4.14 - 4.07 (1H, m), 3.57 (4H, m), 3.50 - 3.43 (3H, m), 2.87 (1H, t, J = 14.4 Hz), 2.35 - 2.28 (1H, m), 2.09 - 2.09 (3H, m), 1.39 - 1.36 (4H, m) ppm.

### Example 138: (13R)-16-chloro-13-methyl-8,14-dioxa-10,19,20-triazatetracyclo [13.5.2.1^{2,6}.0^{18,21}]tricosa-1(20),2,4,6(23),15,17,21-heptaen-9-one

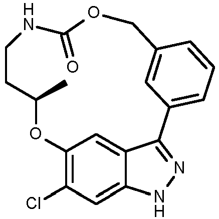

Example 138 is prepared according to the synthesis route described in general Scheme K.

To a solution of (13R)-16-chloro-13-methyl-19-(oxan-2-yl)-8,14-dioxa-10,19,20-triazatetra cyclo[13.5.2.1^{2,6}.0^{18,21}]tricosa-1(20),2,4,6(23),15,17,21-heptaen-9-one (376 mg, 0.83 mmol) in MeOH (49 mL) and water (6 mL) was added p-toluenesulfonic acid monohydrate (785 mg, 4.13 mmol) and the reaction mixture was heated to 65°C for 3 hours and 30 minutes. The reaction mixture was concentrated under vacuo and the crude was neutralized by slow addition of a saturated aqueous sodium hydrogen carbonate solution. It was diluted with EtOAc and after separation, the aqueous layer was extracted with EtOAc (3 x). The combined organic layer was washed with brine, dried over sodium sulfate, filtered and evaporated under reduced pressure. The crude was triturated from ACN then filtrated and washed two times with ACN to give (13R)-16-chloro-13-methyl-8,14-dioxa-10,19,20-triazatetracyclo[13.5.2.1^{2,6}.0^{18,21}]tricosa-1(20),2,4,6(23),15,17,21-heptaen-9-one **example 138** as a white powder.
LCMS method F: [M+H]⁺ = 372, t_{R} = 2.42 min
LCMS method G: [M+H]⁺ = 372, t_{R} = 2.40 min
¹H NMR (400 MHz, d6-DMSO) δ 13.24 (1H, m), 7.94 (1H, m), 7.85 (1H, m), 7.80 (1H, m), 7.74 (1H, s), 7.48 (1H, t, J = 7.7 Hz), 7.37 (1H, s), 7.29 (1H, m), 5.74 (1H, m), 4.8 (1H, m), 4.63 (1H, m), 3.56 (1H, m), 2.92 (1H, m), 2.37 (1H, m), 1.48 (1H, m), 1.44 (3H, d, J = 6 Hz) ppm.

### Example 139: (13R)-13,16-dimethyl-8,14-dioxa-10,19,20-triazatetracyclo[13.5.2.1^{2,6}.0^{18,21}] tricosa-1(20),2,4,6(23),15,17,21-heptaen-9-one

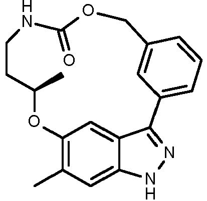

Example 139 is prepared according to the synthesis route described in general Scheme K.

To a solution of (13R)-13,16-dimethyl-19-(oxan-2-yl)-8,14-dioxa-10,19,20-triazatetracyclo [13.5.2.1^{2,6}.0^{18,21}]tricosa-1(20),2,4,6(23),15,17,21-heptaen-9-one (537 mg, 1.23 mmol) in MeOH (72 mL) and water (9 mL) was added p-toluenesulfonic acid monohydrate (1.17 g, 6.17 mmol) and the reaction mixture was heated to 65°C for 3 hours and 30 minutes. The reaction mixture was concentrated under vacuo and the crude was neutralized by slow addition of a saturated aqueous sodium hydrogen carbonate solution. It was diluted with EtOAc and after separation, the aqueous layer was extracted with EtOAc (3 x). The combined organic layer was washed with brine, dried over sodium sulfate, filtered and evaporated under reduced pressure. The crude was triturated from ACN then filtrated and washed two times with ACN to give (13R)-13,16-dimethyl-8,14-dioxa-10,19,20-triazatetracyclo[13.5.2.1^{2,6}.0^{18,21}]tricosa-1(20),2,4,6(23),15,17,21-heptaen-9-one **example 139** as a white powder.
LCMS method F: [M+H]⁺ = 352, t_{R} = 2.39 min
LCMS method G: [M+H]⁺ = 352, t_{R} = 2.38 min
¹H NMR (400 MHz, d6-DMSO) δ = 12.97 (1H, s), 7.94 (1H, m), 7.86 (1H, m), 7.84 (1H, m), 7.45 (1H, t, J = 7.8 Hz), 7.37 (1H, s), 7.26 (1H, m), 7.24 (1H, m), 5.74 (1H, m), 4.82 (1H, m), 4.58 (1H, m), 3.57 (1H, m), 2.9 (1H, m), 2.41 (1H, m), 2.27 (3H, d, J = 0.8 Hz), 1.42 (3H, d, J = 5.9 Hz), 1.39 (1H, m) ppm

### Example 140: (13R)-13-methyl-8,14-dioxa-3,10,19,20,23-pentaazatetracyclo [13.5.2.1^{2,6}.0^{18,21}]tricosa-1(20),2,4,6(23),15,17,21-heptaen-9-one

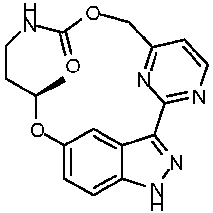

Example 140 is prepared according to the synthesis route described in general Scheme B.

To a solution of (13R)-13-methyl-19-(oxan-2-yl)-8,14-dioxa-3,10,19,20,23-pentaazatetracyclo[13.5.2.1^{2 6}.0^{18 21}]tricosa-1(20),2,4,6(23),15,17,21-heptaen-9-one (77 mg, 0.18 mmol) in MeOH (10.6 mL) and water (1.3 mL) was added p-toluenesulfonic acid monohydrate (173 mg, 0.91 mmol) and the reaction mixture was heated to 65°C for 3 hours and 30 minutes. The mixture was heated at 65 °C for 1 day. The reaction mixture was concentrated under vacuo and the crude was neutralized by slow addition of a saturated aqueous sodium hydrogen carbonate solution. It was diluted with EtOAc and after separation, the aqueous layer was extracted with EtOAc (3 x). The combined organic layer was washed with brine, dried over sodium sulfate, filtered and evaporated under reduced pressure. The crude was triturated from ACN then filtrated and washed two times with ACN to give (13R)-13-methyl-8,14-dioxa-3,10,19,20,23-pentaazatetracyclo[13.5.2.1^{2,6}.0^{18,21}]tricosa-1(20),2,4,6(23), 15,17,21-heptaen-9-one **example 140** as a pale yellow solid.
LCMS method F: [M+H]⁺ = 340, t_{R} = 1.73 min
LCMS method G: [M+H]⁺ = 340, t_{R} = 1.74 min
¹H NMR (400 MHz, d6-DMSO) δ 13.51 (1H, m), 8.81 (1H, d, J = 5 Hz), 7.84 (2H, m), 7.5 (1H, m), 7.33 (1H, m), 6.97 (1H, dd, J = 1.6, 8.4 Hz), 5.59 (1H, m), 5.07 (1H, m), 4.62 (1H, m), 3.48 (1H, m), 2.91 (1H, m), 2.3 (1H, m), 1.41 (1H, m), 1.38 (3H, d, J = 5.5 Hz) ppm.

### Preparation of intermediate 176: benzyl N-[(3S)-3-hydroxybutyl]carbamate

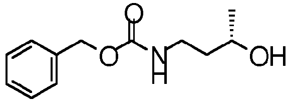

To a solution of (2S)-4-aminobutan-2-ol (10 g, 112.36 mmol) in a mixture of THF (143 mL) and water (143 mL) was added sodium hydrogenocarbonate (10.38 g, 123.59 mmol). The suspension was cooled to 0°C and benzyl chloroformate (17.64 mL, 123.59 mmol) was added dropwise and the reaction mixture was stirred at room temperature for 16 hours. The solution was diluted with water and extracted with EtOAc (3x). The combined organic layer was washed with brine, dried over sodium sulfate, filtered and evaporated under reduced pressure. The oily residue was purified by column chromatography eluting with dichloromethane / ethyl acetate: 100/0 to 80/20 to give benzyl N-[(3S)-3-hydroxybutyl]carbamate intermediate 176 as a colorless oil.
LCMS method F: [M+H]⁺ = 224, t_{R} = 1.94 min

### Preparation of intermediate 177: [(1S)-3-(benzyloxycarbonylamino)-1-methyl-propyl] methanesulfonate

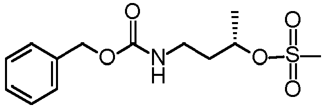

To a cooled solution (0°C) of benzyl N-[(3S)-3-hydroxybutyl]carbamate intermediate 176 (17.06 g, 76.52 mmol) and triethylamine (21.3 mL, 153.04 mmol) in dichloromethane (300 mL) was added dropwise methanesulfonyl chloride (7.7 mL, 99.47 mmol) and the reaction mixture was stirred at room temperature for 19 hours. The reaction mixture was quenched with an aqueous solution of 1N HCl and extracted with dichloromethane (1x). The organic layer was washed with a saturated aqueous solution of NaHCO3 then water and brine, dried over sodium sulfate, filtered and evaporated under reduced pressure to give [(1S)-3-(benzyloxycarbonylamino)-1-methyl-propyl] methanesulfonate intermediate 177 as a yellow oil.
LCMS method F: [M+H]⁺ = 302, t_{R} = 2.26 min

### Preparation of intermediate 178: benzyl N-[(3R)-3-(3-iodo-1-tetrahydropyran-2-yl-indazol-5-yl)oxybutyl]carbamate

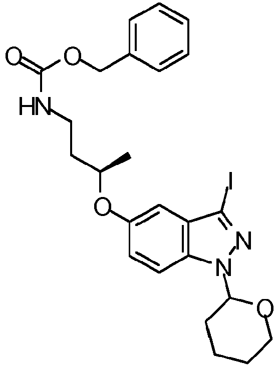

To a solution of 3-iodo-1-tetrahydropyran-2-yl-indazol-5-ol **4** (5.152 g, 14.97 mmol) and cesium carbonate (14.633 g, 44.91 mmol) in DMF (50 mL) was dropwise added a solution of [(1S)-3-(benzyloxycarbonylamino)-1-methyl-propyl] methanesulfonate **177** (5.406 g, 17.96 mmol) in DMF (26 mL) and the reaction mixture was stirred at RT for the week-end. The reaction mixture was filtered then concentrated under reduced pressure. The crude product was diluted with ethyl acetate and a saturated solution of NaHCO3 was added then it was extracted with ethyl acetate (2 x). The organic layer was washed with water then brine, dried over sodium sulfate, filtered and concentrated under reduced pressure. The crude product was purified by flash column chromatography eluting with Cyclohexane / Ethyl acetate - Ethanol (3-1), 100/0 to 70/30, to give benzyl *N*-[(3R)-3-(3-iodo-1-tetrahydropyran-2-yl-indazol-5-yl)oxybutyl]carbamate **178** as a yellow oil.
LCMS method F: [M+H]⁺ = 550.1, t_{R} = 3.20 min

### Preparation of intermediate 179: benzyl N-(3-hydroxybutyl)carbamate

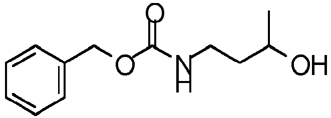

To a solution of 4-aminobutan-2-ol (1.78 g, 20 mmol) in a mixture of THF (30 mL) and water (30 mL) was added sodium hydrogenocarbonate (1.84 g, 22 mmol). The suspension was cooled at 0°C and benzyl chloroformate (3.15 mL, 22 mmol) was added portionwise and the reaction mixture was stirred at room temperature overnight. The solution was diluted with water and extracted with EtOAc (3x). The combined organic extract was washed with brine, dried over Na₂SO₄, filtered and evaporated under reduce pressure. The oily residue was purified by chromatography eluting with cyclohexane/EtOAc: 70/30 to 50/50 to give the expected compound benzyl N-(3-hydroxybutyl)carbamate intermediate **179** as a colorless oil.
LCMS method F: [M+H]⁺ = 224.1, t_{R} = 1.92 min

### Preparation of intermediate 180: [3-(benzyloxycarbonylamino)-1-methyl-propyl]methane sulfonate

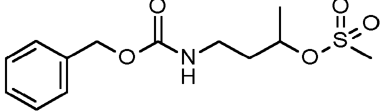

To a cooled (0°C) solution of benzyl N-(3-hydroxybutyl)carbamate **179** (1.67 g, 7.5 mmol) and Et₃N (1.56 mL, 11.25 mmol) in dichoromethane (30 mL) was added dropwise methanesulfonyl chloride (638 µL, 8.25 mmol) and the reaction mixture was stirred at room temperature overnight. The organic phase was washed with a 1N HCl solution, with a saturated solution of NaHCO₃, with brine, dried over Na₂SO₄, filtered and evaporated under reduced pressure to gve the expected compound [3-(benzyloxycarbonylamino)-1-methyl-propyl] methanesulfonate 180 as a colorless oil. The crude compound was used in the next step without further purification.
LCMS method F: [M+H]⁺ = 302, t_{R} = 2.24 min

### Preparation of intermediate 181: N-[3-(3-iodo-1-tetrahydropyran-2-yl-indazol-5-yl)oxybutyl]carbamate

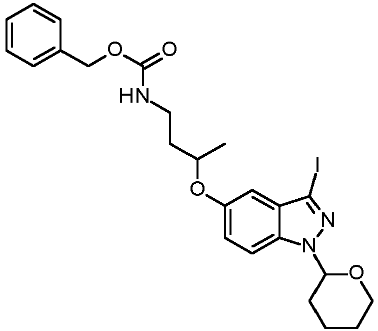

To a solution of 3-iodo-1-tetrahydropyran-2-yl-indazol-5-ol **4** (2 g, 6.08 mmol) and cesium carbonate (5.94 g, 18.25 mmol) in DMF (20 mL) was added dropwise a solution of [3-(benzyloxycarbonylamino)-1-methyl-propyl] methanesulfonate **180** (2.2 g, 7.3 mmol) in DMF (10 mL) and the reaction mixture was stirred at 60°C overnight. The reaction mixture was cooled to room temperature and water was added. The aqueous phase was extracted with EtOAc (3x) and the organic phase was washed with brine, dried over Na₂SO₄, filtered and evaporated under reduced pressure. The residue was purified by chromatography eluting with cyclohexane/EtOAc: 70/30 to give the expected compound N-[3-(3-iodo-1-tetrahydropyran-2-yl-indazol-5-yl)oxybutyl]carbamate **180** as a colorless oil.
LCMS method F: [M+H]⁺ = 550.1, t_{R} = 3.20 min

Intermediates **179** to **181** are used as intermediates in the synthesis of **Examples 50, 71, 92. Examples 62, 63, 96, 97, 100, 101, 102, 105, 106, 113, 114, 117, 120, 125, 127, 128, 129, 130, 131, 133, 136, 137,** and **140** can be obtained by chiral HPLC separation of the corresponding racemates, or through a chiral synthesis using intermediates **176** to **178.**

### Example 141: 8-oxa-10,14,19,20-tetraazatetracyclo[13.5.2.1^{2,6}.0^{18,21}]tricosa-1(20),2(23), 3,5,15(22),16,18(21)-heptaen-9-one

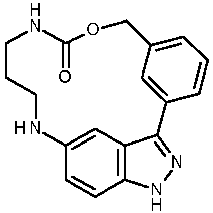

Example 141 is prepared according to the synthesis route described in general Scheme I. To a solution of 19-(oxan-2-yl)-8-oxa-10,14,19,20-tetraazatetracyclo[13.5.2. 1^{2,6}.0^{18,2 1}]tricosa-1(20),2(23),3,5,15(22),16,18(21)-heptaen-9-one (37mg, 0.09mmol) in dioxane (7.5ml) was added HCl 4N dioxane (360µl, 1.8mmol). The reaction mixture was stirred overnight at 60°C. Three drops of HCl 37% were added and the reaction mixture was stirred 1h at 60°C. The solvent was removed under reduced pressure and the mixture was purified by chromatography using a 4g SiO2 column eluted with DCM/MeOH 100/0 to 90/10. The desired fractions were combined and the solvent was removed under reduced pressure to give 8-oxa-10,14,19,20-tetraazatetracyclo[13.5.2.1^{2,6}.0^{18,21}]tricosa-1(20),2(23),3,5,15(22),16,18(21)-heptaen-9-one.hydrochloride **example 141** as a cream powder.
LCMS method F: [M+H]⁺ = 323.2, t_{R} = 1.70 min
LCMS method G: [M+H]⁺ = 323.2, t_{R} = 1.72 min
¹H NMR (400 MHz, d6-DMSO) δ 7.92 (1H, s), 7.85 (2H, d, J=1.5 Hz), 7.53 - 7.45 (2H, m), 7.35 - 7.31 (2H, m), 6.54 (1H, q, J=2.9 Hz), 5.3 (1H, m), 4.61 (2H, d, J=6.1 Hz), 3.68 (2H, t, J=5.5 Hz), 3.27 (2H, m), 2.03 - 1.97 (2H, m), 1.04 (1H, d, J=6.1 Hz) ppm.

### Example 142: 8-oxa-10,19,20-triazatetracyclo[13.5.2.1^{2,6}.0^{18,21}]tricosa-1(20),2(23),3,5, 15(22),16,18(21)-heptaen-9-one

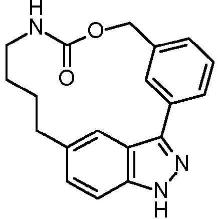

Example 142 is prepared according to the synthesis route described below.

### Preparation of intermediate 182: diisopropylammonium; 4-spirof[7,9-dioxa-8-silanuidabicyclo[4.3.0]nona-1(6),2,4-triene-8,8'-7,9-dioxa-8-silanuidabicyclo[4.3.0]nona-1,3,5-triene]-8-ylbutanenitrile

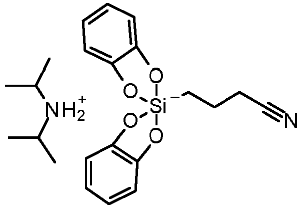

To an oven-dried, 100 mL round bottom flask equipped with a stir bar, reflux condenser, and gas inlet adapter was added catechol (5.67 g, 51.58 mmol) followed by THF (62 mL) and i-Pr₂NH (4.44 mL, 31.74 mmol). The mixture was placed under an argon atmosphere and was allowed to stir at room temperature for 5 minutes. The solution became pale red. 4-Trimethoxysilylbutanenitrile (5.00 g, 26.45 mmol) was added. The solution was heated to reflux in an oil bath and allowed to stir at this temperature for 18 hours. The solvent was removed under reduced pressure. The resulting powder was collected *via* filtration through a medium porosity fritted funnel. The powder was washed with Et₂O (~100 mL) and pentane (~150 mL). The solid was collected and dried further under reduced pressure to give diisopropylammonium;4-spiro[7,9-dioxa-8-silanuidabicyclo[4.3.0]nona-1(6),2,4-triene-8,8'-7,9-dioxa-8-silanuidabicyclo[4.3.0]nona-1,3,5-triene]-8-ylbutanenitrile **182** as a slightly pink powder.
LCMS method F: [M+H]⁺ = not detected, t_{R} = 1.01 min

### Preparation of intermediate 183: 4-(1H-indazol-5-yl)butanenitrile

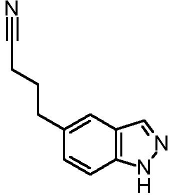

To a 500 mL round bottom flask equipped with a Teflon-coated magnetic stir bar was added 4,4'-di-tert-butyl-2,2'-bipyridine (134 mg, 0.5 mmol), and nickel(II) chloride ethylene glycol dimethyl ether complex (109 mg, 0.5 mmol). The vial was capped and purged with nitrogen, then 30 mL THF was introduced. The resulting suspension was heated briefly with a heat gun until the nickel and ligand were fully solubilized, yielding a pale green solution. The solution was cooled in an ice bath, resulting in the immediate precipitation of an evergreen solid. Solvents were then evaporated under reduced pressure to give a fine coating of the ligated nickel complex.

Once dry, 5-iodo-1H-indazole (2.440 g, 10.0 mmol, 1.0 equiv), diisopropylammonium;4-spiro[7,9-dioxa-8-silanuidabicyclo[4.3.0]nona-1(6),2,4-triene-8,8'-7,9-dioxa-8-silanuida bicyclo[4.3.0]nona-1,3,5-triene]-8-ylbutanenitrile **182** (8.280 g, 20.0 mmol), and tris(2,2'-bipyridine)ruthenium(II) hexafluorophosphate (172 mg, 0.2 mmol) were added in succession. The vial was then capped and purged four times. Under inert atmosphere, DMF (100 mL) was introduced. The vial containing all the reagents was further sealed with parafilm and stirred approximately 10 cm away from a PR160L LED PhotoReaction Lighting setup (2 LED with different wavelengths: 390 nm and 456 nm) and was stirred for 24 hours. A fan was blown across the reaction setup to suppress the heat generated by the latter (the reaction temperatures were estimated to be ~30 °C). The reaction mixture was allowed to stir for 24 more hours. The crude reaction mixture was poured in a separatory funnel and diluted with H₂O (120 mL). The resulting suspension was extracted with Et2O (3 × 180 mL), and the combined organic extracts were washed with a saturated solution of Na₂CO₃ (2 × 120 mL) then H₂O (120 mL), dried over anhydrous magnesium sulfate, filtered and concentrated under reduced pressure. The combined residue was purified by column chromatography on silica gel, eluting with EtOAc and hexanes (from 99/1 to 40/60) to obtain ***4-(1H-indazol-5-yl)butanenitrile* 183** as a white solid.
LCMS method F: [M+H]⁺ = 186.3, t_{R} = 1.75 min

### Preparation of intermediate 184: 4-(1-tetrahydropyran-2-ylindazol-5-yl)butanenitrile

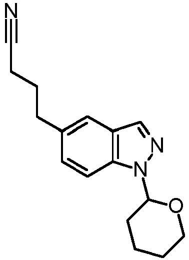

To a solution of ***4-(1H-indazol-5-yl)butanenitrile* 183** (1.256 g, 6.79 mmol) in DCM (25 mL), 3,4-dihydro-2H-pyran (1.140 g, 1.24 mL, 13.58 mmol) and p-toluenesulfonic acid monohydrate (0.644 g, 3.39 mmol) were added and the reaction was stirred at room temperature for 24 hours. The residue was dissolved in ethyl acetate (150 mL), quenched with a 1 M aqueous NaHCO3 solution (5 mL), washed with water (25 mL), brine (25 mL), dried over anhydrous magnesium sulfate, filtered and concentrated under reduced pressure. The crude product was purified by flashchromatography using cyclohexane/ethyl acetate (from 99/1 to 70/30) to give 4-(1-tetrahydropyran-2-ylindazol-5-yl)butanenitrile **184** as a colorless oil.
LCMS method F: [M+H]⁺ = not detected, t_{R} = 2.36 min

### Preparation of intermediate 185: 4-(1-tetrahydropyran-2-ylindazol-5-yl)butan-1-amine

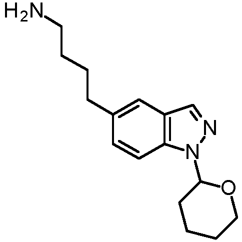

Lithium aluminium hydride 1.0 M solution in THF (14.3 mL, 14.30 mmol) was added dropwise to a solution of 4-(1-tetrahydropyran-2-ylindazol-5-yl)butanenitrile **184** (1.540 g, 5.72 mmol) in THF (20 mL) at 0 °C and stirred at room temperature for 24 hours. The mixture was quenched with a saturated aqueous solution of Rochelle salt (100 mL) and the resulting mixture was stirred overnight to break up the aluminium emulsions. The resulting biphasic medium was extracted with dichloromethane (2 × 150 mL). Combined organic layers were washed with brine (100 mL), dried over anhydrous magnesium sulfate, filtered and concentrated under reduced pressure to afford 4-(1-tetrahydropyran-2-ylindazol-5-yl)butan-1-amine **185** as a colorless oil. LCMS method F: [M+H]⁺ = not detected, t_{R} = 1.37 min

### Preparation of intermediate 186: (3-bromophenyl)methyl N-[4-(1-tetrahydropyran-2-ylindazol-5-yl)butyl]carbamate

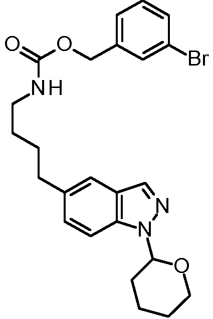

To a solution of 4-(1-tetrahydropyran-2-ylindazol-5-yl)butan-1-amine **185** (1.076 g, 3.94 mmol) in acetonitrile (120 mL) was added 1,1'-carbonyldiimidazole (0.701 g, 4.33 mmol). The reaction mixture was stirred at room temperature for 2 hours. The reaction mixture was then added dropwise to a solution of (3-bromophenyl)methanol (2.36 mL, 19.70 mmol) and cesium carbonate (12.805 g, 39.40 mmol) in acetonitrile (60 mL) at 90°C. The resulting mixture was stirred at the 90°C for 5 hours. The reaction mixture was allowed to cool down to room temperature and filtered over a celite pad. The filtrate was evaporated under reduced pressure to afford a yellow oil. This residue was partitioned between ethyl acetate (150 mL) and water (100 mL). The organic layer was extracted twice with ethyl acetate (2 × 100 mL). The combined organic layer was washed with brine, dried over anhydrous magnesium sulfate, filtered and concentrated under reduced pressure. The resulting oil was purified by column chromatography on silica gel, using DCM/MeOH (from 1/0 to 9/1) as an eluant, the desired fractions were combined and the solvent was removed under reduced pressure to afford (3-bromophenyl)methyl *N*-[4-(1-tetrahydropyran-2-ylindazol-5-yl)butyl]carbamate **186** as a colorless oil.
LCMS method F: [M+H]⁺ = 488.1, t_{R} = 3.13 min

### Preparation of intermediate 187: 19-(oxan-2-yl)-8-oxa-10,19,20-triazatetracyclo [13.5.2.1^{2,6}.0^{18,21}]tricosa-1(20),2(23),3,5,15(22),16,18(21)-heptaen-9-one

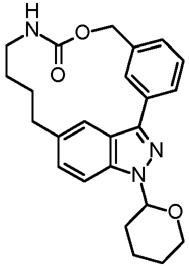

To a solution of (3-bromophenyl)methyl *N*-[4-(1-tetrahydropyran-2-ylindazol-5-yl)butyl] carbamate **186** (340 mg, 0.70 mmol) in dry dioxane (68 mL), was added potassium acetate (137 mg, 1.40 mmol). An argon balloon with a long needle was placed in the reaction mixture, bubbling for 15 minutes. Tricyclohexylphosphine (39 mg, 0.14 mmol) and palladium acetate (15 mg, 0.07 mmol) were added and the reaction mixture was placed in a sand bath, pre-heated at 200 °C, and stirred at this temperature for 16 hours. The reaction mixture was diluted with water. The aqueous layer was extracted with ethyl acetate (2 × 20 mL). The combined organic layer was washed with brine, dried over anhydrous magnesium sulfate, filtered and evaporated under reduced pressure. The crude product was purified by column chromatography on silica gel (DCM/MeOH/EtOAc : from 100/0/0 to 95/2.5/2.5) to afford the first fraction of the expected macrocycle (0.015 g) and another fraction which was a mixture of expected macrocycle and dehalogenated side-product benzyl *N*-[4-(1-tetrahydropyran-2-ylindazol-5-yl)butyl]carbamate (0.119 g). This second fraction was purified again by preparative TLC, using (DCM/MeOH/EtOAc = 95/2.5/2.5) as an eluant. Pure macrocycle was recovered from the TLC plate and combined with the first fraction from column chromatography to afford 19-(oxan-2-yl)-8-oxa-10,19,20-triazatetracyclo[13.5.2.1^{2,6}.0^{18,21}]tricosa-1(20),2(23),3,5,15(22),16,18(21)-heptaen-9-one **187** as a white solid.
LCMS method F: [M+H]⁺ = 406.2, t_{R} = 2.92 min

### Preparation of Example 142: 8-oxa-10,19,20-triazatetracyclo[13.5.2.1^{2,6}.0^{18,21}]tricosa-1(20),2(23),3,5,15(22),16,18(21)-heptaen-9-one

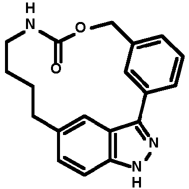

To a solution of 19-(oxan-2-yl)-8-oxa-10,19,20-triazatetracyclo[13.5.2.1^{2,6}.0^{18,21}]tricosa-1(20),2(23),3,5,15(22),16,18(21)-heptaen-9-one **187** (30 mg, 0.074 mmol) in methanol (4.0 mL) and water (0.4 mL) was added *p*-toluenesulfonic acid monohydrate (70 mg, 0.36 mmol) and the reaction mixture was heated to 65 °C for 4 hours. The reaction mixture was concentrated under reduced pressure and the crude was neutralized by slow addition of a saturated aqueous sodium hydrogen carbonate solution. The resulting suspension was diluted with ethyl acetate. After separation, the aqueous layer was extracted with ethyl acetate. The combined organic layers were washed with water and brine, dried over anhydrous magnesium sulfate, filtered and evaporated under reduced pressure. The crude was purified by preparative TLC on silica gel, eluting with DCM/MeOH 95/5, to give the expected product. DCM (5 mL) was added to give a white suspension. The precipitate was filtered off and rinced with CH₂Cl₂ (2 x 3 mL). The solid was recovered. Remaing solvents were removed under reduced pressure at 40°C to obtain 8-oxa-10,19,20-triazatetracyclo[13.5.2.1^{2,6}.0^{18,21}]tricosa-1(20),2(23),3,5,15(22),16,18(21)-heptaen-9-one **example 142** as a white solid.
LCMS method F: [M+H]⁺ = 322.3, t_{R} = 2.25 min
LCMS method G: [M+H]⁺ = 322.3, t_{R} = 2.25 min
¹H NMR (400 MHz, d6-DMSO, 80 °C) δ 12.87 (1H, br. s), 7.92 (1H, s), 7.87 (1H, d, *J=* 8.0 Hz), 7.76 (1H, s), 7.54 (1H, br. s), 7.51 - 7.42 (2H, m), 7.27 (1H, d, *J =* 8.0 Hz), 7.20 (1H, dd, *J = 1.6,* 8.8 Hz), 5.28 (2H, s), 3.22 - 3.19 (2H, m), 2.92 - 2.88 (2H, m), 1.95 - 1.90 (2H, m), 1.72 - 1.63 (2H, m) ppm.

### Example 143: (13R)-5-methoxy-13-methyl-8,14-dioxa-4,10,19,20-tetraazatetracyclo [13.5.2.1^{2,6}.0^{18,21}]tricosa-1(20),2,4,6(23),15,17,21-heptaen-9-one

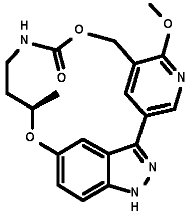

Example 143 is prepared according to the synthesis route described in general Scheme O.

### Preparation of intermediate 184: 5-bromo-2-methoxy-pyridine-3-carbaldehyde

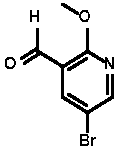

To a solution of 3,5-dibromo-2-methoxy-pyridine (1.62 g, 6.11 mmol) in dry diethyl ether (24 mL) at -78°C was added dropwise a solution of n-BuLi in hexane (4,16 M titrated) (1.47 mL, 6.11 mmol). The reaction mixture was stirred at -78°C for 15 min then *N,N-*dimethylformamide (0.95 mL, 12.22 mmol) was added dropwise and the reaction mixture was stirred at -50°C for 30 min. The reaction mixture was quenched dropwise with a saturated aqueous ammonium chloride solution at -78°C, warmed to room temperature and stirred for 30 min. Diethyl ether was added to the solution. After separation, the aqueous layer was extracted with diethyl ether. The organic layer was washed with brine, dried over anhydrous sodium sulfate, filtered and evaporated under reduced pressure to afford a yellow solid which was recrystallized in hexane to afford 5-bromo-2-methoxy-pyridine-3-carbaldehyde intermediate **184** as a yellow powder. ¹H NMR (400 MHz, CDCl₃) δ 10.32 (1H, s), 8.44 (1H, d, J=2.7 Hz), 8.22 (1H, d, J=2.7Hz), 4.09 (3H, m) ppm.

### Preparation of intermediate 185: (5-bromo-2-methoxy-3-pyridyl)methanol

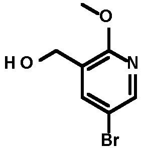

To a solution of 5-bromo-2-methoxy-pyridine-3-carbaldehyde intermediate **184** (997 mg, 4.64 mmol) in methanol (21 mL) at 0°C was added sodium borohydride (176 mg, 4.64 mmol) in one portion. The reaction mixture was stirred at room temperature for 2 h. The reaction mixture was evaporated under reduced pressure. The residue was diluted with dichloromethane and a sodium bicarbonate saturated solution. After separation, the aqueous layer was extracted with dichloromethane. The organic layer was washed with brine, dried over anhydrous sodium sulfate, filtered and evaporated under reduced pressure. The residue was purified by silica gel column chromatography using cyclohexane/ethyl acetate 100/0 to 50/50 as eluent to afford (5-bromo-2-methoxy-3-pyridyl)methanol intermediate **185** as yellow crystals.
LCMS method F: [M+H]⁺ = 218-220, t_{R} = 1.81 min

### Preparation of intermediate 186: [5-[5-[tert-butyl(dimethyl)silyl]oxy-1-tetrahydropyran-2-yl-indazol-3-yl]-2-methoxy-3-pyridyl]methanol

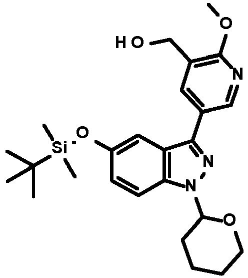

To a solution of tert-butyl-dimethyl-[1-tetrahydropyran-2-yl-3-(4,4,5,5-tetramethyl-1,3,2-dioxaborolan-2-yl)indazol-5-yl]oxy-silane (1.24 g, 2.71 mmol) in 1,4-dioxane (20 mL) and water (2 mL) at room temperature was added (5-bromo-2-methoxy-3-pyridyl)methanol intermediate **185** (588 mg, 2.71 mmol), potassium phosphate tribasic (1.72 g, 8.13 mmol). The reaction mixture was degassed by bubbling argon for 15 min, then XPhos (388 mg, 0.8 mmol) and tetrakis(triphenylphosphine)-palladium(0) (312 mg, 0.27 mmol) were added. The reaction mixture was stirred at 80°C for 12 h. The reaction mixture was evaporated under reduced pressure. The residue was diluted with ethyl acetate and. After separation, the aqueous layer was extracted with ethyl acetate. The organic layer was washed with brine, dried over sodium sulfate, filtered and evaporated under reduced pressure. The residue was purified by silica gel column chromatography using cyclohexane/ethyl acetate 100/0 to 0/100 to afford *[5-[5-[tert-*butyl(dimethyl)silyl]oxy-1-tetrahydropyran-2-yl-indazol-3-yl]-2-methoxy-3-pyridyl]methanol intermediate **186** as a light yellow oil.
LCMS method F: [M+H]⁺ = 470.4, t_{R} = 3.52 min

### Preparation of intermediate 187: (13R)-5-methoxy-13-methyl-19-(oxan-2-yl)-8,14-dioxa-4,10,19,20-tetraazatetracyclo[13.5.2.1²,⁶.0^{18,21}]tricosa-1(20),2,4,6(23),15,17,21-heptaen-9-one

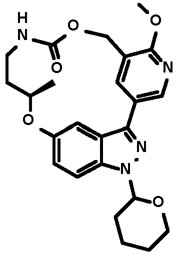

To a solution of [5-[5-[*tert*-butyl(dimethyl)silyl]oxy-1-tetrahydropyran-2-yl-indazol-3-yl]-2-methoxy-3-pyridyl]methanol intermediate **186** (824 mg, 1.76 mmol) in anhydrous acetonitrile (190 mL) was added cesium carbonate (2.86 g, 8.8 mmol) and [(1S)-3-(benzyloxycarbonyl amino)-1-methyl-propyl] methanesulfonate (582 mg, 1.93 mmol) in acetonitrile (10 mL). The reaction mixture was stirred at 80°C for 24 h. The reaction mixture was filtered and the filtrate was evaporated under reduced pressure. The residue was purified by silica gel column chromatography using cyclohexane/ethyl acetate 50/50 as eluent to afford (13R)-5-methoxy-13-methyl-19-(oxan-2-yl)-8,14-dioxa-4,10,19,20-tetraazatetracyclo [13.5.2.1^{2,6}.0^{18,21}]tricosa-1(20),2,4,6(23),15,17,21-heptaen-9-one intermediate **187** as a colorless oil.
LCMS method F: [M+H]⁺ = 453.4, t_{R} = 2.82 min

### Preparation of example 143: (13R)-5-methoxy-13-methyl-8,14-dioxa-4,10,19,20-tetraazatetracyclo[13.5.2.1^{2,6}.0^{18,21}]tricosa-1(20),2,4,6(23),15,17,21-heptaen-9-one

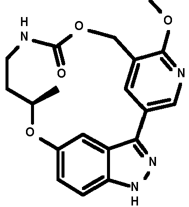

To a solution of (13R)-5-methoxy-13-methyl-19-(oxan-2-yl)-8,14-dioxa-4,10,19,20-tetraaza tetracyclo[13.5.2.1^{2,6}.0^{18,21}]tricosa-1(20),2,4,6(23),15,17,21-heptaen-9-one intermediate **187** (170 mg, 0.38 mmol) in methanol (7 mL) and water (1.2 mL) was added *p*-toluenesulfonic acid monohydrate (357 mg, 1.88 mmol). The reaction mixture was heated at 80 °C for 18 h. The solvent was evaporated under reduced pressure. The residue was dissolved in dichloromethane and a saturated aqueous solution of bicarbonate. After separation, the aqueous layer was extracted with dichloromethane. The organic layer was washed with brine, dried over anhydrous sodium sulfate, filtered and evaporated under reduced pressure. The residue was triturated in diethyl ether, filtered and dried to afford (13R)-5-methoxy-13-methyl-8,14-dioxa-4,10,19,20-tetraazatetracyclo[13.5.2.1^{2,6}.0^{18,21}]tricosa-1(20),2,4,6(23),15,17,21-heptaen-9-one **example 143** as a white solid.
LCMS method F: [M+H]⁺ = 369.3, t_{R} = 2.15 min
LCMS method G: [M+H]⁺ = 369.3, t_{R} = 2.13 min
¹H NMR (400 MHz, d6-DMSO) δ 13.13 (1H, s), 8.67 (1H, d, J=1.7 Hz), 8.11 - 8.07 (2H, m), 7.52 - 7.48 (1H, m), 7.24 (1H, d, J=0.8 Hz), 6.98 (1H, dd, J=2.1, 9.1 Hz), 5.45 - 5.42 (1H, m), 4.95 - 4.91 (1H, m), 4.60 - 4.54 (1H, m), 3.98 (3H, s), 3.53 (1H, s), 2.97 - 2.88 (1H, m), 2.33 (1H, s), 1.41 (4H, d, J=5.9 Hz) ppm.

### Example 144: (13R)-13-methyl-8,14-dioxa-4,10,19,20-tetraazatetracyclo[13.5.2.1^{2,6}.0^{18,2}1] tricosa-1(20),2,6(23),15,17,21-hexaene-5,9-dione

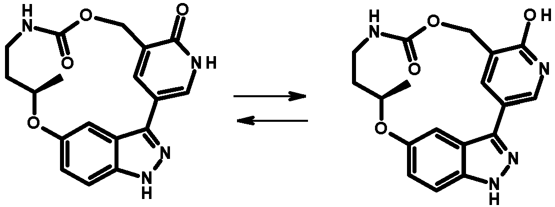

Example 144 is prepared by demethylation and concomitant deprotection of intermediate **187.** A mixture of (13R)-5-methoxy-13-methyl-19-(oxan-2-yl)-8,14-dioxa-4,10,19,20-tetraazatetra-cyclo[13.5.2.1^{1,6}.0^{18,21}]tricosa-1(20),2,4,6(23),15,17,21-heptaen-9-one intermediate **187** (150 mg, 0.33 mmol), sodium iodide (99 mg, 0.66 mmol), chlorotrimethylsilane (83 µL, 0.66 mmol) and acetonitrile (3 mL) was heated at 70°C for 4 h. The solvent was evaporated under reduced pressure. Ethyl acetate and a saturated aqueous solution of bicarbonate were added. After separation, the aqueous layer was extracted with ethyl acetate. The organic layer was washed with brine, dried over anhydrous sodium sulfate, filtered and evaporated under reduced pressure. The residue was purified by silica gel column chromatography using dichloromethane/methanol 90/10 as eluent to afford (13R)-13-methyl-8,14-dioxa-4,10,19,20-tetraazatetracyclo[13.5.2.1^{2,6}.0^{18,21}]tricosa-1(20),2,6(23),15,17,21-hexaene-5,9-dione **example 144** as a light yellow solid.
LCMS method F: [M+H]⁺ = 355.3, t_{R} = 1.70 min
LCMS method G: [M+H]⁺ = 355.3, t_{R} = 1.69 min
¹H NMR (400 MHz, *d*6-DMSO) δ13.00 (1H, s), 12.03 (1H, s), 8.01 (1H, t, J=6.2 Hz), 7.90 (1H, s), 7.81 (1H, s), 7.48 - 7.44 (1H, m), 7.16 (1H, s), 6.96 (1H, dd, J=1.9, 9.1 Hz), 5.25 (1H, m), 4.80 (1H, m), 4.57 - 4.51 (1H, m), 3.53 (1H, m), 2.91 (1H, s), 2.31 (1H, m), 1.39 (4H, d, J=6.1 Hz) ppm.

### Example 145: 4-methyl-8,14-dioxa-3,4,10,19,20-pentaazatetracyclo[13.5.2.1^{2,5}.0^{18,21}] tricosa-1(20),2,5(23),15(22),16,18(21)-hexaen-9-one

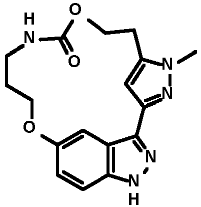

Example 145 is prepared according to the synthesis route described in general Scheme L.

### Preparation of intermediate 188: (2-(5-bromo-2-methyl-pyrazol-3-yl)ethyl N-(3-hydroxy propyl)carbamate

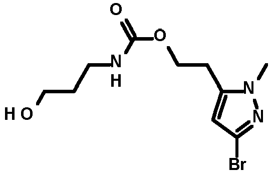

To a solution of 4-nitrophenyl chloroformate (432 mg, 2.15 mmol) and pyridine (0.315 mL, 3.90 mmol) in dichloromethane (10 mL) at room temperature was added dropwise 2-(5-bromo-2-methyl-pyrazol-3-yl)ethanol (400 mg, 1.95 mmol) in dichloromethane (5 mL). The reaction mixture was stirred at room temperature for 1 h. A mixture of 3-aminopropan-1-ol (161 mg, 2.15 mmol) and DIPEA (0.678 mL, 3.90 mmol) in dichloromethane (5 mL) was added. The reaction mixture was stirred at room temperature for 16 h. The residue was diluted with 0.5N aqueous sodium hydroxide solution and extracted with dichloromethane. The combined organic layers were washed once again with 0.5N aqueous sodium hydroxide solution and dried over anhydrous sodium sulfate and concentrated under reduced pressure. The residue was purified by silica gel column chromatography using dichloromethane/ethyl acetate 100/0 to 0/100 as eluent to afford 2-(5-bromo-2-methyl-pyrazol-3-yl)ethyl N-(3-hydroxypropyl)carbamate intermediate **188** as a colorless oil.
LCMS method F: [M+H]⁺ = 306-308, t_{R} = 2.25 min

### Preparation of intermediate 189: 2-[5-[5-[tert-butyl(dimethyl)silyl]oxy-1-tetrahydro-pyran-2-yl-indazol-3-yl]-2-methyl-pyrazol-3-yl]ethyl-N-(3-hydroxypropyl)carbamate

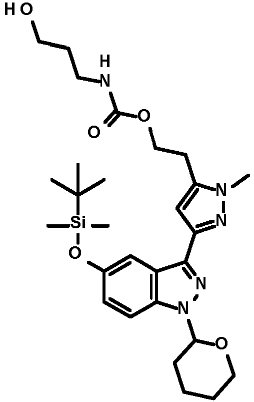

To a solution of *tert*-butyl-dimethyl-[1-tetrahydropyran-2-yl-3-(4,4,5,5-tetramethyl-1,3,2-dioxaborolan-2-yl)indazol-5-yl]oxy-silane (600 mg, 1.31 mmol) in 1,4-dioxane (13 mL) and water (1.3 mL) at room temperature was added 2-(5-bromo-2-methyl-pyrazol-3-yl)ethyl N-(3-hydroxypropyl)carbamate intermediate **188** (441 mg, 1.44 mmol), potassium phosphate tribasic (833 mg, 3.93 mmol), XPhos (62 mg, 0.13 mmol) and tetrakis(triphenylphosphine)palladium(0) (76 mg, 0.07 mmol). The reaction mixture was stirred under microwave conditiond at 90°C for 1 h. The residue was diluted with saturated aqueous sodium chloride solution and extracted with ethyl acetate. The combined organic layers were dried over anhydrous sodium sulfate and concentrated under reduced pressure. The residue was purified by silica gel column chromatography using cyclohexane/ethyl acetate 100/0 to 0/100 as eluent to afford 2-[5-[5-[tert-butyl(dimethyl)silyl]oxy-1-tetrahydropyran-2-yl-indazol-3-yl]-2-methyl-pyrazol-3-yl]ethyl N-(3-hydroxypropyl)carbamate intermediate **189** as a colorless oil.
LCMS method F: [M+H]⁺ = 558.4, t_{R} = 3.17 min

### Preparation of intermediate 190: 3-[2-[5-[5-[tert-butyl(dimethyl)silyl]oxy-1-tetrahydro-pyran-2-yl-indazol-3-yl]-2-methyl-pyrazol-3-yl]ethoxycarbonylamino]propyl methanesulfonate

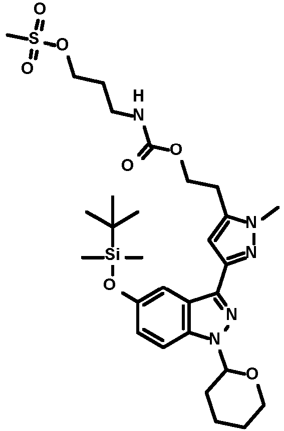

To a solution of 2-[5-[5-[*tert*-butyl(dimethyl)silyl]oxy-1-tetrahydropyran-2-yl-indazol-3-yl]-2-methyl-pyrazol-3-yl]ethyl N-(3-hydroxypropyl)carbamate intermediate **189** (470 mg, 0.84 mmol) and triethylamine (0.235 mL, 1.69 mmol) in dichloromethane (6 mL) was added at 0°C methanesulfonyl chloride (0.085 mL, 1.10 mmol) in dichloromethane (2 mL). The reaction mixture was stirred at room temperature for 16 h. The residue was diluted with saturated sodium chloride solution and extracted with dichloromethane. The combined organic layers were dried over anhydrous sodium sulfate and concentrated under reduced pressure to afford 3-[2-[5-[5-[tert-butyl(dimethyl)silyl]oxy-1-tetrahydropyran-2-yl-indazol-3-yl]-2-methyl-pyrazol-3-yl] ethoxycarbonylamino]propyl methanesulfonate intermediate **190** as a yellow oil which was used in the next step without further purification.
LCMS method F: [M+H]⁺ = 636.4, t_{R} = 3.32 min

### Preparation of intermediate 191: 4-methyl-19-(oxan-2-yl)-8,14-dioxa-3,4,10,19,20-pentaazatetracyclo[13.5.2.1^{2,5}.0^{18,21}]tricosa-1(20),2,5(23),15(22),16,18(21)-hexaen-9-one

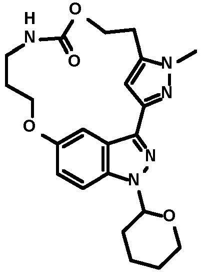

To a suspension of cesium carbonate (0.824 g, 2.53 mmol) in anhydrous *N,N-*dimethylformamide (168 mL) at 80°C was added dropwise 3-[2-[5-[5-[tert-butyl(dimethyl)silyl]oxy-1-tetrahydropyran-2-yl-indazol-3-yl]-2-methyl-pyrazol-3-yl]ethoxy carbonylamino]propyl methanesulfonate intermediate **190** (0.536 g, 0.84 mmol) in *N,N-*dimethylformamide (168 mL). The reaction mixture was stirred at 80°C for 1 h. The reaction mixture was filtered and concentrated under reduced pressure then diluted with saturated aqueous sodium chloride solution and extracted with ethyl acetate. The combined organic layers were dried over anhydrous sodium sulfate and concentrated under reduced pressure. The residue was purified by silica gel column chromatography using dichloromethane/ethyl acetate, 100/0 to 50/50 as eluent to afford 4-methyl-19-(oxan-2-yl)-8,14-dioxa-3,4,10,19,20-pentaazatetracyclo[13.5.2.1^{2,5}.0^{18,21}]tricosa-1(20),2,5(23),15(22),16,18(21)-hexaen-9-one intermediate **191** as a white solid.
LCMS method F: [M+H]⁺ = 426.4, t_{R} = 2.10 min

### Preparation of example 145: 4-methyl-8,14-dioxa-3,4,10,19,20-pentaazatetracyclo [13.5.2.1^{2,5}.0^{18,21}]tricosa-1(20),2,5(23),15(22),16,18(21)-hexaen-9-one

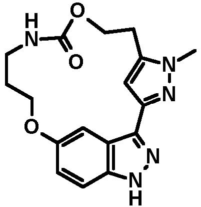

To a solution of 4-methyl-19-(oxan-2-yl)-8,14-dioxa-3,4,10,19,20-pentaazatetracyclo [13.5.2.1^{2,5}.0^{11,21}]tricosa-1(20),2,5(23),15(22),16,18(21)-hexaen-9-one intermediate **191** (55 mg, 0.13 mmol) in methanol (3.5 mL) and water (0.5 mL) was added p-toluenesulfonic acid monohydrate (123 mg, 0.65 mmol). The reaction mixture was stirred at 65°C for 2 h. The reaction mixture was concentrated under reduced pressure and the residue was neutralized by slow addition of saturated aqueous sodium bicarbonate solution. The residue was diluted with ethyl acetate. After separation, the aqueous phase was extracted with ethyl acetate. The combined organic layers were washed with brine, dried over anhydrous sodium sulfate, filtered and evaporated under reduced pressure. The residue was purified by silica gel column chromatography using dichloromethane/methanol 100/0 to 96/4 as eluent. The solid was crystallized in acetonitrile to give 4-methyl-8,14-dioxa-3,4,10,19,20-pentaazatetracyclo [13.5.2.1^{2,5}.0^{18,21}]tricosa-1(20),2,5 (23),15(22),16,18(21)-hexaen-9-one **example 145** as a white solid.
LCMS method F: [M+H]⁺ = 342.3, t_{R} = 1.63 min
LCMS method G: [M+H]⁺ = 342.3, t_{R} = 1.63 min
¹H NMR (400 MHz, d6-DMSO) δ 12.91 (1H, s), 7.74 (1H, t, J=6.0 Hz), 7.45 - 7.41 (1H, m), 7.11 (1H, d, J=1.9 Hz), 6.93 (1H, dd, J=2.2, 8.8 Hz), 6.38 (1H, s), 4.47 - 4.44 (2H, m), 4.27 - 4.21 (2H, m), 3.84 - 3.82 (3H, m), 3.10 - 3.02 (4H, m), 1.91 - 1.84 (2H, m) ppm.

### Example 146: (13R)-16-fluoro-13-methyl-8,14-dioxa-10,19,20,23-tetraazatetracyclo [13.5.2.1^{2,6}.0^{18,21}]tricosa-1(20),2,4,6(23),15,17,21-heptaen-9-one

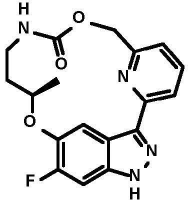

Example 146 is prepared according to the synthesis route described in general Scheme O.

### Preparation of intermediate 192: tert-butyl-[6-fluoro-1-tetrahydropyran-2-yl-3-(4,4,5,5-tetramethyl-1,3,2-dioxaborolan-2-yl)indazol-5-yl]oxy-dimethyl-silane

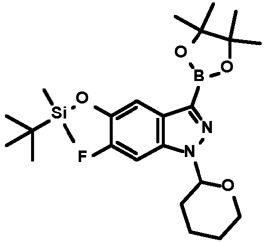

A mixture of *tert*-butyl-dimethyl-(1-tetrahydropyran-2-ylindazol-5-yl)oxy-silane (500 mg, 1.43 mmol), TBME (dried on 3A molecular sieves) (6 mL), 4,4,5,5-tetramethyl-2-(4,4,5,5-tetramethyl-1,3,2-dioxaborolan-2-yl)-1,3,2-dioxaborolane (711 mg, 1.43 mmol), *4,4'-di-tert-*butyl-2,2'-bipyridine(23 mg, 0.09 mmol) and (1,5-cyclooctadiene) (methoxy)iridium(I) dimer (19 mg, 0.03 mmol) was purged with argon and stirred at 80°C for 16 h. The solvent was evaporated under reduced pressure and the residue was dissolved with ethyl acetate and water. After separation, the aqueous phase was extracted with ethyl acetate. The organic layers were dried over anhydrous sodium sulfate, filtered and evaporated under reduced pressure to afford *tert*-butyl-[6-fluoro-1-tetrahydropyran-2-yl-3-(4,4,5,5-tetramethyl-1,3,2-dioxaborolan-2-yl) indazol-5-yl]oxy-dimethyl-silane intermediate 192 as a brown oil which was used in next step without further purification.
LCMS method F: [M+H]⁺ = 477.2, t_{R} = 3.87 min (major pic observed as boronic acid [M+H]⁺ = 395.2, t_{R} = 3.18 min)

### Preparation of intermediate 193: [6-[5-[tert-butyl(dimethyl)silyl]oxy-6-fluoro-1-tetrahydro pyran-2-yl-indazol-3-yl]-2-pyridyl]methanol

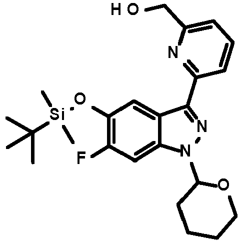

To a solution of *tert*-butyl-[6-fluoro-1-tetrahydropyran-2-yl-3-(4,4,5,5-tetramethyl-1,3,2-dioxaborolan-2-yl)indazol-5-yl]oxy-dimethyl-silane intermediate **192** (1.00 g, 2.10 mmol) in 1,4-dioxane (10 mL) and water (1 ml) at room temperature was added (6-bromo-2-pyridyl)methanol (302 mg, 1.62 mmol) and potassium phosphate tribasic (1.03 g, 4.86 mmol). The reaction mixture was degassed with argon for 15 min, then XPhos (76 mg, 0.16 mmol) and tetrakis(triphenylphosphine)palladium(0) (56 mg, 0.05 mmol) were added. The reaction mixture was stirred at 80°C for 45 min under microwave radiations. The solvent was evaporated under reduced pressure. The residue was dissolved in ethyl acetate and water. After separation, the aqueous layer was extracted with ethyl acetate. The organic layer was washed with brine, dried over anhydrous sodium sulfate, filtered and evaporated under reduced pressure. The residue was purified by silica gel column chromatography using cyclohexane/ethyl acetate 50/50 as eluent to afford [6-[5-[*tert*-butyl(dimethyl)silyl]oxy-6-fluoro-1-tetrahydropyran-2-yl-indazol-3-yl]-2-pyridyl]methanol intermediate **193** as a colorless oil.
LCMS method F: [M+H]⁺ = 458.2, t_{R} = 3.55 min

### Preparation of intermediate 194: (13R)-16-fluoro-13-methyl-19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one

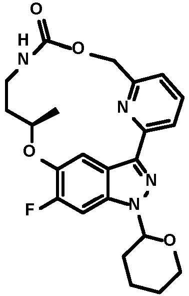

To a solution of [6-[5-[*tert*-butyl(dimethyl)silyl]oxy-6-fluoro-1-tetrahydropyran-2-yl-indazol-3-yl]-2-pyridyl]methanol intermediate **193** (400 mg, 0.87 mmol) in anhydrous acetonitrile (90 mL) was added cesium carbonate (2.83 g, 8.7 mmol) and a solution of [(1S)-3-(benzyloxy carbonylamino)-1-methyl-propyl] methanesulfonate (290 mg, 0.96 mmol) in acetonitrile (10 mL). The reaction mixture was stirred at 80°C for 24 h. The reaction mixture was filtered and the filtrate was evaporated under reduced pressure. The residue was triturated in diethyl ether, filtered and dried to afford (13R)-16-fluoro-13-methyl-19-(oxan-2-yl)-8,14-dioxa-10,19,20,23-tetraazatetracyclo[13.5.2.1^{2,6}.0^{18,21}]tricosa-1(20),2,4,6(23),15,17,21-heptaen-9-one intermediate **194** as an off-white powder.
LCMS method F: [M+H]⁺ = 441.2, t_{R} = 2.78 min

### Preparation of example 146: (13R)-16-fluoro-13-methyl-8,14-dioxa-10,19,20,23-tetraaza tetracyclo[13.5.2.1^{2,6}.0^{18,21}]tricosa-1(20),2,4,6(23),15,17,21-heptaen-9-one

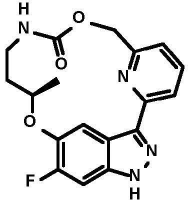

To a solution of afford (13R)-16-fluoro-13-methyl-19-(oxan-2-yl)-8,14-dioxa-10,19,20,23-tetraazatetracyclo[13.5.2.1^{2,6}.0^{11,21}]tricosa-1(20),2,4,6(23),15,17,21-heptaen-9-one intermediate **194** (160 mg, 0.36 mmol) in methanol (4 mL) and water (0.7 mL) was added *p-*toluenesulfonic acid monohydrate (345 mg, 1.82 mmol). The reaction mixture was heated at 80 °C for 18 h. The solvent was evaporated under reduced pressure. The residue was dissolved in dichloromethane (20 mL) and saturated aqueous solution of sodium bicarbonate (20 mL). After separation, the aqueous layer was extracted with dichloromethane (10 mL). The organic layer was washed with brine, dried over anhydrous sodium sulfate, filtered and evaporated under reduced pressure. The residue was triturated with diethyl ether, filtered and dried to afford (13R)-16-fluoro-13-methyl-8,14-dioxa-10,19,20,23-tetraazatetracyclo[13.5.2.1^{2,6}.0^{18,21}] tricosa-1(20),2,4,6(23),15,17,21-heptaen-9-one **example 146** as a white powder.
LCMS method F: [M+H]⁺ = 357.1, t_{R} = 2.10 min
LCMS method G: [M+H]⁺ = 357.2, t_{R} = 2.09 min
¹H NMR (400 MHz, d6-DMSO) δ 13.31 (1H, s), 8.06 (2H, dd, J=8.2, 15.9 Hz), 7.84 (1H, t, J=7.9 Hz), 7.74 (1H, dd, J=4.8, 6.7 Hz), 7.44 (1H, d, J=10.8 Hz), 7.29 - 7.26 (1H, m), 5.58 (1H, s), 5.10 - 5.06 (1H, m), 4.64 (1H, s), 3.52 - 3.50 (1H, m), 2.96 (1H, s), 2.23 - 2.20 (1H, m), 1.51 (1H, s), 1.40 (3H, d, J=6.1 Hz) ppm.

### Example 147: 7,13-dioxa-4-thia-9,18,19,22-tetraazatetracyclo[12.5.2.1^{2,5}.0^{17,20}]docosa-1(19),2,5(22),14(21),15,17(20)-hexaen-8-one

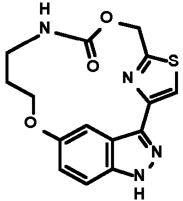

Example 147 is prepared according to the synthesis route described in general Scheme L.

### Preparation of intermediate 195: (4-bromothiazol-2-yl)methyl-N-(3-hydroxypropyl) carbamate

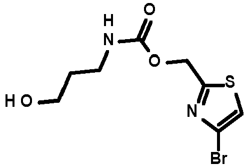

To a solution of 4-nitrophenyl chloroformate (572 mg, 2.84 mmol) and pyridine (0.416 mL, 5.15 mmol) in dichloromethane (10 mL) was added dropwise at room temperature (4-bromothiazol-2-yl)methanol (500 mg, 2.58 mmol) in dichloromethane (3 mL). The reaction mixture was stirred at room temperature for 1 h. A mixture of 3-aminopropan-1-ol (213 mg, 2.84 mmol) and DIPEA (0.896 mL, 5.15 mmol) in dichloromethane (2 mL) was added. The reaction mixture was stirred at room temperature for 2 h. The residue was diluted with 0.5N aqueous sodium hydroxide solution and extracted with dichloromethane. The combined organic layer was washed once again with 0.5N aqueous sodium hydroxide solution and dried over anhydrous sodium sulfate and concentrated under reduced pressure. The residue was purified by silica gel column chromatography using dichloromethane/ethyl acetate 100/0 to 40/60 as eluent to afford (4-bromothiazol-2-yl)methyl N-(3-hydroxypropyl)carbamate intermediate **195** as a colorless oil which crystallized.
LCMS method F: [M+H]⁺ = 295-297, t_{R} = 1.51 min

### Preparation of intermediate 196: 2-[4-[5-[tert-butyl(dimethyl)silyl]oxy-1-tetrahydro pyran-2-yl-indazol-3-yl]thiazol-2-yl]ethyl-N-(3-hydroxypropyl) carbamate

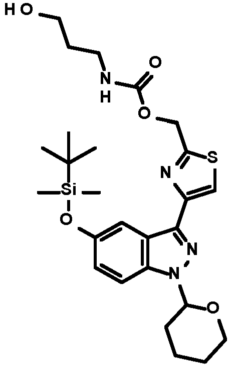

To a solution of *tert*-butyl-dimethyl-[1-tetrahydropyran-2-yl-3-(4,4,5,5-tetramethyl-1,3,2-dioxaborolan-2-yl)indazol-5-yl]oxy-silane (1.091 g, 2.38 mmol) in 1,4-dioxane (15 mL) and water (1.5 mL) at room temperature was added (4-bromothiazol-2-yl)methyl N-(3-hydroxypropyl)carbamate intermediate **195** (585 mg, 1.98 mmol), potassium phosphate tribasic (1.263 g, 5.95 mmol), XPhos (95 mg, 0.20 mmol) and tetrakis(triphenyl phosphine)palladium(0) (115 mg, 0.10 mmol). The reaction mixture was stirred under microwave irradiations at 90°C for 1.5 h. The residue was diluted with saturated sodium chloride solution and extracted with ethyl acetate. The combined organic layer was dried over anhydrous sodium sulfate and concentrated under reduced pressure. The residue was purified by silica gel column chromatography cyclohexane/ethyl acetate 100/0 to 0/100 as eluent to afford 2-[4-[5-[tert-butyl(dimethyl) silyl]oxy-1-tetrahydropyran-2-yl-indazol-3-yl]thiazol-2-yl]ethyl-N-(3-hydroxypropyl) carbamate intermediate **196** as a colorless oil.
LCMS method F: [M+H]⁺ = 547.4, t_{R} = 3.27 min

### Preparation of intermediate 197: 3-[[4-[5-[tert-butyl(dimethyl)silylloxy-1-tetrahydro pyran-2-yl-indazol-3-yl]thiazol-2-yl]methoxycarbonylamino]propylmethanesulfonate

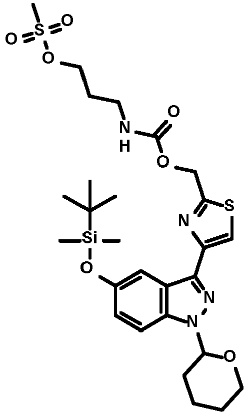

To a solution of [4-[5-[tert-butyl(dimethyl)silyl]oxy-1-tetrahydropyran-2-yl-indazol-3-yl]thiazol-2-yl]methyl N-(3-hydroxypropyl)carbamate intermediate **196** (500 mg, 0.91 mmol) and triethylamine (0.255 mL, 1.83 mmol) in dichloromethane (6 mL) at 0°C was added methanesulfonyl chloride (0.092 mL, 1.19 mmol) in dichloromethane (2 mL). The reaction mixture was stirred at room temperature for 16 h. The residue was diluted with saturated aqueous sodium chloride solution and extracted with dichloromethane. The combined organic layers were dried over anhydrous sodium sulfate and concentrated under reduced pressure to afford 3-[[4-[5-[tert-butyl(dimethyl)silyl]oxy-1-tetrahydropyran-2-yl-indazol-3-yl]thiazol-2-yl]methoxycarbonylamino]propyl methanesulfonate intermediate **197** as a yellow oil which was used in the next step without further purification.
LCMS method F: [M+H]⁺ = 625.4, t_{R} = 3.41 min

### Preparation of intermediate 198: 18-(oxan-2-yl)-7,13-dioxa-4-thia-9,18,19,22-tetraaza tetracyclo[12.5.2.1^{2,5}.0^{1 7,20}]docosa-1(19),2,5(22),14(21),15,17(20)-hexaen-8-one

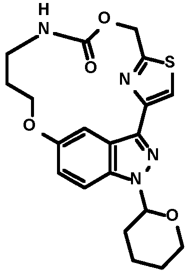

To a suspension of cesium carbonate (532 mg, 1.63 mmol) in anhydrous *N,N-*dimethylformamide (135 mL) at 85°C was added dropwise 3-[[4-[5-[tert-butyl(dimethyl) silyl]oxy-1-tetrahydropyran-2-yl-indazol-3-yl]thiazol-2-yl]methoxycarbonylamino]propyl methanesulfonate intermediate **197** (340 mg, 0.54 mmol) in *N,N*-dimethylformamide (135 mL). The reaction mixture was stirred at 85°C for 30 min. The solvent was evaporated under reduced pressure, diluted with saturated aqueous sodium chloride solution and extracted with ethyl acetate. The organic phase was dried over anhydrous sodium sulfate and evaporated under reduced pressure. The residue was purified by silica gel column chromatography using dichloromethane/ethyl acetate, 100/0 to 80/20 as eluent to afford 18-(oxan-2-yl)-7,13-dioxa-4-thia-9,18,19,22-tetraazatetracyclo[12.5.2.1^{2,5}.0^{17,20}]docosa-1(19),2,5(22),14(21),15,17(20)-hexaen-8-one intermediate **198** as a colorless oil which crystallized.
LCMS method F: [M+H]⁺ = 415.1, t_{R} = 3.47 min

### Preparation of example 147: 7,13-dioxa-4-thia-9,18,19,22-tetraazatetracyclo [12.5.2.1^{2,5}.0^{17,20}]docosa-1(19),2,5(22),14(21),15,17(20)-hexaen-8-one

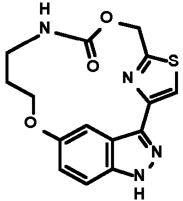

To a solution of 18-(oxan-2-yl)-7,13-dioxa-4-thia-9,18,19,22-tetraazatetracyclo [12.5.2. 1^{2,5}.0^{17,20}]docosa-1(19),2,5(22),14(21),15,17(20)-hexaen-8-one intermediate **198** (67 mg, 0.16 mmol) in methanol (3.5 mL) and water (0.5 mL) was addedp-toluenesulfonic acid monohydrate (154 mg, 0.81 mmol). The reaction mixture was stirred at 65°C for 2 h. The solvent was evaporated under reduced pressure and the residue was neutralized by slow addition of saturated aqueous sodium bicarbonate solution. The residue was diluted with ethyl acetate. After separation, the aqueous phase was extracted with ethyl acetate. The organic layers were washed with brine, dried over anhydrous sodium sulfate, filtered and evaporated under reduced pressure. The residue was triturated in acetonitrile, filtered and dried to afford 7,13-dioxa-4-thia-9,18,19,22-tetraazatetracyclo[12.5.2.1^{2,5}.0^{17,20}]docosa-1(19),2,5(22),14(21),15,17(20)-hexaen-8-one **example 147** as a white solid.
LCMS method F: [M+H]⁺ = 331.2, t_{R} = 1.75 min
LCMS method G: [M+H]⁺ = 331.2, t_{R} = 1.75 min
¹H NMR (400 MHz, d6-DMSO) δ 13.04 (1H, s), 8.00 - 7.94 (3H, m), 7.44 - 7.41 (1H, m), 6.95 (1H, dd, J=2.6, 9.0 Hz), 5.52 (2H, t, J=17.5 Hz), 4.32 - 4.26 (2H, m), 3.12 (2H, s), 2.08 - 1.99 (2H, m) ppm.

### Example 148: (13R)-4,13-dimethyl-8,14-dioxa-5,10,19,20,23-pentaazatetracyclo [13.5.2. 1^{2,6}.0^{18,21}]tricosa-1(20),2(23),3,5,15(22),16,18(21)-heptaen-9-one

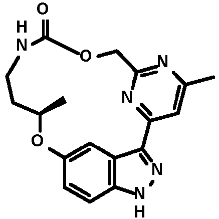

Example 148 is prepared according to the synthesis route described in general Scheme O.

### Preparation of intermediate 199: [(1S)-3-(benzyloxycarbonylamino)-1-methyl-propyl] methane sulfonate

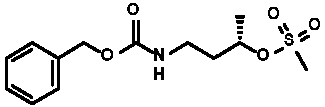

To a cooled solution (0°C) of benzyl N-[(3S)-3-hydroxybutyl]carbamate (17.06 g, 76.52 mmol) and triethylamine (21.3 mL, 153.04 mmol) in dichloromethane (300 mL) was added dropwise methanesulfonyl chloride (7.7 mL, 99.47 mmol) and the reaction mixture was stirred at room temperature for 19 hours. The reaction mixture was quenched with an aqueous solution of 1N HCl and extracted with dichloromethane (1x). The organic layer was washed with a saturated aqueous solution of NaHCO3 then water and brine, dried over sodium sulfate, filtered and evaporated under reduced pressure to give [(1S)-3-(benzyloxycarbonylamino)-1-methylpropyl] methanesulfonate intermediate **199** as a yellow oil.
LCMS method F: [M+H]⁺ = 302, t_{R} = 2.26 min

### Preparation of intermediate 200: [4-[5-[tert-butyl(dimethyl)silyl]oxy-1-tetrahydropyran-2-yl-indazol-3-yl]-6-methyl-pyrimidin-2-yl]methyl acetate

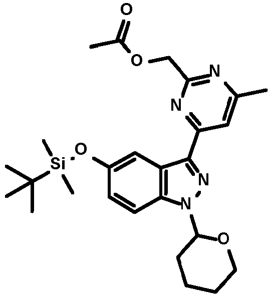

To a solution of *tert*-butyl-dimethyl-[1-tetrahydropyran-2-yl-3-(4,4,5,5-tetramethyl-1,3,2-dioxaborolan-2-yl)indazol-5-yl]oxy-silane (1.37 g, 3 mmol) in 1,4-dioxane (10 mL) and water (1 mL) at room temperature was added (4-chloro-6-methyl-pyrimidin-2-yl)methyl acetate (499 mg, 2.5 mmol), potassium phosphate tribasic (1.59 g, 7.5 mmol). The reaction mixture was purged with nitrogen for 15 min then XPhos (36 mg, 0.075 mmol) and tetrakis(triphenylphosphine)palladium(0) (29 mg, 0.025 mmol) were added. The reaction mixture was stirred at 80°C for 45 min under microwave irradiations. The reaction mixture was filtered over Celite pad and the filtrate was diluted with ethyl acetate and water. After separation, the aqueous layer was extracted with ethyl acetate. The organic layers were washed with brine, dried over anhydrous sodium sulfate, filtered and concentrated under reduced pressure. The residue was purified by silica gel column chromatography using cyclohexane/ethyl acetate 100/0 to 80/20 as eluent to afford *[4-[5-[tert-*butyl(dimethyl)silyl]oxy-1-tetrahydropyran-2-yl-indazol-3-yl]-6-methyl-pyrimidin-2-yl] methyl acetate intermediate **200** as an orange oil.
LCMS method F: [M+H]⁺ = 497.3, t_{R} = 3.68 min

### Preparation of intermediate 201: 3-[2-(hydroxymethyl)-6-methyl-pyrimidin-4-yl]-1-tetrahydropyran-2-yl-indazol-5-ol

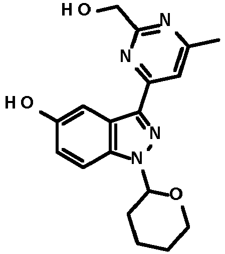

To a solution of [4-[5-[tert-butyl(dimethyl)silyl]oxy-1-tetrahydropyran-2-yl-indazol-3-yl]-6-methyl-pyrimidin-2-yl]methyl acetate intermediate **200** (1.33 g, 2.5 mmol) in methanol (12 mL) and water (12 mL) at room temperature was added potassium carbonate (690 mg, 5 mmol). The reaction mixture was stirred at 50°C for 24 h. Methanol was evaporated under reduced pressure. The resulting precipitate was filtrated, washed with water and dried to afford 3-[2-(hydroxymethyl)-6-methyl-pyrimidin-4-yl]-1-tetrahydropyran-2-yl-indazol-5-ol intermediate 201 as a cream powder.
LCMS method F: [M+H]⁺ = 341.2, t_{R} = 1.99 min

### Preparation of intermediate 202: (13R)-4,13-dimethyl-19-(oxan-2-yl)-8,14-dioxa-5,10,19, 20,23-pentaazatetracyclo[13.5.2.1^{2,6}.0^{18,21}]tricosa-1(20),2(23),3,5,15(22),16,18(21)-heptaen-9-one

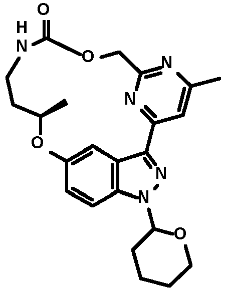

To a mixture of 3-[2-(hydroxymethyl)-6-methyl-pyrimidin-4-yl]-1-tetrahydropyran-2-yl-indazol-5-ol intermediate **201** (361 mg, 1.06 mmol) in N,N-dimethylformamide (8 mL) at room temperature was added cesium carbonate (689 mg, 2.12 mmol). The reaction mixture was stirred for 20 min and [(1S)-3-(benzyloxycarbonylamino)-1-methyl-propyl] methanesulfonate intermediate **199** (383 mg, 1.27 mmol) in *N,N*-dimethylformamide (2 mL) was added. The reaction mixture was stirred at room temperature for 16 h. Additional [(1S)-3-(benzyloxycarbonylamino)-1-methyl-propyl] methanesulfonate intermediate **199** (64 mg, 0.21 mmol) in *N,N-*dimethylformamide (1 mL) was added. The reaction mixture was stirred at room temperature for 2 h then diluted with DMF (106 mL) and cesium carbonate (1.03 g, 3.18 mmol) was added. The reaction mixture was heated at 50°C for 16 h. Additional cesium carbonate (344 mg, 1.06 mmol) was added and the reaction mixture was heated at 50 °C for 3 h. The reaction mixture was concentrated under reduced pressure then diluted with ethyl acetate and water. After separation, the aqueous layer was extracted with ethyl acetate. The combined organic layers were washed with brine, dried over anhydrous sodium sulfate, filtered and concentrated under reduced pressure. The residue was purified by silica gel column chromatography using cyclohexane/ethyl acetate 100/0 to 50/50 as eluent to afford (13R)-4,13-dimethyl-19-(oxan-2-yl)-8,14-dioxa-5,10,19,20,23-pentaazatetracyclo[13.5.2.1^{2,6}.0^{18,21}] tricosa-1(20),2(23),3,5,15(22),16,18(21)-heptaen-9-one intermediate **202** as a white solid.
LCMS method F: [M+H]⁺ = 438.3, t_{R} = 2.57 min

### Preparation of example 148: (13R)-4,13-dimethyl-8,14-dioxa-5,10,19,20,23-pentaazatetra cyclo[13.5.2.1^{2,6}.0^{18,21}]tricosa-1(20),2(23),3,5,15(22),16,18(21)-heptaen-9-one

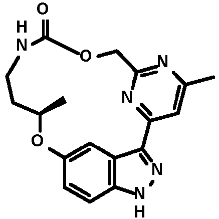

To a solution of (13R)-4,13-dimethyl-19-(oxan-2-yl)-8,14-dioxa-5,10,19,20,23-pentaaza tetracyclo[13.5.2.1^{2,6}.0^{18,21}]tricosa-1(20),2(23),3,5,15(22),16,18(21)-heptaen-9-one intermediate **202** (407 mg, 0.93 mmol) in methanol (16 mL) and water (2.6 mL) was added*p-*toluenesulfonic acid monohydrate (884 mg, 4.66 mmol). The reaction mixture was stirred at 65°C for 16 h. Additional *p*-toluenesulfonic acid monohydrate (176 mg, 0.93 mmol) was added and the reaction mixture was heated at 65 °C for 24 h. The reaction mixture was evaporated under reduced pressure then diluted with dichloromethane and saturated aqueous solution of sodium bicarbonate. After separation, the aqueous layer was extracted with dichloromethane. The combined organic layers were washed with brine, dried over sodium sulfate, filtered and evaporated under reduced pressure. The residue was triturated in acetonitrile, filtrated, washed with acetonitrile and dried to afford (13R)-4,13-dimethyl-8,14-dioxa-5,10,19,20,23-pentaazatetracyclo[13.5.2.1^{2,6}.0^{18,21}]tricosa-1(20),2(23),3,5,15(22),16,18(21)-heptaen-9-one **example 148** as a white powder.
LCMS method F: [M+H]⁺ = 354.3, t_{R} = 1.89 min
LCMS method G: [M+H]⁺ = 354.3, t_{R} = 1.91 min
¹H NMR (400 MHz, d6-DMSO) δ 13.64 (1H, m), 7.94 (1H, s), 7.91 (1H, d, J= 2.3 Hz), 7.86 (1H, m), 7.51 (1H, d, J= 8.9 Hz), 6.99 (1H, dd, J=2.4, 9.0 Hz), 5.54 (1H, m), 4.95 (1H, m), 4.61 (1H, m), 3.52 (1H, m), 2.89 (1H, m), 2.50 (3H, s), 2.34 (1H, m), 1.39 (3H, d, J=6.1 Hz), 1.34 (1H, m) ppm.

### Example 149: 8,14-dioxa-23-thia-4,10,19,20-tetraazatetracyclo[13.5.2.1^{2,5}.0^{18,21}]tricosa-1(20),2,4,15(22),16,18(21)-hexaen-9-one

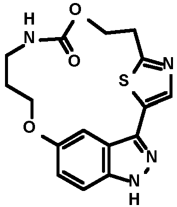

Example 149 is prepared according to the synthesis route described in general Scheme L.

### Preparation of intermediate 203: 2-(5-bromothiazol-2-yl)ethyl N-(3-hydroxypropyl) carbamate

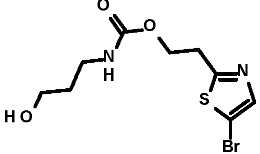

To a solution of 4-nitrophenyl chloroformate (266 mg, 1.32 mmol) and pyridine (0.194 mL, 2.40 mmol) in dichloromethane (5 mL) at room temperature was added dropwise 2-(5-bromothiazol-2-yl)ethanol (250 mg, 1.20 mmol) in dichloromethane (3 mL). The reaction mixture was stirred at room temperature for 1 h then a mixture of 3-aminopropan-1-ol (99 mg, 1.32 mmol) and DIPEA (0.418 mL, 2.40 mmol) in dichloromethane (2 mL) were added. The reaction mixture was stirred at room temperature for 2 h. The residue was diluted with 0.5 N aqueous sodium hydroxide solution and extracted with dichloromethane. The combined organic layers were washed once again with 0.5 N aqueous sodium hydroxide solution, dried over anhydrous sodium sulfate, and concentrated under reduced pressure. The residue was purified by silica gel column chromatography using dichloromethane/ethyl acetate 100/00 to 30/70 as eluent to afford 2-(5-bromothiazol-2-yl)ethyl N-(3-hydroxypropyl)carbamate intermediate 203 as a yellow oil.
LCMS method F: [M+H]⁺ = 309-311, t_{R} = 1.71 min

### Preparation of intermediate 204: 2-[5-[5-[tert-butyl(dimethyl)silyl]oxy-1-tetrahydro pyran-2-yl-indazol-3-yl]thiazol-2-yl]ethyl N-(3-hydroxypropyl)carbamate

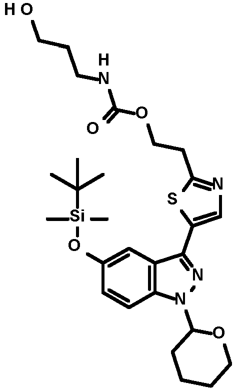

To a solution of *tert*-butyl-dimethyl-[1-tetrahydropyran-2-yl-3-(4,4,5,5-tetramethyl-1,3,2-dioxaborolan-2-yl)indazol-5-yl]oxy-silane (409 mg, 0.89 mmol) in 1,4-dioxane (2 mL) and water (0.2 mL) at room temperature was added ***2-(5-bromothiazol-2-yl)ethyl*** N-(3-hydroxypropyl)carbamate intermediate **203** (230 mg, 0.74 mmol), potassium phosphate tribasic (474 mg, 2.23 mmol), XPhos (35 mg, 0.07 mmol) and tetrakis(triphenylphosphine)palladium(0) (43 mg, 0.04 mmol). The reaction mixture was stirred under microwave irradiations at 90°C for 1.5 h. The reaction mixture was diluted with saturated sodium chloride solution and extracted with ethyl acetate. The combined organic layers were dried over anhydrous sodium sulfate and concentrated under reduced pressure. The residue was purified by silica gel column chromatography using cyclohexane/ethyl acetate 100/0 to 0/100 as eluent to afford 2-[5-[5-[tert-butyl(dimethyl)silyl]oxy-1-tetrahydropyran-2-yl-indazol-3-yl]thiazol-2-yl]ethyl N-(3-hydroxypropyl)carbamate intermediate **204-as** a colorless oil.
LCMS method F: [M+H]⁺ = 561.3, t_{R} = 3.23 min

### Preparation of intermediate 205: 3-[2-[5-[5-[tert-butyl(dimethyl)silyl]oxy-1-tetrahydro pyran-2-yl-indazol-3-yl]thiazol-2-yl]ethoxycarbonylamino]propyl methanesulfonate

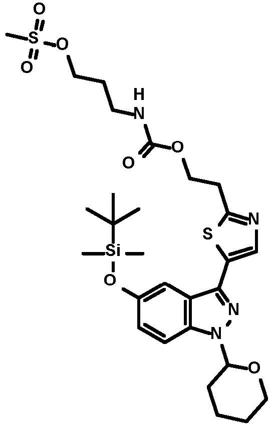

To a solution of 2-[5-[5-[*tert*-butyl(dimethyl)silyl]oxy-1-tetrahydropyran-2-yl-indazol-3-yl]thiazol-2-yl]ethyl N-(3-hydroxypropyl)carbamate intermediate **204** (345m g, 0.62 mmol) and triethylamine (0.172 mL, 1.23 mmol) in dichloromethane (5 mL) at 0°C was added methanesulfonyl chloride (0.062 mL, 0.80 mmol) in dichloromethane (1 mL). The reaction mixture was stirred at room temperature for 2 h. The reaction mixture was diluted with saturated aqueous sodium chloride solution and extracted with dichloromethane. The combined organic layers were dried over anhydrous sodium sulfate and concentrated under reduced pressure to afford 3-[2-[5-[5-[tert-butyl(dimethyl)silyl]oxy-1-tetrahydropyran-2-yl-indazol-3-yl]thiazol-2-yl]ethoxycarbonylamino]propylmethanesulfonate intermediate **205** as a yellow oil which was used in the next step without further purification.
LCMS method F: [M+H]⁺ = 639.3, t_{R} = 3.37 min

### Preparation of intermediate 206: 19-(oxan-2-yl)-8,14-dioxa-23-thia-4,10,19,20-tetraaza tetracyclo[13.5.2.1^{2,5}.0^{18,21}]tricosa-1(20),2,4,15(22),16,18(21)-hexaen-9-one

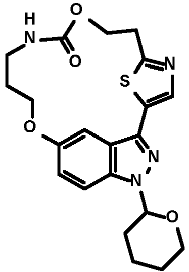

To a suspension of cesium carbonate (601 mg, 1.85 mmol) in anhydrous *N,N-*dimethylformamide (155 mL) at 85°C was added dropwise 3-[2-[5-[5-[tert-butyl(dimethyl)silyl]oxy-1-tetrahydropyran-2-yl-indazol-3-yl]thiazol-2-yl]ethoxycarbonyl amino]propylmethanesulfonate intermediate **205** (393 mg, 0.62 mmol) in *N,N-*dimethylformamide (155 mL). The reaction mixture was stirred at 85°C for 2 h. The solvent was evaporated under reduced pressure, diluted with saturated sodium chloride solution and extracted with ethyl acetate. The combined organic layers were dried over anhydrous sodium sulfate and concentrated under reduced pressure. The residue was purified by silica gel column chromatography using cyclohexane/(ethyl acetate/ethanol 3/1) 100/0 to 60/40 as eluent to afford 19-(oxan-2-yl)-8,14-dioxa-23-thia-4,10,19,20-tetraazatetracyclo[13.5.2.1^{2,5}.0^{18,21}] tricosa-1(20),2,4,15(22),16,18(21)-hexaen-9-one intermediate **206** as a white solid.
LCMS method F: [M+H]⁺ = 429.4, t_{R} = 2.27 min

### Preparation of Example 149: 8,14-dioxa-23-thia-4,10,19,20-tetraazatetracyclo [13.5.2.1^{2,5}.0^{18,21}]tricosa-1(20),2,4,15(22),16,18(21)-hexaen-9-one

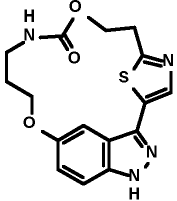

To a solution of 19-(oxan-2-yl)-8,14-dioxa-23-thia-4,10,19,20-tetraazatetracyclo [13.5.2. 1^{2,5}.0^{18,21}]tricosa-1(20),2,4,15(22),16,18(21)-hexaen-9-one intermediate **206** (19 mg, 0.04 mmol) in methanol (14 mL) and water (2 mL) was added *p*-toluenesulfonic acid monohydrate (42 mg, 0.22 mmol) and the reaction mixture was stirred at 70°C for 24 h. The solvent was evaporated under reduced pressure and the residue was quenched by slow addition of saturated aqueous sodium hydrogen carbonate solution. The residue was diluted with ethyl acetate. The phases were separated and the aqueous phase was extracted with ethyl acetate. The combined organic layers were washed with brine, dried over anhydrous sodium sulfate, filtered and evaporated under reduced pressure. The residue was triturated in acetonitrile, filtered and dried to afford 8,14-dioxa-23-thia-4,10,19,20-tetraazatetracyclo[13.5.2.1^{2,5}.0^{18,21}]tricosa-1(20),2,4, 15(22),16, 18(21)-hexaen-9-one **example 149** as a white solid.
LCMS method F: [M+H]⁺ = 345.3, t_{R} = 1.70 min
LCMS method G: [M+H]⁺ = 345.3, t_{R} = 1.71 min
¹H NMR (400 MHz, d6-DMSO) δ 13.23 - 13.23 (1H, m), 8.00 - 8.00 (2H, m), 7.51 - 7.48 (1H, m), 7.42 (1H, d, J=2.3 Hz), 7.00 (1H, dd, J=2.3, 8.9 Hz), 4.41 (2H, t, J=5.2 Hz), 4.34 - 4.29 (2H, m), 3.41 - 3.35 (2H, m), 3.14 - 3.11 (2H, m), 1.91 - 1.84 (2H, m) ppm.

### Example 150: (7S,13R)-7,13-dimethyl-8,14-dioxa-10,19,20,23-tetraazatetracyclo[13.5.2. 1^{2,6}.0^{18,21}]tricosa-1(20),2(23),3,5,15(22),16,18(21)-heptaen-9-one

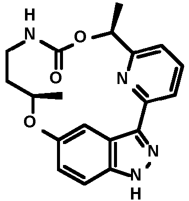

Example 150 is prepared according to the synthesis route described in general Scheme K.

### Preparation of intermediate 207: 1-(6-bromo-2-pyridyl)ethanol

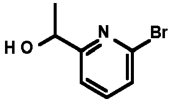

To a solution of 1-(6-bromo-2-pyridyl)ethanone (6.0 g, 30.0 mmol) in dry methanol (80 mL) at 0 °C was added solution sodium borohydride (2.30 mL, 89.9 mmol) in small portions. The reaction mixture was warmed up to room temperature and was stirred for 16 h. The reaction mixture was quenched with water then carefully by the addition of 1M aqueous hydrochloric acid solution. The reaction mixture was extracted with ethyl acetate. The organic phase was washed with brine, dried over anhydrous magnesium, filtered and evaporated to afford 1-(6-bromo-2-pyridyl)ethanol intermediate **207** as a colorless oil.
¹H NMR (500 MHz, d6-DMSO) δ 7.74 (t, 1 H), 7.53 (d, 1 H), 7.49 (d, 1 H), 5.5 (d), 4.67 (m, 1 H), 1.34 (d, 3 H) ppm.

### Preparation of intermediate 208: 1-{6-[5-{[tert-butyl(dimethyl)silyl]oxy}-1-(oxan-2-yl)-1H-indazol-3-yl]pyridin-2-yl}ethan-1-ol

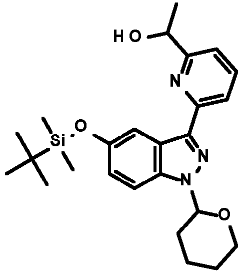

5-{[*tert*-butyl(dimethyl)silyl]oxy}-1-(oxan-2-yl)-3-(4,4,5,5-tetramethyl-1,3,2-dioxaborolan-2-yl)-1H-indazole (2.4 g, 5.2 mmol), 1-(6-bromo-2-pyridyl)ethanol intermediate **207** (1.0 g, 4.9 mmol) and potassium phosphate monohydrate (2.3 g, 9.9 mmol) in a mixture of 1,4-dioxane (50 mL) and water (5 mL) was purged with nitrogen then palladium triphenylphosphane (290 mg, 0.25 mmol) was added. The reaction mixture was heated at 100°C for 1 h. After cooling to room temperature, the reaction mixture was diluted with ethyl acetate and water. The aqueous layer was extracted with ethyl acetate. The combined organic layers were washed with brine, dried over magnesium sulfate, filtered and concentrated under reduced pressure. The residue was purified by silica gel column chromatography using hepthane/ethyl acetate 20/80 to 30/70 to afford 1-{ 6-[5-{[*tert*-butyl(dimethyl)silyl]oxy}-1-(oxan-2-yl)-1*H*-indazol-3-yl]pyridin-2-yl} ethan-1-ol intermediate **208** as a colorless oil.

¹H NMR (500 MHz, d6-DMSO) δ δ (m, 2 H), 8.11 (d, 1 H), 7.97 (d, 1 H), 7.87 (t, 1 H), 7.67 (d, 1 H), 7.48 (d, 1 H), 7.04 (dd, 1 H), 5.88 (d, 1 H), 5.48 (d, 1 H), 4.85 (m, 1 H), 3.91/3.76 (d+m, 2 H), 2.47/2.02 (m+m, 2 H), 2.07/1.77 (m+m, 2 H), 1.5 (d, 3 H), 0.99 (s, 9 H), 0.23 (s, 6 H) ppm.

### Preparation of intermediate 209: benzyl [(3R)-3-({3-[6-(1-hydroxyethyl)pyridin-2-yl]-1-(oxan-2-yl)-1H-indazol-5-yl}oxy)butyl]carbamate

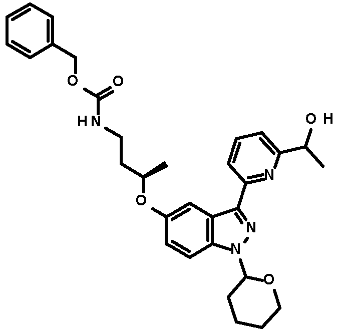

To a solution of 1-{6-[5-{[*tert-*butyl(dimethyl)silyl]oxy}-1-(oxan-2-yl)-1*H*-indazol-3-yl]pyridin-2-yl}ethan-1-ol intermediate **208** (1.84 g, 4.06 mmol) in acetonitrile (81.1 mL) was added [(1S)-3-(benzyloxycarbonylamino)-1-methyl-propyl]methanesulfonate intermediate **199** (1.47 g, 4.87 mmol) in acetonitrile (18.4 mL) and cesium carbonate (3.970 g, 12.2 mmol) at RT. The reaction mixture heated to 50°C and stirred for 22 h. The reaction mixture was filtered and the solvent was evaporated under reduced pressure. The residue was partitioned between ethyl acetate and water. After separation, the aqueous phase washed with ethyl acetate. The combined organic phases were washed with brine, dried over anhydrous magnesium sulfate. The solvent was evaporated under reduced pressure and heptane/ethyl acetate (80/20) was added to the crude product. The precipitate was filtered off to afford benzyl [(3R)-3-({3-[6-(1-hydroxyethyl)pyridin-2-yl]-1-(oxan-2-yl)-1H-indazol-5-yl}oxy)butyl] carbamate intermediate **209** as an off-white solid.

¹H NMR (500 MHz, d6-DMSO) δ 8.12 (d, 1 H), 7.96 (d, 1 H), 7.88 (t, 1 H), 7.68 (d, 1 H), 7.49 (d, 1 H), 7.1 (dd, 1 H), 5.89 (d, 1 H), 5.46 (d, 1 H), 4.99/4.98 (s/s, 2 H), 4.88 (m, 1 H), 4.49 (m, 1 H), 3.91/3.76 (d+m, 2 H), 3.18 (m, 2 H), 1.87/1.79 (m+m, 2 H), 1.53 (d, 3 H), 1.32/1.31 (d/d, 3 H) ppm.

### Preparation of intermediate 210: (7S,13R)-7,13-dimethyl-19-(oxan-2-yl)-8,14-dioxa-10,19,20,23-tetraazatetracyclo[13.5.2.1^{2,6}.0^{18,21}]tricosa-1(20),2(23),3,5,15(22),16,18(21)-heptaen-9-one

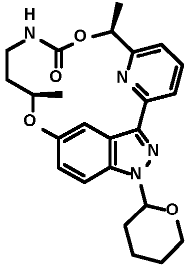

To benzyl [(3R)-3-({3-[6-(1-hydroxyethyl)pyridin-2-yl]-1-(oxan-2-yl)-1H-indazol-5-yl}oxy) butyl]carbamate intermediate **209** (500 mg, 0.918 mmol) in acetonitrile (45 mL) was added potassium hydroxide (258 mg, 4.59 mmol). The reaction mixture was stirred at room temperature for 3 h. The reaction mixture was filtered then ethyl acetate and water were added to the filtrate. After separation, the organic phase was washed with brine, dried over anhydrous magnesium sulfate, filtered and evaporated under reduced pressure. The residue was purified by silica gel column chromatography using heptane/ethyl acetate 90/10 to 70/30 as eluent. The two diastereomers were separated. First eluted (7R,13R)-7,13-dimethyl-19-(oxan-2-yl)-8,14-dioxa-10,19,20,23-tetraazatetracyclo[13.5.2.1^{2,1}.0^{11,21}]tricosa-1(20),2(23),3,5,15(22), 16,18(21)-heptaen-9-one as a white solid. Second eluted the (7S,13R)-7,13-dimethyl-19-(oxan-2-yl)-8,14-dioxa-10,19,20,23-tetraazatetracyclo[13.5.2.1^{2,6}.0^{11,21}]tricosa-1(20),2(23), 3,5,15(22),16,18(21)-heptaen-9-one intermediate 210 as a white solid.

¹H NMR (500 MHz, d6-DMSO) δ (m, 6 H), 8.02 (d, 1 H), 7.87 (t, 1 H), 7.81/7.8 (d/d, 1 H), 7.76/7.74 (t/t, 1 H), 7.66 (d, 1 H), 7.35 (d, 1 H), 7.02 (dd, 1 H), 5.88 (m, 1 H), 5.87 (m, 1 H), 4.55 (m, 1 H), 3.9/3.76 (m+m, 2 H), 3.54/2.82 (m+m, 2 H), 2.27/1.36 (m+m, 2 H), 1.61 (d, 3 H), 1.35 (d, 3 H) ppm.

### Preparation of Example 150: (7S,13R)-7,13-dimethyl-8,14-dioxa-10,19,20,23-tetraaza tetracyclo[13.5.2.1^{2,6}.0^{18,21}]tricosa-1(20),2(23),3,5,15(22),16,18(21)-heptaen-9-one

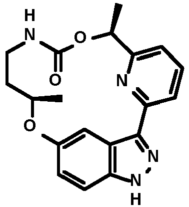

(7S,13R)-7,13-dimethyl-19-(oxan-2-yl)-8,14-dioxa-10,19,20,23-tetraazatetracyclo[ 13.5.2. 1^{2,6}.0^{18,21}]tricosa-1(20),2(23),3,5,15(22),16,18(21)-heptaen-9-one intermediate **210** (200 mg, 0.46 mmol) and p-toluenesulfonic acid monohydrate (0.407 mL, 2.291 mmol) were added to methanol (33 mL) and water (4.5 mL). The reaction mixture was stirred at 65°C for 16 h. Methanol was removed partially by evaporation under reduced pressure and saturated aqueous sodium bicarbonate solution was added. The aqueous phase was washed with ethyl acetate. the organic phase was washed with brine, dried over anhydrous magnesium sulfate, filtered and concentrated under reduced pressure. The residue was purified by silica gel column chromatography using heptane/ethyl acetate 70/30 to 40/60 to afford (7S,13R)-7,13-dimethyl-8,14-dioxa-10,19,20,23-tetraazatetracyclo[13.5.2.1^{2,6}.0^{18,21}]tricosa-1(20),2(23),3,5,15(22),16, 18(21)-heptaen-9-one **example 150** as a white solid.

¹H NMR (500 MHz, d6-DMSO) δ 13.21 (s, 1 H), 8.03 (d, 1 H), 7.84 (t, 1 H), 7.8 (d, 1 H), 7.74 (dd, 1 H), 7.45 (d, 1 H), 7.31 (d, 1 H), 6.95 (dd, 1 H), 5.87 (q, 1 H), 4.54 (m, 1 H), 3.54/2.82 (m+m, 2 H), 2.28/1.35 (m+m, 2 H), 1.61 (d, 3 H), 1.34 (d, 3 H) ppm.
LCMS method F: [M+H]⁺ = 353.3, t_{R} = 2.04 min
LCMS method G: [M+H]⁺ = 353.2, t_{R} = 2.10 min

### Example 151: (13R)-13-methyl-9-oxo-8,14-dioxa-5,10,19,20-tetraazatetracyclo[13.5.2. 1^{2,5}.0^{18,21}]tricosa-1(20),2(23),3,15(22),16,18(21)-hexaene-4-carbonitrile

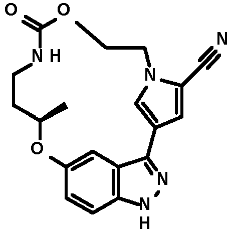

Example 151 is prepared according to the synthesis route described in general Scheme O.

### Preparation of intermediate 211: 4-[5-[tert-butyl(dimethyl)silylloxy-1-tetrahydropyran-2-yl-indazol-3-yl]-1-(2-hydroxyethyl)pyrrole-2-carbonitrile

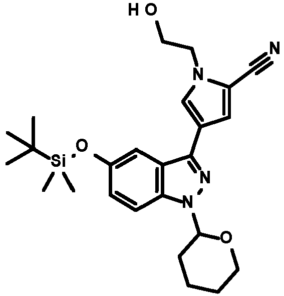

To a solution of tert-butyl-dimethyl-[1-tetrahydropyran-2-yl-3-(4,4,5,5-tetramethyl-1,3,2-dioxaborolan-2-yl)indazol-5-yl]oxy-silane (936 mg, 2.045 mmol), 4-bromo-1-(2-hydroxyethyl)pyrrole-2-carbonitrile (350 mg, 1.635 mmol) and potassium phosphate tribasic (1.04 g, 4.905 mmol) in 1,4-dioxane (3.5 mL) and water (250 µL) were added tetrakis(triphenylphosphine)palladium(0) (94 mg, 0.0817 mmol) and 2-dicyclohexylphosphino-2',4',6'-triisopropylbiphenyl (78 mg, 0.163 mmol). The reaction mixture was heated at 100 °C for 1h. The reaction mixture was filtered through a Celite pad and washed with ethyl acetate. The filtrate was diluted with water and extracted with ethyl acetate. The organic layer was washed with water and brine, dried over anhydrous magnesium sulfate, filtered and concentrated under reduced pressure. The residue was purified by silica gel column chromatography using cyclohexane/ethyl acetate 100/0 to 50/50 as eluent to afford 4-[5-[tert-butyl(dimethyl)silyl]oxy-1-tetrahydropyran-2-yl-indazol-3-yl]-1-(2-hydroxyethyl) pyrrole-2-carbonitrile intermediate **211** as a yellow oil.
LCMS method F: [M+H]⁺ = 467.3, t_{R} = 3.37 min

### Preparation of intermediate 212: (13R)-13-methyl-19-(oxan-2-yl)-9-oxo-8,14-dioxa-5,10,19,20-tetraazatetracyclo[13.5.2.1^{2,5}.0^{18,21}]tricosa-1(20),2(23),3,15(22),16,18(21)-hexaene-4-carbonitrile

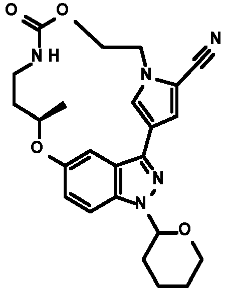

To a suspension of 4-[5-[tert-butyl(dimethyl)silyl]oxy-1-tetrahydropyran-2-yl-indazol-3-yl]-1-(2-hydroxyethyl)pyrrole-2-carbonitrile intermediate 211 (230 mg, 0.493 mmol) and cesium carbonate (480 mg, 1.48mmol) in acetonitrile (60 mL) wad added [(1S)-3-(benzyloxycarbonylamino)-1-methyl-propyl] methanesulfonate (193 mg, 0.642 mmol). The reaction mixture was heated at 50°C 4 h. Additional cesium carbonate (481 mg, 1.48 mmol) and acetonitrile (200 mL) were added. The reaction mixture was stirred at 80°C for 16 h. The reaction mixture was concentrated under reduced pressure. The residue was dissolved between water and ethyl acetate. After separation, the aqueous phase was extracted with ethyl acetate. The organic phase was dried over anhydrous sodium sulfate, filtered and evaporated under reduced pressure. The residue was purified by silica gel column chromatography using cyclohexane/ethyl acetate 100/0 to 70/30 as eluent to afford (13R)-13-methyl-19-(oxan-2-yl)-9-oxo-8,14-dioxa-5,10,19,20-tetraazatetracyclo[13.5.2.1^{2,5}.0^{18,21}]tricosa-1(20),2(23),3,15(22), 16, 18(21)-hexaene-4-carbonitrile intermediate **212** as a white powder.
LCMS method F: [M+H]⁺ = 366.3, t_{R} = 2.10 min

### Preparation of Example 151: (13R)-13-methyl-9-oxo-8,14-dioxa-5,10,19,20-tetraaza tetracyclo[13.5.2.1^{2,5}.0^{18,21}]tricosa-1(20),2(23),3,15(22),16,18(21)-hexaene-4-carbonitrile

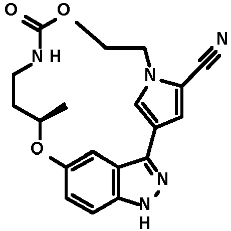

To a solution of (13R)-13-methyl-19-(oxan-2-yl)-9-oxo-8,14-dioxa-5,10,19,20-tetraazatetra cyclo[13.5.2.1^{2,5}.0^{18,21}]tricosa-1(20),2(23),3,15(22),16,18(21)-hexaene-4-carbonitrile intermediate **212** (100 mg, 0.22 mmol) in methanol (10 mL) and water (1.5 mL) was added*p-*toluenesulfonic acid monohydrate (212 mg, 1.11 mmol). The reaction mixture was stirred at 65°C for 2 h. The solvent was evaporated under reduced pressure. The residue was neutralized by slow addition of saturated aqueous sodium hydrogen carbonate solution and was diluted with ethyl acetate. After separation, the aqueous phase was extracted with ethyl acetate. The combined organic layer was washed with a saturated aqueous sodium bicarbonate solution, water and brine, dried over anhydrous sodium sulfate, filtered and evaporated under reduced pressure. The residue was purified by silica gel column chromatography using cyclohexane/ethyl acetate 100/0 to 50/50 as eluent. The oily product was crystallized in dichloromethane to afford (13R)-13-methyl-9-oxo-8,14-dioxa-5,10,19,20-tetraazatetracyclo [13.5.2.1^{2,5}.0^{18,21}]tricosa-1(20),2(23),3,15(22),16,18(21)-hexaene-4-carbonitrile **example 151** as a yellow powder.
LCMS method F: [M+H]⁺ = no mass observed, t_{R} = 2.03 min
LCMS method G: [M+H]⁺ = 366.2, t_{R} = 1.96 min
¹H NMR (400 MHz, d6-DMSO) δ 12.85 (1H, s), 7.84 (1H, dd, J=4.3, 7.7 Hz), 7.54 (1H, d, J=1.7 Hz), 7.44 - 7.40 (1H, m), 7.28 - 7.27 (1H, m), 7.09 (1H, d, J=2.3 Hz), 6.93 (1H, dd, J=2.1, 8.9 Hz), 4.68 - 4.59 (2H, m), 4.46 - 4.37 (2H, m), 4.18 - 4.12 (1H, m), 3.49 - 3.48 (1H, m), 2.96 - 2.88 (1H, m), 2.13 (1H, t, J=14.0 Hz), 1.38 - 1.35 (4H, m) ppm.

### Example 152: 12,12-difluoro-8,14-dioxa-10,19,20-triazatetracyclo[13.5.2.1^{2,6}.0^{18,21}] tricosa-1(20),2(23),3,5,15(22),16,18(21)-heptaen-9-one

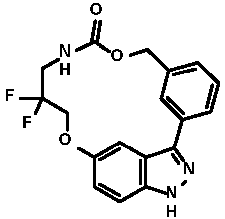

Example 152 is prepared according to the synthesis route described in general Scheme C.

### Preparation of intermediate 213: benzyl N-(2,2-difluoro-3-hydroxy-propyl)carbamate

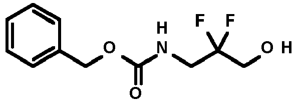

To a solution of 3-amino-2,2-difluoropropan-1-ol (3 g, 27.005 mmol) in a mixture of 40.5 mL of THF and 40.5 mL of water was added sodium hydrogenocarbonate (4.991 g, 59.411 mmol). The suspension was cooled to 0°C and benzyl chloroformate (8.376 mL, 59.411 mmol) was added dropwise and the reaction mixture was stirred at room temperature for 16 h. The product was diluted with EtOAc, an aqueous saturated solution of NaHCO3 was added and the mixture was extracted with EtOAc (x4). The combined organic layers were dried over MgSO4, filtered and the solvents evaporated under reduced pressure. The product was purified by flash chromatography on silica gel, using as eluents heptane/EtOAc (from 100:0 to 75:25). The desired fractions were combined and concentrated under vacuum to afford benzyl (2,2-difluoro-3-hydroxypropyl)carbamate intermediate **213** as a white solid.
LCMS method B: [M+H]⁺ = 246.0, t_{R} = 0.526 min

### Preparation of intermediate 214: benzyl N-[2,2-difluoro-3-(3-iodo-1-tetrahydropyran-2-yl-indazol-5-yl)oxy-propyl]carbamate

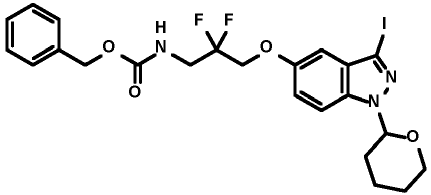

A solution of benzyl (2,2-difluoro-3-hydroxypropyl)carbamate intermediate **213** (2 g, 8.156 mmol) in dry THF (49 mL, 6 mL/mmol) were added 3-iodo-1-(tetrahydro-2H-pyran-2-yl)-1H-indazol-5-ol (3.368 g, 9.787 mmol) and triphenylphosphine (3.209 g, 12.234 mmol) and the mixture was stirred for 5 minutes. DIAD (2.409 mL, 12.234 mmol) was added dropwise and the reaction mixture was stirred at 90°C for 1.5 h. The mixture was contrated under under reduced pressure and the crude product was purified by flash chromatography on silica gel, using as eluents heptane/EtOAc (from 100:0 to 80:20) to afford a mixture of 3-iodo-1-(tetrahydro-2H-pyran-2-yl)-1H-indazol-5-ol and benzyl (2,2-difluoro-3-((3-iodo-1-(tetrahydro-2H-pyran-2-yl)-1H-indazol-5-yl)oxy)propyl)carbamate. The mixture was purified by flash chromatography on silica gel, using as eluents DCM/EtOAc (from 100:0 to 98:2) to afford benzyl (2,2-difluoro-3-((3-iodo-1-(tetrahydro-2H-pyran-2-yl)-1H-indazol-5-yl)oxy) propyl)carbamate intermediate **214** as a white solid.
LCMS method E: [M+H]⁺ = 572.0, t_{R} = 4.275 min

### Preparation of intermediate 215: N-[2,2-difluoro-3-[3-[3-(hydroxymethyl)phenyl]-1-tetrahydro pyran-2-yl-indazol-5-yl]oxy-propyl]carbamate

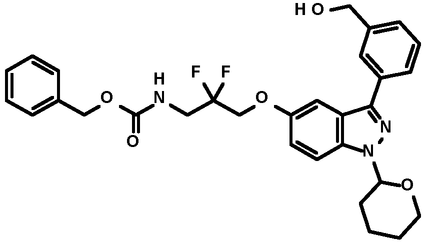

To a solution of benzyl *N*-[2,2-difluoro-3-(3-iodo-1-tetrahydropyran-2-yl-indazol-5-yl)oxy-propyl]carbamate intermediate **214** (250 mg, 0.44 mmol), [3-(hydroxymethyl)phenyl]boronic acid (79 mg, 0.52 mmol) and potassium phosphate tribasic (280 mg, 1.32 mmol) in 1,4-dioxane (3.2 mL) and water (1.6 mL), were added tetrakis(triphenylphosphine)palladium(0) (25 mg, 0.022 mmol) and 2-dicyclohexylphosphino-2',4',6'-triisopropylbiphenyl (21 mg, 0.044 mmol). The reaction mixture was heated at 100 °C for 2 h. The reaction mixture was filtered through a Celite pad and washed with ethyl acetate. The filtrate was diluted with water and extracted with ethyl acetate. The organic layer was washed with water, brine, dried over anhydrous magnesium sulfate, filtered and concentrated under reduced pressure. The residue was purified by silica gel column chromatography using cyclohexane/ethyl acetate 99/1 to 40/60 as a eluent to afford benzyl *N*-[2,2-difluoro-3-[3-[3-(hydroxymethyl)phenyl]-1-tetrahydropyran-2-yl-indazol-5-yl] oxy-propyl]carbamate intermediate **215** as a colorless oil.
LCMS method F: [M+H]⁺ = 552.3, t_{R} = 2.90 min

### Preparation of intermediate 216: 12,12-difluoro-19-(oxan-2-yl)-8,14-dioxa-10,19,20-triaza tetracyclo[13.5.2.1^{2,6}.0^{18,21}]tricosa-1(20),2(23),3,5,15(22),16,18(21)-heptaen-9-one

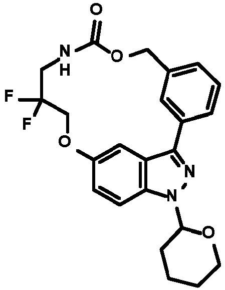

To a solution of benzyl *N*-[2,2-difluoro-3-[3-[3-(hydroxymethyl)phenyl]-1-tetrahydropyran-2-yl-indazol-5-yl]oxy-propyl]carbamate intermediate **215** (243 mg, 0.44 mmol) in dry acetonitrile (28 mL) at room temperature was added potassium hydroxide (0.123 g, 2.20 mmol) in one portion. The reaction mixture was stirred at room temperature for 8 h. The reaction mixture was filtered then washed with ethyl acetate and the filtrate was evaporated under reduced pressure. The residue was purified by silica gel column chromatography using cyclohexane/(ethyl acetate/ethanol (3-1)) 100/0 to 80/20 as eluent to afford 12,12-difluoro-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one intermediate **216** as a white solid.
LCMS method F: [M+H]⁺ = 444.3, t_{R} = 2.78 min

### Preparation of Example 152 : 12,12-difluoro-8,14-dioxa-10,19,20-triazatetracyclo [13.5.2.1^{2,6}.0^{18,21}]tricosa-1(20),2(23),3,5,15(22),16,18(21)-heptaen-9-one

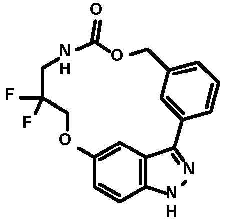

To a solution of 12,12-difluoro-19-(oxan-2-yl)-8,14-dioxa-10,19,20-triazatetracyclo [13.5.2. 1^{2,6}.0^{18,21}]tricosa-1(20),2(23),3,5,15(22),16,18(21)-heptaen-9-one intermediate **216** (65 mg, 0.15 mmol) in dichloromethane (4 mL) was added trifluoroacetic acid (230 µL, 3.0 mmol). The reaction mixture was heated at 50°C for 2 h. The reaction mixture was evaporated under reduced pressure and the residue was dissolved in ethyl acetate and saturated aqueous sodium bicarbonate solution. After separation, the aqueous layer was extracted with ethyl acetate. The organic layers were washed with water then brine, dried over sodium sulfate, filtered and evaporated under reduced pressure. The residue was purified by silica gel column chromatography using dichloromethane/ethanol 100/0 to 95/5 as eluent. The resulting product was triturated in dichloromethane, filtered and dried to afford 12, 12-difluoro-8, 14-dioxa-10,19,20-triazatetracyclo[13.5.2.1^{2,6}.0^{18,21}]tricosa-1(20),2(23),3,5,15(22),16,18(21)-heptaen-9-one **example 152** as a white solid.
LCMS method F: [M+H]⁺ = 360.3, t_{R} = 2.09 min
LCMS method G: [M+H]⁺ = 360.2, t_{R} = 2.10 min
¹H NMR (400 MHz, d6-DMSO, 80°C) δ 12.93 (1H, s), 8.00 - 7.99 (1H, m), 7.90 - 7.88 (2H, m), 7.51 - 7.45 (3H, m), 7.31 - 7.28 (1H, m), 7.13 - 7.07 (1H, m), 5.39 - 5.31 (2H, m), 4.71 (2H, t, *J =* 16.9 Hz), 3.71 - 3.52 (2H, m) ppm.

### Example 153: (13R)-17-fluoro-13-methyl-8,14-dioxa-10,19,20-triazatetracyclo[13.5.2. 1^{2,6}.0^{18,21}]tricosa-1(20),2(23),3,5,15(22),16,18(21)-heptaen-9-one

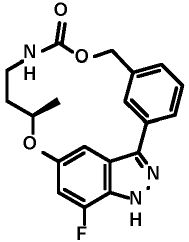

Example 153 is prepared according to the synthesis route described in general Scheme C.

### Preparation of intermediate 217: tert-butyl-[(7-fluoro-1H-indazol-5-yl)oxy]-dimethyl-silane

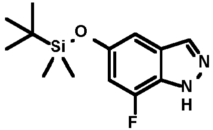

To a stirred solution of 7-fluoro-1H-indazol-5-ol (4.858g, 31.933mmol) in DCM (100mL) was added imidazole (2.609g, 38.32mmol) followed by tert-butyldimethylchlorosilane (5.295g, 35.126mmol). The reaction mixture was stirred at room temperature for 16h. The mixture was filtered over a pad of celite and was washed with dichloromethane. The filtrate was concentrated under vacuum to afford tert-butyl-[(7-fluoro-1H-indazol-5-yl)oxy]-dimethylsilane intermediate **217.** The product was used in the next step without further purification.
LCMS method B: [M+H]⁺ = 267.0, t_{R} = 1.153 min

### Preparation of intermediate 218: tert-butyl-[(7-fluoro-3-iodo-1H-indazol-5-yl)oxy]-dimethyl-silane

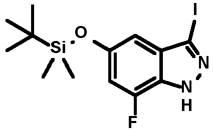

1-iodopyrrolidine-2,5-dione (7.601g, 33.785mmole) in DMF (30mL) was added to a solution of tert-butyl-[(7-fluoro-1H-indazol-5-yl)oxy]-dimethyl-silane intermediate **217** (6 g, 22.523 mmol) in DMF (15 mL). The reaction mixture was stirred at room temperature for 1h. A solution of 10% sodium thiosulfate (300ml) was added at 0°C and the mixture was extracted with EtOAC (4x200ml). The combined organic layers were dried over MgSO4, filtered and the solvent was removed under reduced pressure. The product was purified by column chromatography on silica gel using as eluent heptane/EtOAc (90:10) to afford tert-butyl-[(7-fluoro-3-iodo-1H-indazol-5-yl)oxy]-dimethyl-silane intermediate **218** as a sticky transparent gum.
LCMS method B: [M+H]⁺ = 392.9, t_{R} = 1.316 min

### Preparation of intermediate 219: tert-butyl-(7-fluoro-3-iodo-1-tetrahydropyran-2-yl-indazol-5-yl)oxy-dimethyl-silane

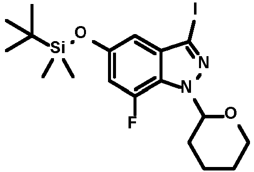

To a solution of tert-butyl-[(7-fluoro-3-iodo-1H-indazol-5-yl)oxy]-dimethyl-silane intermediate **218** (5.340 g, 13.612 mmol) in 54 ml of DCM were added 4-methylbenzenesulfonic acid monohydrate (0.518 g, 2.722 mmol) and 3,4-dihydro-2H-pyran (3.734 ml, 40.836 mmol) and the reaction mixture was stirred at room temperature for 16 hours. The reaction mixture was diluted with DCM and washed with a saturated aqueous NaHCO3 solution (3x500ml) and brine (500ml). The organic phase was dried over MgSO4, filtered and evaporated under reduced pressure. The crude product was purified by flash column chromatography on silica gel using a gradient of n-heptane/EtOAc (100:0 to 90:10) as eluents. The desired fractions were combined and the solvent was removed under reduced pressure to afford tert-butyl-(7-fluoro-3-iodo-1-tetrahydropyran-2-yl-indazol-5-yl)oxy-dimethyl-silane intermediate **219** as a colourless oil.
LCMS method B: [M-84+H]⁺ = 392.9, t_{R} = 1.577 min

### Preparation of intermediate 220: 7-fluoro-3-iodo-1-tetrahydropyran-2-yl-indazol-5-ol

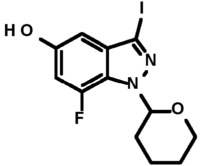

Tert-butyl-(7-fluoro-3-iodo-1-tetrahydropyran-2-yl-indazol-5-yl)oxy-dimethyl-silane intermediate **219** (5.390 g, 11.314 mmol) was dissolved in 35 ml of THF. The mixture was cooled to 0°C and TBAF 1 M in THF (14.708 ml, 14.708 mmol) was added. The reaction mixture was stirred at RT for 2h. The reaction mixture was cooled to 0°C, diluted with EtOAc and the mixture was washed with a saturated aqueous solution of NaHCO3 (x3). The organic layer was dried over MgSO4, filtered and the solvent was removed under reduced pressure to obtain a solid that was triturated with DCM to obain 7-fluoro-3-iodo-1-tetrahydropyran-2-yl-indazol-5-ol intermediate **220** as a white solid.
LCMS method C: [M+H]⁺ = 363.0, t_{R} = 4.563 min

### Preparation of intermediate 221: Benzyl N-[(3R)-3-(7-fluoro-3-iodo-1-tetrahydropyran-2-yl-indazol-5-yl)oxybutyl]carbamate

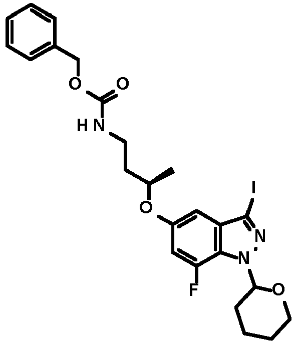

To a solution of 7-fluoro-3-iodo-1-tetrahydropyran-2-yl-indazol-5-ol intermediate **220** (700 mg, 1.93 mmol) in *N,N*-dimethylformamide (8 mL) at room temperature was added cesium carbonate (942 mg, 2.90 mmol). The reaction mixture was stirred at room temperature for 30 min and a solution of [(1S)-3-(benzyloxycarbonylamino)-1-methyl-propyl] methanesulfonate (638 mg, 2.12 mmol) in *N,N*-dimethylformamide (2 mL) was added dropwise. The reaction mixture was stirred at room temperature for 5 h. The reaction mixture was filtered, washed with ethyl acetate and concentrated under reduced pressure. The residue was purified by silica gel column chromatography using cyclohexane/ethyl acetate 100/0 to 80/20 as eluent to afford benzyl N-[(3R)-3-(7-fluoro-3-iodo-1-tetrahydropyran-2-yl-indazol-5-yl)oxybutyl]carbamate intermediate **221** as a colorless oil.
LCMS method F: [M+H]⁺ = 568.2, t_{R} = 3.30 min

### Preparation of intermediate 222: Benzyl N-[(3R)-3-[7-fluoro-3-[3-(hydroxymethyl) phenyl]-1-tetrahydropyran-2-yl-indazol-5-yl]oxybutyl]carbamate

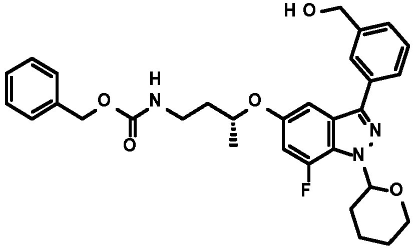

To a solution of benzyl N-[(3R)-3-(7-fluoro-3-iodo-1-tetrahydropyran-2-yl-indazol-5-yl)oxybutyl]carbamate intermediate **221** (755 mg, 1.33 mmol), [3-(hydroxymethyl) phenyl] boronic acid (241 mg, 1.60 mmol) and potassium phosphate tribasic (848 mg, 4.00 mmol) in 1,4-dioxane (12 mL) and water (2 mL), were added tetrakis(triphenylphosphine)palladium(0) (76 mg, 0.066 mmol) and 2-dicyclohexylphosphino-2',4',6'-triisopropylbiphenyl (62 mg, 0.13 mmol). The reaction mixture was stirred at 100 °C for 2 h. The reaction mixture was filtered through a Celite pad and washed with ethyl acetate. The filtrate was diluted with water and extracted with ethyl acetate. The organic layer was washed with water, brine, dried over anhydrous magnesium sulfate, filtered and concentrated under reduced pressure. The residue was purified by silica gel column chromatography using cyclohexane/ethyl acetate using 99/1 to 40/60 as eluent to afford benzyl N-[(3R)-3-[7-fluoro-3-[3-(hydroxymethyl)phenyl]-1-tetrahydropyran-2-yl-indazol-5-yl]oxybutyl]carbamate intermediate **222** as a yellow solid.
LCMS method F: [M+H]⁺ = 548.4, t_{R} = 3.09 min

### Preparation of intermediate 223: (13R)-17-fluoro-13-methyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one

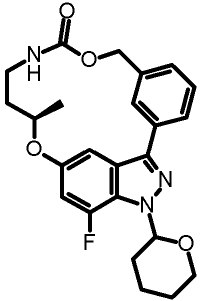

A solution of benzyl *N*-[(3*R*)-3-[7-fluoro-3-[3-(hydroxymethyl)phenyl]-1-tetrahydropyran-2-yl-indazol-5-yl]oxybutyl]carbamate intermediate **222** (550 mg, 1.00 mmol) and cesium carbonate (1.95 g, 6.00 mmol) in dry acetonitrile (150 mL) was stirred at 85 °C for 16 h. The reaction mixture was filtered, washed with ethyl acetate and evaporated under reduced pressure. The residue was purified by silica gel column chromatography using cyclohexane/(ethyl acetate/ethanol 3/1) 100/0 to 80/20 as eluent to afford (13R)-17-fluoro-13-methyl-19-(oxan-2-yl)-8,14-dioxa-10,19,20-triazatetracyclo[13.5.2.1^{2,6}.0^{18,21}]tricosa-1(20),2(23),3,5,15(22), 16,18(21)-heptaen-9-one intermediate **223** as a white solid.
LCMS method F: [M+H]⁺ = 440.3, t_{R} = 3.08 min

### Preparation of Example 153: (13R)-17-fluoro-13-methyl-8,14-dioxa-10,19,20-triazatetra cyclo[13.5.2.1^{2,6}.0^{18,21}]tricosa-1(20),2(23),3,5,15(22),16,18(21)-heptaen-9-one

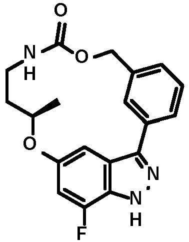

To a solution of (13R)-17-fluoro-13-methyl-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one intermediate **223** (346 mg, 0.79 mmol) in dichloromethane (19 mL) was added trifluoroacetic acid (1.20 mL, 15.8 mmol). The reaction mixture was heated at 50 °C for 16 h. The reaction mixture was evaporated under reduced pressure. The residue was dissolved in ethyl acetate and saturated aqueous solution of sodium bicarbonate was added. After separation, the aqueous layer was extracted with ethyl acetate. The combined organic layers were washed with water, brine, dried over anhydrous sodium sulfate, filtered and evaporated under reduced pressure. The residue was purified by silica gel column chromatography using dichloromethane/methanol 100/0 to 95/5 as eluent. The resulting product was triturated in dichloromethane, filtered and dried to afford (13R)-17-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one **example 153** as a white solid.
LCMS method F: [M+H]⁺ = 356.2, t_{R} = 2.41 min
LCMS method G: [M+H]⁺ = 356.2, t_{R} = 2.33 min
¹H NMR (400 MHz, d6-DMSO) δ 13.70 (1H, s), 7.93 - 7.81 (3H, m), 7.51 - 7.46 (1H, m), 7.33 - 7.29 (1H, m), 7.08 (1H, s), 6.88 (1H, dd, *J* = 1.3, 12.1 Hz), 5.77 - 5.73 (1H, m), 4.84 - 4.80 (1H, m), 4.62 - 4.54 (1H, m), 3.58 - 3.53 (1H, m), 2.93 - 2.85 (1H, m), 2.44 - 2.37 (1H, m), 1.41 - 1.38 (4H, m) ppm.

### Example 154: (7S,13R)-7,13-dimethyl-8,14-dioxa-4,10,19,20,23-pentaazatetracyclo [13.5.2.1^{2,6}.0^{18,21}]tricosa-1(20),2(23),3,5,15(22),16,18(21)-heptaen-9-one

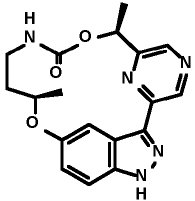

Example 154 is prepared according to the synthesis route described below.

### Preparation of intermediate 224: 1-(6-chloropyrazin-2-yl)ethanol

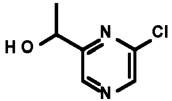

To a solution of 1-(6-chloropyrazin-2-yl)ethanone (1.00 g, 6.39 mmol) in methanol (15 mL) at 0°C was added sodium borohydride (725 mg, 19.2 mmol) in small portions. The reaction mixture was stirred at 0°C for 3 h. Water was added to reaction mixture then 1M aqueous hydrochloric acid solution. The reaction mixture was extracted with ethyl acetate. The organic phase was washed with brine, dried over anhydrous magnesium sulfate, filtered and evaporated under reduced pressure to afford 1-(6-chloropyrazin-2-yl)ethanol intermediate **224** as a colorless oil.

¹H NMR (500 MHz, d6-DMSO) δ 8.74 (s, 1 H), 8.69 (s, 1 H), 5.73 (d, 1 H), 4.79 (m, 1 H), 1.4 (d, 3 H) ppm.

### Preparation of intermediate 225: 1-[6-[5-[tert-butyl(dimethyl)silyl]oxy-1-tetrahydropyran-2-yl-indazol-3-yl]pyrazin-2-yl]ethanol

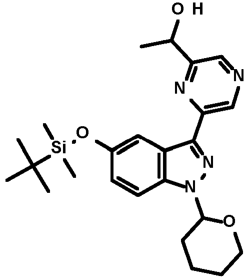

1-(6-chloropyrazin-2-yl)ethanol intermediate **224** (970 mg, 6.12 mmol) in 1,4-dioxane (60 mL) was warmed to 60°C and all the insoluble solid was filtered out. To the filtrate water (6 mL), *tert*-butyl-dimethyl-[1-tetrahydropyran-2-yl-3-(4,4,5,5-tetramethyl-1,3,2-dioxaborolan-2-yl) indazol-5-yl]oxy-silane (3.08 g, 6.73 mmol), potassium carbonate (1.69 g, 12.2 mmol) and finally palladium, triphenylphosphane (212 mg, 0.183 mmol) were added. The reaction mixture was purged with nitrogen for 10 min, then it was refluxed under nitrogen for 40 min. The reaction mixture was cooled to room temperature, diluted with ethyl acetate, washed with water, then the aqueous layer was extracted with ethyl acetate. The combined organic layer was washed with brine, dried over anhydrous magnesium sulfate, filtered and concentrated under reduced pressure. The residue was purified by silica gel column chromatography using heptane/ethyl acetate 80/20 to 50/50 as eluent to afford 1-[6-[5-[*tert*-butyl(dimethyl)silyl]oxy-1-tetrahydropyran-2-yl-indazol-3-yl]pyrazin-2-yl]ethanol intermediate **225** as a brown oil.

¹H NMR (500 MHz, d6-DMSO) δ 9.19 (s, 1 H), 8.7 (s, 1 H), 7.98 (d, 1 H), 7.74 (d, 1 H), 7.09 (dd, 1 H), 5.94(dd, 1 H), 5.7 (d, 1 H), 4.94 (qn, 1 H), 3.93/3.78 (dt+td, 2 H), 2.48/2.05 (m+dd, 2 H), 2.09/1.79 (m+m, 2 H), 1.62 (qn, 2 H), 1.55 (d, 3 H), 0.99 (s, 9 H), 0.24/0.23 (s/s, 6 H) ppm.

### Preparation of intermediate 226: 3-[6-(1-hydroxyethyl)pyrazin-2-yl]-1-tetrahydropyran-2-yl-indazol-5-olate(tetrabutylammoniumsalt)

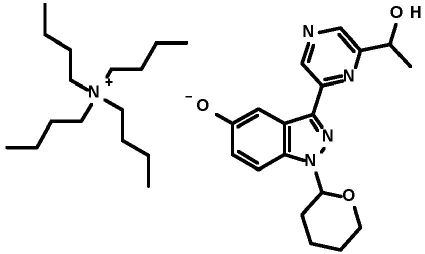

To a solution of 1-[6-[5-[*tert*-Butyl(dimethyl)silyl]oxy-1-tetrahydropyran-2-yl-indazol-3-yl]pyrazin-2-yl]ethanol intermediate **225** (2.61 g, 5.74 mmol) in tetrahydrofuran (15 mL) at room temperature was added 1M tetrabutylammonium fluoride solution in THF (6.3 ML, 6.32 mmol) dropwise. The reaction mixture was stirred for 1 h. The reaction mixture was stirred at 0°C for 10 min and the solid was filtered out then washed with THF (10 mL) and with diethyl ether to afford 3-[6-(1-hydroxyethyl)pyrazin-2-yl]-1-tetrahydropyran-2-yl-indazol-5-olate tetrabutyl ammonium intermediate **226** as an off-white solid. The base acid ratio is ca. 1:2.

¹HNMR (500 MHz, d6-DMSO) δ 9.1 (s, 1 H), 8.59 (s, 1 H), 7.52 (brs., 1 H), 7.39 (d, 1 H), 6.9 (dd, 1 H), 5.78 (dd, 1 H), 4.9 (q, 1 H), 3.91/3.73 (brd+td, 2 H), 2.53-1.53 (m, 6 H), 1.51 (d, 3 H) ppm.

### Preparation of intermediate 227: Benzyl N-[(3R)-3-[3-[6-(1-hydroxyethyl)pyrazin-2-yl]-1-tetrahydropyran-2-yl-indazol-5-yl]oxybutyl] carbamate

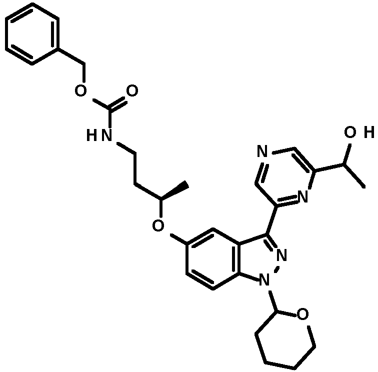

To a suspension of 3-[6-(1-hydroxyethyl)pyrazin-2-yl]-1-tetrahydropyran-2-yl-indazol-5-olate tetrabutylammonium intermediate **226** (2.24 g, 3.96 mmol) inN,N-dimethylformamide (20 mL) at 45°C was added cesium carbonate (5.21 g, 16.0 mmol). The reaction mixture was stirred for 3 min, then a solution of [(1S)-3-(benzyloxycarbonylamino)-1-methyl-propyl]methane sulfonate (1.51 g, 5.00 mmol) in *N,N*-dimethylformamide (5 mL) was added. The reaction mixture was stirred at 45°C for 3 h. The warm solution was filtered and the filtrate was diluted with ethyl acetate then washed with brine. The organic phase was dried over anhydrous sodium sulfate, filtered and evaporated under reduced pressure. The solid was suspended in diethyl ether then filtered and dried to afford benzyl N-[(3R)-3-[3-[6-(1-hydroxyethyl)pyrazin-2-yl]-1-tetrahydropyran-2-yl-indazol-5-yl]oxybutyl]carbamate as a mixture of diastereomers intermediate **227** as a beige solid.

¹H NMR (500 MHz, d6-DMSO) δ 9.19 (s, 1 H), 8.7 (s, 1 H), 7.98 (d, 1 H), 7.74 (d, 1 H), 7.41-7.19 (m, 6 H), 7.15 (dd, 1 H), 5.94 (dd, 1 H), 5.68 (d, 1 H), 4.98 (s, 2 H), 4.97 (m, 1 H), 4.51 (m, 1H), 3.92/3.78 (brd+m, 2 H), 3.18 (m, 2 H), 2.48/2.04 (m+m, 2 H), 2.08/1.78 (m+m, 2 H), 1.87/1.79 (m+m, 2 H), 1.62 (m, 2 H), 1.57 (d, 3 H), 1.31 (d, 3 H) ppm.

### Preparation of intermediate 228: (7S,13R)-7,13-dimethyl-19-(oxan-2-yl)-8,14-dioxa-4,10,19,20,23-pentaazatetracyclo[13.5.2.1^{2,6}.0^{18,21}]tricosa-1(20),2(23),3,5,15(22),16,18(21)-heptaen-9-one

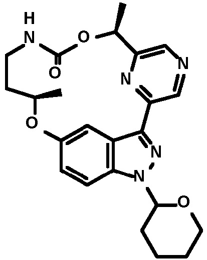

To a solution of benzyl N-[(3R)-3-[3-[6-(1-hydroxyethyl)pyrazin-2-yl]-1-tetrahydropyran-2-yl-indazol-5-yl]oxybutyl]carbamate intermediate **227** (1.80 g, 3.30 mmol) in dry N,N-dimethyl formamide (50 mL) was added potassium hydroxide (555 mg, 9.90 mmol) at 30°C in one portion. The reaction mixture was stirred at 30°C for 60 min. The solid was filtered out and washed with ethyl acetate. The filtrate was diluted with ethyl acetate, washed with brine, dried over anhydrous magnesium sulfate, filtered and evaporated under reduced pressure. The residue was purified and separated as diastereomers by silica gel column chromatography using heptane/ethyl acetate 50/50 to 0/100 as eluent to afford (7S,13R)-7,13-dimethyl-19-(oxan-2-yl)-8,14-dioxa-4,10,19,20,23-pentaazatetracyclo[13.5.2.1^{2,6}.0^{18,21}]tricosa-1(20),2(23),3,5, 15(22),16,18(21)-heptaen-9-one intermediate **228** as a white solid.

¹H NMR (500 MHz, d6-DMSO) δ 9.23 (d, 1 H), 8.66 (d, 1 H), 7.87/7.85 (t/t, 1 H), 7.78/7.77 (d/d, 1 H), 7.72/7.71 (d/d, 1 H), 7.05 (dd, 1 H), 5.94 (q, 1 H), 5.93 (dd, 1 H), 4.56 (m, 1 H), 3.91/3.77 (dq+td, 2 H), 3.53/2.83 (m+m, 2 H), 2.48/2.05 (m+m, 2 H), 2.3/1.33 (td+td, 2 H), 2.09/1.8 (m+m, 2 H), 1.68 (d, 3 H), 1.62 (m, 2 H), 1.37 (d, 3 H) ppm.

### Preparation of Example 154: (7S,13R)-7,13-dimethyl-8,14-dioxa-4,10,19,20,23-pentaaza tetracyclo[13.5.2.1^{2,6}.0^{18,21}]tricosa-1(20),2(23),3,5,15(22),16,18(21)-heptaen-9-one

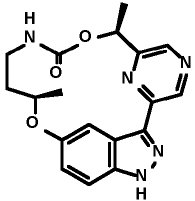

To a suspension of (7S,13R)-7,13-dimethyl-19-(oxan-2-yl)-8,14-dioxa-4,10,19,20,23-pentaaza tetracyclo[13.5.2.1^{2,6}.0^{18,21}]tricosa-1(20),2(23),3,5,15(22),16,18(21)-heptaen-9-one intermediate **228** (460 mg, 1.1 mmol) in a mixture of methanol (77 mL) and water (11 mL) was added p-toluenesulfonic acid monohydrate (0.93 mL, 5.3 mmol). The reaction mixture was stirred at 65°C for 16 h. The reaction mixture was cooled to room temperature and saturated aqueous sodium bicarbonate solution was added. Methanol was evaporated under reduced pressure. The reaction mixture was extracted with ethyl acetate. The combined organic phases were washed with brine, dried over anhydrous magnesium sulfate, filtered and concentrated under reduced pressure. The residue was purified by silica gel column chromatography using dichloromethane/ethyl acetate 90/10 to 0/100 as eluent to afford (7S,13R)-7,13-dimethyl-8,14-dioxa-4,10,19,20,23-pentaazatetracyclo[13.5.2.1^{2,6}.0^{18,21}]tricosa-1(20),2(23),3,5,15(22), 16,18(21)-heptaen-9-one **example 154** as a white solid.
LCMS method F: [M+H]⁺ = 354.2, t_{R} = 2.03 min
LCMS method G: [M+H]⁺ = 354.3, t_{R} = 1.89 min
¹H NMR (500 MHz, d6-DMSO) δ 13.48 (s, 1 H), 9.24 (s, 1 H), 8.62 (s, 1 H), 7.85 (dd, 1 H), 7.77 (d, 1 H), 7.51 (d, 1 H), 6.99 (dd, 1H), 5.94 (q, 1 H), 4.56 (m, 1 H), 3.53/2.83 (dq+td, 2 H), 2.32/1.33 (t+td, 2 H), 1.68 (d, 3 H), 1.37 (d, 3 H) ppm.

### Example 155: (7R,13R)-7,13-dimethyl-8,14-dioxa-4,10,19,20,23-pentaazatetracyclo[13.5. 2.1^{2,6}.0^{18,21}]tricosa-1(20),2(23),3,5,15(22),16,18(21)-heptaen-9-one

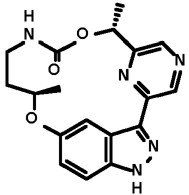

Example 155 is prepared according to the synthesis route described in general Scheme K.

### Preparation of intermediate 229: (7R,13R)-7,13-dimethyl-19-(oxan-2-yl)-8,14-dioxa-4,10,19,20,23-pentaazatetracyclo[13.5.2.1^{2,6}.0^{18,21}]tricosa-1(20),2(23),3,5,15(22),16,18(21)-heptaen-9-one

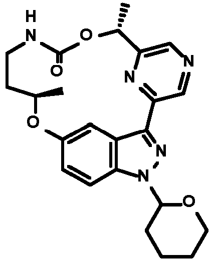

To a solution of benzyl N-[(3R)-3-[3-[6-(1-hydroxyethyl)pyrazin-2-yl]-1-tetrahydropyran-2-yl-indazol-5-yl]oxybutyl]carbamate intermediate **227** (1.80 g, 3.30 mmol) in dry N,N-dimethyl formamide (50 mL) was added potassium hydroxide (555 mg, 9.90 mmol) at 30°C in one portion. The reaction mixture was stirred at 30°C for 60 min. The solid was filtered out and washed with ethyl acetate. The filtrate was diluted with ethyl acetate, washed with brine, dried over anhydrous magnesium sulfate, filtered and evaporated under reduced pressure. The residue was purified and separated as diastereomers by silica gel column chromatography using heptane/ethyl acetate 50/50 to 0/100 as eluent to afford (7R,13R)-7,13-dimethyl-19-(oxan-2-yl)-8,14-dioxa-4,10,19,20,23-pentaazatetracyclo[13.5.2.1^{2,6}.0^{18,21}]tricosa-1(20),2(23),3,5, 15(22),16,18(21)-heptaen-9-one intermediate **229** as a white solid.

¹H NMR (500 MHz, d6-DMSO) δ 9.28/9.1 (s/s, 1 H), 8.65 (s, 1 H), 8.13/7.91 (d/d, 1 H), 7.72/7.69 (d/d, 1 H), 7.56/7.18 (t/t, 1 H), 7.06/7.04 (dd/dd, 1 H), 6.14/5.78 (q/q, 1 H), 5.93 (dd, 1 H), 4.89 (m, 1 H), 3.93/3.78 (dq+td, 2 H), 3.24/2.98 (dq+dt, 2 H), 2.46/2.03 (td+dq, 2 H), 2.07/1.78 (m+m, 2 H), 1.75/1.6 (m+m, 2 H), 1.75 (d, 3 H), 1.61 (m, 2 H), 1.5/1.4 (d/d, 3 H) ppm.

### Preparation of Example 155: (7R,13R)-7,13-dimethyl-8,14-dioxa-4,10,19,20,23-pentaaza tetracyclo[13.5.2.1^{2,6}.0^{18,21}]tricosa-1(20),2(23),3,5,15(22),16,18(21)-heptaen-9-one

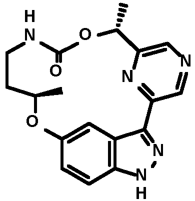

To a suspension of (7R,13R)-7,13-dimethyl-19-(oxan-2-yl)-8,14-dioxa-4,10,19,20,23-pentaazatetracyclo[13.5.2.1^{2,6}.0^{18,21}]tricosa-1(20),2(23),3,5,15(22),16,18(21)-heptaen-9-one intermediate **229** (86 mg, 0.20 mmol) in a mixture of methanol (14 mL) and water (2.0 mL) was added p-toluenesulfonic acid monohydrate (190 mg, 0.98 mmol) and the reaction mixture was stirred at 65°C for 48 h. The reaction mixture was cooled to room temperature and saturated aqueous sodium bicarbonate solution and water were added. Methanol was evaporated meanwhile solid was precipitated. The solid was filtered and dried under reduced pressure. The residue was purified by silica gel column chromatography to afford (7R,13R)-7,13-dimethyl-8,14-dioxa-4,10,19,20,23-pentaazatetracyclo[13.5.2.1^{2,6}.0^{18,21}]tricosa-1(20),2(23),3,5,15(22), 16,18(21)-heptaen-9-one **example 155** as a white solid.
LCMS method F: [M+H]⁺ = 354.2, t_{R} = 2.14 min
LCMS method G: [M+H]⁺ = 354.3, t_{R} = 2.03 min
¹H NMR (500 MHz, d6-DMSO) δ 13.47(s, 1 H), 9.29/9.11 (s/s, 1 H), 8.61 (s, 1 H), 8.15/7.9 (d/d, 1 H), 7.55/7.17 (dd/dd, 1 H), 7.52/7.48 (d/d, 1 H), 7/6.98 (dd/dd, 1 H), 6.13/5.78 (q/q, 1 H), 4.89/4.34 (m/m, 1 H), 3.69/3.24/2.98/2.98 (dq+dq/dq+dq, 2 H), 2.76/1.77/1.59/1.42 (dd+td/dd+td, 2 H), 1.75/1.51 (d/d, 3 H), 1.38/1.37 (d/d, 3 H) ppm.

### Example 156: (13S)-13-methyl-8,14-dioxa-4,10,19,20,23-pentaazatetracyclo[13.5.2. 1^{2,6}.0^{18,21}]tricosa-1(20),2(23),3,5,15(22),16,18(21)-heptaen-9-one

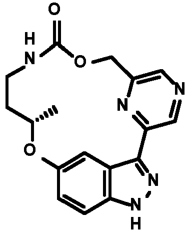

Example 156 is prepared according to the synthesis route described in general Scheme K.

### Preparation of intermediate 230: [6-[5-[tert-butyl(dimethyl)silyl]oxy-1-tetrahydropyran-2-yl-indazol-3-yl]pyrazin-2-yl]methanol

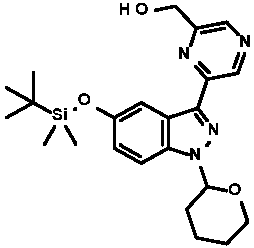

To a solution of (6-chloropyrazin-2-yl)methanol (200 mg, 1.66 mmol) in 1,4-dioxane (10.8 mL) and water (1.2 mL) was added at room temperature tert-butyl-dimethyl-[1-tetrahydropyran-2-yl-3-(4,4,5,5-tetramethyl-1,3,2-dioxaborolan-2-yl)indazol-5-yl]oxy-silane (760 mg, 1.66 mmol) and potassium carbonate (380 mg, 2.76 mmol). The reaction mixture was purged with nitrogen and tetrakis(triphenylphosphine)palladium(0) (161 mg, 0.14 mmol) was added. The reaction mixture was stirred at 80 °C for 16 h. The reaction mixture was filtered through a Celite pad and washed with ethyl acetate. The filtrate was diluted with water and extracted with ethyl acetate. The organic layer was washed with water, brine, dried over anhydrous magnesium sulfate, filtered and concentrated under reduced pressure. The residue was purified by silica gel column chromatography using cyclohexane/ethyl acetate 99/1 to 70/30 as eluent to afford [6-[5-[tert-butyl(dimethyl)silyl]oxy-1-tetrahydropyran-2-yl-indazol-3-yl]pyrazin-2-yl]methanol intermediate **230** as a yellow solid.
LCMS method F: [M+H]⁺ = 441.3, t_{R} = 3.36 min

### Preparation of intermediate 231: 3-[6-(hydroxymethyl)pyrazin-2-yl]-1-tetrahydropyran-2-yl-indazol-5-ol

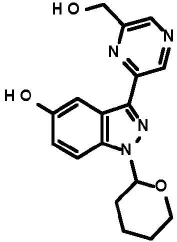

To a solution of [6-[5-[*tert*-butyl(dimethyl)silyl]oxy-1-tetrahydropyran-2-yl-indazol-3-yl] pyrazin-2-yl]methanol intermediate **230** (594 g, 1.35 mmol) in THF (10 mL) was added dropwise at room temperature 1.0 M tetrabutylammonium fluoride solution in THF (1.48 mL, 1.48 mmol). The reaction mixture was stirred at room temperature for 16 h. The reaction mixture was poured into ice water and stirred for 20 min. The aqueous layer was extracted with ethyl acetate then combined organic layers were washed with brine, dried over anhydrous magnesium sulfate, filtered and concentrated under reduced pressure to give 3-[6-(hydroxymethyl)pyrazin-2-yl]-1-tetrahydropyran-2-yl-indazol-5-ol intermediate **231** as a yellow solid which was used in the next step without further purification.
LCMS method F: [M+H]⁺ = 327.3, t_{R} = 2.01 min

### Preparation of intermediate 232 : benzyl N-[(3S)-3-[3-[6-(hydroxymethyl)pyrazin-2-yl]-1-tetrahydropyran-2-yl-indazol-5-yl]oxybutyl]carbamate

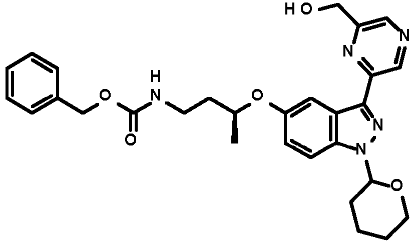

[(1*R*)-3-(benzyloxycarbonylamino)-1-methyl-propyl]methanesulfonate was prepared according to the same synthesis procedures as intermediate **199** starting from (2R)-4-aminobutan-2-ol.

To a mixture of 3-[6-(hydroxymethyl)pyrazin-2-yl]-1-tetrahydropyran-2-yl-indazol-5-ol intermediate **231** (0.414 g, 1.27 mmol) in *N,N-*dimethylformamide (12 mL) was added cesium carbonate (825 mg, 2.54 mmol). The reaction mixture was stirred for 20 min. Then, [(1*R*)-3-(benzyloxycarbonylamino)-1-methyl-propyl] methanesulfonate (421 mg, 1.40 mmol) was added. The reaction mixture was stirred at room temperature for 16 h. Additional [(1*R*)-3-(benzyloxycarbonylamino)-1-methyl-propyl] methanesulfonate was added (57 mg, 0.19 mmol) and the reaction mixture was stirred at room temperature for 2 h. The reaction mixture was concentrated under reduced pressure then diluted with water and extracted with ethyl acetate. The combined organic layer was washed with brine, dried over anhydrous sodium sulfate, filtered and evaporated under reduced pressure. The residue was purified by silica gel column chromatography using dichloromethane/methanol 100/0 to 95/5 as eluent to afford benzyl *N-*[(3*S*)-3-[3-[6-(hydroxymethyl)pyrazin-2-yl]-1-tetrahydropyran-2-yl-indazol-5-yl]oxybutyl] carbamate intermediate **232** as a yellow solid.
LCMS method F: [M+H]⁺ = 532.3, t_{R} = 2.79 min

### Preparation of intermediate 233: (13S)-13-methyl-19-(oxan-2-yl)-8,14-dioxa-4,10,19,20,23-pentaazatetracyclo[13.5.2.1^{2,6}.0^{18,21}]tricosa-1(20),2(23),3,5,15(22),16,18(21)-heptaen-9-one

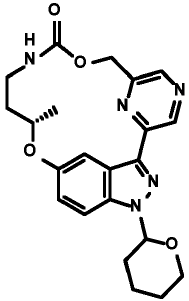

To a solution of benzyl *N*-[(3*S*)-3-[3-[6-(hydroxymethyl)pyrazin-2-yl]-1-tetrahydropyran-2-yl-indazol-5-yl]oxybutyl]carbamate intermediate **232** (562 mg, 1.06 mmol) in dry acetonitrile (53 mL) at room temperature was added potassium hydroxide (297 mg, 5.30 mmol) in one portion. The reaction mixture was stirred at room temperature for 4 h. The reaction mixture was filtered and rinsed with acetonitrile and the filtrate was evaporated under reduced pressure. The residue was purified by silica gel column chromatography using dichloromethane/methanol 100/0 to 95/5 as eluent to afford (13S)-13-methyl-19-(oxan-2-yl)-8,14-dioxa-4,10,19,20,23-pentaazatetra cyclo[13.5.2.1^{2,6}.0^{18,21}]tricosa-1(20),2(23),3,5,15(22),16,18(21)-heptaen-9-one intermediate **233** as a yellow foam.
LCMS method F: [M+H]⁺ = 424.3, t_{R} = 2.54 min

### Preparation of Example 156: (13S)-13-methyl-8,14-dioxa-4,10,19,20,23-pentaazatetra cyclo[13.5.2.1^{2,6}.0^{18,21}]tricosa-1(20),2(23),3,5,15(22),16,18(21)-heptaen-9-one

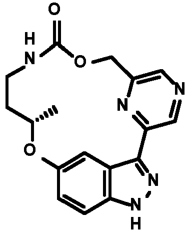

To a solution of (13S)-13-methyl-19-(oxan-2-yl)-8,14-dioxa-4,10,19,20,23-pentaazatetracyclo [13.5.2.1^{2,6}.0^{18,21}]tricosa-1(20),2(23),3,5,15(22),16,18(21)-heptaen-9-one intermediate **233** (183 mg, 0.43 mmol) in methanol (7.4 mL) and water (1.2 mL) was added *p*-toluenesulfonic acid monohydrate (409 mg, 2.15 mmol) and the reaction mixture was stirred at 65 °C for 16 h. The reaction mixture was diluted with dichloromethane and saturated aqueous solution of sodium bicarbonate. After separation, the aqueous layer was extracted with dichloromethane. The combined organic layers were washed brine, dried over anhydrous magnesium sulfate, filtered and evaporated under reduced pressure. The residue was crystallized in dichloromethane, filtered, and dried to afford (13S)-13-methyl-8,14-dioxa-4,10,19,20,23-pentaazatetra cyclo[13.5.2.1^{2,6}.0^{18,21}]tricosa-1(20),2(23),3,5,15(22),16,18(21)-heptaen-9-one **example 156** as a cream powder.
LCMS method F: [M+H]⁺ = 340.2, t_{R} = 1.86 min
LCMS method G: [M+H]⁺ = 340.3, t_{R} = 1.81 min
¹HNMR (400 MHz, d6-DMSO) δ 13.49 - 13.48 (1H, m), 9.27 (1H, s), 8.52 (1H, s), 7.86 - 7.81 (2H, m), 7.51 (1H, d, *J* = 8.8 Hz), 6.99 (1H, dd, *J* =2.4, 9.0 Hz), 5.69 (1H, d, *J* = 15.6 Hz), 5.18 - 5.14 (1H, m), 4.62 - 4.58 (1H, m), 3.52 - 3.46 (1H, m), 2.95 - 2.87 (1H, m), 2.35 - 2.28 (1H, m), 1.40 - 1.37 (4H, m) ppm.

### Example 157: (13R)-13-methyl-8,14-dioxa-10,19,20,22-tetraazatetracyclo[13.5.2. 1^{2,6}.0^{18,21}]tricosa-1(20),2(23),3,5,15,17,21-heptaen-9-one

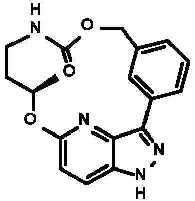

Example 157 is prepared according to the synthesis route described in general Scheme C.

### Preparation of intermediate 234: 5-methoxy-1H-pyrazolo[4,3-b]pyridine

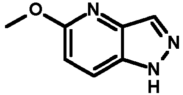

To a solution of 6-methoxy-2-methyl-pyridin-3-amine (4.0 g, 28.95 mmol) in acetic acid (40 mL) at 0°C was added dropwise a solution of sodium nitrate (2.99 g, 43.42 mmol) in water (8 mL). The reaction mixture was stirred at room temperature for 1 h. A saturated aqueous sodium bicarbonate solution was added and the reaction mixture was extracted with ethyl acetate. The combined organic layers were dried over anhydrous magnesium sulfate, filtered and the solvent was removed under reduced pressure. The residue was purified by silica gel column chromatography using cyclohexane/ethyl acetate 100/0 to 80/20 as eluent to afford 5-methoxy-1H-pyrazolo[4,3-b]pyridine intermediate **234** as a brown solid.
LCMS method F: [M+H]⁺ = 150.2, t_{R} = 1.33 min

### Preparation of intermediate 235: 3-iodo-5-methoxy-1H-pyrazolo[4,3-b]pyridine

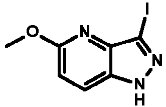

To a solution of 5-methoxy-1H-pyrazolo[4,3-b]pyridine intermediate **234** (1.88 g, 12.6 mmol) in acetonitrile (20 mL) was added *N*-iodosuccinimide (3.40 g, 15.12 mmol). The reaction mixture was heated under microwave irradiations at 120°C for 20 min. The reaction mixture was diluted with a saturated solution of sodium thiosulfate and ethyl acetate was added. After separation, the aqueous layer was extracted with ethyl acetate. The combined organic layers were dried over anhydrous magnesium sulfate, filtered and the solvent was removed under reduced pressure. The residue was purified by silica gel column chromatography using cyclohexane/ethyl acetate 100/0 to 70/30 as eluent to afford 3-iodo-5-methoxy-1H-pyrazolo[4,3-b]pyridine intermediate **235** as an orange solid.
LCMS method F: [M+H]⁺ = 276.0, t_{R} = 2.08 min

### Preparation of intermediate 236: 3-iodo-5-methoxy-1-tetrahydropyran-2-yl-pyrazolo[4,3-b]pyridine

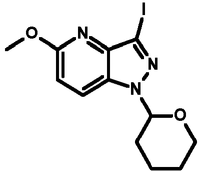

To a solution of 3-iodo-5-methoxy-1H-pyrazolo[4,3-b]pyridine intermediate **235** (700 mg, 2.54 mmol) in dichloromethane (4.6 mL) was added 4-methylbenzenesulfonic acid monohydrate (242 mg, 1.27 mmol) and 3,4-dihydro-2H-pyran (0.46 mL, 5.08 mmol). The reaction mixture was stirred at room temperature for 2 h. The reaction mixture was diluted with dichloromethane and a saturated aqueous sodium bicarbonate solution was added. After separation, the aqueous layer was extracted with dichloromethane. The combined organic layers were dried over anhydrous magnesium sulfate, filtered and the solvent was evaporated under reduced pressure to afford 3-iodo-5-methoxy-1-tetrahydropyran-2-yl-pyrazolo[4,3-b]pyridine intermediate **236** as an orange oil.
LCMS method F: [M+H]⁺ = 360.1, t_{R} = 2.82 min

### Preparation of intermediate 237: 3-iodo-1-tetrahydropyran-2-yl-pyrazolo[4,3-b]pyridin-5-ol

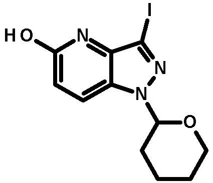

To a solution of 3-iodo-5-methoxy-1-tetrahydropyran-2-yl-pyrazolo[4,3-b]pyridine intermediate **236** (912 mg, 2.54 mmol) in acetonitrile (5 mL) was added sodium iodide (1.14 g, 7.62 mmol) and trimethylchlorosilane (0.97 mL, 7.62 mmol). The reaction mixture was stirred at 80°C for 1.5 h. The reaction mixture was diluted with water and ethyl acetate. After separation, the aqueous layer was extracted with ethyl acetate. The combined organic layers were dried over anhydrous magnesium sulfate, filtered and the solvent was removed under reduced pressure. The residue was purified by silica gel column chromatography using cyclohexane/(ethyl acetate/ethanol (3:1)) 100/0 to 30/70 as eluent. The resulting product was triturated in acetonitrile, filtered and dried to afford 3-iodo-1-tetrahydropyran-2-yl-pyrazolo[4,3-b]pyridin-5-ol intermediate **237** as a pale yellow solid.
LCMS method F: [M+H]⁺ = 346.1, t_{R} = 1.74 min

### Preparation of intermediate 238: benzyl N-[(3R)-3-(3-iodo-1-tetrahydropyran-2-yl-pyrazolo [4,3-b]pyridin-5-yl)oxybutyl]carbamate

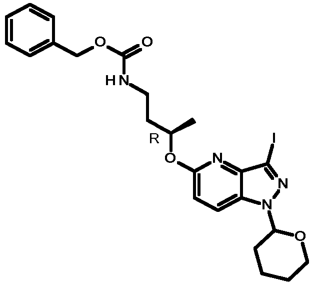

To a solution of 3-iodo-1-tetrahydropyran-2-yl-pyrazolo[4,3-b]pyridin-5-ol intermediate **237** (140 mg, 0.41 mmol) in acetonitrile (3.5 mL) was added cesium carbonate (240 mg, 0.81 mmol) and [(1S)-3-(benzyloxycarbonylamino)-1-methyl-propyl]methanesulfonate intermediate **199** (159 mg, 0.53 mmol). The reaction mixture was stirred at 75°C for 1 h. The reaction mixture was cooled to room temperature and diluted with ethyl acetate. After separation, the aqueous layer was extracted with ethyl acetate and the combined organic layers were dried over anhydrous magnesium sulfate, filtered and concentrated under reduced pressure to afford benzyl N-[(3R)-3-(3-iodo-1-tetrahydropyran-2-yl-pyrazolo[4,3-b]pyridin-5-yl)oxybutyl] carbamate intermediate **238** (as a beige solid.
LCMS method F: [M+H]⁺ = 551.2, t_{R} = 3.17 min

### Preparation of intermediate 239: Benzyl N-[(3R)-3-[3-[3-(hydroxymethyl)phenyl]-1-tetrahydropyran-2-yl-pyrazolo[4,3-b]pyridin-5-yl]oxybutyl]carbamate

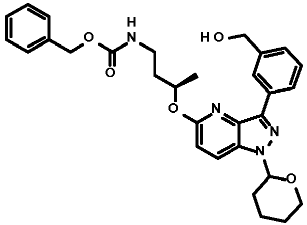

To a suspension of benzyl N-[(3R)-3-(3-iodo-1-tetrahydropyran-2-yl-pyrazolo[4,3-b]pyridin-5-yl)oxybutyl]carbamate intermediate **238** (210 mg, 0.38 mmol) in 1,4-dioxane (2.2 mL) and water (0.11 mL) was added [3-(hydroxymethyl)phenyl]boronic acid (69 mg, 0.46 mmol) and potassium phosphate tribasic (242 mg, 1.14 mmol). The reaction mixture was degassed with argon for 10 min and tetrakis(triphenylphosphine)palladium(0) (22 mg, 0.02 mmol) and Xphos (18 mg, 0.04 mmol) were added. The mixture was stirred at 105°C for 7 h. The reaction mixture was cooled to room temperature and diluted with water and ethyl acetate. After separation, the aqueous layer was extracted with ethyl acetate. The organic layers were washed with brine, dried over anhydrous sodium sulfate, filtered and evaporated under reduced pressure. The residue was purified by silica gel column chromatography using cyclohexane/ethyl acetate 100/0 to 60/40 as eluent to afford benzyl N-[(3R)-3-[3-[3-(hydroxymethyl)phenyl]-1-tetrahydropyran-2-yl-pyrazolo[4,3-b]pyridin-5-yl]oxybutyl]carbamate intermediate **239** as a colorless oil.
LCMS method F: [M+H]⁺ = 531.4, t_{R} = 3.00 min

### Preparation of intermediate 240: (13R)-13-methyl-19-(oxan-2-yl)-8,14-dioxa-10,19, 20,22-tetraazatetracyclo[13.5.2.1^{2,6}.0^{18,21}]tricosa-1(20),2(23),3,5,15,17,21-heptaen-9-one

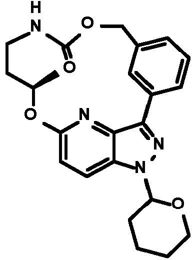

To a solution of benzyl N-[(3R)-3-[3-[3-(hydroxymethyl)phenyl]-1-tetrahydropyran-2-yl-pyrazolo[4,3-b]pyridin-5-yl]oxybutyl]carbamate intermediate **239** (156 mg, 0.29 mmol) in dry acetonitrile (40 mL) was added cesium carbonate (574 mg, 1.76 mmol). The resulting mixture was stirred at 90°C for 2 h. The suspension was filtered and the salts were washed with acetonitrile. The filtrate was evaporated under reduced pressure. The residue was purified by silica gel column chromatography using dichloromethane/methanol 100/0 to 95/5 as eluent to afford (13R)-13-methyl-19-(oxan-2-yl)-8,14-dioxa-10,19,20,22-tetraazatetracyclo [13.5.2.1^{2,6}.0^{18,21}]tricosa-1(20),2(23),3,5,15,17,21-heptaen-9-one intermediate **240** as a colorless oil.
LCMS method F: [M+H]⁺ = 423.4, t_{R} = 2.88 min

### Preparation of Example 157: (13R)-13-methyl-8,14-dioxa-10,19,20,22-tetraazatetracyclo [13.5.2.1^{2,6}.0^{18,21}]tricosa-1(20),2(23),3,5,15,17,21-heptaen-9-one

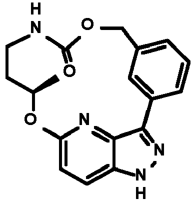

To a solution of (13R)-13-methyl-19-(oxan-2-yl)-8,14-dioxa-10,19,20,22-tetraazatetracyclo [13.5.2.1^{2,6}.0^{18,21}]tricosa-1(20),2(23),3,5,15,17,21-heptaen-9-one intermediate **240** (124 mg, 0.29 mmol) in methanol (5.5 mL) and water (0.8 mL) was added p-toluenesulfonic acid (280 mg, 1.47 mmol). The reaction mixture was stirred at 65°C for 2 h. The reaction mixture was cooled to room temperature and the reaction was carefully quenched with a saturated aqueous sodium bicarbonate solution then ethyl acetate was added. After separation, the aqueous layer was extracted with ethyl acetate and the organic layers were washed with brine, dried over anhydrous sodium sulfate, filtered and evaporated under reduced pressure. The residue was purified by silica gel column chromatography using dichloromethane/methanol 100/0 to 96/4 as eluent to afford (13R)-13-methyl-8,14-dioxa-10,19,20,22-tetraazatetracyclo[13.5.2. 1^{2,6}.0^{18,21}]tricosa-1(20),2(23),3,5,15,17,21-heptaen-9-one **example 157** as a white solid.
LCMS method F: [M+H]⁺ = 339.3, t_{R} = 2.17 min
LCMS method G: [M+H]⁺ = 339.3, t_{R} = 2.15 min
¹H NMR (400 MHz, d6-DMSO) δ 13.25 (1H, brs), 8.58 (1H, s), 8.04 - 7.93 (2H, m), 7.71 (1H, t, J=6.3 Hz), 7.42 (1H, t, J=7.7 Hz), 7.22 - 7.18 (1H, m), 6.81 (1H, d, J=9.1 Hz), 5.61 - 5.53 (1H, m), 5.51 - 5.44 (1H, m), 5.10 - 5.03 (1H, m), 3.43 - 3.36 (1H, m), 3.02 - 2.94 (1H, m), 2.45 - 2.36 (1H, m), 1.39 (3H, d, J=6.2 Hz), 1.38 - 1.34 (1H, m) ppm.

### Example 158: (12R)-4,12-dimethyl-7,13-dioxa-4,9,18,19,22-pentaazatetracyclo[12.5.2. 1^{2,5}.0^{17,20}]docosa-1(19),2,5(22),14(21),15,17(20)-hexaen-8-one

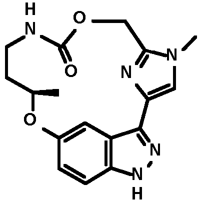

Example 158 is prepared according to the synthesis route described in general Scheme K.

### Preparation of intermediate 241: (4-bromo-1-methyl-imidazol-2-yl)methanol

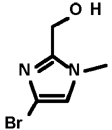

To a suspension of methyl 4-bromo-1-methyl-imidazole-2-carboxylate (3.0 g, 13.76 mmol) in methanol (30 mL) at 0°C was added portion wise sodium borohydride (1.14 g, 30.27 mmol). The reaction mixture was stirred at 0°C for 2 h. Additional sodium borohydride (1.14 g, 30.27 mmol) was added and the reaction mixture was stirred at room temperature for 3 h. The reaction mixture was quenched by addition of water and methanol then evaporated under reduced pressure. The aqueous phase was extracted with ethyl acetate. The organic phases were washed with brine, dried over anhydrous sodium sulfate, filtered and evaporated under reduced pressure to afford (4-bromo-1-methyl-imidazol-2-yl)methanol intermediate **241** as a white solid.
LCMS method H: [M+H]⁺ = 191-193, t_{R} = 0.93 min

### Preparation of intermediate 242: [4-[5-[tert-butyl(dimethyl)silyl]oxy-1-tetrahydropyran-2-yl-indazol-3-yl]-1-methyl-imidazol-2-yl]methanol

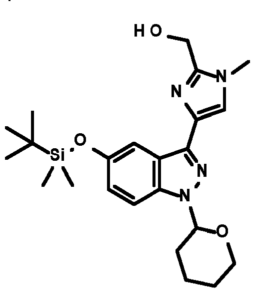

To a solution of *tert*-butyl-dimethyl-[1-tetrahydropyran-2-yl-3-(4,4,5,5-tetramethyl-1,3,2-dioxaborolan-2-yl)indazol-5-yl]oxy-silane (753 mg, 1.64 mmol), (4-bromo-1-methyl-imidazol-2-yl)methanol intermediate **241** (250 mg, 1.31 mmol) and potassium phosphate tribasic (833 mg, 3.93 mmol) in 1,4-dioxane (3 mL) and water (150 µL) were added tetrakis (triphenylphosphine) palladium(0) (76 mg, 0.065 mmol) and 2-dicyclohexylphosphino-2',4',6'-triisopropylbiphenyl (62 mg, 0.131 mmol). The reaction mixture was heated at 100 °C for 1 h. The solvent was evaporated under reduced pressure and the residue was portioned between water and ethyl acetate. The aqueous layer was extracted with ethyl acetate. The organic layer was dried over anhydrous sodium sulfate, filtered and evaporated under reduced pressure. The residue was purified by silica gel column chromatography using cyclohexane/ethyl acetate 100/0 to 50/50 then dichloromethane/methanol 90/10 to afford [4-[5-[*tert-*butyl(dimethyl)silyl]oxy-1-tetrahydropyran-2-yl-indazol-3-yl]-1-methyl-imidazol-2-yl]methanol intermediate **242** as a yellow oil.
LCMS method F: [M+H]⁺ = 443.3, t_{R} = 2.33 min

### Preparation of intermediate 243: benzyl N-[(3R)-3-[3-[2-(hydroxymethyl)-1-methyl-imidazol-4-yl]-1-tetrahydropyran-2-yl-indazol-5-yl]oxybutyl]carbamate

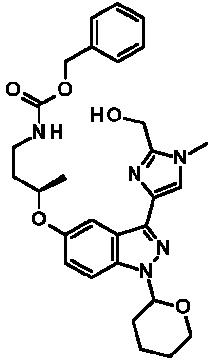

To a suspension of [4-[5-[*tert*-butyl(dimethyl)silyl]oxy-1-tetrahydropyran-2-yl-indazol-3-yl]-1-methyl-imidazol-2-yl]methanol intermediate **242** (100 mg, 0.226 mmol) and cesium carbonate (220 mg, 0.678mmol) in acetonitrile (30 mL) wad added [(1S)-3-(benzyloxycarbonylamino)-1-methyl-propyl] methanesulfonate (88 mg, 0.294 mmol). The reaction mixture was stirred at 50°C for 16 h. Additional cesium carbonate (220 mg, 0.678mmol) was added and the mixture was stirred at 60°C for 16 h. The solvent was evaporated under reduced pressure. The residue was portioned between water and ethyl acetate. After separation, the aqueous layer was extracted with ethyl acetate. The organic phase was dried over anhydrous sodium sulfate, filtered and the solvent was evaporated under reduced pressure. The residue was purified by silica gel column chromatography using cyclohexane/ethyl acetate 100/0 to 70/30 as eluent to afford benzyl N-[(3R)-3-[3-[2-(hydroxymethyl)-1-methyl-imidazol-4-yl]-1-tetrahydropyran-2-yl-indazol-5-yl]oxybutyl]carbamate intermediate **243** as a yellow oil.
LCMS method F: [M+H]⁺ = 534.4, t_{R} = 2.08 min

### Preparation of intermediate 244: (12R)-4,12-dimethyl-18-(oxan-2-yl)-7,13-dioxa-4,9,18,19, 22-pentaazatetracyclo[12.5.2.1^{2,5}.0^{17,20}]docosa-1(19),2,5(22),14(21),15,17(20)-hexaen-8-one

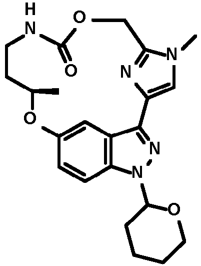

To a solution of benzyl N-[(3R)-3-[3-[2-(hydroxymethyl)-1-methyl-imidazol-4-yl]-1-tetrahydropyran-2-yl-indazol-5-yl]oxybutyl]carbamate intermediate **243** (200 mg, 0.375 mmol) in acetonitrile (20 mL) was added potassium hydroxyde (105 mg, 1.87 mmol). The reaction mixture was stirred at room temperature for 16 h. The solvent was evaporated under reduced pressure and the residue was dissolved in ethyl acetate and washed with water. The organic layer was dried over anhydrous sodium sulfate, filtered and evaporated under reduced pressure. The residue was purified by silica gel column chromatography using dichloromethane/methanol 100/0 to 90/10 as eluent to (12R)-4,12-dimethyl-18-(oxan-2-yl)-7,13-dioxa-4,9,18,19,22-pentaazatetracyclo[12.5.2.1^{2,5}.0^{17,20}]docosa-1(19),2,5(22),14(21),15, 17(20)-hexaen-8-one intermediate **244** as a yellow oil.
LCMS method F: [M+H]⁺ = 426.3, t_{R} = 2.16 min

### Preparation of Example 158 : (12R)-4,12-dimethyl-7,13-dioxa-4,9,18,19,22-pentaazatetra cyclo[12.5.2.1^{2,5}.0^{17,20}]docosa-1(19),2,5(22),14(21),15,17(20)-hexaen-8-one

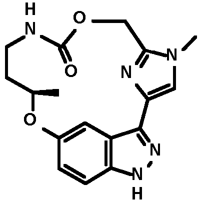

To a solution of (12R)-4,12-dimethyl-18-(oxan-2-yl)-7,13-dioxa-4,9,18,19,22-pentaaza tetracyclo[12.5.2.1^{2,5}.0^{17,20}]docosa-1(19),2,5(22),14(21),15,17(20)-hexaen-8-one intermediate **244** (56 mg, 0.13 mmol) in methanol (5 mL) and water (0.5 mL) was added*p*-toluenesulfonic acid monohydrate (125 mg, 0.66 mmol). The reaction mixture was stirred at 65°C for 2 h. The solvent was evaporated under reduced pressure and the residue was neutralized by slow addition of saturated aqueous sodium bicarbonate solution. The residue was diluted with ethyl acetate. After separation, the aqueous layer was extracted with ethyl acetate. The organic layer was washed with saturated aqueous sodium bicarbonate solution, water and brine, dried over anhydrous sodium sulfate, filtered and evaporated under reduced pressure. The residue was purified by preparative TLC using dichloromethane/methanol 100/0 to 95/5 as eluent to afford (12R)-4,12-dimethyl-7,13-dioxa-4,9,18,19,22-pentaazatetracyclo[12.5.2.1^{2,5}.0^{17,20}]docosa-1(19),2,5(22), 14(21), 15, 17(20)-hexaen-8-one **example 158** as a cream powder.
LCMS method F: [M+H]⁺ = 342.3, t_{R} = 1.67 min
LCMS method G: [M+H]⁺ = 342.3, t_{R} = 1.73 min
¹H NMR (400 MHz, d6-DMSO) δ 12.6 - 12.4 (1H; m), 8.32 (1H, d, J=2.3 Hz), 7.88 - 7.85 (1H, m), 7.42 (1H, s), 7.32 - 7.29 (1H, m), 6.85 (1H, dd, J=2.5, 8.9 Hz), 5.48 (1H, d, J=14.8 Hz), 5.01 - 4.96 (1H, m), 4.62 -4.52 (1H, m), 3.63 - 3.62 (3H, m), 2.91 - 2.78 (1H, m), 2.45 - 2.38 (1H, m), 1.39 (3H, d, J=5.9 Hz), 1.27 - 1.24 (2H, m) ppm.

### Example 159: (13R)-13-methyl-8,14-dioxa-4,5,10,19,20,23-hexaazatetracyclo[13.5.2. 1^{2,5}.0^{18,21}]tricosa-1(20),2(23),3,15,17,21-hexaen-9-one

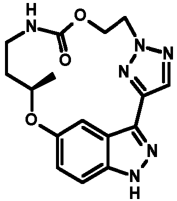

Example 159 is prepared according to the synthesis route described in general Scheme O.

### Preparation of intermediate 245: 4,5-dibromo-2-(2-tetrahydropyran-2-yloxyethyl) triazole

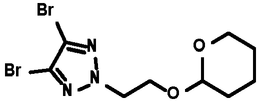

To a solution of 4,5-dibromo-2H-triazole (3.40 g, 15.0 mmol) in *N,N-*dimethylformamide (100 mL) cooled at -10 °C was added potassium carbonate (4.14 g, 30.0 mmol). The reaction mixture was stirred at room temperature for 15 min. 2-(2-bromoethoxy)tetrahydropyran (3.44 g, 16.50 mmol) in *N,N*-dimethylformamide (10 mL) was added dropwise and the reaction mixture was stirred at room temperature for 16 h. The reaction mixture was quenched by addition of water then extracted with ethyl acetate. The combined organic layers were washed with brine, dried over anhydrous magnesium sulfate, filtered and the solvent was removed under reduced pressure. The residue was purified by silica gel column chromatography using heptane/ethyl acetate 90/10 to 80/20 as eluent to afford 4,5-dibromo-2-(2-tetrahydropyran-2-yloxyethyl)triazole intermediate **245** as a colorless liquid.
LCMS method F: [M+H]⁺ = 356.1, t_{R} = 2.69 min

### Preparation of intermediate 246: 4-bromo-2-(2-tetrahydropyran-2-yloxyethyl)triazole

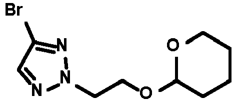

To a solution of 4,5-dibromo-2-(2-tetrahydropyran-2-yloxyethyl) triazole intermediate **245** (1.90 g, 5.35 mmol) in THF (50 mL) cooled at -20 °C was added dropwise a 2M of isopropylmagnesium chloride solution in THF (3.20 mL, 6.42 mmol). The reaction mixture was stirred at room temperature for 16 h then diluted with a saturated ammonium chloride solution. After separation, the aqueous layer was extracted with ethyl acetate. The combined organic layers were washed with brine, dried over anhydrous magnesium sulfate, filtered and evaporated under reduced pressure. The residue was purified by silica gel column chromatography using heptane/ethyl acetate 100/0 to 90/10 as eluent to afford 4-bromo-2-(2-tetrahydropyran-2-yloxyethyl)triazole intermediate **246** as a colorless liquid.
LCMS method F: [M+H]⁺ = 276-278, t_{R} = 2.34 min

### Preparation of intermediate 247: 2-(4-bromotriazol-2-yl)ethanol

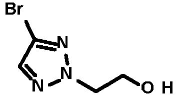

To a solution of 4-bromo-2-(2-tetrahydropyran-2-yloxyethyl)triazole intermediate **246** (2.45 g, 8.88 mmol) in THF (70 mL) cooled at 0 °C was added 4M hydrogen chloride solution in 1,4-dioxane (6.66 mL, 26.61 mmol). The reaction mixture was stirred at room temperature for 24 h. The reaction mixture was diluted with a saturated aqueous sodium bicarbonate solution. After separation, the aqueous layer was extracted with ethyl acetate. The combined organic layers were washed with brine, dried over anhydrous magnesium sulfate, filtered and evaporated under reduced pressure. The residue was purified by silica gel flash chromatography using heptane/ethyl acetate 70/30 as eluent to afford 2-(4-bromotriazol-2-yl)ethanol intermediate **247** as a colorless liquid.
LCMS method F: [M+H]⁺ = 192-194, t_{R} = 1.20 min

### Preparation of intermediate 248: 2-[4-[5-[tert-butyl(dimethyl)silyl]oxy-1-tetrahydropyran-2-yl-indazol-3-yl]triazol-2-yl]ethanol

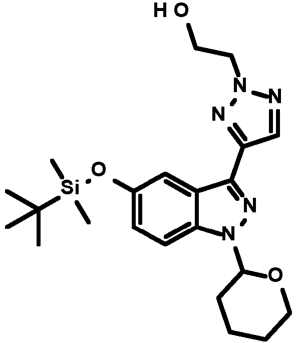

To a solution of 2-(4-bromotriazol-2-yl)ethanol intermediate **247** (420 mg, 2.18 mmol) in 1,4-dioxane (70 mL) and water (7 mL) was added *tert*-butyl-dimethyl-[1-tetrahydropyran-2-yl-3-(4,4,5,5-tetramethyl-1,3,2-dioxaborolan-2-yl)indazol-5-yl]oxy-silane (1.20 g, 2.62 mmol) and potassium phosphate tribasic (1.39 g, 6.56 mmol). The solution was purged with argon and tetrakis(triphenylphosphine)palladium(0) (126 mg, 0.11 mmol) was added. The reaction mixture was stirred at 90 °C for 16 h. The solvent was evaporated under reduced pressure and the residue was diluted with water and ethyl acetate. After separation, the aqueous layer was extracted with ethyl acetate. The combined organic layers were washed with brine, dried over anhydrous magnesium sulfate, filtered and evaporated under reduced pressure. The residue was purified by silica gel flash chromatography using heptane/ethyl acetate 80/20 as eluent to afford 2-[4-[5-[tert-butyl(dimethyl)silyl]oxy-1-tetrahydropyran-2-yl-indazol-3-yl]triazol-2-yl] ethanol intermediate **248** as a pale brown oil.
LCMS method F: [M+H]⁺ = 444.3, t_{R} = 3.28 min

### Preparation of intermediate 249: (13R)-13-methyl-19-(oxan-2-yl)-8,14-dioxa-4,5,10,19,20, 23-hexaazatetracyclo[13.5.2.1^{2,5}.0^{18,21}]tricosa-1(20),2(23),3,15,17,21-hexaen-9-one

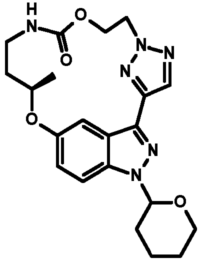

To a suspension of 2-[4-[5-[tert-butyl(dimethyl)silyl]oxy-1-tetrahydropyran-2-yl-indazol-3-yl]triazol-2-yl]ethanol intermediate **248** (480 mg, 1.08 mmol) and cesium carbonate (1.05 g, 3.24 mmol) in acetonitrile (25 mL) was added a solution of [(1S)-3-(benzyloxycarbonylamino)-1-methyl-propyl] methanesulfonate (391 mg, 1.29 mmol) in acetonitrile (5 mL). The resulting mixture was stirred at 50°C for 16 h. Additional acetonitrile (25 mL) and cesium carbonate (1.05 g, 3.24 mmol) were added. The reaction mixture was stirred at 70°C for 3 h. The reaction mixture was filtered to remove salts and washed with acetonitrile. The filtrate was concentrated under reduced pressure. The residue was purified by silica gel column chromatography using heptane/ethyl acetate 70/30 to 60/40 as eluent to afford (13R)-13-methyl-19-(oxan-2-yl)-8,14-dioxa-4,5,10,19,20,23-hexaazatetracyclo[13.5.2.1^{2,5}.0^{18,21}]tricosa-1(20),2(23),3,15,17,21-hexaen-9-one intermediate **249** as a white solid.
LCMS method F: [M+H]⁺ = 427.3, t_{R} = 2.50 min

### Preparation of Example 159: (13R)-13-methyl-8,14-dioxa-4,5,10,19,20,23-hexaaza tetra cyclo[13.5.2.1^{2,5}.0^{18,21}]tricosa-1(20),2(23),3,15,17,21-hexaen-9-one

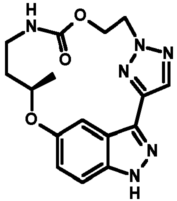

To a solution of (13R)-13-methyl-19-(oxan-2-yl)-8,14-dioxa-4,5,10,19,20,23-hexaazatetra cyclo[13.5.2.1^{2,5}.0^{18,21}]tricosa-1(20),2(23),3,15,17,21-hexaen-9-one intermediate **249** (100 mg, 0.23 mmol) in methanol (14 mL) ) and water (2 mL) was added *p*-toluenesulfonic acid monohydrate (222 mg, 1.17 mmol). The reaction mixture was stirred at 65°C for 16 h. The solvent was partially removed under reduced pressure and a saturated aqueous sodium bicarbonate solution was added. The aqueous layer was extracted with ethyl acetate. The combined organic layers were washed with brine, dried over anhydrous magnesium sulfate, filtered and the solvent was evaporated under reduced pressure. The residue was purified by silica gel column chromatography using dichloromethane/methanol 100/0 to 95/5 as eluent. The resulting product was recrystallized in acetonitrile, filtered and dried to afford (13R)-13-methyl-8,14-dioxa-4,5,10,19,20,23-hexaazatetracyclo[13.5.2.1^{2,5}.0^{18,21}]tricosa-1(20),2(23),3,15,17,21-hexaen-9-one **example 159** as a white solid.
LCMS method F: [M+H]⁺ = 343.3, t_{R} = 1.89 min
LCMS method G: [M+H]⁺ = 343.3, t_{R} = 1.90 min
¹H NMR (400 MHz, d6-DMSO) δ 13.12 (1H, s), 8.12 (1H, s), 7.64 (1H, t, J=6.0 Hz), 7.53 - 7.44 (2H, m), 6.96 (1H, dd, J=2.0, 9.0 Hz), 4.90 - 4.84 (1H, m), 4.78 - 4.72 (2H, m), 4.62 - 4.56 (1H, m), 4.34 - 4.27 (1H, m), 3.42 - 3.29 (1H, m), 2.96 - 2.89 (1H, m), 2.13 - 2.06 (1H, m), 1.38 - 1.34 (4H, m) ppm.

### Example 160: (13R)-13-methyl-8,14-dioxa-23-thia-4,10,19,20-tetraazatetracyclo[13.5.2. 1^{2,5}.0^{18,21}]tricosa-1(20),2,4,15,17,21-hexaen-9-one

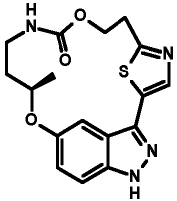

Example 160 is prepared according to the synthesis route described in general Scheme L.

### Preparation of intermediate 250: 2-(5-bromothiazol-2-yl)ethanol

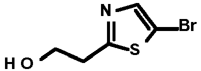

To a solution of ethyl 2-(5-bromothiazol-2-yl)acetate (1.50 g, 6.00 mmol) in THF (20 mL) cooled at 0 °C was added a solution of sodium borohydride (340 mg, 9.00 mmol) in ethanol (4 mL). The reaction mixture was stirred at room temperature for 48 h. The reaction mixture was quenched by addition of water at 0 °C and the reaction mixture was extracted with ethyl acetate. The combined organic layers were washed with brine, dried over anhydrous magnesium sulfate, filtered and the solvent was evaporated under reduced pressure. The residue was purified by silica gel column chromatography using heptane/ethyl acetate 100/0 to 50/50 as eluent to afford 2-(5-bromothiazol-2-yl)ethanol intermediate **250** as a yellow oil.
LCMS method F: [M+H]⁺ = 208-210, t_{R} = 1.46 min

### Preparation of intermediate 251: 2-(5-bromothiazol-2-yl)ethyl N-[(3S)-3-hydroxybutyl] carbamate

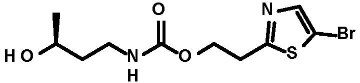

To a solution of 4-nitrophenyl chloroformate (480 mg, 2.38 mmol) and pyridine (0.35 mL, 4.32 mmol) in dichloromethane (5 mL) was added dropwise a solution of 2-(5-bromothiazol-2-yl)ethanol intermediate **250** (450 mg, 2.16 mmol) in dichloromethane (3 mL). The reaction mixture was stirred at room temperature for 1 h. Then a solution of (2S)-4-aminobutan-2-ol (210 mg, 2.38 mmol) and DIPEA (0.75 mL, 4.32 mmol) in dichloromethane (3 mL) was added. The reaction mixture was stirred at room temperature for 16 h. The reaction mixture was diluted with an 0.5 M aqueous sodium hydroxide solution and extracted with dichloromethane. The combined organic layers were washed again with a 0.5 M aqueous sodium hydroxide solution, dried over anhydrous magnesium sulfate, filtered and evaporated under reduced pressure. The residue was purified by silica gel column chromatography using dichloromethane/ethyl acetate 100/0 to 20/80 as eluent to afford 2-(5-bromothiazol-2-yl)ethyl N-[(3S)-3-hydroxybutyl]carbamate intermediate **251** as a yellow oil.
LCMS method F: [M+H]⁺ = 325.1, t_{R} = 1.75 min

### Preparation of intermediate 252: 2-[5-[5-[tert-butyl(dimethyl)silyl]oxy-1-tetrahydropyran-2-yl-indazol-3-yl]thiazol-2-yl]ethyl N-[(3S)-3-hydroxybutyl]carbamate

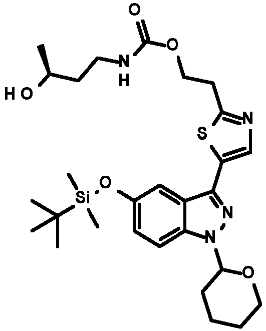

To a solution of 2-(5-bromothiazol-2-yl)ethyl N-[(3S)-3-hydroxybutyl]carbamate intermediate **251** (350 mg, 1.08 mmol) in 1,4-dioxane (10 mL) and water (1 mL) was added *tert*-butyldimethyl-[1-tetrahydropyran-2-yl-3-(4,4,5,5-tetramethyl-1,3,2-dioxaborolan-2-yl)indazol-5-yl]oxy-silane (600 mg, 1.30 mmol) and potassium phosphate tribasic (690 mg, 3.25 mmol). The solution was purged with nitrogen then XPhos (52 mg, 0.11 mmol) and tetrakis(triphenylphosphine) palladium(0) (63 mg, 0.05 mmol) were added. The reaction mixture was stirred under microwave irradiations at 90 °C for 1.5 h. The reaction was diluted with brine and extracted with ethyl acetate. The combined organic layers were dried over anhydrous magnesium sulfate, filtered and the solvent was removed under reduced pressure. The residue was purified by silica gel column chromatography using heptane/ethyl acetate 100/0 to 30/70 as eluent to afford 2-[5-[5-[tert-butyl(dimethyl)silyl]oxy-1-tetrahydropyran-2-yl-indazol-3-yl]thiazol-2-yl]ethyl N-[(3S)-3-hydroxybutyl]carbamate intermediate **252** as a colorless oil.
LCMS method F: [M+H]⁺ = 575.4, t_{R} = 3.32 min

### Preparation of intermediate 253: [(1S)-3-[2-[5-[5-[tert-butyl(dimethyl)silyl]oxy-1-tetrahydro pyran-2-yl-indazol-3-yl]thiazol-2-yl]ethoxycarbonylamino]-1-methyl-propyl]methane sulfonate

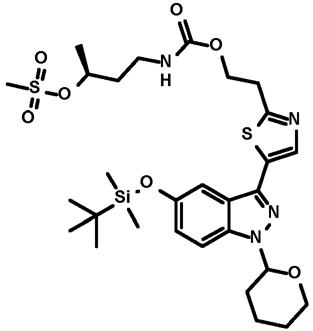

To a solution of 2-[5-[5-[tert-butyl(dimethyl)silyl]oxy-1-tetrahydropyran-2-yl-indazol-3-yl]thiazol-2-yl]ethyl N-[(3S)-3-hydroxybutyl]carbamate intermediate **252** (530 mg, 0.92 mmol) and triethylamine (0.26 mL, 1.84 mmol) in dichlromethane (8 mL) cooled at 0 °C was added methanesulfonyl chloride (0.09 mL, 1.20 mmol) in dichloromethane (2 mL). The reaction mixture was stirred at room temperature for 16 h. The reaction mixture was diluted with brine and extracted with dichloromethane. The combined organic layers were washed with brine, dried over anhydrous magnesium sulfate, filtered and the solvent was removed under reduced pressure to afford [(1S)-3-[2-[5-[5-[tert-butyl(dimethyl)silyl]oxy-1-tetrahydropyran-2-yl-indazol-3-yl]thiazol-2-yl]ethoxycarbonylamino]-1-methyl-propyl]methanesulfonate intermediate **253** as a yellow oil.
LCMS method F: [M+H]⁺ = 653.5, t_{R} = 3.45 min

### Preparation of intermediate 254: (13R)-13-methyl-19-(oxan-2-yl)-8,14-dioxa-23-thia-4,10,19,20-tetraazatetracyclo[13.5.2.1^{2,5}.0^{18,21}]tricosa-1(20),2,4,15,17,21-hexaen-9-one

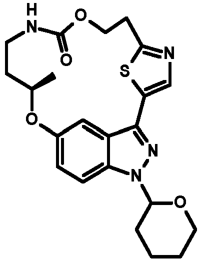

To a solution of [(1S)-3-[2-[5-[5-[tert-butyl(dimethyl)silyl]oxy-1-tetrahydropyran-2-yl-indazol-3-yl]thiazol-2-yl]ethoxycarbonylamino]-1-methyl-propyl]methanesulfonate intermediate **253** (500 mg, 0.77 mmol) in dry *N,N*-dimethylformamide (195 mL) at 80°C was added dropwise over 2 h a solution of cesium carbonate (750 mg, 2.30 mmol) in dry *N,N-*dimethylformamide (190 mL). The reaction mixture was stirred at 80°C for 1 h. The solvent was evaporated under reduced pressure and the residue was diluted with brine and ethyl acetate. The aqueous layer was extracted with ethyl acetate. The combined organic layers were washed with brine, dried over anhydrous magnesium sulfate, filtered and the solvent was evaporated under reduced pressure The residue was purified by silica gel column chromatography using dichloromethane/ethyl acetate 100/0 to 20/80 as eluent to afford (13R)-13-methyl-19-(oxan-2-yl)-8,14-dioxa-23-thia-4,10,19,20-tetraazatetracyclo[13.5.2.1^{2,5}.0^{18,21}]tricosa-1(20),2,4,15, 17,21-hexaen-9-one intermediate **254** as a colorless oil.
LCMS method F: [M+H]⁺ = 443.4, t_{R} = 2.43 min

### Preparation of Example 160: (13R)-13-methyl-8,14-dioxa-23-thia-4,10,19,20-tetraaza tetracyclo[13.5.2.1^{2,5}.0^{18,21}]tricosa-1(20),2,4,15,17,21-hexaen-9-one

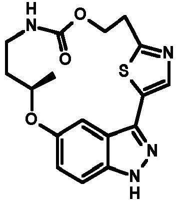

To a solution of (13R)-13-methyl-19-(oxan-2-yl)-8,14-dioxa-23-thia-4,10,19,20-tetraazatetra cyclo[13.5.2.1^{2,5}.0^{18,21}]tricosa-1(20),2,4,15,17,21-hexaen-9-one intermediate **254** (44 mg, 0.10 mmol) in methanol (7 mL) and water (1 mL) was added *p*-toluenesulfonic acid monohydrate (95 mg, 0.50 mmol). The reaction mixture was stirred at 70°C for 24 h. The solvent was partially removed under reduced pressure and a saturated aqueous sodium bicarbonate solution was added. The aqueous layer was extracted with ethyl acetate. The combined organic layers were washed with brine, dried over anhydrous magnesium sulfate, filtered and the solvent was removed under reduced pressure. The residue was triturated in diisopropylether, filtered and dried to afford (13R)-13-methyl-8,14-dioxa-23-thia-4,10,19,20-tetraazatetracyclo [13.5.2.1^{2,5}.0^{18,21}]tricosa-1(20),2,4,15,17,21-hexaen-9-one **example 160** as a light yellow solid.
LCMS method F: [M+H]⁺ = 359.3, t_{R} = 1.87 min
LCMS method G: [M+H]⁺ = 359.3, t_{R} = 1.87 min
¹H NMR (400 MHz, d6-DMSO) δ 13.22 (1H, s), 7.99 (1H, s), 7.92 (1H, dd, J=4.4, 8.2 Hz), 7.50 - 7.47 (1H, m), 7.39 (1H, d, J=2.5 Hz), 6.97 (1H, dd, J=2.4, 9.0 Hz), 4.71 - 4.61 (2H, m), 4.14 - 4.08 (1H, m), 3.61 - 3.53 (1H, m), 3.41 - 3.34 (2H, m), 2.93 - 2.86 (1H, m), 2.08 - 2.08 (1H, m), 1.41 - 1.38 (4H, m) ppm.

### Example 161: (13R)-4,13-dimethyl-8,14-dioxa-4,10,19,20,23-pentaazatetracyclo[13.5.2. 1^{2,5}.0^{18,21}]tricosa-1(20),2,5(23),15(22),16,18(21)-hexaen-9-one

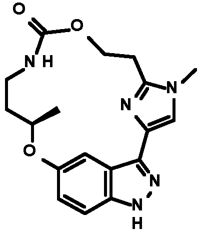

Example 161 is prepared according to the synthesis route described in general Scheme L.

### Preparation of intermediate 255: tert-butyl-[2-(1H-imidazol-2-yl)ethoxy]-dimethyl-silane

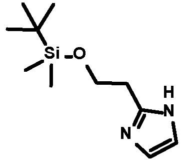

To a solution of 2-(1H-imidazol-2-yl)ethanol (1.2 g, 10.7 mmol) in *N,N*-dimethylformamide (11.5 mL) at room temperature was added imidazole (1.09 g, 16.05 mmol) and *tert*butyldimethylsilyl chloride (1.61 g, 10.7 mmol). The reaction mixture was stirred at room temperature for 16 h. The reaction mixture was poured in water (150 mL) and extracted with ethyl acetate. The combined organic layers were washed with 10 % lithium chloride aqueous solution, water and brine, dried over anhydrous sodium sulfate, filtered and concentrated under reduced pressure to yield *tert*-butyl-[2-(1H-imidazol-2-yl)ethoxy]-dimethyl-silane intermediate **255** as a yellow oil which was used in the next step without further purification.
LCMS method F: [M+H]⁺ = 227.3, t_{R} = 1.64 min

### Preparation of intermediates 256A and 256B: tert-butyl-[2-(4,5-dibromo-1H-imidazol-2-yl)ethoxy]-dimethyl-silane and 2-(4-bromo-1H-imidazol-2-yl)ethoxy-tert-butyl-dimethyl-silane

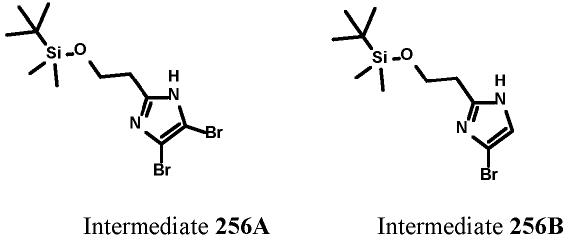

To a suspension of *tert*-butyl-[2-(1H-imidazol-2-yl)ethoxy]-dimethyl-silane intermediate **255** (2.379 g, 10.52 mmol) in THF (350 mL) at room temperature was added *N*-bromosuccinimide (1.955 g, 11.05 mmol). The reaction mixture was stirred at room temperature for 30 min. The reaction mixture was diluted with ethyl acetate, washed with water and brine, dried over anhydrous magnesium sulfate, filtered and concentrated under reduced pressure. The residue was purified by silica gel column chromatography using cyclohexane/ethyl acetate 100/0 to 95/5 as eluent, to afford two different products:
*tert*-butyl-[2-(4,5-dibromo-1H-imidazol-2-yl)ethoxy]-dimethyl-silane intermediate **256A** as a colorless oil; LCMS method F: [M+H]⁺ = 383.1-385.1-387.0, t_{R} = 3.00 min,
and 2-(4-bromo-1H-imidazol-2-yl)ethoxy-*tert*-butyl-dimethyl-silane intermediate **256B** as a colorless oil; LCMS method F: [M+H]⁺ = 305.1-307.1, t_{R} = 2.34 min

### Preparation of intermediate 257: tert-butyl-[2-(4,5-dibromo-1-methyl-imidazol-2-yl)ethoxy]-dimethyl-silane

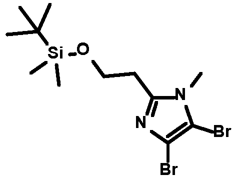

To a suspension of *tert*-butyl-[2-(4,5-dibromo-1H-imidazol-2-yl)ethoxy]-dimethyl-silane intermediate **256A** (600 mg, 1.56 mmol) in *N,N*-dimethylformamide (2.5 mL) at room temperature was added potassium carbonate (473 mg, 3.43 mmol) followed by iodomethane (0.11 mL, 1.87 mmol). The reaction mixture was stirred at room temperature for 16 h. The reaction mixture was diluted with dichloromethane, washed with water and brine, dried over anhydrous magnesium sulfate, filtered and concentrated under reduced pressure to afford *tert-*butyl-[2-(4,5-dibromo-1-methyl-imidazol-2-yl)ethoxy]-dimethyl-silane intermediate **257** as a yellow solid which was used without further purification.
LCMS method F: [M+H]⁺ = 397.0-399.1-401.0, t_{R} = 3.24 min

### Preparation of intermediate 258: 2-(4-bromo-1-methyl-imidazol-2-yl)ethoxy-tert-butyl-dimethyl silane

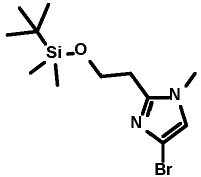

To a degassed solution of *tert*-butyl-[2-(4,5-dibromo-1-methyl-imidazol-2-yl)ethoxy]-dimethyl-silane intermediate **257** (614 mg, 1.54 mmol) in dry THF (24 mL) at -78 °C, was added 2.5 M *n*-BuLi solution in THF (0.6 mL, 1.54 mmol). The reaction mixture was stirred at -78 °C for 30 min. The reaction mixture was quenched by addition of water and warmed to room temperature. The aqueous layer was extracted with ethyl acetate. The combined organic layers were washed with brine, dried over anhydrous sodium sulfate, filtered and concentrated under reduced pressure to afford 2-(4-bromo-1-methyl-imidazol-2-yl)ethoxy-tert-butyldimethyl-silane intermediate **258** as a yellow oil.
LCMS method F: [M+H]⁺ = 319.2-321.2, t_{R} = 2.70 min

### Preparation of intermediate 259: 2-(4-bromo-1-methyl-imidazol-2-yl)ethanol

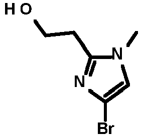

To a solution of 2-(4-bromo-1-methyl-imidazol-2-yl)ethoxy-tert-butyl-dimethyl-silane intermediate **258** (300 mg, 0.94 mmol) in THF (4 mL) at room temperature was added dropwise a solution of tetrabutylammonium fluoride 1 M in THF (1.03 mL, 1.03 mmol). The reaction mixture was stirred at room temperature for 6 h. The reaction mixture was poured into ice water and stirred for 20 min. The aqueous phase was extracted with ethyl acetate. The organic layers were washed with brine, dried over anhydrous magnesium sulfate and concentrated under reduced pressure. The residue was purified by silica gel column chromatography using dichloromethane/methanol 100/0 to 95/5 as eluent to afford 2-(4-bromo-1-methyl-imidazol-2-yl)ethanol intermediate **259** as a white solid.

¹H NMR (400 MHz, CDCl3) δ 6.78 (1H, s), 4.02 (2H, t, *J* = 5.6 Hz), 3.89 (1H, br. s), 3.57 (3H, s), 2.83 (2H, t, *J* = 5.8 Hz) ppm.

### Preparation of intermediate 260: 2-(4-bromo-1-methyl-imidazol-2-yl)ethyl-N-[(3S)-3-hydroxy butyl]carbamate

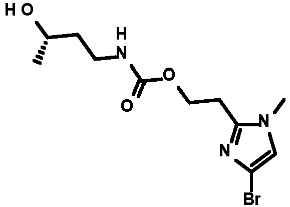

To a solution of 4-nitrophenyl chloroformate (137m g, 0.68 mmol) and pyridine (0.10 mL, 1.24 mmol) in dichloromethane (2.5 mL) at room temperature was added dropwise 2-(4-bromo-1-methyl-imidazol-2-yl)ethanol intermediate **259** (128 mg, 0.62 mmol) in dichloromethane (1.5 mL). The reaction mixture was stirred at room temperature for 1 h. A mixture of (2S)-4-aminobutan-2-ol (60 mg, 0.68 mmol) and DIPEA (0.21 mL, 1.24 mmol) in dichloromethane (1 mL) was added. The reaction mixture was stirred at room temperature for 2 h. The reaction mixture was diluted with 0.5 N aqueous sodium hydroxide solution and extracted with dichloromethane. The combined organic layers were washed with 0.5 N aqueous sodium hydroxide solution and dried over anhydrous magnesium sulfate, filtered and concentrated under reduced pressure. The residue was purified by silica gel column chromatography using dichloromethane/ ethyl acetate 100/0 to 30/70 as eluent to afford 2-(4-bromo-1-methyl-imidazol-2-yl)ethyl N-[(3S)-3-hydroxybutyl]carbamate intermediate 260 as a colorless oil.
LCMS method F: [M+H]⁺ = 320.1-322.1, t_{R} = 1.17 min

### Preparation of intermediate 261: 2-[4-[5-[tert-butyl(dimethyl)silyl]oxy-1-tetrahydropyran-2-yl-indazol-3-yl]-1-methyl-imidazol-2-yl]ethyl-N-[(3S)-3-hydroxybutyl] carbamate

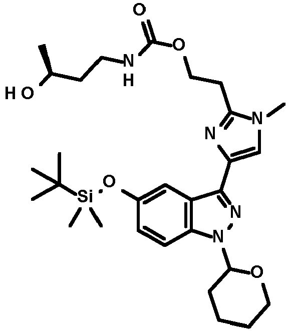

To a degassed solution of 2-(4-bromo-1-methyl-imidazol-2-yl)ethyl-N-[(3S)-3-hydroxybutyl]carbamate intermediate **260** (102 mg, 0.32 mmol), tert-butyl-dimethyl-[1-tetrahydropyran-2-yl-3-(4,4,5,5-tetramethyl-1,3,2-dioxaborolan-2-yl)indazol-5-yl]oxy-silane (175 mg, 0.38 mmol) and potassium phosphate tribasic (204 mg, 0.96 mmol) in 1,4-dioxane (2.7 mL) and water (0.1 mL), were added tetrakis(triphenylphosphine)palladium(0) (19 mg, 0.016 mmol) and 2-dicyclohexylphosphino-2',4',6'-triisopropylbiphenyl (15 mg, 0.032 mmol). The reaction mixture was heated at 125 °C for 1 h. The reaction mixture was filtered through a Celite pad and washed with ethyl acetate. The filtrate was diluted with water and extracted with ethyl acetate. The organic layer was washed with water, brine, dried over anhydrous magnesium sulfate, filtered and concentrated under reduced pressure. The residue was purified by silica gel column chromatography using dichloromethane/methanol 100/0 to 98/2 as eluent to afford 2-[4-[5-[tert-butyl(dimethyl)silyl]oxy-1-tetrahydropyran-2-yl-indazol-3-yl]-1-methyl-imidazol-2-yl]ethyl N-[(3S)-3-hydroxybutyl]carbamate intermediate **261** as a yellow oil.
LCMS method F: [M+H]⁺ = 572.4, t_{R} = 2.28 min

### Preparation of intermediate 262: [(1S)-3-[2-[4-[5-[tert-butyl(dimethyl)silyl]oxy-1-tetrahydro pyran-2-yl-indazol-3-yl]-1-methyl-imidazol-2-yl]ethoxycarbonylamino]-1-methyl-propyl] methanesulfonate

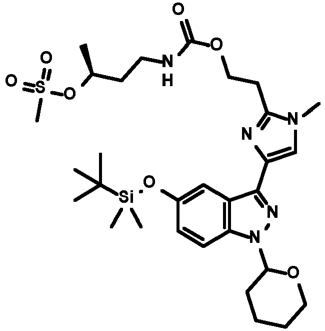

To a solution of 2-[4-[5-[*tert*-butyl(dimethyl)silyl]oxy-1-tetrahydropyran-2-yl-indazol-3-yl]-1-methyl-imidazol-2-yl]ethyl N-[(3S)-3-hydroxybutyl]carbamate intermediate **261** (112 mg, 0.20 mmol) and triethylamine (56 µL, 0.40 mmol) in dichloromethane (2.2 mL) at 0 °C was added dropwise methanesulfonyl chloride (20 µL, 0.26 mmol). The reaction mixture was stirred at room temperature for 2 h. The reaction mixture was diluted with water and extracted with dichloromethane. The combined organics were washed with brine, dried over anhydrous magnesium sulfate, filtered and concentrated under reduced pressure to afford [(1S)-3-[2-[4-[5-[tert-butyl(dimethyl)silyl]oxy-1-tetrahydropyran-2-yl-indazol-3-yl]-1-methyl-imidazol-2-yl] ethoxycarbonylamino]-1-methyl-propyl] methanesulfonate intermediate **262** as a colorless oil.
LCMS method F: [M+H]⁺ = 650.4, t_{R} = 2.53 min

### Preparation of intermediate 263: (13R)-4,13-dimethyl-19-(oxan-2-yl)-8,14-dioxa-4,10,19, 20,23-pentaazatetracyclo[13.5.2.1^{2,5}.0^{18,21}]tricosa-1(20),2,5(23),15(22),16,18(21)-hexaen-9-one

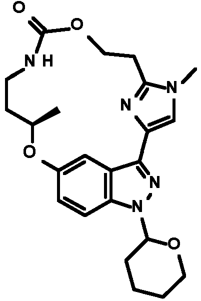

To a suspension of cesium carbonate (112 mg, 0.34 mmol) in anhydrous *N,N-*dimethylformamide (6 mL) at 60 °C was added dropwise [(1S)-3-[2-[4-[5-[tert-butyl(dimethyl)silyl]oxy-1-tetrahydropyran-2-yl-indazol-3-yl]-1-methyl-imidazol-2-yl]ethoxy carbonylamino]-1-methyl-propyl]methanesulfonate intermediate **262** (75 mg, 0.11 mmol) in *N,N*-dimethyl formamide (3 mL). The reaction mixture was stirred at 60 °C for 2 h. The reaction mixture was allowed to cool down to room temperature, then filtered over a Celite pad, washed with ethyl acetate. The filtrate was concentrated under reduced pressure. The residue was purified by silica gel column chromatography using cyclohexane/(ethyl acetate/ethanol (3/1)) 100/0 to 60/40 as eluent to afford (13R)-4,13-dimethyl-19-(oxan-2-yl)-8,14-dioxa-4,10,19,20,23-pentaazatetracyclo[13.5.2.1^{2,5}.0^{18,21}]tricosa-1(20),2,5(23),15(22),16,18(21)-hexaen-9-one intermediate **263** as a yellow oil.
LCMS method F: [M+H]⁺ = 440.3, t_{R} = 1.71 min

### Preparation of Example 161: (13R)-4,13-dimethyl-8,14-dioxa-4,10,19,20,23-pentaazatetra cyclo[13.5.2.1^{2,5}.0^{18,21}]tricosa-1(20),2,5(23),15(22),16,18(21)-hexaen-9-one

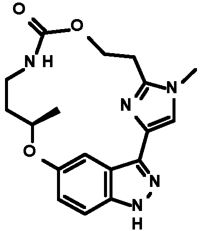

To a solution of (13R)-4,13-dimethyl-19-(oxan-2-yl)-8,14-dioxa-4,10,19,20,23-pentaazatetra cyclo[13.5.2.1^{2,5}.0^{18,21}]tricosa-1(20),2,5(23),15(22),16,18(21)-hexaen-9-one intermediate **263** (38 mg, 0.086 mmol) in methanol (6 mL) and water (0.8 mL) was added *p*-toluenesulfonic acid monohydrate (82 mg, 0.43 mmol). The reaction mixture was stirred at 65°C for 22 h. The reaction mixture was diluted with dichloromethane and saturated aqueous sodium bicarbonate solution. After separation, the aqueous layer was extracted with ethyl Acetate. The combined organic layer was washed with brine, dried over anhydrous sodium sulfate, filtered and evaporated under reduced pressure. The residue was purified by preparative TLC on silica gel using dichloromethane/methanol 90/10 as eluent to afford (13R)-4,13-dimethyl-8,14-dioxa-4,10,19,20,23-pentaazatetracyclo[13.5.2.1^{2,5}.0^{18,21}]tricosa-1(20),2,5(23),15(22),16,18(21)-hexaen-9-one **example 161** as a white solid.
LCMS method F: [M+H]⁺ = 356.3, t_{R} = 1.27 min
LCMS method G: [M+H]⁺ = 356.3, t_{R} = 1.76 min
¹H NMR (400 MHz, CDCl₃) δ 7.89-7.85 (1H, m), 7.34 (1H, s), 7.24 (1H, d, *J* = 9.6 Hz), 6.96 (1H, dd, *J* = 2.4, 9.6 Hz), 5.58 (1H, br. s), 4.77 - 4.67 (2H, m), 4.60-4.55 (1H, m), 3.67-3.62 (4H, m), 3.45-3.32 (2H, m), 3.04-2.94 (2H, m), 2.18-2.09 (1H, m), 1.64-1.50 (1H, m), 1.46-1.39 (3H, m) ppm.

### Example 162: (13R)-13-methyl-8,14-dioxa-10,16,19,20-tetraazatetracyclo[13.5.2. 1^{2,6}.0^{18,21}]tricosa-1(20),2(23),3,5,15(22),16,18(21)-heptaen-9-one

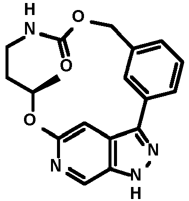

Example 162 is prepared according to the synthesis route described in detail below.

### Preparation of intermediate 264: 2-[(3R)-3-[(4-methyl-5-nitro-2-pyridyl)oxy[butyl] isoindoline-1,3-dione

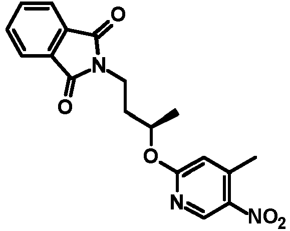

A pressure vial containing a mixture of cesium carbonate (26.41 g, 83.56 mmol), Pd₂(dba)₃ (1.64 g, 1.791 mmol), rac-BINAP (2.23 g, 3.581 mmol), (R)-2-(3-hydroxybutyl)isoindoline-1,3-dione (18.32 g, 83.56 mmol) and 2-chloro-4-methyl-5-nitropyridine (10.3 g, 59.686 mmol) were suspended in toluene (78 mL) under nitrogen atmosphere. The reaction mixture was stirred at 110°C for 16 h. The reaction mixture was diluted with ethyl acetate and filtered over a pad of celite. The filtrate was concentrated under reduced pressure. The residue was purified by silica gel column chromatography using heptane/ethl acetate 100/0 to 90/10 as eluent to afford 2-[(3R)-3-[(4-methyl-5-nitro-2-pyridyl)oxy]butyl]isoindoline-1,3-dione intermediate **264** as a white solid.
LCMS method B: [M+H]⁺ = 356.0, t_{R} = 1.020 min

### Preparation of intermediate 265: 2-[(3R)-3-[(5-amino-4-methyl-2-pyridyl)oxy]butyl] isoindoline-1,3-dione

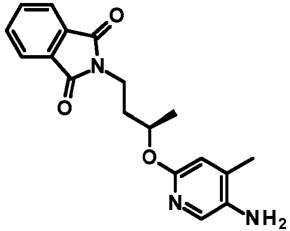

To a solution of 2-[(3R)-3-[(4-methyl-5-nitro-2-pyridyl)oxy]butyl]isoindoline-1,3-dione intermediate **264** (14.6 g, 41.086 mmol) in ethyl acetate (240 mL) under nitrogen atmosphere wxas added palladium 10%wt on carbon (2.9 g). The reaction mixture was stirred under hydrogen atmosphere at RT for 20 h. The suspension was filtered over a pad of celite and washed with ethyl acetate. The solvent was removed under reduced pressure. The residue was purified by silica gel column chromatography using heptane/ethyl acetate 100/0 to 40/60 as eluent to afford 2-[(3R)-3-[(5-amino-4-methyl-2-pyridyl)oxy]butyl]isoindoline-1,3-dione intermediate **265** as a orange foam.
LCMS method E: [M+H]⁺ = 3526.1, t_{R} = 2.092 min

### Preparation of intermediate 266: N-[6-[(1R)-3-(1,3-dioxoisoindolin-2-yl)-1-methyl-propoxy]-4-methyl-3-pyridyl]acetamide

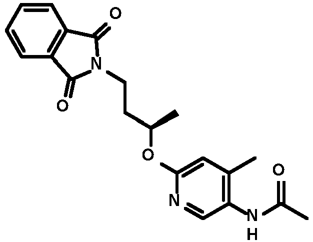

To solution of 2-[(3R)-3-[(5-amino-4-methyl-2-pyridyl)oxy]butyl]isoindoline-1,3-dione intermediate **265** (5.0 g, 15.36 mmol) and triethylamine (4.26 mL, 30.37 mmol) in dichloromethane (75 mL) at 0°C was added acetic anhydride (2.18 mL, 23.05 mmol). The reaction mixture was stirred at 0°C for 5 min then warmed to room temperature and stirred for 1 h. The reaction mixture was filtered and rinsed with dichloromethane. The filtrate was evaporated under reduced pressure and the residue was purified by silica gel column chromatography using cyclohexane/ethyl acetate 100/0 to 50/50 as eluent to afford N-[6-[(1R)-3-(1,3-dioxoisoindolin-2-yl)-1-methyl-propoxy]-4-methyl-3-pyridyl]acetamide intermediate **266** as a white solid.
LCMS method F: [M+H]⁺ = 368.2, t_{R} = 2.13 min

### Preparation of intermediate 267: 2-[(3R)-3-(1-acetylpyrazolo[3,4-c]pyridin-5-yl)oxybutyl] isoindoline-1,3-dione

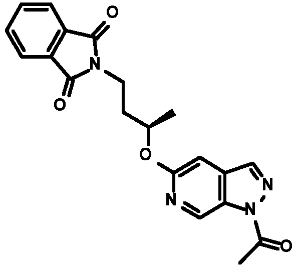

A solution of N-[6-[(1R)-3-(1,3-dioxoisoindolin-2-yl)-1-methyl-propoxy]-4-methyl-3-pyridyl]acetamide intermediate **266** (5.0 g, 13.60 mmol), acetic anhydride (5.79 mL, 61.24 mmol) and potassium acetate (2.39 g, 20.41 mmol) in toluene (70 mL) was stirred at 80°C. Isoamyl nitrite (7.33 mL, 54.437 mmol) was added and the resulting mixture was stirred at 80°C for 16 h. The reaction mixture was cooled to room temperature and filtered. The filtrate was concentrated under reduced pressure. The residue was purified by silica gel column chromatography using cyclohexane/ethyl acetate 100/0 to 2/1 as eluent to afford 2-[(3R)-3-(1-acetylpyrazolo[3,4-c]pyridin-5-yl)oxybutyl]isoindoline-1,3-dione intermediate **267** as a yellow solid.
LCMS method F: [M+H]⁺ = 379.3, t_{R} = 2.63 min

### Preparation of intermediate 268: [(3R)-3-(1H-pyrazolo[3,4-c]pyridin-5-yloxy)butyl] isoindoline-1,3-dione

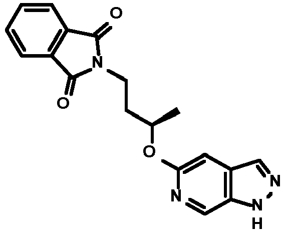

To a solution of 2-[(3R)-3-(1-acetylpyrazolo[3,4-c]pyridin-5-yl)oxybutyl]isoindoline-1,3-dione intermediate **267** (2.75 g, 7.28 mmol) in methanol (36 mL) was added 7N ammonia solution in methanol (5.20 mL, 36.42 mmol). The reaction mixture was stirred at room temperature for 2 h. Solvents were concentrated under reduced pressure and the residue was purified by silica gel column chromatography using cyclohexane/ethyl acetate 100/0 to 50/50 as eluent to afford 2-[(3R)-3-(1H-pyrazolo[3,4-c]pyridin-5-yloxy)butyl]isoindoline-1,3-dione intermediate **268** as a pale orange solid.
LCMS method F: [M+H]⁺ = 337.3, t_{R} = 2.09 min

### Preparation of intermediate 269: 2-[(3R)-3-[(3-iodo-1H-pyrazolo[3,4-c]pyridin-5-yl)oxy]butyl]isoindoline-1,3-dione

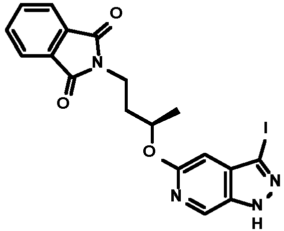

To a solution of 2-[(3R)-3-(1H-pyrazolo[3,4-c]pyridin-5-yloxy)butyl]isoindoline-1,3-dione intermediate **268** (673 mg, 2 mmol) in DMF (20 mL) was added N-iodo-succinimide (675 mg, 3 mmol). The reaction mixture was stirred at room temperature for 16 h. The reaction mixture was partitioned between ethyl acetate and water. The aqueous layer was extracted with ethyl acetate and the combined organic extracts were washed with saturated aqueous sodium chloride solution, dried over anhydrous sodium sulfate, filtered and concentrated under reduced pressure. The residue was purified by silica gel column chromatography using cyclohexane/ethyl acetate 100/0 to 50/50 as eluent to afford 2-[(3R)-3-[(3-iodo-1H-pyrazolo[3,4-c]pyridin-5-yl)oxy]butyl]isoindoline-1,3-dione intermediate **269** as a yellow solid.
LCMS method F: [M+H]⁺ = 463.2, t_{R} = 2.54 min

### Preparation of intermediate 270: 2-[(3R)-3-(3-iodo-1-tetrahydropyran-2-yl-pyrazolo[3,4-c]pyridin-5-yl)oxybutyl] isoindoline-1,3-dione

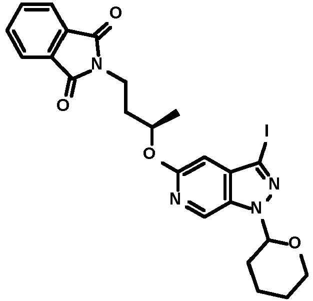

To a solution of 2-[(3R)-3-[(3-iodo-1H-pyrazolo[3,4-c]pyridin-5-yl)oxy]butyl]isoindoline-1,3-dione intermediate **269** (897 mg, 1.94 mmol) and DHP (0.53 mL, 5.82 mmol) in dichloromethane (15 mL) and THF (5 mL) was added methanesulfonic acid (25 µL, 0.38 mmol). The reaction mixture was stirred at room temperature for 3 h. Saturated aqueous sodium bicarbonate solution was added. After separation, the aqueous layer was extracted with dichloromethane and the combined organic extracts were washed with saturated aqueous sodium chloride solution, dried over anhydrous sodium sulfate, filtered and concentrated under reduced pressure. The residue was purified by silica gel column chromatography using cyclohexane/ethyl acetate 100/0 to 35/65 as eluent to afford 2-[(3R)-3-(3-iodo-1-tetrahydropyran-2-yl-pyrazolo[3,4-c]pyridin-5-yl)oxybutyl]isoindoline-1,3-dione intermediate **270** as a yellow solid.
LCMS method F: [M+H]⁺ = 547.2, t_{R} = 3.17 min

### Preparation of intermediate 271: (3R)-3-(3-iodo-1-tetrahydropyran-2-yl-pyrazolo[3,4-c]pyridin-5-yl)oxybutan-1-amine

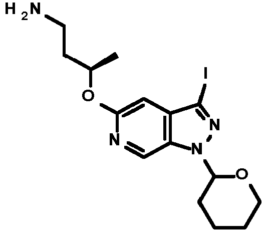

To a solution of 2-[(3R)-3-(3-iodo-1-tetrahydropyran-2-yl-pyrazolo[3,4-c]pyridin-5-yl)oxybutyl] isoindoline-1,3-dione intermediate **270** (800 mg, 1.464 mmol) in ethanol (15.0 mL) at 0°C was added hydrazine monohydrate (0.22 mL, 4.392 mmol). The reaction mixture was stirred at 0°C for 5 minutes and at room temperature for 16 h. THF (60 mL) was added and the mixture was stirred for 5 minutes, filtered and the solvent was removed under reduced pressure. The residue was diluted with THF (15 mL), filtered and the filtrate was concentrated under reduced pressure to afford crude (3R)-3-(3-iodo-1-tetrahydropyran-2-yl-pyrazolo[3,4-c]pyridin-5-yl)oxybutan-1-amine intermediate **271** as an orange solid. The product was used in the next step without further purification.
LCMS method F: [M+H]⁺ = 417.2, t_{R} = 1.63 min

### Preparation of intermediate 272: benzyl N-[(3R)-3-(3-iodo-1-tetrahydropyran-2-yl-pyrazolo [3,4-c]pyridin-5-yl)oxybutyl] carbamate

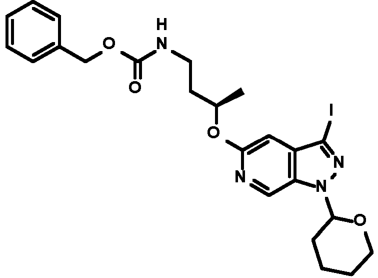

To a solution of (3R)-3-(3-iodo-1-tetrahydropyran-2-yl-pyrazolo[3,4-c]pyridin-5-yl)oxybutan-1-amine intermediate **271** (650 mg, 1.464 mmol) in dichloromethane (15 mL) at 0°C were successively added triethylamine (0.24 mL, 1.75 mmol) and benzyl chloroformate (0.23 mL, 1.610 mmol). The reaction mixture was stirred at 0°C for 5 min then warmed to room temperature and stirred for 16 h. The reaction mixture was diluted with water. After separation, the aqueous layer was extracted with dichloromethane and the combined organic extracts were washed with saturated aqueous sodium chloride, dried over anhydrous sodium sulfate, filtered and concentrated under reduced pressure. The residue was purified by silica gel column chromatography using cyclohexane/ethyl acetate 100:0 to 50:50 as eluent to afford benzyl N-[(3R)-3-(3-iodo-1-tetrahydropyran-2-yl-pyrazolo[3,4-c]pyridin-5-yl)oxybutyl]carbamate intermediate **272** as a transparent oil.
LCMS method F: [M+H]⁺ = 551.1, t_{R} = 3.17 min

### Preparation of intermediate 273: benzyl N-[(3R)-3-[3-[3-(hydroxymethyl)phenyl]-1-tetra hydropyran-2-yl-pyrazolo[3,4-c]pyridin-5-yl]oxybutyl]carbamate

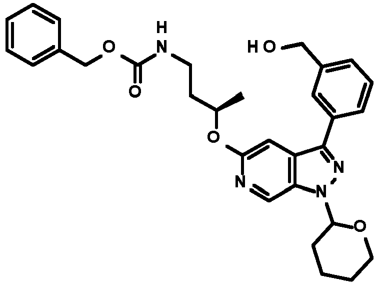

To a with argon degassed solution of benzyl N-[(3R)-3-(3-iodo-1-tetrahydropyran-2-yl-pyrazolo[3,4-c]pyridin-5-yl)oxybutyl]carbamate intermediate **272** (320 mg, 0.581 mmol), 3-(hydroxy methyl)phenylboronic acid (106 mg, 0.698 mmol), potassium phosphate tribasic (370 mg, 1.744 mmol) and XPhos (28 mg, 0.058 mmol) in 1,4-dioxane (5.5 mL) and water (0.5 mL) was added tetrakis(triphenylphosphine)palladium(0) (34 mg, 0.029 mmol). The reaction mixture was heated at 100°C for 1 h. The reaction mixture was cooled and diluted with ethyl acetate and water. After separation, the aqueous layer was extracted with ethyl acetate and the combined organic extracts were washed with saturated aqueous sodium chloride solution, dried over anhydrous sodium sulfate, filtered and concentrated under reduced pressure. The residue was purified by column chromatography using cyclohexane/ethyl acetate 100/0 to 1/1 as eluent to afford benzyl N-[(3R)-3-[3-[3-(hydroxymethyl)phenyl]-1-tetrahydropyran-2-yl-pyrazolo [3,4-c]pyridin-5-yl]oxybutyl]carbamate intermediate **273** as a pale yellow oil.
LCMS method F: [M+H]⁺ = 531.3, t_{R} = 2.90 min

### Preparation of intermediate 274: (13R)-13-methyl-19-(oxan-2-yl)-8,14-dioxa-10,16,19,20-tetraazatetracyclo[13.5.2.1^{2,6}.0^{18,21}]tricosa-1(20),2(23),3,5,15(22),16,18(21)-heptaen-9-one

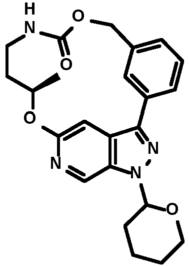

To a solution of of benzyl N-[(3R)-3-[3-[3-(hydroxymethyl)phenyl]-1-tetrahydropyran-2-yl-pyrazolo[3,4-c]pyridin-5-yl]oxybutyl]carbamate intermediate **273** (180 mg, 0.339 mmol) in acetonitrile (170 mL) was added potassium hydroxide (95 mg, 1.69 mmol). The reaction mixture was heated at 50°C for 16 h. The reaction mixture was cooled to room temperature, filtered and concentrated under reduced pressure. The residue was purified by silica gel column chromatography using cyclohexane/ethyl acetate 100/0 to 50/50 as eluent to afford (13R)-13-methyl-19-(oxan-2-yl)-8,14-dioxa-10,16,19,20-tetraazatetracyclo[13.5.2.1^{2,6}.0^{18,21}]tricosa-1(20),2(23),3,5,15(22), 16,18(21)-heptaen-9-one intermediate **274** as a yellow solid.
LCMS method F: [M+H]⁺ = 423.4, t_{R} = 2.49 min

### Preparation of Example 162: (13R)-13-methyl-8,14-dioxa-10,16,19,20-tetraazatetracyclo [13.5.2.1^{2,6}.0^{18,21}]tricosa-1(20),2(23),3,5,15(22),16,18(21)-heptaen-9-one

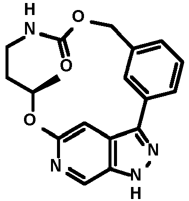

To a solution of (13R)-13-methyl-19-(oxan-2-yl)-8,14-dioxa-10,16,19,20-tetraazatetracyclo [13.5.2.1^{2,6}.0^{18,21}]tricosa-1(20),2(23),3,5,15(22),16,18(21)-heptaen-9-one intermediate **274** (84 mg, 0.199 mmol) in methanol (1.8 mL) and water (0.2 mL) was added p-toluenesulfonic acid monohydrate (189 mg, 0.994 mmol). The reaction mixture was stirred at 65°C for 80 h. Solvents were evaporated under reduced pressure and the residue was partitioned between ethyl acetate and water. The aqueous layer was extracted with ethyl acetate and the combined organic extracts were washed with saturated aqueous sodium bicarbonate solution then with saturated aqueous sodium chloride solution, dried over anhydrous sodium sulfate, filtered and concentrated under reduced pressure. The reaction mixture was triturated with diisopropyl ether, filtered and dried to afford (13R)-13-methyl-8,14-dioxa-10,16,19,20-tetraazatetracyclo [13.5.2.1^{2,6}.0^{18,21}]tricosa-1(20),2(23),3,5,15(22),16,18(21)-heptaen-9-one **example 162** as a white solid.
LCMS method F: [M+H]⁺ = 339.3, t_{R} = 2.45 min
LCMS method G: [M+H]⁺ = 339.3, t_{R} = 2.48 min
¹H NMR (400 MHz, d6-DMSO) δ 13.64 (s, 1H), 8.76 (s, 1H), 7.97 (dd, *J* = 4.8, 6.8 Hz, 1H), 7.88 (d, *J* = 7.6 Hz, 1H), 7.84 (s, 1H), 7.49 (t, *J* = 7.2 Hz, 1H), 7.31 (d, *J* = 8.4 Hz, 1H), 7.10 (s, 1H), 5.76 (d, *J* = 12.8 Hz, 1H), 4.84 (d, *J* = 15.2 Hz, 1H), 4.62 - 4.54 (m, 1H), 3.61 - 3.58 (m, 1H), 2.91 (t, *J* = 14.0 Hz, 1H), 2.36 (t, *J* = 12.4 Hz, 1H), 1.48 - 1.46 (m, 1H), 1.44 (d, *J* = 5.6 Hz, 3H) ppm.

### Example 163: 14-methyl-8-oxa-10,14,19,20-tetraazatetracyclo[13.5.2.1^{2,6}.0^{18,21}]tricosa-1(20),2(23),3,5,15,17,21-heptaen-9-one

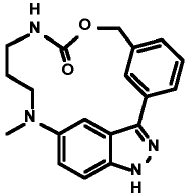

Example 163 is prepared according to the synthesis route described in detail below.

### Preparation of intermediate 275: N-(3-iodo-1-tetrahydropyran-2-yl-indazol-5-yl)-2-nitro-benzenesulfonamide

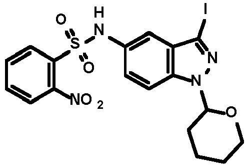

To a mixture of 3-iodo-1-(oxan-2-yl)-1H-indazol-5-amine (2.0 g, 5.83 mmol) in acetonitrile (100 mL) was added pyridine (943 µL, 11.66mmol) and 2-nitrobenzenesulfonyl chloride (1.29 g, 5.83mmol). The reaction mixture was stirred at room temperature for 16 h. The solvent was removed under reduced pressure and the residue was portioned between ethyl acetate and water. After separation, the aqueous phase was extracted with ethyl acetate. The organic phases were dried over anhydrous sodium sulfate, filtered and evaporated under reduced pressure. The residue was purified by silica gel column chromatography using cyclohexane/ethyl acetate 100/0 to 70/30 as eluent to afford N-(3-iodo-1-tetrahydropyran-2-yl-indazol-5-yl)-2-nitrobenzenesulfonamide intermediate **275** as a pink solid.
LCMS method F: [M+H]⁺ = 529.1, t_{R} = 2.83 min

### Preparation of intermediate 276: Benzyl N-[3-[(3-iodo-1-tetrahydropyran-2-yl-indazol-5-yl)-(2-nitrophenyl)sulfonyl-amino]propyl]carbamate

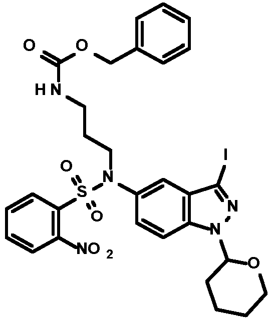

To a mixture of N-(3-iodo-1-tetrahydropyran-2-yl-indazol-5-yl)-2-nitro-benzenesulfonamide intermediate **275** (800 mg, 1.51 mmol) in acetonitrile (200 mL) was added potassium carbonate (229 mg, 1.66 mmol) and benzyl N-(3-bromopropyl)carbamate (410 mg, 1.51 mmol). The reaction mixture was stirred at 90°C for 16 h. Additional potassium carbonate (229 mg, 1.66 mmol) and benzyl N-(3-bromopropyl)carbamate (410 mg, 1.51 mmol) were added. The reaction mixture was stirred at 90°C for 16 h. The solvent was removed under reduced pressure and the reaction mixture was dissolved with ethyl acetate and water. After separation, the aqueous layer was extracted with ethyl acetate. The organic layers were dried over anhydrous sodium sulfate, filtered and evaporated under reduced pressure. The residue was purified by silica gel column chromatography using cyclohexane/ethyl acetate 100/0 to 50/50 as eluent to afford benzyl N-[3-[(3-iodo-1-tetrahydropyran-2-yl-indazol-5-yl)-(2-nitrophenyl)sulfonyl-amino]propyl]carbamate intermediate **276** as a yellow oil.
LCMS method F: [M+H]⁺ = 720.2, t_{R} = 3.09 min

### Preparation of intermediate 277: benzyl N-[3-[(3-iodo-1-tetrahydropyran-2-yl-indazol-5-yl)amino]propyl]carbamate

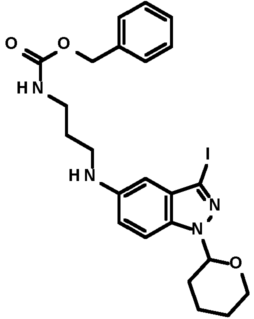

To a suspension of benzyl N-[3-[(3-iodo-1-tetrahydropyran-2-yl-indazol-5-yl)-(2-nitrophenyl)sulfonyl-amino]propyl]carbamate intermediate **276** (810 mg, 1.15 mmol) and cesium carbonate (749 mg, 2.30 mmol) in *N,N*-dimethylformamide (40 mL) was added 4-methylbenzenethiol (171 mg, 1.38 mmol). The reaction mixture was stirred at room temperature for 16 h. The solvent was evaporated under reduced pressure. The reaction mixture was diluted with ethyl acetate and washed with water and brine. The organic layer was dried over anhydrous sodium sulfate, filtered and the solvent was removed under reduced pressure. The residue was purified by silica gel column chromatography using cyclohexane/ethyl acetate 100/0 to 50/50 as eluent to afford benzyl N-[3-[(3-iodo-1-tetrahydropyran-2-yl-indazol-5-yl)amino]propyl]carbamate intermediate **277** as a white solid.
LCMS method F: [M+H]⁺ = 535.1, t_{R} = 2.75 min

### Preparation of intermediate 278: benzyl N-[3-[(3-iodo-1-tetrahydropyran-2-yl-indazol-5-yl)-methyl-amino]propyl]carbamate

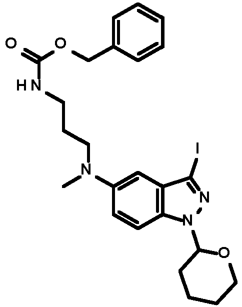

To a solution of benzyl N-[3-[(3-iodo-1-tetrahydropyran-2-yl-indazol-5-yl)amino]propyl] carbamate intermediate **277** (360 mg, 0.673 mmol) in acetonitrile (54 mL) was added formaldehyde (37% solution) (18 mL) and sodium triacetoxyborohydride (570 mg, 2.69 mmol). The reaction mixture was stirred at room temperature for 16 h. The solvent was evaporated under reduced pressure. The residue was diluted with ethyl acetate and washed with water. The organic layer was dried over anhydrous sodium sulfate, filtered and evaporated under reduced pressure. The residue was purified by silica gel column chromatography using cyclohexane/ethyl acetate 100/0 to 50/50 to afford benzyl N-[3-[(3-iodo-1-tetrahydropyran-2-yl-indazol-5-yl)-methyl-amino]propyl] carbamate intermediate **278** as a brown oil.
LCMS method F: [M+H]⁺ = 549.2, t_{R} = 2.73 min

### Preparation of intermediate 279: benzyl N-[3-[[3-[3-(hydroxymethyl)phenyl]-1-tetrahydro pyran-2-yl-indazol-5-yl]-methyl-amino]propyl]carbamate

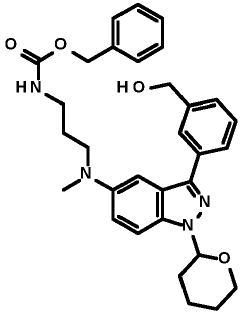

To a suspension of benzyl N-[3-[(3-iodo-1-tetrahydropyran-2-yl-indazol-5-yl)-methyl-amino]propyl]carbamate intermediate **278** (150 mg, 0.273 mmol) in 1,4-dioxane (2.2 mL) and water (0.11 mL) was added [3-(hydroxymethyl)phenyl]boronic acid (50 mg, 0.327 mmol) and potassium phosphate tribasic (173mg, 0.819mmol). The reaction was purged with argon for 10 min and tetrakis(triphenylphosphine)palladium(0) (15 mg, 0.0136 mmol) and XPhos (13 mg, 0.0273 mmol) were added. The resulting mixture was stirred at 100°C for 16 h. The reaction mixture was cooled to room temperature and evaporated under reduced pressure. The residue was diluted with ethyl acetate and water. After separation, the aqueous layer was extracted with ethyl acetate. The organic layers were washed with brine, dried over anhydrous sodium sulfate, filtered and evaporated under reduced pressure. The residue was purified on silica gel column chromatography using cyclohexane/ethyl acetate 100/0 to 40/60 as eluent to afford benzyl N-[3-[[3-[3-(hydroxymethyl)phenyl]-1-tetrahydropyran-2-yl-indazol-5-yl]-methyl-amino] propyl]carbamate intermediate **279** as a yellow oil.
LCMS method F: [M+H]⁺ = 529.4, t_{R} = 2.23 min

### Preparation of intermediate 280: 14-methyl-19-(oxan-2-yl)-8-oxa-10,14,19,20-tetraaza tetracyclo[13.5.2.1^{2,6}.0^{18,21}]tricosa-1(20),2(23),3,5,15,17,21-heptaen-9-one

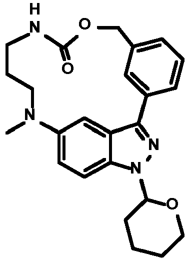

To a solution of benzyl N-[3-[[3-[3-(hydroxymethyl)phenyl]-1-tetrahydropyran-2-yl-indazol-5-yl]-methyl-amino]propyl]carbamate intermediate **279** (110 mg, 0.208 mmol) in dry acetonitrile (30 mL) was added cesium carbonate (406 mg, 1.24 mmol). The reaction mixture was stirred at 90°C for 16 h. The suspension was filtered and washed with acetonitrile. The filtrate was evaporated under reduced pressure. The residue was purified on silica gel column chromatography using cyclohexane/ethyl acetate 100/0 to 50/50 as eluent to afford 14-methyl-19-(oxan-2-yl)-8-oxa-10,14,19,20-tetraazatetracyclo[13.5.2.1^{2,6}.0^{18,21}]tricosa-1(20),2(23),3,5,15,17,21-heptaen-9-one intermediate **280** as a yellow solid.
LCMS method F: [M+H]⁺ = 421.3, t_{R} = 2.59 min

### Preparation of Example 163: 14-methyl-8-oxa-10,14,19,20-tetraazatetracyclo[13.5.2. 1^{2,6}.0^{18,21}]tricosa-1(20),2(23),3,5,15,17,21-heptaen-9-one

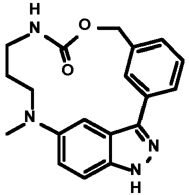

To a solution of 14-methyl-19-(oxan-2-yl)-8-oxa-10,14,19,20-tetraazatetracyclo [13.5.2. 1^{2,6}.0^{18,21}]tricosa-l(20),2(23),3,5,15,17,21-heptaen-9-one intermediate **280** (40 mg, 0.095 mmol) in methanol (1.8 mL) and water (0.26 mL) was added *p*-toluenesulfonic acid monohydrate (90 mg, 0.475 mmol). The reaction mixture was stirred at 65°C for 16 h. The reaction mixture was cooled to room temperature and quenched with a saturated aqueous sodium bicarbonate solution. Ethyl acetate was added. After separation, the aqueous layer was extracted with ethyl acetate and the organic layers were washed with brine, dried over anhydrous sodium sulfate, filtered and evaporated under reduced pressure. The residue was purified on silica gel column chromatography using dichloromethane/methanol 100/0 to 95/5 as eluent to afford 14-methyl-8-oxa-10,14,19,20-tetraazatetracyclo[13.5.2.1^{2,6}.0^{18,21}]tricosa-1(20),2(23),3,5,15,17,21-heptaen-9-one **example 163** as a yellow solid.
LCMS method F: [M+H]⁺ = 337.1, t_{R} = 1.70 min
LCMS method G: [M+H]⁺ = 337.3, t_{R} = 2.12 min
¹H NMR (400 MHz, CDCl₃) δ 9.97 (1H, bs), 8.03 (1H, s), 7.98 - 7.94 (1H, m), 7.48 - 7.36 (2H, m), 7.23 - 7.20 (2H, m), 7.01 (1H, dd, J=2.3, 9.1 Hz), 5.45 - 5.26 (3H, m), 3.49-3.27 (4H, m), 3.06 - 3.05 (3H, s), 2.05 - 1.97 (2H, m) ppm.

### Example 164: (13R)-13-methyl-8,14-dioxa-4,10,19,20,22-pentaazatetracyclo[13.5.2. 1^{2,6}.0^{18,21}]tricosa-1(20),2(23),3,5,15,17,21-heptaen-9-one

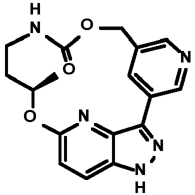

Example 164 is prepared according to the synthesis route described in detail below.

### Preparation of intermediate 281: 5-chloro-3-iodo-1H-pyrazolo[4,3-b]pyridine

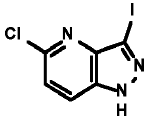

To a solution of 5-chloro-1H-pyrazolo[4,3-b]pyridine (1.0 g, 6.51 mmol) in acetonitrile (10 mL) was added *N*-iodosuccinimide (1.75 g, 7.81 mmol). The reaction mixture was heated under microwave irradiations at 100°C for 25 min. The solvent was partially evaporated under reduced pressure and the residue was diluted with water and ethyl acetate. After separation, the organic layer was washed with a saturated sodium thiosulfate solution. The aqueous layer was extracted with ethyl acetate and the combined organic layers were dried over anhydrous sodium sulfate, filtered and evaporated under reduced pressure. The residue was purified on silica gel column chromatography using cyclohexane/ethyl acetate 100/0 to 60/40 as eluent to afford 5-chloro-3-iodo-1H-pyrazolo[4,3-b]pyridine intermediate **281** as a yellow solid.
LCMS method F: [M+H]⁺ = 279.9, t_{R} = 2.00 min

### Preparation of intermediate 282: 5-chloro-3-iodo-1-tetrahydropyran-2-yl-pyrazolo[4,3-b] pyridine

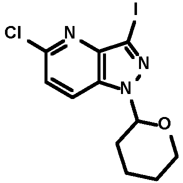

To a solution of 5-chloro-3-iodo-1H-pyrazolo[4,3-b]pyridine intermediate **281** (7.00 g, 25.05 mmol) in dichloromethane (45 mL) were added *p*-toluenesulfonic acid monohydrate (2.38 g, 12.53 mmol) and then 3,4-dihydro-2H-pyran (4.11 mL, 45.09 mmol). The reaction mixture was stirred at room temperature for 1 h. The reaction mixture was diluted with a saturated aqueous sodium bicarbonate solution and more dichloromethane was added. After separation, the aqueous layer was extracted with dichloromethane and the combined organic layers were dried over anhydrous sodium sulfate, filtered and evaporated under reduced pressure. The residue was purified on silica gel column chromatography using cyclohexane/ethyl acetate 100/0 to 75/25 as eluent to afford 5-chloro-3-iodo-1-tetrahydropyran-2-yl-pyrazolo[4,3-b]pyridine intermediate **282** as a pale yellow solid.
LCMS method F: [M+H]⁺ = 364.0, t_{R} = 2.75 min

### Preparation of intermediate 283: 3-iodo-5-methoxy-1-tetrahydropyran-2-yl-pyrazolo[4,3-b]pyridine

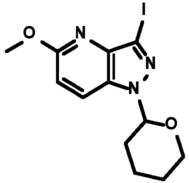

To a solution of 5-chloro-3-iodo-1-tetrahydropyran-2-yl-pyrazolo[4,3-b]pyridine intermediate **282** (4.10 g, 11.28 mmol) in dry N,N-dimethylformamide (160 mL) was added sodium hydride (60 % dispersion in mineral oil) (2.25 g, 56.38 mmol) and methanol (2.28 mL, 56.38 mmol). The reaction mixture was stirred at 50°C for 2 h. The reaction mixture was cooled to room temperature and carefully quenched with water. The solvent was partially evaporated reduced pressure and water and ethyl acetate were added. After separation, the aqueous layer was extracted with ethyl acetate and the combined organic layers were dried over anhydrous sodium sulfate, filtered and evaporated under reduced pressure. The residue was purified on silica gel column chromatography using cyclohexane/ethyl acetate 100/0 to 85/15 as eluent to afford 3-iodo-5-methoxy-1-tetrahydropyran-2-yl-pyrazolo[4,3-b]pyridine intermediate **283** as a pale yellow oil.
LCMS method F: [M+H]⁺ = 360.0, t_{R} = 2.82 min

### Preparation of intermediate 284: 3-iodo-1-tetrahydropyran-2-yl-pyrazolo[4,3-b]pyridin-5-ol

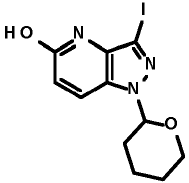

To a solution of 3-iodo-5-methoxy-1-tetrahydropyran-2-yl-pyrazolo[4,3-b]pyridine intermediate **283** (1.73 g, 4.84 mmol) in acetonitrile (18 mL) and 1,2-dichloroethane (18 mL) were added sodium iodide (2.17 g, 14.52 mmol) and then trimethylchlorosilane (1.22 mL, 9.67 mmol). The reaction mixture was stirred at 40°C for 4 h. The reaction mixture was diluted with a saturated aqueous sodium thiosulfate solution and ethyl acetate. After separation, the aqueous layer was extracted with ethyl acetate and the combined organic layers were washed with brine, dried over anhydrous sodium sulfate, filtered and evaporated under reduced pressure. The residue was purified on silica gel column chromatography using cyclohexane/(ethyl acetate/ethanol (3/1)) 100/0 to 30/70 as eluent. The resulting solid was triturated in acetonitrile, filtered and dried to afford 3-iodo-1-tetrahydropyran-2-yl-pyrazolo[4,3-b]pyridin-5-ol intermediate **284** as a white solid.
LCMS method F: [M+H]⁺ = 346.0, t_{R} = 1.85 min

### Preparation of intermediate 285: benzyl N-[(3R)-3-(3-iodo-1-tetrahydropyran-2-yl-pyrazolo [4,3-b]pyridin-5-yl)oxybutyl]carbamate

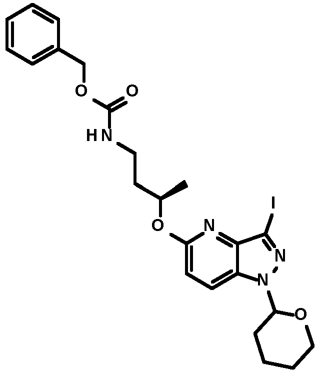

To a solution of [(1S)-3-(benzyloxycarbonylamino)-1-methyl-propyl]methanesulfonate intermediate **199** (783 mg, 2.6 mmol) in dry acetonitrile (20 mL) was added cesium carbonate (1.3 g, 4.0 mmol) and 3-iodo-1-tetrahydropyran-2-yl-pyrazolo[4,3-b]pyridin-5-ol intermediate **284** (702 mg, 2.0 mmol). The reaction mixture was stirred at reflux for 16 h. The reaction mixture was evaporated under reduced pressure and partitioned with water and ethyl acetate. After separation, the aqueous layer was extracted with ethyl acetate and the combined organic layers were washed with brine, dried over anhydrous sodium sulfate, filtered and evaporated under reduced pressure. The residue was purified by silica gel column chromatography using cyclohexane/ethyl acetate 100/0 to 40/60 as eluent to afford benzyl N-[(3R)-3-(3-iodo-1-tetrahydropyran-2-yl-pyrazolo[4,3-b]pyridin-5-yl)oxybutyl]carbamate intermediate **285** as a white solid.
LCMS method F: [M+H]⁺ = 551.2, t_{R} = 3.17 min

### Preparation of intermediate 286: Benzyl N-[(3R)-3-[3-[5-(hydroxymethyl)-3-pyridyl]-1-tetrahydropyran-2-yl-pyrazolo[4,3-b]pyridin-5-ylloxybutyl]carbamate

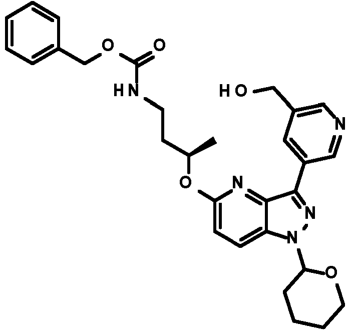

To a suspension of benzyl N-[(3R)-3-(3-iodo-1-tetrahydropyran-2-yl-pyrazolo[4,3-b]pyridin-5-yl) oxybutyl]carbamate intermediate **285** (420 mg, 0.76 mmol) in 1,4-dioxane (4.0 mL) and water (0.2 mL) was added (5-(4,4,5,5-tetramethyl-1,3,2-dioxaborolan-2-yl)pyridin-3-yl)methanol (214 mg, 0.91 mmol) and potassium phosphate tribasic (484 mg, 2.28 mmol). The reaction was purged with argon for 10 min then Xphos (36 mg, 0.08 mmol) and tetrakis(triphenylphosphine)palladium(0) (44 mg, 0.04 mmol) were added. The reaction mixture was stirred at reflux for 4 h. The reaction mixture was cooled to room temperature and diluted with water and ethyl acetate. After separation, the aqueous layer was extracted with ethyl acetate and the combined organic layers were washed with brine, dried over anhydrous sodium sulfate, filtered and evaporated under reduced pressure. The residue was purified on silica gel column chromatography using cyclohexane/(ethyl acetate/ethanol (3/1)) 90/10 to 50/50 as eluent to afford benzyl N-[(3R)-3-[3-[5-(hydroxymethyl)-3-pyridyl]-1-tetrahydropyran-2-yl-pyrazolo[4,3-b]pyridin-5-yl]oxybutyl]carbamate intermediate **286** as a white foam.
LCMS method F: [M+H]⁺ = 532.3, t_{R} = 2.23 min

### Preparation of intermediate 287: (13R)-13-methyl-19-(oxan-2-yl)-8,14-dioxa-4,10,19,20,22-pentaazatetracyclo[13.5.2.1^{2,6}.0^{18,21}]tricosa-1(20),2(23),3,5,15,17,21-heptaen-9-one

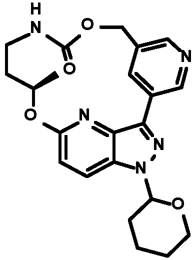

To a solution of benzyl N-[(3R)-3-[3-[5-(hydroxymethyl)-3-pyridyl]-1-tetrahydropyran-2-yl-pyrazolo [4,3-b]pyridin-5-yl]oxybutyl]carbamate intermediate **286** (250 mg, 0.47 mmol) in dry acetonitrile (80 mL) was added cesium carbonate (919 mg, 2.82 mmol). The reaction mixture was stirred at 90°C for 2 h. The suspension was filtered and the salts were washed with acetonitrile. The filtrate was evaporated under reduced pressure. The residue was purified on silica gel column chromatography using dichloromethane/methanol 100/0 to 95/5 as eluent to afford (13R)-13-methyl-19-(oxan-2-yl)-8,14-dioxa-4,10,19,20,22-pentaazatetracyclo[13.5.2. 1^{2,6}.0^{18,21}]tricosa-1(20),2(23),3,5,15,17,21-heptaen-9-one intermediate 287 as a colorless oil.
LCMS method F: [M+H]⁺ = 424.3, t_{R} = 2.17 min

### Preparation of Example 164: (13R)-13-methyl-8,14-dioxa-4,10,19,20,22-pentaaza tetracyclo[13.5.2.1^{2,6}.0^{18,21}]tricosa-1(20),2(23),3,5,15,17,21-heptaen-9-one

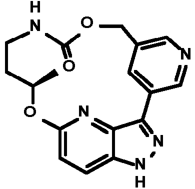

To a solution of (13R)-13-methyl-19-(oxan-2-yl)-8,14-dioxa-4,10,19,20,22-pentaazatetracyclo[13.5.2.1^{2,6}.0^{18,21}]tricosa-1(20),2(23),3,5,15,17,21-heptaen-9-one intermediate **287** (200 mg, 0.47 mmol) in methanol (11 mL) and water (1.6 mL) was added*p-*toluenesulfonic acid monohydrate (446 mg, 2.35 mmol). The reaction mixture was stirred at 65°C for 2 h then cooled to room temperature and carefully quenched with a saturated aqueous sodium bicarbonate solution. Ethyl acetate was added. After separation, the aqueous layer was extracted with ethyl acetate and the combined organic layers were washed with brine, dried over anhydrous sodium sulfate, filtered and evaporated under reduced pressure. The residue was purified on silica gel column chromatography using cyclohexane/(ethyl acetate-ethanol (3-1)) 80/20 to 20/80 as eluent. The resulting solid was triturated in diethyl ether, filtered then dissolved in dichloromethane/methanol and evaporated under reduced pressure to afford (13R)-13-methyl-8,14-dioxa-4,10,19,20,22-pentaazatetracyclo[13.5.2.1^{2,6}.0^{18,21}]tricosa-1(20),2(23), 3,5,15,17,21-heptaen-9-one **example 164** as a white solid.
LCMS method F: [M+H]⁺ = 340.3, t_{R} = 1.63 min
LCMS method G: [M+H]⁺ = 340.3, t_{R} = 1.97 min
¹H NMR (400 MHz, d6-DMSO) δ 13.45 (1H, s), 9.19 - 9.18 (1H, m), 8.88 (1H, s), 8.46 - 8.44 (1H, m), 8.02 - 7.98 (1H, m), 7.87 - 7.82 (1H, m), 6.86 - 6.83 (1H, m), 5.57 - 5.46 (2H, m), 5.12 - 5.08 (1H, m), 3.46 - 3.40 (1H, m), 3.00 - 2.92 (1H, m), 2.45 (1H, d, J=14.3 Hz), 1.41 - 1.38 (4H, m) ppm.

### Example 165: (13R)-13-methyl-8,14-dioxa-10,17,19,20-tetraazatetracyclo[13.5.2. 1^{2,6}.0^{18,21}]tricosa-1(20),2(23),3,5,15(22),16,18(21)-heptaen-9-one

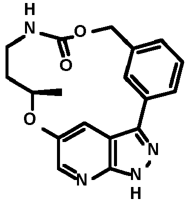

Example 165 is prepared according to the synthesis route described in detail below.

### Preparation of intermediate 288: 5-bromo-1-tetrahydropyran-2-yl-pyrazolo[3,4-b]pyridine

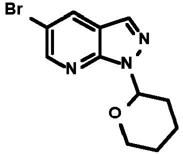

To a solution of 5-bromo-1H-pyrazolo[3,4-b]pyridine (3.96 g, 20.0 mmol) and 3,4-dihydropyran (5.49 mL, 60.0 mmol) in dichloromethane (45 mL) and THF (15 mL) was added methanesulfonic acid (0.26 mL, 4.0 mmol). The reaction mixture was stirred at room temperature for 3 h. Saturated aqueous sodium bicarbonate solution was added. After separation, the aqueous layer was extracted with dichloromethane and the combined organic extracts were washed with brine, dried over anhydrous sodium sulfate, filtered and concentrated under reduced pressure. The residue was purified by silica gel column chromatography using cyclohexane/ethyl acetate 100/0 to 70/30 as eluent to afford 5-bromo-1-tetrahydropyran-2-yl-pyrazolo[3,4-b]pyridine intermediate **288** as a pale yellow oil.
LCMS method F: [M+H]⁺ = 282.0-284.0, t_{R} = 2.45 min

### Preparation of intermediate 289: 1-tetrahydropyran-2-yl-5-(4,4,5,5-tetramethyl-1,3,2-dioxaborolan-2-yl)pyrazolo[3,4-b]pyridine

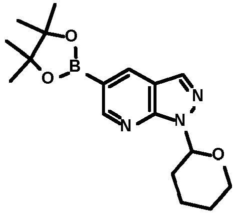

To an argon degassed mixture of 5-bromo-1-tetrahydropyran-2-yl-pyrazolo[3,4-b]pyridine intermediate **288** (1.25 g, 4.43 mmol), bis(pinacolato)diboron (2.81 g, 11.07 mmol), potassium acetate (1.30 g, 13.29 mmol) in DMF (20 mL) was added [1,1'-Bis(diphenylphosphino)ferrocene] dichloropalladium(II), complex with dichloromethane (91 mg, 0.111 mmol). The reaction mixture heated to 100°C for 2 h. The solvent was concentrated under reduced pressure and the residue was partitioned between ethyl acetate and water. The aqueous layer was extracted with ethyl acetate and the combined organic extracts were washed with brine, dried over anhydrous sodium sulfate, filtered and concentrated under reduced pressure to afford 1-tetrahydropyran-2-yl-5-(4,4,5,5-tetramethyl-1,3,2-dioxaborolan-2-yl)pyrazolo [3,4-b]pyridine intermediate **289** as a brown oil which was used in the next step without further purification.
LCMS method F: [M+H]⁺ = 330.3, t_{R} = 2.73 min

### Preparation of intermediate 290: 1-tetrahydropyran-2-ylpyrazolo[3,4-b]pyridin-5-ol

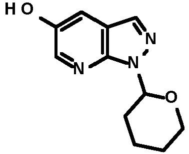

To a solution of 1-tetrahydropyran-2-yl-5-(4,4,5,5-tetramethyl-1,3,2-dioxaborolan-2-yl) pyrazolo[3,4-b]pyridine intermediate **289** (6.0 g, 17.72 mmol) in THF (45 mL) and 1 N aqueous sodium hydroxide solution (45 mL) was added hydrogen peroxide (30%wt aqueous solution, 2.0 mL, 53.166 mmol). The reaction mixture was stirred at room temperature for 2 h. The reaction mixture was quenched with saturated aqueous sodium thiosulfate solution and diluted with ethyl acetate. After separation, the aqueous layer was extracted with ethyl acetate and the combined organic extracts were washed with brine, dried over anhydrous sodium sulfate, filtered and concentrated under reduced pressure to afford 1-tetrahydropyran-2-ylpyrazolo[3,4-b]pyridin-5-ol intermediate **290** as a brown oil.
LCMS method F: [M+H]⁺ = 220.3, t_{R} = 1.58 min

### Preparation of intermediate 291: tert-butyl-dimethyl-(1H-pyrazolo[3,4-b]pyridin-5-yloxy) silane

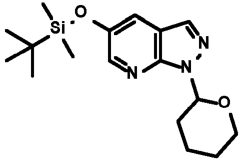

To a solution of 1-tetrahydropyran-2-ylpyrazolo[3,4-b]pyridin-5-ol intermediate **290** (2.37 g, 17.59 mmol) and imidazole (1.43 g, 21.11 mmol) in DMF (18 mL) at 0°C was added *tert*-butyldimethylsilyl chloride (2.91 g, 19.35 mmol). The reaction mixture was stirred at 0°C for 5 min then warmed to room temperature and stirred for 16 h. Solvents were evaporated under reduced pressure and the residue was partitioned between ethyl acetate and water. The aqueous layer was extracted with ethyl acetate and the combined organic extracts were washed with brine, dried over anhydrous sodium sulfate, filtered and concentrated under reduced pressure to afford *tert*-butyl-dimethyl-(1H-pyrazolo[3,4-b]pyridin-5-yloxy)silane intermediate **291** as a brown oil which was used in the next step without further purification.
LCMS method F: [M+H]⁺ = 334.2, t_{R} = 3.29 min

### Preparation of intermediate 292: 1H-pyrazolo[3,4-b]pyridin-5-ol

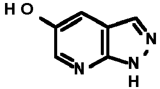

To a solution of *tert*-butyl-dimethyl-(1-tetrahydropyran-2-ylpyrazolo[3,4-b]pyridin-5-yl)oxy-silane intermediate **291** (4.69 g, 14.50 mmol) in methanol (60 mL) and water (12 mL) was added toluenesulfonic acid monohydrate (5.52 g, 29.01 mmol). The solution was stirred at room temperature for 2 h. Solvents were evaporated and the residue was partitioned between ethyl acetate and water. The aqueous layer was washed with ethyl acetate, collected and evaporated under reduced pressure. The residue was purified by reverse-phase column chromatography using acetonitrile/water 10/90 to 50/50 as eluent. The requisite fraction were concentrated under reduced pressure and triturated with diisopropyl ether to afford 1H-pyrazolo[3,4-b]pyridin-5-ol intermediate **292** as a beige solid.
LCMS method F: [M+H]⁺ = 136.1, t_{R} = 1.36 min

### Preparation of intermediate 293: tert-butyl-dimethyl-(1H-pyrazolo[3,4-b]pyridin-5-yloxy) silane

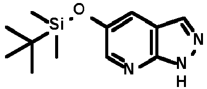

To a solution of crude 1H-pyrazolo[3,4-b]pyridin-5-ol intermediate **292** (500 mg, 3.70 mmol) in DMF (16 mL) were successively added imidazole (504 mg, 7.40 mmol) and *tert*-butyldimethylsilyl chloride (669 mg, 4.44 mmol). The reaction mixture was stirred at room temperature for 16 h. Saturated aqueous ammonium chloride and ethyl acetate were added. After separation, the aqueous layer was extracted with ethyl acetate and the combined organic extracts were washed with brine, dried over anhydrous sodium sulfate, filtered and concentrated under reduced pressure to afford tert-butyl-dimethyl-(1H-pyrazolo[3,4-b]pyridin-5-yloxy)silane intermediate **293** as a yellow oil which was used in the next step without further purification.
LCMS method F: [M+H]⁺ = 250.3, t_{R} = 2.73 min

### Preparation of intermediate 294: tert-butyl-[(3-iodo-1H-pyrazolo[3,4-b]pyridin-5-yl)oxy]-dimethyl-silane

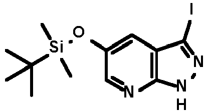

To a solution of tert-butyl-dimethyl-(1H-pyrazolo[3,4-b]pyridin-5-yloxy)silane intermediate **293** (3.00 g, 12.03 mmol) in DMF (60 mL) was added *N*-Iodosuccinimide (3.24 g, 14.43 mmol).

The reaction mixture was stirred at room temperature for 16 h. Additional *N*-Iodosuccinimide (1.12 g, 5.0 mmol) was added and the reaction mixture was stirred for 5 h. Solvents were concentrated under reduced pressure and the residue was partitioned between ethyl acetate and saturated aqueous sodium thiosulfate solution. The aqueous layer was extracted with ethyl acetate and the combined organic extracts were washed with saturated aqueous sodium bicarbonate solution then with brine, dried over anhydrous anhydrous sodium sulfate, filtered and concentrated under reduced pressure to afford *tert*-butyl-[(3-iodo-1H-pyrazolo[3,4-b]pyridin-5-yl)oxy]-dimethyl-silane intermediate **294** as a yellow solid which was used in the next step without further purification.
LCMS method F: [M+H]⁺ = 376.2, t_{R} = 3.20 min

### Preparation of intermediate 295: tert-butyl-(3-iodo-1-tetrahydropyran-2-yl-pyrazolo[3,4-b] pyridin-5-yl)oxy-dimethyl-silane

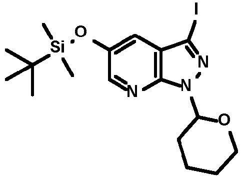

To a solution of *tert*-butyl-[(3-iodo-1H-pyrazolo[3,4-b]pyridin-5-yl)oxy]-dimethyl-silane intermediate **294** (1.16 g, 3.10 mmol) and DHP (0.85 mL, 9.303 mmol) in dichloromethane (12 mL) and THF (4 mL) was added methanesulfonic acid (40 µL, 0.62 mmol). The reaction mixture was stirred at room temperature for 16 h. Saturated aqueous sodium bicarbonate solution was added. After separation, the aqueous layer was extracted with dichloromethane and the combined organic extracts were washed with brine, dried over anhydrous sodium sulfate, filtered and concentrated under reduced pressure. The residue was purified by silica gel column chromatography using cyclohexane/ethyl acetate 100/0 to 70/30 as eluent to afford *tert-*butyl-(3-iodo-1-tetrahydropyran-2-yl-pyrazolo[3,4-b]pyridin-5-yl)oxy-dimethyl-silane intermediate **295** as a pale yellow oil.
LCMS method F: [M+H]⁺ = 460.1, t_{R} = 3.67 min

### Preparation of intermediate 296: 3-iodo-1-tetrahydropyran-2-yl-pyrazolo[3,4-b]pyridin-5-ol

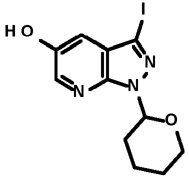

To a 0°C solution of *tert*-butyl-(3-iodo-1-tetrahydropyran-2-yl-pyrazolo[3,4-b]pyridin-5-yl)oxy-dimethyl-silane intermediate **295** (630 mg, 1.371 mmol) in THF (15 mL) was added 1M TBAF solution in THF (1.64 mL, 1.64 mmol) dropwise over 5 min. The reaction mixture was stirred at 0°C for 5 min then warmed to room temperature and stirred for 16 h. Solvents were evaporated under reduced pressure and the residue was partitioned between ethyl acetate and saturated aqueous ammonium chloride solution. The aqueous layer was extracted with ethyl acetate and the combined organic extracts were washed with brine, dried over anhydrous sodium sulfate, filtered and concentrated under reduced pressure to afford 3-iodo-1-tetrahydropyran-2-yl-pyrazolo[3,4-b]pyridin-5-ol intermediate **296** as a yellow solid which was used in the next step without further purification.
LCMS method F: [M+H]⁺ = 346.1, t_{R} = 2.17 min

### Preparation of intermediate 297: benzyl N-[(3R)-3-(3-iodo-1-tetrahydropyran-2-yl-pyrazolo [3,4-b]pyridin-5-yl)oxybutyl]carbamate

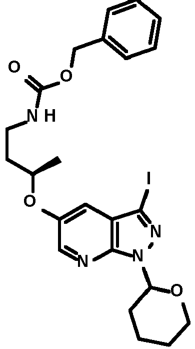

To a solution of 3-iodo-1-tetrahydropyran-2-yl-pyrazolo[3,4-b]pyridin-5-ol intermediate **296** (270 mg, 0.781 mmol) in DMF (4 mL) was added cesium carbonate (382 mg, 1.17 mmol). The reaction mixture was stirred at room temperature for 30 min, then a solution of [(1S)-3-(benzyloxycarbonylamino)-1-methyl-propyl] methanesulfonate intermediate **199** (259 mg, 0.859 mmol) in DMF (4 mL) was added dropwise over 2 min. The reaction mixture was stirred at room temperature for 72 h. The reaction mixture was filtered. Ethyl acetate and water were added. After separation, the aqueous layer was extracted with ethyl acetate and the combined organic extracts were washed with brine, dried over anhydrous sodium sulfate, filtered and concentrated under reduced pressure to afford benzyl N-[(3R)-3-(3-iodo-1-tetrahydropyran-2-yl-pyrazolo[3,4-b]pyridin-5-yl)oxybutyl]carbamate intermediate **297** as a yellow oil which was used in the next step without further purification.
LCMS method F: [M+H]⁺ = 551.2, t_{R} = 3.06 min

### Preparation of intermediate 298: benzyl N-[(3R)-3-[3-[3-(hydroxymethyl)phenyl]-1-tetra hydropyran-2-yl-pyrazolo[3,4-b]pyridin-5-yl]oxybutyl]carbamate

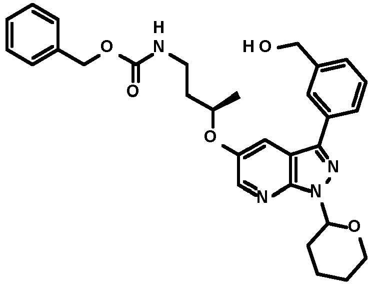

To an argon degassed solution of benzyl N-[(3R)-3-(3-iodo-1-tetrahydropyran-2-yl-pyrazolo[3,4-b]pyridin-5-yl)oxybutyl]carbamate intermediate **297** (260 mg, 0.472 mmol), [3-(hydroxy methyl)phenyl]boronic acid (79 mg, 0.52 mmol), potassium phosphate tribasic (301 mg, 1.41 mmol) and XPhos (22 mg, 0.047 mmol) in dioxane (4.75 mL) and water (0.25 mL) was added tetrakis(triphenylphosphine)-palladium(0) (28 mg, 0.024 mmol). The reaction mixture was stirred at 90°C for 1h. The reaction mixture was cooled to room temperature and ethyl acetate and water were added. After separation, the aqueous layer was extracted with ethyl acetate and the combined organic extracts were washed with brine, dried over anhydrous sodium sulfate, filtered and concentrated under reduced pressure. The residue was purified by silica gel column chromatography using cyclohexane/ethyl acetate 100/0 to 60/40 as eluent to afford benzyl N-[(3R)-3-[3-[3-(hydroxymethyl)phenyl]-1-tetrahydropyran-2-yl-pyrazolo[3,4-b]pyridin-5-yl]oxybutyl] carbamate intermediate **298** as a pale yellow solid.
LCMS method F: [M+H]⁺ = 531.4, t_{R} = 2.82 min

### Preparation of intermediate 299: (13R)-13methyl-19-(oxan-2-yl)-8,14-dioxa-10,17,19,20-tetraazatetracyclo[13.5.2.1^{2,6}.0^{18,21}]tricosa-1(20),2(23),3,5,15(22),16,18(21)-heptaen-9-one

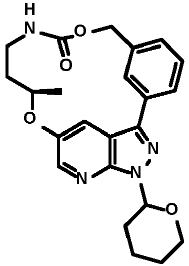

To a solution of benzyl N-[(3R)-3-[3-[3-(hydroxymethyl)phenyl]-1-tetrahydropyran-2-yl-pyrazolo[3,4-b]pyridin-5-yl]oxybutyl]carbamate intermediate **298** (300 mg, 0.565 mmol) in acetonitrile (300 mL) was added potassium hydroxide (159 mg, 2.82 mmol). The reaction mixture was stirred at room temperature for 2 h. The reaction mixture was filtered and solvents were concentrated under reduced pressure. The residue was partitioned between ethyl acetate and water. after separation, the aqueous layer was extracted with ethyl acetate and the combined organic extracts were washed with brine, dried over anhydrous sodium sulfate, filtered and concentrated under reduced pressure. The residue was purified by silica gel column chromatography using dichloromethane/methanol 100/0 to 95/5 as eluent to afford (13R)-13-methyl-19-(oxan-2-yl)-8,14-dioxa-10,17,19,20-tetraazatetracyclo[13.5.2.1^{2,6}.0^{18,21}]tricosa-1(20),2(23),3,5,15(22),16,18(21)-heptaen-9-one intermediate **299** as a pale yellow foam.
LCMS method F: [M+H]⁺ = 423.2, t_{R} = 2.73 min

### Preparation of Example 165: (13R)-13-methyl-8,14-dioxa-10,17,19,20-tetraazatetracyclo [13.5.2.1^{2,6}.0^{18,21}]tricosa-1(20),2(23),3,5,15(22),16,18(2t)-heptaen-9-one

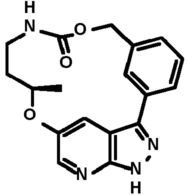

To a solution of (13R)-13-methyl-19-(oxan-2-yl)-8,14-dioxa-10,17,19,20-tetraazatetracyclo [13.5.2.1^{2,6}.0^{18,21}]tricosa-1(20),2(23),3,5,15(22),16,18(21)-heptaen-9-one intermediate **299** (160 mg, 0.378 mmol) in methanol (3 mL) and water (1 mL) was added p-toluenesulfonic acid monohydrate (360 mg, 1.89 mmol). The reaction mixture at 50°C for 16 h. Solvents were concentrated under reduced pressure and the residue was partitioned between ethyl acetate and saturated aqueous sodium bicarbonate solution. After separation, the aqueous layer was extracted with ethyl acetate and the combined organic extracts were washed with brine, dried over anhydrous sodium sulfate, filtered and concentrated under reduced pressure. The residue was purified by silica gel column chromatography using dichloromethane/methanol 100/0 to 90/10 as eluent. The requisite fractions were concentrated under reduced pressure and triturated in pentane/diethyl ether (10/1) solution to afford (13R)-13-methyl-8,14-dioxa-10,17,19,20-tetraazatetracyclo[13.5.2.1^{2,6}.0^{18,21}]tricosa-1(20),2(23),3,5,15(22),16,18(21)-heptaen-9-one **example 165** as a white solid.
LCMS method F: [M+H]⁺ = 339.3, t_{R} = 2.76 min
LCMS method G: [M+H]⁺ = 339.3, t_{R} = 2.75 min
¹H NMR (400 MHz, d6-DMSO) δ 13.73 (s, 1H), 8.27 (d, *J* = 2.9 Hz, 1H), 7.97 (dd, *J* = 4.9, 7.2 Hz, 1H), 7.87 (d, *J=* 7.9 Hz, 1H), 7.81 (s, 1H), 7.69 (d, *J =* 2.7 Hz, 1H), 7.50 (t, *J =* 7.7 Hz, 1H), 7.31 (d, *J=* 8.0 Hz, 1H), 5.79 - 5.74 (m, 1H), 4.83 (d, *J=* 13.5 Hz, 1H), 4.58 (dd, *J* = 5.9*,* 10.5 Hz, 1H), 3.60 - 3.56 (m, 1H), 2.91 (t, *J =* 13.6 Hz, 1H), 2.33 (t, *J =* 12.7 Hz, 1H), 1.45 (d, *J =* 6.2 Hz, 3H), 1.42 - 1.39 (m, 1H) ppm.

### Example 166: 8,14-dioxa-4,5,10,19,20,23-hexaazatetracyclo[13.5.2.1^{2,5}.0^{18,21}]tricosa-1(20),2(23),3,15(22),16,18(21)-hexaen-9-one

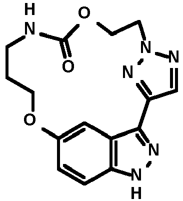

Example 166 is prepared according to the synthesis route described in general scheme K.

### Preparation of intermediate 300: benzyl N-[3-[3-[2-(2-hydroxyethyl)triazol-4-yl]-1-tetrahydropyran-2-yl-indazol-5-yl]oxypropyl]carbamate

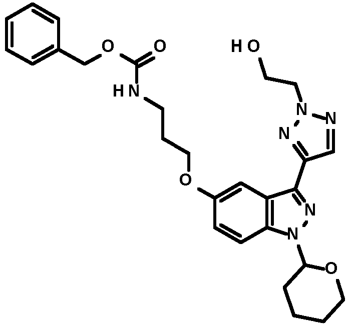

To a stirred mixture of 2-[4-[5-[tert-butyl(dimethyl)silyl]oxy-1-tetrahydropyran-2-yl-indazol-3-yl]triazol-2-yl]ethanol intermediate **248** (443 mg, 1 mmol), and cesium carbonate (977 mg, 3.24 mmol) in acetonitrile (20 mL) was added a solution of benzyl N-(3-bromopropyl)carbamate (272 mg, 1.1 mmol) in acetonitrile (2 mL). The reaction mixture was stirred at 50°C for 16 hours. The salts were removed by filtration, rinced with acetonitrile and the filtrate was concentrated under reduced pressure. The residue was purified by silica gel column chromatography using cyclohexane/ethyl acetate 50/50 to 20/80 as eluent to afford benzyl N-[3-[3-[2-(2-hydroxyethyl)triazol-4-yl]-1-tetrahydropyran-2-yl-indazol-5-yl]oxy propyl]carbamate intermediate **300** as a white solid.
LCMS method F: [M+H]⁺ = 521.3, t_{R} = 2.64 min

### Preparation of intermediate 301: 19-(oxan-2-yl)-8,14-dioxa-4,5,10,19,20,23-hexaazatetra cyclo[13.5.2.1^{2,5}.0^{18,21}]tricosa-1(20),2(23),3,15(22),16,18(21)-hexaen-9-one

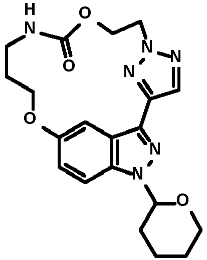

To a solution of benzyl N-[3-[3-[2-(2-hydroxyethyl)triazol-4-yl]-1-tetrahydropyran-2-yl-indazol-5-yl]oxypropyl]carbamate intermediate **300** (230 mg, 0.44 mmol, 1eq) in acetonitrile (50 mL) was added cesium carbonate (860 mg, 2.65 mmol). The suspension was stirred at reflux for 48 h. The suspension was filtered and the salts were rinced with acetonitrile. The filtrate was evaporated under reduced pressure and the residue was purified by silica gel column chromatography using cyclohexane/ethyl acetate 50/50 to 30/70 to afford 19-(oxan-2-yl)-8,14-dioxa-4,5,10,19,20,23-hexaazatetracyclo[13.5.2.1^{2,5}.0^{18,21}]tricosa-1(20),2(23),3,15(22),16, 18(21)-hexaen-9-one intermediate **301** as a white solid.
LCMS method F: [M+H]⁺ = 413.2, t_{R} = 2.33 min

### Preparation of Example 166: 8,14-dioxa-4,5,10,19,20,23-hexaazatetracyclo[13.5.2. 1^{2,5}.0^{18,21}]tricosa-1(20),2(23),3,15(22),16,18(21)-hexaen-9-one

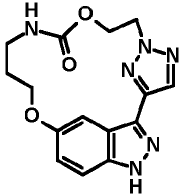

To a suspension of 19-(oxan-2-yl)-8,14-dioxa-4,5,10,19,20,23-hexaazatetracyclo [13.5.2.1^{2,5}.0^{18,21}]tricosa-1(20),2(23),3,15(22),16,18(21)-hexaen-9-one intermediate **301** (50 mg, 0.12 mmol) in methanol (7 mL) and water (1 mL) was added para-toluenesulfonic acid monohydrate (115 mg, 0.6 mmol). The reaction mixture was heated at 65°C for 6 h. Methanol was partially removed under reduced pressure and a saturated aqueous NaHCO₃ was added until pH basic. The aqueous phase was extracted with ethyl acetate. The organic layer was washed with brine, dried over anhydrous sodium sulfate, filtered and evaporated under reduced pressure. The residue was purified by silica gel column chromatography using dichloromethane/methanol 100/0 to 95/5 as eluent. The solid was triturated with diethyl ether, filtered and dried to give 8,14-dioxa-4,5,10,19,20,23-hexaazatetracyclo[13.5.2.1^{2,5}.0^{18,21}] tricosa-1(20),2(23),3,15(22),16,18(21)-hexaen-9-one **example 166** as a white solid.
LCMS method F: [M+H]⁺ = 329.2, t_{R} = 1.72 min
LCMS method G: [M+H]⁺ = 329.2, t_{R} = 1.75 min
¹H NMR (400 MHz, *d*6-DMSO) δ 13.15 (1H, s), 8.13 (1H, s), 7.69 (1H, t, J=6.1 Hz), 7.53 - 7.45 (2H, m), 6.98 (1H, dd, J=2.3, 8.9 Hz), 4.84 - 4.79 (2H, m), 4.53 (2H, dd, J=2.9, 5.2 Hz), 4.31 - 4.24 (2H, m), 3.09 - 3.06 (2H, m), 1.91 - 1.85 (2H, m) ppm.

### Example 167: 12,12-difluoro-8,14-dioxa-4,5,10,19,20,23-hexaazatetracyclo[13.5.2. 1^{2,5}.0^{18,21}]tricosa-1(20),2(23),3,15(22),16,18(21)-hexaen-9-one

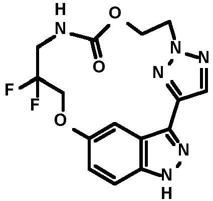

Example 167 is prepared according to the synthesis route described in general scheme K.

### Preparation of intermediate 302: [3-(benzyloxycarbonylamino)-2,2-difluoro-propyl] trifluoromethanesulfonate

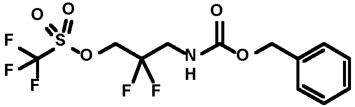

To a solution of benzyl N-(2,2-difluoro-3-hydroxy-propyl)carbamate intermediate **213** (2.63 g, 10.725 mmol) and triethylamine (2.97 mL, 21.45 mmol) in dichloromethane (100 mL) at 0°C under argon atmosphere was added dropwise over a period of 5 min Tf₂O (2.65 mL, 16.088 mmol). The reaction mixture was stirred at 0°C for 5 min and at room temperature for 16 h. A saturated aqueous NaHCO₃ solution was added and layers were separated. The aqueous layer was extracted with dichloromethane and the combined organic layers were washed with brine, dried over anhydrous sodium sulfate, filtered and the solvent was removed under reduced pressure to afford crude [3-(benzyloxycarbonylamino)-2,2-difluoro-propyl] trifluoromethane sulfonate intermediate **302** as a brown oil. The product was used in the next step without further purification.
¹H NMR (400 MHz, d6 DMSO) *δ* 7.38 - 7.32 (m, 5H), 5.14 - 5.06 (m, 3H), 3.64 (dt, *J =* 6.5, 14.5 Hz, 2H), 3.14 - 3.07 (m, 1H). [*1 labile proton was not visible in this solvent]*

### Preparation of intermediate 303: benzyl N-[2,2-difluoro-3-[3-[2-(2-hydroxyethyl)triazol-4-yl]-1-tetrahydropyran-2-yl-indazol-5-ylloxy-propyl]carbamate

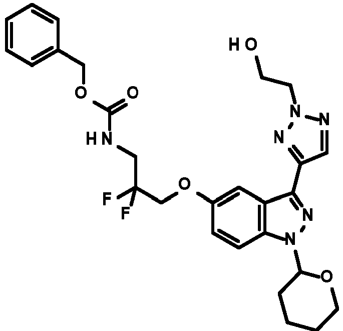

To a mixture of 3-[2-(2-hydroxyethyl)triazol-4-yl]-1-tetrahydropyran-2-yl-indazol-5-ol (210 mg, 0.638 mmol) and [[3-(benzyloxycarbonylamino)-2,2-difluoro-propyl] trifluoromethane sulfonate intermediate **302** (361 mg, 0.957 mmol ) in dry acetonitrile (20 mL) under argon atmosphere was added cesium carbonate (249 mg, 0.766 mmol) . The reaction mixture was stirred at 70°C for 16 h. Additional [[3-(benzyloxycarbonylamino)-2,2-difluoro-propyl]trifluoro methanesulfonate intermediate **302** (361 mg, 0.957 mmol ) was added. The reaction mixture was stirred 4 h at 70°C. Additional [[3-(benzyloxycarbonylamino)-2,2-difluoro-propyl] trifluoro methanesulfonate itermediate **302** (361 mg, 0.957 mmol ) was added. The reaction mixture was stirred overnight at 70°C. The solvent was removed under reduced pressure, ethyl acetate and water were added, the layers were separated and the aqueous layer was extracted with ethyl acetate. The combined organic layers were dried over anhydrous sodium sulfate, filtered and evaporated under reduced pressure. The residue was purified by silica gel column chromatographychromatography using cyclohexane/ethyl acetate 100/0 to 50/50 to afford benzyl N-[2,2-difluoro-3-[3-[2-(2-hydroxyethyl)triazol-4-yl]-1-tetrahydro pyran-2-yl-indazol-5-yl]oxy-propyl]carbamate intermediate **303** as a yellow oil.
LCMS method F: [M+H]⁺ = 557.4, t_{R} = 2.66 min

### Preparation of intermediate 304: 12,12-difluoro-19-(oxan-2-yl)-8,14-dioxa-4,5,10,19,20,23-hexaazatetracyclo[13.5.2.1^{2,5}.0^{18,21}]tricosa-1(20),2(23),3,15(22),16,18(21)-hexaen-9-one

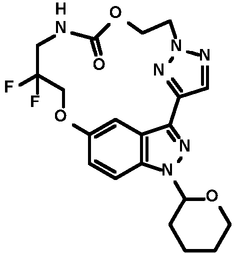

A stirred solution of benzyl N-[2,2-difluoro-3-[3-[2-(2-hydroxyethyl)triazol-4-yl]-1-tetrahydro pyran-2-yl-indazol-5-yl]oxy-propyl]carbamate intermediate **303** (240 mg, 0.431 mmol) and cesium carbonate (844 mg, 2.59 mmol) in acetonitrile (42 mL) was heated at reflux temperature for 24 h. The reaction mixture was filtered and the salts were rinced with acetonitrile. The filtrate was then evaporated under reduced pressure. The residue was purified by silica gel column chromatography using cyclohexane/ethyl acetate 70/30/ to 50/50 as eluent to afford 12,12-difluoro-19-(oxan-2-yl)-8,14-dioxa-4,5,10,19,20,23-exaazatetracyclo[13.5.2.1^{2,5}.0^{18,21}] tricosa-1(20), 2(23),3,15(22),16,18(21)-hexaen-9-one interlediate **304** as a cream solid.
LCMS method F: [M+H]⁺ = 449.1, t_{R} = 2.45 min

### Preparation of Example 167; 12,12-difluoro-8,14-dioxa-4,5,10,19,20,23-hexaazatetra cyclo[13.5.2.1^{2,5}.0^{18,21}]tricosa-1(20),2(23),3,15(22),16,18(21)-hexaen-9-one

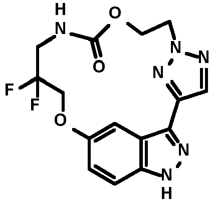

To a suspension of 12,12-difluoro-19-(oxan-2-yl)-8,14-dioxa-4,5,10,19,20,23-hexaazatetra cyclo[13.5.2.1^{2,5}.0^{18,21}]tricosa-1(20),2(23),3,15(22),16,18(21)-hexaen-9-one intermediate **304** (85 mg, 0.19 mmol in methanol (12 mL) and water (1.7 mL) was added para-toluenesulfonic acid monohydrate (180 mg, 0.948 mmol). The reaction mixture was heated at 65°C for 16 h. Methanol was partially removed under reduced pressure and a saturated aqueous NaHCO₃ solution of was added until pH basic. The aqueous phase was extracted with EtOAc and the organic extract was washed with brine, dried over anhydrous sodium sulfate, filtered and evaporated under reduced pressure. The residue was triturated in dichloromethane and filtered. The solid obtained was recrystallized from acetonitrile to give 12,12-difluoro-8,14-dioxa-4,5,10,19,20,23-hexaazatetracyclo[13.5.2.1^{2,5}.0^{18,21}]tricosa-1(20),2(23),3,15(22),16,18(21)-hexaen-9-one **example 167** as a white solid.
LCMS method F: [M+H]⁺ = 365.1, t_{R} = 1.89 min
LCMS method G: [M+H]⁺ = 365.2, t_{R} = 1.87 min
¹H NMR (400 MHz, *d*6-DMSO) δ 13.37 (1H, s), 8.16 - 8.15 (2H, m), 7.62 (1H, s), 7.51 - 7.46 (1H, m), 7.13 - 7.08 (1H, m), 4.84 (2H, t, J=4.4 Hz), 4.68 - 4.59 (4H, m), 3.59 - 3.52 (2H, m).

### Example 168: (12R)-12-fluoro-8,14-dioxa-10,19,20-triazatetracyclo[13.5.2.1^{2,6}.0^{18,21}] tricosa-1(20),2(23),3,5,15(22),16,18(21)-heptaen-9-one

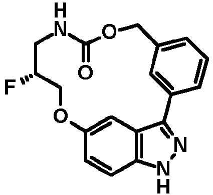

Example 168 is prepared according to the synthesis route described in general scheme E.

### Preparation of intermediate 305: methyl (2S)-2-(dibenzylamino)-3-hydroxy-propanoate

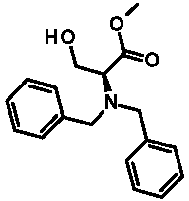

To a solution of L-serine methyl ester hydrochloride (2 g, 12.9 mmol) in acetontrile (30 mL) was added at room temperature potassium carbonate (3.56 g, 25.8 mmol) and benzyl bromide (3.1 mL, 25.8 mmol). The reaction mixture was stirred at room temperature for 3.5 h. The reaction mixture was filtered and concentrated under reduced pressure. The crude product was purified by silica gel column chromatography using cyclohexane/ethyl acetate 100/0 to 80/20 as eluent to afford methyl (2S)-2-(dibenzylamino)-3-hydroxy-propanoate intermediate 305 as a colorless oil.
LCMS method F: [M+H]⁺ = 300.2, t_{R} = 2.64 min

### Preparation of intermediate 306: methyl (2R)-3-(dibenzylamino)-2-fluoro-propanoate

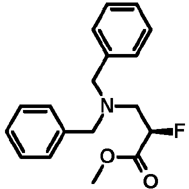

To a solution of DAST (1 mL, 7.91 mmol) in dry THF (15 mL) was slowly added, at room temperature a solution of methyl (2S)-2-(dibenzylamino)-3-hydroxy-propanoate intermediate **305** (2.15 g, 7.19 mmol) in dry THF (15 mL). The reaction mixture was stirred at room temperature for 30 min. Ice water and ethyl acetate were added. The organic layer was washed 5% aqueous NaHCO₃ solution then brine, dried over anhydrous sodium sulfate, filtered and concentrated to afford methyl (2R)-3-(dibenzylamino)-2-fluoro-propanoate intermediate **306** as a yellow viscous oil. The product was used in the next step without further purification.
LCMS method F: [M+H]⁺ = 302.3, t_{R} = 2.81 min

### Preparation of intermediate 307: (2R)-3-(dibenzylamino)-2-fluoro-propan-1-ol

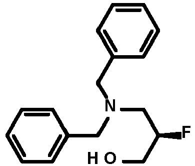

To a degassed solution of lithium borohydride (302 mg, 7.97 mmol) in dry THF (10 mL) at - 10 °C was added dropwise a solution of methyl (2R)-3-(dibenzylamino)-2-fluoro-propanoate intermediate **306** (2 g, 6.64 mmol) in dry THF (10 mL). The mixture was allowed to warm to room temperature and stirred overnight. The reaction mixture was cooled to 0°C and quenched with a saturated aqueous solution of ammonium chloride. The mixture was extracted with ethyl acetate. The combined organic layers were dried over anhydrous sodium suflate, filtered and concentrated under reduced pressure. The crude product was dissolved in 2 M hydrochloric acid (9 mL, until pH = 2) and the aqueous layer was washed with diethyl ether. The aqueous layer was basified to approximately pH = 10 with a saturated aqueous solution of NaHCO₃ and extracted with EtOAc. The combined organic layers were washed with brine, dried over anhydrous sodium sulfate, filtered and evaporated. The crude was dissolved in ethanol and filtered, then the filtrate was concentrated to afford (2R)-3-(dibenzylamino)-2-fluoro-propan-1-ol intermediate **307** as a white viscous oil.
LCMS method F: [M+H]⁺ = 274.3, t_{R} = 1.32 min

### Preparation of intermediate 308: (2R)-3-amino-2-fluoro-propan-1-ol

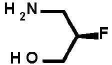

To a solution of (2R)-3-(dibenzylamino)-2-fluoro-propan-1-ol itermediate **307** (1.55 g, 5.68 mmol) in ethanol (20 mL) was added at room temperature palladium hydroxide 10 wt. % loading (155 mg). The reaction mixture was stirred under hydrogen atmosphere at room temperature for 2 days. The reaction mixture was filtered and the filtrate was concentrated under reduced pressure to give (2R)-3-amino-2-fluoro-propan-1-ol intermediate **308** as a yellow liquid. The product was used in the next step without further purification.
¹H NMR (400 MHz, CDCl₃) δ 4.62 - 4.5 (1H, m), 3.9 (1H, m), 3.84 (1H, m), 3.1 (1H, m), 3.04 (1H, m), 2.0 (3H, br).

### Preparation of intermediate 309: benzyl N-[(2R)-2-fluoro-3-hydroxy-propyl]carbamate

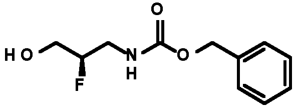

To a solution of (2R)-3-amino-2-fluoro-propan-1-ol itermediate **308** (541 mg, 5.68 mmol) in a mixture of THF (9 mL) and water (9 mL) was added sodium hydrogenocarbonate (525 mg, 6.25 mmol). The suspension was cooled to 0 °C and benzyl chloroformate (0.89 mL, 6.25 mmol) was added dropwise and the reaction mixture was stirred at room temperature overnight. The reaction mixture was diluted with water and extracted with ethyl acetate. The combined organic layers were washed with brine, dried over anhydrous sodium sulfate, filtered and evaporated under reduced pressure. The crude product was purified by silica gel column chromatography using cyclohexane/EtOAc 100/0 to 60/40 as eluent to afford benzyl N-[(2R)-2-fluoro-3-hydroxy-propyl]carbamate intermediate **309** as a white solid.
LCMS method F: [M+Na]⁺ = 250.1, t_{R} = 1.79 min

### Preparation of intermediate 310: [(2R)-3-(benzyloxycarbonylamino)-2-fluoro-propyl] methanesulfonate

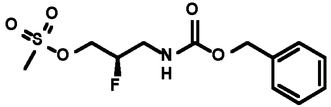

To a solution of benzyl N-[(2R)-2-fluoro-3-hydroxy-propyl]carbamate intermediate **309** (726 mg, 3.2 mmol) in dicholoromethane (13 mL) was added triethylamine (0.89 mL, 6.4 mmol). At 0°C was added methanesulfonyl chloride (0.32 mL, 4.16 mmol). The reaction mixture was stirred at room temperature for 1.5 h. The reaction mixture was quenched by addition of ammonium chloride solution and the mixture was extracted with dichloromethane. The organic layer was washed with a saturated aqueous NaHCO₃ solution and brine, dried over anhydrous sodium sulfate, filtered and concentrated under reduced pressure to give [(2R)-3-(benzyloxycarbonylamino)-2-fluoro-propyl]methanesulfonate intermediate 310 as an orange oil. The product was used in the next step without further purification.
LCMS method F: [M+H]⁺ = 306.1, t_{R} = 2.15 min

### Preparation of intermediate 311: benzyl N-[(2R)-2-fluoro-3-[3-[3-(hydroxymethyl)phenyl]-1-tetrahydropyran-2-yl-indazol-5-yl]oxy-propyl]carbamate

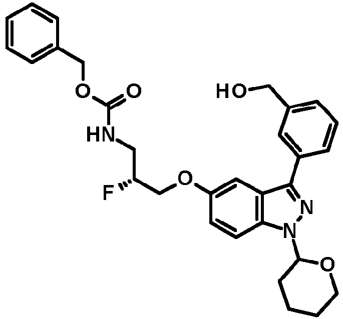

To a mixture of 3-[3-(hydroxymethyl)phenyl]-1-tetrahydropyran-2-yl-indazol-5-ol (518 mg, 1.60 mmol) in DMF (18 mL) was added cesium carbonate (1.04 g, 3.20 mmol). The reaction mixture was stirred for 20 min. [(2R)-3-(benzyloxycarbonylamino)-2-fluoro-propyl] methane sulfonate intermediate **310** (1.032 g, 3.2 mmol) in DMF (6 mL) was added. The reaction mixture was stirred at room temperature for 4 days. The reaction mixture was filtered and rinced with ethyl acetate. The filtrate was diluted with water and extracted with ethyl acetate. The combined organic layers were washed with brine, dried over anhydrous sodium sulfate, filtered and concentrated. The crude was purified by silica gel column chromatography using cyclohexane/ethyl acetate 100/0 to 60/40 as eluent to afford benzyl N-[(2R)-2-fluoro-3-[3-[3-(hydroxymethyl)phenyl]-1-tetrahydropyran-2-yl-indazol-5-yl]oxy-propyl]carbamate intermediate **311** as a white oil which crystallized at room temperature.
LCMS method F: [M+H]⁺ = 534.2, t_{R} = 2.83 min

### Preparation of intermediate 312: (12R)-12-fluoro-19-(oxan-2-yl)-8,14-dioxa-10,19,20-triazatetracyclo[13.5.2,1^{2,6}.0^{18,21}]tricosa-1(20),2(23),3,5,15(22),16,18(21)-heptaen-9-one

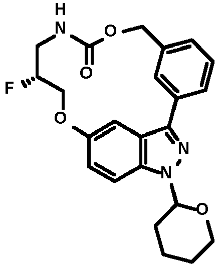

To a solution of benzyl N-[(2R)-2-fluoro-3-[3-[3-(hydroxymethyl)phenyl]-1-tetrahydropyran-2-yl-indazol-5-yl]oxy-propyl]carbamate intermediate **311** (167 mg, 0.31 mmol) in dry acetonitrile (16 mL) was added at room temperature finely powdered potassium hydroxide (87 mg, 1.55 mmol) in one portion. The reaction mixture was stirred at room temperature overnight. The reaction mixture was filtered, rinced with acetonitrile and evaporated under reduced pressure. The residue was purified by silica gel coumn chromatography using cyclohexane/ethyl acetate 100/0 to 70/30 as eluent to afford (12R)-12-fluoro-19-(oxan-2-yl)-8,14-dioxa-10,19,20-triazatetracyclo[13.5.2.1^{2,6}.0^{11,21}]tricosa-1(20),2(23),3,5,15(22), 16,18(21)-heptaen-9-one intermediate **312** as a white solid.
LCMS method F: [M+H]⁺ = 426.4, t_{R} = 2.76 min

### Preparation of Example 168; (12R)-12-fluoro-8,14-dioxa-10,19,20-triazatetracyclo [13.5.2. 1^{2,6}.0^{18,21}]tricosa-1(20),2(23),3,5,15(22),16,18(21)-heptaen-9-one

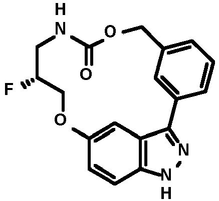

To a solution of (12R)-12-fluoro-19-(oxan-2-yl)-8,14-dioxa-10,19,20-triazatetracyclo [13.5.2.1^{2,6}.0^{18,21}]tricosa-1(20),2(23),3,5,15(22),16,18(21)-heptaen-9-one intermediate **312** (116 mg, 0.27 mmol) in methanol (4.6 mL) and water (0.8 mL) was added p-toluenesulfonic acid monohydrate (259 mg, 1.36 mmol). The reaction mixture was stirred at 65°C overnight. The reaction mixture was diluted with dichloromethane and a saturated aqueous NaHCO₃ solution. The aqueous layer was extracted with dichloromethane. The combined organic layers were washed with brine, dried over sodium sulfate, filtered and evaporated under reduced pressure. The residue was purified by silica gel column chromatography using cyclohexane/ethyl acetate 100/0 to 60/40 as eluent. The cream solid was triturated with diethyl ether, filtered and dried to afford (12R)-12-fluoro-8,14-dioxa-10,19,20-triazatetracyclo[13.5.2.1^{2,6}.0^{18,21}]tricosa-1(20),2(23),3,5,15(22),16,18(21)-heptaen-9-one **example 168** as a white powder.
LCMS method F: [M+H]⁺ = 342.2, t_{R} = 2.08 min
LCMS method G: [M+H]⁺ = 342.3, t_{R} = 2.07 min
¹H NMR (400 MHz, *d*6-DMSO) δ 13.21 (1H, s), 8.05 (1H, t, J=5.7 Hz), 7.86 (1H, d, J=7.9 Hz), 7.77 (1H, s), 7.56 (1H, d, J=8.5 Hz), 7.49 (1H, t, J=7.5 Hz), 7.31 (1H, m), 7.15 (1H, m), 7.08 (1H, dd, J=9.2, 2.2 Hz), 5.80 (1H, m), 5.16 (1H, m), 4.85 (1H, m), 4.61 (1H, m), 4.35 (1H, m, 3.61 (1H, m), 3.11 (1H, m) ppm.

### Example 169: (12S)-12-fluoro-8,14-dioxa-10,19,20-triazatetracyclo[13.5.2.1^{2,6}.0^{18,21}] tricosa-1(20),2(23),3,5,15(22),16,18(21)-heptaen-9-one

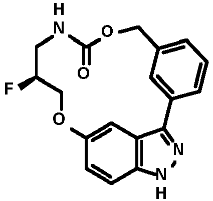

Example 169 is prepared according to the synthesis route described in general scheme E and according to the same methods described for example 168.

### Preparation of intermediate 313: (12S)-12-fluoro-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one

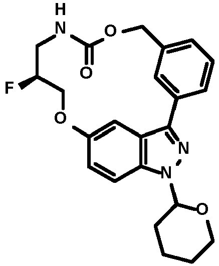

To a solution of benzyl N-[(2S)-2-fluoro-3-[3-[3-(hydroxymethyl)phenyl]-1-tetrahydropyran-2-yl-indazol-5-yl]oxy-propyl]carbamate (0.265 g, 0.50 mmol) in dry acetonitrile (32 mL) was added at room temperature finely powdered potassium hydroxide (140 mg, 2.50 mmol) in one portion. The reaction mixture was stirred at room temperature for 16 h. The reaction mixture was filtered and rinced with acetonitrile. The filtrate was evaporated under reduced pressure. The residue was purified by silica gel column chromatography using cyclohexane/ethyl acetate 90/10 to 50/50 as eluent to afford (12S)-12-fluoro-19-(oxan-2-yl)-8,14-dioxa-10,19,20-triazatetracyclo[13.5.2.1^{2,6}.0^{18,21}]tricosa-1(20),2(23),3,5,15(22),16,18(21)-heptaen-9-one intermediate **313** as a white solid.
LCMS method F: [M+H]⁺ = 426.2, t_{R} = 2.78 min

### Preparation of Example 169: (12S)-12-fluoro-8,14-dioxa-10,19,20-triazatetracyclo[13.5.2. 1^{2,6}.0^{18,21}]tricosa-1(20),2(23),3,5,15(22),16,18(21)-heptaen-9-one

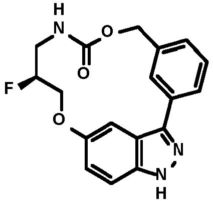

To a solution of (12S)-12-fluoro-19-(oxan-2-yl)-8,14-dioxa-10,19,20-triazatetracyclo[13.5.2. 1^{2,6}.0^{18,21}]tricosa-1(20),2(23),3,5,15(22),16,18(21)-heptaen-9-one intermediate **313** (115 mg, 0.27 mmol) in methanol (4.6 mL) and water (0.8 mL) was added p-toluenesulfonic acid monohydrate (259 mg, 1.36 mmol). The reaction mixture was stirred at 65 °C overnight. The reaction mixture was diluted with dichloromethane and a saturated aqueous NaHCO₃ solution. The aqueous layer was extracted with dichloromethane. The combined organic layers were washed with brine, dried over sodium sulfate, filtered and evaporated under reduced pressure. The residue was purified by silica gel coumn chromatography using cyclohexane/ethyl acetate 100/0 to 60/40 as eluent. The white solid was triturated with diethyl ether, filtered and dried to afford (12S)-12-fluoro-8,14-dioxa-10,19,20-triazatetracyclo[13.5.2.1^{2,6}.0^{18,21}]tricosa-1(20), 2(23),3,5,15(22),16,18(21)-heptaen-9-one **example 169** as a white powder.
LCMS method F: [M+H]⁺ = 342.2, t_{R} = 2.08 min
LCMS method G: [M+H]⁺ = 342.2, t_{R} = 2.07 min
¹H NMR (400 MHz, *d*6-DMSO) δ 13.21 (1H, s), 8.05 (1H, t, J = 5.7 Hz), 7.86 (1H, d, J = 7.9 Hz), 7.77 (1H, s), 7.55 (1H, d, J = 8.5 Hz), 7.49 (1H, t, J = 7.5 Hz), 7.31 (1H, m), 7.15 (1H, m), 7.08 (1H, dd, J = 9.2, 2.2 Hz), 5.80 (1H, m), 5.16 (1H, m), 4.85 (1H, m), 4.61 (1H, m), 4.35 (1H, m), 3.61 (1H, m), 3.11 (1H, m) ppm.

### Example 170: 12,12-Difluoro-8,14-dioxa-4,10,19,20,23-pentaazatetracyclo [13.5.2. 1^{2,6}.0^{18,21}]tricosa-1(20),2(23),3,5,15(22),16,18(21)-heptaen-9-one

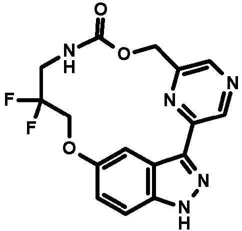

Example 170 is prepared according to the synthesis route described in general Scheme K.

### Preparation of intermediate 314: benzyl N-[2,2-difluoro-3-[3-[6-(hydroxymethyl)pyrazin-2-yl]-1-tetrahydropyran-2-yl-indazol-5-yl]oxy-propyl]carbamate

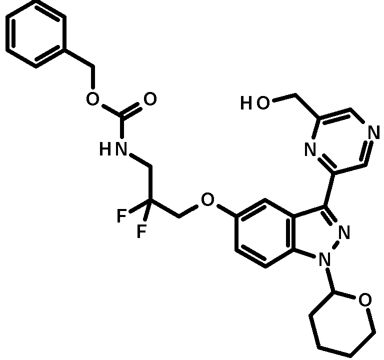

To a mixture of 3-[6-(hydroxymethyl)pyrazin-2-yl]-1-tetrahydropyran-2-yl-indazol-5-ol (125 mg, 0.38 mmol) and [3-(benzyloxycarbonylamino)-2,2-difluoro-propyl]trifluoromethane sulfonate (158 mg, 0.42 mmol) in acetonitrile (4 mL) was added potassium carbonate (157 mg, 1.14 mmol). The reaction mixture was stirred at 75 °C for 2 h. The reaction mixture was filtered and rinsed with ethyl acetate. The filtrate was concentrated under reduced pressure. The residue was purified by silica gel column chromatography using cyclohexane/ethyl acetate 99/1 to 50/50 as eluent to afford benzyl N-[2,2-difluoro-3-[3-[6-(hydroxymethyl)pyrazin-2-yl]-1-tetrahydropyran-2-yl-indazol-5-yl]oxy-propyl]carbamate **314** as a yellow solid.
LCMS method F: [M+H]⁺ = 554.2, t_{R} = 2.75 min

### Preparation of intermediate 315: 12,12-difluoro-19-(oxan-2-yl)-8,14-dioxa-4,10,19,20,23-pentaazatetracyclo[13.5.2.1^{2,618,21}]tricosa-1(20),2(23),3,5,15(22),16,18(21)-heptaen-9-one

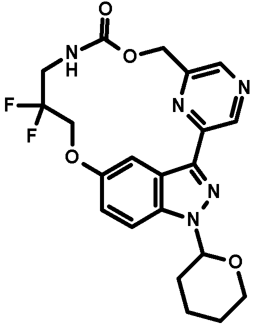

To a stirred solution of benzyl *N*-[2,2-difluoro-3-[3-[6-(hydroxymethyl)pyrazin-2-yl]-1-tetra hydropyran-2-yl-indazol-5-yl]oxy-propyl]carbamate **314** (57 mg, 0.11 mmol) in acetonitrile (12 mL) was added cesium carbonate (208 mg, 0.64 mmol) and the reaction mixture was stirred at reflux for 24 h. The reaction mixture was filtered and rinsed with acetonitrile. The filtrate was evaporated under reduced pressure. The residue was purified by silica gel column chromatography using cyclohexane/ethyl acetate 70/30 to 50/50 as eluent to afford 12,12-difluoro-19-(oxan-2-yl)-8,14-dioxa-4,10,19,20,23-pentaazatetracyclo[13.5.2.1^{2,6}.0^{18,21}]tricosa-1(20),2(23),3,5,15(22),16,18(21)-heptaen-9-one **315** as a white solid.
LCMS method F: [M+H]⁺ = 446.1, t_{R} = 2.50 min

### Preparation of example 170: 12,12-difluoro-8,14-dioxa-4,10,19,20,23-pentaazatetracyclo [13.5.2.1^{2,6}.0^{18,21}]tricosa-1(20),2(23),3,5,15(22),16,18(21)-heptaen-9-one

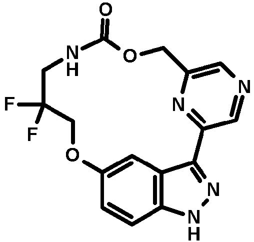

To a solution of 12,12-difluoro-19-(oxan-2-yl)-8,14-dioxa-4,10,19,20,23-pentaazatetracyclo [13.5.2.1^{2,6}.0^{18,21}]tricosa-1(20),2(23),3,5,15(22),16,18(21)-heptaen-9-one **315** (13 mg, 0.029 mmol) in methanol (0.55 mL) and water (85 µL) was added *p*-toluenesulfonic acid monohydrate (28 mg, 0.145 mmol). The reaction mixture was stirred at 65°C overnight. Additional *p*-toluenesulfonic acid monohydrate (28 mg, 0.145 mmol), methanol (0.55 mL) and water (85 µL) were added and the reaction mixture was stirred at 65°C for 24 h. The reaction mixture was diluted with dichloromethane and saturated aqueous NaHCO₃ solution. After separation, the aqueous layer was extracted with dichloromethane. The combined organic layer was washed brine, dried over anhydrous sodium sulfate, filtered and evaporated under reduced pressure. The residue was purified by silica gel column chromatography using cyclohexane/ethyl acetate 100/0 to 60/40 as eluent. The resulting solid was triturated with dichloromethane, filtered and dried to afford 12, 12-difluoro-8,14-dioxa-4, 10, 19,20,23-pentaazatetracyclo [13.5.2.1^{2,6}.0^{18,21}] tricosa-1(20),2(23),3,5,15(22),16,18(21)-heptaen-9-one **example 170** as a white powder.
LCMS method F: [M+H]⁺ = 362.2, t_{R} = 1.88 min
LCMS method G: [M+H]⁺ = 362.2, t_{R} = 1.85 min
¹H NMR (400 MHz, DMSO) : 13.57 (1H, s), 9.29 (1H, s), 8.57 (1H, s), 8.19 (1H, t, J=6.6 Hz), 8.00 (1H, d, J=1.1 Hz), 7.55 (1H, d, J=8.9 Hz), 7.14 (1H, dd, J=2.6, 9.0 Hz), 4.73 (2H, m), 3.61 (2H, m), 3.51 (2H, s) ppm.

### Example 171: (12S)-12-Fluoro-8,14-dioxa-4,10,19,20,23-pentaazatetracyclo [13.5.2. 1^{2,6}.0^{18,21}]tricosa-1(20),2(23),3,5,15,17,21-heptaen-9-one

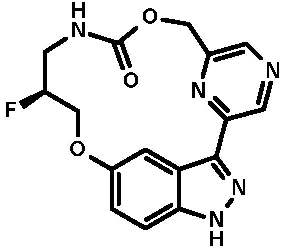

Example 171 is prepared according to the synthesis route described in general Scheme K.

### Preparation of intermediate 316: (12S)-12fluoro-19-(oxan-2yl)-8,14-dioxa-4,10,19,20,23-pentaazatetracyclo [13.5.2.1^{2,6}.0^{18,21}]tricosa-1(20),2(23),3,5,15,1 7,21-heptaen-9-one

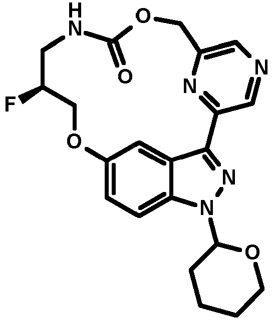

To a mixture of 3-[6-(hydroxymethyl)pyrazin-2-yl]-1-tetrahydropyran-2-yl-indazol-5-ol (195 mg, 0.597 mmol) and benzyl [(2S)-3-(benzyloxycarbonylamino)-2-fluoro-propyl]methane sulfonate (260 mg, 0.66 mmol) in acetonitrile (10 mL) was added potassium carbonate (247 mg, 1.79 mmol). The reaction mixture was stirred at 75°C for 2 h then overnight. Additional cesium carbonate (583 mg, 1.79 mmol) was added and the reaction was stirred at reflux for 3 h. Solvent was evaporated under reduced pressure and the residue was partitioned between ethyl acetate and water. The organic layer dried over anhydrous sodium sulfate, filtered and evaporated under reduced pressure. The residue was purified by silica gel column chromatography using dichloromethane/methanol 100/0 to 95/5 as eluent to afford (12S)-12-fluoro-19-(oxan-2-yl)-8,14-dioxa-4,10,19,20,23-pentaazatetracyclo[13.5.2.1^{2,6}.0^{11,21}]tricosa-1(20),2(23),3,5,15,17,21-heptaen-9-one **316** as a white solid.
LCMS method J : [M+H]⁺ = 428.4, t_{R} = 3.58 min

### Preparation of example 171: (12S)-12-fluoro-8,14-dioxa-4,10,19,20,23-pentaazatetracyclo [13.5.2.1^{2,6}.0^{18,21}]tricosa-1(20),2(23),3,5,15,17,21-heptaen-9-one

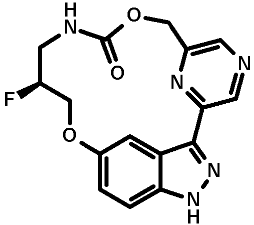

To a solution of afford (12S)-12-fluoro-19-(oxan-2-yl)-8,14-dioxa-4,10,19,20,23-pentaazatetra cyclo[13.5.2.1^{2,6}.0^{18,21}]tricosa-1(20),2(23),3,5,15,17,21-heptaen-9-one **316** (30 mg, 0.07 mmol) in methanol (3 mL) and water (1 mL) was added *p*-toluenesulfonic acid monohydrate (67 mg, 0.35 mmol) and the reaction mixture was stirred at 75°C overnight. Additional *p-*toluenesulfonic acid monohydrate (67 mg, 0.35 mmol) was added and the reaction mixture was stirred at 75°C for 6 h then at 60°C 72 h. The reaction mixture was diluted with ethyl acetate and saturated aqueous NaHCO₃ solution. After separation, the aqueous layer was extracted with ethyl acetate. The combined organic layers were washed with brine, dried over anhydrous sodium sulfate, filtered and evaporated under reduced pressure. The residue was purified by NH₂ silica gel column chromatography using dichloromethane/methanol 100/0 to 95/5 as eluent. The resulting solid was successively triturated with hot methanol then hot water, filtered and dried to afford (12S)-12-fluoro-8,14-dioxa-4,10,19,20,23-pentaazatetracyclo [13.5.2.1^{2,6}.0^{18,21}]tricosa-1(20), 2(23),3,5,15,17,21-heptaen-9-one **example 171** as a cream powder.
LCMS method F: [M+H]⁺ = 344.2, t_{R} = 1.87 min
LCMS method G: [M+H]⁺ = 344.2, t_{R} = 1.88 min
¹H NMR (400 MHz, DMSO) 13.59 (1H, s), 9.30-9.29 (1H, m), 8.56 - 8.54 (1H, m), 8.04-8.00 (1H, m), 7.73-7.71 (1H, m), 7.61-7.58 (1H, m), 7.11 (1H, dd, J=2.4, 9.0 Hz), 5.78-4.92 (3H, m), 4.74-4.58 (1H, m), 4.41-4.28 (1H, m), 3.56-3.47 (1H, m), 3.14 (1H, s) ppm.

### Example 172 : (12R)-12-Fluoro-8,14-dioxa-4,10,19,20,23-pentaazatetracyclo [13.5.2. 1^{2,6}.0^{18,21}]tricosa-1(20),2(23),3,5,15,17,21-heptaen-9-one

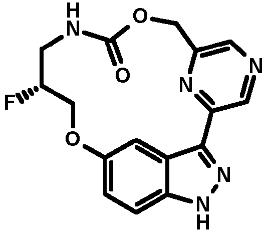

Example 172 is prepared according to the synthesis route described in general Scheme K.

### Preparation of intermediate 317: benzyl N-[(2R)-2-fluoro-3-([3-[6-(hydroxymethyl)pyrazin-2-yl]-1-(oxan-2-yl)-1H-indazol-5-yl}oxy)propyl]carbamate

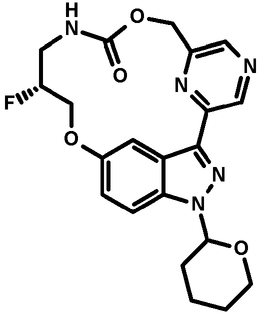

To a solution of 3-[6-(hydroxymethyl)pyrazin-2-yl]-1-tetrahydropyran-2-yl-indazol-5-ol (400 mg, 1.23 mmol) and [(2R)-3-(benzyloxycarbonylamino)-2-fluoro-propyl]methanesulfonate (460 mg, 1.36 mmol) in acetonitrile (20 mL) was added cesium carbonate (1.2 g, 3.69 mmol). The reaction mixture was stirred at reflux for 4 h. Solvent was evaporated under reduced pressure. The residue was partitioned between ethyl acetate and water. The organic layer was dried over anhydrous sodium sulfate, filtered and evaporated under reduced pressure. The residue was purified by silica gel column chromatography using dichloromethane/methanol 100/0 to 95/5 as eluent to afford (12R)-12-fluoro-19-(oxan-2-yl)-8,14-dioxa-4,10,19,20,23-pentaazatetracyclo[13.5.2.1^{2,6}.0^{18,21}]tricosa-1(20),2(23),3,5,15,17,21-heptaen-9-one **317** as a light yellow solid.
LCMS method F: [M+H]⁺ = 428.4.2, t_{R} = 2.48 min

### Preparation of example 172: (12R)-12-fluoro-8,14-dioxa-4,10,19,20,23-pentaazatetracyclo [13.5.2.1^{2,6}.0^{18,21}]tricosa-1(20),2(23),3,5,15,17,21-heptaen-9-one

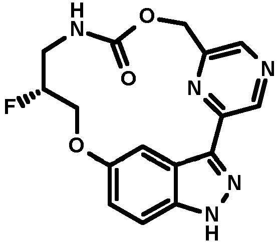

To a solution of (12R)-12-fluoro-19-(oxan-2-yl)-8,14-dioxa-4,10,19,20,23-pentaazatetracyclo [13.5.2.1^{2,6}.0^{18,21}]tricosa-1(20),2(23),3,5,15,17,21-heptaen-9-one **317** (291 mg, 0.681 mmol) in methanol (25 mL) and water (8 mL) was added *p*-toluenesulfonic acid monohydrate (647 mg, 3.4 mmol). The reaction mixture was stirred at 60 °C for 72 h. Additional *p*-toluenesulfonic acid monohydrate (647 mg, 3.4 mmol) was added and the mixture was stirred at 75°C for 2 h.

The solvents were evaporated under reduced pressure and the residue was diluted with ethyl acetate and saturated aqueous NaHCO₃ solution. After separation, the aqueous layer was extracted with ethyl acetate. The combined organic layers were washed with brine, dried over anhydrous sodium sulfate, filtered and evaporated under reduced pressure. The residue was purified by NH₂ silica gel column chromatography using dichloromethane/methanol 100/0 to 95/5 as eluent. The resulting solid was triturated with hot water, filtered an dried to afford (12R)-12-fluoro-8,14-dioxa-4,10,19,20,23-pentaazatetracyclo[13.5.2.1^{2,6}.0^{18,21}]tricosa-1(20), 2(23),3,5,15,17,21-heptaen-9-one **example 172** as a cream solid.
LCMS method F: [M+H]⁺ = 344.3, t_{R} = 1.80 min
LCMS method G: [M+H]⁺ = 344.2, t_{R} = 1.80 min
¹H NMR (400 MHz, DMSO) 13.59 (1H, s), 9.30 (1H, s), 8.56-8.54 (1H, m), 8.02 (1H, t, J=5.0 Hz), 7.73-7.71 (1H, m), 7.62-7.58 (1H, m), 7.11 (1H, dd, J=2.4, 9.0 Hz), 5.78-4.92 (3H, m), 4.73-4.58 (1H, m), 4.40-4.28 (1H, m), 3.52-3.48 (1H, m), 3.14 (1H, s) ppm.

### Example 173: (12S)-12-fluoro-8,14-dioxa-4,5,10,19,20,23-hexaazatetracyclo[13.5.2. 1^{2,5}.0^{18,21}]tricosa-1(20),2(23),3,15,17,21-hexaen-9-one

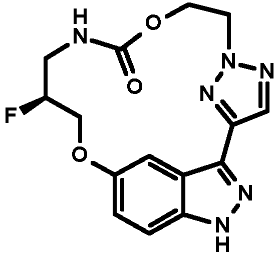

Example **173** is prepared according to the synthesis route described in general Scheme O.

### Preparation of intermediate 318: (12S)-12-fluoro-19-(oxan-2-yl)-8,14-dioxa-4,5,10,19,20, 23-hexaazatetracyclo[13.5.2.1^{2,5}.0^{18,21}]tricosa-1(20),2(23),3,15,17,21-hexaen-9-one

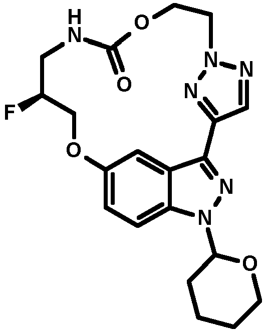

To a mixture of 3-[2-(2-hydroxyethyl)triazol-4-yl]-1-tetrahydropyran-2-yl-indazol-5-ol (250 mg, 0.759 mmol) and [(2S)-3-(benzyloxycarbonylamino)-2-fluoro-propyl] methanesulfonate (348 mg, 1.14 mmol) in acetonitrile (50 mL) was added cesium carbonate (743 mg, 2.28 mmol). The reaction mixture was stirred at reflux for 6 h. The solvents were evaporated under reduced pressure. The residue was partitioned between ethyl acetate and water. The organic layer was dried over anhydrous sodium sulfate, filtered and evaporated under reduced pressure. The resulting solid was triturated with methanol, filtered and dried to afford (12S)-12-fluoro-19-(oxan-2-yl)-8,14-dioxa-4,5,10,19,20,23-hexaazatetracyclo[13.5.2.1^{2,5}.0^{18,21}]tricosa-1(20), 2(23),3,15, 17,21-hexaen-9-one **318** as a white solid.
LCMS method F: [M+H]⁺ = 431.4, t_{R} = 2.46 min

### Preparation of example 173: (12S)-12-fluoro-8,14-dioxa-4,5,10,19,20,23-hexaazatetra cyclo[13.5.2.1^{2,5}.0^{18,21}]tricosa-1(20),2(23),3,15,17,21-hexaen-9-one

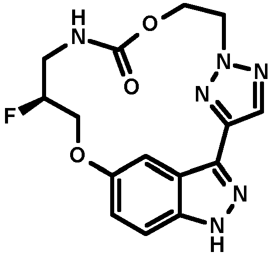

To a solution of (12S)-12-fluoro-19-(oxan-2-yl)-8,14-dioxa-4,5,10,19,20,23-hexaazatetracyclo [13.5.2.1^{2,5}.0^{18,21}]tricosa-1(20),2(23),3,15,17,21-hexaen-9-one **318** (130 mg, 0.302 mmol) in methanol (12 mL) and water (4 mL) was added *p*-toluenesulfonic acid monohydrate (287 mg, 1.51 mmol). The reaction mixture was stirred at 75 °C for 8 h. Methanol was evaporated under reduced pressure. The resulting solid was triturated with saturated aqueous NaHCO₃ solution and filtered then washed with water and dried to afford (12S)-12-fluoro-8,14-dioxa-4,5,10,19,20,23-hexaazatetracyclo[13.5.2.1^{2,5}.0^{18,21}]tricosa-1(20),2(23),3,15,17,21-hexaen-9-one **example 173** as a white solid.
LCMS method F: [M+H]⁺ = 347.3, t_{R} = 1.82 min
LCMS method G: [M+H]⁺ = 347.3, t_{R} = 1.75 min
¹H NMR (400 MHz, DMSO) 13.24 (1H, s), 8.16 (1H, s), 7.88-7.84 (1H, m), 7.55-7.51 (1H, m), 7.39 (1H, d, J=2.1 Hz), 7.08 (1H, dd, J=2.4, 9.0 Hz), 4.94-4.43 (6H, m), 4.35-4.24 (1H, m), 3.47-3.42 (1H, m), 3.20-3.11 (1H, m) ppm.

### Example 174: (12R)-12-fluoro-8,14-dioxa-4,5,10,19,20,23-hexaazatetracyclo[13.5.2. 1^{2,5}.0^{18,21}]tricosa-1(20),2(23),3,15,17,21-hexaen-9-one

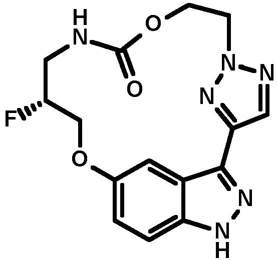

Example **174** is prepared according to the synthesis route described in general Scheme O.

### Preparation of intermediate 319: (12R)-12-fluoro-19-(oxan-2-yl)-8,14-dioxa-4,5,10,19,20, 23-hexaazatetracyclo[13.5.2.1^{2,5}0^{18,21}]tricosa-1(20),2(23),3,15,17,21-hexaen-9-one

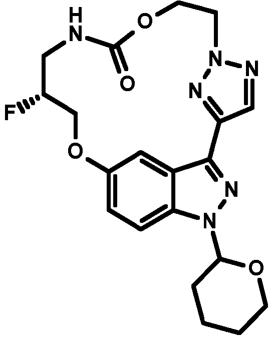

To a solution of 3-[2-(2-hydroxyethyl)triazol-4-yl]-1-tetrahydropyran-2-yl-indazol-5-ol (345 mg, 1.05 mmol) and [(2R)-3-(benzyloxycarbonylamino)-2-fluoro-propyl]methanesulfonate (530 mg, 0.482 mmol) in acetonitrile (70 mL) was added cesium carbonate (1.03 g, 3.15 mmol).

The reaction mixture was stirred at reflux for 8 h. Solvent was removed under reduced pressure. The residue was triturated with methanol, filtered, rinsed with methanol and dried to afford (12R)-12-fluoro-19-(oxan-2-yl)-8,14-dioxa-4,5,10,19,20,23-hexaazatetracyclo[13.5.2. 1^{2,5}.0^{18,21}]tricosa-1(20),2(23),3,15,17,21-hexaen-9-one **319** as a white solid.
LCMS method F: [M+H]⁺ = 431.4, t_{R} = 2.44 min

### Preparation of example 174: (12R)-12-fluoro-8,14-dioxa-4,5,10,19,20,23-hexaazatetra cyclo[13.5.2.1^{2,5}.0^{18,21}]tricosa-1(20),2(23),3,15,17,21-hexaen-9-one

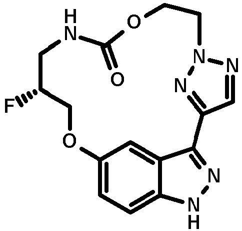

To a solution of (12R)-12-fluoro-19-(oxan-2-yl)-8,14-dioxa-4,5,10,19,20,23-hexaazatetracyclo [13.5.2.1^{2,5}.0^{18,21}]tricosa-1(20),2(23),3,15,17,21-hexaen-9-one **319** (230 mg, 0.534 mmol) in methanol (24 mL) and water (8 mL) was added p-toluenesulfonic acid monohydrate (508 mg, 2.67 mmol). The reaction mixture was stirred at 65 °C overnight. The solvents were evaporated under reduced pressure. The resulting solid was triturated with saturated aqueous NaHCO₃ solution. After filtration the solid was washed with water and dried to afford (12R)-12-fluoro-8,14-dioxa-4,5,10,19,20,23-hexaazatetracyclo[13.5.2.12,5.018,21]tricosa-1(20),2(23),3,15,17, 21-hexaen-9-one **example 174** as a cream solid.
LCMS method F: [M+H]⁺ = 347.2, t_{R} = 1.82 min
LCMS method G: [M+H]⁺ = 347.3, t_{R} = 1.75 min
¹H NMR (400 MHz, DMSO) 13.24 (1H, s), 8.16 (1H, s), 7.89-7.83 (1H, m), 7.57-7.46 (1H, m), 7.40-7.37 (1H, m), 7.15-7.05 (1H, m), 4.93-4.85 (1H, m), 4.81-4.57 (4H, m), 4.49-4.43 (1H, m), 4.36-4.23 (1H, m), 3.50-3.41 (1H, m), 3.21-3.12 (1H, m) ppm.

### Example 175: 8',14'-Dioxa-10',19',20'-triazaspiro[cyclopropane-1,13'-tetracyclo [13.5.2. 1^{2,6}.0^{18,21}]tricosane]-1'(20'),2'(23'),3',5',15'(22'),16',18'(21')-heptaen-9'-one

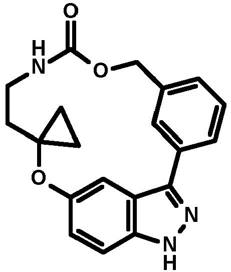

Example **175** is prepared according to the synthesis route described in detail below.

### Preparation of intermediate 320: (3-iodo-1-tetrahydropyran-2-yl-indazol-5-yl)3-(dibenzyl amino)propanoate

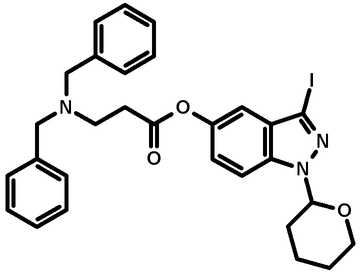

To a solution of the 3-(dibenzylamino)propanoic acid hydrochloride (864 mg, 2.83 mmol) in DMF (10 mL) was added N-ethyl-N,N-diisopropylamine (1.47 mL, 8.50 mmol). The reaction mixture was cooled to 0 °C then N-(3-dimethylaminopropyl)-N'-ethylcarbodiimide hydrochloride (1.088 g, 5.67 mmol), 1-hydroxybenzotriazole (867 mg, 5.67 mmol) and and the 3-iodo-1-tetrahydropyran-2-yl-indazol-5-ol (1.46 g, 4.25 mmol) were added. The reaction mixture was stirred at RT overnight. The mixture was poured onto water then extracted with ethyl acetate. The organic layer was successively washed with a saturated aqueous NaHCO₃ solution, water, saturated aqueous ammonium chloride solution then brine, dried over anhydrous magnesium sulfate, filtered and concentrated under reduced pressure. The residue was purified by silica gel column chromatography using cyclohexane/ethyl acetate 99/1 to 80/20 as eluent to provide (3-iodo-1-tetrahydropyran-2-yl-indazol-5-yl)-3-(dibenzylamino) propanoate **320** as a colorless oil.
LCMS method F: [M+H]⁺ = 596.1, t_{R} = 2.73 min

### Preparation of intermediate 321: N,N-dibenzyl-3-(3-iodo-1-tetrahydropyran-2-yl-indazol-5-yl)oxy-but-3-en-1-amine

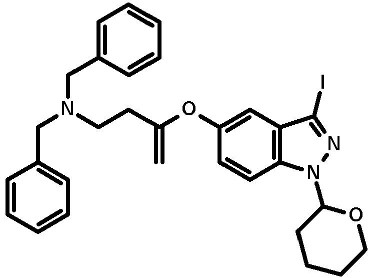

To a solution of (3-iodo-1-tetrahydropyran-2-yl-indazol-5-yl)-3-(dibenzylamino)propanoate **320** (1.541 g, 2.59 mmol) in dry THF (15 mL) at 0°C was added dropwise Tebbe reagent (0.5 M solution in toluene) (6.22 mL, 3.11 mmol). The reaction mixture was allowed to reach RT and stirred overnight. The reaction mixture was poured into ice/water then 1M aqueous sodium hydroxide solution was added. The aqueous layer was extracted with ethyl acetate. The combined organic layers were washed with brine, dried over anhydrous magnesium sulfate, filtered and concentrated under reduced pressure. The residue was purified by silica gel column chromatography using cyclohexane/ethyl acetate 99/1 to 80/20 as eluent to afford *N,N-*dibenzyl-3-(3-iodo-1-tetrahydropyran-2-yl-indazol-5-yl)oxy-but-3-en-1-amine **321** as a colorless oil.
LCMS method F: [M+H]⁺ = 594.1, t_{R} = 2.65 min

### Preparation of intermediate 322: N,N-dibenzyl-3-[3-[3-[[tert-butyl(dimethyl)silyl]oxymethyl] phenyl]-1-tetrahydropyran-2-yl-indazol-5-yl]oxy-but-3-en-1-amine

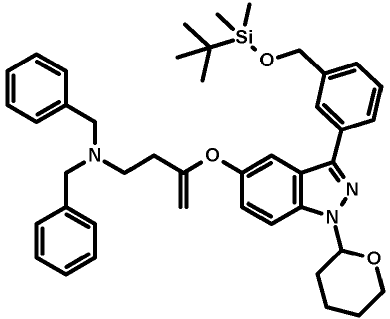

To a degassed solution of *N*,*N*-dibenzyl-3-(3-iodo-1-tetrahydropyran-2-yl-indazol-5-yl)oxy-but-3-en-1-amine **321** (642 mg, 1.08 mmol), *tert*-butyl-dimethyl-[[3-(4,4,5,5-tetramethyl-1,3,2-dioxaborolan-2-yl)phenyl]methoxy]silane (487 mg, 1.40 mmol) and potassium phosphate tribasic (686 mg, 3.24 mmol) in dioxane (5.35 mL) and water (1.50 mL) was added tetrakis(triphenylphosphine)palladium(0) (57 mg, 0.05 mmol) and 2-dicyclohexylphosphino-2',4',6'-triisopropylbiphenyl (52 mg, 0.11 mmol). The reaction mixture was stirred at 100°C for 1 h. The reaction mixture was diluted with water and extracted with ethyl acetate. The organic layer was washed with water, brine, dried over anhydrous magnesium sulfate, filtered and concentrated under reduced pressure. The residue was purified by silica gel column chromatography using cyclohexane/ethyl acetate 100/0 to 85/15 as eluent to afford *N,N*-di benzyl-3-[3-[3-[[*tert*-butyl(dimethyl)silyl]oxymethyl]phenyl]-1-tetrahydropyran-2-yl-indazol-5-yl]oxy-but-3-en-1-amine **322** as a slightly yellow oil.
LCMS method J: [M+H]⁺ = 688.6, t_{R} = 5.42 min

### Preparation of intermediate 323: N,N-dibenzyl-2-[1-[3-[3-[[tert-butyl(dimethyl)silyl]oxy methyl]phenyl]-1-tetrahydropyran-2-yl-indazol-5-yl]oxycyclopropyl]ethanamine

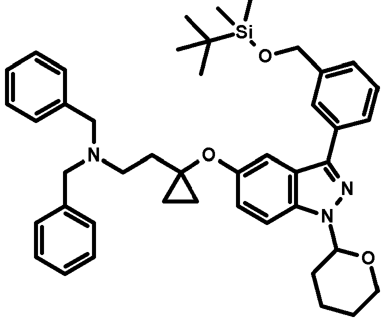

Dry DCE (20 mL) was degassed in a sealed tube then diethylzinc (1 M in heptane) (2.43 mL, 2.43 mmol) and diiodomethane (210 µL, 2.43 mmol) were added at RT. The solution was stirred at RT for 5 min. A solution of N,N-dibenzyl-3-[3-[3-[[tert-butyl(dimethyl)silyl]oxymethyl] phenyl]-1-tetrahydropyran-2-yl-indazol-5-yl]oxy-but-3-en-1-amine **322** (745 mg, 1.06 mmol) in dry DCE (20 mL) was added dropwise and the resulting mixture was stirred at RT for 16.5 h. In a separate flask, under argon, dry DCE (20 mL) was degassed then diethylzinc 1M in heptane (2.43 mL, 2.43 mmol) and diiodomethane (210 µL, 2.43 mmol) were added at RT for 5 min. The resulting suspension was added to the initial reaction mixture and allowed to stirred at RT overnight. The reaction mixture was quenched by pouring onto an aqueous solution of ammonium chloride then extracted with dichloromethane. The combined organic layers were washed with water, dried over anhydrous magnesium sulfate, filtered and concentrated under reduced pressure. The residue was purified by silica gel column chromatography using cyclohexane/ethyl acetate 100/0 to 85/15 as eluent to afford N,N-dibenzyl-2-[1-[3-[3-[[tert-butyl(dimethyl)silyl]oxymethyl]phenyl]-1-tetrahydropyran-2-yl-indazol-5-yl]oxycyclopropyl] ethanamine **323** as a colorless oil.
LCMS method F: [M+H]⁺ = 702.4, t_{R} = 3.14 min

### Preparation of intermediate 324: 2-[1-[3-[3-[[tert-butyl(dimethyl)silyl]oxymethyl[phenyl]-1-tetrahydropyran-2-yl-indazol-5-yl]oxycyclopropyl]ethanamine

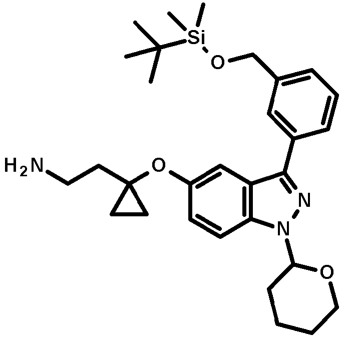

To a solution of *N*,*N*-dibenzyl-2-[1-[3-[3-[[tert-butyl(dimethyl)silyl]oxymethyl]phenyl]-1-tetrahydropyran-2-yl-indazol-5-yl]oxycyclopropyl]ethanamine **323** (210 mg, 0.30 mmol) in methanol (3 mL) and dichloromethane (3 mL) at RT was added palladium hydroxide on carbon 10 wt. % loading (5 mg). The reaction mixture was stirred under hydrogen atmosphere at RT for 2 h. The reaction mixture was filtered, rinsed with ethyl acetate and evaporated under reduced pressure to afford 2-[1-[3-[3-[[*tert*-butyl(dimethyl)silyl]oxymethyl]phenyl]-1-tetra hydropyran-2-yl-indazol-5-yl]oxycyclopropyl]ethanamine **324** as a white foam which was used in the next step without any further purification.
LCMS method F: [M+H]⁺ = 522.5, t_{R} = 2.68 min

### Preparation of intermediate 325: benzyl N-[2-[1-[3-[3-[[tert-butyl(dimethyl)silyl]oxymethyl] phenyl]-1-tetrahydropyran-2-yl-indazol-5-yl]oxycyclopropyl]ethyl]carbamate

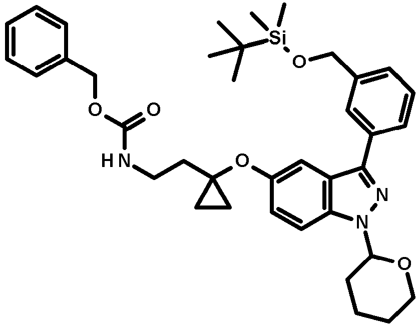

To a solution of 2-[1-[3-[3-[[*tert*-butyl(dimethyl)silyl]oxymethyl]phenyl]-1-tetrahydropyran-2-yl-indazol-5-yl]oxycyclopropyl]ethanamine **324** (156 mg, 0.30 mmol) in THF (2 mL) and water (1.5 mL) was added sodium hydrogenocarbonate (28 mg, 0.33 mmol). The suspension was cooled to 0 °C and benzyl chloroformate (50 µL, 0.33 mmol) was added dropwise. The reaction mixture was stirred at RT for 2 h. The solution was diluted with water and extracted with ethyl acetate. The combined organic layers were washed with brine, dried over anhydrous sodium sulfate, filtered and evaporated under reduced pressure. The residue was purified by silica gel column chromatography using cyclohexane/ethyl acetate 100/0 to 85/15 as eluent to afford benzyl *N*-[2-[1-[3-[3-[[*tert*-butyl(dimethyl)silyl]oxymethyl]phenyl]-1-tetrahydropyran-2-yl-indazol-5-yl]oxycyclopropyl]ethyl]carbamate **325** as a colorless oil.
LCMS method F: [M+H]⁺ = 656.3, t_{R} = 3.89 min

### Preparation of intermediate 326: benzyl N-[2-[1-[3-[3-(hydroxymethyl)phenyl]-1-tetrahydro pyran-2-yl-indazol-5-yl]oxycyclopropyl]ethyl]carbamate

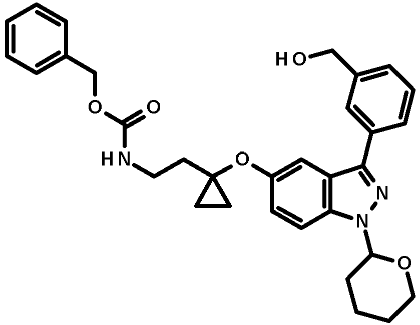

To a solution of benzyl *N*-[2-[1-[3-[3-[[*tert*-butyl(dimethyl)silyl]oxymethyl]phenyl]-1-tetra hydropyran-2-yl-indazol-5-yl]oxycyclopropyl]ethyl]carbamate **325** (90 mg, 0.14 mmol) in THF (1.5 mL) at RT was added dropwise tetrabutylammonium fluoride (1.0 M in THF) (0.15 mL, 0.15 mmol). The reaction mixture was stirred at RT overnight. The reaction mixture was poured into ice water and stirred for 20 min. The aqueous layer was extracted with ethyl acetate and the combined organic layers were washed with brine, dried over anhydrous magnesium sulfate, filtered and concentrated under reduced pressure to afford benzyl *N*-[2-[1-[3-[3-(hydroxymethyl)phenyl]-1-tetrahydropyran-2-yl-indazol-5-yl]oxycyclopropyl]ethyl] carbamate **326** as a colorless oil which was used in the next step without further purification.
LCMS method J: [M+H]⁺ = 542.4, t_{R} = 4.57 min

### Preparation of intermediate 327: 19'-(oxan-2-yl)-8',14'-dioxa-10',19',20'-triazaspiro[cyclo propane-1,13'-tetracyclo[13.5.2.1^{2,6}.0^{18,21}]tricosane]-1'(20'),2'(23'),3',5',15'(22'),16', 18'(21')-heptaen-9'-one

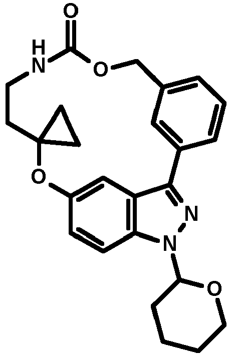

To a solution of benzyl N-[2-[1-[3-[3-(hydroxymethyl)phenyl]-1-tetrahydropyran-2-yl-indazol-5-yl]oxycyclopropyl]ethyl]carbamate **326** (65 mg, 0.12 mmol) in dry acetonitrile (10 mL) was added at RT potassium hydroxide (33 mg, 0.60 mmol) in one portion. The reaction mixture was stirred at RT for 16 h. The reaction mixture was filtered then rinsed with ethyl acetate and evaporated under reduced pressure. The residue was purified by silica gel column chromatography using dichloromethane/ethyl acetate 100/0 to 80/20 as eluent to afford 19'-(oxan-2-yl)-8',14'-dioxa-10',19',20'-triazaspiro[cyclopropane-1,13'-tetracyclo[13.5.2.1^{2,6}.0^{18,21}] tricosane]-1'(20'),2'(23'),3',5',15'(22'),16',18'(21')-heptaen-9'-one **327** as a white solid.
LCMS method J: [M+H]⁺ = 434.4, t_{R} = 4.43 min

### Preparation of example 175: 8',14'-dioxa-10',19',20'-triazaspiro[cyclopropane-1,13'-tetra cyclo[13.5.2.1^{2,6}.0^{18,21}]tricosane]-1'(20'),2'(23'),3',5',15'(22'),16',18'(21')-heptaen-9'-one

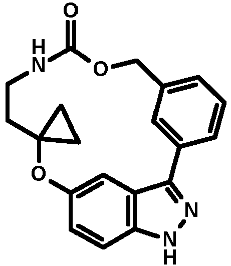

To a solution of 19'-(oxan-2-yl)-8',14'-dioxa-10',19',20'-triazaspiro[cyclopropane-1,13'-tetra cyclo[13.5.2.1^{2,6}.0^{18,21}]tricosane]-1'(20'),2'(23'),3',5',15'(22'),16',18'(21')-heptaen-9'-one **327** (41 mg, 0.095 mmol) in methanol (2 mL) and water (0.3 mL) was added *p*-toluenesulfonic acid monohydrate (90 mg, 0.47 mmol). The reaction mixture was stirred at 65°C overnight. The reaction mixture was diluted with dichloromethane and saturated aqueous NaHCO₃ solution. After separation, the aqueous layer was extracted with dichloromethane. The combined organic layers were washed brine, dried over anhydrous magnesium sulfate, filtered and evaporated under reduced pressure. The residue was purified by preparative TLC using dichloromethane/ methanol 95/5 as eluent to afford 8',14'-dioxa-10',19',20'-triazaspiro[cyclopropane-1,13'-tetra cyclo[13.5.2.1^{2,6}.0^{18,21}]tricosane]-1'(20'),2'(23'),3',5',15'(22'),16',18'(21')-heptaen-9'-one **example 175** as a white solid.
LCMS method F: [M+H]⁺ = 350.1, t_{R} = 2.30 min
LCMS method G: [M+H]⁺ = 350.2, t_{R} = 2.18 min
¹H NMR (400 MHz, DMSO-d6, 80 °C): 12.85 (1H, s), 7.95 (1H, s), 7.86 (1H, d, *J =* 7.6 Hz), 7.51 (1H, d, *J=* 2.0 Hz), 7.49-7.40 (3H, m), 7.30-7.27 (1H, m), 6.90 (1H, dd, *J=* 2.3, 9.1 Hz), 5.27 (2H, s), 3.01-2.95 (2H, m), 2.58-2.52 (2H, m), 1.13-1.11 (2H, m), 0.79-0.75 (2H, m) ppm.

Table 1 provides the example number, the IUPAC name and the general scheme according to which the compounds have been made.

**Table 1**

| Example number | IUPAC Name | General scheme |
|---|---|---|
| Example 1 | 8,14-dioxa-4,10,19,20-tetraazatetracyclo[13.5.2.1^{2,6}.0^{18,21}]tricosa-1(20),2,4,6(23),15,17,21-heptaen-9-one | Scheme A |
| Example 2 | 10-methyl-8,14-dioxa-4,10,19,20-tetraazatetracyclo[13.5.2.1^{2,6}.0^{18,21}]tricosa-1(20),2,4,6(23),15,17,21-heptaen-9-one | Scheme A |
| Example 3 | 4-fluoro-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 4 | 8,14-dioxa-10,19,20,23-tetraazatetracyclo[13.5.2.1^{2,6}.0^{18,21}]tricosa-1(20),2,4,6(23),15,17,21-heptaen-9-one | Scheme B |
| Example 5 | 8,14-dioxa-10,19,20-triazatetracyclo[13.5.2.1^{2,6}.0^{18,21}]tricosa-1(20),2,4,6(23),15,17,21-heptaen-9-one | Scheme A |
| Example 6 | 10-(propan-2-yl)-8,14-dioxa-4,10,19,20-tetraazatetracyclo[13.5.2.1^{2,6}.0^{18,21}]tricosa-1(20),2,4,6(23),15,17,21-heptaen-9-one | Scheme A |
| Example 7 | 8,14-dioxa-5,10,19,20-tetraazatetracyclo[13.5.2.1^{2,6}.0^{18,21}]tricosa-1(20),2,4,6(23),15,17,21-heptaen-9-one | Scheme B |
| Example 8 | 4-methoxy-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 9 | 4-bromo-8,14-dioxa-10,19,20-triazatetracyclo[13.5.2.1^{2,6}.0^{18,21}]tricosa-1(20),2,4,6(23),15,17,21-heptaen-9-one | Scheme D |
| Example 10 | 5-fluoro-8,14-dioxa-10,19,20-triazatetracyclo[13.5.2.1^{2,6}.0^{18,21}]tricosa-1(20),2,4,6(23),15,17,21-heptaen-9-one | Scheme E |
| Example 11 | 5-methyl-8,14-dioxa-10,19,20-triazatetracyclo[13.5.2.1^{2,6}.0^{18,21}]tricosa-1(20),2,4,6(23), 15, 17,21-heptaen-9-one | Scheme F |
| Example 12 | 4-(pyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 13 | 4-[4-(propan-2-yl)piperazin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 14 | 4-{2-oxa-6-azaspiro[3.4]octan-6-yl }-8, 14-dioxa-10,19,20-triazatetracyclo[13.5.2.1^{2,6}.0^{18,21}]tricosa-1(20),2,4,6(23),15,17,21-heptaen-9-one | Scheme C |
| Example 15 | 4-[4-(oxetan-3-yl)piperazin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 16 | 4-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 17 | 4-[(2R,6S)-2,6-dimethylmorpholin-4-yl]-8,14-dioxa-10,19,20-triazatetracyclo[13.5.2.1^{2,6}.0^{18,21}] tricosa-1(20),2,4,6(23),15,17,21-heptaen-9-one | Scheme C |
| Example 18 | 4-methyl-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 19 | 5-methoxy-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 20 | 4-(4,4-difluoropiperidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 21 | 4-(3,3-difluoropyrrolidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 22 | 7-methyl-8,14-dioxa-10,19,20-triazatetracyclo[13.5.2.1^{2,6}.0^{18,21}]tricosa-1(20),2,4,6(23), 15, 17,21-heptaen-9-one | Scheme C |
| Example 23 | 4-[4-(2-methoxyethyl)piperidin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 24 | 9,14-dioxa-11,19,20-triazatetracyclo[13.5.2.1^{2,6}.0^{18,21}]tricosa-1(20),2,4,6(23),15,17,21-heptaen-10-one | Scheme C |
| Example 25 | 4-[(3R)-3-hydroxypyrrolidin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 26 | 4-[(2-methoxyethyl)(methyl)amino]-8,14-dioxa-10,19,20-triazatetracyclo[13.5.2.1^{2,6}.0^{18,21l}]tricosa-1(20),2,4,6(23),15,17,21-heptaen-9-one | Scheme C |
| Example 27 | 4-chloro-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 28 | 4-fluoro-5-methyl-8,14-dioxa-10,19,20-triazatetracyclo[13.5.2.1^{2,6}.0^{18,21}]tricosa-1(20),2,4,6(23),15,17,21-heptaen-9-one | Scheme G |
| Example 29 | 4,5-difluoro-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 30 | 5-bromo-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 31 | 4-(4-methylpiperazin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 32 | 4-(3-methoxyazetidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 33 | 1-{9-oxo-8,14-dioxa-10,19,20-triazatetra cyclo[13.5.2.1^{2,6}.0^{18,21}]tricosa-1(20),2,4,6(23), 15,17,21-heptaen-4-yl}piperidine-4-carbonitrile | Scheme C |
| Example 34 | 4-[4-(pyrrolidin-1-yl)piperidin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 35 | 4-(azetidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 36 | 4-(piperidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 37 | 4-(2,5-dihydrofuran-3-yl)-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 38 | 4-[4-(morpholin-4-yl)piperidin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 39 | 4-(1-methyl-1,2,3,6-tetrahydropyridin-4-yl)-8,14-dioxa-l0,19,20-triazatetracyclo[13.5.2.1^{2,6}.0^{18,21}] tricosa-1(20),2,4,6(23),15,17,21-heptaen-9-one | Scheme A |
| Example 40 | 4-[(2S,5S)-2,5-dimethylmorpholin-4-yl]-8,14-dioxa-10,19,20-tiiazatetracyclo[13.5.2.1^{2,6}.0^{18,21}] tricosa-1(20),2,4,6(23),15,17,21-heptaen-9-one | Scheme C |
| Example 41 | 4-[(morpholin-4-yl)methyl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 42 | 4-[(pyrrolidin-1-yl)methyl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 43 | 4-[(piperidin-1-yl)methyl]-8, 14-dioxa-10,19,20-triazatetracyclo[13.5.2.1^{2,6}.0^{18,21}]tricosa-1(20),2,4,6(23),15,17,21-heptaen-9-one | Scheme C |
| Example 44 | 4-[(4-methylpiperazin-1-yl)methyl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 45 | 5-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 46 | 4-[4-(2-methoxyethyl)piperazin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 47 | 4-(diethylamino)-8,14-dioxa-10,19,20-triazatetracyclo[13.5.2.1^{2,6}.0^{18,2}1]tricosa-1(20),2,4,6(23),15,17,21-heptaen-9-one | Scheme A |
| Example 48 | 4-cyclopropyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 49 | 5-(4-methylpiperazin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 50 | 13-methyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 51 | 8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme C |
| Example 52 | 4-[methyl(oxetan-3-yl)amino]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 53 | 4-[(dimethylamino)methyl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 54 | 4,10-dimethyl-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 55 | 4-(propan-2-yloxy)-8,14-dioxa-10,19,20-triazatetracyclo[13.5.2.1^{2,6}.0^{18,21}]tricosa-1(20),2,4,6(23), 15, 17,21-heptaen-9-one | Scheme F |
| Example 56 | 4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 57 | 4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 58 | 4-(3-methylpiperidin-1-yl)-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 59 | 4-[(3 S)-3-hydroxypyrrolidin-1-yl]-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 60 | 4-fluoro-8,14-dioxa-10,19,20-triazatetracyclo[13.5.2.1^{2,6}.0^{18,21}]tetracosa-1(20),2(24),3,5,15(22),16,18(21)-heptaen-9-one | Synthesis described in details |
| Example 61 | 4-(oxolan-3-yl)-8,14-dioxa-10,19,20-triazatetracyclo[13.5.2.1^{2,6}.0^{18,21}]tricosa-1(20),2,4,6(23), 15, 17,21-heptaen-9-one | Scheme A |
| Example 62 | (13S)-13-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 63 | (13R)-13-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 64 | 4-(1-methyl-1H-pyrazol-3-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 65 | (7S)-7-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 66 | 4-[2-(morpholin-4-yl)ethoxy]-8,14-dioxa-10,19,20-triazatetracyclo[13.5.2.1^{2,6}.0^{18,21}]tricosa-1(20),2(23),3,5,15(22),16,18(21)-heptaen-9-one | Scheme F |
| Example 67 | 4-(2-methoxyethyl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 68 | (7R)-7-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 69 | 5-cyclopropyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 70 | 4-(2-methoxyethoxy)-8,14-dioxa-10,19,20-triazatetracyclo[13.5.2.1^{2,6}.0^{18,21}]tricosa-1(20),2,4,6(23),15,17,21-heptaen-9-one | Scheme F |
| Example 71 | 4-fluoro-13-methyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 72 | 11-methyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 73 | 4-(3-oxomorpholin-4-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 74 | 4-(2-oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 75 | 5-(2-oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 76 | 4-(2-methylpyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 77 | 2-{9-oxo-8,14-dioxa-10,19,20-triazatetracyclo [13.5.2.1^{2,6}.0^{18,21}]tricosa-1(20),2(23),3,5,15(22), 16,18(21)-heptaen-4-yl}acetonitrile | Scheme C |
| Example 78 | (11R) or (11S)-11-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 79 | (11R) or (11S)-11-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 80 | 4-ethynyl-8, 14-dioxa-10, 19,20-triazatetracyclo[13.5.2.1^{2,6}.0^{18,21}]tricosa-1(20),2(23),3,5,15(22),16,18(21)-heptaen-9-one | Scheme C |
| Example 81 | 4-(piperazin-1-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 82 | 4-(1,2,3,6-tetrahydropyridin-4-yl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 83 | 11-(methoxymethyl)-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 84 | 8,14-dioxa-5,10,19,20,23-pentaazatetracyclo[13.5.2.1^{2,5}.0^{18,21}]tricosa-1(20),2(23),3,15(22),16,18(21)-hexaen-9-one | Scheme K |
| Example 85 | 11-methyl-8, 14-dioxa-4,5, 10,19,20-pentaazatetracyclo[13.5.2.1^{2,5}.0^{18,21}]tricosa-1(20),2(23),3,15(22),16,18(21)-hexaen-9-one | Scheme C |
| Example 86 | 12-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme E |
| Example 87 | 11-ethyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme E |
| Example 88 | 4-fluoro-5,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 89 | 4-fluoro-5-methoxy-7-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 90 | 5-fluoro-4,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 91 | 8,14-dioxa-10,19,20-triazatetracyclo[13.5.2.1^{2,6}.1^{7,10}.0^{18,21}]tetracosa-1(20),2(24),3,5,15(22),16,18(21)-heptaen-9-one | Synthesis described in details |
| Example 92 | 13-methyl-8,14-dioxa-10,19,20,23-tetraazatetracyclo[13.5.2.1^{2,6}.0^{18,21}]tricosa-1(20),2(23),3,5,15(22),16,18(21)-heptaen-9-one | Scheme B |
| Example 93 | 12-methyl-8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme C |
| Example 94 | 7-methyl-8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme C |
| Example 95 | 5-fluoro-4-methoxy-7-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme A |
| Example 96 | (7R,13R)-7,13-dimethyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 97 | (13R)-13-methyl-8,14-dioxa-4,5,10,19,20-pentaazatetracyclo[13.5.2.1^{2,5}.0^{18,21}]tricosa-1(20),2(23),3,15,17,21-hexaen-9-one | Scheme C |
| Example 98 | 8,15-dioxa-4,10,20,21-tetraazapentacyclo [14.5.2.1^{2,6}.1^{10,13}.0^{19,22}]pentacosa-1(21),2(25),3,5,16(23),17,19(22)-heptaen-9-one | Scheme N |
| Example 99 | 8,14-dioxa-5,10,19,20-tetraazatetracyclo[13.5.2.1^{2,5}.0^{18,21}]tricosa-1(20),2(23),3,15(22),16,18(21)-hexaen-9-one | Scheme J |
| Example 100 | (13R) or (13S)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 101 | (13R) or (13S)-4-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 102 | (13R)-13-methyl-8,14-dioxa-4,10,19,20-tetraazatetracyclo[13.5.2.1^{2,6}.0^{18,21}]tricosa-1(20),2(23),3,5,15(22),16,18(21)-heptaen-9-one | Scheme C |
| Example 103 | 6-cyclopropyl-8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme B |
| Example 104 | 7-ethyl-8,14-dioxa-10,19,20-triazatetracyclo[13.5.2.1^{2,6}.0^{18,21}]tricosa-1(20),2,4,6(23),15,17,21-heptaen-9-one | Scheme G |
| Example 105 | (13R)-13-methyl-8,14-dioxa-5,10,19,20,23-pentaazatetracyclo[13.5.2.1^{2,6}.0^{18,21}]tricosa-1(20),2,4,6(23),15,17,21-heptaen-9-one | Scheme B |
| Example 106 | (7R,13R)-4-fluoro-7,13-dimethyl-8,14-dioxa-10,19,20-triazatetracyclo[13.5.2.1^{2,6}.0^{18,21}]tricosa-1(20),2,4,6(23),15,17,21-heptaen-9-one | Scheme C |
| Example 107 | 7-methyl-8,14-dioxa-4,10,19,20-tetraazatetracyclo[13.5.2.1^{2,6}.0^{18,21}]tricosa-1(20),2,4,6(23),15,17,21-heptaen-9-one | Scheme H |
| Example 108 | (7R)- or (7S)-4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 109 | (7R)- or (7S)-4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one | Scheme A |
| Example 110 | 6-methyl-8,14-dioxa-4,5,10,19,20-pentaazatetracyclo[13.5.2.1^{2,5}.0^{18,21}]tricosa-1(20),2(23),3,15,17,21-hexaen-9-one | Scheme B |
| Example 111 | 7-methyl-8,14-dioxa-10,19,20,23-tetraazatetracyclo[13.5.2.1^{2,6}.0^{18,21}]tricosa- | Scheme H |
| Example 112 | 6-(propan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme B |
| Example 113 | (13R)-7,13-dimethyl-8,14-dioxa-4,5,10,19,20-pentaazatetracyclo[13.5.2.1^{2,5}.0^{18,21}]tricosa-1(20),2(23),3,15(22),16,18(21)-hexaen-9-one | Scheme C |
| Example 114 | (13R)-13-methyl-8,14-dioxa-10,19,20,23-tetraazatetracyclo[13.5.2.1^{2,6}.0^{18,21}]tricosa-1(20),2,4,6(23),15,17,21-heptaen-9-one | Scheme K |
| Example 115 | (7R)- or (7S)-7-ethyl-8,14-dioxa-10,19,20-triazatetracyclo[13.5.2.1^{2,6}.0^{18,21}]tricosa-1(20),2,4,6(23),15,17,21-heptaen-9-one | Scheme G |
| Example 116 | (7R)- or (7S)-7-ethyl-8,14-dioxa-10,19,20-triazatetracyclo[13.5.2.1^{2,6}.0^{18,21}]tricosa-1(20),2,4,6(23),15,17,21-heptaen-9-one | Scheme G |
| Example 117 | (13R)-13-methyl-8,14-dioxa-5,10,19,20-tetraazatetracyclo[13.5.2.1^{2,6}.0^{18,21}]tricosa-1(20),2,4,6(23),15,17,21-heptaen-9-one | Scheme K |
| Example 118 | 6-(oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-one | Scheme B |
| Example 119 | 4-ethyl-8,14-dioxa-5,10,19,20,23-pentaazatetracyclo[13.5.2.1^{2,5}.0^{18,21}]tricosa-1(20),2(23),15,17,21-pentaen-9-one | Scheme B |
| Example 120 | (13R)-23-fluoro-13-methyl-8,14-dioxa-10,19,20-triazatetracyclo[13.5.2.1^{2,6}.0^{18,21}]tricosa- | Scheme C |
| Example 121 | 9,14-dioxa-4,5,11,19,20-pentaazatetracyclo[13.5.2.1^{2,5}.0^{18,21}]tricosa-1(20),2(23),3,15,17,21-hexaen-10-one | Scheme A |
| Example 122 | 4-ethyl-8,14-dioxa-5,10,19,20,23-pentaazatetracyclo[13.5.2.1^{2,5}.0^{18,21}]tricosa-1(20),2(23),3,15,17,21-hexaen-9-one | Scheme B |
| Example 123 | 3,9,15-trioxa-4,11,20,21-tetraazatetracyclo[14.5.2.1^{2,5}.0^{19,22}]tetracosa-1(21),2(24),4, 16, 18,22-hexaen-10-one | Scheme M |
| Example 124 | (13R)-16-fluoro-13-methyl-8,14-dioxa-4,10,19,20-tetraazatetracyclo[13.5.2.1^{2,6}.0^{18,21}] tricosa-1(20),2,4,6(23), 15, 17,21-heptaen-9-one | Scheme C |
| Example 125 | (13R)-4-chloro-13-methyl-8,14-dioxa-10,19,20, 23-tetraazatetracyclo[13.5.2.1^{2,6}.0^{18,21}] tricosa-1(20),2(23),3,5,15(22),16,18(21)-heptaen-9-one | Scheme K |
| Example 126 | 8,14-dioxa-2,4,10,19,20-pentaazatetracyclo[13.5.2.1^{2,5}0^{18,21}]tricosa-1(20),3,5(23),15(22),16,18(21)-hexaen-9-one | Scheme A |
| Example 127 | (13R)-4-methoxy-13-methyl-8,14-dioxa-10,19,20, 23-tetraazatetracyclo[13.5.2.1^{2,6}.0^{18,21}]tricosa-1(20),2,4,6(23),15,17,21-heptaen-9-one | Scheme K |
| Example 128 | (13R)-13-methyl-9-oxo-8,14-dioxa-10,19,20-triazatetracyclo[13.5.2.1^{2,6}.0^{18,21}]tricosa-1(20),2,4,6(23),15,17,21-heptaene-5-carbonitrile | Scheme B |
| Example 129 | (13R)-13-methyl-4-(pyrrolidin-1-yl)-8,14-dioxa-5,10,19,20,23-pentaazatetracyclo[13.5.2.1^{2,6}.0^{18,21}] tricosa-1(20),2,4,6(23), 15, 17,21-heptaen-9-one | Synthesis described in details |
| Example 130 | (7R,13R)- or (7S,13R)-7,13-dimethyl-8,14-dioxa-5,10,19,20,23-pentaazatetracyclo[13.5.2.1^{2,6}.0^{18,21}] tricosa-1(20),2,4,6(23),15,17,21-heptaen-9-one | Synthesis described in details |
| Example 131 | (7R,13R)- or (7S,13R)-7,13-dimethyl-8,14-dioxa-5,10,19,20,23-pentaazatetracyclo[13.5.2.1^{2,6}.0^{18,21}] tricosa-1(20),2,4,6(23),15,17,21-heptaen-9-one | Scheme K |
| Example 132 | (13R)-16-fluoro-13-methyl-8,14-dioxa-10,19,20-triazatetracyclo[13.5.2.1^{2,6}.0^{18,21}]tricosa- | Scheme C |
| Example 133 | (13R)-13-methyl-8,14-dioxa-4,10,19,20,23-pentaazatetracyclo[13.5.2.1^{2,6}.0^{18,21}]tricosa- | Scheme K |
| Example 134 | 8,14-dioxa-4-thia-10,19,20,23-tetraazatetracyclo[13.5.2.1^{2,5}.0^{18,21}]tricosa-1(20),2,5(23),15,17,21-hexaen-9-one | Scheme L |
| Example 135 | 8,14-dioxa-3-thia-10,19,20,23-tetraazatetracyclo[13.5.2.1^{2,5}.0^{18,21}]tricosa-1(20),2(23),4,15,17,21-hexaen-9-one | Scheme L |
| Example 136 | (7R,13R)-7,13-dimethyl-8,14-dioxa-10,19,20,23-tetraazatetracyclo[13.5.2.1^{2,6}.0^{18,21}]tricosa-1(20),2,4,6(23),15,17,21-heptaen-9-one | Scheme K |
| Example 137 | (13R)-4-[(3R)-3-methoxypyrrolidin-1-yl]-13-methyl-8,14-dioxa-5,10,19,20,23-pentaazatetracyclo[13.5.2.1^{2,6}.0^{18,21}]tricosa- | Scheme K |
| Example 138 | (13R)-16-chloro-13-methyl-8,14-dioxa-10,19,20-triazatetracyclo[13.5.2.1^{2,6}.0^{18,21}]tricosa-1(20),2,4,6(23),15,17,21-heptaen-9-one | Scheme K |
| Example 139 | (13R)-13,16-dimethyl-8,14-dioxa-10,19,20-triazatetracyclo[13.5.2.1^{2,6}.0^{18,21}]tricosa-1(20),2,4,6(23),15,17,21-heptaen-9-one | Scheme K |
| Example 140 | (13R)-13-methyl-8,14-dioxa-3,10,19,20,23-pentaazatetracyclo[13.5.2.1^{2,6}.0^{18,21}]tricosa-1(20),2,4,6(23),15,17,21-heptaen-9-one | Scheme B |
| Example 141 | 8-oxa-10,14,19,20-tetraazatetracyclo [13.5.2.1^{2,6}.0^{18,21}]tricosa-1(20),2(23),3,5,15(22),16,18(21)-heptaen-9-one | Scheme I |
| Example 142 | 8-oxa-10,19,20-triazatetracyclo [13.5.2.1^{2,6}.0^{18,21}]tricosa-1(20),2(23),3,5,15(22),16,18(21)-heptaen-9-one | Synthesis described in details |
| Example 143 | (13R)-5-methoxy-13-methyl-8,14-dioxa-4,10,19,20-tetraazatetracyclo[13.5.2.1^{2,6}.0^{18,21}] tricosa-1(20),2,4,6(23),15,17,21-heptaen-9-one | Scheme O |
| Example 144 | (13R)-13-methyl-8, 14-dioxa-4, 10, 19,20-tetraazatetracyclo[13.5.2.1^{2,6}.0^{18,21}]tricosa-1(20),2,6(23),15,17,21-hexaene-5,9-dione | Demethyla tion of example 143 |
| Example 145 | 4-methyl-8,14-dioxa-3,4,10,19,20-pentaazatetracyclo[13.5.2.1^{2,5}.0^{18,21}]tricosa-1(20),2,5(23),15(22),16,18(21)-hexaen-9-one | Scheme L |
| Example 146 | (13R)-16-fluoro-13-methyl-8,14-dioxa-10,19,20,23-tetraazatetracyclo[13.5.2.1^{2,6}.0^{18,21}] tricosa-1(20),2,4,6(23),15,17,21-heptaen-9-one | Scheme O |
| Example 147 | 7,13-dioxa-4-thia-9,18,19,22-tetraazatetracyclo[12.5.2.1^{2,5}.0^{17,20}]docosa-1(19),2,5(22),14(21),15,17(20)-hexaen-8-one | Scheme L |
| Example 148 | (13R)-4,13-dimethyl-8,14-dioxa-5,10,19,20,23-pentaazatetracyclo[13.5.2.1^{2,6}.0^{18,21}]tricosa-1(20),2(23),3,5,15(22),16,18(21)-heptaen-9-one | Scheme O |
| Example 149 | 8, 14-dioxa-23-thia-4, 10,19,20-tetraazatetracyclo[13.5.2.1^{2,5}0^{18,21}]tricosa-1(20),2,4,15(22),16,18(21)-hexaen-9-one | Scheme L |
| Example 150 | (7S,13R)-7,13-dimethyl-8,14-dioxa-10,19,20,23-tetraazatetracyclo[13.5.2.1^{2,6}.0^{18,21}]tricosa-1(20),2(23),3,5,15(22),16,18(21)-heptaen-9-one | Scheme K |
| Example 151 | (13R)-13-methyl-9-oxo-8,14-dioxa-5,10,19,20-tetraazatetracyclo[13.5.2.1^{2,5}.0^{18,21}]tricosa-1(20), 2(23),3,15(22),16,18(21)-hexaene-4-carbonitrile | Scheme O |
| Example 152 | 12,12-difluoro-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 153 | (13R)-17-fluoro-13-methyl-8,14-dioxa-10,19,20-triazatetracyclo[13.5.2.1^{2,6}.0^{18,21}]tricosa-1(20),2(23),3,5,15(22),16,18(21)-heptaen-9-one | Scheme C |
| Example 154 | (7S,13R)-7,13-dimethyl-8,14-dioxa-4,10,19, 20,23-pentaazatetracyclo[13.5.2.1^{2,6}.0^{18,21}] tricosa-1(20),2(23),3,5,15(22),16,18(21)-heptaen-9-one | Synthesis described in details |
| Example 155 | (7R,13R)-7,13-dimethyl-8,14-dioxa-4,10,19,20, 23-pentaazatetracyclo[13.5.2.1^{2,6}.0^{18,21}] tricosa-1(20),2(23),3,5,15(22),16,18(21)-heptaen-9-one | Scheme K |
| Example 156 | (13S)-13-methyl-8,14-dioxa-4,10,19,20,23-pentaazatetracyclo[13.5.2.1^{2,6}.0^{18,21}]tricosa-1(20),2(23),3,5,15(22),16,18(21)-heptaen-9-one | Scheme K |
| Example 157 | (13R)-13-methyl-8,14-dioxa-10,19,20,22-tetraazatetracyclo[13.5.2.1^{2,6}.0^{18,21}]tricosa-1(20),2(23),3,5,15,17,21-heptaen-9-one | Scheme C |
| Example 158 | (12R)-4,12-dimethyl-7,13-dioxa-4,9,18,19,22-pentaazatetracyclo[12.5.2.1^{2,5}.0^{17,20}]docosa-1(19),2,5(22),14(21),15,17(20)-hexaen-8-one | Scheme K |
| Example 159 | (13R)-13-methyl-8, 14-dioxa-4,5, 1 0, 19,20,23-hexaazatetracyclo[13.5.2.1^{2,5}.0^{18,21}]tricosa-1(20),2(23),3,15,17,21-hexaen-9-one | Scheme O |
| Example 160 | (13R)-13-methyl-8,14-dioxa-23-thia-4,10,19,20-tetraazatetracyclo[13.5.2.1^{2,5}.0^{18,21}]tricosa-1(20),2,4,15,17,21-hexaen-9-one | Scheme L |
| Example 161 | (13R)-4,13-dimethyl-8,14-dioxa-4,10,19,20,23-pentaazatetracyclo[13.5.2.1^{2,5}.0^{18,21}]tricosa-1(20),2,5(23),15(22),16,18(21)-hexaen-9-one | Scheme L |
| Example 162 | (13R)-13-methyl-8,14-dioxa-10,16,19,20-tetraazatetracyclo[13.5.2.1^{2,6}.0^{18,21}]tricosa-1(20),2(23),3,5,15(22),16,18(21)-heptaen-9-one | Synthesis described in details |
| Example 163 | 14-methyl-8-oxa-10,14,19,20-tetraazatetracyclo[13.5.2.1^{2,6}.0^{18,21}]tricosa-1(20),2(23),3,5,15,17,21-heptaen-9-one | Synthesis described in details |
| Example 164 | (13R)-13-methyl-8,14-dioxa-4,10,19,20,22-pentaazatetracyclo[13.5.2.1^{2,6}.0^{18,21}]tricosa-1(20),2(23),3,5,15,17,21-heptaen-9-one | Synthesis described in details |
| Example 165 | (13R)-13-methyl-8,14-dioxa-10,17,19,20-tetraazatetracyclo[13.5.2.1^{2,6}.0^{18,21}]tricosa-1(20),2(23),3,5,15(22),16,18(21)-heptaen-9-one | Synthesis described in details |
| Example 166 | 8,14-dioxa-4,5,10,19,20,23-hexaazatetracyclo [13.5.2.1^{2,5}.0^{18,21}]tricosa-1(20),2(23),3,15(22), 16,18(21)-hexaen-9-one | Scheme K |
| Example 167 | 12,12-difluoro-8,14-dioxa-4,5,10,19,20,23-hexaazatetracyclo[13.5.2.1^{2,5}.0^{18,21}]tricosa-1(20),2(23),3,15(22),16,18(21)-hexaen-9-one | Scheme K |
| Example 168 | (12R)-12-fluoro-8,14-dioxa-10,19,20-triazatetracyclo[13.5.2.1^{2,6}.0^{18,21}]tricosa-1(20),2(23),3,5,15(22),16,18(21)-heptaen-9-one | Scheme E |
| Example 169 | (12S)-12-fluoro-8,14-dioxa-10,19,20-triazatetracyclo[13.5.2.1^{2,6}.0^{18,21}]tricosa-1(20),2(23),3,5,15(22),16,18(21)-heptaen-9-one | Scheme E |
| Example 170 | 12, 12-difluoro-8, 14-dioxa-4, 10,19,20,23-pentaazatetracyclo [13.5.2.1^{2,6}.0^{18,21}]tricosa-1(20),2(23),3,5,15(22),16,18(21)-heptaen-9-one | Scheme K |
| Example 171 | (12S)-12-fluoro-8,14-dioxa-4,10,19,20,23-pentaazatetracyclo [13.5.2.1^{2,6}.0^{18,21}]tricosa-1(20),2(23),3,5,15,17,21-heptaen-9-one | Scheme K |
| Example 172 | (12R)-12-fluoro-8,14-dioxa-4,10,19,20,23-pentaazatetracyclo [13.5.2.1^{2,6}.0^{18,21}]tricosa-1(20),2(23),3,5,15,17,21-heptaen-9-one | Scheme K |
| Example 173 | (12S)-12-fluoro-8,14-dioxa-4,5,10,19,20,23-hexaazatetracyclo[13.5.2.1^{2,5}.0^{18,21}]tricosa-1(20),2(23),3,15,17,21-hexaen-9-one | Scheme O |
| Example 174 | (12R)-12-fluoro-8,14-dioxa-4,5,10,19,20,23-hexaazatetracyclo[13.5.2.1^{2,5}.0^{18,21}]tricosa-1(20),2(23),3,15,17,21-hexaen-9-one | Scheme O |
| Example 175 | 8',14'-dioxa-10',19',20'-triazaspiro[cyclopropane-1,13'-tetracyclo[13.5.2.1^{2,6}.0^{18,21}]tricosane]-1'(20'), 2'(23'),3',5',15'(22'),16',18'(21')-heptaen-9'-one | Synthesis described in details |

### PHARMACOLOGICAL STUDY

### EXAMPLE A: LRRK2 Kinase Activity Assay

### Protocol

LRRK2 Kinase reactions were carried out in 384-well white polystyrene plates in a final volume of 6 µl using ADP-Glo^{™} Kinase Assay kit (Promega Corp.). Compound and substrates (LRRKtide peptide and ATP) in assay buffer were first dispensed in wells. Kinase reaction was then started by the addition of human recombinant LRRK2 protein. After 1h-incubation at 37°C, the enzymatic reaction was stopped by the addition of 6 µl of ADP-Glo Reagent-1 and an additional 40-minutes incubation at 23°C (residual ATP depletion). A final 30-minutes incubation after 12µL reagent-2 addition (ADP to ATP conversion and luciferin/luciferase reaction) was performed before luminescent signal acquisition (EnVision^{™} multimode plate reader - PerkinElmer, Inc.). Data from 10 individual concentrations of tested compounds (N=2) were fitted (XLfit^{™} - ID Business Solutions Ltd) to deliver IC₅₀s (compound concentration leading to 50% inhibition of reference enzymatic activity).

### Compounds

The compounds are dissolved to 5 mM in DMSO. When needed, solutions are sonicated in a bath sonicator.

Table 2 provides the IC₅₀ values of the compounds according to the invention, obtained using the above-mentioned kinase assay. Activities are represented as +++, ++ and +, having the following meanings:
+++ means IC₅₀ < 10 nM
++ means 10 nM ≤ IC₅₀ < 100 nM
+ means 100 nM ≤ IC₅₀ < 1 µM.

**Table 2**

| Example number | IC₅₀ LRRK2 wt ADP-Glo | Example number | IC₅₀ LRRK2 wt ADP-Glo |
|---|---|---|---|
| Example 1 | +++ | Example 89 | + |
| Example 2 | ++ | Example 90 | +++ |
| Example 3 | +++ | Example 91 | +++ |
| Example 4 | +++ | Example 92 | +++ |
| Example 5 | +++ | Example 93 | +++ |
| Example 6 | ++ | Example 94 | +++ |
| Example 7 | +++ | Example 95 | +++ |
| Example 8 | +++ | Example 96 | +++ |
| Example 9 | +++ | Example 97 | +++ |
| Example 10 | +++ | Example 98 | +++ |
| Example 11 | +++ | Example 99 | +++ |
| Example 12 | +++ | Example 100 | +++ |
| Example 13 | +++ | Example 101 | +++ |
| Example 14 | +++ | Example 102 | +++ |
| Example 15 | +++ | Example 103 | +++ |
| Example 16 | +++ | Example 104 | ++ |
| Example 17 | +++ | Example 105 | +++ |
| Example 18 | +++ | Example 106 | +++ |
| Example 19 | +++ | Example 107 | +++ |
| Example 20 | +++ | Example 108 | ++ |
| Example 21 | +++ | Example 109 | +++ |
| Example 22 | +++ | Example 110 | +++ |
| Example 23 | +++ | Example 111 | ++ |
| Example 24 | ++ | Example 112 | ++ |
| Example 25 | +++ | Example 113 | +++ |
| Example 26 | +++ | Example 114 | +++ |
| Example 27 | +++ | Example 115 | + |
| Example 28 | +++ | Example 116 | +++ |
| Example 29 | +++ | Example 117 | +++ |
| Example 30 | +++ | Example 118 | + |
| Example 31 | +++ | Example 119 | ++ |
| Example 32 | +++ | Example 120 | + |
| Example 33 | +++ | Example 121 | + |
| Example 34 | +++ | Example 122 | +++ |
| Example 35 | +++ | Example 123 | +++ |
| Example 36 | +++ | Example 124 | +++ |
| Example 37 | +++ | Example 125 | +++ |
| Example 38 | +++ | Example 126 | ++ |
| Example 39 | +++ | Example 127 | +++ |
| Example 40 | +++ | Example 128 | +++ |
| Example 41 | +++ | Example 129 | +++ |
| Example 42 | +++ | Example 130 | ++ |
| Example 43 | ++ | Example 131 | +++ |
| Example 44 | +++ | Example 132 | +++ |
| Example 45 | +++ | Example 133 | +++ |
| Example 46 | +++ | Example 134 | +++ |
| Example 47 | +++ | Example 135 | +++ |
| Example 48 | +++ | Example 136 | +++ |
| Example 49 | ++ | Example 137 | +++ |
| Example 50 | +++ | Example 138 | +++ |
| Example 51 | +++ | Example 139 | +++ |
| Example 52 | +++ | Example 140 | ++ |
| Example 53 | ++ | Example 141 | + |
| Example 54 | +++ | Example 142 | +++ |
| Example 55 | +++ | Example 143 | +++ |
| Example 56 | +++ | Example 144 | +++ |
| Example 57 | +++ | Example 145 | + |
| Example 58 | +++ | Example 146 | +++ |
| Example 59 | +++ | Example 147 | +++ |
| Example 60 | +++ | Example 148 | +++ |
| Example 61 | +++ | Example 149 | +++ |
| Example 62 | +++ | Example 150 | ++ |
| Example 63 | +++ | Example 151 | +++ |
| Example 64 | +++ | Example 152 | +++ |
| Example 65 | ++ | Example 153 | +++ |
| Example 66 | +++ | Example 154 | ++ |
| Example 67 | +++ | Example 155 | +++ |
| Example 68 | +++ | Example 156 | +++ |
| Example 69 | +++ | Example 157 | +++ |
| Example 70 | +++ | Example 158 | ++ |
| Example 71 | +++ | Example 159 | +++ |
| Example 72 | +++ | Example 160 | +++ |
| Example 73 | +++ | Example 161 | ++ |
| Example 74 | +++ | Example 162 | +++ |
| Example 75 | +++ | Example 163 | +++ |
| Example 76 | +++ | Example 164 | +++ |
| Example 77 | +++ | Example 165 | +++ |
| Example 78 | +++ | Example 166 | +++ |
| Example 79 | +++ | Example 167 | +++ |
| Example 80 | +++ | Example 168 | +++ |
| Example 81 | +++ | Example 169 | +++ |
| Example 82 | +++ | Example 170 | +++ |
| Example 83 | +++ | Example 171 | +++ |
| Example 84 | ++ | Example 172 | +++ |
| Example 85 | ++ | Example 173 | +++ |
| Example 86 | +++ | Example 174 | +++ |
| Example 87 | +++ | Example 175 | +++ |
| Example 88 | ++ | | |

### EXAMPLE B: Pharmaceutical composition: Tablets

| | |
|---|---|
| 1000 tablets containing a dose of 5 mg of a compound selected from Examples 1 to 175 | 5 g |
| Wheat starch | 20 g |
| Maize starch | 20 g |
| Lactose | 30 g |
| Magnesium stearate | 2 g |
| Silica | 1 g |
| Hydroxypropylcellulose | 2 g |

## Claims

1. Compound of formula (I): 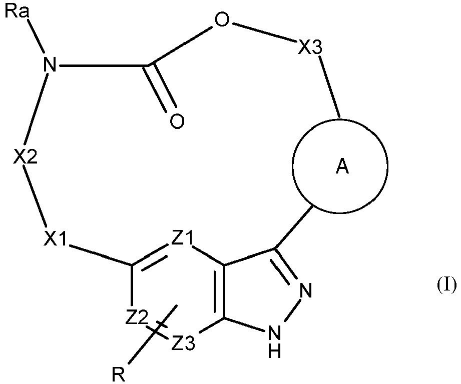 wherein:
◆ R represents a hydrogen atom, a halogen atom or an alkyl group,
◆ Z1, Z2, Z3, independently each represents a carbon or a nitrogen atom, it being understood that the 6-membered cycle containing Z1, Z2 and Z3 can have 0, 1 or 2 nitrogen atoms,
◆ -X1- is absent or represents -O-, -S-, or -N(R'a)-, wherein R'a represents a hydrogen atom or an alkyl group,
◆ -X2- represents an alkanediyl group optionally substituted with one or more substituents, identical or different, selected from halogen atoms, polyhalogenoalkyl group, alkoxy group, hydroxy group, amino group, alkylamino group, dialkylamino group and cyano group,
it being understood that the carbon atom in the alpha position of -N(Ra), and the carbon atom in the alpha position of -X1- when -X1- represents -O-, -S-, or -N(R'a)-, cannot be substituted with an oxygen or a nitrogen heteroatom,
◆ -X3- represents an alkanediyl group optionally substituted with one or more substituents, identical or different, selected from halogen atoms, polyhalogenoalkyl group, alkoxy group, hydroxy group, amino group, alkylamino group, dialkylamino group, cyano group, cycloalkyl group and heterocycloalkyl group,
it being understood that the carbon atom in the alpha position of -O-, and the carbon atom in the alpha position of A1 when A1 represents a nitrogen atom, cannot be substituted with an oxygen or a nitrogen heteroatom,
◆ Ra represents a hydrogen atom or an alkyl group,
it being understood that when Ra represents an alkyl group, one carbon atom of Ra can be linked to a carbon atom of -X2-, or to a carbon atom of -X3- to form a cyclic moiety containing 5 or 6 ring-members,
◆ A represents
- an aromatic or partially hydrogenated cyclic group of the formula (a): 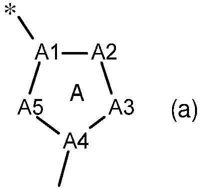 wherein
✔ A1, A4 each independently represents a carbon atom or a nitrogen atom,
✔ A2, A3, A5 each independently represents a carbon atom, an oxygen atom, a sulfur atom or a nitrogen atom,
it being understood that A1, A2, A3, A4 and A5 cannot simultaneously represent a heteroatom,
- or an aromatic or partially hydrogenated cyclic group of the formula (b): 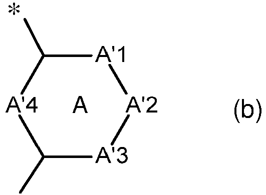 wherein A'1, A'2, A'3, A'4 each independently represents a carbon atom or a nitrogen atom,
it being understood that * means that the bond is linked to X3,
the aromatic or partially hydrogenated cyclic group A such defined being optionally substituted with one or more substituents, identical or different, selected from halogen atoms, alkyl group, alkoxy group, hydroxy group, oxo group, alkoxyalkyl group, alkoxyalkoxy group, polyhalogenoalkyl group, polyhalogenoalkoxy group, heterocycloalkyl group, heterocycloalkylalkyl group, (alkoxyalkyl)(alkyl)amino group, amino group, alkylamino group, dialkylamino group, cycloalkyl group, (heterocycloalkyl)(alkyl)amino group, dialkylaminoalkyl group, heterocycloalkylalkoxy group, cyano group and cyanoalkyl group,
wherein the heterocycloalkyl and cycloalkyl group such defined can be optionally substituted by one or more substituents chosen from alkyl group, halogen atoms, polyhalogenoalkyl group, polyhalogenoalkoxy group, alkoxy group, alkoxyalkyl group, hydroxy group, cyano group and oxo group,
their enantiomers, diastereoisomers, tautomers, racemic, hydrates, solvates, N-oxide, isotopes, deuterated derivatives and addition salts thereof with a pharmaceutically acceptable acid or base.

2. Compound according to claim 1, wherein R represents a hydrogen atom.

3. Compound according to claim 1, wherein R represents a halogen atom.

4. Compound according to any of claims 1 to 3, wherein Z1, Z2 and Z3 represent simultaneously a carbon atom.

5. Compound according to any of claim 1 to 3, wherein one of Z1 or Z2 represents a nitrogen atom and Z3 represents a carbon atom.

6. Compound according to any of claims 1 to 5, wherein -X1- represents -O-.

7. Compound according to any of claims 1 to 6, wherein -X2- represents an alkanediyl group linear or branched having 2, 3, 4 or 5 carbon atoms.

8. Compound according to claim 7, wherein -X2- represents -(CH₂)₃-, -CH(CH₃)-(CH₂)₂-, -CH₂-CHF-CH₂-, -CH₂-CF₂-CH₂-, or -(CH₂)₂-CH(CH₃)-.

9. Compound according to any of claims 1 to 8, wherein Ra is a hydrogen atom.

10. Compound according to any of claims 1 to 9, wherein -X3- represents an alkanediyl group linear or branched having 1, 2, 3, 4 or 5 carbon atoms.

11. Compound according to claim 10, wherein -X3- represents -(CH₂)₂-, -CH₂- or -CH(CH₃)-.

12. Compound according to any of claims 1 to 11, wherein A represents a group of formula (b): 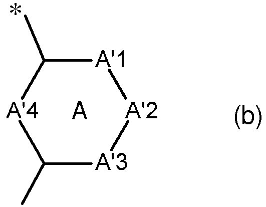 wherein A'1, A'2, A'3, A'4 and * are as defined in claim 1.

13. Compound according to claim 12, wherein A represents 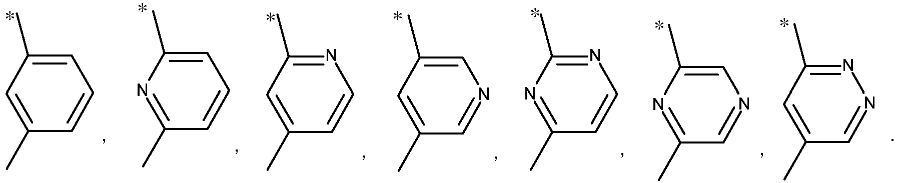 such defined A groups being not substituted or optionally substituted.

14. Compound according to claim 12 wherein A represents a phenyl group.

15. Compound according to claim 12 wherein A represents a pyridinyl group.

16. Compound according to claim 12 wherein A represents a pyrazinyl group.

17. Compound according to claims 1 to 11, wherein A represents a group of formula (a): 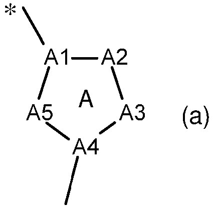 wherein A1, A2, A3, A4, A5 and * are as defined in claim 1.

18. Compound according to claim 17, wherein A represents 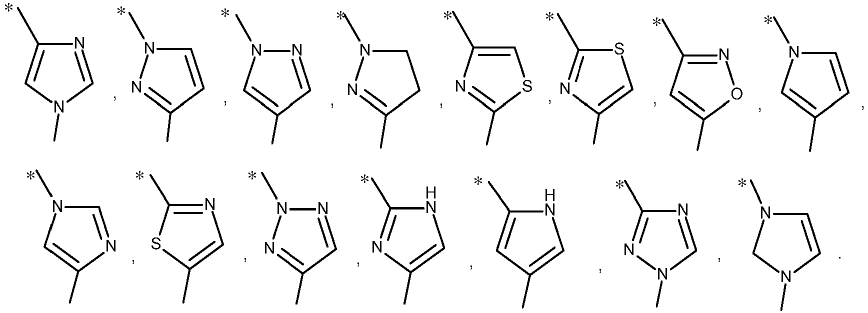 such defined A groups being not substituted or optionally substituted.

19. Compound according to claim 17 wherein A represents a triazolyl group.

20. Compound according to claim 17 wherein A represents a pyrazolyl group.

21. Compound according to claims 12 to 20, wherein A is not substituted.

22. Compound according to claims 12 to 20, wherein A is substituted with one or more groups chosen from halogen atoms, cyano group, cyanoalkyl group, oxo group, alkoxy group, alkyl group, cycloalkyl group and heterocycloalkyl group.

23. Compound according to claim 1, which is a compound of formula (I-a): 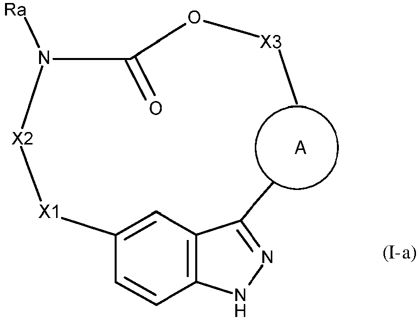 wherein X1, X2, X3, Ra and A are as defined in claim 1.

24. Compound according to claim 23, which is a compound of formula (I-b): 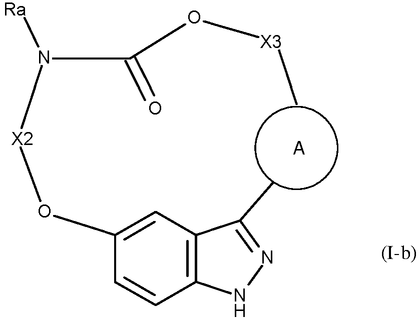 wherein X2, X3, Ra and A are as defined in claim 1.

25. Compound according to claim 23, which is a compound of formula (I-c) or (I-c'): 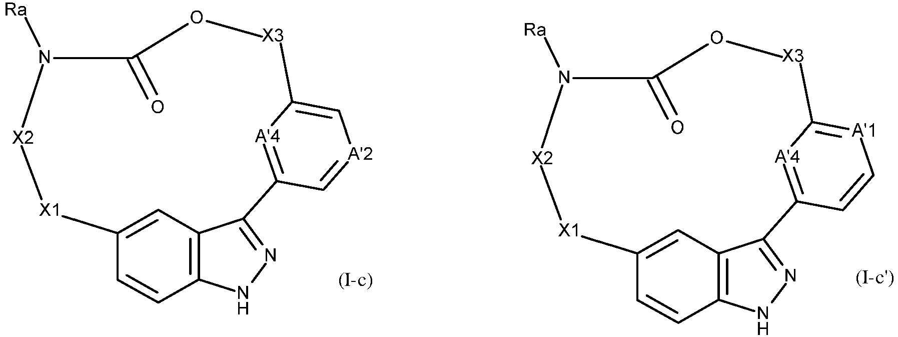 wherein X1, X2, X3, Ra, A'1, A'2 and A'4 are as defined in claim 1.

26. Compound according to claim 23 or 25, which is a compound of formula (I-d) or (I-d'): 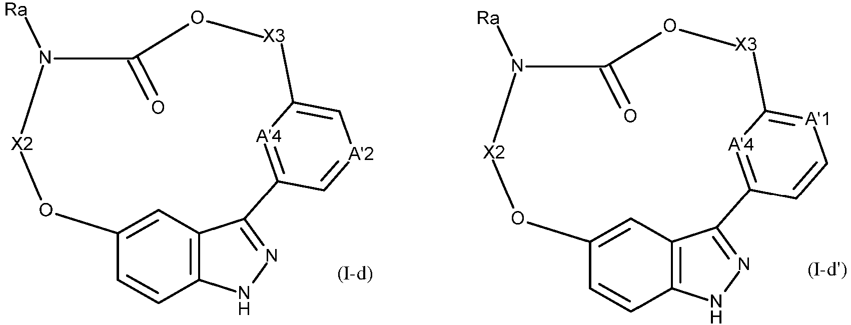 wherein X2, X3, Ra, A'1, A'2 and A'4 are as defined for formula (I).

27. Compound according to claim 23, which is a compound of formula (I-e): 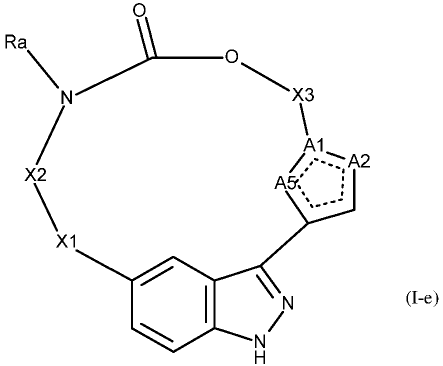 wherein X1, X2, X3, Ra, A1, A2 and A5 are as defined for formula (I).

28. Compound according to claim 23 or 27, which is a compound of formula (I-f): 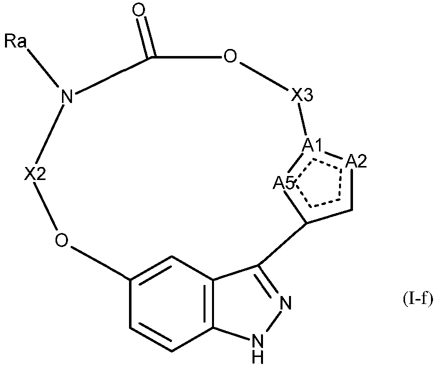 wherein X2, X3, Ra, A1, A2 and A5 are as defined for formula (I).

29. Compound according to claim 23, 25 or 27, wherein the -X1-X2-N(Ra)-C(O)O-X3-chain represents -O-(CH₂)₃-NHC(O)O-CH₂-, -O-CH(CH₃)-(CH₂)₂-NHC(O)O-CH₂-, -O-CH₂-CHF-CH₂-NHC(O)O-CH₂-, -O-CH₂-CF₂-CH₂-NHC(O)O-CH₂-, -O-CH(CH₃)-(CH₂)₂-NHC(O)O-(CH₂)₂- or -O-CH(CH₃)-(CH₂)₂-NH-C(O)O-CH(CH₃)-.

30. Compounds according to claim 1 which are:
- 8,14-dioxa-4,10,19,20-tetraazatetracyclo[13.5.2.1^{2,6}.0^{18,21}]tricosa-1(20),2,4,6(23),15,17,21-heptaen-9-one;
- 10-methyl-8,14-dioxa-4,10,19,20-tetraazatetracyclo[13.5.2.1^{2,6}.0^{18,21}]tricosa-1(20),2,4, 6(23),15,17,21-heptaen-9-one;
- 4-fluoro-8,14-dioxa-10,19,20-triazatetracyclo[13.5.2.1^{2,6}.0^{18,21}]tricosa-1(20),2,4,6(23), 15,17,21-heptaen-9-one;
- 8,14-dioxa-10,19,20,23-tetraazatetracyclo[13.5.2.1^{2,6}.0^{18,21}]tricosa-1(20),2,4,6(23), 15,17,21-heptaen-9-one;
- 8,14-dioxa-10,19,20-triazatetracyclo[13.5.2.1^{2,6}.0^{18,21}]tricosa-1(20),2,4,6(23),15,17,21-heptaen-9-one;
- 10-(propan-2-yl)-8,14-dioxa-4,10,19,20-tetraazatetracyclo[13.5.2.1^{2,6}.0^{18,21}]tricosa-1(20),2,4,6(23),15,17,21-heptaen-9-one;
- 8,14-dioxa-5,10,19,20-tetraazatetracyclo[13.5.2.1^{2,6}.0^{18,21}]tricosa-1(20),2,4,6(23), 15,17,21-heptaen-9-one;
- 4-methoxy-8,14-dioxa-10,19,20-triazatetracyclo[13.5.2.1^{2,6}.0^{18,21}]tricosa-1(20),2,4, 6(23),15,17,21-heptaen-9-one;
- 4-bromo-8,14-dioxa-10,19,20-triazatetracyclo[13.5.2.1^{2,6}.0^{18,21}]tricosa-1(20),2,4,6(23), 15,17,21-heptaen-9-one;
- 5-fluoro-8,14-dioxa-10,19,20-triazatetracyclo[13.5.2.1^{2,6}.0^{18,21}]tricosa-1(20),2,4,6(23), 15,17,21-heptaen-9-one;
- 5-methyl-8,14-dioxa-10,19,20-triazatetracyclo[13.5.2.1^{2,6}.0^{18,21}]tricosa-1(20),2,4,6(23), 15,17,21-heptaen-9-one;
- 4-(pyrrolidin-1-yl)-8,14-dioxa-10,19,20-triazatetracyclo[13.5.2.1^{2,6}.0^{18,21}]tricosa-1(20), 2,4,6(23),15,17,21-heptaen-9-one;
- 4-[4-(propan-2-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-{2-oxa-6-azaspiro[3.4]octan-6-yl}-8,14-dioxa-10,19,20-triazatetracyclo [13.5.2.1^{2,6}.0^{18,21}]tricosa-1(20),2,4,6(23),15,17,21-heptaen-9-one;
- 4-[4-(oxetan-3-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(morpholin-4-yl)-8,14-dioxa-10,19,20-triazatetracyclo[13.5.2.1^{2,6}.0^{18,21}]tricosa-1(20), 2,4,6(23),15,17,21-heptaen-9-one;
- 4-[(2R,6S)-2,6-dimethylmorpholin-4-yl]-8,14-dioxa-10,19,20-triazatetracyclo [13.5.2.1^{2,6}.0^{18,21}]tricosa-1(20),2,4,6(23),15,17,21-heptaen-9-one;
- 4-methyl-8,14-dioxa-10,19,20-triazatetracyclo[13.5.2.1^{2,6}.0^{18,21}]tricosa-1(20),2,4,6(23), 15,17,21-heptaen-9-one;
- 5-methoxy-8,14-dioxa-10,19,20-triazatetracyclo[13.5.2.1^{2,6}.0^{18,21}]tricosa-1(20),2,4, 6(23),15, 17,21-heptaen-9-one;
- 4-(4,4-difluoropiperidin-1-yl)-8,14-dioxa-10,19,20-triazatetracyclo[13.5.2. 1^{2,6}.0^{18,21}]tricosa-1(20),2,4,6(23),15,17,21-heptaen-9-one;
- 4-(3,3-difluoropyrrolidin-1-yl)-8,14-dioxa-10,19,20-triazatetracyclo [13.5.2.1^{2,6}.0^{18,21}]tricosa-1(20),2,4,6(23),15,17,21-heptaen-9-one;
- 7-methyl-8,14-dioxa-10,19,20-triazatetracyclo[13.5.2.1^{2,6}.0^{18,21}]tricosa-1(20),2,4,6(23),15,17,21-heptaen-9-one;
- 4-[4-(2-methoxyethyl)piperidin-1-yl]-8,14-dioxa-10,19,20-triazatetracyclo [13.5.2.1^{2,6}.0^{18,21}]tricosa -1(20),2,4,6(23),15,17,21-heptaen-9-one;
- 9,14-dioxa-11,19,20-triazatetracyclo[13.5.2.1^{2,6}.0^{18,21}]tricosa-1(20),2,4,6(23),15,17,21-heptaen-10-one;
- 4-[(3R)-3-hydroxypyrrolidin-1-yl]-8,14-dioxa-10,19,20-triazatetracyclo [13.5.2.1^{2 6}.0^{18,21}]tricosa-1(20),2,4,6(23),15,17,21-heptaen-9-one;
- 4-[(2-methoxyethyl)(methyl)amino]-8,14-dioxa-10,19,20-triazatetracyclo [13.5.2.1^{2,6}.0^{18,21}]tricosa-1(20),2,4,6(23),15,17,21-heptaen-9-one;
- 4-chloro-8,14-dioxa-10,19,20-triazatetracyclo[13.5.2.1^{2,6}.0^{18,21}]tricosa-1(20),2,4,6(23), 15,17,21-heptaen-9-one;
- 4-fluoro-5-methyl-8,14-dioxa-10,19,20-triazatetracyclo[13.5.2.1^{2,6}.0^{18,21}]tricosa-1(20),2, 4,6(23),15,17,21-heptaen-9-one;
- 4,5-difluoro-8,14-dioxa-10,19,20-triazatetracyclo[13.5.2.1^{2,6}.0^{18,21}]tricosa-1(20),2,4, 6(23),15,17,21-heptaen-9-one;
- 5-bromo-8,14-dioxa-10,19,20-triazatetracyclo[13.5.2.1^{2,6}.0^{18,21}]tricosa-1(20),2,4,6(23),15,17,21-heptaen-9-one;
- 4-(4-methylpiperazin-1-yl)-8,14-dioxa-10,19,20-triazatetracyclo[13.5.2.1^{2,6}.0^{18,21}] tricosa-1(20),2,4,6(23),15,17,21-heptaen-9-one;
- 4-(3-methoxyazetidin-1-yl)-8,14-dioxa-10,19,20-triazatetracyclo[13.5.2.1^{2,6}.0^{18,21}] tricosa-1(20),2, 4,6(23),15,17,21-heptaen-9-one;
- 1-{9-oxo-8,14-dioxa-10,19,20-triazatetracyclo[13.5.2.1^{2,6}.0^{18,21}]tricosa-1(20),2,4,6(23), 15,17,21-heptaen-4-yl}piperidine-4-carbonitrile;
- 4-[4-(pyrrolidin-1-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(azetidin-1-yl)-8,14-dioxa-10,19,20-triazatetracyclo[13.5.2.1^{2,6}.0^{18,21}]tricosa-1(20),2, 4,6(23),15,17,21-heptaen-9-one;
- 4-(piperidin-1-yl)-8,14-dioxa-10,19,20-triazatetracyclo[13.5.2.1^{2,6}.0^{18,21}]tricosa-1(20),2, 4,6(23),15,17,21-heptaen-9-one;
- 4-(2,5-dihydrofuran-3-yl)-8,14-dioxa-10,19,20-triazatetracyclo[13.5.2.1^{2,6}.0^{18,21}] tricosa-1(20),2,4,6(23),15,17,21-heptaen-9-one;
- 4-[4-(morpholin-4-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(1-methyl-1,2,3,6-tetrahydropyridin-4-yl)-8,14-dioxa-10,19,20-triazatetracyclo [13.5.2.1^{2,6}.0^{18,21}] tricosa-1(20),2,4,6(23),15,17,21-heptaen-9-one;
- 4-[(2S,5S)-2,5-dimethylmorpholin-4-yl]-8,14-dioxa-10,19,20-triazatetracyclo [13.5.2.1^{2,6}.0^{18,21}] tricosa-1(20),2,4,6(23),15,17,21-heptaen-9-one;
- 4-[(morpholin-4-yl)methyl]-8,14-dioxa-10,19,20-triazatetracyclo[13.5.2.1^{2,6}.0^{18,21}] tricosa-1(20),2,4,6(23),15,17,21-heptaen-9-one;
- 4-[(pyrrolidin-1-yl)methyl]-8,14-dioxa-10,19,20-triazatetracyclo[13.5.2.1^{2,6}.0^{18,21}] tricosa-1(20),2,4,6(23),15,17,21-heptaen-9-one;
- 4-[(piperidin-1-yl)methyl]-8,14-dioxa-10,19,20-triazatetracyclo[13.5.2.1^{2,6}.0^{18,21}] tricosa-1(20),2,4,6(23),15,17,21-heptaen-9-one;
- 4-[(4-methylpiperazin-1-yl)methyl]-8,14-dioxa-10,19,20-triazatetracyclo [13.5.2.1^{2,6}.0^{18,21}]tricosa-1(20),2,4,6(23),15,17,21-heptaen-9-one;
- 5-(morpholin-4-yl)-8,14-dioxa-10,19,20-triazatetracyclo[13.5.2.1^{2,6}.0^{18,21}]tricosa-1(20),2,4,6(23),15,17,21-heptaen-9-one;
- 4-[4-(2-methoxyethyl)piperazin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-(diethylamino)-8,14-dioxa-10,19,20-triazatetracyclo[13.5.2.1^{2,6}.0^{18,21}]tricosa-1(20),2, 4,6(23),15,17,21-heptaen-9-one;
- 4-cyclopropyl-8,14-dioxa-10,19,20-triazatetracyclo[13.5.2.1^{2,6}.0^{18,21}]tricosa-1(20),2, 4,6(23),15,17,21-heptaen-9-one;
- 5-(4-methylpiperazin-1-yl)-8,14-dioxa-10,19,20-triazatetracyclo[13.5.2.1^{2,6}.0^{18,21}] tricosa-1(20),2,4,6(23),15,17,21-heptaen-9-one;
- 13-methyl-8,14-dioxa-10,19,20-triazatetracyclo[13.5.2.1^{2,6}.0^{18,21}]tricosa-1(20),2,4,6(23), 15,17,21-heptaen-9-one;
- 8,14-dioxa-4,5,10,19,20-pentaazatetracyclo[13.5.2.1^{2,5}.0^{18,21}]tricosa-1(20),2(23),3, 15(22),16,18(21)-hexaen-9-one;
- 4-[methyl(oxetan-3-yl)amino]-8,14-dioxa-10,19,20-triazatetracyclo[13.5.2. 1^{2,6}.0^{18,21}]tricosa-1(20),2,4,6(23),15,17,21-heptaen-9-one;
- 4-[(dimethylamino)methyl]-8,14-dioxa-10,19,20-triazatetracyclo[13.5.2.1^{2,6}.0^{18,21}] tricosa-1(20),2,4,6(23),15,17,21-heptaen-9-one;
- 4,10-dimethyl-8,14-dioxa-10,19,20-triazatetracyclo[13.5.2.1^{2,6}.0^{18,21}]tricosa-1(20),2,4, 6(23),15,17,21-heptaen-9-one;
- 4-(propan-2-yloxy)-8,14-dioxa-10,19,20-triazatetracyclo[13.5.2.1^{2,6}.0^{18,21}]tricosa-1(20),2,4,6(23),15,17,21-heptaen-9-one;
- 4-fluoro-7-methyl-8,14-dioxa-10,19,20-triazatetracyclo[13.5.2.1^{2,6}.0^{18,21}]tricosa-1(20),2,4,6(23),15,17,21-heptaen-9-one;
- 4-[1-(oxetan-3-yl)-1,2,3,6-tetrahydropyridin-4-yl]-8,14-dioxa-10,19,20-triazatetracyclo[13.5.2.1^{2,6}.0^{18,21}]tricosa-1(20),2,4,6(23),15,17,21-heptaen-9-one;
- 4-(3-methylpiperidin-1-yl)-8,14-dioxa-10,19,20-triazatetracyclo[13.5.2.1^{2,6}.0^{18,21}] tricosa-1(20),2,4,6(23),15,17,21-heptaen-9-one;
- 4-[(3S)-3-hydroxypyrrolidin-1-yl]-8,14-dioxa-10,19,20-triazatetracyclo [13.5.2.1^{2,6}.0^{18,21}]tricosa-1(20),2,4,6(23),15,17,21-heptaen-9-one;
- 4-fluoro-8,14-dioxa-10,19,20-triazapentacyclo[13.5.2.1^{2,6}.1^{7,10}.0^{18,21}]tetracosa-1(20), 2(24),3,5,15(22),16,18(21)-heptaen-9-one;
- 4-(oxolan-3-yl)-8,14-dioxa-10,19,20-triazatetracyclo[13.5.2.1^{2,6}.0^{18,21}]tricosa-1(20), 2,4,6(23),15,17,21-heptaen-9-one;
- (13S)-13-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- (13R)-13-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-(1-methyl-1H-pyrazol-3-yl)-8,14-dioxa-10,19,20-triazatetracyclo [13.5.2.1^{2,6}.0^{18,21}]tricosa-1(20),2(23),3,5,15(22),16,18(21)-heptaen-9-one;
- (7S)-7-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-[2-(morpholin-4-yl)ethoxy]-8,14-dioxa-10,19,20-triazatetracyclo[13.5.2. 1^{2,6}.0^{18,21}]tricosa-1(20),2(23),3,5,15(22),16,18(21)-heptaen-9-one;
- 4-(2-methoxyethyl)-8,14-dioxa-10,19,20-triazatetracyclo[13.5.2.1^{2,6}.0^{18,21}]tricosa-1(20), 2(23), 3,5,15(22),16,18(21)-heptaen-9-one;
- (7R)-7-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 5-cyclopropyl-8,14-dioxa-10,19,20-triazatetracyclo[13.5.2.1^{2,6}.0^{18,21}]tricosa-1(20),2,4,6(23),15,17,21-heptaen-9-one;
- 4-(2-methoxyethoxy)-8,14-dioxa-10,19,20-triazatetracyclo[13.5.2.1^{2,6}.0^{18,21}]tricosa-1(20),2,4,6(23),15,17,21-heptaen-9-one;
- 4-fluoro-13-methyl-8,14-dioxa-10,19,20-triazatetracyclo[13.5.2.1^{2,6}.0^{18,21}]tricosa-1(20), 2,4,6(23),15,17,21- heptaen-9-one;
- 11-methyl-8,14-dioxa-10,19,20-triazatetracyclo[13.5.2.1^{2,6}.0^{18,21}]tricosa-1(20),2,4,6(23), 15,17,21-heptaen-9-one;
- 4-(3-oxomorpholin-4-yl)-8,14-dioxa-10,19,20-triazatetracyclo[13.5.2.1^{2,6}.0^{18,21}] tricosa-1(20),2(23),3,5,15(22),16,18(21)-heptaen-9-one;
- 4-(2-oxopyrrolidin-1-yl)-8,14-dioxa-10,19,20-triazatetracyclo[13.5.2.1^{2,6}.0^{18,21}] tricosa-1(20),2(23),3,5,15(22),16,18(21)-heptaen-9-one;
- 5-(2-oxopyrrolidin-1-yl)-8,14-dioxa-10,19,20-triazatetracyclo[13.5.2.1^{2,6}.0^{18,21}] tricosa-1(20),2(23),3,5,15(22),16,18(21)-heptaen-9-one;
- 4-(2-methylpyrrolidin-1-yl)-8,14-dioxa-10,19,20-triazatetracyclo[13.5.2.1^{2,6}.0^{18,21}] tricosa-1(20),2(23),3,5,15(22),16,18(21)-heptaen-9-one;
- 2-{9-oxo-8,14-dioxa-10,19,20-triazatetracyclo[13.5.2.1^{2,6}.0^{18,21}]tricosa-1(20),2(23), 3,5,15(22),16,18(21)-heptaen-4-yl}acetonitrile;
- (11R)-11-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- (11S)-11-methyl-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-ethynyl-8,14-dioxa-10,19,20-triazatetracyclo[13.5.2.1^{2,6}.0^{18,21}]tricosa-1(20),2(23),3,5,15(22),16,18(21)-heptaen-9-one;
- 4-(piperazin-1-yl)-8,14-dioxa-10,19,20-triazatetracyclo[13.5.2.1^{2,6}.0^{18,21}]tricosa-1(20), 2(23),3,5,15(22),16,18(21)-heptaen-9-one;
- 4-(1,2,3,6-tetrahydropyridin-4-yl)-8,14-dioxa-10,19,20-triazatetracyclo [13.5.2.1^{2,6}.0^{18,21}]tricosa-1(20),2(23),3,5,15(22),16,18(21)-heptaen-9-one;
- 11-(methoxymethyl)-8,14-dioxa-10,19,20-triazatetracyclo[13.5.2.1^{2,6}.0^{18,21}]tricosa-1(20),2(23),3,5,15(22),16,18(21)-heptaen-9-one;
- 8,14-dioxa-5,10,19,20,23-pentaazatetracyclo[13.5.2.1^{2,5}.0^{18,21}]tricosa-1(20),2(23),3, 15(22),16,18(21)-hexaen-9-one;
- 11-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 12-methyl-8,14-dioxa-10,19,20-triazatetracyclo[13.5.2.1^{2,6}.0^{18,21}]tricosa-1(20),2(23),3,5, 15(22),16,18(21)-heptaen-9-one;
- 11-ethyl-8,14-dioxa-10,19,20-triazatetracyclo[13.5.2.1^{2,6}.0^{18,21}]tricosa-1(20),2(23),3,5, 15(22),16,18(21)-heptaen-9-one;
- 4-fluoro-5,7-dimethyl-8,14-dioxa-10,19,20-triazatetracyclo[13.5.2.1^{2,6}.0^{18,21}] tricosa-1(20),2(23), 3,5,15(22),16,18(21)-heptaen-9-one;
- 4-fluoro-5-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-one;
- 5-fluoro-4,7-dimethyl-8,14-dioxa-10,19,20-triazatetracyclo[13.5.2.1^{2,6}.0^{18,21}] tricosa-1(20),2(23),3,5,15(22),16,18(21)-heptaen-9-one;
- 8,14-dioxa-10,19,20-triazapentacyclo[13.5.2.1^{2,6}.1^{7,10}.0^{18,21}]tetracosa-1(20),2(24),3,5, 15(22),16,18(21)-heptaen-9-one;
- 13-methyl-8,14-dioxa-10,19,20,23-tetraazatetracyclo[13.5.2.1^{2,6}.0^{18,21}]tricosa-1(20), 2(23),3,5,15(22),16,18(21)-heptaen-9-one;
- 12-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 7-methyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 5-fluoro-4-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-one;
- (7R,13R)-7,13-dimethyl-8,14-dioxa-10,19,20-triazatetracyclo[13.5.2.1^{2,6}.0^{18,21}] tricosa-1(20),2(23),3,5,15(22),16,18(21)-heptaen-9-one;
- (13R)-13-methyl-8,14-dioxa-4,5,10,19,20-pentaazatetracyclo[13.5.2.1^{2,5}.0^{18,21}] tricosa-1(20),2(23),3,15,17,21-hexaen-9-one;
- 8,15-dioxa-4,10,20,21-tetraazapentacyclo[14.5.2.1^{2,6}.1^{10,13}.0^{19,22}]pentacosa-1(21),2(25), 3,5,16(23),17,19(22)-heptaen-9-one;
- 8,14-dioxa-5,10,19,20-tetraazatetracyclo[13.5.2.1^{2,5}.0^{18,21}]tricosa-1(20),2(23),3,15(22),16,18(21)-hexaen-9-one;
- (13S)-4-fluoro-13-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- (13R)-4-fluoro-13-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- (13R)-13-methyl-8,14-dioxa-4,10,19,20-tetraazatetracyclo[13.5.2. 1^{2,6}.0^{18,21}]tricosa-1(20),2(23),3,5,15(22),16,18(21)-heptaen-9-one;
- 6-cyclopropyl-8,14-dioxa-4,5,10,19,20-pentaazatetracyclo[13.5.2.1^{2,5}.0^{18,21}]tricosa-1(20), 2(23),3,15(22),16,18(21)-hexaen-9-one;
- 7-ethyl-8,14-dioxa-10,19,20-triazatetracyclo[13.5.2.1^{2,6}.0^{18,21}]tricosa-1(20),2,4,6(23),15,17,21-heptaen-9-one;
- (13R)-13-methyl-8,14-dioxa-5,10,19,20,23-pentaazatetracyclo[13.5.2.1^{2,6}.0^{18.21}] tricosa-1(20),2,4,6(23), 15,17,21-heptaen-9-one;
- (7R,13R)-4-fluoro-7,13-dimethyl-8,14-dioxa-10,19,20-triazatetracyclo [13.5.2.1^{2,6}.0^{18,21}]tricosa-1(20),2,4,6(23),15,17,21-heptaen-9-one;
- 7-methyl-8,14-dioxa-4,10,19,20-tetraazatetracyclo[13.5.2.1^{2,6}.0^{18,21}]tricosa-1(20),2,4, 6(23),15,17,21-heptaen-9-one;
- (7R)-4-fluoro-7-methyl-8,14-dioxa-10,19,20-triazatetracyclo[13.5.2.1^{2,6}.0^{18,21}] tricosa-1(20),2,4,6(23),15,17,21-heptaen-9-one;
- (7S)-4-fluoro-7-methyl-8,14-dioxa-10,19,20-triazatetracyclo[13.5.2.1^{2,6}.0^{18,21}] tricosa-1(20),2,4,6(23),15,17,21-heptaen-9-one;
- 6-methyl-8,14-dioxa-4,5,10,19,20-pentaazatetracyclo[13.5.2.1^{2,5}.0^{18,21}]tricosa-1(20), 2(23),3,15,17,21-hexaen-9-one;
- 7-methyl-8,14-dioxa-10,19,20,23-tetraazatetracyclo[13.5.2.1^{2,6}.0^{18,21}]tricosa-1(20),2,4, 6(23),15,17,21-heptaen-9-one;
- 6-(propan-2-yl)-8,14-dioxa-4,5,10,19,20-pentaazatetracyclo[13.5.2.1^{2,5}.0^{18,21}] tricosa-1(20),2(23),3,15(22),16,18(21)-hexaen-9-one;
- (13R)-7,13-dimethyl-8,14-dioxa-4,5,10,19,20-pentaazatetracyclo[13.5.2.1^{2,5}.0^{18.21}] tricosa-1(20),2(23),3,15(22),16,18(21)-hexaen-9-one;
- (13R)-13-methyl-8,14-dioxa-10,19,20,23-tetraazatetracyclo[13.5.2.1^{2,6}.0^{18,21}] tricosa-1(20),2,4,6(23),15,17,21-heptaen-9-one;
- (7R)-7-ethyl-8,14-dioxa-10,19,20-triazatetracyclo[13.5.2.1^{2,6}.0^{18,21}]tricosa-1(20),2,4, 6(23),15, 17,21-heptaen-9-one;
- (7S)-7-ethyl-8,14-dioxa-10,19,20-triazatetracyclo[13.5.2.1^{2,6}.0^{18,21}]tricosa-1(20),2,4, 6(23),15,17,21-heptaen-9-one;
- (13R)-13-methyl-8,14-dioxa-5,10,19,20-tetraazatetracyclo[13.5.2.1^{2,6}.0^{18,21}]tricosa-1(20),2,4,6(23),15,17,21-heptaen-9-one;
- 6-(oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-one;
- 4-ethyl-8,14-dioxa-5,10,19,20,23-pentaazatetracyclo[13.5.2.1^{2,5}.0^{18,21}]tricosa-1(20), 2(23),15,17,21-pentaen-9-one;
- (13R)-23-fluoro-13-methyl-8,14-dioxa-10,19,20-triazatetracyclo[13.5.2.1^{2,6}.0^{18,21}] tricosa-1(20),2,4,6(23),15,17,21-heptaen-9-one;
- 9,14-dioxa-4,5,11,19,20-pentaazatetracyclo[13.5.2.1^{2,5}.0^{18,21}]tricosa-1(20),2(23), 3,15,17,21-hexaen-10-one;
- 4-ethyl-8,14-dioxa-5,10,19,20,23-pentaazatetracyclo[13.5.2.1^{2,5}.0^{18,21}]tricosa-1(20), 2(23),3,15,17,21-hexaen-9-one;
- 3,9,15-trioxa-4,11,20,21-tetraazatetracyclo[14.5.2.1^{2,5}.0^{19,22}]tetracosa-1(21),2(24), 4,16,18,22-hexaen-10-one;
- (13R)-16-fluoro-13-methyl-8,14-dioxa-4,10,19,20-tetraazatetracyclo [13.5.2.1^{2,6}.0^{18,21}]tricosa-1(20),2,4,6(23),15,17,21-heptaen-9-one;
- (13R)-4-chloro-13-methyl-8,14-dioxa-10,19,20,23-tetraazatetracyclo [13.5.2.1^{2,6}.0^{18,21}]tricosa-1(20),2(23),3,5,15(22),16,18(21)-heptaen-9-one;
- 8,14-dioxa-2,4,10,19,20-pentaazatetracyclo[13.5.2.1^{2,5}.0^{18,21}]tricosa-1(20),3,5(23), 15(22),16,18(21)-hexaen-9-one;
- (13R)-4-methoxy-13-methyl-8,14-dioxa-10,19,20,23-tetraazatetracyclo [13.5.2.1^{2,6}.0^{18,21}]tricosa-1(20),2,4,6(23),15,17,21-heptaen-9-one;
- (13R)-13-methyl-9-oxo-8,14-dioxa-10,19,20-triazatetracyclo [13.5.2.1^{2,6}.0^{18,21}]tricosa-1(20),2,4,6(23),15,17,21-heptaene-5-carbonitrile;
- (13R)-13-methyl-4-(pyrrolidin-1-yl)-8,14-dioxa-5,10,19,20,23-pentaazatetracyclo [13.5.2.1^{2,6}.0^{18,21}]tricosa-1(20),2,4,6(23),15,17,21-heptaen-9-one;
- (7S,13R)-7,13-dimethyl-8,14-dioxa-5,10,19,20,23-pentaazatetracyclo [13.5.2.1^{2,6}.0^{18,21}]tricosa-1(20),2,4,6(23),15,17,21-heptaen-9-one;
- (7R,13R)-7,13-dimethyl-8,14-dioxa-5,10,19,20,23-pentaazatetracyclo[13.5.2. 1^{2,6}.0^{18,21}]tricosa-1(20),2,4,6(23),15,17,21-heptaen-9-one;
- (13R)-16-fluoro-13-methyl-8,14-dioxa-10,19,20-triazatetracyclo[13.5.2.1^{2,6}.0^{18,21}] tricosa-1(20),2,4,6(23),15,17,21-heptaen-9-one;
- (13R)-13-methyl-8,14-dioxa-4,10,19,20,23-pentaazatetracyclo[13.5.2.1^{2,6}.0^{18,21}] tricosa-1(20),2,4,6(23),15,17,21-heptaen-9-one;
- 8,14-dioxa-4-thia-10,19,20,23-tetraazatetracyclo[13.5.2.1^{2,5}.0^{18,21}]tricosa-1(20),2,5(23), 15,17,21-hexaen-9-one;
- 8,14-dioxa-3-thia-10,19,20,23-tetraazatetracyclo[13.5.2.1^{2,5}.0^{18,21}]tricosa-1(20),2(23),4,15,17,21-hexaen-9-one;
- (7R,13R)-7,13-dimethyl-8,14-dioxa-10,19,20,23-tetraazatetracyclo[13.5.2. 1^{2,6}.0^{18,21}]tricosa-1(20),2,4,6(23),15,17,21-heptaen-9-one;
- (13R)-4-[(3R)-3-methoxypyrrolidin-1-yl]-13-methyl-8,14-dioxa-5,10,19,20,23-pentaazatetracyclo[13.5.2.1^{2,6}.0^{18,21}]tricosa-1(20),2,4,6(23),15,17,21-heptaen-9-one;
- (13R)-16-chloro-13-methyl-8,14-dioxa-10,19,20-triazatetracyclo[13.5.2.1^{2,6}.0^{18,21}] tricosa-1(20),2, 4,6(23),15,17,21-heptaen-9-one;
- (13R)-13,16-dimethyl-8,14-dioxa-10,19,20-triazatetracyclo[13.5.2.1^{2,6}.0^{18,21}] tricosa-1(20),2,4, 6(23),15,17,21-heptaen-9-one;
- (13R)-13-methyl-8,14-dioxa-3,10,19,20,23-pentaazatetracyclo[13.5.2.1^{2,6}.0^{18,21}] tricosa-1(20),2,4,6(23),15,17,21-heptaen-9-one;
- 8-oxa-10,14,19,20-tetraazatetracyclo[13.5.2.1^{2,6}.0^{18,21}]tricosa-1(20),2(23),3,5, 15(22),16,18(21)-heptaen-9-one hydrochloride;
- 8-oxa-10,19,20-triazatetracyclo[13.5.2.1^{2,6}.0^{18,21}]tricosa-1(20),2(23),3,5,15(22),16, 18(21)-heptaen-9-one;
- (13R)-5-methoxy-13-methyl-8,14-dioxa-4,10,19,20-tetraazatetracyclo [13.5.2.1^{2,6}.0^{18,21}]tricosa-1(20),2,4,6(23),15,17,21-heptaen-9-one;
- (13R)-13-methyl-8,14-dioxa-4,10,19,20-tetraazatetracyclo[13.5.2.1^{2,6}.0^{18,21}]tricosa-1(20),2, 6(23),15,17,21-hexaene-5,9-dione;
- 4-methyl-8,14-dioxa-3,4,10,19,20-pentaazatetracyclo[13.5.2.1^{2,5}.0^{18,21}]tricosa-1(20),2, 5(23),15(22),16,18(21)-hexaen-9-one;
- (13R)-16-fluoro-13-methyl-8,14-dioxa-10,19,20,23-tetraazatetracyclo [13.5.2.1^{2,6}.0^{18,21}]tricosa-1(20),2,4,6(23),15,17,21-heptaen-9-one;
- 7,13-dioxa-4-thia-9,18,19,22-tetraazatetracyclo[12.5.2.1^{2,5}.0^{17,20}]docosa-1(19),2,5(22), 14(21), 15, 17(20)-hexaen-8-one;
- (13R)-4,13-dimethyl-8,14-dioxa-5,10,19,20,23-pentaazatetracyclo[13.5.2.1^{2,6}.0^{18,21}] tricosa-1(20),2(23),3,5,15(22),16,18(21)-heptaen-9-one;
- 8,14-dioxa-23-thia-4,10,19,20-tetraazatetracyclo[13.5.2.1^{2,5}.0^{18,21}]tricosa-1(20),2,4,15(22), 16,18(21)-hexaen-9-one;
- (7S,13R)-7,13-dimethyl-8,14-dioxa-10,19,20,23-tetraazatetracyclo [13.5.2.1^{2,6}.0^{18,21}]tricosa-1(20),2(23),3,5,15(22),16,18(21)-heptaen-9-one;
- (13R)-13-methyl-9-oxo-8,14-dioxa-5,10,19,20-tetraazatetracyclo[13.5.2.1^{2,5}.0^{18,21}] tricosa-1(20), 2(23),3,15(22),16,18(21)-hexaene-4-carbonitrile;
- 12,12-difluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- (13R)-17-fluoro-13-methyl-8,14-dioxa-10,19,20-triazatetracyclo[13.5.2.1^{2,6}.0^{18,21}] tricosa-1(20),2(23),3,5,15(22),16,18(21)-heptaen-9-one;
- (7S,13R)-7,13-dimethyl-8,14-dioxa-4,10,19,20,23-pentaazatetracyclo [13.5.2.1^{2,6}.0^{18,21}]tricosa-1(20),2(23),3,5,15(22),16,18(21)-heptaen-9-one;
- (7R,13R)-7,13-dimethyl-8,14-dioxa-4,10,19,20,23-pentaazatetracyclo [13.5.2.1^{2,6}.0^{18,21}]tricosa-1(20),2(23),3,5,15(22),16,18(21)-heptaen-9-one;
- (13S)-13-methyl-8,14-dioxa-4,10,19,20,23-pentaazatetracyclo[13.5.2.1^{2,6}.0^{18,21}] tricosa-1(20),2(23),3,5,15(22),16,18(21)-heptaen-9-one;
- (13R)-13-methyl-8,14-dioxa-10,19,20,22-tetraazatetracyclo[13.5.2.1^{2,6}.0^{18,21}] tricosa-1(20), 2(23),3,5,15,17,21-heptaen-9-one ;
- (12R)-4,12-dimethyl-7,13-dioxa-4,9,18,19,22-pentaazatetracyclo[12.5.2.1^{2,5}.0^{17,20}] docosa-1(19),2,5(22),14(21),15,17(20)-hexaen-8-one;
- (13R)-13-methyl-8,14-dioxa-4,5,10,19,20,23-hexaazatetracyclo[13.5.2.1^{2,5}.0^{18,21}] tricosa-1(20),2(23),3,15,17,21-hexaen-9-one;
- (13R)-13-methyl-8,14-dioxa-23-thia-4,10,19,20-tetraazatetracyclo[13.5.2.1^{2,5}.0^{18,21}] tricosa-1(20),2,4,15,17,21-hexaen-9-one ;
- (13R)-4,13-dimethyl-8,14-dioxa-4,10,19,20,23-pentaazatetracyclo[13.5.2.1^{2,5}.0^{18,21}] tricosa-1(20),2,5(23),15(22),16,18(21)-hexaen-9-one;
- (13R)-13-methyl-8,14-dioxa-10,16,19,20-tetraazatetracyclo[13.5.2.1^{2,6}.0^{18,21}] tricosa-1(20),2(23),3,5,15(22),16,18(21)-heptaen-9-one;
- 14-methyl-8-oxa-10,14,19,20-tetraazatetracyclo[13.5.2.1^{2,6}.0^{18,21}]tricosa-1(20),2(23),3,5, 15,17,21-heptaen-9-one;
- (13R)-13-methyl-8,14-dioxa-4,10,19,20,22-pentaazatetracyclo[13.5.2.1^{2,6}.0^{18,21}] tricosa-1(20),2(23),3,5,15,17,21-heptaen-9-one;
- (13R)-13-methyl-8,14-dioxa-10,17,19,20-tetraazatetracyclo[13.5.2.1^{2,6}.0^{18,21}] tricosa-1(20),2(23),3,5,15(22),16,18(21)-heptaen-9-one;
- 8,14-dioxa-4,5,10,19,20,23-hexaazatetracyclo[13.5.2.1^{2,5}.0^{18,21}]tricosa-1(20),2(23),3, 15(22),16,18(21)-hexaen-9-one;
- 12,12-difluoro-8,14-dioxa-4,5,10,19,20,23-hexaazatetracyclo[13.5.2.1^{2,5}.0^{18,21}] tricosa-1(20),2(23),3,15(22),16,18(21)-hexaen-9-one;
- (12R)-12-fluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- (12S)-12-fluoro-8,14-dioxa-10,19,20-triazatetracyclo[13.5.2.1^{2,6}.0^{18,21}]tricosa-1(20),2(23), 3,5,15(22),16,18(21)-heptaen-9-one;
- 12,12-difluoro-8,14-dioxa-4,10,19,20,23-pentaazatetracyclo[13.5.2.1^{2,6}.0^{18,21}] tricosa-1(20),2(23),3,5,15(22),16,18(21)-heptaen-9-one;
- (12S)-12-fluoro-8,14-dioxa-4,10,19,20,23-pentaazatetracyclo[13.5.2.1^{2,6}.0^{18,21}] tricosa-1(20),2(23),3,5,15,17,21-heptaen-9-one;
- (12R)-12-fluoro-8,14-dioxa-4,10,19,20,23-pentaazatetracyclo[13.5.2.1^{2,6}.0^{18,21}] tricosa-1(20),2(23),3,5,15,17,21-heptaen-9-one;
- (12S)-12-fluoro-8,14-dioxa-4,5,10,19,20,23-hexaazatetracyclo[13.5.2.1^{2,5}.0^{18,21}] tricosa-1(20),2(23),3,15,17,21-hexaen-9-one;
- (12R)-12-fluoro-8,14-dioxa-4,5,10,19,20,23-hexaazatetracyclo[13.5.2.1^{2,5}.0^{18,21}] tricosa-1(20),2(23),3,15,17,21-hexaen-9-one; or
- 8',14'-dioxa-10',19',20'-triazaspiro[cyclopropane-1,13'-tetracyclo[13.5.2.1^{2,6}.0^{18,21}] tricosane]-1'(20'),2'(23'),3',5',15'(22'),16',18'(21')-heptaen-9'-one.

31. Pharmaceutical composition comprising a compound of formula (I) according to any one of claims 1 to 30 or an addition salt thereof with a pharmaceutically acceptable acid or base in combination with one or more pharmaceutically acceptable excipients.

32. Pharmaceutical composition according to claim 31 for use in the treatment of neurological diseases, inflammatory diseases, bacterial, viral and parasitic infections, cardiovascular diseases, autoimmune diseases, cancers and endosomal-lysosomal disorders selected from Niemann-Pick Type A, B or C disease, Gaucher's disease, Krabbe's disease, Fabry's disease and disorders with mitochondrial deficits.

33. Pharmaceutical composition for use according to claim 32 wherein the neurological disease is selected from Parkinson's disease, Alzheimer's disease, amyotrophic lateral sclerosis (ALS), dementia, diabetic neuropathy, age related memory disfunction, mild cognitive impairment, argyrophilic grain disease, Pick's disease, epilepsy, tauopathies such as progressive supranuclear palsy and corticobasal degeneration, other synucleinopathies such as multiple system atrophy, frontotemporal dementia, inherited frontotemporal dementia and parkinsonism linked to chromosome 17 (FTDP-17), withdrawal symptoms/relapse associated with drug addiction, L-Dopa induced dyskinesia, ischemic stroke, traumatic brain injury, spinal cord injury and multiple sclerosis.

34. Pharmaceutical composition for use according to claim 33 wherein the neurological disease is Parkinson's disease or Alzheimer's disease.

35. Pharmaceutical composition for use according to claim 32 wherein the inflammatory disease is selected from vasculitis, pulmonary diseases such as chronic obstructive pulmonary disease, idiopathic pulmonary fibrosis, inflammatory myopathies, and ankylosing spondylitis.

36. Pharmaceutical composition for use according to claim 32 wherein the autoimmune disease is selected from Crohn's disease, inflammatory bowel disease, rheumatoid arthritis, ulcerative colitis, lupus, autoimmune hemolytic anemia, pure red cell aplasia, idiopathic thrombocytopenic purpura, type I diabetes mellitus, obesity, Evans syndrome, bullous skin disorders, Sjogren's syndrome, Devic's disease and leprosy.

37. Pharmaceutical composition for use according to claim 32 wherein the cancer is selected from thyroid cancer, renal cancer, breast cancer, hormone-related cancer, adeno-and squamous lung cancer, non-small-cell lung cancer, colon cancer, prostate cancers, skin cancers, leukemias and lymphomas.

38. Pharmaceutical composition for use according to claim 32 wherein the cardiovascular disease is stroke.

39. Pharmaceutical composition for use according to claim 32 wherein the bacterial or viral infections are selected from leprosy, tuberculosis, SARS-CoV, MERS-CoV and SARS-CoV-2, HIV, West Nile virus and chikungunya virus.

40. Compound of formula (I) according to any one of claims 1 to 30, or an addition salt thereof with a pharmaceutically acceptable acid or base, for use in the treatment of Parkinson's disease, Alzheimer's disease, amyotrophic lateral sclerosis (ALS), dementia, diabetic neuropathy, age related memory disfunction, mild cognitive impairment, argyrophilic grain disease, Pick's disease, epilepsy, tauopathies such as progressive supranuclear palsy and corticobasal degeneration, other synucleinopathies, frontotemporal dementia, inherited frontotemporal dementia and parkinsonism linked to chromosome 17 (FTDP-17), withdrawal symptoms/ relapse associated with drug addiction, L-Dopa induced dyskinesia, ischemic stroke, traumatic brain injury, spinal cord injury, multiple sclerosis, Niemann-Pick Type A, B or C disease, Gaucher's disease, Krabbe's disease, Fabry's disease, disorders with mitochondrial deficits, Crohn's disease, inflammatory bowel disease, rheumatoid arthritis, ulcerative colitis, lupus, autoimmune hemolytic anemia, pure red cell aplasia, idiopathic thrombocytopenic purpura, type I diabetes mellitus, obesity, Evans syndrome, bullous skin disorders, Sjogren's syndrome, Devic's disease, leprosy, thyroid cancer, renal cancer (including papillary renal), breast cancer, hormone-related cancer, adeno-and squamous lung cancer, non-small-cell lung cancer, colon cancer, prostate cancers, skin cancers, leukemias (including acute myelogenous leukemia), lymphomas, stroke, leprosy, tuberculosis, and SARS-CoV, MERS-CoV, SARS-CoV-2, HIV, West Nile virus and chikungunya virus infections.

## Patentansprüche

1. Verbindung der Formel (I): 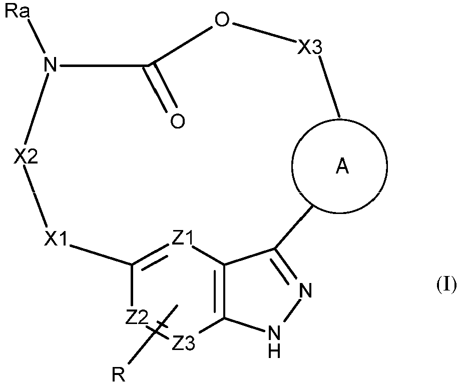 wobei:
◆ R ein Wasserstoffatom, ein Halogenatom oder eine Alkylgruppe darstellt,
◆ Z1, Z2, Z3 unabhängig voneinander jeweils ein Kohlenstoff- oder ein Stickstoffatom darstellen, wobei der 6-gliedrige Ring, der Z1, Z2 und Z3 enthält, 0, 1 oder 2 Stickstoffatome aufweisen kann,
◆ -X1- abwesend ist oder -O-, -S- oder -N(R'a)- darstellt, wobei R'a ein Wasserstoffatom oder eine Alkylgruppe darstellt,
◆ -X2- eine Alkandiylgruppe darstellt, die optional mit einem oder mehreren Substituenten substituiert ist, die gleich oder unterschiedlich sind und aus Halogenatomen, Polyhalogenalkylgruppen, Alkoxygruppen, Hydroxygruppen, Aminogruppen, Alkylaminogruppen, Dialkylaminogruppen und Cyanogruppen ausgewählt sind,
wobei das Kohlenstoffatom in der Alpha-Position von -N(Ra) und das Kohlenstoffatom in der Alpha-Position von -X1-, wenn -X1- für -O-, -S- oder - N(R'a)- steht, nicht durch ein Sauerstoff- oder Stickstoff-Heteroatom substituiert sein können,
◆ -X3- eine Alkandiylgruppe darstellt, die optional mit einem oder mehreren Substituenten substituiert ist, die gleich oder unterschiedlich sind, die ausgewählt sind aus Halogenatomen, Polyhalogenalkylgruppen, Alkoxygruppen, Hydroxygruppen, Aminogruppen, Alkylaminogruppen, Dialkylaminogruppen, Cyanogruppen, Cycloalkylgruppen und Heterocycloalkylgruppen,
wobei das Kohlenstoffatom in der Alpha-Position von -O- und das Kohlenstoffatom in der Alpha-Position von A1, wenn A1 ein Stickstoffatom darstellt, nicht durch ein Sauerstoff- oder Stickstoffheteroatom substituiert werden kann,
◆ Ra ein Wasserstoffatom oder eine Alkylgruppe darstellt,
wobei ein Kohlenstoffatom von Ra mit einem Kohlenstoffatom von -X2- oder mit einem Kohlenstoffatom von -X3- verbunden sein kann, wenn Ra eine Alkylgruppe darstellt, um eine zyklische Einheit mit 5 oder 6 Ringgliedern zu bilden,
◆ A steht für
- eine aromatische oder teilweise hydrierte cyclische Gruppe der Formel (a): 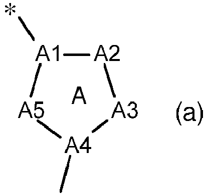 wobei
✔ A1, A4 jeweils unabhängig voneinander ein Kohlenstoffatom oder ein Stickstoffatom darstellen,
✔ A2, A3, A5 jeweils unabhängig voneinander ein Kohlenstoffatom, ein Sauerstoffatom, ein Schwefelatom oder ein Stickstoffatom darstellen,
wobei A1, A2, A3, A4 und A5 nicht gleichzeitig ein Heteroatom darstellen können,
- oder eine aromatische oder teilweise hydrierte cyclische Gruppe der Formel (b): 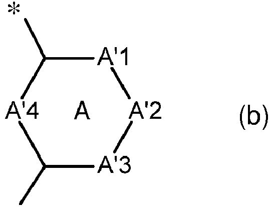
wobei A'1, A'2, A'3, A'4 jeweils unabhängig voneinander ein Kohlenstoffatom oder ein Stickstoffatom darstellen,
wobei * bedeutet, dass die Bindung an X3 gekoppelt ist,
wobei die aromatische oder teilweise hydrierte cyclische Gruppe A optional mit einem oder mehreren gleichen oder unterschiedlichen Substituenten substituiert ist, ausgewählt aus Halogenatomen, Alkylgruppen, Alkoxygruppen, Hydroxygruppen, Oxogruppen, Alkoxyalkylgruppen, Alkoxyalkoxygruppen, Polyhalogenalkylgruppe, Polyhalogenalkoxygruppe, Heterocycloalkylgruppe, Heterocycloalkylalkylgruppe, (Alkoxyalkyl)(alkyl)aminogruppen, Aminogruppen, Alkylaminogruppen, Dialkylaminogruppen, Cycloalkylgruppen, (Heterocycloalkyl)(alkyl)aminogruppen, Dialkylaminoalkylgruppen, Heterocycloalkylalkoxygruppen, Cyanogruppen und Cyanalkylgruppen,
wobei die so definierte heterocyclische Alkyl- und Cycloalkylgruppe optional durch einen oder mehrere Substituenten substituiert sein kann, ausgewählt aus Alkylgruppen, Halogenatomen, Polyhalogenalkylgruppen, Polyhalogenalkoxygruppen, Alkoxygruppen, Alkoxyalkylgruppen, Hydroxygruppen, Cyanogruppen und Oxogruppen,
ihren Enantiomeren, Diastereomeren, Tautomeren, racemischen Formen, Hydraten, Solvaten, N-Oxiden, Isotopen, deuterierten Derivaten und Additionssalzen davon mit einer pharmazeutisch verträglichen Säure oder einer Basis.

2. Verbindung nach Anspruch 1, wobei R ein Wasserstoffatom darstellt.

3. Verbindung nach Anspruch 1, wobei R ein Halogenatom darstellt.

4. Verbindung nach einem der Ansprüche 1 bis 3, wobei Z1, Z2 und Z3 gleichzeitig ein Kohlenstoffatom darstellen.

5. Verbindung nach einem der Ansprüche 1 bis 3, wobei eines von Z1 oder Z2 ein Stickstoffatom darstellt und Z3 ein Kohlenstoffatom darstellt.

6. Verbindung nach einem der Ansprüche 1 bis 5, wobei -X1- -O- darstellt.

7. Verbindung nach einem der Ansprüche 1 bis 6, wobei -X2- eine lineare oder verzweigte Alkandiylgruppe darstellt, die 2, 3, 4 oder 5 Kohlenstoffatome aufweist.

8. Verbindung nach Anspruch 7, wobei -(CH₂)₃-, -CH(CH₃)-(CH₂)₂-, -CH₂-CHF-CH₂-, -CH₂-CF₂-CH₂- oder -(CH₂)₂-CH(CH₃)- darstellt.

9. Verbindung nach einem der Ansprüche 1 bis 8, wobei Ra ein Wasserstoffatom ist.

10. Verbindung nach einem der Ansprüche 1 bis 9, wobei -X3- eine lineare oder verzweigte Alkandiylgruppe darstellt, die 1, 2, 3, 4 oder 5 Kohlenstoffatome aufweist.

11. Verbindung nach Anspruch 10, wobei -X3- -(CH₂)₂-, -CH₂- oder - CH(CH₃)- darstellt.

12. Verbindung nach einem der Ansprüche 1 bis 11, wobei A eine Gruppe der Formel (b) darstellt: 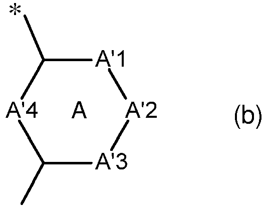 wobei A'1, A'2, A'3, A'4 und * wie in Anspruch 1 definiert sind.

13. Verbindung nach Anspruch 12, wobei A Folgendes darstellt 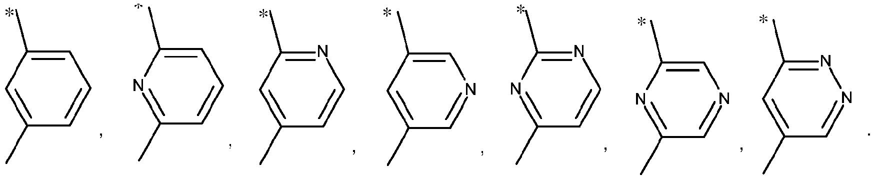 wobei solche definierten A-Gruppen nicht substituiert oder optional substituiert sind.

14. Verbindung nach Anspruch 12, wobei A eine Phenylgruppe darstellt.

15. Verbindung nach Anspruch 12, wobei A eine Pyridinylgruppe darstellt.

16. Verbindung nach Anspruch 12, wobei A eine Pyrazinylgruppe darstellt.

17. Verbindung nach einem der Ansprüche 1 bis 11, wobei A eine Gruppe der Formel (a) darstellt: 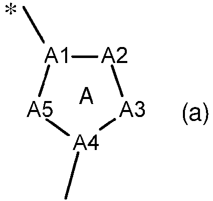 wobei A1, A2, A3, A4, A5 und * wie in Anspruch 1 definiert sind.

18. Verbindung nach Anspruch 17, wobei A Folgendes darstellt 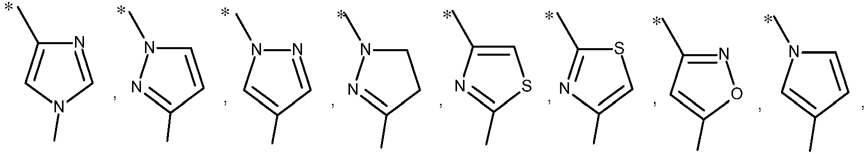 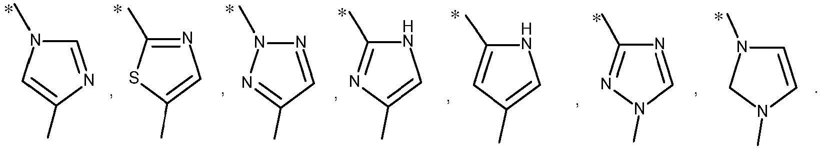 wobei solche definierten A-Gruppen nicht substituiert oder optional substituiert sind.

19. Verbindung nach Anspruch 17, wobei A eine Triazolylgruppe darstellt.

20. Verbindung nach Anspruch 17, wobei A eine Pyrazolylgruppe darstellt.

21. Verbindung nach den Ansprüchen 12 bis 20, wobei A nicht substituiert ist.

22. Verbindung nach den Ansprüchen 12 bis 20, wobei A durch eine oder mehrere Gruppen substituiert ist, ausgewählt aus Halogenatomen, Cyanogruppen, Cyanalkylgruppen, Oxogruppen, Alkoxygruppen, Alkylgruppen, Cycloalkylgruppen und Heterocycloalkylgruppen.

23. Verbindung nach Anspruch 1, die eine Verbindung der Formel (I-a) ist: 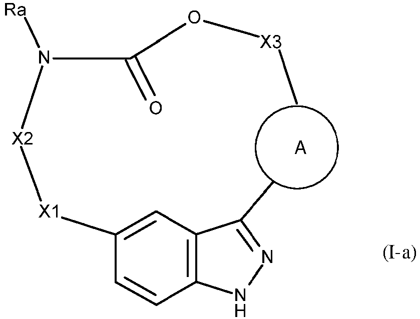 wobei X1, X2, X3, Ra und A wie in Anspruch 1 definiert sind.

24. Verbindung nach Anspruch 23, die eine Verbindung der Formel (I-b) ist: 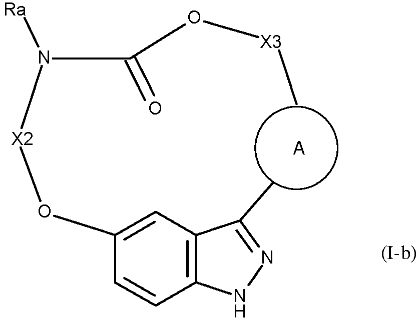 wobei X2, X3, Ra und A wie in Anspruch 1 definiert sind.

25. Verbindung nach Anspruch 23, die eine Verbindung der Formel (I-c) oder (I-c') ist: 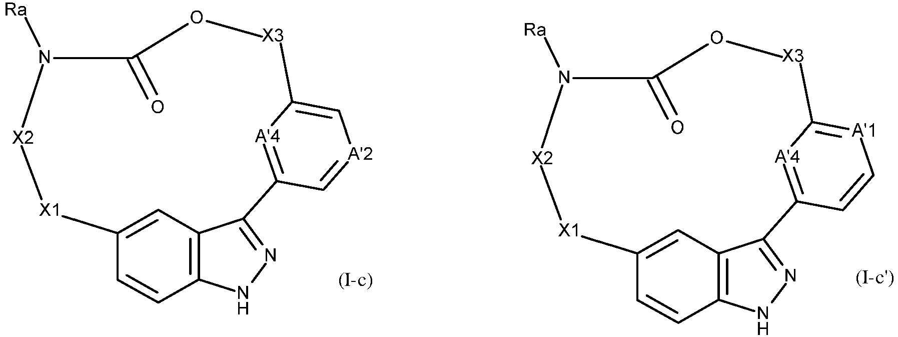 wobei X1, X2, X3, Ra, A'1, A'2 und A'4 wie in Anspruch 1 definiert sind.

26. Verbindung nach Anspruch 23 oder 25, die eine Verbindung der Formel (I-d) oder (I-d') ist: 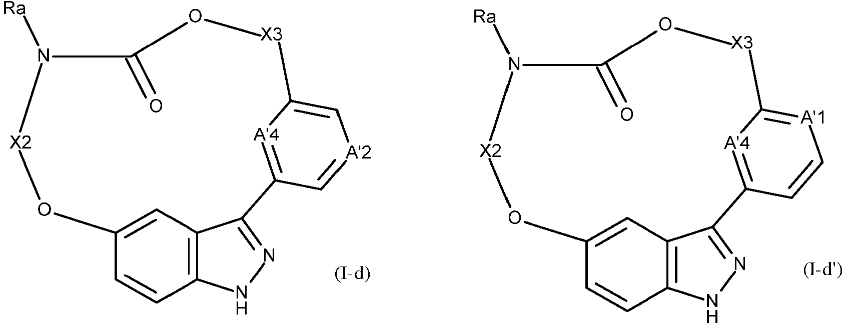 wobei X2, X3, Ra, A'1, A'2 und A'4 wie für Formel (I) definiert sind.

27. Verbindung nach Anspruch 23, die eine Verbindung der Formel (I-e) ist: 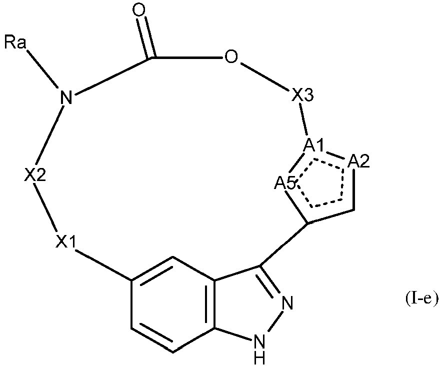 wobei X1, X2, X3, Ra, A1, A2 und A5 wie für Formel (I) definiert sind.

28. Verbindung nach Anspruch 23 oder 27, die eine Verbindung der Formel (I-f) ist: 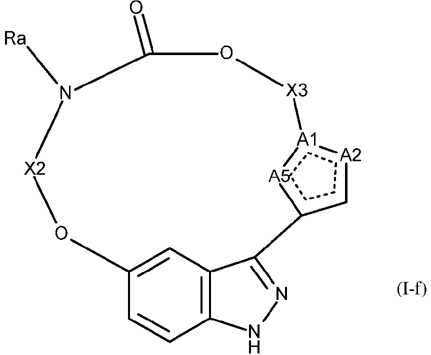 wobei X2, X3, Ra, A1, A2 und A5 wie für Formel (I) definiert sind.

29. Verbindung nach Anspruch 23, 25 oder 27, wobei die Kette -X1-X2-N(Ra)-C(O)O-X3- O-(CH₂)₃-NHC(O)O-CH₂-, -O-CH(CH₃)-(CH₂)₂-NHC(O)O-CH₂-,
-O-CH₂-CHF-CH₂-NHC(O)O-CH₂-, -O-CH₂-CF₂-CH₂-NHC(O)O-CH₂-,
-O-CH(CH₃)-(CH₂)₂-NHC(O)O-(CH₂)₂- oder
-O-CH(CH₃)-(CH₂)₂-NH-C(O)O-CH(CH₃)- darstellt.

30. Verbindungen nach Anspruch 1, die Folgendes sind:
- 8,14-Dioxa-4,10,19,20-tetraazatetracyclo[13.5.2.1^{2,6}.0^{18,21}]tricosa-1(20),2,4,6(23),15,17,21-heptaen-9-on;
- 10-Methyl-8,14-dioxa-4,10,19,20-tetraazatetracyclo[13.5.2.1^{2,6}.0^{18,21}]tricosa-1(20),2,4, 6(23),15,17,21-heptaen-9-on;
- 4-Fluor-8,14-dioxa-10,19,20-triazatetracyclo[ 13.5.2.1^{2,6}.0^{18,21}]tricosa-1(20),2,4,6(23), 15,17,21-heptaen-9-on;
- 8,14-Dioxa-10,19,20,23-tetraazatetracyclo[13.5.2.1^{2,6}.0^{18,21}]tricosa-1(20),2,4,6(23), 15,17,21-heptaen-9-on;
- 8,14-Dioxa-10,19,20-triazatetracyclo[13.5.2.1^{2,6}.0^{18,21}]tricosa-1(20),2,4,6(23),15,17,21-heptaen-9-on;
- 10-(Propan-2-yl)-8,14-dioxa-4,10,19,20-tetraazatetracyclo[13.5.2.1^{2,6}.0^{18,21}]tricosa-1(20),2,4,6(23),15,17,21-heptaen-9-on;
- 8,14-Dioxa-5,10,19,20-tetraazatetracyclo[13.5.2.1^{2,6}.0^{18,21}]tricosa-1 (20),2,4,6(23), 15,17,21-heptaen-9-on;
- 4-Methoxy-8,14-dioxa-10,19,20-triazatetracyclo[13.5.2.1^{2,6}.0^{18,21}]tricosa-1(20),2,4, 6(23),15,17,21-heptaen-9-on;
- 4-Brom-8,14-dioxa-10,19,20-triazatetracyclo[13.5.2.1^{2,6}.0^{18,21}]tricosa-1(20),2,4,6(23), 15,17,21-heptaen-9-on;
- 5-Fluor-8,14-dioxa-10,19,20-triazatetracyclo[13.5.2.1^{2,6}.0^{18,21}]tricosa-1(20),2,4,6(23), 15,17,21-heptaen-9-on;
- 5-Methyl-8,14-dioxa-10,19,20-triazatetracyclo[13.5.2.1^{2,6}.0^{18,21}]tricosa-1(20),2,4,6(23), 15,17,21-heptaen-9-on;
- 4-(Pyrrolidin-1-yl)-8,14-dioxa-10,19,20-triazatetracyclo[13.5.2.1^{2,6}.0^{18,21}]tricosa-1(20), 2,4,6(23),15,17,21-heptaen-9-on;
- 4-[4-(Propan-2-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-on;
- 4-{2-Oxa-6-azaspiro[3.4]octan-6-yl}-8,14-dioxa-10,19,20-triazatetracyclo [13.5.2.1^{2,6}.0^{18,21}]tricosa-1(20),2,4,6(23),15,17,21-heptaen-9-on;
- 4-[4-(Oxetan-3-yl)piperazin-1-yl]-8,14-dioxa-10,19,20-triazatetracyclo [13.5.2.1^{2,6}.0^{18,21}]tricosa-1(20),2,4,6(23),15,17,21-heptaen-9-on;
- 4-(Morpholin-4-yl)-8,14-dioxa-10,19,20-triazatetracyclo[13.5.2.1^{2,6}.0^{18,21}]tricosa-1(20), 2,4,6(23),15,17,21-heptaen-9-on;
- 4-[(2R,6S)-2,6-Dimethylmorpholin-4-yl]-8,14-dioxa-10,19,20-triazatetracyclo [13.5.2.1^{2,6}.0^{18,21}]tricosa-1(20),2,4,6(23),15,17,21-heptaen-9-on;
- 4-Methyl-8,14-dioxa-10,19,20-triazatetracyclo[13.5.2.1^{2,6}.0^{18,21}]tricosa-1(20),2,4,6(23), 15,17,21-heptaen-9-on;
- 5-Methoxy-8,14-dioxa-10,19,20-triazatetracyclo[ 13.5.2.12,6.0^{18,21}]tricosa-1 (20),2,4, 6(23),15, 17,21-heptaen-9-on;
- 4-(4,4-Difluoropiperidin-1-yl)-8,14-dioxa-10,19,20-triazatetracyclo [13.5.2. 1^{2,6}.0^{18,21}]tricosa-1(20),2,4,6(23), 15,17,21 -heptaen-9-on;
- 4-(3,3-Difluoropyrrolidin-1-yl)-8,14-dioxa-10,19,20-triazatetracyclo [13.5.2.1^{2,6}.0^{18,21}]tricosa-1(20),2,4,6(23),15,17,21-heptaen-9-on;
- 7-Methyl-8,14-dioxa-10,19,20-triazatetracyclo[13.5.2.1^{2,6}.0^{18,21}]tricosa-1(20),2,4,6(23),15,17,21-heptaen-9-on;
- 4-[4-(2-Methoxyethyl)piperidin-1-yl] -8,14-dioxa-10,19,20-triazatetracyclo [13.5.2.1^{2,6}.0^{18,21}]tricosa -1(20),2,4,6(23),15,17,21-heptaen-9-on,
- 9,14-Dioxa-11,19,20-triazatetracyclo[13.5.2.1^{2,6}.0^{18,21}] tricosa-1 (20),2,4,6(23),15,17,21-heptaen-10-on;
- 4-[(3R)-3-Hydroxypyrrolidin-1-yl]-8,14-dioxa-10,19,20-triazatetracyclo [13.5.2.1^{2,6}.0^{18,21}]tricosa-1(20),2,4,6(23),15,17,21-heptaen-9-on;
- 4-[(2-Methoxyethyl)(methyl)amino]-8,14-dioxa-10,19,20-triazatetracyclo [13.5.2.1^{2,6}.0^{18,21}]tricosa-1(20),2,4,6(23),15,17,21-heptaen-9-on;
- 4-Chlor-8,14-dioxa-10,19,20-triazatetracyclo[13.5.2.1^{2,6}.0^{18,21}]tricosa-1(20),2,4,6(23), 15,17,21-heptaen-9-on;
- 4-Fluor-5-methyl-8,14-dioxa-10,19,20-triazatetracyclo[13.5.2.1^{2,6}.0^{18,21}]tricosa-1(20),2, 4,6(23),15,17,21-heptaen-9-on;
- 4,5-Difluor-8,14-dioxa-10,19,20-triazatetracyclo[13.5.2.1^{2,6}.0^{18,21}]tricosa-1(20),2,4, 6(23),15,17,21-heptaen-9-on;
- 5-Brom-8,14-dioxa-10,19,20-triazatetracyclo[13.5.2.1^{2,6}.0^{18,21}]tricosa-1(20),2,4,6(23),15,17,21-heptaen-9-on;
- 4-(4-Methylpiperazin-1-yl)-8,14-dioxa-10,19,20-triazatetracyclo[13.5.2.1^{2,6}.0^{18,21}] tricosa-1(20),2,4,6(23), 15,17,21 -heptaen-9-on;
- 4-(3-Methoxyazetidin-1-yl)-8,14-dioxa-10,19,20-triazatetracyclo[13.5.2.1^{2,6}.0^{18,21}] tricosa-1(20),2,4,6(23), 15,17,21 -heptaen-9-on;
- 1-{9-Oxo-8,14-dioxa-10,19,20-triazatetracyclo[13.5.2.1^{2,6}.0^{18,21}]tricosa-1 (20),2,4,6(23), 15,17,21-heptaen-4-yl}piperidin-4-carbonitril;
- 4-[4-(Pyrrolidin-1-yl)piperidin-1-yl] -8,14-dioxa-10,19,20-triazatetracyclo [13.5.2.1^{2,6}.0^{18,21}]tricosa-1(20),2,4,6(23),15,17,21-heptaen-9-on;
- 4-(Azetidin-1-yl)-8,14-dioxa-10,19,20-triazatetracyclo[13.5.2.1^{2,6}.0^{18,21}]tricosa-1(20),2, 4,6(23),15,17,21-heptaen-9-on;
- 4-(Piperidin-1-yl)-8,14-dioxa-10,19,20-triazatetracyclo[13.5.2.1^{2,6}.0^{18,21}]tricosa-1(20),2, 4,6(23),15,17,21-heptaen-9-on;
- 4-(2,5-Dihydrofuran-3-yl)-8,14-dioxa-10,19,20-triazatetracyclo[13.5.2.1^{2,6}.0^{18,21}] tricosa-1 (20),2,4,6(23), 15,17,21-heptaen-9-on;
- 4-[4-(Morpholin-4-yl)piperidin-1-yl]-8,14-dioxa-10,19,20-triazatetracyclo [13.5.2.1^{2,6}.0^{18,21}]tricosa-1(20),2,4,6(23),15,17,21-heptaen-9-on;
- 4-(1-Methyl-1,2,3,6-tetrahydropyridin-4-yl)-8,14-dioxa-10,19,20-triazatetracyclo [13.5.2.1^{2,6}.0^{18,21}] tricosa-1 (20),2,4,6(23),15,17,21-heptaen-9-on;
- 4-[(2S,5S)-2,5-Dimethylmorpholin-4-yl]-8,14-dioxa-10,19,20-triazatetracyclo [13.5.2.1^{2,6}.0^{18,21}] tricosa-1(20),2,4,6(23),15,17,21-heptaen-9-on;
- 4-[(Morpholin-4-yl)methyl]-8,14-dioxa-10,19,20-triazatetracyclo[13.5.2.1^{2,6}.0^{18,21}] tricosa-1(20),2,4,6(23),15,17,21 -heptaen-9-on;
- 4-[(Pyrrolidin-1-yl)methyl]-8,14-dioxa-10,19,20-triazatetracyclo[13.5.2.1^{2,6}.0^{18,21}] tricosa-1(20),2,4,6(23),15,17,21 -heptaen-9-on;
- 4-[(Piperidin-1-yl)methyl]-8,14-dioxa-10,19,20-triazatetracyclo[13.5.2.1^{2,6}.0^{18,21}] tricosa-1(20),2,4,6(23),15,17,21 -heptaen-9-on;
- 4-[(4-Methylpiperazin-1-yl)methyl] -8,14-dioxa-10,19,20-triazatetracyclo [13.5.2.1^{2,6}.0^{18,21}]tricosa-1(20),2,4,6(23),15,17,21-heptaen-9-on;
- 5-(Morpholin-4-yl)-8,14-dioxa-10,19,20-triazatetracyclo[13.5.2.12^{2,6}.0^{18,21}]tricosa-1(20),2,4,6(23),15,17,21-heptaen-9-on;
- 4-[4-(2-Methoxyethyl)piperazin-1-yl]-8,14-dioxa-10,19,20-triazatetracyclo [13.5.2.1^{2,6}.0^{18,21}]tricosa-1(20),2,4,6(23),15,17,21-heptaen-9-on;
- 4-(Diethylamino)-8,14-dioxa-10,19,20-triazatetracyclo[13.5.2.1^{2,6}.0^{18,21}]tricosa-1(20),2, 4,6(23),15,17,21-heptaen-9-on;
- 4-Cyclopropyl-8,14-dioxa-10,19,20-triazatetracyclo[13.5.2.1^{2,6}.0^{18,21}]tricosa-1(20),2, 4,6(23),15,17,21-heptaen-9-on;
- 5-(4-Methylpiperazin-1-yl)-8,14-dioxa-10,19,20-triazatetracyclo[13.5.2.1^{2,6}.0^{18,21}] tricosa-1(20),2,4,6(23),15,17,21 -heptaen-9-on;
- 13-Methyl-8,14-dioxa-10,19,20-triazatetracyclo[13.5.2.1^{2,6}.0^{18,21}]tricosa-1(20),2,4,6(23),15,17,21 -heptaen-9-on,
- 8,14-Dioxa-4,5,10,19,20-pentaazatetracyclo[13.5.2.1^{2,5}.0^{18,21}]tricosa-1(20),2(23),3, 15(22),16,18(21)-hexaen-9-on;
- 4-[Methyl(oxetan-3-yl)amino]-8,14-dioxa-10,19,20-triazatetracyclo[13.5.2. 12,6.0^{18,21}]tricosa-1(20),2,4,6(23),15,17,21 -heptaen-9-on;
- 4-[(Dimethylamino)methyl]-8,14-dioxa-10,19,20-triazatetracyclo[13.5.2.1^{2,6}.0^{18,21}] tricosa-1(20),2,4,6(23),15,17,21 -heptaen-9-on;
- 4,10-Dimethyl-8,14-dioxa-10,19,20-triazatetracyclo[13.5.2.1^{2,6}.0^{18,21}]tricosa-1(20),2,4, 6(23),15,17,21-heptaen-9-on;
- 4-(Propan-2-yloxy)-8,14-dioxa-10,19,20-triazatetracyclo[13.5.2.12^{2,6}.0^{18,21}]tricosa-1(20),2,4,6(23),15,17,21-heptaen-9-on;
- 4-Fluor-7-methyl-8,14-dioxa-10,19,20-triazatetracyclo[13.5.2.12^{2,6}.0^{18,21}]tricosa-1(20),2,4,6(23),15,17,21-heptaen-9-on;
- 4-[1-(Oxetan-3-yl)-1,2,3,6-tetrahydropyridin-4-yl]-8,14-dioxa-10,19,20-triazatetracyclo[13.5.2.12^{2,6}.0^{18,21}]tricosa-1(20),2,4,6(23),15,17,21-heptaen-9-on;
- 4-(3-Methylpiperidin-1-yl)-8,14-dioxa-10,19,20-triazatetracyclo[13.5.2.1^{2,6}.0^{18,21}] tricosa-1(20),2,4,6(23),15,17,21 -heptaen-9-on;
- 4-[(3S)-3-Hydroxypyrrolidin-1-yl]-8,14-dioxa-10,19,20-triazatetracyclo[13.5.2.12^{2,6}.0^{18,21}]tricosa-1(20),2,4,6(23),15,17,21-heptaen-9-on;
- 4-Fluor-8,14-dioxa-10,19,20-triazapentacyclo[13.5.2.1^{2,6}1^{7,10}.0^{18,21}]tetracosa-1(20), 2(24),3,5,15(22), 16,18(21)-heptaen-9-on;
- 4-(Oxolan-3-yl)-8,14-dioxa-10,19,20-triazatetracyclo[13.5.2.1^{2,6}.0^{18,21}]tricosa-1(20), 2,4,6(23), 15,17,21-heptaen-9-on;
- (13S)-13-Methyl-8,14-dioxa-10,19,20-triazatetracyclo[13.5.2.1^{2,6}.0^{18,21}]tricosa-1(20), 2(23),3,5,15(22), 16,18(21)-heptaen-9-on;
- (13R)-13-Methyl-8,14-dioxa-10,19,20-triazatetracyclo[13.5.2.1^{2,6}.0^{18,21}]tricosa-1(20),2(23),3,5,15(22), 16,18(21)-heptaen-9-on;
- 4-(1-Methyl-1H-pyrazol-3-yl)-8,14-dioxa-10,19,20-triazatetracyclo [13.5.2.1^{2,6}.0^{18,21}]tricosa-1(20),2(23),3,5,15(22),16,18(21)-heptaen-9-on;
- (7S)-7-Methyl-8,14-dioxa-10,19,20-triazatetracyclo[13.5.2.1^{2,6}.0^{18,21}]tricosa-1(20), 2(23),3,5,15(22),16,18(21)-heptaen-9-on;
- 4-[2-(Morpholin-4-yl)ethoxy]-8,14-dioxa-10,19,20-triazatetracyclo [13.5.2.1^{2,6}.0^{18,21}]tricosa-1(20),2(23),3,5,15(22),16,18(21)-heptaen-9-on;
- 4-(2-Methoxyethyl)-8,14-dioxa-10,19,20-triazatetracyclo[13.5.2.1^{2,6}.0^{18,21}]tricosa-1(20),2(23),3,5,15(22),16,18(21)-heptaen-9-on;
- (7R)-7-Methyl-8,14-dioxa-10,19,20-triazatetracyclo[13.5.2.1^{2,6}.0^{18,21}]tricosa-1(20), 2(23),3,5,15(22),16,18(21)-heptaen-9-on;
- 5-Cyclopropyl-8,14-dioxa-10,19,20-triazatetracyclo[13.5.2.12^{2,6}.0^{18,21}]tricosa-1(20),2,4,6(23),15,17,21-heptaen-9-on;
- 4-(2-Methoxyethoxy)-8,14-dioxa-10,19,20-triazatetracyclo[13.5.2.12^{2,6}.0^{18,21}]tricosa-1(20),2,4,6(23),15,17,21-heptaen-9-on;
- 4-Fluor-13-methyl-8,14-dioxa-10,19,20-triazatetracyclo[13.5.2.12^{2,6}.0^{18,21}]tricosa-1(20),2,4,6(23),15,17,21-heptaen-9-on;
- 11-Methyl-8,14-dioxa-10,19,20-triazatetracyclo[13.5.2.1^{2,6}.0^{18,21}]tricosa-1(20),2,4,6(23),15,17,21-heptaen-9-on;
- 4-(3-Oxomorpholin-4-yl)-8,14-dioxa-10,19,20-triazatetracyclo[13.5.2.1^{2,6}.0^{18,21}]tricosa-1(20),2(23),3,5,15(22),16,18(21)-heptaen-9-on;
- 4-(2-Oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-on;
- 5-(2-Oxopyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-on;
- 4-(2-Methylpyrrolidin-1-yl)-8,14-dioxa-10,19,20-triazatetracyclo[13.5.2.1^{2,6}.0^{18,21}]tricosa-1(20),2(23),3,5,15(22),16,18(21)-heptaen-9-on;
- 2-{9-Oxo-8,14-dioxa-10,19,20-triazatetracyclo[13.5.2.1^{2.6}.0^{18,21}]tricosa-1 (20),2(23),3,5,15(22),16,18(21)-heptaen-4-yl}acetonitril;
- (11R)-11-Methyl-8,14-dioxa-10,19,20-triazatetracyclo [13.5 3.1^{2,6}.0^{18,21}]tricosa-1(20),2(23),3,5,15(22),16,18(21)-heptaen-9-on;
- (11S)-11-Methyl-8,14-dioxa-10,19,20-triazatetracyclo [13.5.3.1^{2,6}.0^{18,21}]tricosa-1(20),2(23)3,5,15(22),16,18(21)-heptaen-9-on;
- 4-Ethynyl-8,14-dioxa-10,19,20-triazatetracyclo[13.5.2.1^{2,6}.0^{18,2}']tricosa-1(20),2(23),3,5,15(22),16,18(21)-heptaen-9-on;
- 4-(Piperazin-1-yl)-8,14-dioxa-10,19,20-triazatetracyclo[13.5.2.1^{2,6}.0^{18,21}]tricosa-1(20),2(23)3,5,15(22),16,18(21)-heptaen-9-on;
- 4-(1,2,3,6-Tetrahydropyridin-4-yl)-8,14-dioxa-10,19,20-triazatetracyclo [13.5.2.1^{2,6}.0^{18,21}]tricosa-1(20),2(23),3,5,15(22),16,18(21)-heptaen-9-on;
- 11-(Methoxymethyl)-8,14-dioxa-10,19,20-triazatetracyclo[13.5.2.1^{2,6}.0^{18,21}]tricosa-1(20),2(23),3,5,15(22),16,18(21)-heptaen-9-on;
- 8,14-Dioxa-5,10,19,20,23-pentaazatetracyclo[13.5.2.1^{2,5}.0^{18,21}]tricosa-1(20),2(23),3,15(22),16,18(21)-hexaen-9-on;
- 11-Methyl-8,14-dioxa-4,5,10,19,20-pentaazatetracyclo[13.5.2.1²,⁵.0¹⁸,²¹]tricosa-1(20),2(23),3,15(22),16,18(21)-hexaen-9-on;
- 12-Methyl-8,14-dioxa-10,19,20-triazatetracyclo[13.5.2.1^{2,6}.0^{18,21}]tricosa-1(20),2(23),3,5,15(22),16,18(21)-heptaen-9-on;
- 11-Ethyl-8,14-dioxa-10,19,20-triazatetracyclo[13.5.2.1^{2,6}.0^{18,21}]tricosa-1 (20)3(23)3,5, 15(22),16,18(21)-heptaen-9-on;
- 4-Fluor-5,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-on;
- 4-Fluor-5-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-on;
- 5-Fluor-4,7-dimethyl-8,14-dioxa-10,19,20-triazatetracyclo[13.5.2.1^{2,6}.0^{18,21}]tricosa-1(20),2(23),3,5,15(22),16,18(21)-heptaen-9-on;
- 8,14-Dioxa-10,19,20-triazapentacyclo[13.5.2.1^{2,6}.1^{7,10}.0^{18,21}]tetracosa-1(20),2(24),3,5,15(22),16,18(21)-heptaen-9-on;
- 13-Methyl-8,14-dioxa-10,19,20,23-tetraazatetracyclo[13.5.2.1^{2,6}.0^{18,21}]tricosa-1(20),2(23),3,5,15(22),16,18(21)-heptaen-9-on;
- 12-Methyl-8,14-dioxa-4,5,10,19,20-pentaazatetracyclo[13.5.2.1^{2,5}.0^{18,21}]tricosa-1(20),2(23),3,15(22),16,18(21)-hexaen-9-on;
- 7-Methyl-8,14-dioxa-4,5,10,19,20-pentaazatetracyclo[13.5.2.1^{2,5}.0^{18,21}]tricosa-1(20),2(23),3,15(22),16,18(21)-hexaen-9-on;
- 5-Fluor-4-methoxy-7-methyl-8,14-dioxa-10,19,20-triazatetracyclo [13.5.2.1^{2,6}.0^{18,21}]tricosa-1(20),2(23),3,5,15(22),16,18(21)-heptaen-9-on;
- (7R,13R)-7,13-Dimethyl-8,14-dioxa-10,19,20-triazatetracyclo[13.5.2.1^{2,6}.0^{18,21}]tricosa-1(20),2(23),3,5,15(22),16,18(21)-heptaen-9-on;
- (13R)-13-Methyl-8,14-dioxa-4,5,10,19,20-pentaazatetracyclo[13.5.2.1^{2,5}.0^{18,21}]tricosa-1(20),2(23),3,15,17,21-hexaen- 9-on;
- 8,15-Dioxa-4,10,20,21-tetraazapentacyclo[14.5.2.1^{2,6}.1^{10,13}.0^{19,22}]pentacosa-1(21),2(25),3,5,16(23),17,19(22)-heptaen-9-on;
- 8,14-Dioxa- 5,10,19,20-tetraazatetracyclo [ 13.5.2.1²,⁵.0¹⁸,²¹]tricosa-1(20),2(23),3,15(22),16,18(21)-hexaen-9-on;
- (13S)-4-Fluor-13-methyl-8,14-dioxa-10,19,20-triazatetracyclo[13.5.2.1^{2,6}.0^{18,21}]tricosa-1(20),2(23),3,5,15(22),16,18(21)-heptaen-9-on;
- (13R)-4-Fluor-13-methyl-8,14-dioxa-10,19,20-triazatetracyclo[13.5.2.1^{2,6}.0^{18,21}]tricosa-1(20),2(23),3,5,15(22),16,18(21)-heptaen-9-on;
- (13R)-13-Methyl-8,14-dioxa-4,10,19,20-tetraazatetracyclo[13.5.2.1^{2,6}.0^{18,21}]tricosa-1(20),2(23),3,5,15(22),16,18(21)-heptaen-9-on;
- 6-Vyclopropyl-8,14-dioxa-4,5,10,19,20-pentaazatetracyclo[13.5.2.1^{2,5}.0^{18,21}]tricosa-1(20),2(23),3,15(22),16,18(21)-hexaen-9-on;
- 7-Ethyl-8,14-dioxa-10,19,20-triazatetracyclo[13.5.2.1^{2,6}.0^{18,21}]tricosa-1(20),2,4,6(23),15,17,21-heptaen-9-on;
- (13R)-13-Methyl-8,14-dioxa-5,10,19,20,23-pentaazatetracyclo[13.5.2.1^{2,6}.0^{18,21}]tricosa-1(20),2,4,6(23),15,17,21-heptaen-9-on;
- (7R,13R)-4-Fluor-7,13-dimethyl-8,14-dioxa-10,19,20-triazatetracyclo[13.5.2.12^{2,6}.0^{18,21}]tricosa-1(20),2,4,6(23),15,17,21-heptaen-9-on;
- 7-Methyl-8,14-dioxa-4,10,19,20-tetraazatetracyclo[13.5.2.1^{2,6}.0^{18,21}]tricosa-1(20),2,4,6(23),15,17,21-heptaen-9-on;
- (7R)-4-Fluor-7-methyl-8,14-dioxa-10,19,20-triazatetracyclo[13.5.2.12^{2,6}.0^{18,21}]tricosa-1(20),2,4,6(23),15,17,21-heptaen-9-on;
- (7S)-4-Fluor-7-methyl-8,14-dioxa-10,19,20-triazatetracyclo[13.5.2.12^{2,6}.0^{18,21}]tricosa-1(20),2,4,6(23),15,17,21-heptaen-9-on;
- 6-Methyl-8,14-dioxa-4,5,10,19,20-pentaazatetracyclo[13.5.2.1^{2,5}.0^{18,21}]tricosa-1(20),2(23),3,15,17,21-hexaen-9-on;
- 7-Methyl-8,14-dioxa-10,19,20,23-tetraazatetracyclo[ 13.5.2.1^{2,6}.0^{18,21}]tricosa-1(20),2,4,6(23),15,17,21-heptaen-9-on;
- 6-(Propan-2-yl)-8,14-dioxa-4,5,10,19,20-pentaazatetracyclo[13.5.2.1^{2,5}.0^{18,21}]tricosa-1(20),2(23),3,15(22),16,18(21)-hexaen-9-on;
- (13R)-7,13-Dimethyl-8,14-dioxa-4,5,10,19,20-pentaazatetracyclo[13.5.2.1^{2,5}.0^{18,21}]tricosa-1(20),2(23),3,15(22),16,18(21)-hexaen-9-on;
- (13R)-13-Methyl-8,14-dioxa-10,19,20,23-tetraazatetracyclo[13.5.2.1^{2,6}.0^{18,21}]tricosa-1(20),2,4,6(23),15,17,21-heptaen-9-on;
- (7R)-7-Ethyl-8,14-dioxa-10,19,20-triazatetracyclo[13.5.2.1^{2,6}.0^{18,21}]tricosa-1(20),2,4, 6(23),15, 17,21-heptaen-9-on;
- (7S)-7-Ethyl-8,14-dioxa-10,19,20-triazatetracyclo[13.5.2.1^{2,6}.0^{18,21}]tricosa-1(20),2,4, 6(23),15,17,21-heptaen-9-on;
- (13R)-13-Methyl-8,14-dioxa-5,10,19,20-tetraazatetracyclo[13.5.2.1²⁶.0^{18,21}]tricosa-1(20),2,4,6(23),15,17,21-heptaen-9-on;
- 6-(Oxan-4-yl)-8,14-dioxa-4,5,10,19,20-pentaazatetracyclo[13.5.2.1^{2,5}.0^{18,21}]tricosa-1(20),2(23),3,15,17,21-hexaen-9-on;
- 4-Ethyl-8,14-dioxa-5,10,19,20,23-pentaazatetracyclo[13.5.2.1^{2,5}.0^{18,21}]tricosa-1(20), 2(23),15,17,21-pentaen-9-on;
- (13R)-23-Fluor-13-methyl- 8,14-dioxa-10,19,20-triazatetracyclo[13.5.2.12^{2,6}.0^{18,21}]tricosa-1(20),2,4,6(23),15,17,21-heptaen-9-on;
- 9,14-Dioxa-4,5,11,19,20-pentaazatetracyclo[13.5.2.1^{2,5}.0^{18,21}]tricosa-1(20),2(23), 3,15,17,21-hexaen-10-on;
- 4-Ethyl-8,14-dioxa-5,10,19,20,23-pentaazatetracyclo[13.5.2.1^{2,5}.0^{18,21}]tricosa-1(20),2(23),3,15,17,21-hexaen-9-on;
- 3,9, 15-Trioxa-4,11,20,21 -tetraazatetracyclo[14.5.2.1^{2,5}.0^{19,22}]tetracosa-1(21),2(24), 4,16, 18,22-hexaen- 10-on;
- (13R)-16-Fluor-13-methyl-8,14-dioxa-4,10,19,20-tetraazatetracyclo[13.5.2.1^{2,6}.0^{18,21}]tricosa-1(20),2,4,6(23),15,17,21-heptaen-9-on;
- (13R)-4-Chlor-13-methyl-8,14-dioxa-10,19,20,23-tetraazatetracyclo[13.5.2.1^{2,6}.0^{18,21}]tricosa-1(20),2(23),3,5,15(22),16,18(21)-heptaen-9-on;
- 8,14-Dioxa-2,4,10,19,20-pentaazatetracyclo[13.5.2.1^{2,5}.0^{18,21}]tricosa-1(20),3,5(23),15(22),16,18(21)-hexaen-9-on;
- (13R)-4-Methoxy-13-methyl-8,14-dioxa-10,19,20,23-tetraazatetracyclo[13.5.2.1^{2,6}.0^{18,21}]tricosa-1(20),2,4,6(23),15,17,21-heptaen-9-on;
- (13R)-13-Methyl-9-oxo-8,14-dioxa-10,19,20-triazatetracyclo[13.5.2.1^{2,6}.0^{18,21}]tricosa-1(20),2,4,6(23),15,17,21-heptaen-5-carbonitril;
- (13R)-13-Methyl-4-(pyrrolidin-1-yl)-8,14-dioxa-5,10,19,20,23-pentaazatetracyclo[13.5.2.1^{2,6}.0^{18,21}]tricosa-1(20),2,4,6(23),15,17,21-heptaen-9-on;
- (7S,13R)-7,13-Dimethyl- 8,14-dioxa-5,10,19,20,23-pentaazatetracyclo[13.5.2.1^{2,6}.0^{18,21}]tricosa-1(20),2,4,6(23),15,17,21-heptaen-9-on;
- (7R,13R)-7,13-Dimethyl-8,14-dioxa-5,10,19,20,23-pentaazatetracyclo[13.5.2.1^{2,6}.0^{18,21}]tricosa-1(20),2,4,6(23),15,17,21-heptaen-9-on;
- (13R)-16-Fluor-13-methyl-8,14-dioxa-10,19,20-triazatetracyclo[13.5.2.12^{2,6}.0^{18,21}]tricosa-1(20),2,4,6(23),15,17,21-heptaen-9-on;
- (13R)-13-Methyl-8,14-dioxa-4,10,19,20,23-pentaazatetracyclo[13.5.2.1^{2,6}.0^{18,21}]tricosa-1(20),2,4,6(23),15,17,21-heptaen-9-on;
- 8,14-Dioxa-4-thia-10,19,20,23-tetraazatetracyclo[13.5.2.1^{2,5}.0^{18,21}]tricosa-1(20),2,5(23),15,17,21-hexaen-9-on;
- 8,14-Dioxa-3-thia-10,19,20,23-tetraazatetracyclo[13.5.2.1^{2,5}.0^{18,21}]tricosa-1(20),2(23),4,15,17,21-hexaen-9-on;
- (7R,13R)-7,13-Dimethyl-8,14-dioxa-10,19,20,23-tetraazatetracyclo [13.5.2.1^{2,6}.0^{18,21}]tricosa-1 (20),2,4,6(23),15, 17,21-heptaen-9-on;
- (13R)-4-[(3R)-3-Methoxypyrrolidin-1-yl]-13-methyl-8,14-dioxa-5,10,19,20,23-pentaazatetracyclo1[13.5.2.1^{2,6}.0^{18,21}]tricosa-1(20),2,4,6(23),15,17,21-heptaen-9-on;
- (13R)-16-Chlor-13-methyl-8,14-dioxa-10,19,20-triazatetracyclo[13.5.2.12^{2,6}.0^{18,21}]tricosa-1(20),2,4,6(23),15,17,21-heptaen-9-on;
- (13R)-13,16-Dimethyl-8,14-dioxa-10,19,20-triazatetracyclo[13.5.2.12^{2,6}.0^{18,21}]tricosa-1(20),2,4,6(23),15,17,21-heptaen-9-on;
- (13R)-13-Methyl-8,14-dioxa-3,10,19,20,23-pentaazatetracyclo[13.5.2.1^{2,6}.0^{18,21}]tricosa-1(20),2,4,6(23),15,17,21-heptaen-9-on;
- 8-Oxa-10,14,19,20-tetraazatetracyclo[13.5.2.1^{2,6}.0^{18,21}]tricosa-1 (20),2(23),3,5,15(22),16,18(21)-heptaen-9-on-hydrochlorid;
- 8-Oxa-10,19,20-triazatetracyclo[13.5.2.1^{2,6}.0^{18,21}]tricosa-1 (20),2(23),3,5,15(22),16,18(21)-heptaen-9-on;
- (13R)-5-Methoxy-13-methyl-8,14-dioxa-4,10,19,20-tetraazatetracyclo [13.5.2.1^{2,6}.0^{18,21}]tricosa-1 (20),2,4,6(23),15, 17,21-heptaen-9-on;
- (13R)-13-Methyl-8,14-dioxa-4,10,19,20-tetraazatetracyclo[13.5.2.1^{2,6}.0^{18,21}]tricosa-1(20),2,6(23),15,17,21-hexaene-5,9-dion;
- 4-Methyl-8,14-dioxa-3,4,10,19,20-pentaazatetracyclo[13.5.2.1^{2,5}.0^{18,21}]tricosa-1(20),2,5(23),15(22),16,18(21)-hexaen-9-on;
- (13R)-16-Fluor-13-methyl-8,14-dioxa-10,19,20,23-tetraazatetracyclo[13.5.2.1^{2,6}.0^{18,21}]tricosa-1(20),2,4,6(23),15,17,21-heptaen-9-on;
- 7,13-Dioxa-4-thia-9,18,19,22-tetraazatetracyclo[12.5.2.1^{2,5}.0^{17,20}]docosa-1(19),2,5(22),14(21),15,17(20)-hexaen-8-on;
- (13R)-4,13-Dimethyl-8,14-dioxa-5,10,19,20,23-pentaazatetracyclo[13.5.2. 1^{2,6}.0^{18,21}]tricosa-1(20),2(23),3,5,15(22),16,18(21)-heptaen-9-on;
- 8,14-Dioxa-23-thia-4,10,19,20-tetraazatetracyclo[13.5.2.1^{2,5}.0^{18,21}]tricosa-1(20),2,4,15(22),16,18(21)-hexaen-9-on;
- (7S, 13R)-7,13-Dimethyl-8,14-dioxa-10,19,20,23-tetraazatetracyclo[13.5.2.1^{2,6}.0^{18,21}]tricosa-1(20),2(23),3,5,15(22),16,18(21)-heptaen-9-on;
- (13R)-13-Methyl-9-oxo-8,14-dioxa-5,10,19,20-tetraazatetracyclo[13.5.2.1^{2,5}.0^{18,21}]tricosa-1(20),2(23),3,15(22),16,18(21)-hexaen-4-carbonitril;
- 12,12-Difluor-8,14-dioxa-10,19,20-triazatetracyclo[13.5.2.1^{2,6}.0¹⁸,²¹]tricosa-1(20),2(23),3,5,15(22),16,18(21)-heptaen-9-on;
- (13R)-17-Fluor-13-methyl-8,14-dioxa-10,19,20-triazatetracyclo[13.5.2.1^{2,6}.0^{18,21}]tricosa-1(20),2(23),3,5,15(22),16,18(21)-heptaen-9-on;
- (7S, 13R)-7,13-Dimethyl-8,14-dioxa-4,10,19,20,23-pentaazatetracyclo[13.5.2.1^{2,6}.0^{18,21}]tricosa-1(20),2(23),3,5,15(22),16,18(21)-heptaen-9-on;
- (7R,13R)-7,13-Dimethyl-8,14-dioxa-4,10,19,20,23-pentaazatetracyclo[13.5.2.1^{2,6}.0^{18,21}]tricosa-1(20),2(23),3,5,15(22),16,18(21)-heptaen-9-on;
- (13S)-13-Methyl-8,14-dioxa-4,10,19,20,23-pentaazatetracyclo[13.5.2.1^{2,6}.0^{18,21}]tricosa-1(20),2(23),3,5,15(22),16,18(21)-heptaen-9-on;
- (13R)-13-Methyl-8,14-dioxa-10,19,20,22-tetraazatetracyclo[13.5.2.1^{2,6}.0^{18,21}]tricosa-1(20),2(23),3,5,15,17,21-heptaen-9-on;
- (12R)-4,12-Dimethyl-7,13-dioxa-4,9,18,19,22-pentaazatetracyclo[12.5.2.1^{2,5}.0^{17,20}]docosa-1(19),2,5(22),14(21),15,17(20)-hexaen-8-on;
- (13R)-13-Methyl-8,14-dioxa-4,5,10,19,20,23-hexaazatetracyclo[13.5.2.1^{2,5}.0^{18,21}]tricosa-1(20),2(23),3,15,17,21-hexaen-9-on;
- (13R)-13-Methyl-8,14-dioxa-23-thia-4,10,19,20-tetraazatetracyclo[13.5.2.1^{2,5}.0^{18,21}]tricosa-1(20),2,4,15,17,21-hexaen-9-on;
- (13R)-4,13-Dimethyl-8,14-dioxa-4,10,19,20,23-pentaazatetracyclo[13.5.2.1^{2,5}.0^{18,21}]tricosa-1(20),2,5(23),15(22),16,18(21)-hexaen-9-on;
- (13R)-13-Methyl-8,14-dioxa-10,16,19,20-tetraazatetracyclo[13.5.2.1^{2,6}.0^{18,21}]tricosa-1(20),2(23),3,5,15(22),16,18(21)-heptaen-9-on;
- 14-Methyl-8-oxa-10,14,19,20-tetraazatetracyclo[13.5.2.1^{2,6}.0^{18,21}]tricosa-1(20),2(23),3,5,15,17,21-heptaen-9-on;
- (13R)-13-Methyl- 8,14-dioxa-4,10,19,20,22-pentaazatetracyclo[13.5.2.1^{2,6}.0^{18,21}]tricosa-1(20),2(23),3,5,15,17,21-heptaen-9-on;
- (13R)-13-Methyl-8,14-dioxa-10,17,19,20-tetraazatetracyclo[13.5.2.1^{2,6}.0^{18,21}]tricosa-1(20),2(23),3,5,15(22),16,18(21)-heptaen-9-on;
- 8,14-Dioxa-4,5,10,19,20,23-hexaazatetracyclo[13.5.2.1^{2,5}.0^{18,21}]tricosa-1(20),2(23),3,15(22),16,18(21)-hexaen-9-on;
- 12,12-Difluor-8,14-dioxa-4,5,10,19,20,23-hexaazatetracyclo[13.5.2.1^{2,5}.0^{18,21}]tricosa-1(20),2(23),3,15(22),16,18(21)-hexaen-9-on;
- (12R)-12-Fluor-8,14-dioxa-10,19,20-triazatetracyclo[13.5.2.1^{2,6}.0^{18,21}]tricosa-1(20),2(23),3,5,15(22),16,18(21)-heptaen-9-on;
- (12S)-12-Fluor-8,14-dioxa-10,19,20-triazatetracyclo[13.5.2.1^{2,6}.0^{18,21}]tricosa-1(20),2(23),3,5,15(22),16,18(21)-heptaen-9-on;
- 12,12-Difluor-8,14-dioxa-4,10,19,20,23-pentaazatetracyclo[13.5.2.1^{2,6}.0^{18,21}]tricosa-1(20),2(23),3,5,15(22),16,18(21)-heptaen-9-on;
- (12S)-12-Fluor-8,14-dioxa-4,10,19,20,23-pentaazatetracyclo[13.5.2.1^{2,6}.0^{18,21}]tricosa-1(20),2(23),3,5,15,17,21-heptaen-9-on;
- (12R)-12-Fluor-8,14-dioxa-4,10,19,20,23-pentaazatetracyclo[13.5.2.1^{2,6}.0^{18,21}]tricosa-1(20),2(23),3,5,15,17,21-heptaen-9-on;
- (12S)-12-Fluor-8,14-dioxa-4,5,10,19,20,23-hexaazatetracyclo[13.5.2.1^{2,5}.018,21]tricosa-1(20),2(23),3,15,17,21-hexaen-9-on;
- (12R)-12-Fluor-8,14-dioxa-4,5,10,19,20,23-hexaazatetracyclo[13.5.2.1^{2,5}.018,21]tricosa-1(20),2(23),3,15,17,21-hexaen-9-on; oder
- 8',14'-Dioxa-10',19',20'-triazaspiro[cyclopropane-1,13'-tetracyclo[13.5.2.1^{2,6}.0^{18,21}]tricosane]-1'(20'),2'(23'),3',5',15'(22'),16',18'(21')-heptaen-9'-on.

31. Pharmazeutische Zusammensetzung, umfassend die Verbindung der Formel (I) nach einem der Ansprüche 1 bis 30 oder ein Additionssalz davon mit einer pharmazeutisch verträglichen Säure oder Base in Kombination mit einem oder mehreren pharmazeutisch verträglichen Hilfsstoffen.

32. Pharmazeutische Zusammensetzung nach Anspruch 31 zur Verwendung bei der Behandlung von neurologischen Erkrankungen, entzündlichen Erkrankungen, bakteriellen, viralen und parasitären Infektionen, Herz-Kreislauf-Erkrankungen, Autoimmunerkrankungen, Krebserkrankungen und endosomalenlysosomalen Störungen, ausgewählt aus Niemann-Pick-Krankheit Typ A, B oder C, Gaucher-Krankheit, Krabbe-Krankheit, Fabry-Krankheit und Störungen mit mitochondrialen Defiziten.

33. Pharmazeutische Zusammensetzung zur Verwendung nach Anspruch 32, wobei die neurologische Krankheit ausgewählt ist aus Parkinsonkrankheit, Alzheimer-Krankheit, amyotropher Lateralsklerose (ALS), Demenz, diabetischer Neuropathie, altersbedingter Gedächtnisstörung, leichter kognitiver Beeinträchtigung, argyrophiler Kornkrankheit, Pick-Krankheit, Epilepsie, Tauopathien wie progressive supranukleäre Lähmung und kortikobasale Degeneration, andere Synucleinopathien wie multiple Systematrophie, frontotemporale Demenz, vererbte frontotemporale Demenz und Parkinsonismus in Verbindung mit Chromosom 17 (FTDP-17), Entzugserscheinungen/Rückfälle im Zusammenhang mit Medikamentenabhängigkeit, L-Dopa-induzierte Dyskinesie, ischämischer Schlaganfall, traumatische Hirnverletzung, Rückenmarksverletzung und Multiple Sklerose.

34. Pharmazeutische Zusammensetzung zur Verwendung nach Anspruch 33, wobei die neurologische Krankheit die Parkinsonkrankheit oder die Alzheimer-Krankheit ist.

35. Pharmazeutische Zusammensetzung zur Verwendung nach Anspruch 32, wobei die entzündliche Krankheit ausgewählt ist aus Vaskulitis, Lungenerkrankungen wie chronisch obstruktiver Lungenerkrankung, idiopathischer Lungenfibrose, entzündlichen Myopathien und ankylosierender Spondylitis.

36. Pharmazeutische Zusammensetzung zur Verwendung nach Anspruch 32, wobei die Autoimmunerkrankung ausgewählt ist aus Morbus Crohn, entzündlichen Darmerkrankungen, rheumatoider Arthritis, Colitis ulcerosa, Lupus, autoimmuner hämolytischer Anämie, reiner roter Zellaplasie, idiopathischer thrombozytopenischer Purpura, Typ-I-Diabetes mellitus, Adipositas, Evans-Syndrom, bullösen Hauterkrankungen, Sjögren-Syndrom, Devic-Krankheit und Lepra.

37. Pharmazeutische Zusammensetzung zur Verwendung nach Anspruch 32, wobei der Krebs ausgewählt ist aus Schilddrüsenkrebs, Nierenkrebs, Brustkrebs, hormonbedingtem Krebs, Adeno- und Plattenepithelkarzinom der Lunge, nicht-kleinzelligem Lungenkrebs, Darmkrebs, Prostatakrebs, Hautkrebs, Leukämien und Lymphomen.

38. Pharmazeutische Zusammensetzung zur Verwendung nach Anspruch 32, wobei die Herz-Kreislauf-Krankheit ein Schlaganfall ist.

39. Pharmazeutische Zusammensetzung zur Verwendung nach Anspruch 32, wobei die bakteriellen oder viralen Infektionen ausgewählt sind aus Lepra, Tuberkulose, SARS-CoV, MERS-CoV und SARS-CoV-2, HIV, West-Nil-Virus und Chikungunya-Virus.

40. Verbindung der Formel (I) nach einem der Ansprüche 1 bis 30 oder ein Additionssalz davon mit einer pharmazeutisch verträglichen Säure oder eine Basis zur Verwendung bei der Behandlung von Parkinsonkrankheit, Alzheimer-Krankheit, amyotropher Lateralsklerose (ALS), Demenz, diabetischer Neuropathie, altersbedingter Gedächtnisstörung, leichter kognitiver Beeinträchtigung, argyrophiler Kornkrankheit, Pick-Krankheit, Epilepsie, Tauopathien wie progressive supranukleäre Lähmung und kortikobasale Degeneration, andere Synucleinopathien, frontotemporale Demenz, vererbte frontotemporale Demenz und Parkinsonismus in Verbindung mit Chromosom 17 (FTDP-17), Entzugserscheinungen/Rückfälle im Zusammenhang mit Medikamentenabhängigkeit, L-Dopa-induzierte Dyskinesie, ischämischer Schlaganfall, traumatische Hirnverletzung, Rückenmarksverletzung, Multiple Sklerose, Niemann-Pick Typ A, B oder C, Morbus Gaucher, Morbus Krabbe, Morbus Fabry, Störungen mit mitochondrialen Defiziten, Morbus Crohn, entzündliche Darmerkrankungen, rheumatoide Arthritis, Colitis ulcerosa, Lupus, autoimmune hämolytische Anämie, reine rote Zellaplasie, idiopathische thrombozytopenische Purpura, Typ-I-Diabetes mellitus, Adipositas, Evans-Syndrom, bullöse Hautstörungen, Sjögren-Syndrom, Devic-Krankheit, Lepra, Schilddrüsenkrebs, Nierenkrebs (einschließen papillärem Nierenkrebs), Brustkrebs, hormonabhängiger Krebs, Adeno- und Plattenepithelkarzinom der Lunge, nicht-kleinzelliges Lungenkarzinom, Dickdarmkrebs, Prostatakrebs, Hautkrebs, Leukämien (einschließen akuter myeloischer Leukämie), Lymphome, Schlaganfall, Lepra, Tuberkulose und SARS-CoV-, MERS-CoV-, SARS-CoV-2-, HIV-, West-Nil-Virus- und Chikungunya-Virus-Infektionen.

## Revendications

1. Composé de formule (I) : 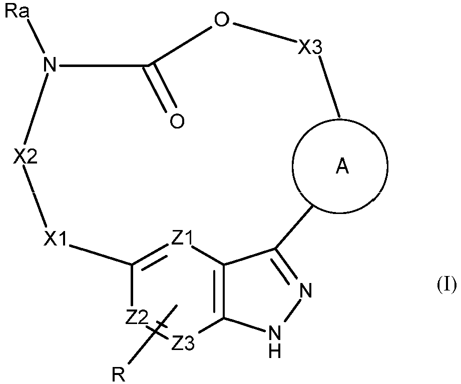 dans lequel :
◆ R représente un atome d'hydrogène, un atome d'halogène ou un groupe alkyle,
◆ Z1, Z2, Z3 représentent chacun indépendamment un atome de carbone ou d'azote, étant entendu que le cycle à 6 chaînons contenant Z1, Z2 et Z3 peut avoir 0, 1 ou 2 atomes d'azote,
◆ -X1- est absent ou représente -O-, -S-, ou -N(R'a)-, dans lequel R'a représente un atome d'hydrogène ou un groupe alkyle,
◆ -X2- représente un groupe alcanediyle facultativement substitué par un ou plusieurs substituants, identiques ou différents, choisis parmi atomes d'halogène, groupe polyhalogénoalkyle, groupe alcoxy, groupe hydroxy, groupe amino, groupe alkylamino, groupe dialkylamino et groupe cyano,
étant entendu que l'atome de carbone en position alpha de -N(Ra) et l'atome de carbone en position alpha de -X1- lorsque -X1- représente -O-, -S-, ou - N(R'a)-, ne peuvent être substitués par un hétéroatome d'oxygène ou d'azote,
◆ -X3- représente un groupe alcanediyle facultativement substitué par un ou plusieurs substituants, identiques ou différents, choisis parmi atomes d'halogène, groupe polyhalogénoalkyle, groupe alcoxy, groupe hydroxy, groupe amino, groupe alkylamino, groupe dialkylamino, groupe cyano, groupe cycloalkyle et groupe hétérocycloalkyle,
étant entendu que l'atome de carbone en position alpha de -O- et l'atome de carbone en position alpha de A1 lorsque A1 représente un atome d'azote, ne peuvent être substitués par un hétéroatome d'oxygène ou d'azote,
◆ Ra représente un atome d'hydrogène ou un groupe alkyle,
étant entendu que lorsque Ra représente un groupe alkyle, un atome de carbone de Ra peut être lié à un atome de carbone de -X2-, ou à un atome de carbone de -X3- pour former une fraction cyclique contenant 5 ou 6 chaînons de cycle,
◆ A représente
- un groupe cyclique aromatique ou partiellement hydrogéné de la formule (a) : 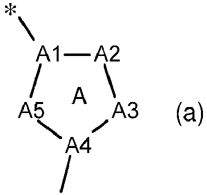 dans lequel
_{✔} A1, A4 représentent chacun indépendamment un atome de carbone ou un atome d'azote,
_{✔} A2, A3, A5 représentent chacun indépendamment un atome de carbone, un atome d'oxygène, un atome de soufre ou un atome d'azote,
étant entendu que A1, A2, A3, A4 et A5 ne peuvent représenter simultanément un hétéroatome,
- ou un groupe cyclique aromatique ou partiellement hydrogéné de la formule (b) : 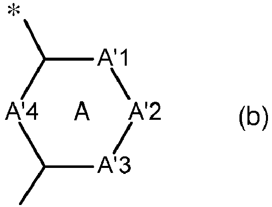
dans lequel A'1, A'2, A'3, A'4 représentent chacun indépendamment un atome de carbone ou un atome d'azote,
étant entendu que * signifie que la liaison est liée à X3,
le groupe A cyclique aromatique ou partiellement hydrogéné ainsi défini étant facultativement substitué par un ou plusieurs substituants, identiques ou différents, choisis parmi atomes d'halogène, groupe alkyle, groupe alcoxy, groupe hydroxy, groupe oxo, groupe alcoxyalkyle, groupe alcoxyalcoxy, groupe polyhalogénoalkyle, groupe polyhalogénoalcoxy, groupe hétérocycloalkyle, groupe hétérocycloalkylalkyle, groupe (alcoxyalkyl)(alkyl)amino, groupe amino, groupe alkylamino, groupe dialkylamino, groupe cycloalkyle, groupe (hétérocycloalkyl)(alkyl)amino, groupe dialkylaminoalkyle, groupe hétérocycloalkylalcoxy, groupe cyano et groupe cyanoalkyle,
dans lequel le groupe hétérocycloalkyle et le groupe cycloalkyle ainsi définis peuvent être facultativement substitués par un ou plusieurs substituants choisis parmi groupe alkyle, atomes d'halogène, groupe polyhalogénoalkyle, groupe polyhalogénoalcoxy, groupe alcoxy, groupe alcoxyalkyle, groupe hydroxy, groupe cyano et groupe oxo,
leurs énantiomères, diastéréo-isomères, tautomères, racémiques, hydrates, solvates, N-oxyde, isotopes, dérivés deutérés et sels d'addition de ceuxci avec un acide ou une base pharmaceutiquement acceptable.

2. Composé selon la revendication 1, dans lequel R représente un atome d'hydrogène.

3. Composé selon la revendication 1, dans lequel R représente un atome d'halogène.

4. Composé selon l'une quelconque des revendications 1 à 3, dans lequel Z1, Z2 et Z3 représentent simultanément un atome de carbone.

5. Composé selon l'une quelconque des revendications 1 à 3, dans lequel Z1 ou Z2 représente un atome d'azote et Z3 représente un atome de carbone.

6. Composé selon l'une quelconque des revendications 1 à 5, dans lequel - X1- représente -O-.

7. Composé selon l'une quelconque des revendications 1 à 6, dans lequel - X2- représente un groupe alcanediyle linéaire ou ramifié ayant 2, 3, 4 ou 5 atomes de carbone.

8. Composé selon la revendication 7, dans lequel -X2- représente -(CH₂)₃-, - CH(CH₃)-(CH₂)₂-, -CH₂-CHF-CH₂-, -CH₂-CF₂-CH₂-, ou -(CH₂)₂-CH(CH₃)-.

9. Composé selon l'une quelconque des revendications 1 à 8, dans lequel Ra est un atome d'hydrogène.

10. Composé selon l'une quelconque des revendications 1 à 9, dans lequel - X3- représente un groupe alcanediyle linéaire ou ramifié ayant 1, 2, 3, 4 ou 5 atomes de carbone.

11. Composé selon la revendication 10, dans lequel -X3- représente -(CH₂)₂-, -CH₂- ou -CH(CH₃)-.

12. Composé selon l'une quelconque des revendications 1 à 11, dans lequel A représente un groupe de formule (b) : 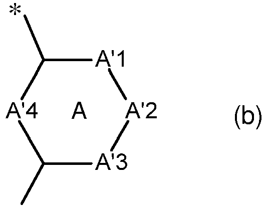 dans lequel A'1, A'2, A'3, A'4 et * sont tels que définis dans la revendication 1.

13. Composé selon la revendication 12, dans lequel A représente 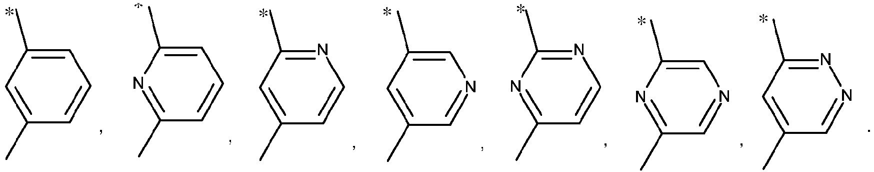 ces groupes A définis étant non substitués ou facultativement substitués.

14. Composé selon la revendication 12, dans lequel A représente un groupe phényle.

15. Composé selon la revendication 12, dans lequel A représente un groupe pyridinyle.

16. Composé selon la revendication 12, dans lequel A représente un groupe pyrazinyle.

17. Composé selon les revendications 1 à 11, dans lequel A représente un groupe de formule (a) : 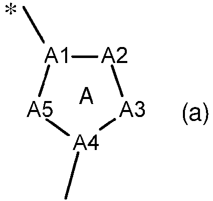 dans lequel A1, A2, A3, A4, A5 et * sont tels que définis dans la revendication 1.

18. Composé selon la revendication 17, dans lequel A représente 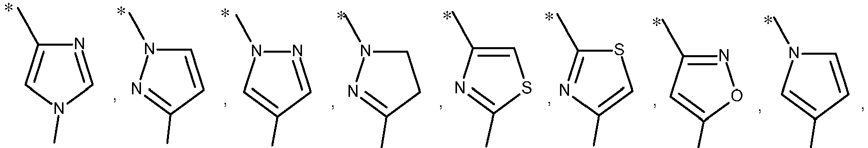 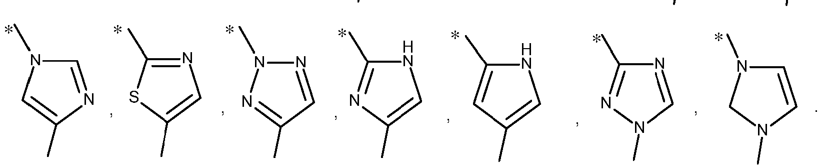 ces groupes A définis étant non substitués ou facultativement substitués.

19. Composé selon la revendication 17, dans lequel A représente un groupe triazolyle.

20. Composé selon la revendication 17, dans lequel A représente un groupe pyrazolyle.

21. Composé selon les revendications 12 à 20, dans lequel A n'est pas substitué.

22. Composé selon les revendications 12 à 20, dans lequel A est substitué par un ou plusieurs groupes choisis parmi atomes d'halogène, groupe cyano, groupe cyanoalkyle, groupe oxo, groupe alcoxy, groupe alkyle, groupe cycloalkyle et groupe hétérocycloalkyle.

23. Composé selon la revendication 1, qui est un composé de formule (I-a) : 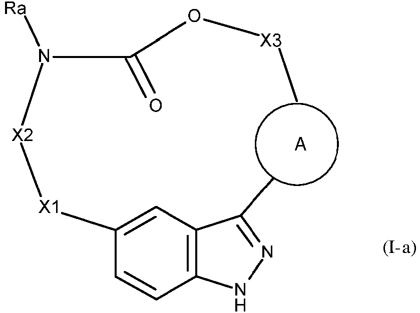 dans lequel X1, X2, X3, Ra et A sont tels que définis dans la revendication 1.

24. Composé selon la revendication 23, qui est un composé de formule (I-b) : 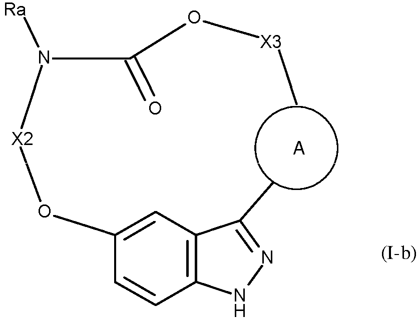 dans lequel X2, X3, Ra et A sont tels que définis dans la revendication 1.

25. Composé selon la revendication 23, qui est un composé de formule (I-c) ou (I-c') : 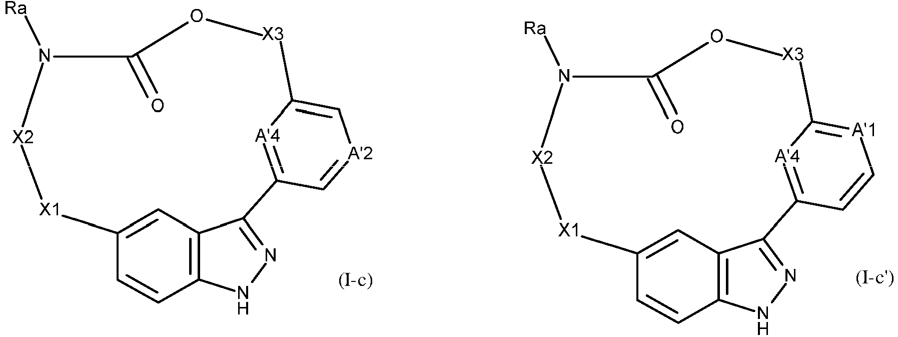 dans lequel X1, X2, X3, Ra, A'1, A'2 et A'4 sont tels que définis dans la revendication 1.

26. Composé selon la revendication 23 ou 25, qui est un composé de formule (I-d) ou (I-d') : 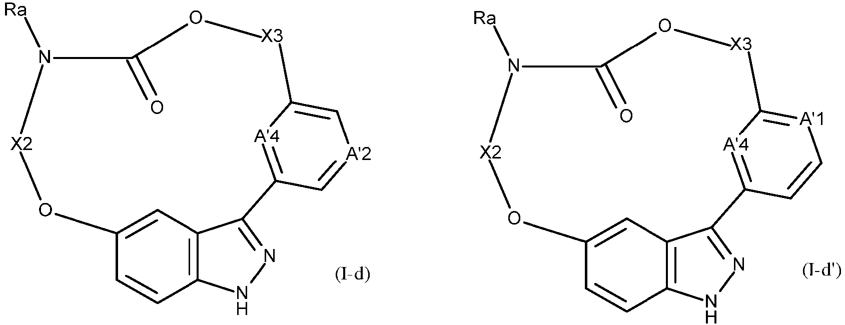 dans lequel X2, X3, Ra, A'1, A'2 et A'4 sont tels que définis pour la formule (I).

27. Composé selon la revendication 23, qui est un composé de formule (I-e) : 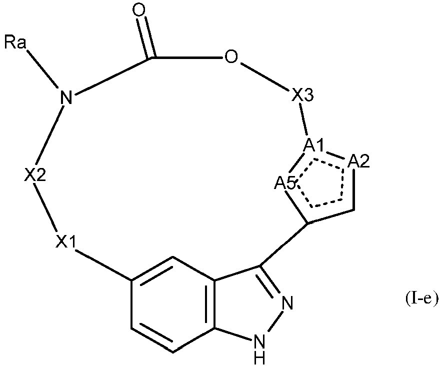 dans lequel X1, X2, X3, Ra, A1, A2 et A5 sont tels que définis pour la formule (I).

28. Composé selon la revendication 23 ou 27, qui est un composé de formule (I-f) : 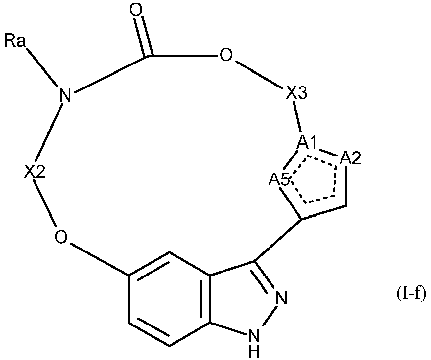 dans lequel X2, X3, Ra, A1, A2 et A5 sont tels que définis pour la formule (I).

29. Composé selon la revendication 23, 25 ou 27, dans lequel la chaîne -X1-X2-N(Ra)-C(O)O-X3- représente -O-(CH₂)₃-NHC(O)O-CH₂-, -O-CH(CH₃)-(CH₂)₂-NHC(O)O-CH₂-,
-O-CH₂-CHF-CH₂-NHC(O)O-CH₂-, -O-CH₂-CF₂-CH₂-NHC(O)O-CH₂-,
-O-CH(CH₃)-(CH₂)₂-NHC(O)O-(CH₂)₂- ou
-O-CH(CH₃)-(CH₂)₂-NH-C(O)O-CH(CH₃)-.

30. Composés selon la revendication 1 qui sont :
- 8,14-dioxa-4,10,19,20-tétraazatétracyclo[13.5.2.1^{2,6}.0^{18,21}]tricosa-1(20),2,4,6(23),15,17,21-heptaén-9-one ;
- 10-méthyl-8,14-dioxa-4,10,19,20-tétraazatétracyclo[13.5.2.1^{2,6}.0^{18,21}]tricosa-1(20),2,4,6(23),15,17,21-heptaén-9-one ;
- 4-fluoro-8,14-dioxa-10,19,20-triazatétracyclo[13.5.2.1^{2,6}.0^{18,21}]tricosa-1(20),2,4,6(23),15,17,21-heptaén-9-one ;
- 8,14-dioxa-10,19,20,23-tétraazatétracyclo[13.5.2.1^{2,6}.0^{18,21}]tricosa-1(20),2,4,6(23),15,17,21-heptaén-9-one ;
- 8,14-dioxa-10,19,20-triazatétracyclo[13.5.2.1^{2,6}.0^{18,21}]tricosa-1(20),2,4,6(23),15,17,21-heptaén-9-one ;
- 10-(propan-2-yl)-8,14-dioxa-4,10,19,20-tétraazatétracyclo[13.5.2.1^{2,6}.0^{18,21}]tricosa-1(20),2,4,6(23),15,17,21-heptaén-9-one ;
- 8,14-dioxa-5,10,19,20-tétraazatétracyclo[13.5.2.1^{2,6}.0^{18,21}]tricosa-1(20),2,4,6(23),15,17,21-heptaén-9-one ;
- 4-méthoxy-8,14-dioxa-10,19,20-triazatétracyclo[13.5.2.1^{2,6}.0^{18,21}]tricosa-1(20),2,4,6(23),15,17,21-heptaén-9-one ;
- 4-bromo-8,14-dioxa-10,19,20-triazatétracyclo[13.5.2.1^{2,6}.0^{18,21}]tricosa-1(20),2,4,6(23),15,17,21-heptaén-9-one ;
- 5-fluoro-8,14-dioxa-10,19,20-triazatétracyclo[13.5.2.1^{2,6}.0^{18,21}]tricosa-1(20),2,4,6(23),15,17,21-heptaén-9-one ;
- 5-méthyl-8,14-dioxa-10,19,20-triazatétracyclo[13.5.2.1^{2,6}.0^{18,21}]tricosa-1(20),2,4,6(23),15,17,21-heptaén-9-one ;
- 4-(pyrrolidin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[4-(propan-2-yl)pipérazin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-{2-oxa-6-azaspiro[3.4]octan-6-yl}-8,14-dioxa-10,19,20-triazatétracyclo[13.5.2.1^{2,6}.0^{18,21}]tricosa-1(20),2,4,6(23),15,17,21-heptaén-9-one ;
- 4-[4-(oxétan-3-yl)pipérazin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-(morpholin-4-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[(2R,6S)-2,6-diméthylmorpholin-4-yl]-8,14-dioxa-10,19,20-triazatétracyclo[13.5.2.1^{2,6}.0^{18,21}]tricosa-1(20),2,4,6(23),15,17,21-heptaén-9-one ;
- 4-méthyl-8,14-dioxa-10,19,20-triazatétracyclo[13.5.2.1^{2,6}.0^{18,21}]tricosa-1(20),2,4,6(23),15,17,21-heptaén-9-one ;
- 5-méthoxy-8,14-dioxa-10,19,20-triazatétracyclo[l3.5.2.1^{2,6}.0^{18,21}]tricosa-1(20),2,4,6(23),15,17,21-heptaén-9-one ;
- 4-(4,4-difluoropipéridin-1-yl)-8,14-dioxa-10,19,20-triazatétracyclo[13.5.2. 12,6.0^{18,21}]tricosa-1(20),2,4,6(23),15,17,21-heptaén-9-one ;
- 4-(3,3-difluoropyrrolidin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 7-méthyl-8,14-dioxa-10,19,20-triazatétracyclo[13.5.2.1^{2,6}.0^{18,21}]tricosa-1(20),2,4,6(23),15,17,21-heptaén-9-one ;
- 4-[4-(2-méthoxyéthyl)pipéridin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 9,14-dioxa-11,19,20-triazatétracyclo[13.5.2.1^{2,6}.0^{18,21}]tricosa-1(20),2,4,6(23),15,17,21-heptaén-10-one ;
- 4-[(3R)-3-hydroxypyrrolidin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-[(2-méthoxyéthyl)(méthyl)amino]-8,14-dioxa-10,19,20-triazatétracyclo[13.5.2.1^{2,6}.0^{18,21}]tricosa-1(20),2,4,6(23),15,17,21-heptaén-9-one ;
- 4-chloro-8,14-dioxa-10,19,20-triazatétracyclo[13.5.2.1^{2,6}.0^{18,21}]tricosa-1(20),2,4,6(23),15,17,21-heptaén-9-one ;
- 4-fluoro-5-méthyl-8,14-dioxa-10,19,20-triazatétracyclo[13.5.2.1^{2,6}.0^{18,21}]tricosa-1(20),2,4,6(23),15,17,21-heptaén-9-one ;
- 4,5-difluoro-8,14-dioxa-10,19,20-triazatétracyclo[13.5.2.1^{2,6}.0^{18,21}]tricosa-1(20),2,4,6(23),15,17,21-heptaén-9-one ;
- 5-bromo-8,14-dioxa-10,19,20-triazatétracyclo[13.5.2.1^{2,6}.0^{18,21}]tricosa-1(20),2,4,6(23),15,17,21-heptaén-9-one ;
- 4-(4-méthylpipérazin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 4-(3-méthoxyazétidin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 1-{9-oxo-8,14-dioxa-10,19,20-triazatétracyclo[13.5.2.1^{2,6}.0^{18,21}]tricosa-1(20),2,4,6(23),15,17,21-heptaén-4-yl}pipéridine-4-carbonitrile ;
- 4-[4-(pyrrolidin-1-yl)pipéridin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-(azétidin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 4-(pipéridin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 4-(2,5-dihydrofuran-3-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[4-(morpholin-4-yl)pipéridin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-(1-méthyl-1,2,3,6-tétrahydropyridin-4-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[(2S,5S)-2,5-diméthylmorpholin-4-yl]-8,14-dioxa-10,19,20-triazatétracyclo[13.5.2.1^{2,6}.0^{18,21}]tricosa-1(20),2,4,6(23),15,17,21-heptaén-9-one ;
- 4-[(morpholin-4-yl)méthyl]-8,14-dioxa-10,19,20-triazatétracyclo[13.5.2.1^{2,6}.0^{18,21}]tricosa-1(20),2,4,6(23),15,17,21-heptaén-9-one ;
- 4-[(pyrrolidin-1-yl)méthyl]-8,14-dioxa-10,19,20-triazatétracyclo[13.5.2.1^{2,6}.0^{18,21}]tricosa-1(20),2,4,6(23),15,17,21-heptaén-9-one ;
- 4-[(pipéridin-1-yl)méthyl]-8,14-dioxa-10,19,20-triazatétracyclo[13.5.2.1^{2,6}.0^{18,21}]tricosa-1(20),2,4,6(23),15,17,21-heptaén-9-one ;
- 4-[(4-méthylpipérazin-1-yl)méthyl]-8,14-dioxa-10,19,20-triazatétracyclo[13.5.2.1^{2,6}.0^{18,21}]tricosa-1(20),2,4,6(23),15,17,21-heptaén-9-one ;
- 5-(morpholin-4-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[4-(2-méthoxyéthyl)pipérazin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-(diéthylamino)-8,14-dioxa-10,19,20-triazatétracyclo[13.5.2.1^{2,6}.0^{18,21}]tricosa-1(20),2,4,6(23),15,17,21-heptaén-9-one ;
- 4-cyclopropyl-8,14-dioxa-10,19,20-triazatétracyclo[13.5.2.1^{2,6}.0^{18,21}]tricosa-1(20),2,4,6(23),15,17,21-heptaén-9-one ;
- 5-(4-méthylpipérazin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 13-méthyl-8,14-dioxa-10,19,20-triazatétracyclo[13.5.2.1^{2,6}.0^{18,21}]tricosa-1(20),2,4,6(23),15,17,21-heptaén-9-one ;
- 8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- 4-[méthyl(oxétan-3-yl)amino]-8,14-dioxa-10,19,20-triazatétracyclo[13.5.2. 12,6.0^{18,21}]tricosa-1(20),2,4,6(23),15,17,21-heptaén-9-one ;
- 4-[(diméthylamino)méthyl]-8,14-dioxa-10,19,20-triazatétracyclo[13.5.2.1^{2,6}.0^{18,21}]tricosa-1(20),2,4,6(23),15,17,21-heptaén-9-one ;
- 4,10-diméthyl-8,14-dioxa-10,19,20-triazatétracyclo[13.5.2.1^{2,6}.0^{18,21}]tricosa-1(20),2,4,6(23),15,17,21-heptaén-9-one ;
- 4-(propan-2-yloxy)-8,14-dioxa-10,19,20-triazatétracyclo[13.5.2.1^{2,6}.0^{18,21}]tricosa-1(20),2,4,6(23),15,17,21-heptaén-9-one ;
- 4-fluoro-7-méthyl-8,14-dioxa-10,19,20-triazatétracyclo[13.5.2.1^{2,6}.0^{18,21}]tricosa-1(20),2,4,6(23),15,17,21-heptaén-9-one ;
- 4-[1-(oxétan-3-yl)-1,2,3,6-tétrahydropyridin-4-yl]-8,14-dioxa-10,19,20-triazatétracyclo[13.5.2.1^{2,6}.0^{18,21}]tricosa-1(20),2,4,6(23),15,17,21-heptaén-9-one ;
- 4-(3-méthylpipéridin-1-yl)-8,14-dioxa-10,19,20-triazatétracyclo[13.5.2.1^{2,6}.0^{18,21}]tricosa-1(20),2,4,6(23),15,17,21-heptaén-9-one ;
- 4-[(3S)-3-hydroxypyrrolidin-1-yl]-8,14-dioxa-10,19,20-triazatétracyclo[13.5.2.1^{2,6}.0^{18,21}]tricosa-1(20),2,4,6(23),15,17,21-heptaén-9-one ;
- 4-fluoro-8,14-dioxa-10,19,20-triazapentacyclo[13.5.2.1^{2,6}.1^{7,10}.0^{18,21}]tétracosa-1(20),2(24),3,5,15(22),16,18(21)-heptaén-9-one ;
- 4-(oxolan-3-yl)-8,14-dioxa-10,19,20-triazatétracyclo[13.5.2.1^{2,6}.0^{18,21}]tricosa-1(20),2,4,6(23),15,17,21-heptaén-9-one ;
- (13S)-13-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (13R)-13-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-(1-méthyl-1H-pyrazol-3-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- (7S)-7-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-[2-(morpholin-4-yl)éthoxy]-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-(2-méthoxyéthyl)-8,14-dioxa-10,19,20-triazatétracyclo[13.5.2.1^{2,6}.0^{18,21}]tricosa-1(20),2(23),3,5,15(22),16,18(21)-heptaén-9-one ;
- (7R)-7-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 5-cyclopropyl-8,14-dioxa-10,19,20-triazatétracyclo[13.5.2.1^{2,6}.0^{18,21}]tricosa-1(20),2,4,6(23),15,17,21-heptaén-9-one ;
- 4-(2-méthoxyéthoxy)-8,14-dioxa-10,19,20-triazatétracyclo[13.5.2.1^{2,6}.0^{18,21}]tricosa-1(20),2,4,6(23),15,17,21-heptaén-9-one ;
- 4-fluoro-13-méthyl-8,14-dioxa-10,19,20-triazatétracyclo[13.5.2.1^{2,6}.0^{18,21}]tricosa-1(20),2,4,6(23),15,17,21-heptaén-9-one ;
- 11-méthyl-8,14-dioxa-10,19,20-triazatétracyclo[13.5.2.1^{2,6}.0^{18,21}]tricosa-1(20),2,4,6(23),15,17,21-heptaén-9-one ;
- 4-(3-oxomorpholin-4-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-(2-oxopyrrolidin-1-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 5-(2-oxopyrrolidin-1-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-(2-méthylpyrrolidin-1-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 2-{9-oxo-8,14-dioxa-10,19,20-triazatétracyclo[13.5.2.1^{2,6}.0^{18,21}]tricosa-1(20),2(23),3,5,15(22),16,18(21)-heptaén-4-yl}acétonitrile ;
- (11R)-11-méthyl-8,14-dioxa-10,19,20-triazatétracyclo[13.5.3.1^{2,6}.0^{18,21}]tricosa-1(20),2(23),3,5,15(22),16,18(21)-heptaén-9-one ;
- (11S)-11-méthyl-8,14-dioxa-10,19,20-triazatétracyclo[13.5.3.1^{2,6}.0^{18,21}]tricosa-1(20),2(23)3,5,15(22),16,18(21)-heptaén-9-one ;
- 4-éthynyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-(pipérazin-1-yl)-8,14-dioxa-10,19,20-triazatétracyclo[13.5.2.1^{2,6}.0^{18,21}]tricosa-1(20),2(23)3,5,15(22),16,18(21)-heptaén-9-one ;
- 4-(1,2,3,6-tétrahydropyridin-4-yl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 11-(méthoxyméthyl)-8,14-dioxa-10,19,20-triazatétracyclo[13.5.2.1^{2,6}.0^{18,21}]tricosa-1(20),2(23),3,5,15(22),16,18(21)-heptaén-9-one ;
- 8,14-dioxa-5,10,19,20,23-pentaazatétracyclo[13.5.2.1^{2,5}.0^{18,21}]tricosa-1(20),2(23),3,15(22),16,18(21)-hexaén-9-one ;
- 11-méthyl-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- 12-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 11-éthyl-8,14-dioxa-10,19,20-triazatétracyclo[13.5.2.1^{2,6}.0^{18,21}]tricosa-1(20)3(23)3,5,15(22),16,18(21)-heptaén-9-one ;
- 4-fluoro-5,7-diméthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 4-fluoro-5-méthoxy-7-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 5-fluoro-4,7-diméthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- 8,14-dioxa-10,19,20-triazapentacyclo[13.5.2.1^{2,6}.1^{7,10}.0^{18,21}]tétracosa-1(20),2(24),3,5,15(22),16,18(21)-heptaén-9-one ;
- 13-méthyl-8,14-dioxa-10,19,20,23-tétraazatétracyclo[13.5.2.1^{2,6}.0^{18,21}]tricosa-1(20),2(23),3,5,15(22),16,18(21)-heptaén-9-one ;
- 12-méthyl-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- 7-méthyl-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- 5-fluoro-4-méthoxy-7-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (7R,13R)-7,13-diméthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (13R)-13-méthyl-8,14-dioxa-4,5,10,19,20-pentaazatétracyclo[13.5.2.1^{2,5}.0^{18,21}]tricosa-1(20),2(23),3,15,17,21-hexaén-9-one ;
- 8,15-dioxa-4,10,20,21-tétraazapentacyclo[14.5.2.1^{2,6}.1^{10,13}.0^{19,22}]pentacosa-1(21),2(25), 3,5,16(23),17,19(22)-heptaén-9-one ;
- 8,14-dioxa-5,10,19,20-tétraazatétracyclo[13.5.2.1^{2,5}.0^{18,21}]tricosa-1(20),2(23),3,15(22),16,18(21)-hexaén-9-one ;
- (13S)-4-fluoro-13-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (13R)-4-fluoro-13-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (13R)-13-méthyl-8,14-dioxa-4,10,19,20-tétraazatétracyclo[13.5.2.1^{2,6}.0^{18,21}]tricosa-1(20),2(23),3,5,15(22),16,18(21)-heptaén-9-one ;
- 6-cyclopropyl-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- 7-éthyl-8,14-dioxa-10,19,20-triazatétracyclo[13.5.2.1^{2,6}.0^{18,21}]tricosa-1(20),2,4,6(23),15,17,21-heptaén-9-one ;
- (13R)-13-méthyl-8,14-dioxa-5,10,19,20,23-pentaazatétracyclo[13.5.2.1^{2,6}.0^{18,21}]tricosa-1(20),2,4,6(23),15,17,21-heptaén-9-one ;
- (7R,13R)-4-fluoro-7,13-diméthyl-8,14-dioxa-10,19,20-triazatétracyclo[13.5.2.1^{2,6}.0^{18,21}]tricosa-1(20),2,4,6(23),15,17,21-heptaén-9-one ;
- 7-méthyl-8,14-dioxa-4,10,19,20-tétraazatétracyclo[13.5.2.1^{2,6}.0^{18,21}]tricosa-1(20),2,4,6(23),15,17,21-heptaén-9-one ;
- (7R)-4-fluoro-7-méthyl-8,14-dioxa-10,19,20-triazatétracyclo[13.5.2.1^{2,6}.0^{18,21}]tricosa-1(20),2,4,6(23),15,17,21-heptaén-9-one ;
- (7S)-4-fluoro-7-méthyl-8,14-dioxa-10,19,20-triazatétracyclo[13.5.2.1^{2,6}.0^{18,21}]tricosa-1(20),2,4,6(23),15,17,21-heptaén-9-one ;
- 6-méthyl-8,14-dioxa-4,5,10,19,20-pentaazatétracyclo[13.5.2.1^{2,5}.0^{18,21}]tricosa-1(20),2(23),3,15,17,21-hexaén-9-one ;
- 7-méthyl-8,14-dioxa-10,19,20,23-tétraazatétracyclo[13.5.2.1^{2,6}.0^{18,21}]tricosa-1(20),2,4,6(23),15,17,21-heptaén-9-one ;
- 6-(propan-2-yl)-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- (13R)-7,13-diméthyl-8,14-dioxa-4,5,10,19,20-pentaazatétracyclo[13.5.2.1^{2,5}.0^{18,21}]tricosa-1(20),2(23),3,15(22),16,18(21)-hexaén-9-one ;
- (13R)-13-méthyl-8,14-dioxa-10,19,20,23-tétraazatétracyclo[13.5.2.1^{2,6}.0^{18,21}]tricosa-1(20),2,4,6(23),15,17,21-heptaén-9-one ;
- (7R)-7-éthyl-8,14-dioxa-10,19,20-triazatétracyclo[13.5.2.1^{2,6}.0^{18,21}]tricosa-1(20),2,4,6(23),15, 17,21-heptaén-9-one ;
- (7S)-7-éthyl-8,14-dioxa-10,19,20-triazatétracyclo[13.5.2.1^{2,6}.0^{18,21}]tricosa-1(20),2,4,6(23),15,17,21-heptaén-9-one ;
- (13R)-13-méthyl-8,14-dioxa-5,10,19,20-tétraazatétracyclo[13.5.2.1^{2,6}.0^{18,21}]tricosa-1(20),2,4,6(23),15,17,21-heptaén-9-one ;
- 6-(oxan-4-yl)-8,14-dioxa-4,5,10,19,20-pentaazatétracyclo[13.5.2.1^{2,5}.0^{18,21}]tricosa-1(20),2(23),3,15,17,21-hexaén-9-one ;
- 4-éthyl-8,14-dioxa-5,10,19,20,23-pentaazatétracyclo[13.5.2.1^{2,5}.0^{18,21}]tricosa-1(20),2(23),15,17,21-pentaén-9-one ;
- (13R)-23-fluoro-13-méthyl-8,14-dioxa-10,19,20-triazatétracyclo[13.5.2.1^{2,6}.0^{18,21}]tricosa-1(20),2,4,6(23),15,17,21-heptaén-9-one ;
- 9,14-dioxa-4,5,11,19,20-pentaazatétracyclo[13.5.2.1^{2,5}.0^{18,21}]tricosa-1(20),2(23),3,15,17,21-hexaén-10-one ;
- 4-éthyl-8,14-dioxa-5,10,19,20,23-pentaazatétracyclo[13.5.2.1^{2,5}.0^{18,21}]tricosa-1(20),2(23),3,15,17,21-hexaén-9-one ;
- 3,9,15-trioxa-4,11,20,21-tétraazatétracyclo[14.5.2.1^{2,5}.0^{18,22}]tétracosa-1(21),2(24),4,16,18,22-hexaén-10-one ;
- (13R)-16-fluoro-13-méthyl-8,14-dioxa-4,10,19,20-tétraazatétracyclo[13.5.2.1^{2,6}.0^{18,21}]tricosa-1(20),2,4,6(23),15,17,21-heptaén-9-one ;
- (13R)-4-chloro-13-méthyl-8,14-dioxa-10,19,20,23-tétraazatétracyclo[13.5.2.1^{2,6}.0^{18,21}]tricosa-1(20),2(23),3,5,15(22),16,18(21)-heptaén-9-one ;
- 8,14-dioxa-2,4,10,19,20-pentaazatétracyclo[13.5.2.1^{2,5}.0^{18,21}]tricosa-1(20),3,5(23),15(22),16,18(21)-hexaén-9-one ;
- (13R)-4-méthoxy-13-méthyl-8,14-dioxa-10,19,20,23-tétraazatétracyclo[13.5.2.1^{2,6}.0^{18,21}]tricosa-1(20),2,4,6(23),15,17,21-heptaén-9-one ;
- (13R)-13-méthyl-9-oxo-8,14-dioxa-10,19,20-triazatétracyclo[13.5.2.1^{2,6}.0^{18,21}]tricosa-1(20),2,4,6(23),15,17,21-heptaène-5-carbonitrile ;
- (13R)-13-méthyl-4-(pyrrolidin-1-yl)-8,14-dioxa-5,10,19,20,23-pentaazatétracyclo[13.5.2.1^{2,6}.0^{18,21}]tricosa-1(20),2,4,6(23),15,17,21-heptaén-9-one ;
- (7S,13R)-7,13-diméthyl-8,14-dioxa-5,10,19,20,23-pentaazatétracyclo[13.5.2.1^{2,6}.0^{18,21}]tricosa-1(20),2,4,6(23),15,17,21-heptaén-9-one ;
- (7R,13R)-7,13-diméthyl-8,14-dioxa-5,10,19,20,23-pentaazatétracyclo[13.5.2.1^{2,6}.0^{18,21}]tricosa-1(20),2,4,6(23),15,17,21-heptaén-9-one ;
- (13R)-16-fluoro-13-méthyl-8,14-dioxa-10,19,20-triazatétracyclo[13.5.2.1^{2,6}.0^{18,21}]tricosa-1(20),2,4,6(23),15,17,21-heptaén-9-one ;
- (13R)-13-méthyl-8,14-dioxa-4,10,19,20,23-pentaazatétracyclo[13.5.2.1^{2,6}.0^{18,21}]tricosa-1(20),2,4,6(23),15,17,21-heptaén-9-one ;
- 8,14-dioxa-4-thia-10,19,20,23-tétraazatétracyclo[13.5.2.1^{2,5}.0^{18,21}]tricosa-1(20),2,5(23),15,17,21-hexaén-9-one ;
- 8,14-dioxa-3-thia-10,19,20,23-tétraazatétracyclo[13.5.2.1^{2,5}.0^{18,21}]tricosa-1(20),2(23),4,15,17,21-hexaén-9-one ;
- (7R,13R)-7,13-diméthyl-8,14-dioxa-10,19,20,23-tétraazatétracyclo[13.5.2.1^{2,6}.0^{18,21}]tricosa-1(20),2,4,6(23),15,17,21-heptaén-9-one ;
- (13R)-4-[(3R)-3-méthoxypyrrolidin-1-yl]-13-méthyl-8,14-dioxa-5,10,19,20,23-pentaazatétracyclo[13.5.2.1^{2,6}.0^{18,21}]tricosa-1(20),2,4,6(23),15,17,21-heptaén-9-one ;
- (13R)-16-chloro-13-méthyl-8,14-dioxa-10,19,20-triazatétracyclo[13.5.2.1^{2,6}.0^{18,21}]tricosa-1(20),2,4,6(23),15,17,21-heptaén-9-one ;
- (13R)-13,16-diméthyl-8,14-dioxa-10,19,20-triazatétracyclo[13.5.2.1^{2,6}.0^{18,21}]tricosa-1(20),2,4,6(23),15,17,21-heptaén-9-one ;
- (13R)-13-méthyl-8,14-dioxa-3,10,19,20,23-pentaazatétracyclo[13.5.2.1^{2,6}.0^{18,21}]tricosa-1(20),2,4,6(23),15,17,21-heptaén-9-one ;
- chlorhydrate de 8-oxa-10,14,19,20-tétraazatétracyclo[13.5.2.1^{2,6}.0^{18,21}]tricosa-1(20),2(23),3,5,15(22),16,18(21)-heptaén-9-one ;
- 8-oxa-10,19,20-triazatétracyclo[13.5.2.1^{2,6}.0^{18,21}]tricosa-1(20),2(23),3,5,15(22),16,18(21)-heptaén-9-one ;
- (13R)-5-méthoxy-13-méthyl-8,14-dioxa-4,10,19,20-tétraazatétracyclo[13.5.2.1^{2,6}.0^{18,21}]tricosa-1(20),2,4,6(23),15,17,21-heptaén-9-one ;
- (13R)-13-méthyl-8,14-dioxa-4,10,19,20-tétraazatétracyclo[13.5.2.1^{2,6}.0^{18,21}]tricosa-1(20),2,6(23),15,17,21-hexaène-5,9-dione ;
- 4-méthyl-8,14-dioxa-3,4,10,19,20-pentaazatétracyclo[13.5.2.1^{2,5}.0^{18,21}]tricosa-1(20),2,5(23),15(22),16,18(21)-hexaén-9-one ;
- (13R)-16-fluoro-13-méthyl-8,14-dioxa-10,19,20,23-tétraazatétracyclo[13.5.2.1^{2,6}.0^{18,21}]tricosa-1(20),2,4,6(23),15,17,21-heptaén-9-one ;
- 7,13-dioxa-4-thia-9,18,19,22-tétraazatétracyclo[12.5.2.1^{2,5}.0^{17,20}]docosa-1(19),2,5(22),14(21),15,17(20)-hexaén-8-one ;
- (13R)-4,13-diméthyl-8,14-dioxa-5,10,19,20,23-pentaazatétracyclo[13.5.2.1^{2,6}.0^{18,21}]tricosa-1(20),2(23),3,5,15(22),16,18(21)-heptaén-9-one ;
- 8,14-dioxa-23-thia-4,10,19,20-tétraazatétracyclo[13.5.2.1^{2,5}.0^{18,21}]tricosa-1(20),2,4,15(22),16,18(21)-hexaén-9-one ;
- (7S,13R)-7,13-diméthyl-8,14-dioxa-10,19,20,23-tétraazatétracyclo[13.5.2.1^{2,6}.0^{18,21}]tricosa-1(20),2(23),3,5,15(22),16,18(21)-heptaén-9-one ;
- (13R)-13-méthyl-9-oxo-8,14-dioxa-5,10,19,20-tétraazatétracyclo[13.5.2.1^{2,5}.0^{18,21}]tricosa-1(20),2(23),3,15(22),16,18(21)-hexaène-4-carbonitrile ;
- 12,12-difluoro-8,14-dioxa-10,19,20-triazatétracyclo[13.5.2.1^{2,6}.0^{18,21}]tricosa-1(20),2(23),3,5,15(22),16,18(21)-heptaén-9-one ;
- (13R)-17-fluoro-13-méthyl-8,14-dioxa-10,19,20-triazatétracyclo[13.5.2.1^{2,6}.0^{18,21}]tricosa-1(20),2(23),3,5,15(22),16,18(21)-heptaén-9-one ;
- (7S,13R)-7,13-diméthyl-8,14-dioxa-4,10,19,20,23-pentaazatétracyclo[13.5.2.1^{2,6}.0^{18,21}]tricosa-1(20),2(23),3,5,15(22),16,18(21)-heptaén-9-one ;
- (7R,13R)-7,13-diméthyl-8,14-dioxa-4,10,19,20,23-pentaazatétracyclo[13.5.2.1^{2,6}.0^{18,21}]tricosa-1(20),2(23),3,5,15(22),16,18(21)-heptaén-9-one ;
- (13S)-13-méthyl-8,14-dioxa-4,10,19,20,23-pentaazatétracyclo[13.5.2.1^{2,6}.01^{8,21}]tricosa-1(20),2(23),3,5,15(22),16,18(21)-heptaén-9-one ;
- (13R)-13-méthyl-8,14-dioxa-10,19,20,22-tétraazatétracyclo[13.5.2.1^{2,6}.0^{18,21}]tricosa-1(20),2(23),3,5,15,17,21-heptaén-9-one ;
- (12R)-4,12-diméthyl-7,13-dioxa-4,9,18,19,22-pentaazatétracyclo[12.5.2.1^{2,5}.0^{17,20}]docosa-1(19),2,5(22),14(21),15,17(20)-hexaén-8-one ;
- (13R)-13-méthyl-8,14-dioxa-4,5,10,19,20,23-hexaazatétracyclo[13.5.2.1^{2,5}.0^{18,21}]tricosa-1(20),2(23),3,15,17,21-hexaén-9-one ;
- (13R)-13-méthyl-8,14-dioxa-23-thia-4,10,19,20-tétraazatétracyclo[13.5.2.1^{2,5}.0^{18,21}]tricosa-1(20),2,4,15,17,21-hexaén-9-one ;
- (13R)-4,13-diméthyl-8,14-dioxa-4,10,19,20,23-pentaazatétracyclo[13.5.2.1^{2,5}.0^{18,21}]tricosa-1(20),2,5(23),15(22),16,18(21)-hexaén-9-one ;
- (13R)-13-méthyl-8,14-dioxa-10,16,19,20-tétraazatétracyclo[13.5.2.1^{2,6}.0^{18,21}]tricosa-1(20),2(23),3,5,15(22),16,18(21)-heptaén-9-one ;
- 14-méthyl-8-oxa-10,14,19,20-tétraazatétracyclo[13.5.2.1^{2,6}.0¹⁸,²¹]tricosa-1(20),2(23),3,5,15,17,21-heptaén-9-one ;
- (13R)-13-méthyl-8,14-dioxa-4,10,19,20,22-pentaazatétracyclo[13.5.2.1^{2,6}.0^{18,21}]tricosa-1(20),2(23),3,5,15,17,21-heptaén-9-one ;
- (13R)-13-méthyl-8,14-dioxa-10,17,19,20-tétraazatétracyclo[13.5.2.1^{2,6}.0^{18,21}]tricosa-1(20),2(23),3,5,15(22),16,18(21)-heptaén-9-one ;
- 8,14-dioxa-4,5,10,19,20,23-hexaazatétracyclo[13.5.2.1^{2,5}.0^{18,21}]tricosa-1 (20),2(23),3,15(22),16,18(21)-hexaén-9-one ;
- 12,12-difluoro-8,14-dioxa-4,5,10,19,20,23-hexaazatétracyclo[13.5.2.1^{2,5}.0^{18,21}]tricosa-1(20),2(23),3,15(22),16,18(21)-hexaén-9-one ;
- (12R)-12-fluoro-8,14-dioxa-10,19,20-triazatétracyclo[13.5.2.1^{2,6}.0^{18,21}]tricosa-1(20),2(23),3,5,15(22),16,18(21)-heptaén-9-one ;
- (12S)-12-fluoro-8,14-dioxa-10,19,20-triazatétracyclo[13.5.2.1^{2,6}.0^{18,21}]tricosa-1(20),2(23),3,5,15(22),16,18(21)-heptaén-9-one ;
- 12,12-difluoro-8,14-dioxa-4,10,19,20,23-pentaazatétracyclo[13.5.2.1^{2,6}.0^{18,21}]tricosa-1(20),2(23),3,5,15(22),16,18(21)-heptaén-9-one ;
- (12S)-12-fluoro-8,14-dioxa-4,10,19,20,23-pentaazatétracyclo[13.5.2.1^{2,6}.0^{18,21}]tricosa-1(20),2(23),3,5,15,17,21-heptaén-9-one ;
- (12R)-12-fluoro-8,14-dioxa-4,10,19,20,23-pentaazatétracyclo[13.5.2.1^{2,6}.0^{18,21}]tricosa-1(20),2(23),3,5,15,17,21-heptaén-9-one ;
- (12S)-12-fluoro-8,14-dioxa-4,5,10,19,20,23-hexaazatétracyclo[13.5.2.1^{2,5}.0^{18,21}]tricosa-1(20),2(23),3,15,17,21-hexaén-9-one ;
- (12R)-12-fluoro-8,14-dioxa-4,5,10,19,20,23-hexaazatétracyclo[13.5.2.1^{2,5}.0^{18,21}]tricosa-1(20),2(23),3,15,17,21-hexaén-9-one ; ou
- 8',14'-dioxa-10',19',20'-triazaspiro[cyclopropane-1,13'-tétracyclo[13.5.2.1^{2,6}.0^{18,21}]tricosane]-1'(20'),2'(23'),3',5',15'(22'),16',18'(21')-heptaén-9'-one.

31. Composition pharmaceutique comprenant un composé de formule (I) selon l'une quelconque des revendications 1 à 30 ou un sel d'addition de celui-ci avec un acide ou une base pharmaceutiquement acceptable en combinaison avec un ou plusieurs excipients pharmaceutiquement acceptables.

32. Composition pharmaceutique selon la revendication 31 pour utilisation dans le traitement de maladies neurologiques, maladies inflammatoires, infections bactériennes, virales et parasitaires, maladies cardiovasculaires, maladies autoimmunes, cancers et troubles endosomaux-lysosomaux choisis parmi maladie de Niemann-Pick de type A, B ou C, maladie de Gaucher, maladie de Krabbe, maladie de Fabry et troubles à déficits mitochondriaux.

33. Composition pharmaceutique pour utilisation selon la revendication 32, la maladie neurologique étant choisie parmi maladie de Parkinson, maladie d'Alzheimer, sclérose latérale amyotrophique (SLA), démence, neuropathie diabétique, troubles de la mémoire liés à l'âge, troubles cognitifs légers, maladie des grains argyrophiles, maladie de Pick, épilepsie, tauopathies telles que paralysie supranucléaire progressive et dégénérescence corticobasale, autres synucléinopathies telles que atrophie du système multiple, démence frontotemporale, démence frontotemporale héréditaire et parkinsonisme lié au chromosome 17 (FTDP-17), symptômes de sevrage/rechute associés à la toxicomanie, dyskinésie induite par L-Dopa, accident vasculaire cérébral ischémique, lésion cérébrale traumatique, lésion de la moelle épinière et sclérose en plaques.

34. Composition pharmaceutique pour utilisation selon la revendication 33, la maladie neurologique étant la maladie de Parkinson ou la maladie d'Alzheimer.

35. Composition pharmaceutique pour utilisation selon la revendication 32, la maladie inflammatoire étant choisie parmi vascularites, maladies pulmonaires telles que broncho-pneumopathie chronique obstructive, fibrose pulmonaire idiopathique, myopathies inflammatoires et spondylarthrite ankylosante.

36. Composition pharmaceutique pour utilisation selon la revendication 32, la maladie auto-immune étant choisie parmi maladie de Crohn, maladies inflammatoires de l'intestin, polyarthrite rhumatoïde, colite ulcéreuse, lupus, anémie hémolytique auto-immune, aplasie érythrocytaire pure, purpura thrombocytopénique idiopathique, diabète sucré de type I, obésité, syndrome d'Evans, affections cutanées bulleuses, syndrome de Sjögren, maladie de Devic et lèpre.

37. Composition pharmaceutique pour utilisation selon la revendication 32, le cancer étant choisi parmi cancer de la thyroïde, cancer du rein, cancer du sein, cancer hormonodépendant, cancer du poumon adénosquameux, cancer du poumon non à petites cellules, cancer du côlon, cancer de la prostate, cancers de la peau, leucémies et lymphomes.

38. Composition pharmaceutique selon la revendication 32, la maladie cardiovasculaire étant un accident vasculaire cérébral.

39. Composition pharmaceutique pour utilisation selon la revendication 32, les infections bactériennes ou virales étant choisies parmi lèpre, tuberculose, SARS-CoV, MERS-CoV et SARS-CoV-2, VIH, virus du Nil occidental et virus du chikungunya.

40. Composé de formule (I) selon l'une quelconque des revendications 1 à 30, ou un sel d'addition de celui-ci avec un acide ou une base pharmaceutiquement acceptable, pour utilisation dans le traitement de la maladie de Parkinson, de la maladie d'Alzheimer, de la sclérose latérale amyotrophique (SLA), de la démence, de la neuropathie diabétique, des troubles de la mémoire liés à l'âge, des troubles cognitifs légers, de la maladie des grains argyrophiles, de la maladie de Pick, de l'épilepsie, des tauopathies telles que la paralysie supranucléaire progressive et la dégénérescence corticobasale, d'autres synucléinopathies, de la démence frontotemporale, de la démence frontotemporale héréditaire et du parkinsonisme lié au chromosome 17 (FTDP-17), des symptômes de sevrage/rechute associés à la toxicomanie, de la dyskinésie induite par L-Dopa, d'accident vasculaire cérébral ischémique, de lésion cérébrale traumatique, de lésion de la moelle épinière, de la sclérose en plaques, de la maladie de Niemann-Pick de type A, B ou C, de la maladie de Gaucher, de la maladie de Krabbe, de la maladie de Fabry, des troubles à déficits mitochondriaux, de la maladie de Crohn, de la maladie inflammatoire de l'intestin, de la polyarthrite rhumatoïde, de la colite ulcéreuse, du lupus, de l'anémie hémolytique auto-immune, de l'aplasie érythrocytaire pure, du purpura thrombocytopénique idiopathique, du diabète sucré de type I, de l'obésité, du syndrome d'Evans, des troubles cutanés bulleux, de la maladie de Devic, de la lèpre, du cancer de la thyroïde, du cancer du rein (y compris cancer papillaire du rein), du cancer du sein, du cancer hormonodépendant, du cancer du poumon adénosquameux, du cancer du poumon non à petites cellules, du cancer du côlon, des cancers de la prostate, des cancers de la peau, des leucémies (y compris leucémie myélogène aiguë), des lymphomes, des accidents vasculaires cérébraux, de la lèpre, de la tuberculose, et des infections par le SRAS-CoV, le MERS-CoV, le SRAS-CoV-2, le VIH, le virus du Nil occidental et le virus du chikungunya.
